# EUROPEAN PATENT APPLICATION

(11) **EP 4 671 252 A1**
(43) Date of publication of application: **31.12.2025**
(21) Application number: 24774261.2
(22) Date of filing: 22.03.2024
(51) Int. Cl.: C07D 519/00, C07D 403/14, C07D 403/04, C07D 491/052, A61K 31/517, A61K 31/519, A61P 35/00, A61P 35/02

(54) **HETEROCYCLIC-SUBSTITUTED PYRIMIDOPYRAN COMPOUND AND USE THEREOF**

(30) Priority: 23.03.2023 CN 202310290481; 30.11.2023 CN 202311634664; 18.03.2024 CN 202410309827
(71) Applicant: D3 Bio(Wuxi) Co., Ltd., Wuxi, JiangSu, 214092 (CN)
(72) Inventor: CHEN, Shuhui, Shanghai 200131 (CN); CHEN, Zhijian, Shanghai 200120 (CN); MOOK, Jr. Robert A., Chapel Hill North Carolina 27516 (US); WU, Wentao, Shanghai 200131 (CN); YANG, Ping, Shanghai 200131 (CN); ZHANG, Jing, Shanghai 200120 (CN); ZHANG, Yang, Shanghai 200131 (CN); ZHU, Jiuxiang, Shanghai 200092 (CN)
(74) Representative: Cabinet Becker et Associés
(86) International application number: PCT/CN2024/083328
(87) International publication number: WO 2024/193698

(57) **Abstract**

Disclosed in the present invention are a piperazine bridge ring-substituted pyrimidopyran compound and a use thereof, and particularly disclosed are a compound as represented by formula (I"), (I'), or (I), a stereoisomer thereof, and a pharmaceutically acceptable salt thereof.

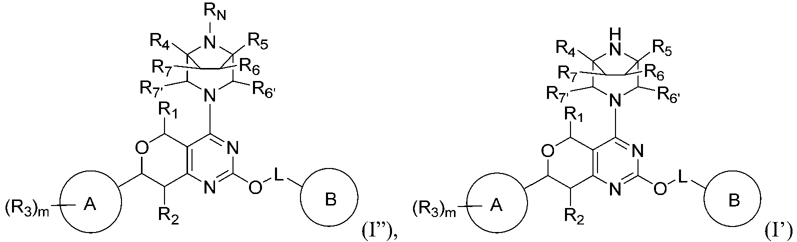

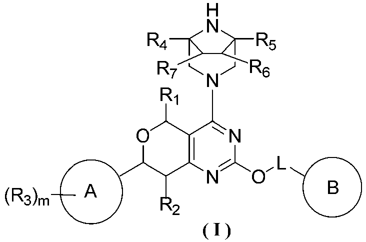

## Description

### TECHNICAL FIELD

The present disclosure relates to a class of bridged piperazine ring substituted pyrimidopyran compounds and uses thereof, and specifically to a compound represented by formula (I"), formula (1'), or formula (I), a stereoisomer thereof, and a pharmaceutically acceptable salt thereof.

### BACKGROUND

KRAS is the most common oncogenic mutation gene. KRAS mutations occurs in approximately 1 in 7 cancers. KRAS mutation/KRAS amplification is most common in colorectal cancer (US: ~45%, China: ~49%), pancreatic cancer (US: ~90%, China: ~87%), and non-small cell lung cancer (US: ~35%, China: ~13%). Among them, KRAS^{G12D}, KRAS^{G12V}, and KRAS^{G12C} account for the largest proportion.

KRAS is a murine sarcoma viral oncogene and an important member of the RAS proteins. KRAS acts like a molecular switch that regulates the path of cell growth when its function is normal.After KRAS gene mutation, it can independently transmit growth and proliferation signals to downstream pathways independent of upstream growth factor receptor signaling, resulting in uncontrolled cell growth and tumor progression. At the same time, whether the KRAS gene is mutated is also an important indicator of tumor prognosis.

At present, small molecules that directly target KRAS mutations are mainly concentrated in the field of KRAS^{G12C}. Among them, AMG510 from Amgen and MRTX849 from Mirati Therapeutics have both been approved for marketing, both of which have shown good therapeutic effects on tumor patients with KRAS^{G12C} mutations. Also, MRTX1133, a small molecule drug targeting KRAS^{G12D} mutations, has entered Phase I clinical trials and demonstrated excellent anti-tumor properties in preclinical trials. However, this class of compounds still has some problems, and tumor patients with KRAS^{G12D} mutations have not yet benefited from precision medicine, and the continued development of small molecule inhibitors targeting KRAS^{G12D} is of great significance.

The present disclosure discloses a series of small molecule inhibitors targeting KRAS^{G12D} and methods of preparing the same.

### SUMMARY

In some aspects, the present disclosure provides a compound represented by formula (I") or a stereoisomer thereof, or a pharmaceutically acceptable salt thereof: 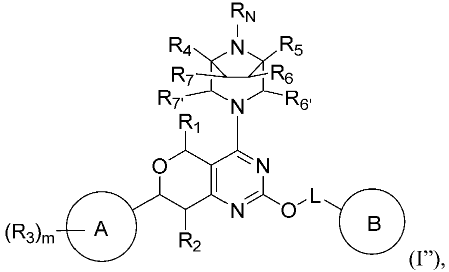 wherein,
R_{N} is selected from H and C₁₋₃ alkyl, wherein the C₁₋₃ alkyl is optionally substituted with 1, 2 or 3 F or Cl;
Ring A is selected from C₆ aryl and 5- to 6-membered heteroaryl;
Ring B is selected from 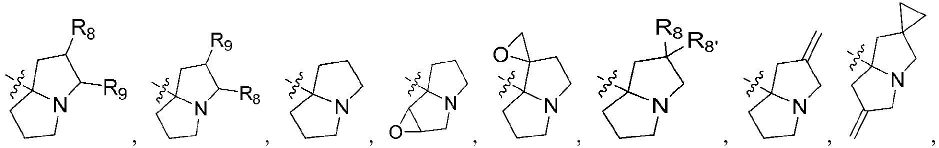 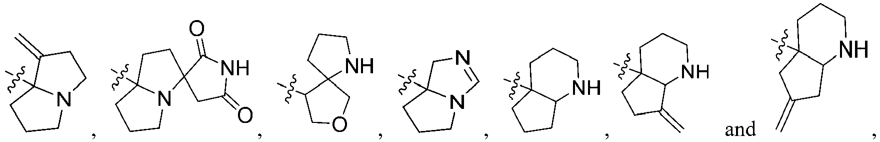
wherein ring B is optionally substituted with 1, 2, 3 or 4 R₁₀;
L is selected from -C(R_{L1}R_{L2})-, wherein R_{L1} and R_{L2} are each independently selected from H, D and C₁₋₃ alkyl;
R₁ and R₂ are each independently selected from oxo, H, F, Cl, Br, I and CN;
each R₃ is independently selected from F, Cl, Br, I, OH, NH₂, CN, C₁₋₃ alkyl, C₁₋₃ alkoxy, C₁₋₃ alkylamino, di-C₁₋₃ alkylamino, C₂₋₄ alkenyl, C₂₋₄ alkynyl and C₃₋₅ cycloalkyl, wherein the C₁₋₃ alkyl, C₁₋₃ alkoxy, C₁₋₃ alkylamino, di-C₁₋₃ alkylamino, C₂₋₄ alkenyl, C₂₋₄ alkynyl and C₃₋₅ cycloalkyl are each independently optionally substituted with 1, 2, 3, 4 or 5 Rₐ;
each Rₐ is independently selected from D, F, Cl, Br and I;
R₄, R₅*,* R₆, R₇, R_{6'} and R_{7'} are each independently selected from oxo, H, F, Cl, Br, I, OH, NH₂, CN, C₁₋₃ alkyl, C₂₋₄ alkenyl, C₁₋₃ alkoxy, -C(=O)-R_{d}, -C(=O)-NR_{b1}R_{b2} and =NO(C₁₋₃ alkyl), wherein the C₁₋₃ alkyl, C₂₋₄ alkenyl and C₁₋₃ alkoxy are each independently optionally substituted with 1, 2, 3, 4 or 5 R_{b};
alternatively, R₆ and R₇, together with the carbon atom to which they are attached, form a 3- to 5-membered heterocycloalkyl group;
each R_{b} is independently selected from D, F, Cl, Br, I, OH, NH₂, CN, C₁₋₃ alkoxy, and -C(=O)-NR_{b1}R_{b2};
R₈ is selected from H, F, Cl, Br, I, OH, NH₂, CN, C₁₋₃ alkyl and C₁₋₃ alkoxy, wherein each of the C₁₋₃ alkyl and C₁₋₃ alkoxy is independently optionally substituted with 1, 2, 3, 4 or 5 Rₐ;
alternatively, R₈ and R_{8'}, together with the carbon atom to which they are attached, form a C₃₋₅ cycloalkyl or a 3- to 5-membered heterocycloalkyl, wherein the C₃₋₅ cycloalkyl and the 3- to 5-membered heterocycloalkyl are each independently optionally substituted with 1, 2 or 3 R₁₀;
R₉ is selected from -C(=O)-NR_{b3}R_{b4} and -CH₂R_{c};
each R₁₀ is independently selected from oxo, D, F, Cl, Br, I, OH, NH₂, CN, C₁₋₃ alkyl, C₁₋₃ alkoxy, C₁₋₃ alkylamino, di-C₁₋₃ alkylamino, -C(=O)-R_{d}, -S-R_{d}, -S(=O)-R_{d}, -S(=O)₂-R_{d}, -NH-C(=O)-R_{d}, C₆₋₁₀ aryl, and 5-to 10-membered heteroaryl, wherein the C₁₋₃ alkyl is optionally substituted with 1, 2, or 3 OH or F, and the C₆₋₁₀ aryl and the 5- to 10-membered heteroaryl are each independently optionally substituted with 1, 2, 3, 4, or 5 Rₛ₁;
R_{b1} and R_{b2} are each independently selected from H, C₁₋₆ alkyl, C₁₋₆ alkoxy, C₃₋₁₀ cycloalkyl, 3- to 10-membered heterocycloalkyl, C₆₋₁₀ aryl and 5- to 10-membered heteroaryl, wherein the C₁₋₆ alkyl, C₁₋₆ alkoxy, C₃₋₁₀ cycloalkyl, 3- to 10-membered heterocycloalkyl, C₆₋₁₀ aryl and 5- to 10-membered heteroaryl are each independently optionally substituted with 1, 2, 3 or 4 Rₑ₁;
alternatively, R_{b1} and R_{b2}, together with the nitrogen atom to which they are attached, form a 3- to 6-membered heterocycloalkyl group, wherein the 3- to 6-membered heterocycloalkyl group is optionally substituted with 1, 2, 3 or 4 Rₑ₁;
R_{b3} and R_{b4} are each independently selected from H, C₁₋₆ alkyl, C₁₋₆ alkoxy, C₃₋₁₀ cycloalkyl, 3- to 10-membered heterocycloalkyl, C₆₋₁₀ aryl and 5- to 10-membered heteroaryl, wherein the C₁₋₆ alkyl, C₁₋₆ alkoxy, C₃₋₁₀ cycloalkyl, 3- to 10-membered heterocycloalkyl, C₆₋₁₀ aryl and 5- to 10-membered heteroaryl are each independently optionally substituted with 1, 2, 3 or 4 Rₑ₂;
alternatively, R_{b3} and R_{b4}, together with the nitrogen atom to which they are attached, form a 3- to 6-membered heterocycloalkyl group, wherein the 3- to 6-membered heterocycloalkyl group is optionally substituted with 1, 2, 3 or 4 Rₑ₂;
R_{c} is selected from F, Cl, Br, I, OH, NH₂, -(C=O)NR_{C1}R_{C2}, -O(C=O)NR_{C1}R_{C2}, -NR_{C0}(C=O)R_{C1} and - NR_{C0}(C=O)NR_{C1}R_{C2};
R_{C0}, R_{C1} and R_{C2} are each independently selected from H, C₁₋₆ alkyl, C₃₋₆ cycloalkyl and 3- to 6-membered heterocycloalkyl;
R_{d} is C₁₋₃ alkyl;
Rₑ₁ is selected from F, Cl, Br, I, OH, NH₂, NO₂, C₁₋₃ alkyl, C₁₋₃ alkylamino, di-C₁₋₃ alkylamino, CN, C₁₋₃ alkoxy, -S(=O)₂-(C₁₋₃ alkyl), -(C=O)(C₁₋₃ alkyl), -(C=O)O(C₁₋₃ alkyl), -(C=O)NH(C₁₋₃ alkyl), -(C=O)N(C₁₋₃ alkyl)₂, C₃₋₁₀ cycloalkyl, 3- to 10-membered heterocycloalkyl, C₆₋₁₀ aryl and 5- to 10-membered heteroaryl;
Rₑ₂ is selected from F, Cl, Br, I, OH, NH₂, NO₂, C₁₋₃ alkyl, C₁₋₃ alkylamino, di-C₁₋₃ alkylamino, CN, C₁₋₃ alkoxy, -S(=O)₂-(C₁₋₃ alkyl), -(C=O)(C₁₋₃ alkyl), -(C=O)O(C₁₋₃ alkyl), -(C=O)NH(C₁₋₃ alkyl), -(C=O)N(C₁₋₃ alkyl)₂, C₃₋₁₀ cycloalkyl, 3- to 10-membered heterocycloalkyl, C₆₋₁₀ aryl and 5- to 10-membered heteroaryl, wherein the C₃₋₁₀ cycloalkyl, 3- to 10-membered heterocycloalkyl, C₆₋₁₀ aryl and 5- to 10-membered heteroaryl are each independently optionally substituted with 1, 2, 3, 4 or 5 Rₛ₁, and wherein the C₁₋₃ alkyl and C₁₋₃ alkoxy are each independently optionally substituted with 1, 2, 3, 4 or 5 Rₛ₂;
alternatively, two or more Rₑ₂, together with the carbon atom (s) to which they are attached, form a C₆ aryl group or a 5- or 6-membered heteroaryl group;
Rₛ₁ is selected from oxo, F, Cl, Br, I, OH, NH₂, NO₂, C₁₋₆ alkyl, C₁₋₆ alkylamino, di-C₁₋₆ alkylamino, CN, C₁₋₆ alkoxy, -S(=O)₂-(C₁₋₃ alkyl), -(C=O)(C₁₋₃ alkyl), -(C=O)O(C₁₋₃ alkyl), -(C=O)NH(C₁₋₃ alkyl) and - (C=O)N(C₁₋₃ alkyl)₂;
Rₛ₂ is selected from F, Cl, Br, I, OH, NH₂, C₁₋₆ alkylamino, di-C₁₋₆ alkylamino, CN, C₁₋₆ alkoxy, - S(=O)₂-(C₁₋₃ alkyl), -(C=O)(C₁₋₃ alkyl), -(C=O)O(C₁₋₃ alkyl), -(C-O)NH(C₁₋₃ alkyl) and -(C=O)N(C₁₋₃ alkyl)₂; and
m is selected from 0, 1, 2, 3, 4 and 5;
with the proviso that,
   1) when ring B is 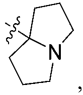 wherein the 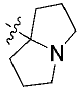 is substituted with one R₁₀, and R₁₀ is F, at least one of R₁, R₂, R₄, R₅*,* R₆, R₇, R₆, and R_{7'} is not H;
   2) when ring B is 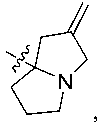 wherein the 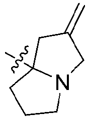 is optionally substituted with 1, 2, 3 or 4 R₁₀, at least one of R₁, R₂, R₄, R₅*,* R₆, R₇, R₆, and R_{7'} is not H; and
   3) the compound is not

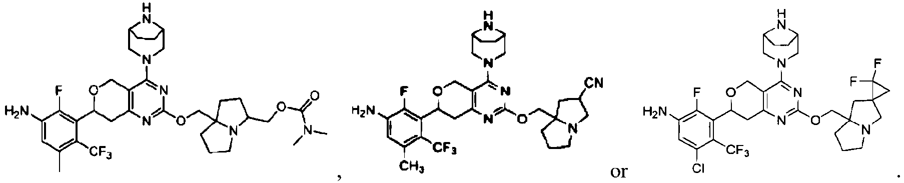

In some embodiments, R_{N} is H.

In some embodiments, R_{N} is C₁₋₃ alkyl optionally substituted with 1, 2, or 3 F or Cl.

The present disclosure provides a compound represented by formula (I'), a stereoisomer thereof, or a pharmaceutically acceptable salt thereof: 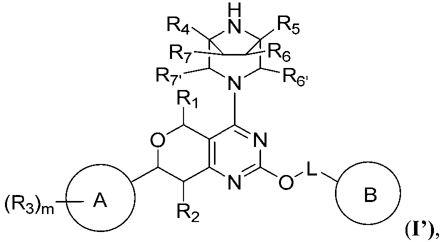 wherein,
Ring A is selected from C₆ aryl and 5- to 6-membered heteroaryl;
Ring B is selected from 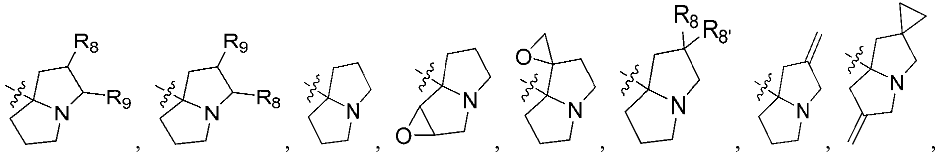 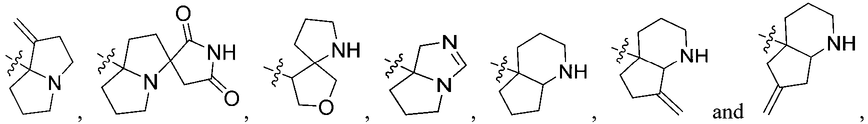
the ring B is optionally substituted with 1, 2, 3 or 4 R₁₀;
L is -CH₂-, wherein the -CH₂- is optionally substituted with 1 or 2 D;
R₁ and R₂ are each independently selected from oxo, H, F, Cl, Br, I and CN;
each R₃ is independently selected from F, Cl, Br, I, OH, NH₂, CN, C₁₋₃ alkyl, C₁₋₃ alkoxy, C₁₋₃ alkylamino, di-C₁₋₃ alkylamino, C₂₋₄ alkenyl, C₂₋₄ alkynyl and C₃₋₅ cycloalkyl, wherein the C₁₋₃ alkyl, C₁₋₃ alkoxy, C₁₋₃ alkylamino, di-C₁₋₃ alkylamino, C₂₋₄ alkenyl, C₂₋₄ alkynyl and C₃₋₅ cycloalkyl are each independently optionally substituted with 1, 2, 3, 4 or 5 Rₐ;
each Rₐ is independently selected from D, F, Cl, Br and I;
R₄, R₅*,* R₆, R₇, R_{6'} and R_{7'} are each independently selected from oxo, H, F, Cl, Br, I, OH, NH₂, CN, C₁₋₃ alkyl, C₂₋₄ alkenyl, C₁₋₃ alkoxy, -C(=O)-R_{d}, -C(=O)-NRₚ₁Rₚ₂ and =NO(C₁₋₃ alkyl), wherein the C₁₋₃ alkyl, C₂₋₄ alkenyl and C₁₋₃ alkoxy are each independently optionally substituted with 1, 2, 3, 4 or 5 R_{b};
alternatively, R₆ and R₇ together with the carbon atoms to which they are attached form a 3- to 5-membered heterocycloalkyl group;
each R_{b} is independently selected from D, F, Cl, Br, I, OH, NH₂, CN, C₁₋₃ alkoxy and -C(=O)-NR_{b1}R_{b2};
R₈ is selected from H, F, Cl, Br, I, OH, NH₂, CN, C₁₋₃ alkyl and C₁₋₃ alkoxy, wherein the C₁₋₃ alkyl and C₁₋₃ alkoxy are each independently optionally substituted with 1, 2, 3, 4 or 5 Rₐ;
alternatively, R₈ and R_{8'} together with the carbon atom to which they are attached form a C₃₋₅ cycloalkyl or 3- to 5-membered heterocycloalkyl, wherein the C₃₋₅ cycloalkyl or 3- to 5-membered heterocycloalkyl is each independently optionally substituted with 1, 2 or 3 R₁₀;
R₉ is selected from -C(=O)-NR_{b1}R_{b2} and -CH₂R_{c};
each R₁₀ is independently selected from oxo, D, F, Cl, Br, I, OH, NH₂, CN, C₁₋₃ alkyl, C₁₋₃ alkoxy, C₁₋₃ alkylamino, di-C₁₋₃ alkylamino, -S-R_{d}, -S(=O)-R_{d}, -S(=O)₂-R_{d} and -NH-C(=O)-R_{d}, wherein the C₁₋₃ alkyl is optionally substituted with 1, 2 or 3 OH;
R_{b1} and R_{b2} are each independently selected from H, C₁₋₆ alkyl, C₃₋₁₀ cycloalkyl, 3- to 10-membered heterocycloalkyl, C₆₋₁₀ aryl and 5- to 10-membered heteroaryl, wherein the C₁₋₆ alkyl, C₃₋₁₀ cycloalkyl, 3- to 10-membered heterocycloalkyl, C₆₋₁₀ aryl and 5- to 10-membered heteroaryl are each independently optionally substituted with 1, 2 or 3 Rₑ;
alternatively, R_{b1} and R_{b2} together with the nitrogen atom to which they are attached form a 3- to 6-membered heterocycloalkyl group;
R_{c} is selected from F, Cl, Br, I, OH, NH₂, -O(C=O)NR_{C1}R_{C2}, -NR_{C0}(C=O)R_{C1} and - NR_{C0}(C=O)NR_{C1}R_{C2};
R_{C0}, R_{C1} and R_{C2} are each independently selected from H, C₁₋₆ alkyl, C₃₋₆ cycloalkyl and 3- to 6-membered heterocycloalkyl;
R_{d} is C₁₋₃ alkyl;
Rₑ is selected from F, Cl, Br, I, OH, NH₂, C₁₋₃ alkylamino, di-C₁₋₃ alkylamino, CN, C₁₋₃ alkoxy, C₃₋₁₀ cycloalkyl, 3- to 10-membered heterocycloalkyl, C₆₋₁₀ aryl, and 5- to 10-membered heteroaryl;
m is selected from 0, 1, 2, 3, 4 and 5;
with the proviso that,
   1) when ring B is 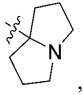 wherein the 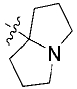 is substituted with one R₁₀, and R₁₀ is F, at least one of R₁, R₂, R₄, R₅*,* R₆, R₇, R_{6'} and R_{7'} is not H;
   2) when ring B is 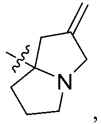 wherein the 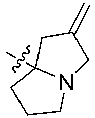 is optionally substituted with 1, 2, 3 or 4 R₁₀, at least one of R₁, R₂, R₄, R₅*,* R₆, R₇, R_{6'} and R_{7'} is not H; and
   3) the compound is not

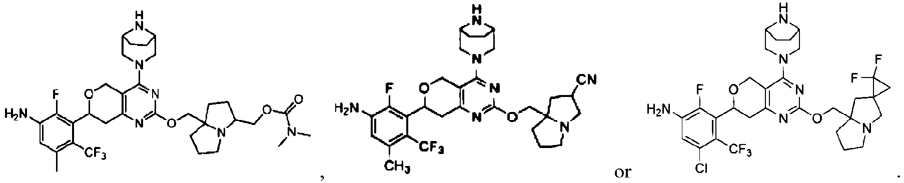

The present disclosure also provides a compound represented by formula **(I'-1),** a stereoisomer thereof, or a pharmaceutically acceptable salt thereof: 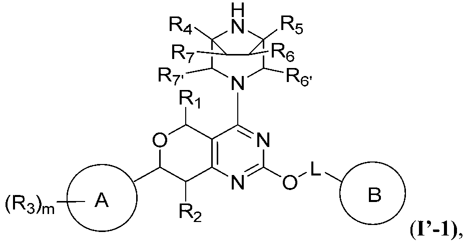 wherein,
Ring A is C₆ aryl;
Ring B is selected from 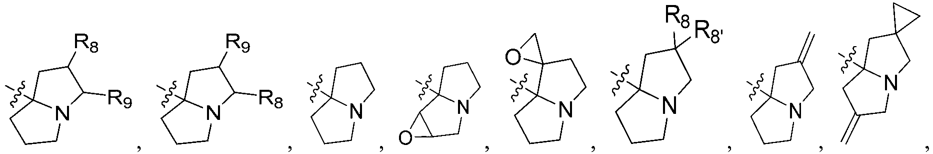 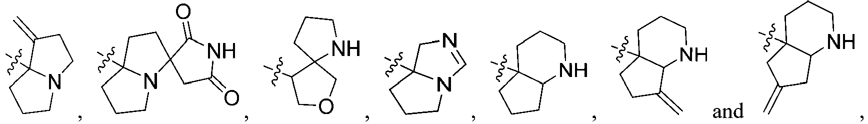
the ring B is optionally substituted with 1, 2, 3 or 4 R₁₀;
L is -CH₂-, wherein the -CH₂- is optionally substituted with 1 or 2 D;
R₁ and R₂ are each independently selected from oxo, H, F, Cl, Br, I and CN;
each R₃ is independently selected from F, Cl, Br, I, OH, NH₂, CN, C₁₋₃ alkyl, C₁₋₃ alkoxy, C₁₋₃ alkylamino, di-C₁₋₃ alkylamino, C₂₋₄ alkenyl, C₂₋₄ alkynyl and C₃₋₅ cycloalkyl, wherein the C₁₋₃ alkyl, C₁₋₃ alkoxy, C₁₋₃ alkylamino, di-C₁₋₃ alkylamino, C₂₋₄ alkenyl, C₂₋₄ alkynyl and C₃₋₅ cycloalkyl are each independently optionally substituted with 1, 2, 3, 4 or 5 Rₐ;
each Rₐ is independently selected from D, F, Cl, Br and I;
R₄, R₅*,* R₆, R₇, R_{6'} and R_{7'} are each independently selected from oxo, H, F, Cl, Br, I, OH, NH₂, CN, C₁₋₃ alkyl, C₂₋₄ alkenyl, C₁₋₃ alkoxy, -C(=O)-R_{d}, -C(=O)-NRₚ₁Rₚ₂ and =NO(C₁₋₃ alkyl), wherein the C₁₋₃ alkyl, C₂₋₄ alkenyl and C₁₋₃ alkoxy are each independently optionally substituted with 1, 2, 3, 4 or 5 R_{b};
alternatively, R₆ and R₇ together with the carbon atoms to which they are attached form a 3- to 5-membered heterocycloalkyl group;
each R_{b} is independently selected from D, F, Cl, Br, I, OH, NH₂, CN, C₁₋₃ alkoxy and -C(=O)-NR_{b1}R_{b2};
R₈ is selected from H, F, Cl, Br, I, OH, NH₂, CN, C₁₋₃ alkyl and C₁₋₃ alkoxy, wherein the C₁₋₃ alkyl and C₁₋₃ alkoxy are each independently optionally substituted with 1, 2, 3, 4 or 5 Rₐ;
alternatively, R₈ and R_{8'} together with the carbon atom to which they are attached form a C₃₋₅ cycloalkyl or 3- to 5-membered heterocycloalkyl, wherein the C₃₋₅ cycloalkyl or 3- to 5-membered heterocycloalkyl is each independently optionally substituted with 1, 2 or 3 R₁₀;
R₉ is selected from -C(=O)-NR_{b1}R_{b2} and -CH₂R_{c};
each R₁₀ is independently selected from oxo, D, F, Cl, Br, I, OH, NH₂, CN, C₁₋₃ alkyl, C₁₋₃ alkoxy, C₁₋₃ alkylamino, di-C₁₋₃ alkylamino, -S-R_{d}, -S(=O)-R_{d}, -S(=O)₂-R_{d} and -NH-C(=O)-R_{d}, wherein the C₁₋₃ alkyl is optionally substituted with 1, 2 or 3 OH;
R_{b1} and R_{b2} are each independently selected from H, C₁₋₆ alkyl, C₃₋₁₀ cycloalkyl, 3- to 10-membered heterocycloalkyl, C₆₋₁₀ aryl and 5- to 10-membered heteroaryl, wherein the C₁₋₆ alkyl, C₃₋₁₀ cycloalkyl, 3- to 10-membered heterocycloalkyl, C₆₋₁₀ aryl and 5- to 10-membered heteroaryl are each independently optionally substituted with 1, 2 or 3 Rₑ;
alternatively, R_{b1} and R_{b2} together with the nitrogen atom to which they are attached form a 3- to 6-membered heterocycloalkyl group;
R_{c} is selected from F, Cl, Br, I, OH, NH₂, -O(C=O)NR_{C1}R_{C2}, -NR_{C0}(C=O)R_{C1} and - NR_{C0}(C=O)NR_{C1}R_{C2};
R_{C0}, R_{C1} and R_{C2} are each independently selected from H, C₁₋₆ alkyl, C₃₋₆ cycloalkyl and 3- to 6-membered heterocycloalkyl;
R_{d} is C₁₋₃ alkyl;
Rₑ is selected from F, Cl, Br, I, OH, NH₂, C₁₋₃ alkylamino, di-C₁₋₃ alkylamino, CN, C₁₋₃ alkoxy, C₃₋₁₀ cycloalkyl, 3- to 10-membered heterocycloalkyl, C₆₋₁₀ aryl, and 5- to 10-membered heteroary;
m is selected from 0, 1, 2, 3, 4 and 5;
with the proviso that,
   1) when ring B is 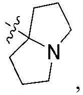 wherein the 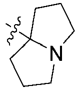 is substituted with one R₁₀, and R₁₀ is F, at least one of R₁, R₂, R₄, R₅*,* R₆, R₇, R_{6'} and R_{7'} is not H;
   2) when ring B is 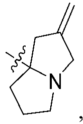 wherein the 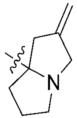 is optionally substituted with 1, 2, 3 or 4 R₁₀, at least one of R₁, R₂, R₄, R₅*,* R₆, R₇, R_{6'} and R_{7'} is not H; and
   3) the compound is not

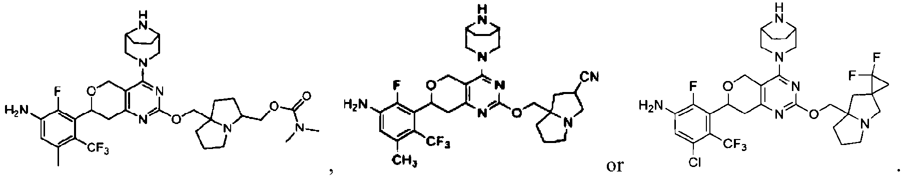

The present disclosure also provides a compound represented by formula (**I'-2**), a stereoisomer thereof, or a pharmaceutically acceptable salt thereof: 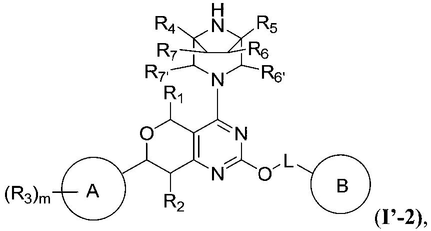 wherein,
Ring A is 5-membered heteroaryl;
Ring B is selected from 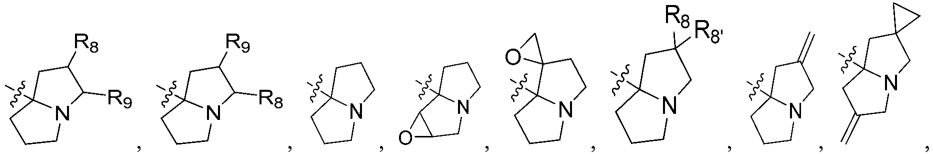 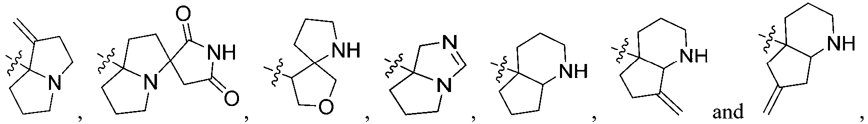
the ring B is optionally substituted with 1, 2, 3 or 4 R₁₀;
L is -CH₂-, wherein the -CH₂- is optionally substituted with 1 or 2 D;
R₁ and R₂ are each independently selected from oxo, H, F, Cl, Br, I and CN;
each R₃ is independently selected from F, Cl, Br, I, OH, NH₂, CN, C₁₋₃ alkyl, C₁₋₃ alkoxy, C₁₋₃ alkylamino, di-C₁₋₃ alkylamino, C₂₋₄ alkenyl, C₂₋₄ alkynyl and C₃₋₅ cycloalkyl, wherein the C₁₋₃ alkyl, C₁₋₃ alkoxy, C₁₋₃ alkylamino, di-C₁₋₃ alkylamino, C₂₋₄ alkenyl, C₂₋₄ alkynyl and C₃₋₅ cycloalkyl are each independently optionally substituted with 1, 2, 3, 4 or 5 Rₐ;
each Rₐ is independently selected from D, F, Cl, Br and I;
R₄, R₅*,* R₆, R₇, R_{6'} and R_{7'} are each independently selected from oxo, H, F, Cl, Br, I, OH, NH₂, CN, C₁₋₃ alkyl, C₂₋₄ alkenyl, C₁₋₃ alkoxy, -C(=O)-R_{d}, -C(=O)-NR_{b1}R_{b2} and =NO(C₁₋₃ alkyl), wherein the C₁₋₃ alkyl, C₂₋₄ alkenyl and C₁₋₃ alkoxy are each independently optionally substituted with 1, 2, 3, 4 or 5 R_{b};
alternatively, R₆ and R₇ together with the carbon atoms to which they are attached form a 3- to 5-membered heterocycloalkyl group;
each R_{b} is independently selected from D, F, Cl, Br, I, OH, NH₂, CN, C₁₋₃ alkoxy and -C(=O)-NR_{b1}R_{b2};
R₈ is selected from H, F, Cl, Br, I, OH, NH₂, CN, C₁₋₃ alkyl and C₁₋₃ alkoxy, wherein the C₁₋₃ alkyl and C₁₋₃ alkoxy are each independently optionally substituted with 1, 2, 3, 4 or 5 Rₐ;
alternatively, R₈ and R_{8'} together with the carbon atom to which they are attached form a C₃₋₅ cycloalkyl or 3- to 5-membered heterocycloalkyl, wherein the C₃₋₅ cycloalkyl or 3- to 5-membered heterocycloalkyl is each independently optionally substituted with 1, 2 or 3 R₁₀;
R₉ is selected from -C(=O)-NR_{b1}R_{b2} and -CH₂R_{c};
each R₁₀ is independently selected from oxo, D, F, Cl, Br, I, OH, NH₂, CN, C₁₋₃ alkyl, C₁₋₃ alkoxy, C₁₋₃ alkylamino, di-C₁₋₃ alkylamino, -S-R_{d}, -S(=O)-R_{d}, -S(=O)₂-R_{d} and -NH-C(=O)-R_{d}, wherein the C₁₋₃ alkyl is optionally substituted with 1, 2 or 3 OH;
R_{b1} and R_{b2} are each independently selected from H, C₁₋₆ alkyl, C₃₋₁₀ cycloalkyl, 3- to 10-membered heterocycloalkyl, C₆₋₁₀ aryl and 5- to 10-membered heteroaryl, wherein the C₁₋₆ alkyl, C₃₋₁₀ cycloalkyl, 3- to 10-membered heterocycloalkyl, C₆₋₁₀ aryl and 5- to 10-membered heteroaryl are each independently optionally substituted with 1, 2 or 3 Rₑ;
alternatively, R_{b1} and R_{b2} together with the nitrogen atom to which they are attached form a 3- to 6-membered heterocycloalkyl group;
R_{c} is selected from F, Cl, Br, I, OH, NH₂, -O(C=O)NR_{C1}R_{C2}, -NR_{C0}(C=O)R_{C1} and - NR_{C0}(C=O)NR_{C1}R_{C2};
R_{C0}, R_{C1} and R_{C2} are each independently selected from H, C₁₋₆ alkyl, C₃₋₆ cycloalkyl and 3- to 6-membered heterocycloalkyl;
R_{d} is C₁₋₃ alkyl;
Rₑ is selected from F, Cl, Br, I, OH, NH₂, C₁₋₃ alkylamino, di-C₁₋₃ alkylamino, CN, C₁₋₃ alkoxy, C₃₋₁₀ cycloalkyl, 3- to 10-membered heterocycloalkyl, C₆₋₁₀ aryl, and 5- to 10-membered heteroary;
m is selected from 0, 1, 2, 3, 4 and 5;
with the proviso that,
   1) when ring B is 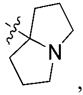 wherein the 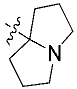 is substituted with one R₁₀, and R₁₀ is F, at least one of R₁, R₂, R₄, R₅*,* R₆, R₇, R_{6'} and R_{7'} is not H;
   2) when ring B is 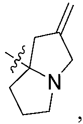 wherein the 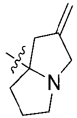 is optionally substituted with 1, 2, 3 or 4 R₁₀, at least one of R₁, R₂, R₄, R₅*,* R₆, R₇, R_{6'} and R_{7'} is not H; and
   3) the compound is not

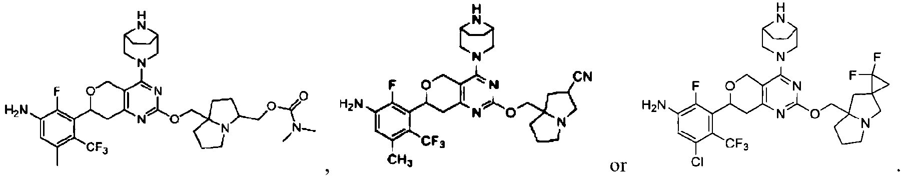

In some embodiments of the present disclosure, ring B is selected from 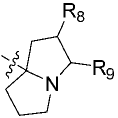 and 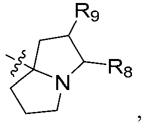 and said ring B is optionally substituted with 1, 2, 3 or 4 R₁₀.

In some embodiments of the present disclosure, ring B is 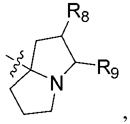 wherein ring B is optionally substituted with 1, 2, 3, or 4 R₁₀. In some embodiments, ring B is unsubstituted. In some embodiments, ring B is substituted with 1 or 2 R₁₀. In some embodiments, ring B is substituted with 1 R₁₀. In some embodiments, ring B is substituted with 2 R₁₀.

In some embodiments of the present disclosure, the compound represented by formula **(I'-1),** a stereoisomer thereof, or a pharmaceutically acceptable salt thereof is selected from compounds of formula (**I'-1-i)** and **(I'-2-i),** stereoisomers thereof or pharmaceutically acceptable salts thereof,

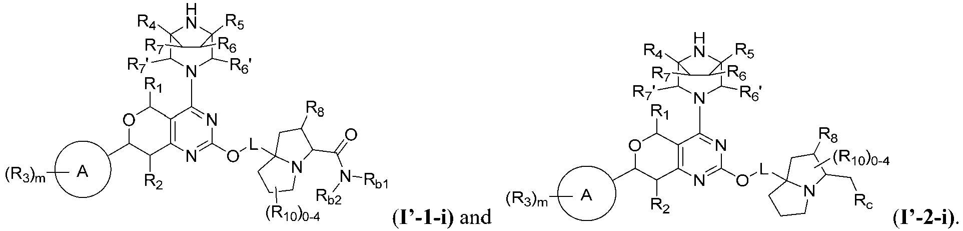

In some embodiments of the present disclosure, the compound represented by formula (**I'-1**) of the present disclosure, a stereoisomer thereof or a pharmaceutically acceptable salt thereof is the compounds represented by formula **(I'-1-i),** stereoisomers thereof or pharmaceutically acceptable salts thereof.

In some embodiments of the present disclosure, the compound represented by formula (**I'-1**) of the present disclosure, a stereoisomer thereof or a pharmaceutically acceptable salt thereof is the compounds represented by formula (**I'-2-i**), stereoisomers thereof or pharmaceutically acceptable salts thereof.

In some embodiments of the present disclosure, ring B is 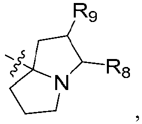 wherein ring B is optionally substituted with 1, 2, 3, or 4 R₁₀. In some embodiments, ring B is unsubstituted. In some embodiments, ring B is substituted with 1 or 2 R₁₀. In some embodiments, ring B is substituted with 1 R₁₀. In some embodiments, ring B is substituted with 2 R₁₀.

In some embodiments of the present disclosure, the compound represented by formula **(I'-1),** a stereoisomer thereof or a pharmaceutically acceptable salt thereof is selected from the compounds of formulas **(I'-1-ii)** and **(I'-2-ii),** stereoisomers thereof or pharmaceutically acceptable salts thereof,

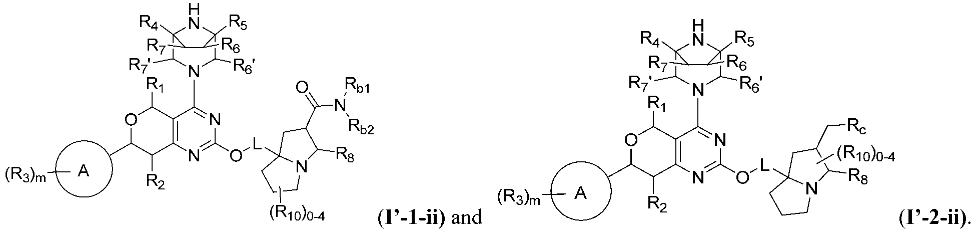

In some embodiments of the present disclosure, the compound represented by formula **(I'-1)** of the present disclosure, a stereoisomer thereof or a pharmaceutically acceptable salt thereof is the compounds represented by formula **(I'-1-ii),** stereoisomers thereof or pharmaceutically acceptable salts thereof.

In some embodiments of the present disclosure, the compound represented by formula **(I'-1)** of the present disclosure, a stereoisomer thereof or a pharmaceutically acceptable salt thereof is the compounds represented by formula **(I'-2-ii),** stereoisomers thereof or pharmaceutically acceptable salts thereof.

In some embodiments of the present disclosure, ring B is selected from 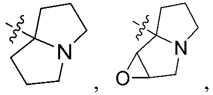 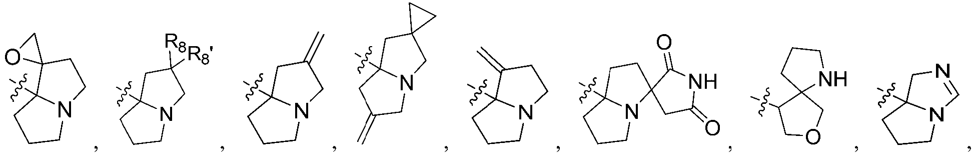 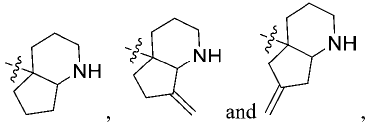 and said ring B is optionally substituted with 1, 2, 3 or 4 R₁₀. In some embodiments, ring B is unsubstituted. In some embodiments, ring B is substituted with 1 or 2 R₁₀. In some embodiments, ring B is substituted with 1 R₁₀. In some embodiments, ring B is substituted with 2 R₁₀.

In some embodiments of the present disclosure, the compound of formula (**I'-1**) of the present disclosure, a stereoisomer thereof, or a pharmaceutically acceptable salt thereof is selected from compounds of formulas (**I'-3**), **(I'-4),** (**I'-5**), **(I'-6),** (**I'-7**), (**I'-8**), (**I'-9**), **(I'-10), (I**'**-11**), (**I'-12**) and (**I'-13**), stereoisomers thereof or pharmaceutically acceptable salts thereof, 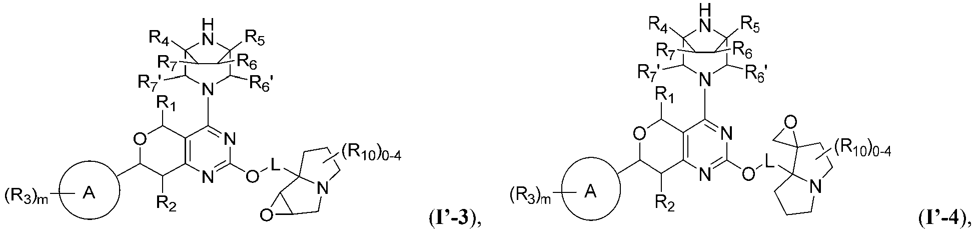 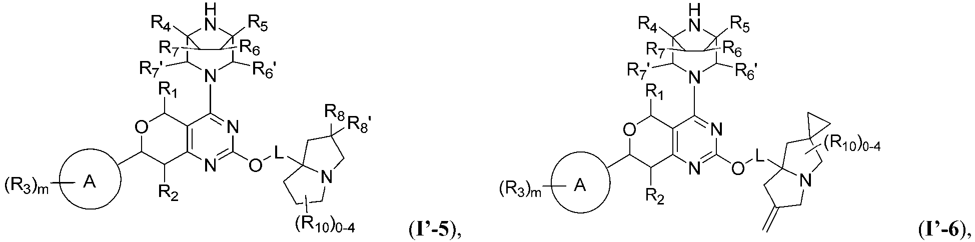 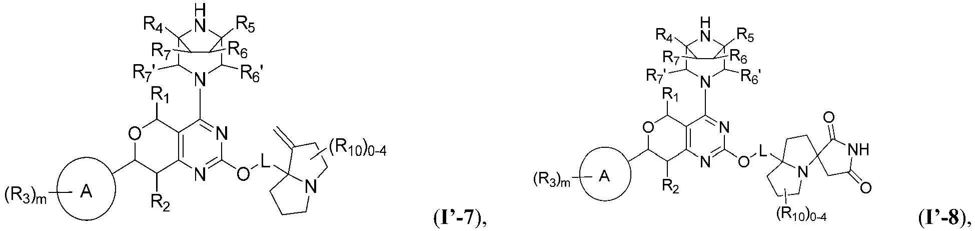 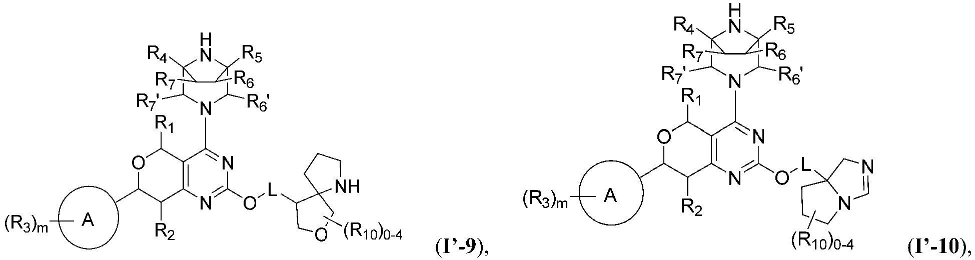 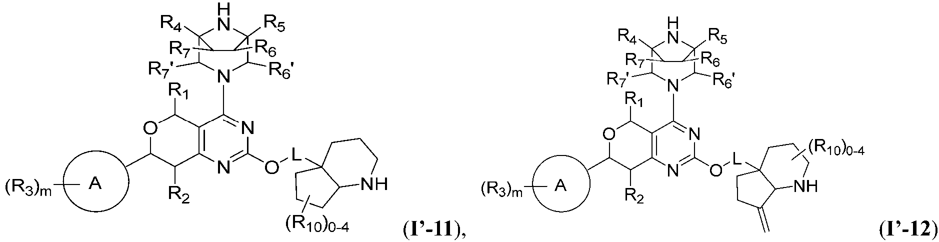 and

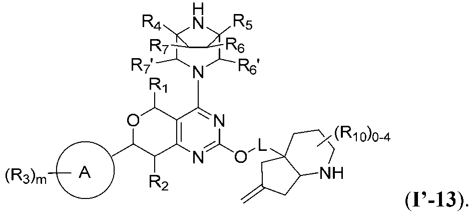

In some embodiments of the present disclosure, the compound represented by formula (**I'-1**) of the present disclosure, a stereoisomer thereof or a pharmaceutically acceptable salt thereof is selected from the compounds represented by formulas **(I'-14)** and **(I'-15),** stereoisomers thereof or pharmaceutically acceptable salts thereof,

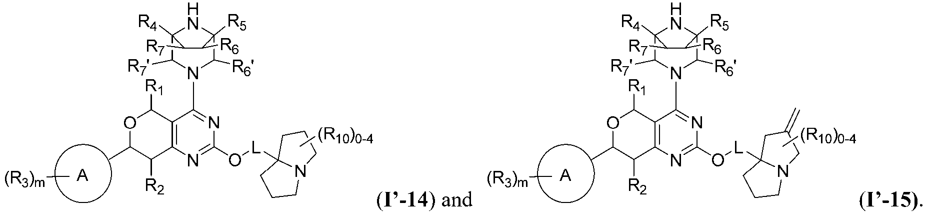

In some embodiments of the present disclosure, ring A is phenyl, wherein the phenyl is substituted with at least one R₃, and each R₃ is independently selected from F, Cl, Br, I, OH, NH₂, CN, C₁₋₃ alkyl, C₁₋₃ alkoxy, C₁₋₃ alkylamino, C₂₋₄ alkenyl, C₂₋₄ alkynyl and C₃₋₅ cycloalkyl, wherein the C₁₋₃ alkyl, C₁₋₃ alkoxy, C₁₋₃ alkylamino, C₂₋₄ alkenyl, C₂₋₄ alkynyl and C₃₋₅ cycloalkyl are each independently optionally substituted with 1, 2, 3, 4 or 5 Rₐ.

In some embodiments of the present disclosure, ring A is phenyl, wherein the phenyl is substituted with at least one R₃, and each R₃ is independently selected from F, OH, NH₂, CF₃, OCH₃, -C≡CCH₃, -C≡CCH₂F , -C≡CCHF₂, -C≡CCF₃, -C≡CCD₃, -C≡CH, -C≡CCl, -C≡CF and cyclopropyl. In some embodiments of the present disclosure, ring A is phenyl, wherein the phenyl is substituted with two R₃, and each R₃ is independently selected from F, OH, NH₂, CF₃, OCH₃, -C≡CCH₃, -C≡CCH₂F , -C≡CCHF₂, -C≡CCF₃, -C≡CCD₃, -C≡CH, -C≡CCl, -C≡CF and cyclopropyl. In some embodiments of the present disclosure, ring A is phenyl, wherein the phenyl is substituted with three R₃, and each R₃ is independently selected from F, OH, NH₂, CF₃, OCH₃, -C≡CCH₃, -C≡CCH₂F , -C≡CCHF₂, -C≡CCF₃, -C≡CCD₃, -C≡CH, -C≡CCl, -C≡CF and cyclopropyl. In some embodiments of the present disclosure, ring A is phenyl, wherein the phenyl is substituted with four R₃, and each R₃ is independently selected from F, OH, NH₂, CF₃, OCH₃, -C≡CCH₃, -C≡CCH₂F , -C≡CCHF₂, -C≡CCF₃, -C≡CCD₃, -C≡CH, -C≡CCl, -C≡CF and cyclopropyl.

In some embodiments of the present disclosure, 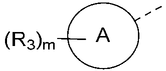 is selected from

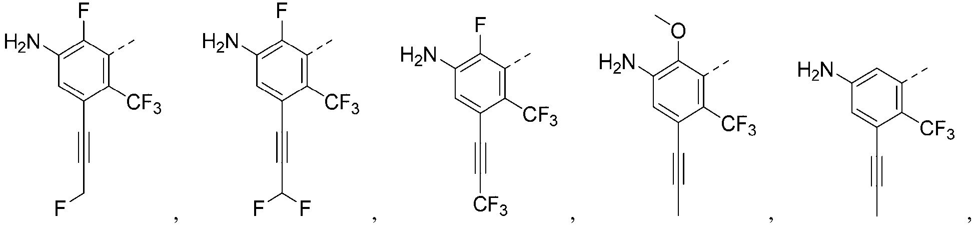

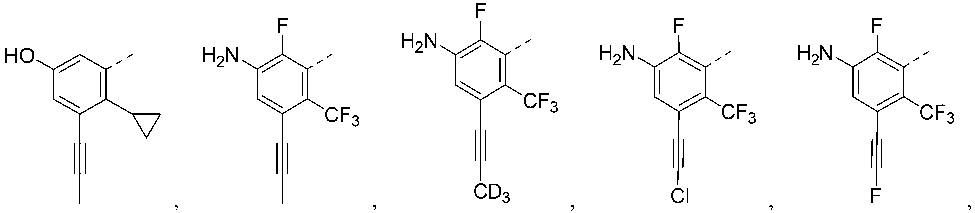

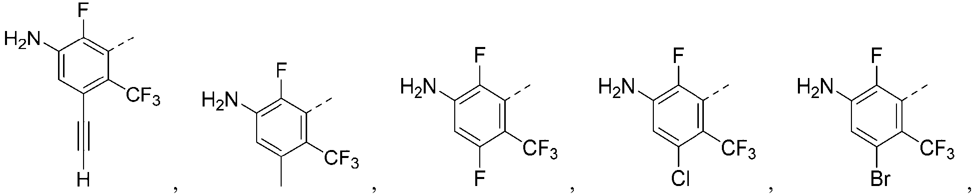

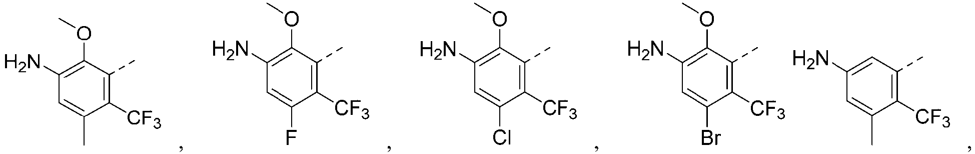

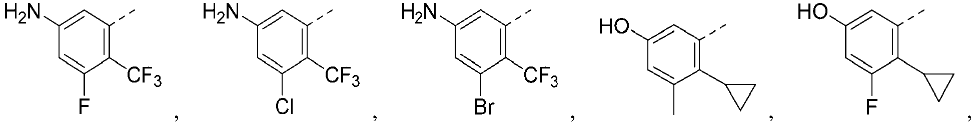

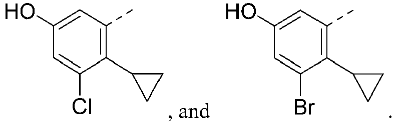

In some embodiments of the present disclosure, 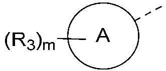 is selected from

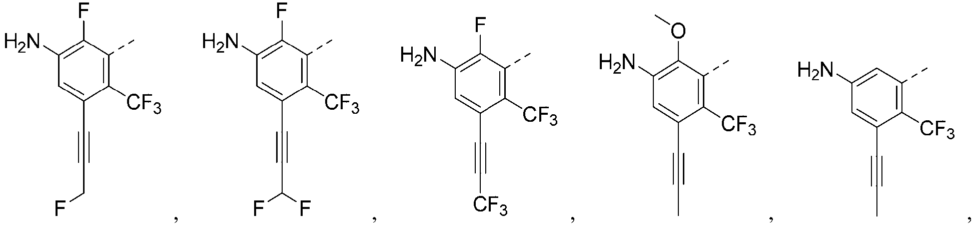

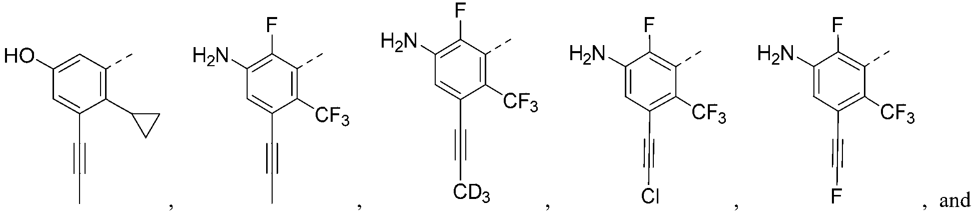

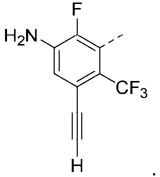

In some embodiments of the present disclosure, 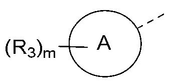 is

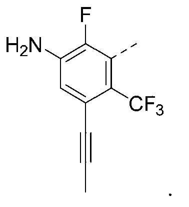

In some embodiments of the present disclosure, 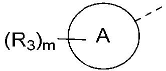 is selected from 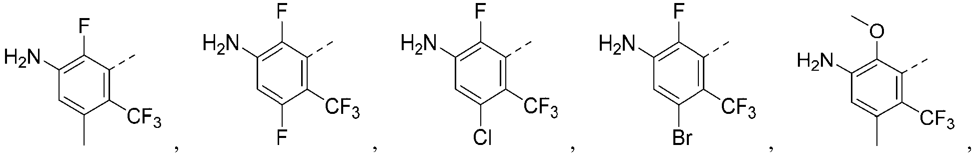 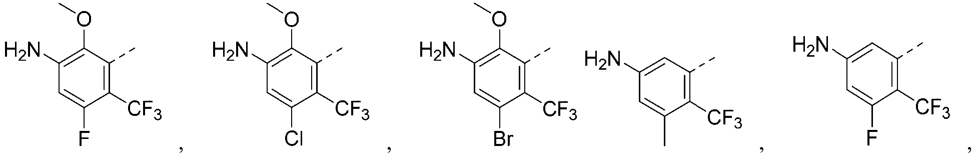 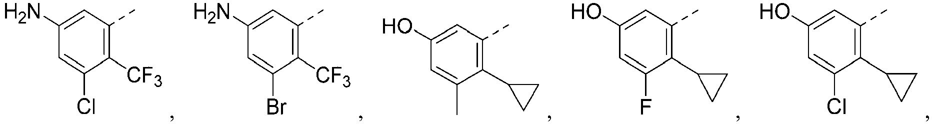 and

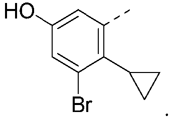

In some embodiments of the present disclosure, ring A is a 5- to 6-membered heteroaryl group (e.g., pyridyl), wherein the 5- to 6-membered heteroaryl group is substituted with at least one R₃, and each R₃ is independently selected from F, Cl, Br, I, OH, NH₂, CN, C₁₋₃ alkyl, C₁₋₃ alkoxy, C₁₋₃ alkylamino, C₂₋₄ alkenyl, C₂₋₄ alkynyl and C₃₋₅ cycloalkyl, wherein the C₁₋₃ alkyl, C₁₋₃ alkoxy, C₁₋₃ alkylamino, C₂₋₄ alkenyl, C₂₋₄ alkynyl and C₃₋₅ cycloalkyl are each independently optionally substituted with 1, 2, 3, 4 or 5 Rₐ.

In some embodiments of the present disclosure, ring A is a 5- to 6-membered heteroaryl group (e.g., pyridyl), wherein the 5- to 6-membered heteroaryl group is substituted with at least one R₃, and each R₃ is independently selected from F, OH, NH₂, CF₃, OCH₃, -C≡CCH₃, -C≡CCH₂F, -C≡CCHF₂, -C≡CCF₃, -C≡CCD₃-C≡CH, -C≡CCl, -C≡CF and cyclopropyl. In some embodiments of the present disclosure, ring A is a 5- to 6-membered heteroaryl group (e.g., pyridyl), wherein the 5- to 6-membered heteroaryl group is substituted with two R₃, and each R₃ is independently selected from F, OH, NH₂, CF₃, OCH₃, -C≡CCH₃ -C≡CCH₂F -C≡CCHF₂, -C≡CCF₃, -C≡CCD₃, -C≡CH, -C≡CCl, -C≡CF and cyclopropyl. In some embodiments of the present disclosure, ring A is a 5- to 6-membered heteroaryl group (e.g., pyridyl), wherein the 5- to 6-membered heteroaryl group is substituted with three R₃, and each R₃ is independently selected from F, OH, NH₂, CF₃, OCH₃, -C≡CCH₃, -C≡CCH₂F , -C≡CCHF₂ , -C≡CCF₃, -C≡CCD₃, -C≡CH, -C≡CCl, -C≡CF and cyclopropyl. In some embodiments of the present disclosure, ring A is a 5- to 6-membered heteroaryl group (e.g., pyridyl), wherein the 5- to 6-membered heteroaryl group is substituted with four R₃, and each R₃ is independently selected from F, OH, NH₂, CF₃, OCH₃, -C≡CCH₃, -C≡CCH₂F, -C≡CCHF₂, -C≡CCF₃, -C≡CCD₃, -C≡CH, -C≡CCl, -C≡CF and cyclopropyl.

In some embodiments of the present disclosure, ring A is a 5-membered heteroaryl group, wherein the 5-membered heteroaryl group is substituted with at least one R₃, and each R₃ is independently selected from F, Cl, Br, I, OH, NH₂, CN, C₁₋₃ alkyl, C₁₋₃ alkoxy, C₁₋₃ alkylamino, C₂₋₄ alkenyl, C₂₋₄ alkynyl and C₃₋₅ cycloalkyl, wherein the C₁₋₃ alkyl, C₁₋₃ alkoxy, C₁₋₃ alkylamino, C₂₋₄ alkenyl, C₂₋₄ alkynyl and C₃₋₅ cycloalkyl groups are each independently optionally substituted with 1, 2, 3, 4 or 5 Rₐ.

In some embodiments of the present disclosure, ring A is selected from a 5-membered heteroaryl group, wherein the 5-membered heteroaryl group is substituted with at least one R₃, and each R₃ is independently selected from F, OH, NH₂, CF₃, OCH₃, -C≡CCH₃, -C≡CCH₂F , -C≡CCHF₂ , -C≡CCF₃, , -C≡CCD₃, -C≡CH , -C≡CCl, -C≡CF, and cyclopropyl. In some embodiments of the present disclosure, ring A is a 5-membered heteroaryl group, wherein the 5-membered heteroaryl group is substituted with two R₃, and each R₃ is independently selected from F, OH, NH₂, CF₃, OCH₃, -C≡CCH₃, -C≡CCH₂F, -C≡CCHF₂, -C≡CCF₃, -C≡CCD₃, -C≡CH , -C≡CCl, -C≡CF , and cyclopropyl. In some embodiments of the present disclosure, ring A is a 5-membered heteroaryl group, wherein the 5-membered heteroaryl group is substituted with three R₃, and each R₃ is independently selected from F, OH, NH₂, CF₃, OCH₃, -C≡CCH₃, -C≡CCH₂F , -C≡CCHF₂ , -C≡CCF₃, -C≡CCD₃, -C≡CH , -C≡CCl, -C≡CF , and cyclopropyl. In some embodiments of the present disclosure, ring A is 5-membered heteroaryl, wherein the 5-membered heteroaryl is substituted with 4 R₃, and each R₃ is independently selected from F, OH, NH₂, CF₃, OCH₃, -C≡CCH₃, -C≡CCH₂F , -C≡CCHF₂ , -C≡CCF₃, -C≡CCD₃, -C≡CH -C≡CCl, -C≡CF and cyclopropyl.

In some embodiments of the present disclosure, ring A is a 6-membered heteroaryl group, wherein the 6-membered heteroaryl group is substituted with at least one R₃, and each R₃ is independently selected from F, Cl, Br, I, OH, NH₂, CN, C₁₋₃ alkyl, C₁₋₃ alkoxy, C₁₋₃ alkylamino, C₂₋₄ alkenyl, C₂₋₄ alkynyl and C₃₋₅ cycloalkyl, wherein the C₁₋₃ alkyl, C₁₋₃ alkoxy, C₁₋₃ alkylamino, C₂₋₄ alkenyl, C₂₋₄ alkynyl and C₃₋₅ cycloalkyl are each independently optionally substituted with 1, 2, 3, 4 or 5 Rₐ.

In some embodiments of the present disclosure, ring A is a 6-membered heteroaryl group, wherein the 6-membered heteroaryl group is substituted with at least one R₃, and each R₃ is independently selected from F, OH, NH₂, CF₃, OCH₃, -C≡CCH₃, -C≡CCH₂F , -C≡CCHF₂ , -C≡CCF₃, , -C≡CCD₃, -C≡CH -C≡CCl, -C≡CF , and cyclopropyl. In some embodiments of the present disclosure, ring A is a 6-membered heteroaryl group, wherein the 6-membered heteroaryl group is substituted with two R₃, and each R₃ is independently selected from F, OH, NH₂, CF₃, OCH₃, -C≡CCH₃, -C≡CCH₂F, -C≡CCHF₂ , -C≡CCF₃, -C≡CCD₃-C≡CH , -C≡CCl, -C≡CF , and cyclopropyl. In some embodiments of the present disclosure, ring A is a 6-membered heteroaryl group, wherein the 6-membered heteroaryl group is substituted with three R₃, and each R₃ is independently selected from F, OH, NH₂, CF₃, OCH₃, -C≡CCH₃, -C≡CCH₂F , -C≡CCHF₂, -C≡CCF₃, -C≡CCD₃, -C≡CH , -C≡CCl, -C≡CF , and cyclopropyl. In some embodiments of the present disclosure, ring A is a 6-membered heteroaryl group, wherein the 6-membered heteroaryl group is substituted with 4 R₃, and each R₃ is independently selected from F, OH, NH₂, CF₃, OCH₃, -C≡CCH₃, -C≡CCH₂F , -C≡CCHF₂ , -C≡CCF₃, , -C≡CCD₃, -C≡CH , -C≡CCl, -C≡C^{F}, and cyclopropyl.

In some embodiments of the present disclosure, the compound represented by formula (**I'-1**) of the present disclosure, a stereoisomer thereof or a pharmaceutically acceptable salt thereof is selected from the compounds of formulas (**I'-1'-i**) and (**I'-2'-i**), stereoisomers thereof or pharmaceutically acceptable salts thereof,

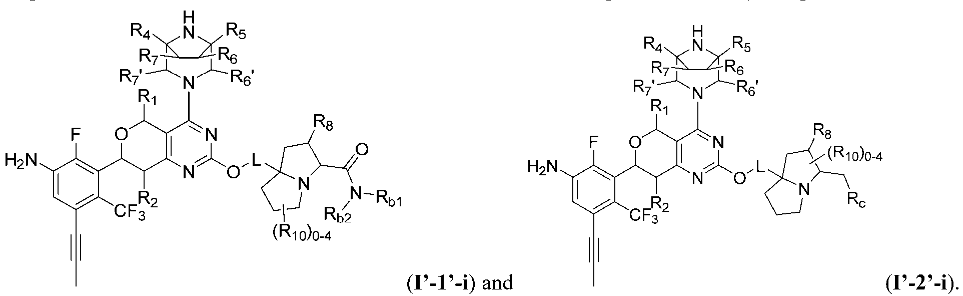

In some embodiments of the present disclosure, the compound represented by formula **(I'-1)** of the present disclosure, a stereoisomer thereof or a pharmaceutically acceptable salt thereof is the compounds represented by formula **(I'-1'-i),** stereoisomers thereof or pharmaceutically acceptable salts thereof.

In some embodiments of the present disclosure, the compound represented by formula **(I'-1)** of the present disclosure, a stereoisomer thereof or a pharmaceutically acceptable salt thereof is the compounds represented by formula (**I'-2'-i**), stereoisomers thereof or pharmaceutically acceptable salts thereof.

In some embodiments of the present disclosure, the compound represented by formula (**I'-1**) of the present disclosure, a stereoisomer thereof or a pharmaceutically acceptable salt thereof is selected from the compounds of formulas **(I'-1'-ii)** and (**I'-2'-ii**), stereoisomers thereof or pharmaceutically acceptable salts thereof,

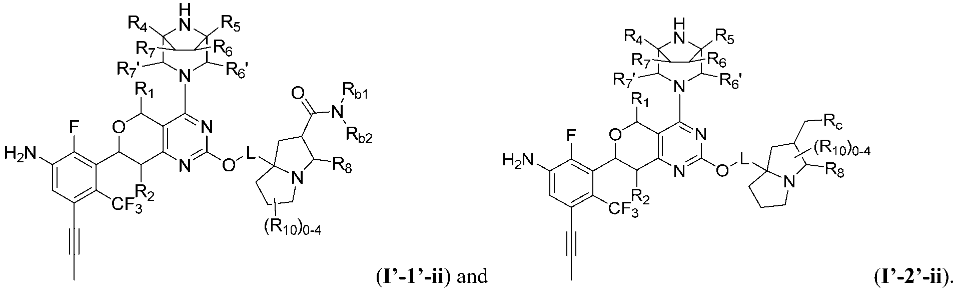

In some embodiments of the present disclosure, the compound represented by formula **(I'-1)** of the present disclosure, a stereoisomer thereof or a pharmaceutically acceptable salt thereof is the compounds represented by formula **(I'-1'-ii),** stereoisomers thereof or pharmaceutically acceptable salts thereof.

In some embodiments of the present disclosure, the compound represented by formula **(I'-1)** of the present disclosure, a stereoisomer thereof or a pharmaceutically acceptable salt thereof is the compounds represented by formula **(I'-2'-ii),** stereoisomers thereof or pharmaceutically acceptable salts thereof.

In some embodiments of the present disclosure, the compound of formula (**I'-1**) of the present disclosure, a stereoisomer thereof, or a pharmaceutically acceptable salt thereof is selected from compounds of formulas **(I'-3'),** (**I'-4'**), **(I'-5'), (I'-6'),** (**I'-7'**), (**I'-8'**), (**I'-9'**), **(I'-10'), (I'-11'), (I'-12')** and **(I'-13'),** stereoisomers thereof or pharmaceutically acceptable salts thereof, 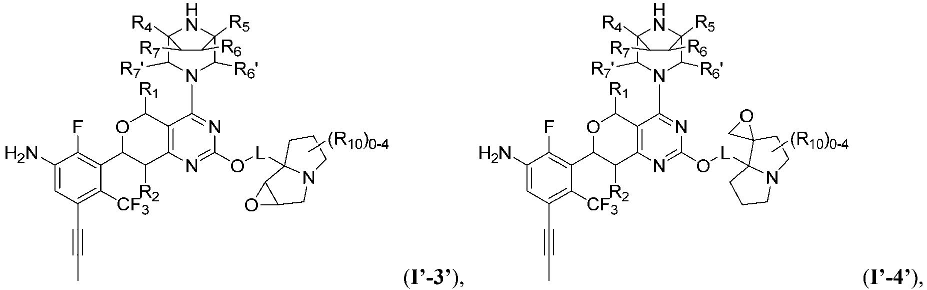 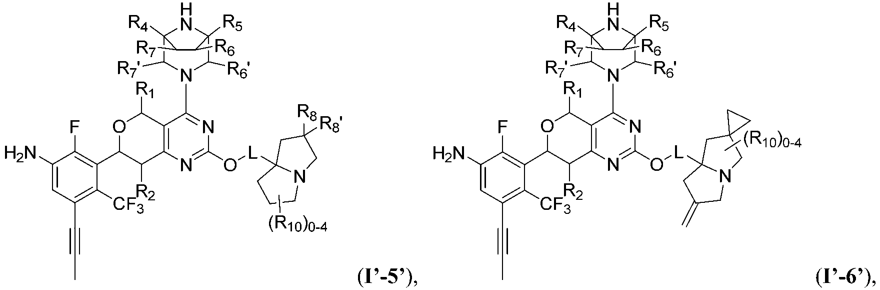 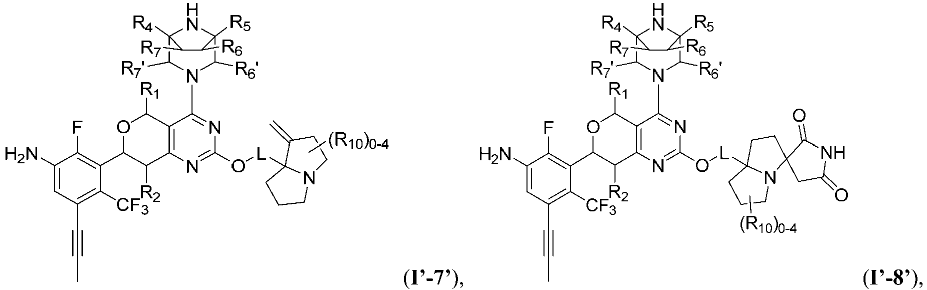 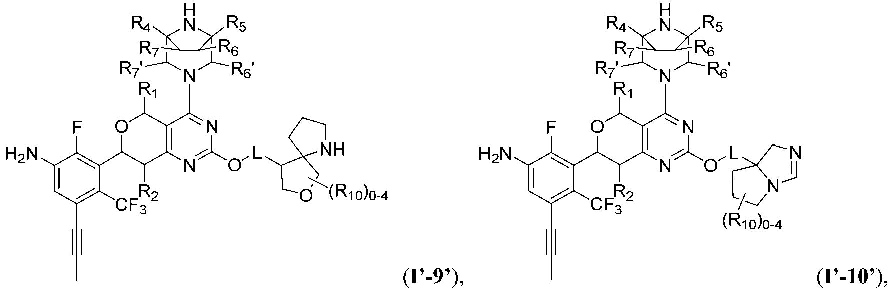 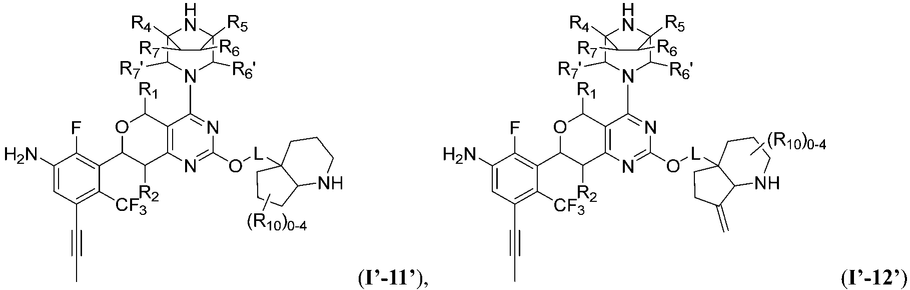 and

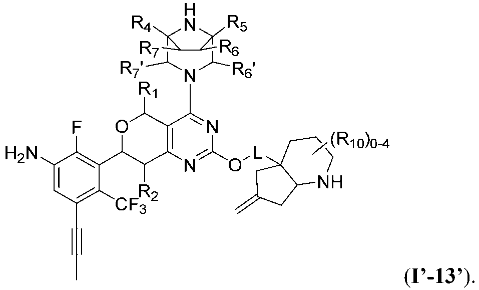

In some embodiments of the present disclosure, the compound represented by formula (**I'-1**) of the present disclosure, a stereoisomer thereof, or a pharmaceutically acceptable salt thereof is selected from compounds of formulas (**I'-14'**) and (**I'-15'**), stereoisomers thereof or pharmaceutically acceptable salts thereof,

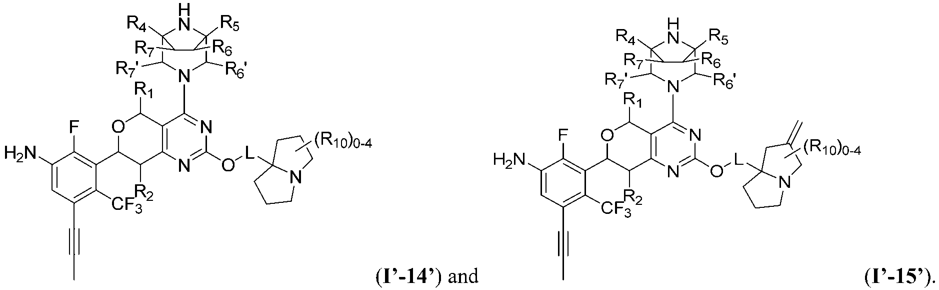

In some embodiments of the present disclosure, ring A is selected from C₆ aryl and 5- to 6-membered heteroaryl (e.g., a heteroaryl including a ring containing 1-4 heteroatoms selected from O, N, and S), as described herein. In some embodiments of the present disclosure, ring A is phenyl, as described herein. In some embodiments of the present disclosure, ring A is 5- to 6-membered heteroaryl, as described herein. In some embodiments of the present disclosure, ring A is 5 membered heteroaryl, as described herein. In some embodiments of the present disclosure, ring A is 6 membered heteroaryl, as described herein. In some embodiments of the present disclosure, ring A is unsubstituted. In some embodiments of the present disclosure, ring A is substituted, as described herein. In some embodiments of the present disclosure, ring A is substituted with at least one R₃ (e.g., 2, 3, or 4 R₃), as described herein.

In some embodiments of the present disclosure, L is -C(R_{L1}R_{L2})-, wherein the R_{L1} and R_{L2} are each independently selected from H and D.

In some embodiments of the present disclosure, L is -C(R_{L1}R_{L2})-, wherein the R_{L1} and R_{L2} are each independently H.

In some embodiments of the present disclosure, L is -C(R_{L1}R_{L2})-, wherein at least one of R_{L1} and R_{L2} is C₁₋₃ alkyl.

In some embodiments of the present disclosure, L is -CH₂-, wherein the -CH₂- is optionally substituted with 1 or 2 D.

In some embodiments of the present disclosure, L is -CH₂-. In some embodiments of the present disclosure, L is -CD₂-. In some embodiments of the present disclosure, L is -CHD-.

In some embodiments of the present disclosure, L is selected from -CH₂- and -CD₂-.

In some embodiments of the present disclosure, R₁ and R₂ are each independently selected from oxo, H, F, Cl, Br, I and CN.

In some embodiments of the present disclosure, R₁ and R₂ are H.

In some embodiments of the present disclosure, R₁ is selected from oxo, H, F, Cl, Br, I, and CN. In some embodiments of the present disclosure, R₂ is selected from oxo, F, Cl, and CN.

In some embodiments of the present disclosure, R₂ is selected from oxo, H, F, Cl, Br, I, and CN. In some embodiments of the present disclosure, R₁ is selected from oxo, F, Cl, and CN.

In some embodiments of the present disclosure, each R₃ is independently selected from F, Cl, Br, I, OH, NH₂, CN, C₁₋₃ alkyl (e.g., methyl (C₁), ethyl (C₂), n-propyl (C₃) or isopropyl (C₃)), C₁₋₃ alkoxy (e.g., methoxy (C₁), ethoxy (C₂), propoxy (C₃) or isopropyloxy (C₃)), C₁₋₃ alkylamino (e.g., methylamino, ethylamino, n-propylamino or isopropylamino), di-C₁₋₃ alkylamino (e.g., dimethylamino, diethylamino, di-n-propylamino, diisopropylamino, methylethylamino, methyl-n-propylamino, methylisopropylamino, ethyl-n-propylamino, ethylisopropylamino or n-propylisopropylamino), C₂₋₄ alkenyl (e.g., ethenyl (C₂), 1-propenyl (C₃), 2-propenyl (C₃), 1-butenyl (C₄), 2-butenyl (C₄) or butadienyl (C₄)), C₂₋₄ alkynyl (e.g., ethynyl (C₂), 1-propynyl (C₃), 2-propynyl (C₃), 1-butynyl (C₄) or 2-butynyl (C₄)) and C₃₋₅ cycloalkyl (e.g., cyclopropyl (C₃), cyclobutyl (C₄), cyclopentyl (C₅)), wherein the C₁₋₃ alkyl, C₁₋₃ alkoxy, C₁₋₃ alkylamino, di-C₁₋₃ alkylamino, C₂₋₄ alkenyl, C₂₋₄ alkynyl and C₃₋₅ cycloalkyl are each independently optionally substituted with 1, 2, 3, 4 or 5 Rₐ.

In some embodiments of the present disclosure, each R₃ is independently selected from F, OH, NH₂, CF₃, OCH₃, -C≡CCH₃, -C≡CCH₂F, -C≡CCHF₂, -C≡CCF₃, -C≡CCD₃, -C≡CH, -C≡CCl, -C≡CF and cyclopropyl.

In some embodiments of the present disclosure, each Rₐ is independently selected from D, F, Cl, Br and I. In some embodiments of the present disclosure, each Rₐ is independently selected from D, F and I.

In some embodiments of the present disclosure, m is selected from 0, 1, 2, 3, 4, and 5. In some embodiments of the present disclosure, m is 0. In some embodiments of the present disclosure, m is 1. In some embodiments of the present disclosure, m is 2. In some embodiments of the present disclosure, m is 3. In some embodiments of the present disclosure, m is 4. In some embodiments of the present disclosure, m is 5.

In some embodiments of the present disclosure, m is 4.

In some embodiments of the present disclosure, R₄, R₅, R₆, R₇, R_{6'} and R_{7'} are each independently selected from oxo, H, F, Cl, Br, I, OH, NH₂, CN, C₁₋₃ alkyl (e.g., methyl (C₁), ethyl (C₂), n-propyl (C₃) or isopropyl (C₃)), C₂₋₄ alkenyl (e.g., vinyl (C₂), 1-propenyl (C₃), 2-propenyl (C₃), 1-butenyl (C₄), 2-butenyl (C₄) or butadienyl (C₄)), C₁₋₃ alkoxy (e.g., methoxy (C₁), ethoxy (C₂), propoxy (C₃) or isopropoxy (C₃)), -C(=O)-R_{d}, -C(=O)-NR_{b1}R_{b2} and =NO(C₁₋₃ alkyl), wherein the C₁₋₃ alkyl, C₂₋₄ alkenyl and C₁₋₃ alkoxy are each independently optionally substituted with 1, 2, 3, 4 or 5 R_{b}.

In some embodiments of the present disclosure, R₄, R₅, R₆, R₇, R_{6'} and R_{7'} are H.

In some embodiments of the present disclosure, at least one of R₄, R₅, R₆, R₇, R_{6'} and R_{7'} is not H.

In some embodiments of the present disclosure, R₆ and R₇ together with the carbon atoms to which they are attached form a 3- to 5-membered heterocycloalkyl group (e.g., a 3- to 5-membered heterocycloalkyl group containing 1-2 heteroatoms selected from N, O, and S). In some embodiments of the present disclosure, R₆ and R₇ together with the carbon atoms to which they are attached form a 3-membered heterocycloalkyl group containing 1 heteroatom selected from N, O, and S. In some embodiments of the present disclosure, R₆ and R₇ together with the carbon atoms to which they are attached form a 3-membered heterocycloalkyl group containing 1 heteroatom selected from N and O. In some embodiments of the present disclosure, R₆ and R₇, together with the carbon atoms to which they are attached, form a 4-membered heterocycloalkyl group containing 1-2 heteroatoms selected from N, O, and S. In some embodiments of the present disclosure, R₆ and R₇, together with the carbon atoms to which they are attached, form a 4-membered heterocycloalkyl group containing 1-2 heteroatoms selected from N and O. In some embodiments of the present disclosure, R₆ and R₇, together with the carbon atoms to which they are attached, form a 4-membered heterocycloalkyl group containing 1 heteroatom selected from N and O. In some embodiments of the present disclosure, R₆ and R₇, together with the carbon atoms to which they are attached, form a 5-membered heterocycloalkyl group containing 1-2 heteroatoms selected from N, O and S. In some embodiments of the present disclosure, R₆ and R₇, together with the carbon atoms to which they are attached, form a 5-membered heterocycloalkyl group containing 1-2 heteroatoms selected from N and O. In some embodiments of the present disclosure, R₆ and R₇, together with the carbon atoms to which they are attached, form a 5-membered heterocycloalkyl group containing 1 heteroatom selected from N and O.

In some embodiments of the present disclosure, each R_{b} is independently selected from D, F, Cl, Br, I, OH, NH₂, CN, C₁₋₃ alkoxy (e.g., methoxy (C₁), ethoxy (C₂), propoxy (C₃) or isopropoxy (C₃)) and -C(=O)-NR_{b1}R_{b2}. In some embodiments of the present disclosure, each R_{b} is independently selected from D, F, Cl, OH, NH₂, CN, C₁₋₃ alkoxy (e.g., methoxy (C₁), ethoxy (C₂), propoxy (C₃) or isopropoxy (C₃)) and -C(=O)-NR_{b1}R_{b2}.

In some embodiments of the present disclosure, R₈ is selected from H, F, Cl, Br, I, OH, NH₂, CN, C₁₋₃ alkyl (e.g., methyl (C₁), ethyl (C₂), n-propyl (C₃) or isopropyl (C₃)) and C₁₋₃ alkoxy (e.g., methoxy (C₁), ethoxy (C₂), propoxy (C₃) or isopropoxy (C₃)), wherein the C₁₋₃ alkyl and C₁₋₃ alkoxy are each independently optionally substituted with 1, 2, 3, 4 or 5 Rₐ.

In some embodiments of the present disclosure, R₈ is H.

In some embodiments of the present disclosure, R₈ is selected from F, Cl, Br, I, OH, NH₂, CN, C₁₋₃ alkyl (e.g., methyl (C₁), ethyl (C₂), n-propyl (C₃) or isopropyl (C₃)) and C₁₋₃ alkoxy (e.g., methoxy (C₁), ethoxy (C₂), propoxy (C₃) or isopropoxy (C₃)), wherein the C₁₋₃ alkyl and C₁₋₃ alkoxy are each independently optionally substituted with 1, 2, 3, 4 or 5 Rₐ.

In some embodiments of the present disclosure, R₈ is selected from F, Cl, NH₂, CN, C₁₋₃ alkyl (e.g., methyl (C₁), ethyl (C₂), n-propyl (C₃) or isopropyl (C₃)) and C₁₋₃ alkoxy (e.g., methoxy (C₁), ethoxy (C₂), propoxy (C₃) or isopropoxy (C₃)), wherein the C₁₋₃ alkyl and C₁₋₃ alkoxy are each independently optionally substituted with 1, 2, 3, 4 or 5 Rₐ.

In some embodiments of the present disclosure, R₈ and R_{8'} together with the carbon atom to which they are attached form a C₃₋₅ cycloalkyl group (e.g., cyclopropyl (C₃), cyclobutyl (C₄), cyclopentyl (C₅)) or a 3-to 5-membered heterocycloalkyl group (e.g., a heterocycloalkyl including a ring containing 1 to 3 heteroatoms selected from O, N and S), wherein the C₃₋₅ cycloalkyl group or the 3- to 5-membered heterocycloalkyl group are each independently optionally substituted with 1, 2 or 3 R₁₀.

In some embodiments of the present disclosure, R₈ and R_{8'} together with the carbon atom to which they are attached form a C₃₋₅ cycloalkyl group (e.g., cyclopropyl (C₃), cyclobutyl (C₄), cyclopentyl (C₅)), which is optionally substituted with 1, 2 or 3 R₁₀.

In some embodiments of the present disclosure, R₈ and R_{8'}, together with the carbon atom to which they are attached, form a 3- to 5-membered heterocycloalkyl group (e.g., a 3- to 5-membered heterocycloalkyl including a ring containing 1 to 2 heteroatoms selected from O, N, and S), wherein the 3- to 5-membered heterocycloalkyl group is each independently optionally substituted with 1, 2, or 3 R₁₀. In some embodiments of the present disclosure, R₈ and R_{8'}, together with the carbon atom to which they are attached, form a 3-membered heterocycloalkyl group containing 1 heteroatom selected from N, O, and S. In some embodiments of the present disclosure, R₈ and R_{8'}, together with the carbon atom to which they are attached, form a 3-membered heterocycloalkyl group containing 1 heteroatom selected from N and O. In some embodiments of the present disclosure, R₈ and R_{8'}, together with the carbon atom to which they are attached, form a 4-membered heterocycloalkyl group containing 1-2 heteroatoms selected from N, O, and S. In some embodiments of the present disclosure, R₈ and R_{8'}, together with the carbon atom to which they are attached, form a 4-membered heterocycloalkyl group containing 1-2 heteroatoms selected from N and O. In some embodiments of the present disclosure, R₈ and R_{8'}, together with the carbon atom to which they are attached, form a 4-membered heterocycloalkyl group containing 1 heteroatom selected from N and O. In some embodiments of the present disclosure, R₈ and R_{8'}, together with the carbon atom to which they are attached, form a 5-membered heterocycloalkyl group containing 1-2 heteroatoms selected from N, O and S. In some embodiments of the present disclosure, R₈ and R_{8'}, together with the carbon atom to which they are attached, form a 5-membered heterocycloalkyl group containing 1-2 heteroatoms selected from N and O. In some embodiments of the present disclosure, R₈ and R_{8'}, together with the carbon atom to which they are attached, form a 5-membered heterocycloalkyl group containing 1 heteroatom selected from N and O.

In some embodiments of the present disclosure, R₉ is selected from -C(=O)-NR_{b3}R_{b4} and -CH₂R_{c}. In some embodiments of the present disclosure, R₉ is selected from -C(=O)-NR_{b3}R_{b4}. In some embodiments of the present disclosure, R₉ is selected from -CH₂R_{c}.

In some embodiments of the present disclosure, R₉ is -C(=O)-NR_{b1}R_{b2}.

In some embodiments of the present disclosure, each R₁₀ is independently selected from oxo, D, F, Cl, Br, I, OH, NH₂, CN, C₁₋₃ alkyl (e.g., methyl (C₁), ethyl (C₂), n-propyl (C₃) or isopropyl (C₃)), C₁₋₃ alkoxy (e.g., methoxy (C₁), ethoxy (C₂), propoxy (C₃) or isopropyloxy (C₃)), C₁₋₃ alkylamino (e.g., methylamino, ethylamino, n-propylamino or isopropylamino), di-C₁₋₃ alkylamino (e.g., dimethylamino, diethylamino, di-n-propylamino, diisopropylamino, methylethylamino, methyl-n-propylamino, methylisopropylamino, ethyl-n-propylamino, ethylisopropylamino or n-propylisopropylamino), -C(=O)-R_{d}, -S-R_{d}, -S(=O)-R_{d}, -S(=O)₂-R_{d}, -NH-C(=O)-R_{d}, C₆₋₁₀ aryl and 5- to 10-membered heteroaryl, wherein the C₁₋₃ alkyl is optionally substituted with 1, 2 or 3 OH or F, and the C₆₋₁₀ aryl and 5- to 10-membered heteroaryl are each independently optionally substituted with 1, 2, 3, 4 or 5 Rₛ₁.

In some embodiments of the present disclosure, at least one R₁₀ is selected from oxo, D, F, Cl, Br, I, OH, NH₂, CN, C₁₋₃ alkyl (e.g., methyl (C₁), ethyl (C₂), n-propyl (C₃) or isopropyl (C₃)), C₁₋₃ alkoxy (e.g., methoxy (C₁), ethoxy (C₂), propoxy (C₃) or isopropyloxy (C₃)), C₁₋₃ alkylamino (e.g., methylamino, ethylamino, n-propylamino or isopropylamino), di-C₁₋₃ alkylamino (e.g., dimethylamino, diethylamino, di-n-propylamino, diisopropylamino, methylethylamino, methyl-n-propylamino, methylisopropylamino, ethyl-n-propylamino, ethylisopropylamino or n-propylisopropylamino), -S-R_{d}, -S(=O)-R_{d}, -S(=O)₂-R_{d} and -NH-C(=O)-R_{d}, wherein the C₁₋₃ alkyl group is optionally substituted with 1, 2 or 3 OH or F groups.

In some embodiments of the present disclosure, at least one R₁₀ is selected from oxo, D, F, Cl, OH, NH₂, CN, C₁₋₃ alkyl (e.g., methyl (C₁), ethyl (C₂), n-propyl (C₃) or isopropyl (C₃)), C₁₋₃ alkoxy (e.g., methoxy (C₁), ethoxy (C₂), propoxy (C₃) or isopropyloxy (C₃)), C₁₋₃ alkylamino (e.g., methylamino, ethylamino, n-propylamino or isopropylamino), di-C₁₋₃ alkylamino (e.g., dimethylamino, diethylamino, di-n-propylamino, diisopropylamino, methylethylamino, methyl-n-propylamino, methylisopropylamino, ethyl-n-propylamino, ethylisopropylamino or n-propylisopropylamino), -S-R_{d}, -S(=O)-R_{d}, -S(=O)₂-R_{d} and -NH-C(=O)-R_{d}, wherein the C₁₋₃ alkyl group is optionally substituted with 1, 2 or 3 OH or F groups.

In some embodiments of the present disclosure, at least one R₁₀ is selected from oxo, F, Cl, C₁₋₃ alkyl (e.g., methyl (C₁), ethyl (C₂), n-propyl (C₃) or isopropyl (C₃)), C₁₋₃ alkoxy (e.g., methoxy (C₁), ethoxy (C₂), propoxy (C₃) or isopropyloxy (C₃)), C₁₋₃ alkylamino (e.g., methylamino, ethylamino, n-propylamino or isopropylamino), di-C₁₋₃ alkylamino (e.g., dimethylamino, diethylamino, di-n-propylamino, diisopropylamino, methylethylamino, methyl-n-propylamino, methylisopropylamino, ethyl-n-propylamino, ethylisopropylamino or n-propylisopropylamino), wherein the C₁₋₃ alkyl is optionally substituted with 1, 2 or 3 OH or F groups.

In some embodiments of the present disclosure, at least one R₁₀ is selected from -C(=O)-R_{d}, -S-R_{d},-S(=O)-R_{d}, -S(=O)₂-R_{d}, and -NH-C(=O)-R_{d}.

In some embodiments of the present disclosure, at least one R₁₀ is selected from C₆₋₁₀ aryl and 5- to 10-membered heteroaryl, wherein the C₆₋₁₀ aryl and 5- to 10-membered heteroaryl are each independently optionally substituted with 1, 2, 3, 4 or 5 Rₛ₁.

In some embodiments of the present disclosure, R_{b1} and R_{b2} are each independently selected from H, C₁₋₆ alkyl (e.g., methyl (C₁), ethyl (C₂), n-propyl (C₃), isopropyl (C₃), n-butyl (C₄), isobutyl (C₄), m-butyl (C₄), tert-butyl (C₄), pentyl (C₅) or hexyl (C₆)), C₁₋₆ alkoxy (e.g., methoxy (C₁), ethoxy (C₂), propoxy (C₃), isopropoxy (C₃), butoxy (C₄), isobutoxy (C₄), sec-butoxy (C₄), tert-butoxy (C₄), pentyloxy (C₅), hexyloxy (C₆)), C₃₋₁₀ cycloalkyl (e.g., cyclopropyl (C₃), cyclobutyl (C₄), cyclopentyl (C₅), cyclohexyl (C₆)), C₃₋₁₀ cycloalkyl (e.g., cyclopropyl (C₃), cyclobutyl (C₄), cyclopentyl (C₅), cyclohexyl (C₆), cycloheptyl (C₇), cyclooctyl (C₈), bicyclo[2.2.1]heptyl (C₇), bicyclo[2.2.2]octyl (C₈), cyclononyl (C₉), cyclodecyl (C₁₀), octahydro-1H-indenyl (C₉), decahydronaphthyl (C₁₀) or spiro[4.5]decyl (C₁₀)), 3- to 10-membered heterocycloalkyl (e.g., a heterocycloalkyl including one or two 3- to 8-membered rings and 1-5 heteroatoms selected from N, O and S), C₆₋₁₀ aryl (e.g., phenyl or naphthyl) and 5- to 10-membered heteroaryl (e.g., a heteroaryl including one or two 5-membered or 6-membered rings and 1-5 heteroatoms selected from N, O and S), wherein the C₁₋₆ alkyl, C₁₋₆ alkoxy, C₃₋₁₀ cycloalkyl, 3- to 10-membered heterocycloalkyl, C₆₋₁₀ aryl and 5- to 10-membered heteroaryl are each independently optionally substituted with 1, 2, 3 or 4 Rₑ₁.

In some embodiments of the present disclosure, at least one of R_{b1} and R_{b2} is selected from C₁₋₆ alkyl (e.g., methyl (C₁), ethyl (C₂), n-propyl (C₃), isopropyl (C₃), n-butyl (C₄), isobutyl (C₄), m-butyl (C₄), tert-butyl (C₄), pentyl (C₅) or hexyl (C₆)), C₃₋₆ cycloalkyl (e.g., cyclopropyl (C₃), cyclobutyl (C₄), cyclopentyl (C₅), cyclohexyl (C₆)), C₁₋₆ alkoxy (e.g., methoxy (C₁), ethoxy (C₂), propoxy (C₃), isopropyl (C₃), butoxy (C₄), isobutoxy (C₄), sec-butoxy (C₄), tert-butoxy (C₄), pentyloxy (C₅), hexyloxy (C₆)), C₃₋₁₀ cycloalkyl (e.g., cyclopropyl (C₃), cyclobutyl (C₄), cyclopentyl (C₅), cyclohexyl (C₆), cycloheptyl (C₇), cyclooctyl (C₈), bicyclo[2.2.1]heptyl (C₇), bicyclo[2.2.2]octyl (C₈), cyclononyl (C₉), cyclodecyl (C₁₀), octahydro-1H-indenyl (C₉), decahydronaphthyl (C₁₀) or spiro[4.5]decyl (C₁₀)), 3- to 10-membered heterocycloalkyl (for example, the heterocycloalkyl including one or two 3- to 8-membered rings and 1 to 5 heteroatoms selected from N, O and S, wherein the heterocycloalkyl can be monocyclic or polycyclic, such as spirocyclic, bridged or fused polycyclic), C₆₋₁₀ aryl (for example, phenyl or naphthyl) and 5- to 10-membered heteroaryl (for example, the heteroaryl including one or two 5-membered or 6-membered rings and 1 to 5 heteroatoms selected from N, O and S), wherein the C₁₋₆ alkyl, C₁₋₆ alkoxy, C₃₋₁₀ cycloalkyl, 3- to 10-membered heterocycloalkyl, C₆₋₁₀ aryl and 5- to 10-membered heteroaryl are each independently optionally substituted with 1, 2, 3 or 4 Rₑ₁.

In some embodiments of the present disclosure, at least one of R_{b1} and R_{b2} is selected from C₁₋₆ alkyl (e.g., methyl (C₁), ethyl (C₂), n-propyl (C₃), isopropyl (C₃), n-butyl (C₄), isobutyl (C₄), m-butyl (C₄), tert-butyl (C₄), pentyl (C₅) or hexyl (C₆)), C₃₋₆ cycloalkyl (e.g., cyclopropyl (C₃), cyclobutyl (C₄), cyclopentyl (C₅), cyclohexyl (C₆)) and C₁₋₆ alkoxy (e.g., methoxy (C₁), ethoxy (C₂), propoxy (C₃), isopropyl (C₃), butoxy (C₄), isobutoxy (C₄), sec-butoxy (C₄), tert-butoxy (C₄), pentyloxy (C₅), hexyloxy (C₆)), wherein the C₁₋₆ alkyl and C₁₋₆ alkoxy groups are each independently optionally substituted with 1, 2, 3 or 4 Rₑ₁ groups.

In some embodiments of the present disclosure, at least one of R_{b1} and R_{b2} is selected from C₃₋₁₀ cycloalkyl (for example, cyclopropyl (C₃), cyclobutyl (C₄), cyclopentyl (C₅), cyclohexyl (C₆), cycloheptyl (C₇), cyclooctyl (C₈), bicyclo[2.2.1]heptyl (C₇), bicyclo[2.2.2]octyl (C₈), cyclononyl (C₉), cyclodecyl (C₁₀), octahydro-1H-indenyl (C₉), decahydronaphthyl (C₁₀) or spiro[4.5]decyl (C₁₀)), 3- to 10-membered heterocycloalkyl (for example, a heterocycloalkyl including one or two 3- to 8-membered rings and 1-5 heteroatoms selected from N, O and S, wherein the heterocycloalkyl group may be monocyclic or polycyclic, such as spirocyclic, bridged or fused polycyclic), C₆₋₁₀ aryl (e.g., phenyl or naphthyl) and 5- to 10-membered heteroaryl (e.g., a heteroaryl including one or two 5-membered or 6-membered rings and 1 to 5 heteroatoms selected from N, O and S), wherein the C₃₋₁₀ cycloalkyl, 3- to 10-membered heterocycloalkyl, C₆₋₁₀ aryl and 5-to 10-membered heteroaryl are each independently optionally substituted with 1, 2, 3 or 4 Rₑ₁.

In some embodiments of the present disclosure, at least one of R_{b1} and R_{b2} is selected from C₃₋₆ cycloalkyl (e.g., cyclopropyl (C₃), cyclobutyl (C₄), cyclopentyl (C₅), cyclohexyl (C₆)), 3- to 6-membered heterocycloalkyl, C₆- aryl and 5- to 6-membered heteroaryl (e.g., a heteroaryl including 1-3 heteroatoms selected from N, O and S), wherein the C₃₋₆ cycloalkyl, 3- to 6-membered heterocycloalkyl, C₆- aryl and 5- to 6-membered heteroaryl are each independently optionally substituted with 1, 2, 3 or 4 Rₑ₁.

In some embodiments of the present disclosure, R_{b1} and R_{b2} together with the nitrogen atom to which they are attached form a 3- to 6-membered heterocycloalkyl group (e.g., a heterocycloalkyl including one or two 3- to 6-membered rings and 1-4 heteroatoms selected from N, O and S), wherein the 3- to 6-membered heterocycloalkyl group is optionally substituted with 1, 2, 3 or 4 Rₑ₁ groups.

In some embodiments of the present disclosure, R_{b3} and R_{b4} are independently H, C₁₋₆ alkyl, C₁₋₆ alkoxy (e.g., methoxy (C₁), ethoxy (C₂), propoxy (C₃), isopropoxy (C₃), butoxy (C₄), isobutoxy (C₄), sec-butoxy (C₄), tert-butoxy (C₄), pentyloxy (C₅), hexyloxy (C₆)), C₃₋₁₀ cycloalkyl (e.g., cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), cycloheptyl (C₇), cycloheptenyl (C₇),cycloheptadienyl (C₇), cycloheptatrienyl (C₇), cyclooctyl (C₈), cyclooctenyl (C₈), bicyclo[2.2.1]heptanyl (C₇), bicyclo[2.2.2]octanyl (C₈), cyclononyl (C₉), cyclononenyl (C₉), cyclodecyl (C₁₀), cyclodecenyl (C₁₀), octahydro-*1H*-indenyl (C₉), decalinyl (C₁₀) or spiro[4.5]decyl (C₁₀)), 3- to 10-membered heterocycloalkyl (e.g., a heterocycloalkyl including one or two 3- to 8-membered rings and 1-5 heteroatoms selected from N, O and S), C₆₋₁₀ aryl (e.g., phenyl or naphthyl), 5 to 10 membered heteroaryl (e.g., a heteroaryl including one or two 5- or 6-membered rings and 1-5 heteroatoms selected from N, O and S), wherein the alkyl, alkoxy, cycloalkyl, heterocycloalkyl, aryl or heteroaryl group is optionally substituted with 1, 2, 3 or 4 Rₑ₂.

In some embodiments of the present disclosure, at least one of R_{b1} and R_{b2} is C₁₋₆ alkyl or C₁₋₆ alkoxy (e.g., methoxy (C₁), ethoxy (C₂), propoxy (C₃), isopropoxy (C₃), butoxy (C₄), isobutoxy (C₄), sec-butoxy (C₄), tert-butoxy (C₄), pentyloxy (C₅), hexyloxy (C₆)), wherein the alkyl or alkoxy is optionally substituted with 1, 2, 3 or 4 Rₑ₂.

In some embodiments of the present disclosure, at least one of R_{b1} and R_{b2} is C₃₋₁₀ cycloalkyl (e.g., cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), cycloheptyl (C₇), cycloheptenyl (C₇),cycloheptadienyl (C₇), cycloheptatrienyl (C₇), cyclooctyl (C₈), cyclooctenyl (C₈), bicyclo[2.2.1]heptyl (C₇), bicyclo[2.2.2]octyl (C₈), cyclononyl (C₉), cyclononenyl (C₉), cyclodecyl (C₁₀), cyclodecenyl (C₁₀), octahydro-1H-indenyl (C₉), decahydronaphthyl (C₁₀) or spiro[4.5]decyl (C₁₀)), 3- to 10-membered heterocycloalkyl (e.g., a heterocycloalkyl including one or two 3- to 8-membered rings and 1-5 heteroatoms selected from N, O and S, wherein the heterocycloalkyl can be monocyclic or polycyclic, such as spiro polycyclic, bridged polycyclic or fused polycyclic), C₆₋₁₀ aryl (e.g., phenyl or naphthyl), 5- to 10-membered heteroaryl (e.g., a heteroaryl comprising one or two 5- or 6-membered rings and 1-5 heteroatoms selected from N, O and S), wherein the cycloalkyl, heterocycloalkyl, aryl or heteroaryl is optionally substituted with 1, 2, 3 or 4 Rₑ₂.

In some embodiments of the present disclosure, at least one of R_{b1} and R_{b2} is C₃₋₆ cycloalkyl (e.g., cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆)), 3- to 6-membered heterocycloalkyl (e.g., a heterocycloalkyl containing 1-3 heteroatoms selected from N, O and S), C₆ aryl, 5- to 6-membered heteroaryl (e.g., a heteroaryl containing 1-4 heteroatoms selected from N, O and S), wherein the cycloalkyl, heterocycloalkyl, aryl or heteroaryl is optionally substituted with 1, 2, 3 or 4 Rₑ₂.

In some embodiments of the present disclosure, R_{b3} and R_{b4}, together with the nitrogen atom to which they are attached, form a 3- to 6-membered heterocycloalkyl group (e.g., a heterocycloalkyl group containing 1-4 heteroatoms selected from N, O and S), wherein the 3- to 6-membered heterocycloalkyl group is optionally substituted with 1, 2, 3 or 4 Rₑ₂.

In some embodiments of the present disclosure, R_{c} is selected from F, Cl, Br, I, OH, NH₂,-(C=O)NR_{C1}R_{C2}, -O(C=O)NR_{C1}R_{C2}, -NR_{C0}(C=O)R_{C1} and -NR_{C0}(C=O)NR_{C1}R_{C2}. In some embodiments of the present disclosure, R_{c} is selected from F, Cl, OH, NH₂, -O(C=O)NR_{C1}R_{C2}, -NR_{C0}(C=O)R_{C1} and-NR_{C0}(C=O)NR_{C1}R_{C2}. In some embodiments of the present disclosure, R_{c} is selected from F, Cl,-O(C=O)NR_{C1}R_{C2}, -NR_{C0}(C=O)R_{C1} and -NR_{C0}(C=O)NR_{C1}R_{C2}. In some embodiments of the present disclosure, R_{c} is selected from F, Cl, OH and NH₂. In some embodiments of the present disclosure, R_{c} is selected from-O(C=O)NR_{C1}R_{C2}, -NR_{C0}(C=O)R_{C1} and -NR_{C0}(C=O)NR_{C1}R_{C2}.

In some embodiments of the present disclosure, R_{C0}, R_{C1} and R_{C2} are each independently selected from H, C₁₋₆ alkyl (e.g., methyl (C₁), ethyl (C₂), n-propyl (C₃), isopropyl (C₃), n-butyl (C₄), isobutyl (C₄), m-butyl (C₄), tert-butyl (C₄), pentyl (C₅) or hexyl (C₆)), C₃₋₆ cycloalkyl (e.g., cyclopropyl (C₃), cyclobutyl (C₄), cyclopentyl (C₅), cyclohexyl (C₆)) and 3- to 6-membered heterocycloalkyl (e.g., a heterocycloalkyl including one or two 3- to 6-membered rings and 1-4 heteroatoms selected from N, O and S).

In some embodiments of the present disclosure, R_{d} is selected from C₁₋₃ alkyl (e.g., methyl (C₁), ethyl (C₂), n-propyl (C₃) or isopropyl (C₃)).

In some embodiments of the present disclosure, Rₑ₁ is F, Cl, Br, I, OH, NH₂, NO₂, C₁₋₃ alkyl (e.g., methyl (C₁), ethyl (C₂), n-propyl (C₃) or isopropyl (C₃)), C₁₋₃ alkylamino (e.g., methylamino, ethylamino, n-propylamino or isopropylamino), di-C₁₋₃ alkylamino (e.g., dimethylamino, diethylamino, di-n-propylamino, diisopropylamino, methylethylamino, methyl-n-propylamino, methyl-isopropylamino, ethyl-n-propylamino, ethyl-isopropylamino or n-propyl-isopropylamino), CN, C₁₋₃ alkoxy (e.g., methoxy (C₁), ethoxy (C₂), propoxy (C₃) or isopropyloxy (C₃)), -S(=O)₂-(C₁₋₃ alkyl), -(C=O)(C₁₋₃ alkyl), -(C=O)O(C₁₋₃ alkyl), -(C=O)NH(C₁₋₃ alkyl), -(C=O)N(C₁₋₃ alkyl)₂, C₃₋₁₀ cycloalkyl (e.g., cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), cycloheptyl (C₇), cycloheptenyl (C₇),cycloheptadienyl (C₇), cycloheptatrienyl (C₇), cyclooctyl (C₈), cyclooctenyl (C₈), bicyclo[2.2.1]heptanyl (C₇), bicyclo[2.2.2]octyl (C₈), cyclononyl (C₉), cyclononenyl (C₉), cyclodecyl (C₁₀), cyclodecenyl (C₁₀), octahydro-*1H*-indenyl (C₉), decalinyl (C₁₀) or spiro[4.5]decanyl (C₁₀)), 3 to 10 membered heterocycloalkyl (for example, a heterocycloalkyl including one or two 3 to 8 membered rings and 1 to 5 heteroatoms selected from N, O and S, wherein the heterocycloalkyl may be monocyclic or polycyclic, such as spiro polycyclic, bridged polycyclic or fused polycyclic), C₆₋₁₀ aryl (for example, phenyl or naphthyl), 5 to 10 membered heteroaryl (for example, a heteroaryl comprising one or two 5- or 6-membered rings and 1 to 5 heteroatoms selected from N, O and S).

In some embodiments of the present disclosure, Rₑ₁ is F, Cl, Br, I, OH, NH₂, NO₂, C₁₋₃ alkyl (e.g., methyl (C₁), ethyl (C₂), n-propyl (C₃) or isopropyl (C₃)), C₁₋₃ alkylamino (e.g., methylamino, ethylamino, n-propylamino or isopropylamino), di-C₁₋₃ alkylamino (e.g., dimethylamino, diethylamino, di-n-propylamino, diisopropylamino, methylethylamino, methyl-n-propylamino, methyl-isopropylamino, ethyl-n-propylamino, ethyl-isopropylamino or n-propyl-isopropylamino), CN, C₁₋₃ alkoxy (e.g., methoxy (C₁), ethoxy (C₂), propoxy (C₃) or isopropyloxy (C₃)), -S(=O)₂-(C₁₋₃ alkyl), -(C=O)(C₁₋₃ alkyl), -(C=O)O(C₁₋₃ alkyl), -(C=O)NH(C₁₋₃ alkyl) or -(C=O)N(C₁₋₃ alkyl)₂.

In some embodiments of the present disclosure, Rₑ₁ is F, Cl, Br, I, OH, NH₂, NO₂, C₁₋₃ alkyl (e.g. methyl (C₁), ethyl (C₂), n-propyl (C₃) or isopropyl (C₃)), C₁₋₃ alkylamino (e.g. methylamino, ethylamino, n-propylamino or isopropylamino), di-C₁₋₃ alkylamino (e.g. dimethylamino, diethylamino, di-n-propylamino, diisopropylamino, methylethylamino, methyl-n-propylamino, methyl-isopropylamino, ethyl-n-propylamino, ethyl-isopropylamino or n-propyl-isopropylamino), CN or C₁₋₃ alkoxy (e.g. methoxy (C₁), ethoxy (C₂), propoxy (C₃) or isopropyloxy (C₃)).

In some embodiments of the present disclosure, Rₑ₁ is -S(=O)₂-(C₁₋₃ alkyl), -(C=O)(C₁₋₃ alkyl),-(C=O)O(C₁₋₃ alkyl), -(C=O)NH(C₁₋₃ alkyl) or -(C=O)N(C₁₋₃ alkyl)₂.

In some embodiments of the present disclosure, Rₑ₁ is selected from F, Cl, Br, I, OH, NH₂, NO₂, C₁₋₃ alkyl (e.g., methyl (C₁), ethyl (C₂), n-propyl (C₃) or isopropyl (C₃)), C₁₋₃ alkylamino (e.g., methylamino, ethylamino, n-propylamino or isopropylamino), di-C₁₋₃ alkylamino (e.g., dimethylamino, diethylamino, di-n-propylamino, diisopropylamino, methylethylamino, methyl-n-propylamino, methylisopropylamino, ethyl-n-propylamino, ethylisopropylamino or n-propylisopropylamino), CN, C₁₋₃ alkoxy (e.g., methoxy (C₁), ethoxy (C₂), propoxy (C₃) or isopropyloxy (C₃)), C₃₋₁₀ cycloalkyl (e.g., cyclopropyl (C₃), cyclobutyl (C₄), cyclopentyl (C₈), cyclohexyl (C₆), cycloheptyl (C₇), cyclooctyl (C₈), bicyclo[2.2.1]heptyl (C₇), bicyclo[2.2.2]octyl (C₈), cyclononyl (C₉), cyclodecyl (C₁₀), octahydro-1H-indenyl (C₉), decahydronaphthyl (C₁₀) or spiro[4.5]decyl (C₁₀)), 3- to 10-membered heterocycloalkyl (for example, a heterocycloalkyl including one or two 3- to 8-membered rings and 1-5 heteroatoms selected from N, O and S, wherein the heterocycloalkyl group may be monocyclic or polycyclic, for example, a spirocyclic, bridged or fused polycyclic ring), C₆₋₁₀ aryl (e.g., phenyl or naphthyl) and 5- to 10-membered heteroaryl (e.g., a heteroaryl including one or two 5-membered or 6-membered rings and 1-5 heteroatoms selected from N, O and S).

In some embodiments of the present disclosure, Rₑ₁ is C₃₋₁₀ cycloalkyl (e.g., cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), cycloheptyl (C₇), cycloheptenyl (C₇),cycloheptadienyl (C₇), cycloheptatrienyl (C₇), cyclooctyl (C₈), cyclooctenyl (C₈), bicyclo[2.2.1]heptyl (C₇), bicyclo[2.2.2]octyl (C₈), cyclononyl (C₉), cyclononenyl (C₉), cyclodecyl (C₁₀), cyclodecenyl (C₁₀), octahydro-1H-indenyl (C₉), decalinyl (C₁₀) or spiro[4.5]decanyl (C₁₀)), 3- to 10-membered heterocycloalkyl (for example, a heterocycloalkyl including one or two 3- to 8-membered rings and 1-5 heteroatoms selected from N, O and S, wherein the heterocycloalkyl may be monocyclic or polycyclic, such as spiro polycyclic, bridged polycyclic or fused polycyclic), C₆₋₁₀ aryl (for example, phenyl or naphthyl), 5- to 10-membered heteroaryl (for example, a heteroaryl comprising one or two 5- or 6-membered rings and 1-5 heteroatoms selected from N, O and S).

In some embodiments of the present disclosure, Rₑ₂ is F, Cl, Br, I, OH, NH₂, NO₂, C₁₋₃ alkyl (e.g., methyl (C₁), ethyl (C₂), n-propyl (C₃) or isopropyl (C₃)), C₁₋₃ alkylamino (e.g., methylamino, ethylamino, n-propylamino or isopropylamino), di-C₁₋₃ alkylamino (e.g., dimethylamino, diethylamino, di-n-propylamino, diisopropylamino, methylethylamino, methyl-n-propylamino, methylisopropylamino, ethyl-n-propylamino, ethylisopropylamino or n-propylisopropylamino), CN, C₁₋₃ alkoxy (e.g., methoxy (C₁), ethoxy (C₂), propoxy (C₃) or isopropoxy (C₃)), -S(=O)₂-(C₁₋₃ alkyl), -(C=O)(C₁₋₃ alkyl), -(C=O)O(C C₁₋₃ alkyl), -(C=O)NH(C₁₋₃ alkyl), -(C=O)N(C₁₋₃ alkyl)₂, C₃₋₁₀ cycloalkyl (e.g., cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), cycloheptyl (C₇), cycloheptenyl (C₇),cycloheptadienyl (C₇), cycloheptatrienyl (C₇), cyclooctyl (C₈), cyclooctenyl (C₈), bicyclo[2.2.1]heptyl (C₇), bicyclo[2.2.2]octyl (C₈), cyclononyl (C₉), cyclononenyl (C₉), cyclodecyl (C₁₀), cyclodecenyl (C₁₀), octahydro-*1H*-indenyl (C₉), decalinyl (C₁₀) or spiro[4.5]decanyl (C₁₀)), 3 to 10-membered heterocycloalkyl (e.g., a heterocycloalkyl including one or two 3 to 8-membered rings and 1-5 heteroatoms selected from N, O and S, wherein the heterocycloalkyl can be monocyclic or polycyclic, such as spiro polycyclic, bridged polycyclic or fused polycyclic), C₆₋₁₀ aryl (e.g., phenyl or naphthyl), 5 to 10-membered heteroaryl (e.g., a heteroaryl comprising one or two 5- or 6-membered rings and 1-5 heteroatoms selected from N, O and S), wherein the cycloalkyl, heterocycloalkyl, aryl and heteroaryl are each independently optionally substituted with 1, 2, 3, 4 or 5 Rₛ₁, and wherein the alkyl and alkoxy groups are each independently optionally substituted with 1, 2, 3, 4 or 5 Rₛ₂.

In some embodiments of the present disclosure, Rₑ₂ is F, Cl, Br, I, OH, NH₂, NO₂, C₁₋₃ alkyl (e.g., methyl (C₁), ethyl (C₂), n-propyl (C₃) or isopropyl (C₃)), C₁₋₃ alkylamino (e.g., methylamino, ethylamino, n-propylamino or isopropylamino), di-C₁₋₃ alkylamino (e.g., dimethylamino, diethylamino, di-n-propylamino, diisopropylamino, methylethylamino, methyl-n-propylamino, methylisopropylamino, ethyl-n-propylamino, ethylisopropylamino or n-propylisopropylamino), CN, C₁₋₃ alkoxy (e.g., methoxy (C₁), ethoxy (C₂), propoxy (C₃) or isopropoxy (C₃)), -S(=O)₂-(C₁₋₃ alkyl), -(C=O)(C₁₋₃ alkyl), -(C=O)O(C₁₋₃ alkyl), -(C=O)NH(C₁₋₃ alkyl) or -(C=O)N(C₁₋₃ alkyl)₂, wherein the alkyl and alkoxy groups are each independently optionally substituted with 1, 2, 3, 4 or 5 Rₛ₂.

In some embodiments of the present disclosure, Rₑ₂ is F, Cl, Br, I, OH, NH₂, NO₂, C₁₋₃ alkyl (e.g. methyl (C₁), ethyl (C₂), n-propyl (C₃) or isopropyl (C₃)), C₁₋₃ alkylamino (e.g. methylamino, ethylamino, n-propylamino or isopropylamino), di-C₁₋₃ alkylamino (e.g. dimethylamino, diethylamino, di-n-propylamino, diisopropylamino, methylethylamino, methyl-n-propylamino, methylisopropylamino, ethyl-n-propylamino, ethylisopropylamino or n-propylisopropylamino), CN or C₁₋₃ alkoxy (e.g. methoxy (C₁), ethoxy (C₂), propoxy (C₃) or isopropoxy (C₃)), wherein the alkyl and alkoxy groups are each independently optionally substituted with 1, 2, 3, 4 or 5 Rₛ₂.

In some embodiments of the present disclosure, Rₑ₂ is -S(=O)₂-(C₁₋₃ alkyl), -(C=O)(C₁₋₃ alkyl),-(C=O)O(C₁₋₃ alkyl), -(C=O)NH(C₁₋₃ alkyl) or -(C=O)N(C₁₋₃ alkyl)₂.

In some embodiments of the present disclosure, Rₑ₂ is F, Cl, Br, I, OH, NH₂, NO₂, C₁₋₃ alkyl (e.g., methyl (C₁), ethyl (C₂), n-propyl (C₃) or isopropyl (C₃)), C₁₋₃ alkylamino (e.g., methylamino, ethylamino, n-propylamino or isopropylamino), di-C₁₋₃ alkylamino (e.g., dimethylamino, diethylamino, di-n-propylamino, diisopropylamino, methylethylamino, methyl-n-propylamino, methylisopropylamino, ethyl-n-propylamino, ethylisopropylamino or n-propylisopropylamino), CN, C₁₋₃ alkoxy (e.g., methoxy (C₁), ethoxy (C₂), propoxy (C₃) or isopropyloxy (C₃)), C₃₋₁₀ cycloalkyl (e.g., cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), cycloheptyl (C₇), cycloheptenyl (C₇),cycloheptadienyl (C₇), cycloheptatrienyl (C₇), cyclooctyl (C₈), cyclooctenyl (C₈), bicyclo[2.2.1]heptyl (C₇), bicyclo[2.2.2]octyl (C₈), cyclononyl (C₉), cyclononenyl (C₉), cyclodecyl (C₁₀), cyclodecenyl (C₁₀), octahydro-*1H*-indenyl (C₉), decalinyl (C₁₀) or spiro[4.5]decyl (C₁₀)), 3 to 10 membered heterocycloalkyl (e.g., a heterocycloalkyl including one or two 3 to 8 membered rings and 1-5 heteroatoms selected from N, O and S, wherein the heterocycloalkyl can be monocyclic or polycyclic, such as spiro polycyclic, bridged polycyclic or fused polycyclic), C₆₋₁₀ aryl (e.g., phenyl or naphthyl), 5 to 10 membered heteroaryl (e.g., a heteroaryl comprising one or two 5- or 6-membered rings and 1-5 heteroatoms selected from N, O and S), wherein the cycloalkyl, heterocycloalkyl, aryl and heteroaryl are each independently optionally substituted with 1, 2, 3, 4 or 5 Rₛ₁, wherein the alkyl and alkoxy are each independently optionally substituted with 1, 2, 3, 4 or 5 Rₛ₂.

In some embodiments of the present disclosure, Rₑ₂ is C₃₋₁₀ cycloalkyl (e.g., cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), cycloheptyl (C₇), cycloheptenyl (C₇),cycloheptadienyl (C₇), cycloheptatrienyl (C₇), cyclooctyl (C₈), cyclooctenyl (C₈), bicyclo[2.2.1]heptyl (C₇), bicyclo[2.2.2]octyl (C₈), cyclononyl (C₉), cyclononenyl (C₉), cyclodecyl (C₁₀), cyclodecenyl (C₁₀), octahydro-*1H*-indenyl (C₉), decalinyl (C₁₀) or spiro[4.5]decyl (C₁₀)), 3 to 10 membered heterocycloalkyl (e.g., a heterocycloalkyl including one or two 3 to 8 membered rings and 1-5 heteroatoms selected from N, O and S, wherein the heterocycloalkyl can be monocyclic or polycyclic, such as spiro polycyclic, bridged polycyclic or fused polycyclic), C₆₋₁₀ aryl (e.g., phenyl or naphthyl), 5 to 10 membered heteroaryl (e.g., a heteroaryl comprising one or two 5- or 6-membered rings and 1-5 heteroatoms selected from N, O and S), wherein the cycloalkyl, heterocycloalkyl, aryl and heteroaryl are each independently optionally substituted with 1, 2, 3, 4 or 5 Rₛ₁.

In some embodiments of the present disclosure, two or more Rₑ₂ groups together with the carbon atom (s) to which they are attached form a C₆ aryl group or a 5- or 6-membered heteroaryl group.

In some embodiments of the present disclosure, Rₛ₁ is oxo, F, Cl, Br, I, OH, NH₂, NO₂, C₁₋₆ alkyl (e.g., methyl (C₁), ethyl (C₂), n-propyl (C₃), isopropyl (C₃), n-butyl (C₄), isobutyl (C₄), sec-butyl (C₄), tert-butyl (C₄), pentyl (C₅) or hexyl (C₆)), C₁₋₆ alkylamino (e.g., methylamino, ethylamino, n-propylamino or isopropylamino), di-C₁₋₆ alkylamino (e.g., dimethylamino, diethylamino, di-n-propylamino, diisopropylamino, methylethylamino, methyl-n-propylamino, methylisopropylamino, ethyl-n-propylamino, ethylisopropylamino or n-propylisopropylamino), CN, C₁₋₆ alkoxy (e.g., methoxy (C₁), ethoxy (C₂), propoxy (C₃), isopropoxy (C₃), butoxy (C₄), isobutoxy (C₄), sec-butoxy (C₄), tert-butoxy (C₄), pentyloxy (C₅), hexyloxy (C₆)), -S(=O)₂-(C₁₋₃ alkyl),-(C=O)(C₁₋₃ alkyl), -(C=O)O(C₁₋₃ alkyl), -(C=O)NH(C₁₋₃ alkyl), or -(C=O)N(C₁₋₃ alkyl)₂.

In some embodiments of the present disclosure, Rₛ₁ is oxo, F, Cl, Br, I, OH, NH₂, NO₂, C₁₋₆ alkyl (e.g., methyl (C₁), ethyl (C₂), n-propyl (C₃), isopropyl (C₃), n-butyl (C₄), isobutyl (C₄), sec-butyl (C₄), tert-butyl (C₄), pentyl (C₅) or hexyl (C₆)), C₁₋₆ alkylamino (e.g., methylamino, ethylamino, n-propylamino or isopropylamino), di-C₁₋₆ alkylamino (e.g., dimethylamino, diethylamino, di-n-propylamino, diisopropylamino, methylethylamino, methyl-n-propylamino, methylisopropylamino, ethyl-n-propylamino, ethylisopropylamino or n-propylisopropylamino), CN or C₁₋₆ alkoxy (e.g., methoxy (C₁), ethoxy (C₂), propoxy (C₃), isopropoxy (C₃), butoxy (C₄), isobutoxy (C₄), sec-butoxy (C₄), tert-butoxy (C₄), pentyloxy (C₅), hexyloxy (C₆)).

In some embodiments of the present disclosure, Rₛ₁ is C₁₋₆ alkyl (e.g., methyl (C₁), ethyl (C₂), n-propyl (C₃), isopropyl (C₃), n-butyl (C₄), isobutyl (C₄), sec-butyl (C₄), tert-butyl (C₄), pentyl (C₅) or hexyl (C₆)), C₁₋₆ alkylamino (e.g., methylamino, ethylamino, n-propylamino or isopropylamino), di-C₁₋₆ alkylamino (e.g., dimethylamino, diethylamino, di-n-propylamino, diisopropylamino, methylethylamino, methyl-n-propylamino, methylisopropylamino, ethyl-n-propylamino, ethylisopropylamino or n-propylisopropylamino), CN, C₁₋₆ alkoxy (e.g., methoxy (C₁), ethoxy (C₂), propoxy (C₃), isopropyloxy (C₃), butoxy (C₄)), isobutoxy(C₄), sec-butoxy(C₄), tert-butoxy(C₄), pentyloxy (C₅), hexyloxy (C₆)), -S(=O)₂-(C₁₋₃ alkyl), -(C=O)(C₁₋₃ alkyl), -(C=O)O(C₁₋₃ alkyl), -(C=O)NH(C₁₋₃ alkyl), or -(C=O)N(C₁₋₃ alkyl)₂.

In some embodiments of the present disclosure, Rₛ₁ is oxo, F, Cl, Br, I, OH, NH₂, NO₂, -S(=O)₂-(C₁₋₃ alkyl), -(C=O)(C₁₋₃ alkyl), -(C=O)O(C₁₋₃ alkyl), -(C=O)NH(C₁₋₃ alkyl) or -(C= O)N(C₁₋₃ alkyl)₂.

In some embodiments of the present disclosure, Rₛ₁ is -(C=O)(C₁₋₃ alkyl), -(C=O)O(C₁₋₃ alkyl),-(C=O)NH(C₁₋₃ alkyl) or -(C=O)N(C₁₋₃ alkyl)₂.

In some embodiments of the present disclosure, Rₛ₂ is F, Cl, Br, I, OH, NH₂, C₁₋₆ alkylamino (e.g., methylamino, ethylamino, n-propylamino or isopropylamino), di-C₁₋₆ alkylamino (e.g., dimethylamino, diethylamino, di-n-propylamino, diisopropylamino, methylethylamino, methyl-n-propylamino, methylisopropylamino, ethyl-n-propylamino, ethylisopropylamino or n-propylisopropylamino), CN, C₁₋₆ alkoxy (e.g., methoxy (C₁), ethoxy (C₂), propoxy (C₃), isopropoxy (C₃), butoxy (C₄), isobutoxy (C₄), sec-butoxy (C₄), tert-butoxy (C₄), pentyloxy (C₅), hexyloxy (C₆)), -S(=O)₂-(C₁₋₃ alkyl), -(C=O)(C₁₋₃ alkyl), -(C=O)O(C₁₋₃ alkyl), -(C=O)NH(C₁₋₃ alkyl) or -(C=O)N(C₁₋₃ alkyl)₂.

In some embodiments of the present disclosure, Rₛ₂ is F, Cl, Br, I, OH, NH₂, C₁₋₆ alkylamino (e.g., methylamino, ethylamino, n-propylamino or isopropylamino), di-C₁₋₆ alkylamino (e.g., dimethylamino, diethylamino, di-n-propylamino, diisopropylamino, methylethylamino, methyl-n-propylamino, methylisopropylamino, ethyl-n-propylamino, ethylisopropylamino or n-propylisopropylamino), CN or C₁₋₆ alkoxy (e.g., methoxy (C₁), ethoxy (C₂), propoxy (C₃), isopropoxy (C₃), butoxy (C₄), isobutoxy (C₄), sec-butoxy (C₄), tert-butoxy (C₄), pentyloxy (C₅), hexyloxy (C₆)).

In some embodiments of the present disclosure, Rₛ₂ is C₁₋₆ alkylamino (e.g., methylamino, ethylamino, n-propylamino or isopropylamino), di-C₁₋₆ alkylamino (e.g., dimethylamino, diethylamino, di-n-propylamino, diisopropylamino, methylethylamino, methyl-n-propylamino, methylisopropylamino, ethyl-n-propylamino, ethylisopropylamino or n-propylisopropylamino), CN, C₁₋₆ alkoxy (e.g., methoxy (C₁), ethoxy (C₂), propoxy (C₃), isopropoxy (C₃), butoxy (C₄), isobutoxy (C₄), sec-butoxy (C₄), tert-butoxy (C₄), pentyloxy (C₅), hexyloxy (C₆)), -S(=O)₂-(C₁₋₃ alkyl), -(C=O)(C₁₋₃ alkyl), -(C=O)O (C₁₋₃ alkyl), -(C=O)NH(C₁₋₃ alkyl) or -(C=O)N(C₁₋₃ alkyl)₂.

In some embodiments of the present disclosure, Rₛ₂ is F, Cl, Br, I, OH, NH₂, -S(=O)₂-(C₁₋₃ alkyl), - (C=O)(C₁₋₃ alkyl), -(C=O)O(C₁₋₃ alkyl), -(C=O)NH(C₁₋₃ alkyl) or -(C=O)N(C₁₋₃ alkyl)₂.

In some embodiments of the present disclosure, Rₛ₂ is -(C=O)(C₁₋₃ alkyl), -(C=O)O(C₁₋₃ alkyl), - (C=O)NH(C₁₋₃ alkyl) or -(C=O)N(C₁₋₃ alkyl)₂.

The present disclosure provides a compound represented by formula (I) or a pharmaceutically acceptable salt thereof, 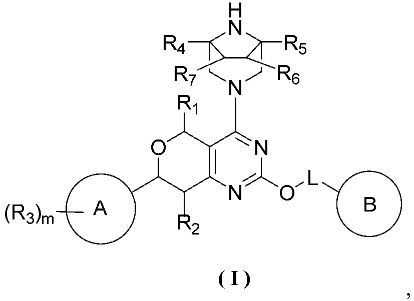 wherein,
Ring A is selected from C₆₋₁₀ aryl and 5- to 10-membered heteroaryl;
Ring B is selected from 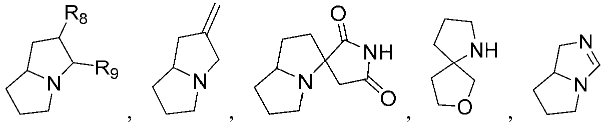 and 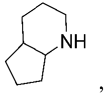 wherein the 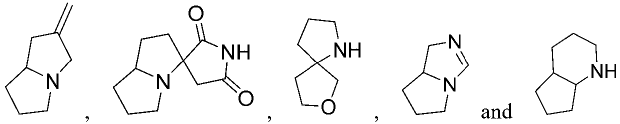 are each independently optionally substituted with 1, 2, 3 or 4 R₁₀;
L is -CH₂-, wherein the -CH₂- is optionally substituted with 1 or 2 D;
R₁ and R₂ are each independently selected from H, F, Cl, Br, I, C₁₋₃ alkyl and C₃₋₅ cycloalkyl, wherein the C₁₋₃ alkyl and C₃₋₅ cycloalkyl are each independently optionally substituted with 1, 2 or 3 Rₐ;
each R₃ is independently selected from F, Cl, Br, I, OH, NH₂, CN, C₁₋₃ alkyl, C₁₋₃ alkoxy, C₁₋₃ alkylamino, C₂₋₄ alkenyl, C₂₋₄ alkynyl and C₃₋₅ cycloalkyl, wherein the C₁₋₃ alkyl, C₁₋₃ alkoxy, C₁₋₃ alkylamino, C₂₋₄ alkenyl, C₂₋₄ alkynyl and C₃₋₅ cycloalkyl are each independently optionally substituted with 1, 2, 3, 4 or 5 Rₐ;
R₄, R₅, R₆ and R₇ are each independently selected from H, OH, NH₂, CN, C₁₋₃ alkyl and C₁₋₃ alkoxy, wherein the C₁₋₃ alkyl and C₁₋₃ alkoxy are each independently optionally substituted with 1, 2, 3, 4 or 5 R_{b};
R₈ is selected from H, F, Cl, Br, I, OH, NH₂, CN, C₁₋₃ alkyl and C₁₋₃ alkoxy, wherein the C₁₋₃ alkyl and C₁₋₃ alkoxy are each independently optionally substituted with 1, 2, 3, 4 or 5 Rₐ;
R₉ is selected from -C(=O)-NR_{b1}R_{b2} and -CH₂R_{c};
each R₁₀ is independently selected from D, F, Cl, Br, I, OH, NH₂, C₁₋₃ alkyl, C₁₋₃ alkylamino, and - NH-C(=O)-R_{d};
each Rₐ is independently selected from D, F, Cl, Br and I;
each R_{b} is independently selected from D, F, Cl, Br, I, OH, NH₂, CN and -C(=O)-NR_{b1}R_{b2};
R_{b1} and R_{b2} are each independently selected from H and C₁₋₃ alkyl;
R_{c} is selected from F, Cl, Br, I, OH and NH₂;
R_{d} is C₁₋₃ alkyl;
m is selected from 0, 1, 2, 3, 4 and 5.

In some embodiments of the present disclosure, the above L is selected from -CH₂- and -CD₂-, and other variables are as defined in the present disclosure.

In some embodiments of the present disclosure, each Rₐ mentioned above is independently selected from D, F and Cl, and other variables are as defined in the present disclosure.

In some embodiments of the present disclosure, each R_{b} mentioned above is independently selected from OH, CN and -C(=O)-NH₂, and other variables are as defined in the present disclosure.

In some embodiments of the present disclosure, R_{b1} and R_{b2} mentioned above are each independently selected from H and CH₃, and other variables are as defined in the present disclosure.

In some embodiments of the present disclosure, the above R_{c} is OH, and other variables are as defined in the present disclosure.

In some embodiments of the present disclosure, the above R_{d} is CH₃, and other variables are as defined in the present disclosure.

In some embodiments of the present disclosure, the above R₁ is H, and the other variables are as defined in the present disclosure.

In some embodiments of the present disclosure, the above R₂ is H, and the other variables are as defined in the present disclosure.

In some embodiments of the present disclosure, each R₃ mentioned above is independently selected from F, Cl, Br, I, OH, NH₂, CN, CH₃, CH₂CH₃, OCH₃, OCH₂CH₃, NHCH₃, N(CH₃)₂, -C≡CH, -C≡CCH₃, cyclopropyl and cyclobutyl; wherein the CH₃, CH₂CH₃, OCH₃, OCH₂CH₃, NHCH₃, N(CH₃)₂, -C≡CH, -C≡CCH₃, cyclopropyl and cyclobutyl are each independently optionally substituted with 1, 2, 3, 4 or 5 Rₐ; and other variables are as defined herein.

In some embodiments of the present disclosure, the present disclosure provides a compound represented by formula (I-a) or a pharmaceutically acceptable salt thereof, 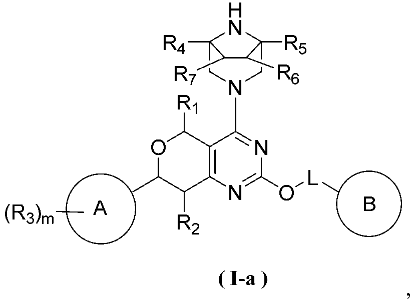 wherein,
Ring A is selected from C₆ aryl and 5-membered heteroaryl;
Ring B is 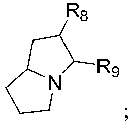 and
other variables in formula (I-a) are as defined in formula (I).

In some embodiments of the present disclosure, in the compound represented by the above formula (I-a) or a pharmaceutically acceptable salt thereof, ring A is C₆ aryl.

In some embodiments of the present disclosure, in the compound represented by the above formula (I-a) or a pharmaceutically acceptable salt thereof, ring A is a 5-membered heteroaryl group.

In some embodiments of the present disclosure, the present disclosure provides a compound represented by formula (I-b) or a pharmaceutically acceptable salt thereof, 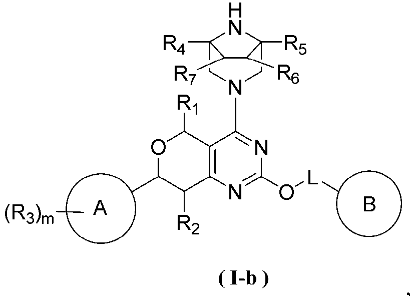 wherein,
Ring A is selected from C₆ aryl and 5-membered heteroaryl;
Ring B is selected from 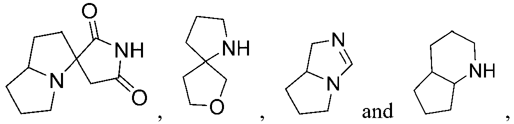 wherein the 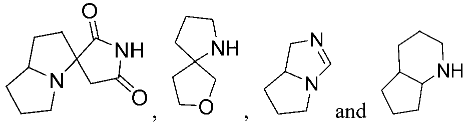 are each independently optionally substituted with 1, 2, 3 or 4 R₁₀; and
other variables in formula (I-b) are as defined in formula (I).

In some embodiments of the present disclosure, in the compound represented by the above formula (I-b) or a pharmaceutically acceptable salt thereof, ring A is C₆ aryl.

In some embodiments of the present disclosure, in the compound represented by the above formula (I-b) or a pharmaceutically acceptable salt thereof, ring A is a 5-membered heteroaryl group.

In some embodiments of the present disclosure, each R₃ is independently selected from F, OH, NH₂, CF₃, OCH₃, -C≡CCH₃, -C≡CCH₂F, -C≡CCHF₂, -C≡CCF₃, -C≡CCD₃, -C≡CCl, -C≡CF and cyclopropyl, and other variables are as defined in the present disclosure.

In some embodiments of the present disclosure, the above ring A is phenyl, and other variables are as defined herein.

In some embodiments of the present disclosure, the above structural moiety 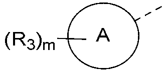 is selected from 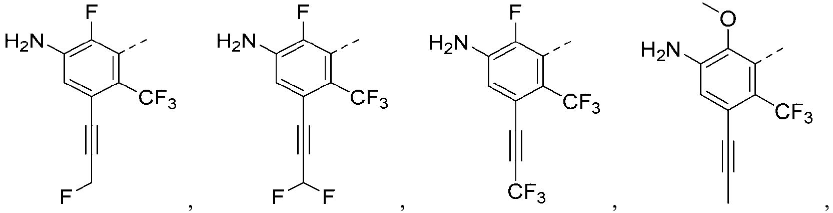 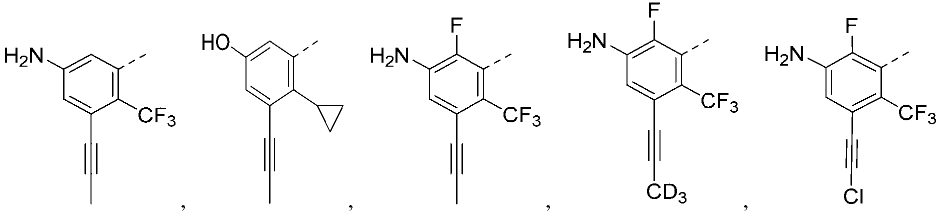 and 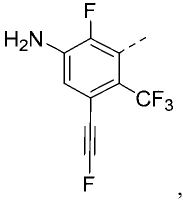 and other variables are as defined in the present disclosure.

In some embodiments of the present disclosure, the above R₅ is selected from H, OH, NH₂, CN, CH₃, CH₂CH₃, OCH₃ and OCH₂CH₃, wherein the CH₃, CH₂CH₃, OCH₃ and OCH₂CH₃ are each independently optionally substituted with 1, 2, 3, 4 or 5 R_{b}, and other variables are as defined herein.

In some embodiments of the present disclosure, the above R₅ is selected from H, CH₃, CH₂CN, CH₂OH and CH₂CONH₂, and other variables are as defined in the present disclosure.

In some embodiments of the present disclosure, the above R₆ is selected from H and OH, and the other variables are as defined in the present disclosure.

In some embodiments of the present disclosure, the above R₄ is H, and the other variables are as defined in the present disclosure.

In some embodiments of the present disclosure, the above R₇ is H, and the other variables are as defined in the present disclosure.

In some embodiments of the present disclosure, the above R₈ is selected from H and F, and the other variables are as defined in the present disclosure.

In some embodiments of the present disclosure, R₉ is selected from -C(=O)-NH₂, -C(=O)-NHCH₃, - C(=O)-N(CH₃)₂ and -CH₂OH, and other variables are as defined in the present disclosure.

In some embodiments of the present disclosure, each R₁₀ is independently selected from D, F, NH₂, CH₃, -N(CH₃)₂ and -NH-C(=O)-CH₃, and other variables are as defined in the present disclosure.

In some embodiments of the present disclosure, the above ring B is selected from 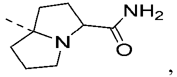 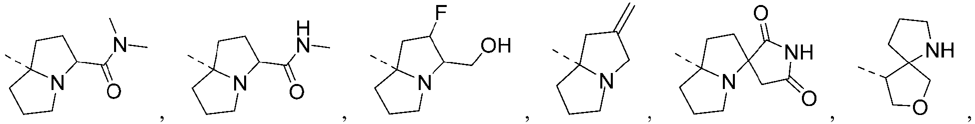 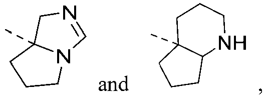 wherein the 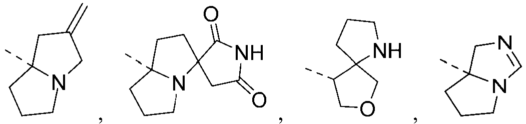 and 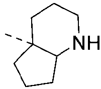 are each independently optionally substituted with 1, 2, 3 or 4 R₁₀, and other variables are as defined in the present disclosure.

In some embodiments of the present disclosure, the above ring B is selected from 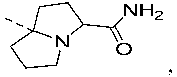 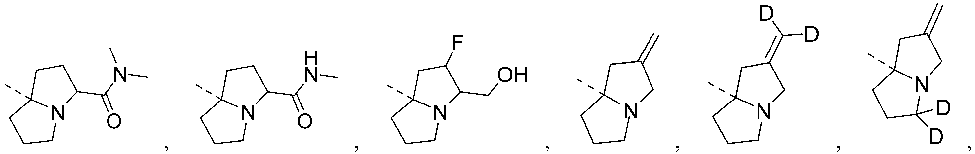 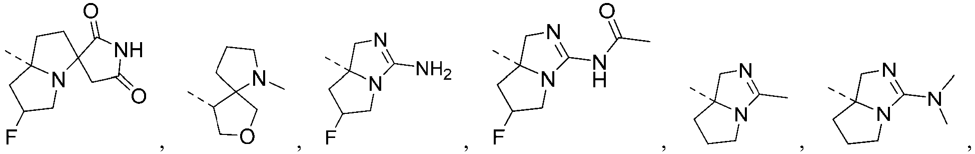 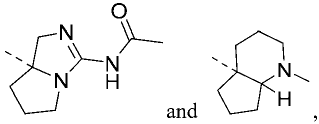 and other variables are as defined in the present disclosure.

The present disclosure also includes some embodiments derived from any combination of the above variables..

The present disclosure provides the compounds shown in Table 1 or pharmaceutically acceptable salts thereof.

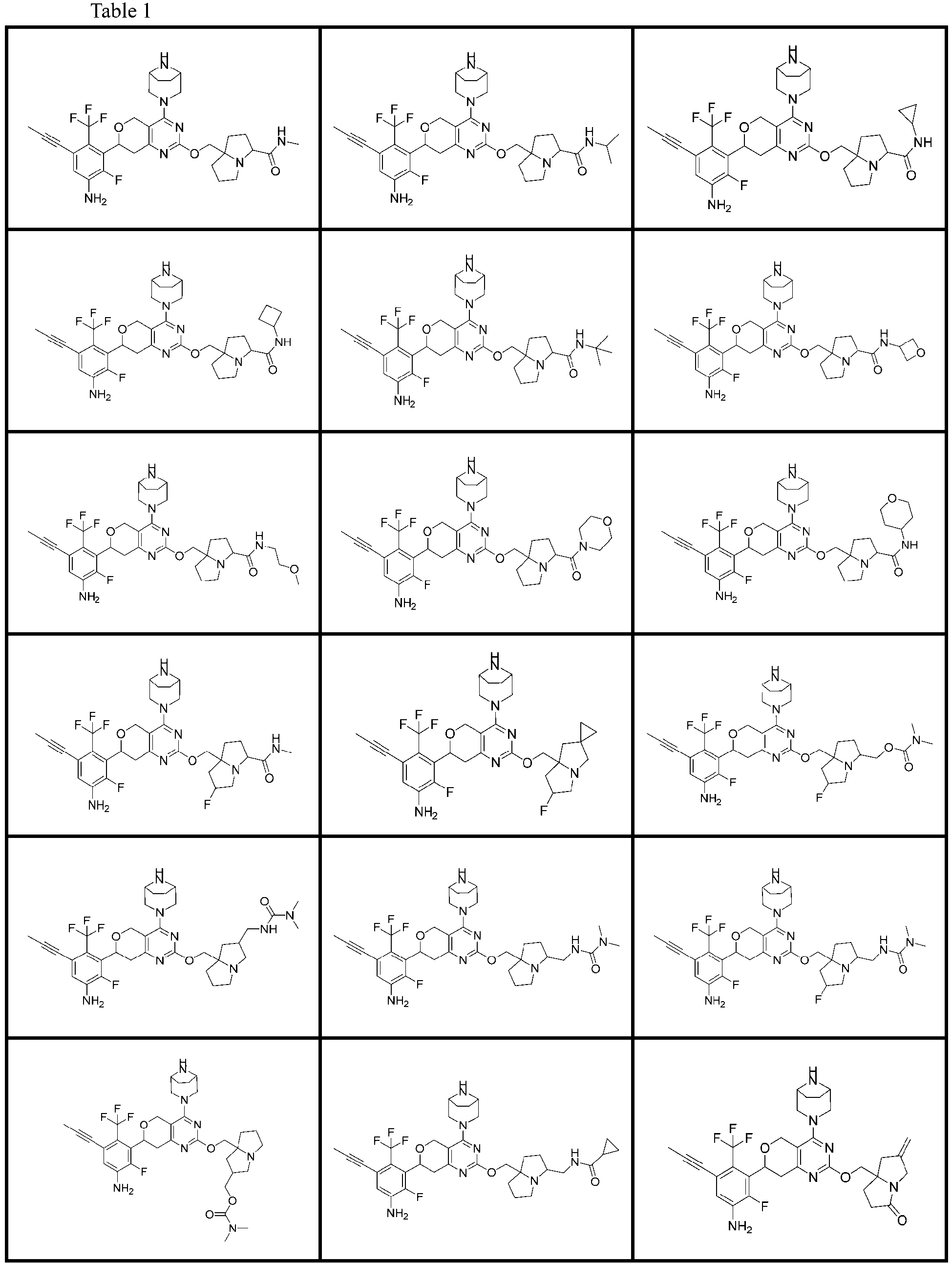

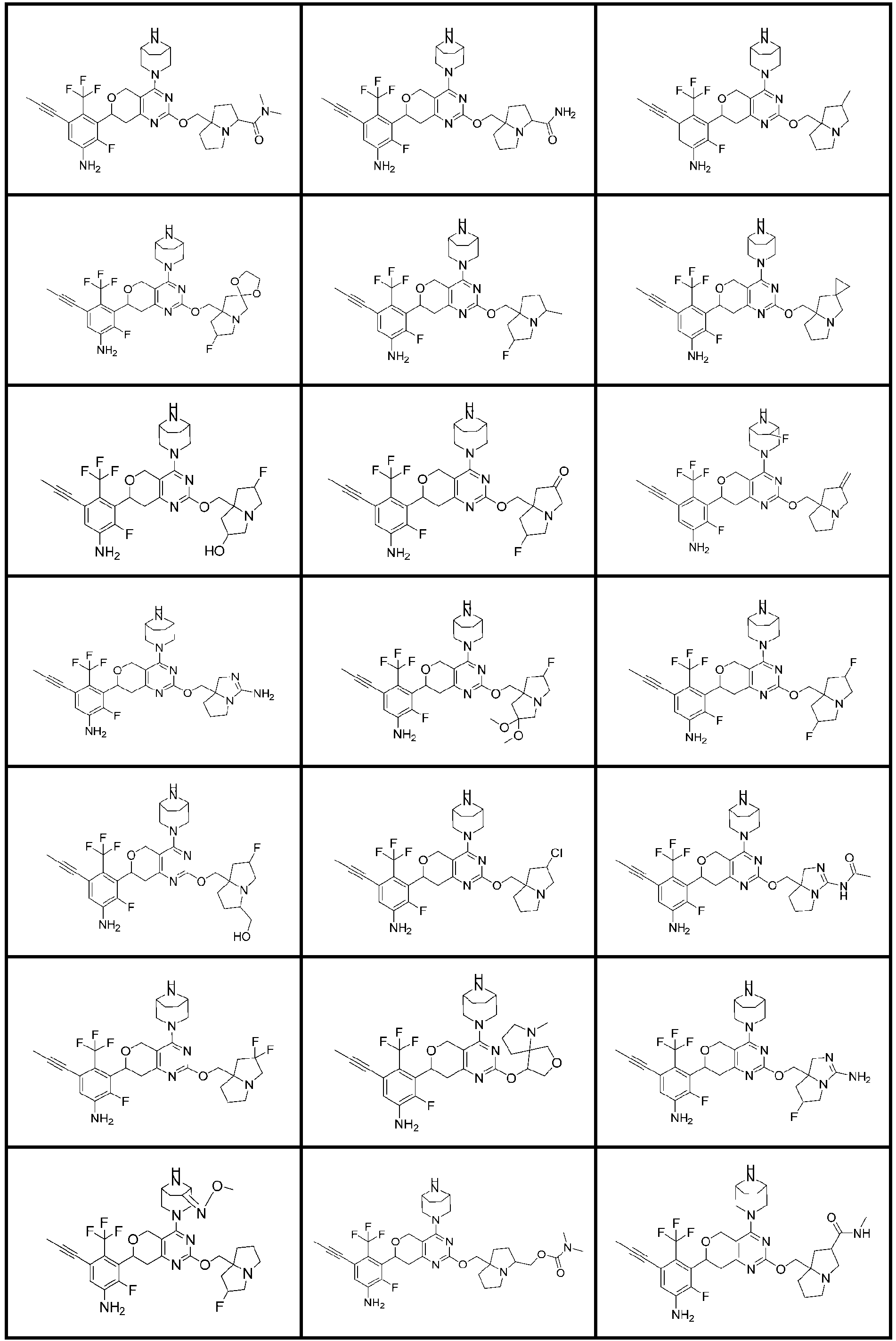

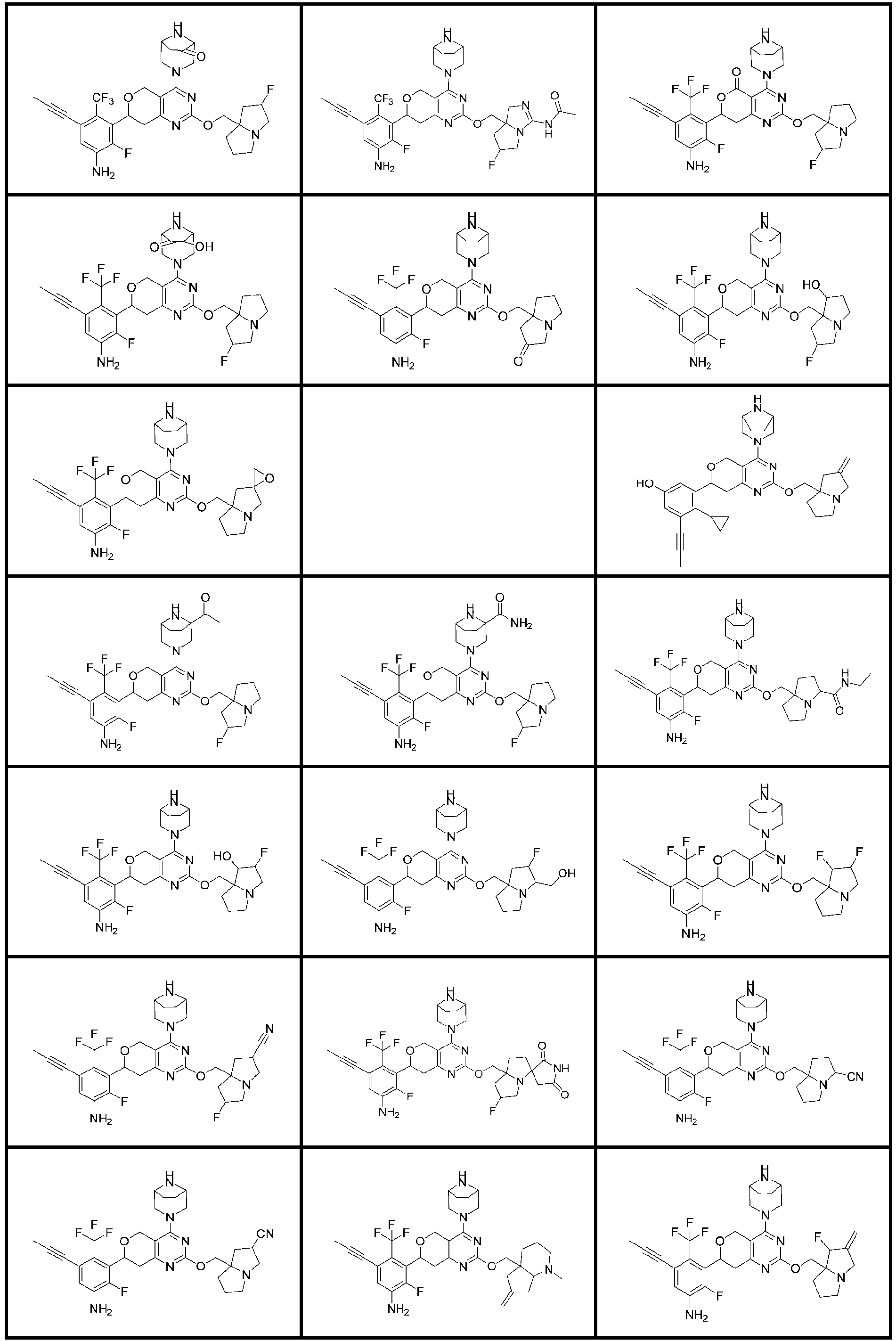

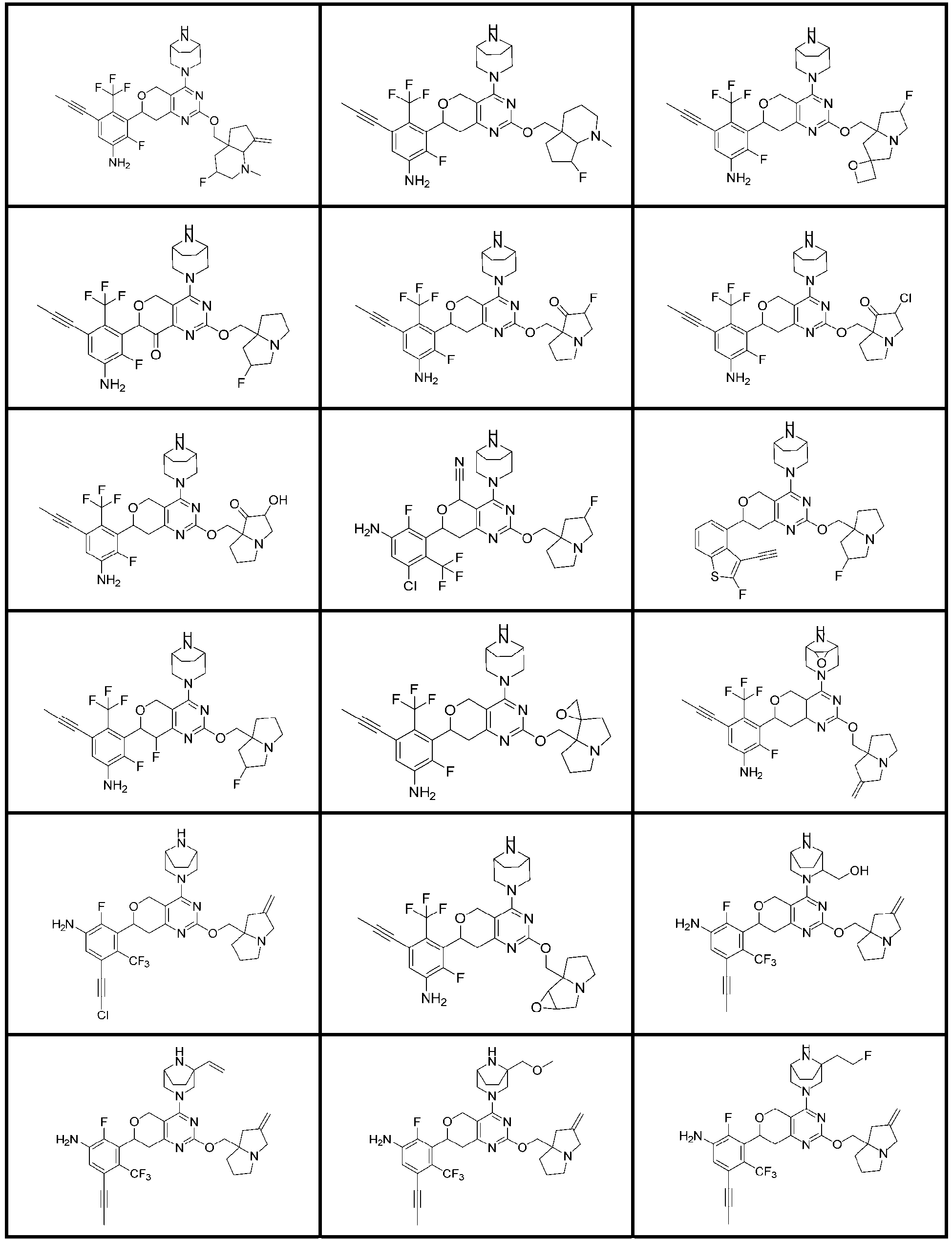

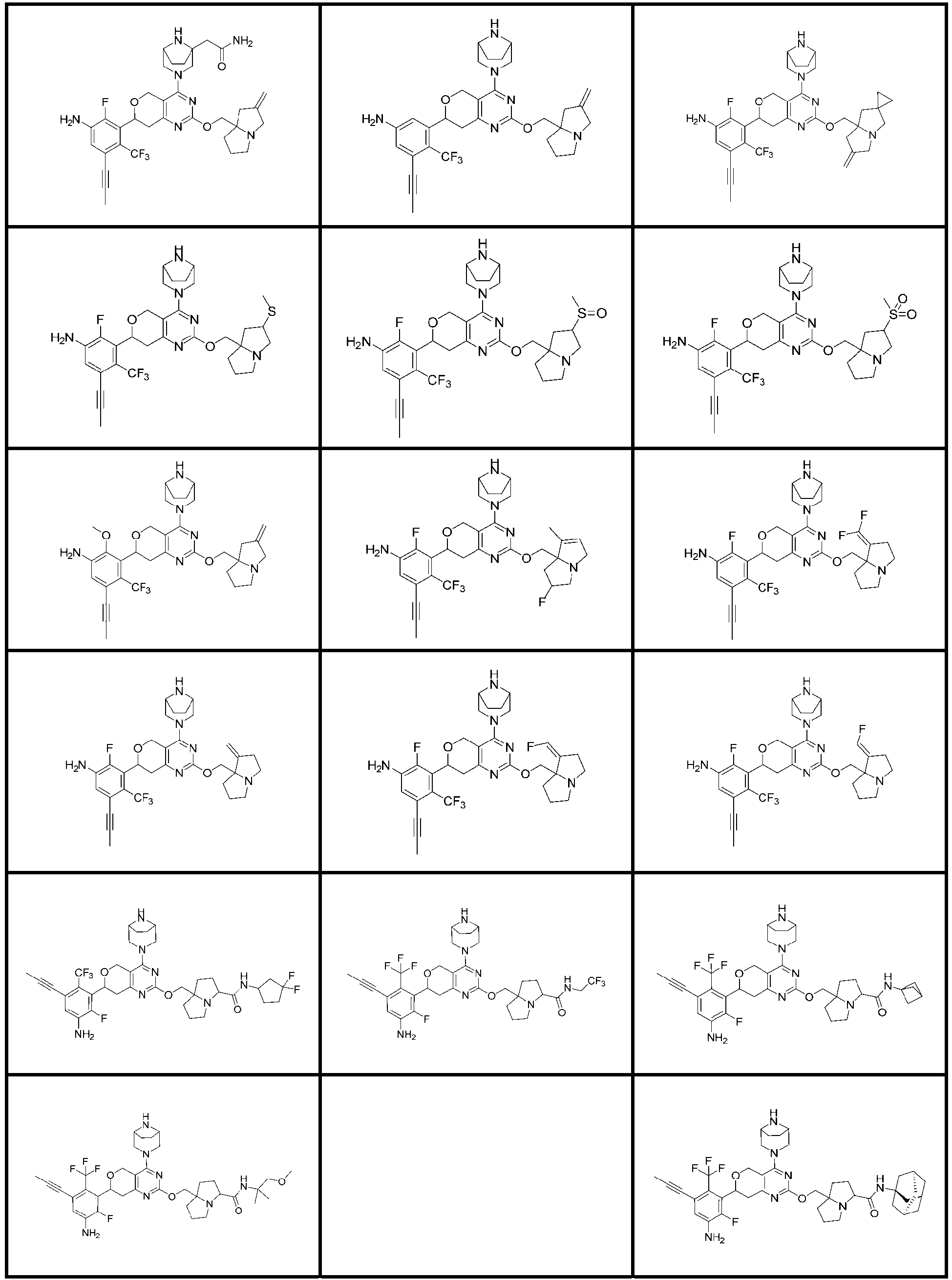

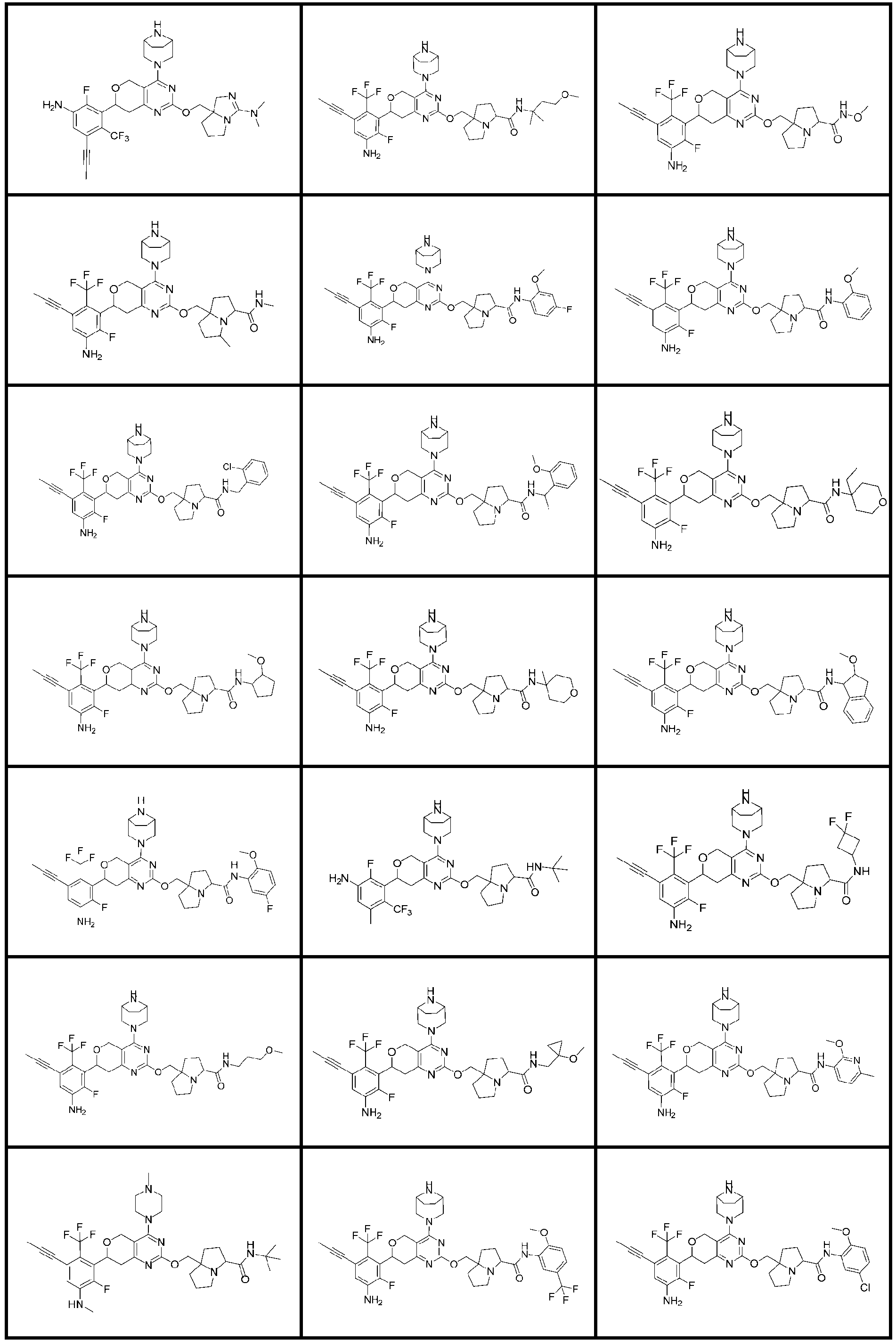

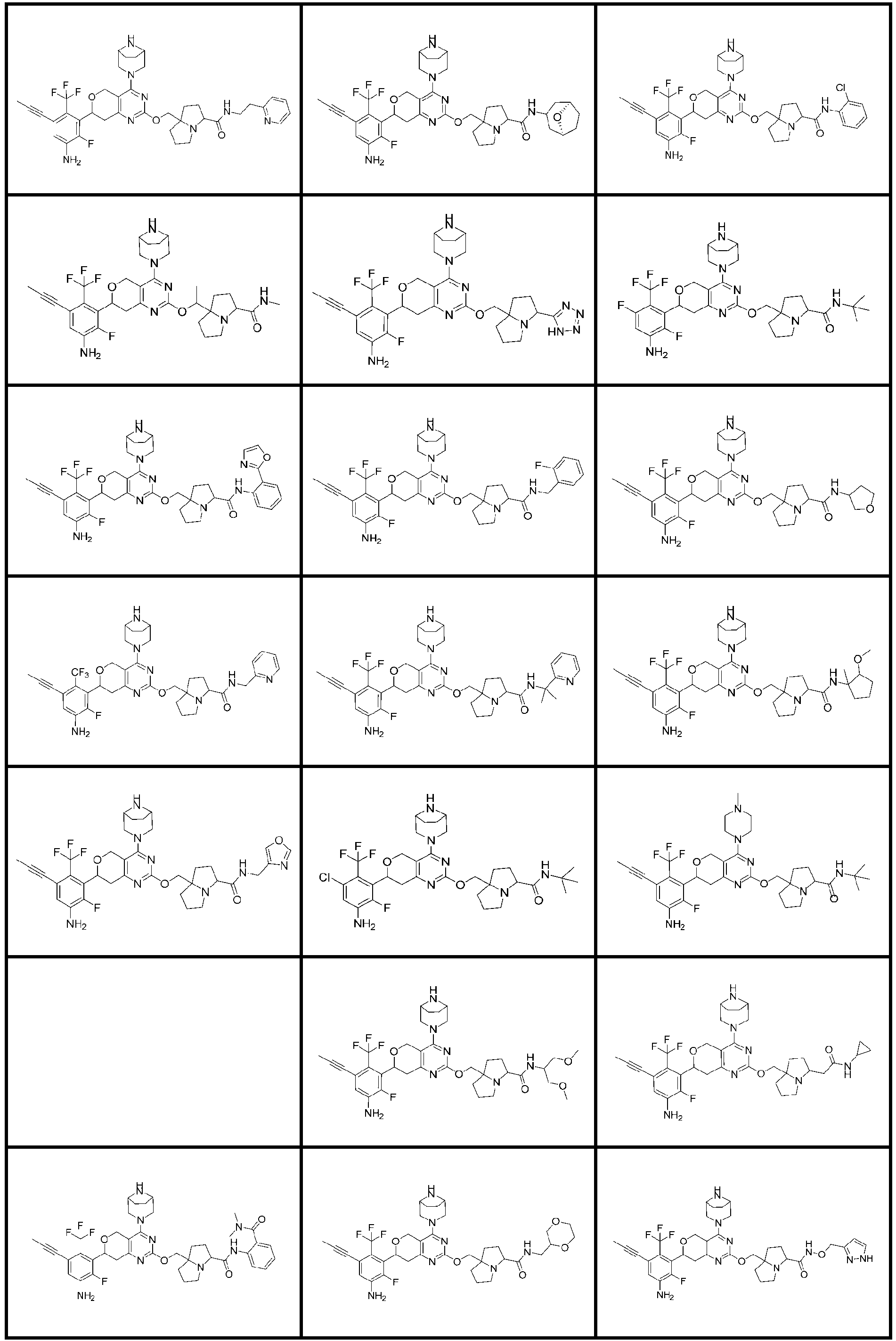

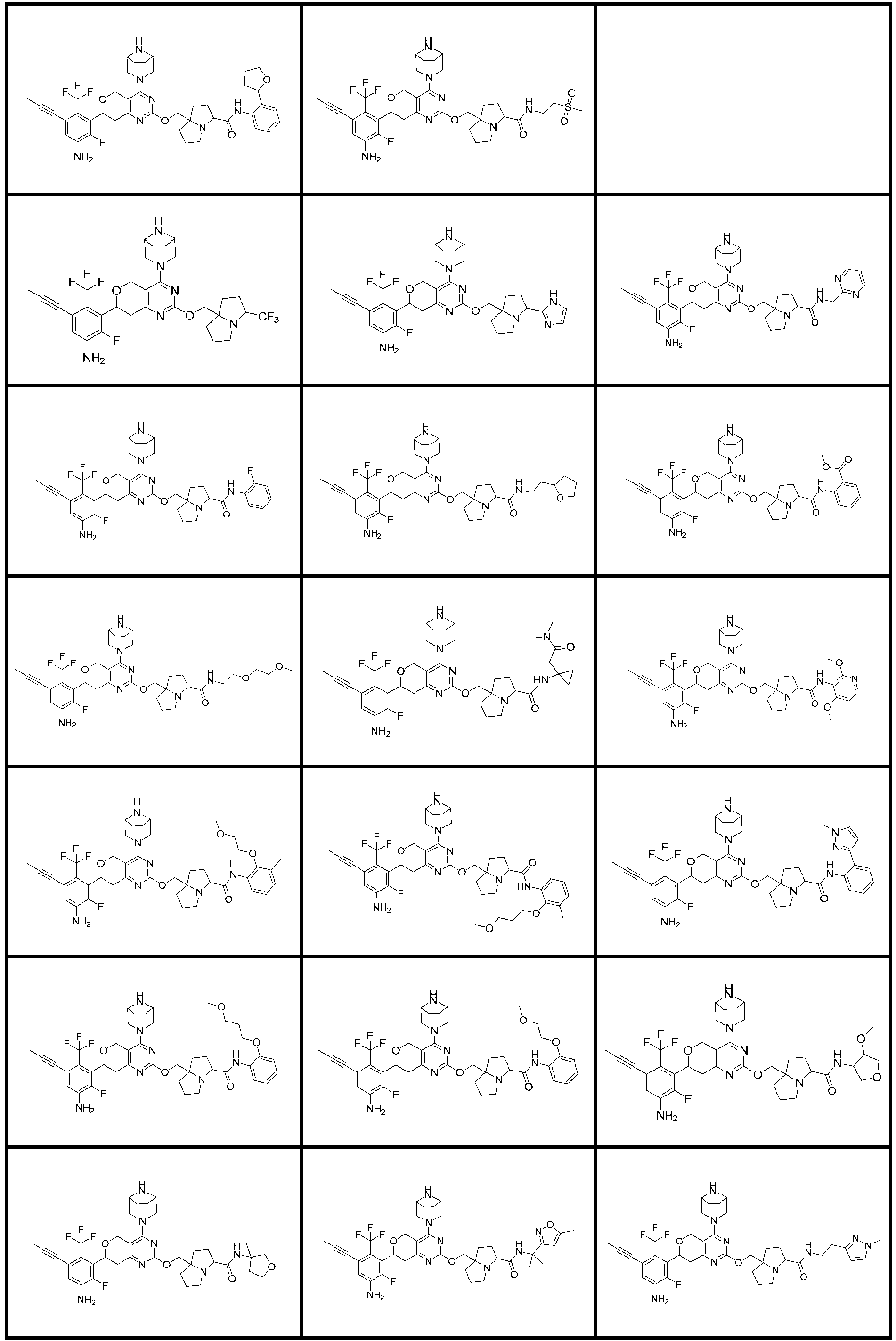

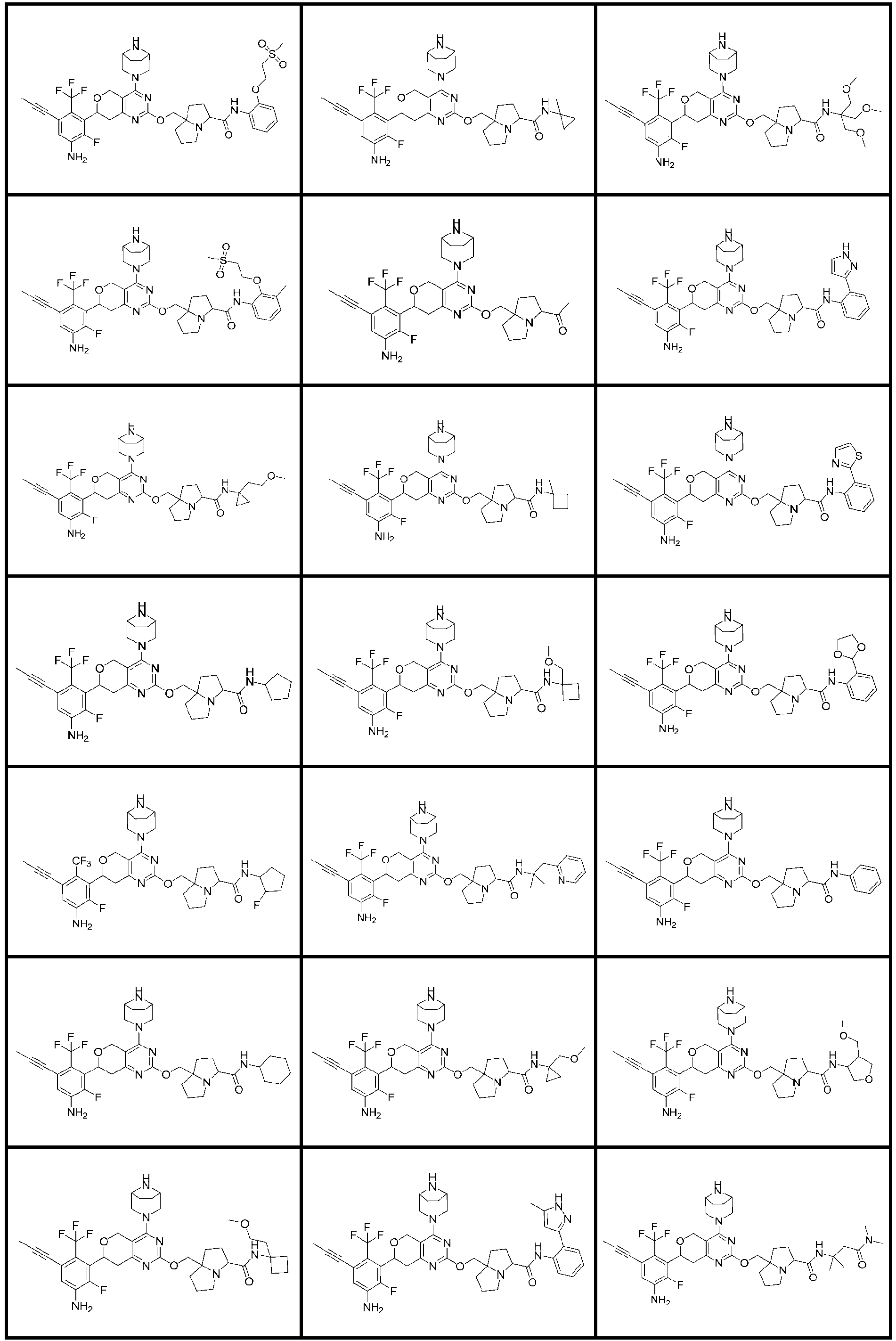

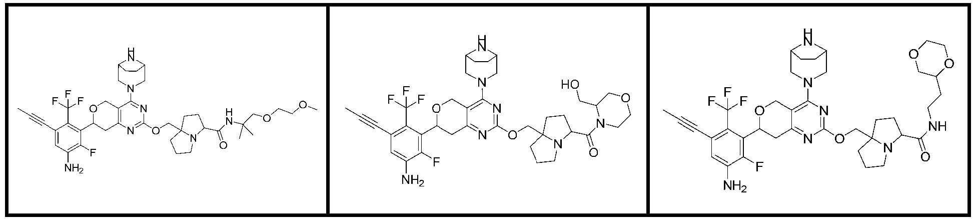

The present disclosure provides the compounds shown in Table 2 or pharmaceutically acceptable salts thereof.

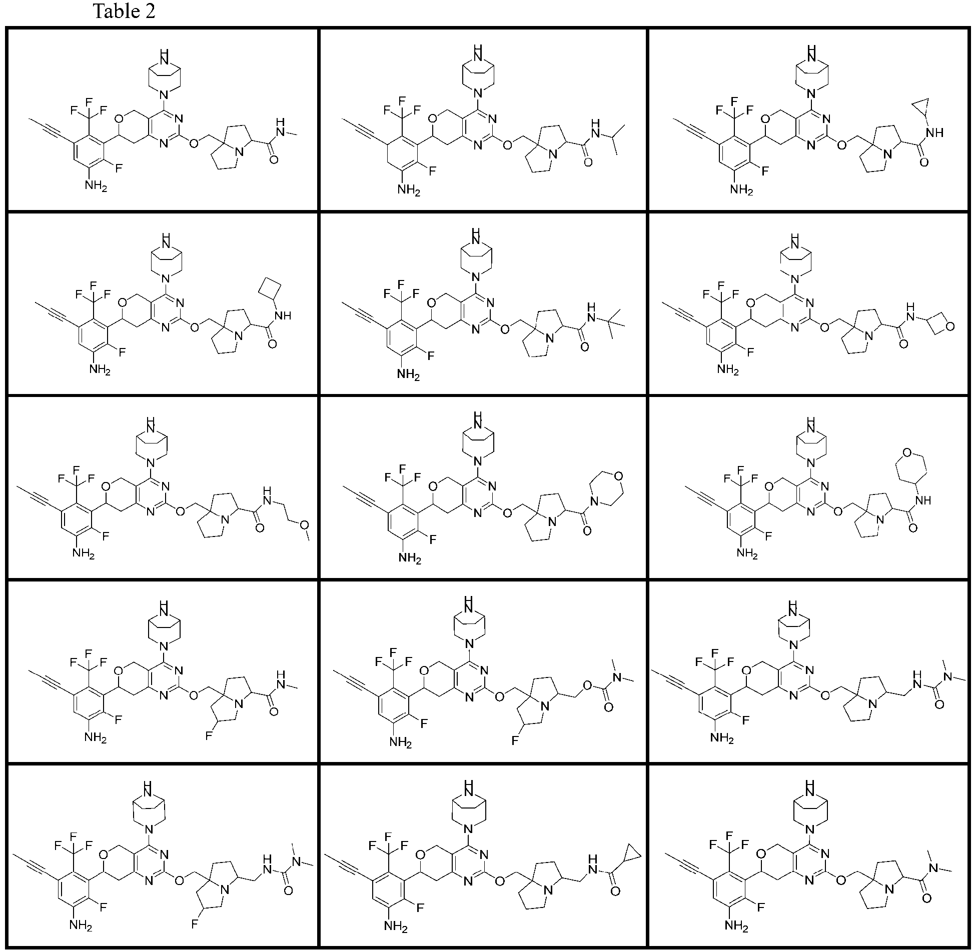

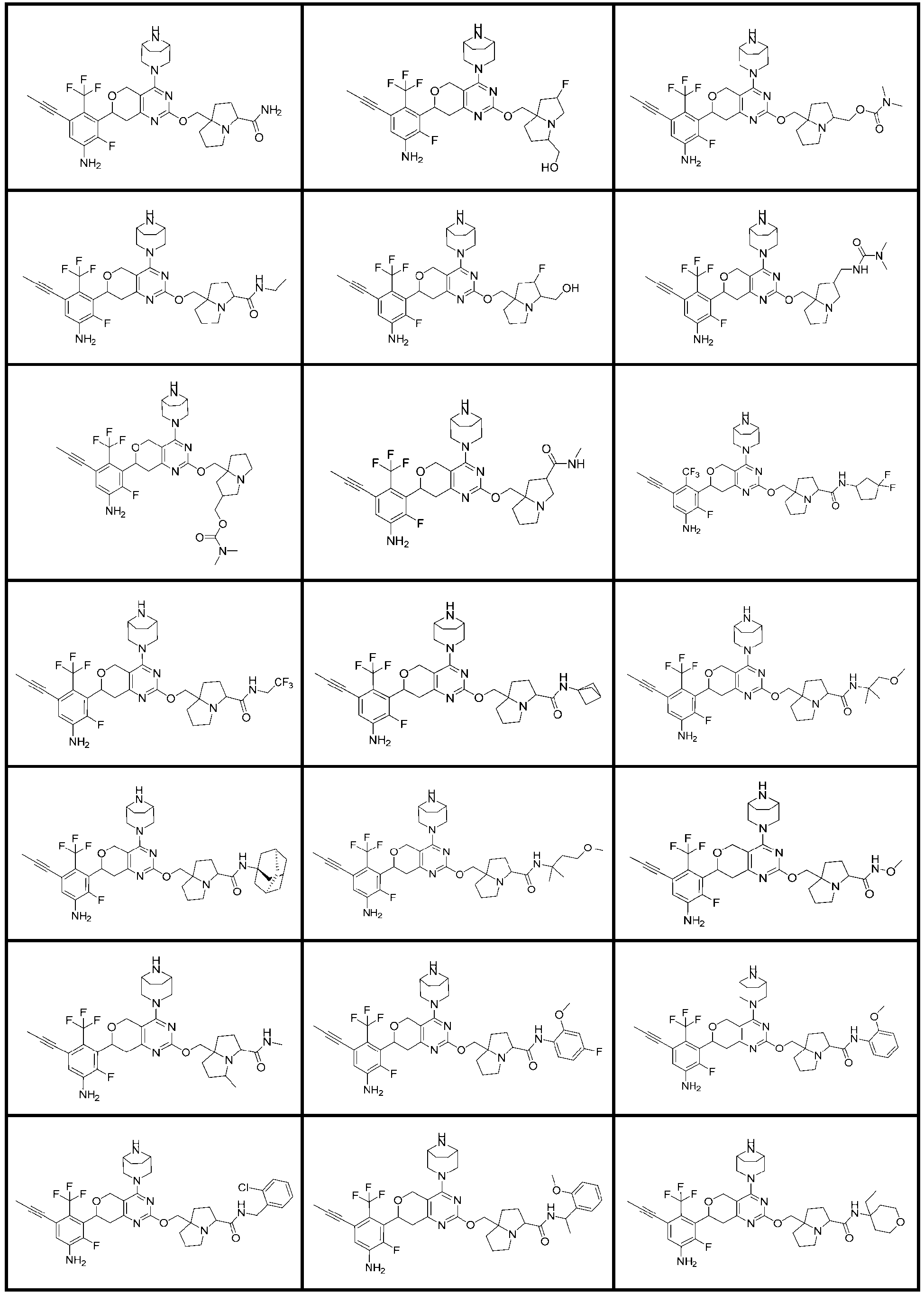

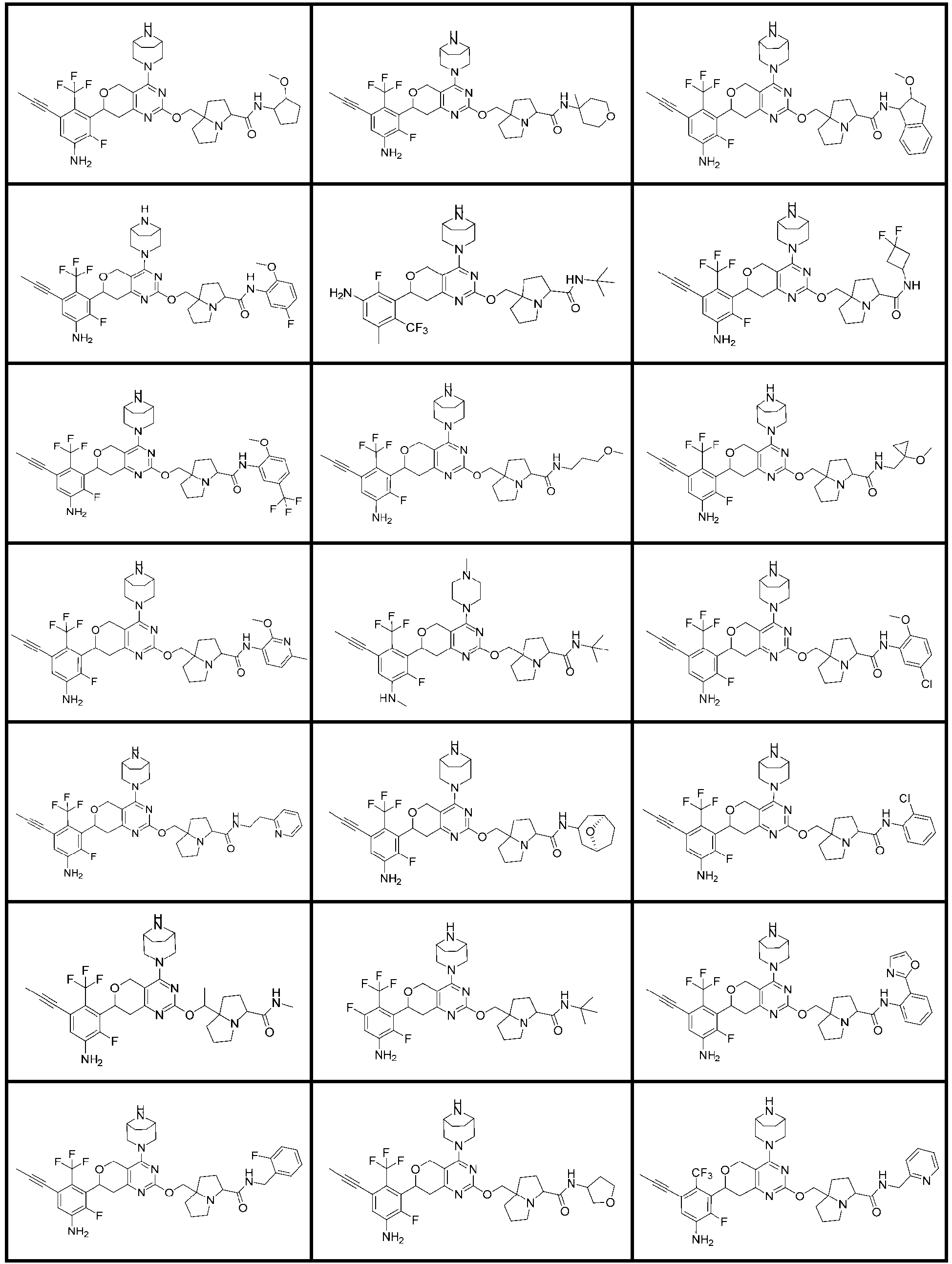

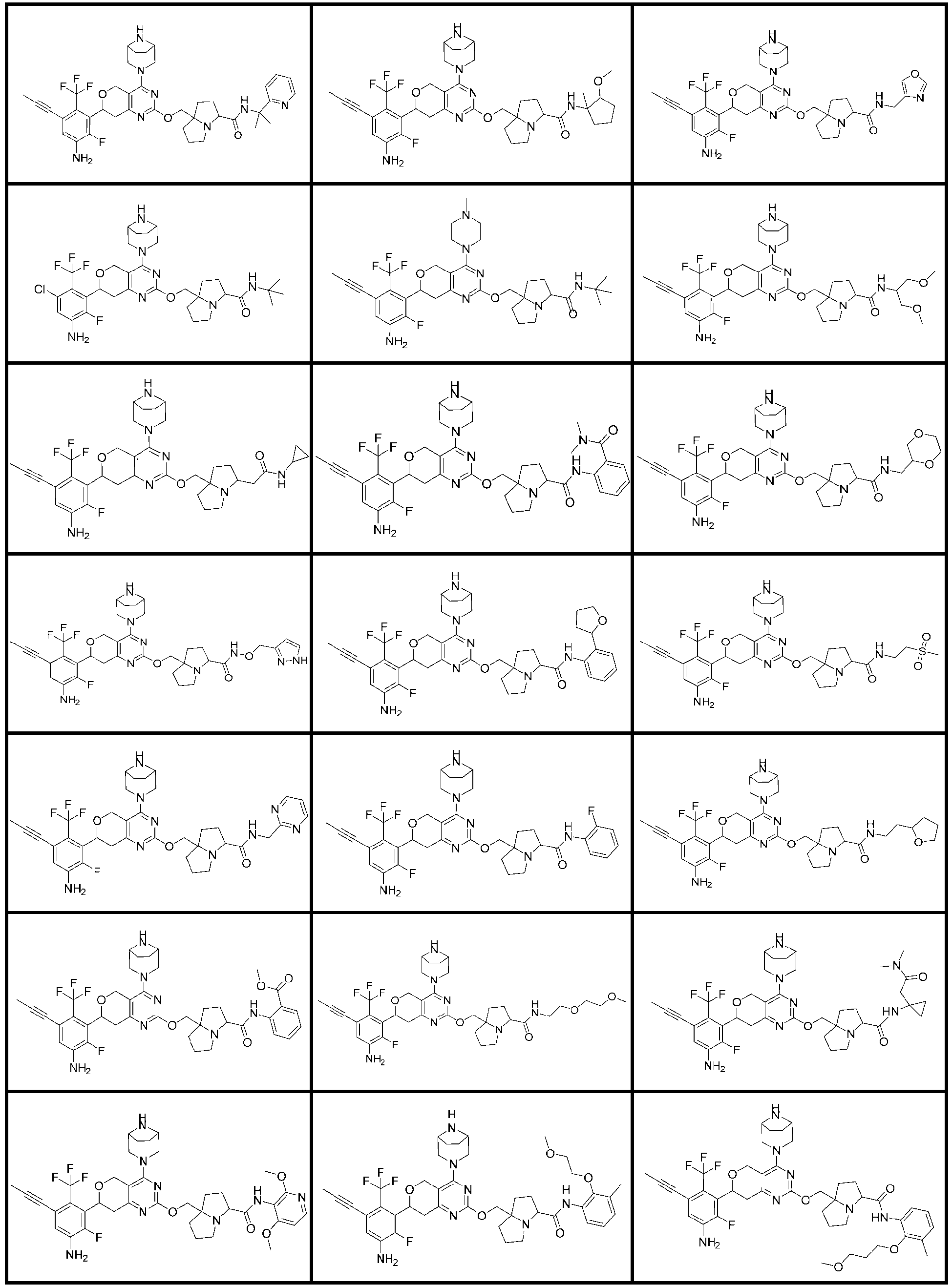

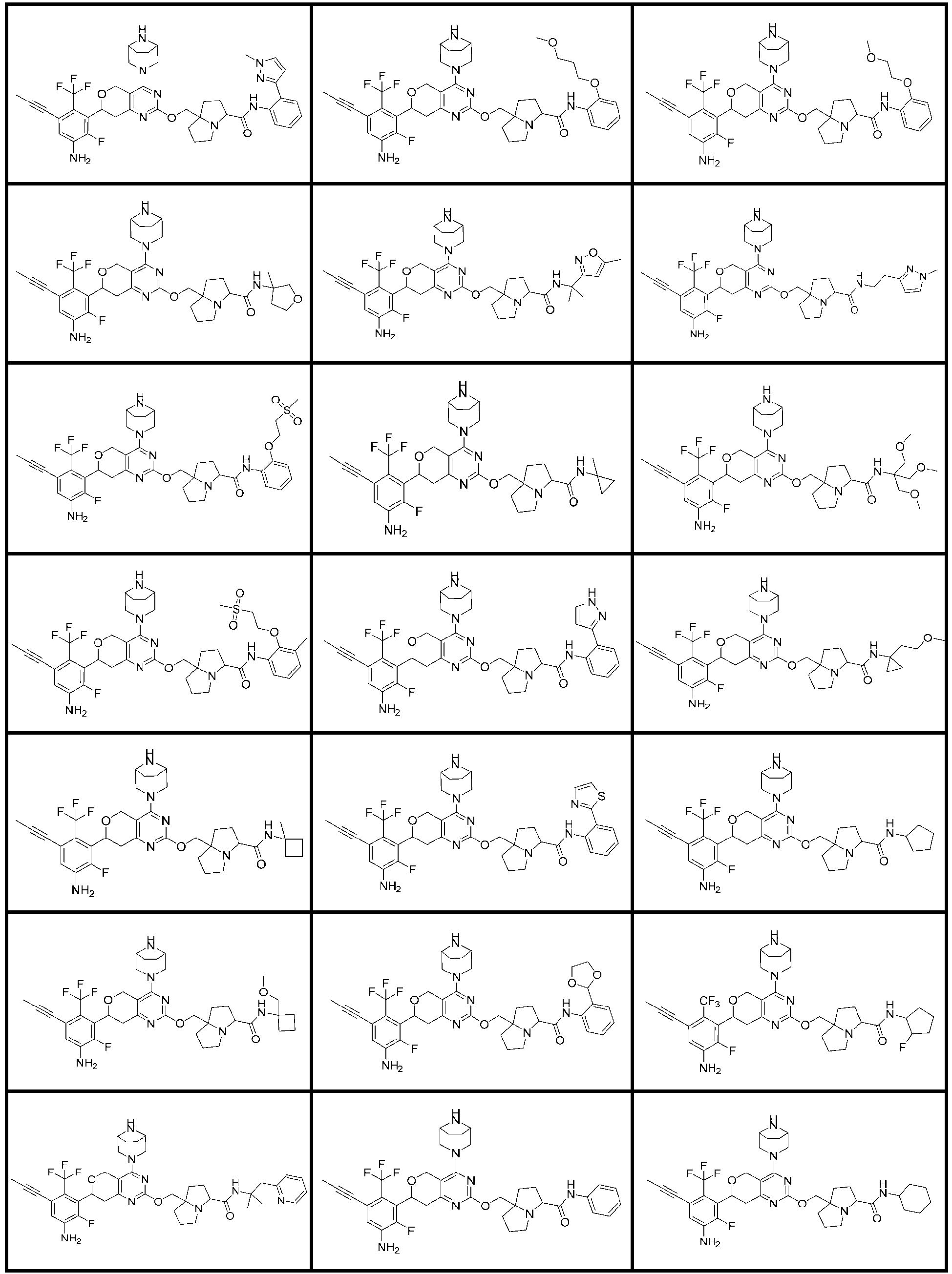

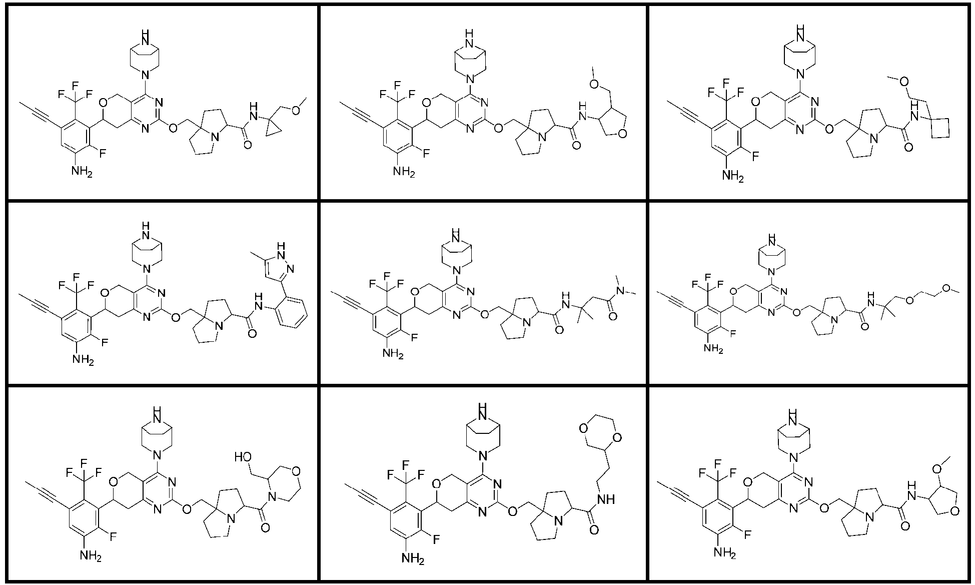

The present disclosure provides a compound as shown in Table 2a or a pharmaceutically acceptable salt thereof,

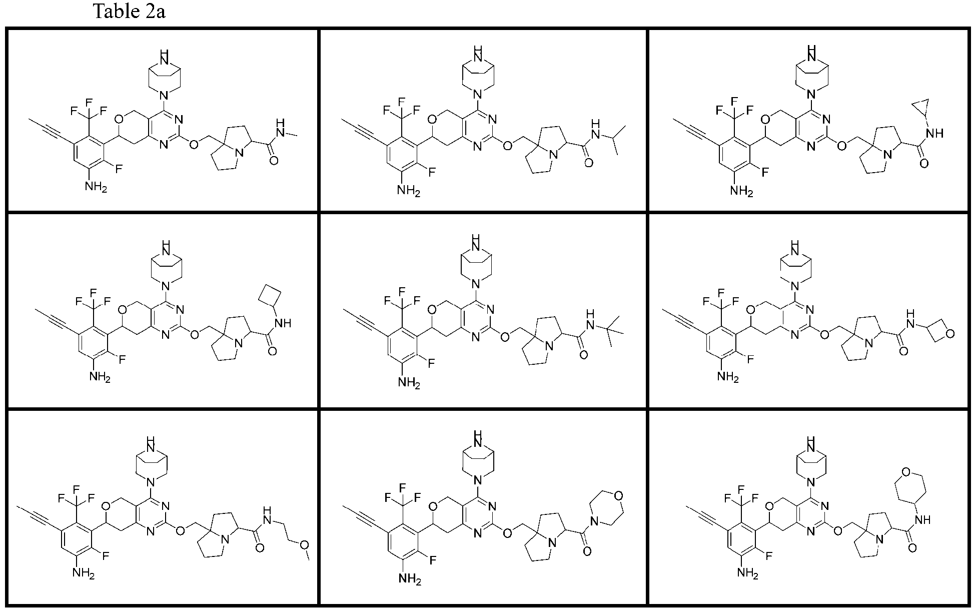

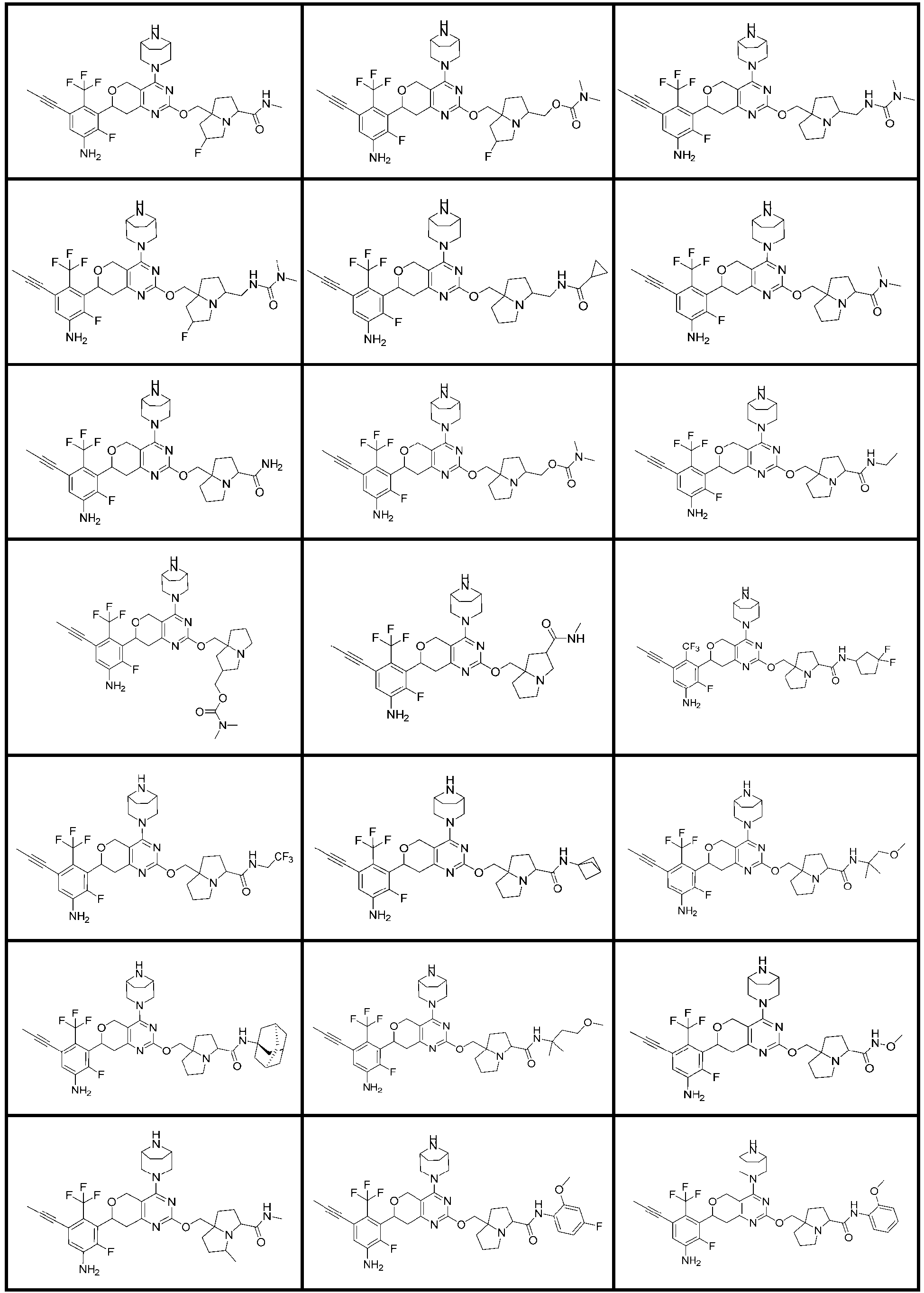

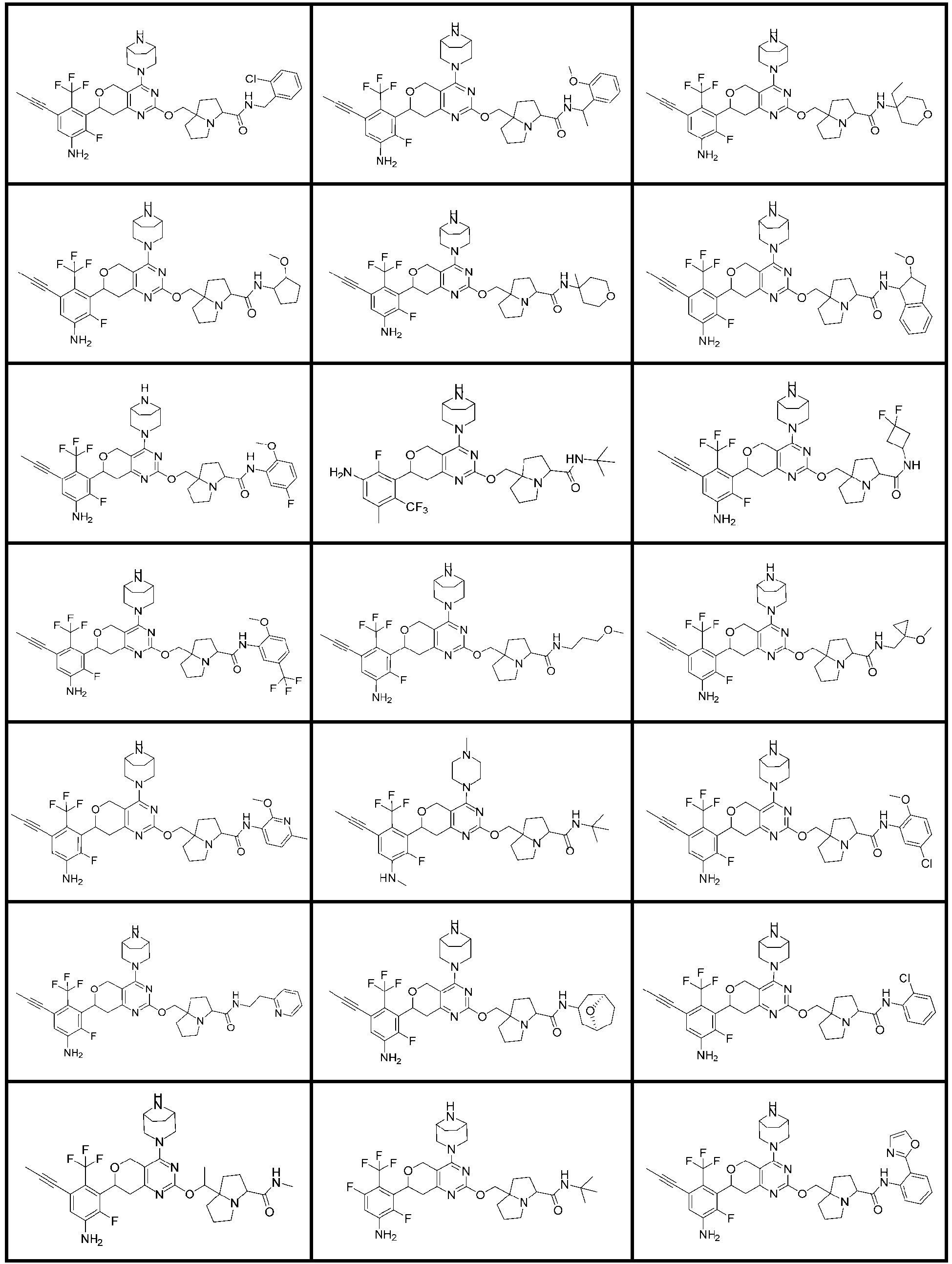

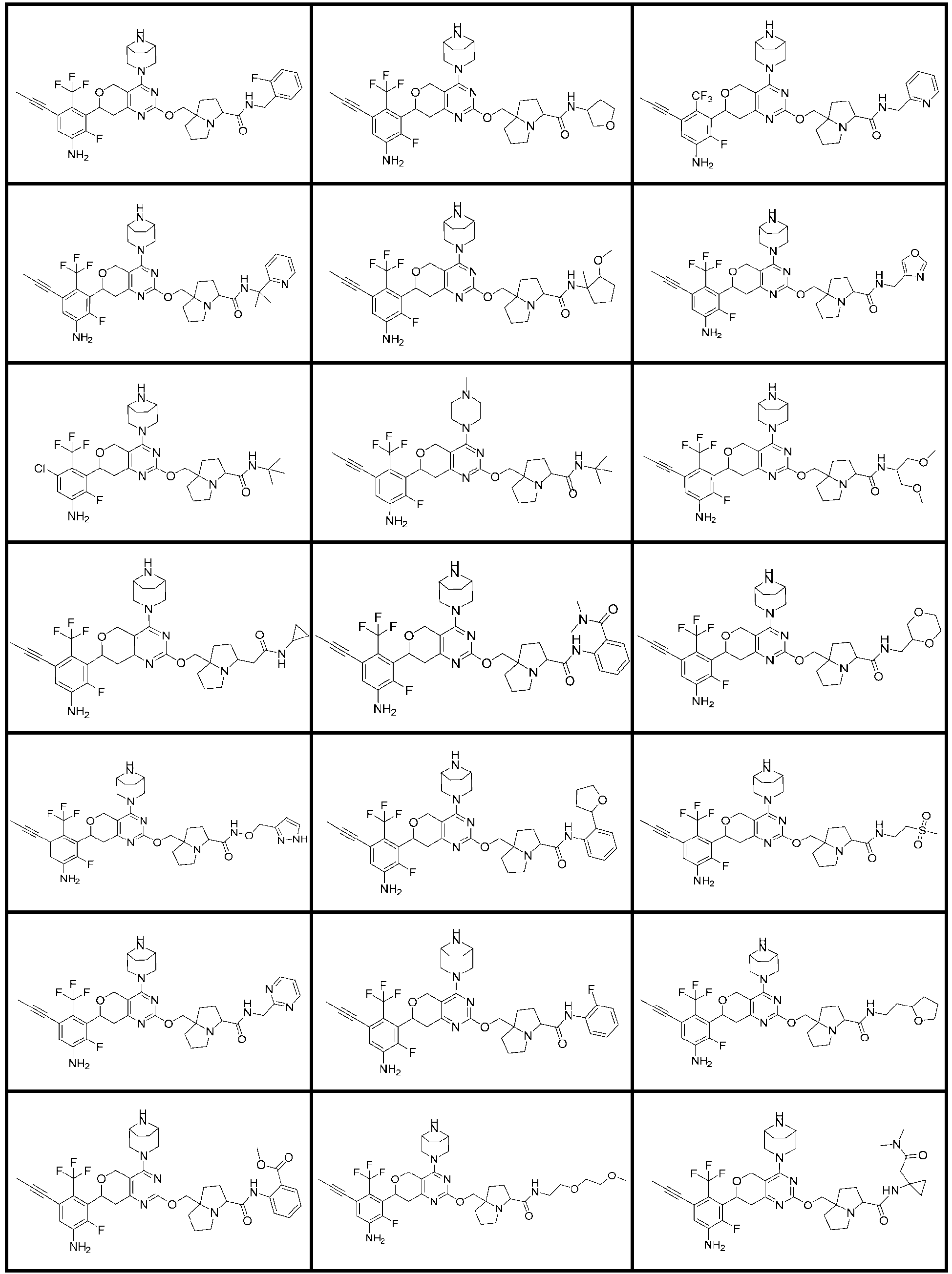

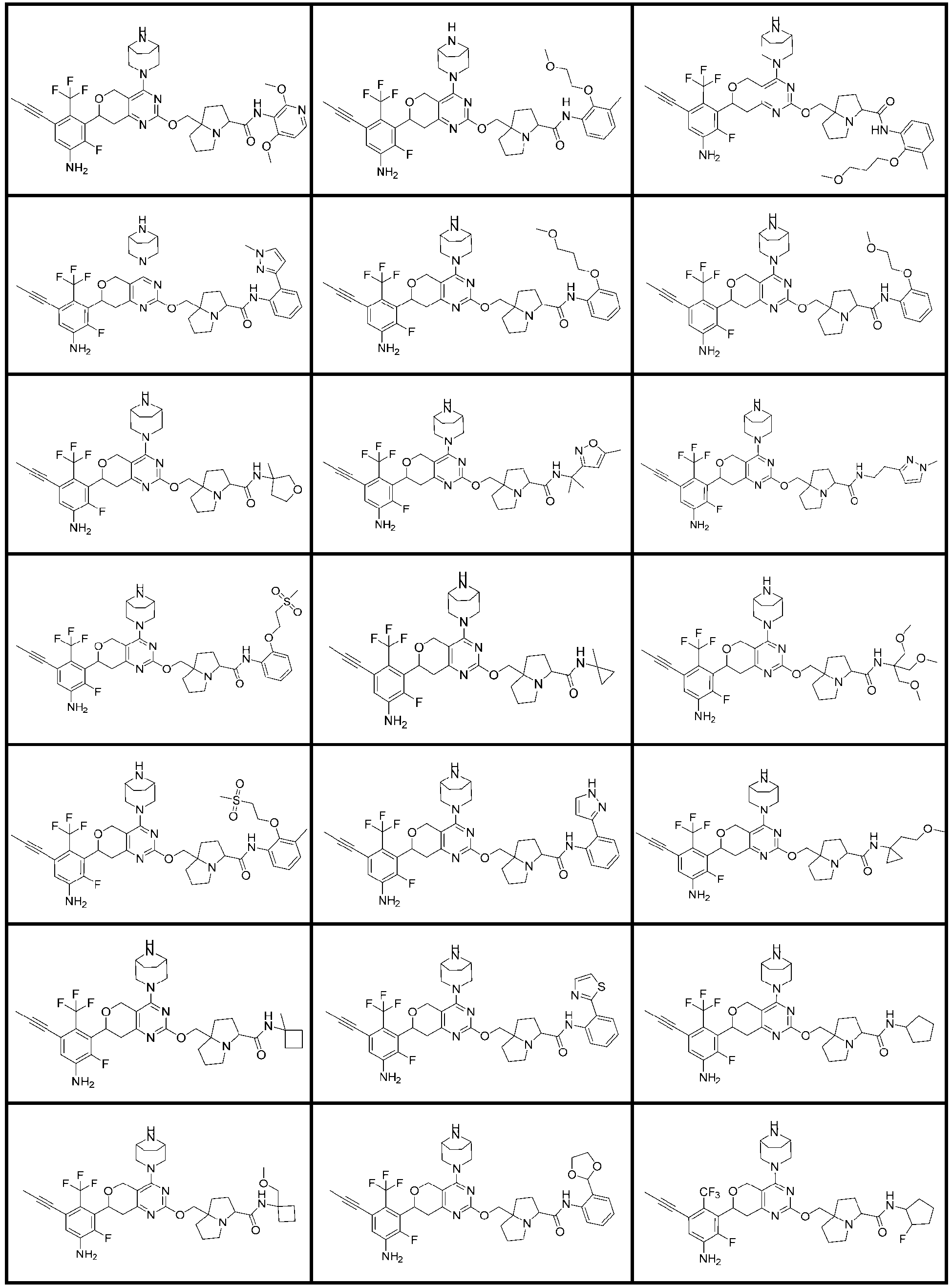

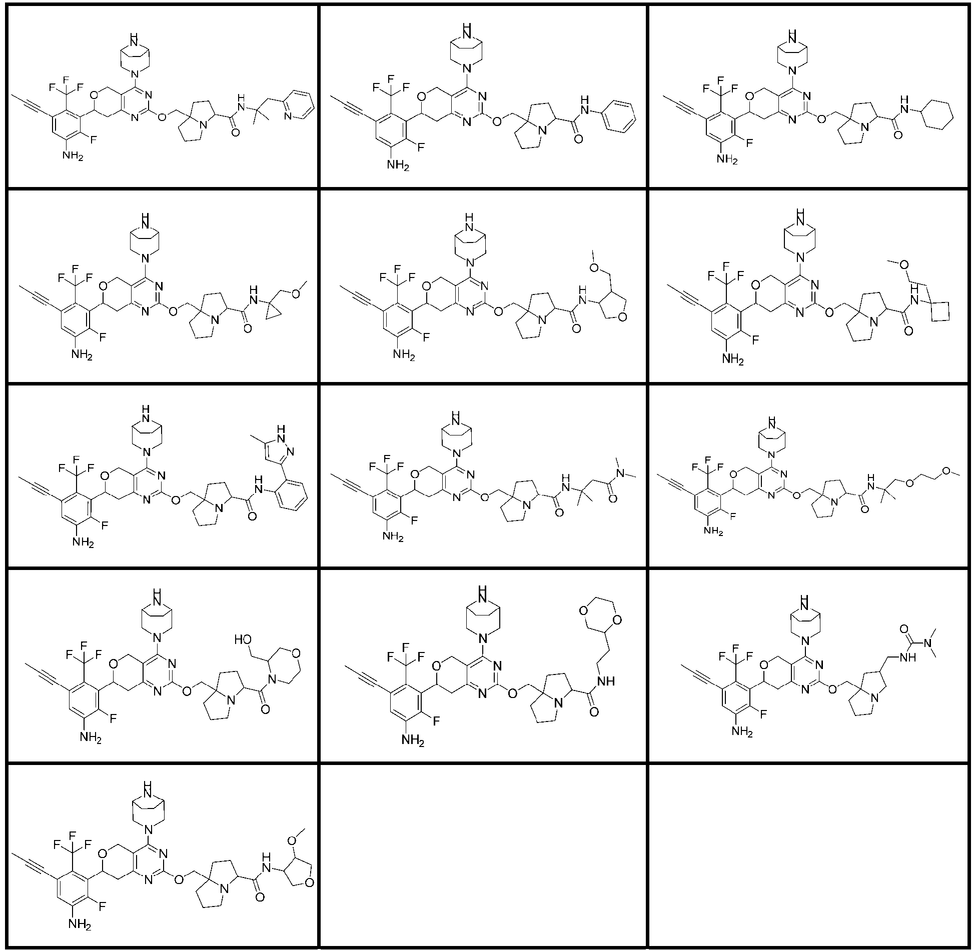

The present disclosure provides compounds as shown in Table 3 or pharmaceutically acceptable salts thereof.

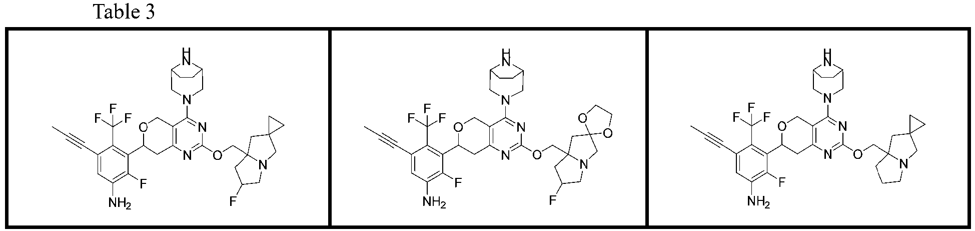

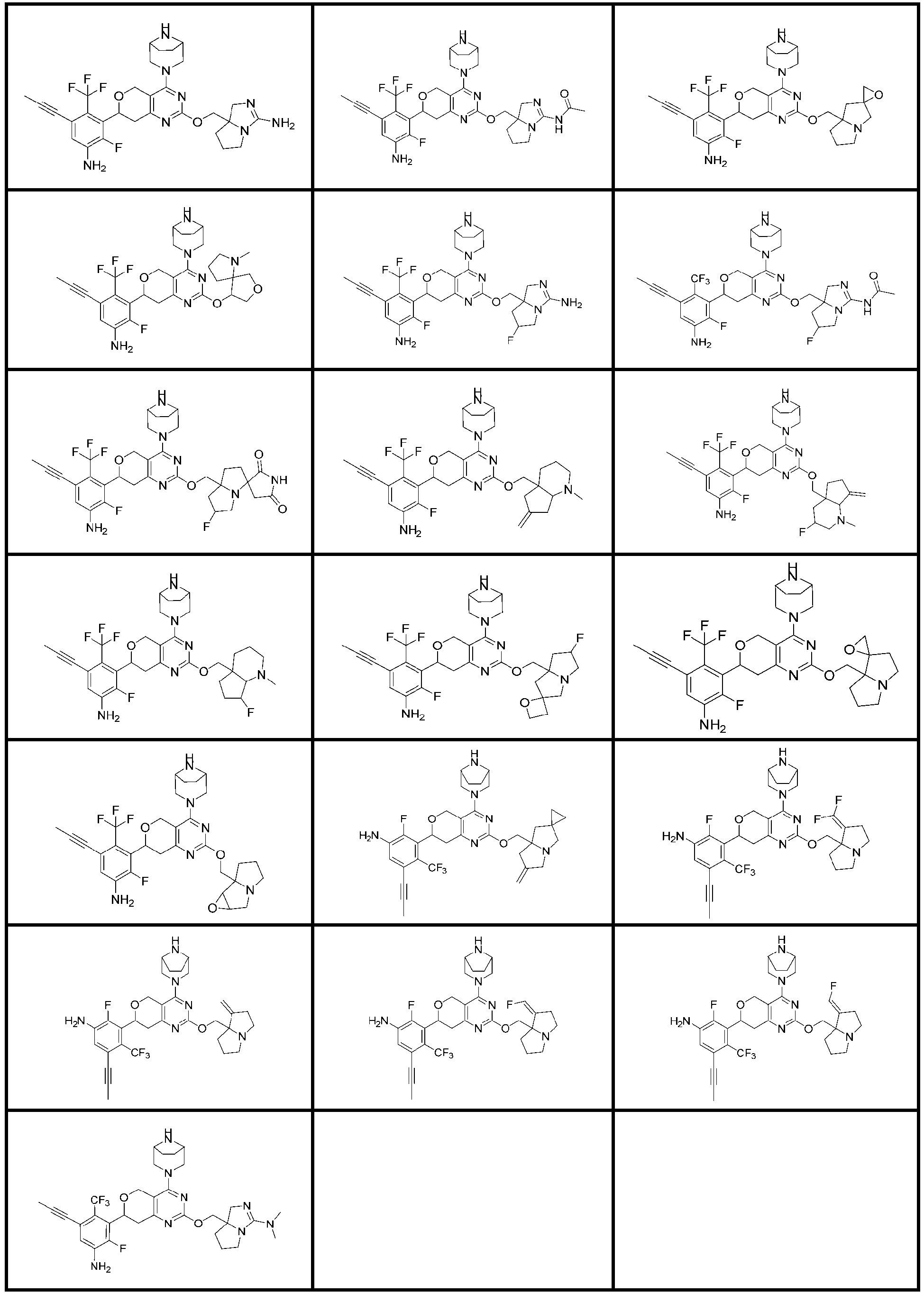

The present disclosure provides the compounds shown in Table 4 or pharmaceutically acceptable salts thereof.

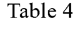

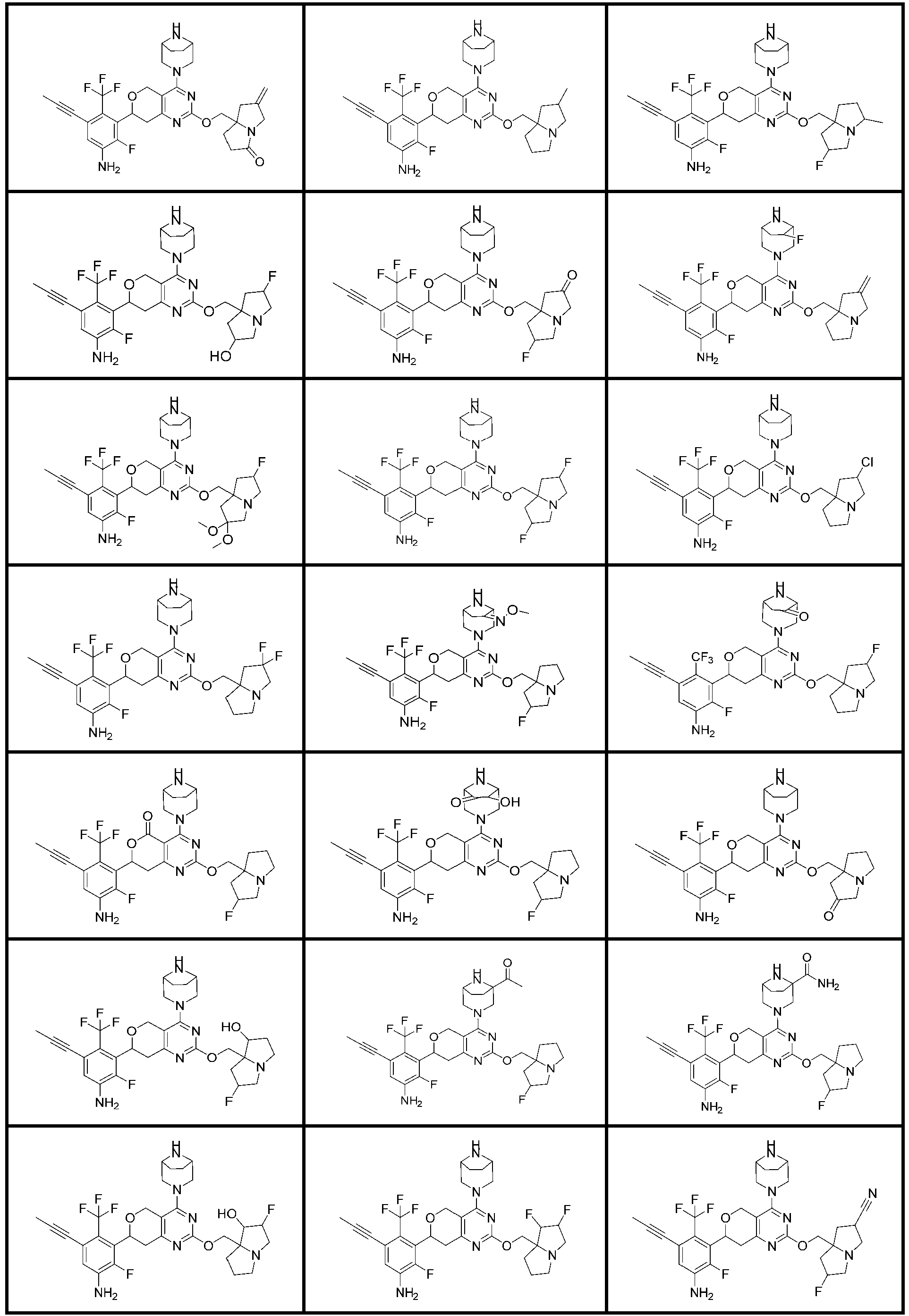

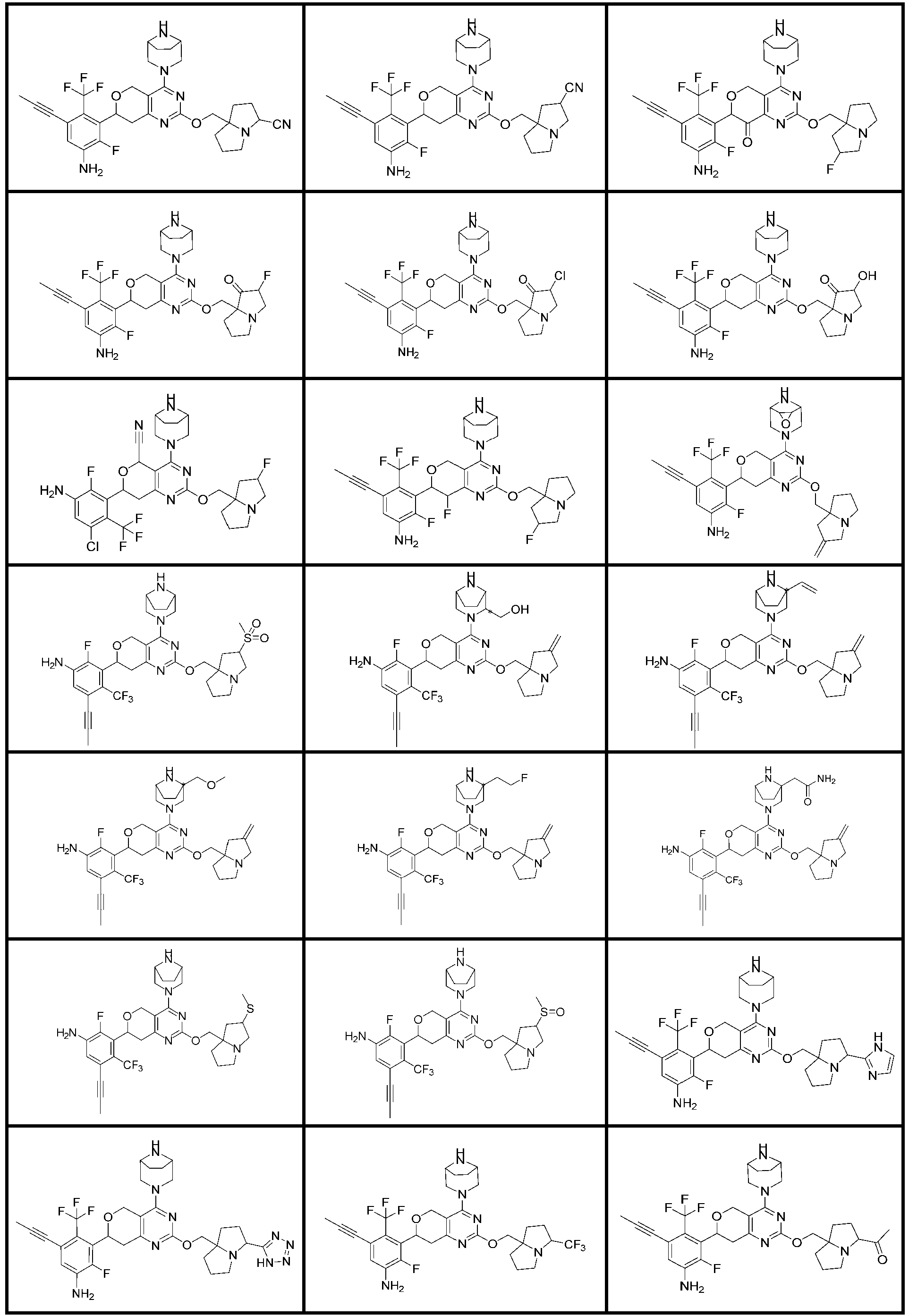

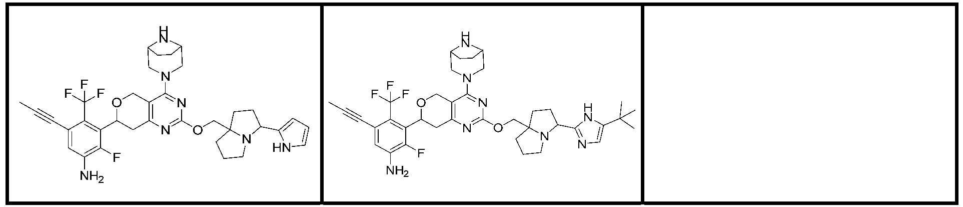

The present disclosure provides the following compounds or pharmaceutically acceptable salts thereof:

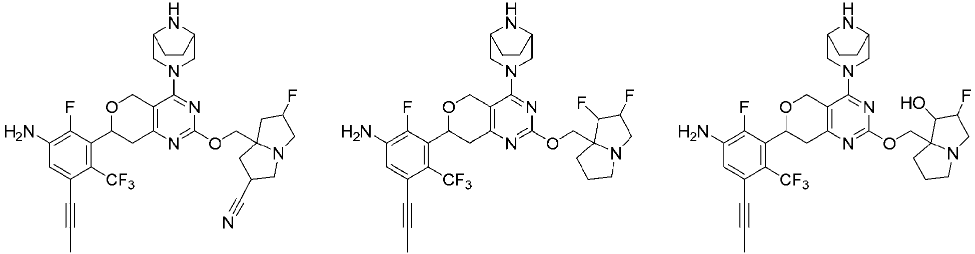

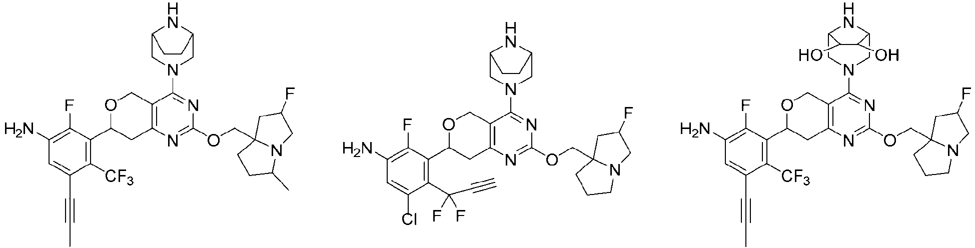

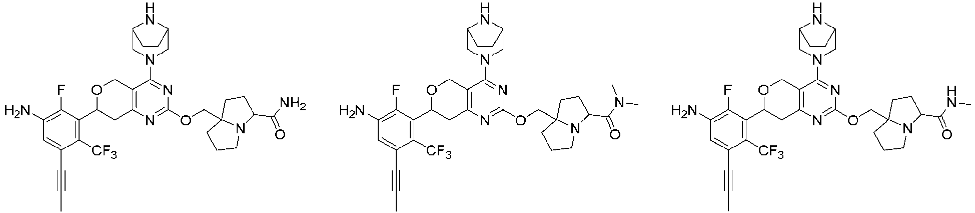

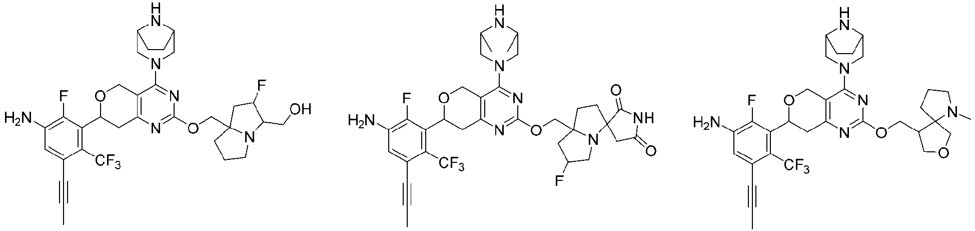

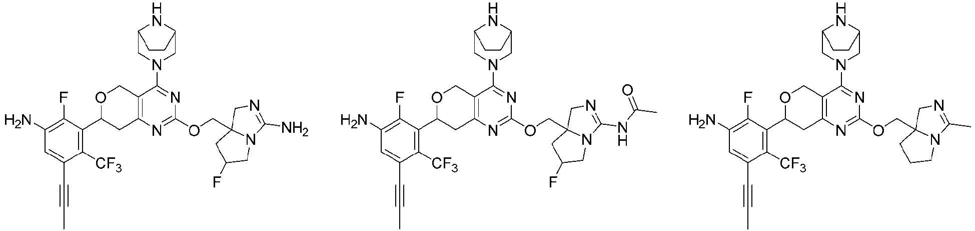

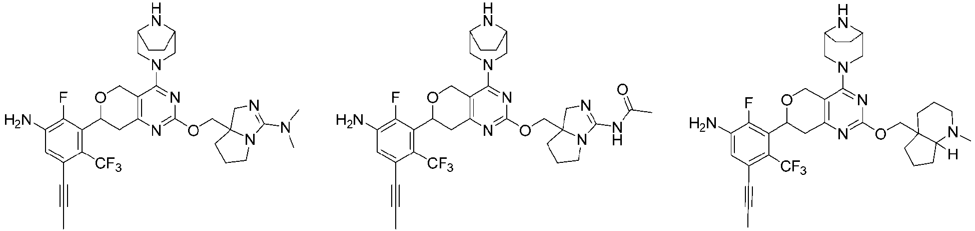

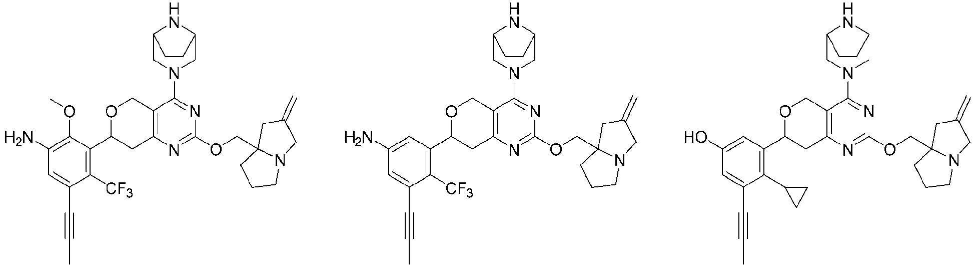

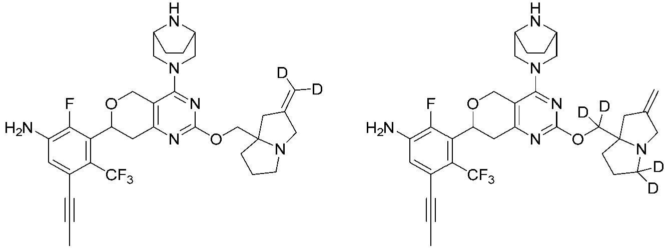

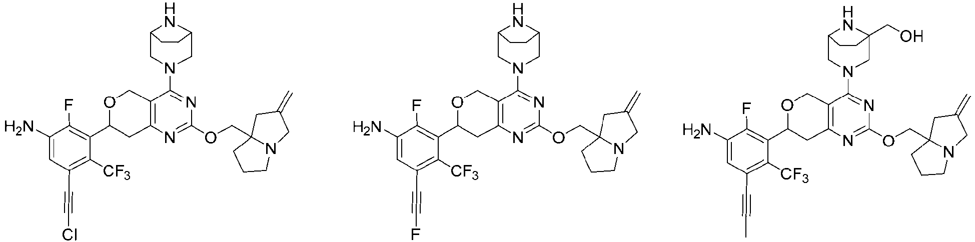

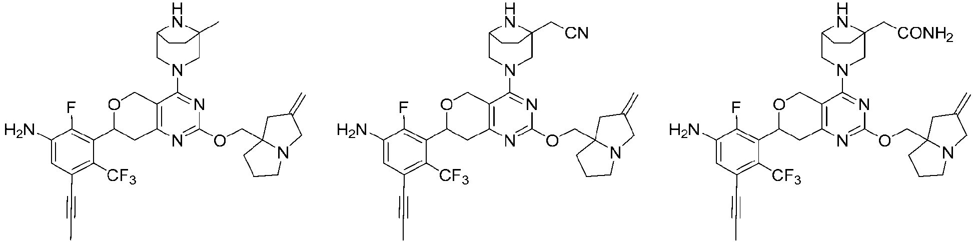

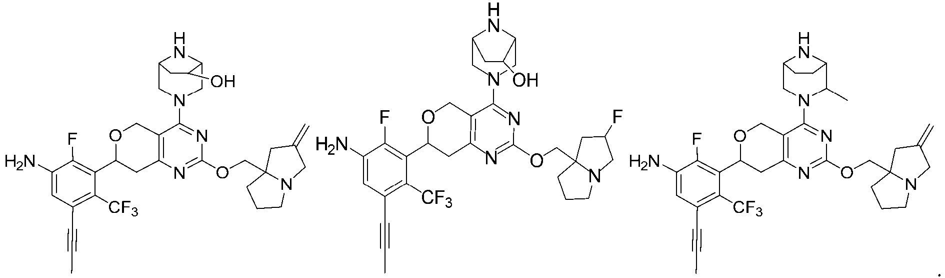

In some embodiments of the present disclosure, the compound or a pharmaceutically acceptable salt thereof is selected from:

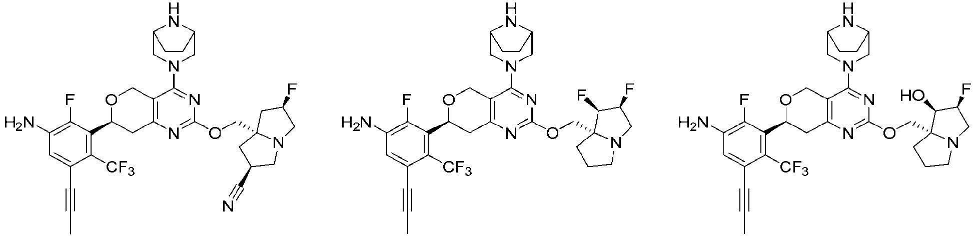

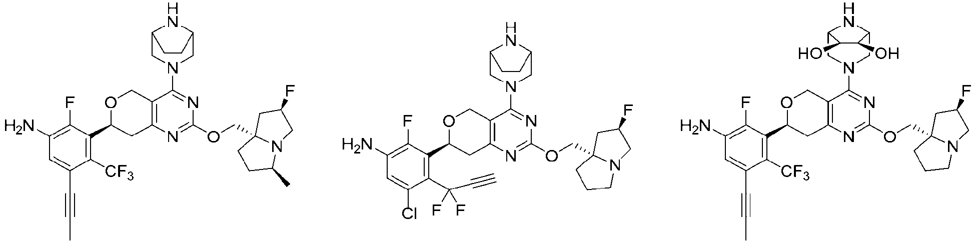

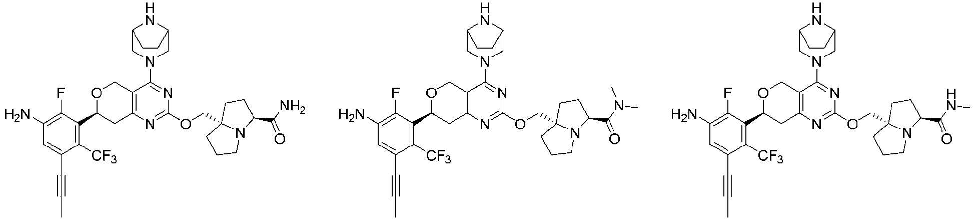

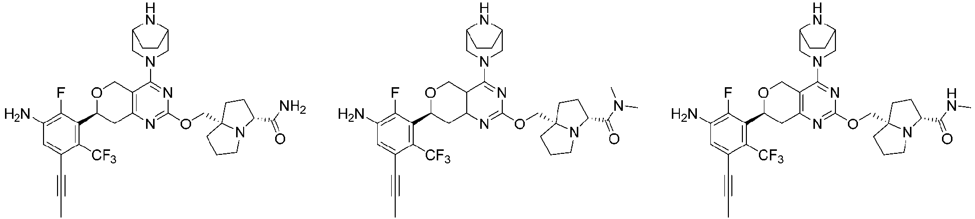

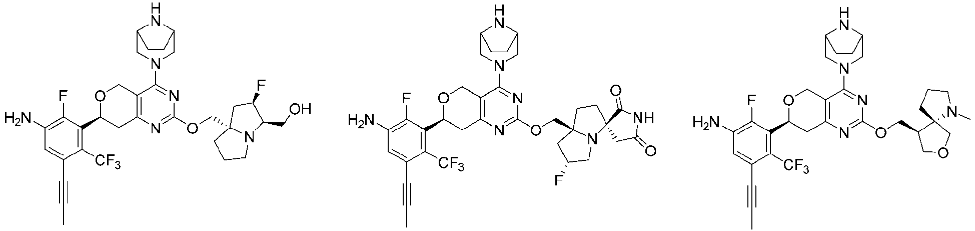

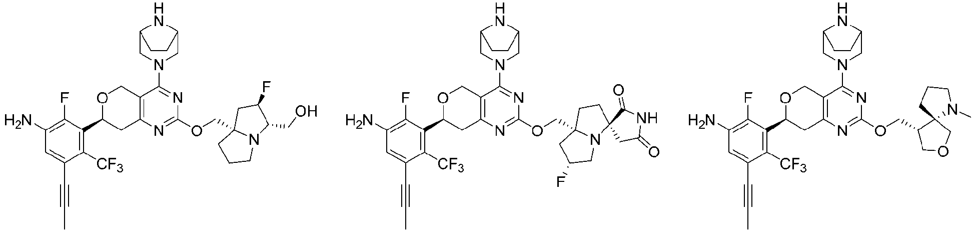

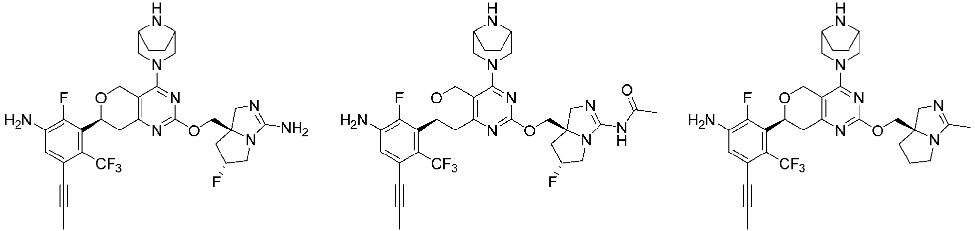

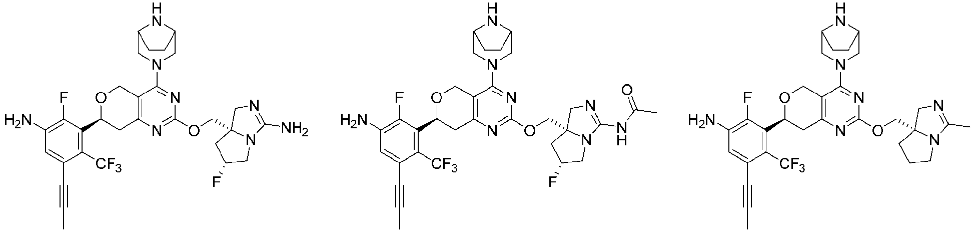

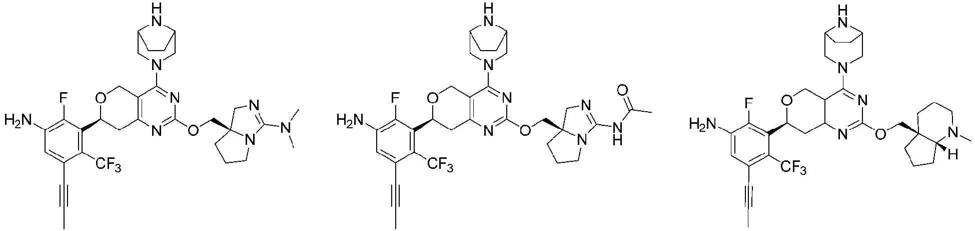

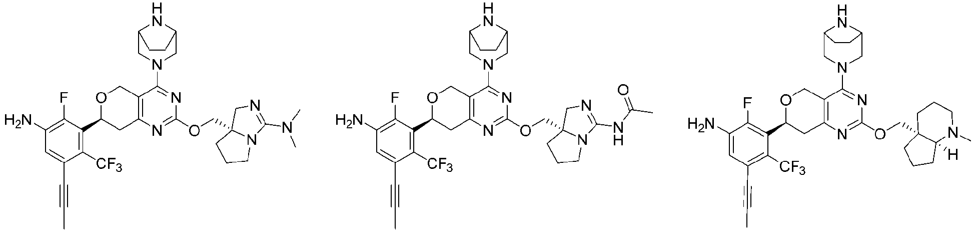

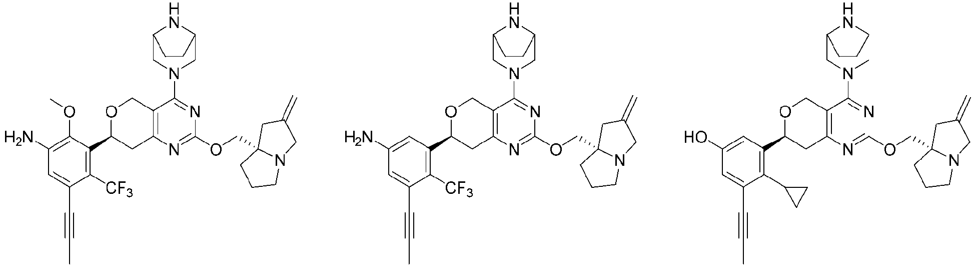

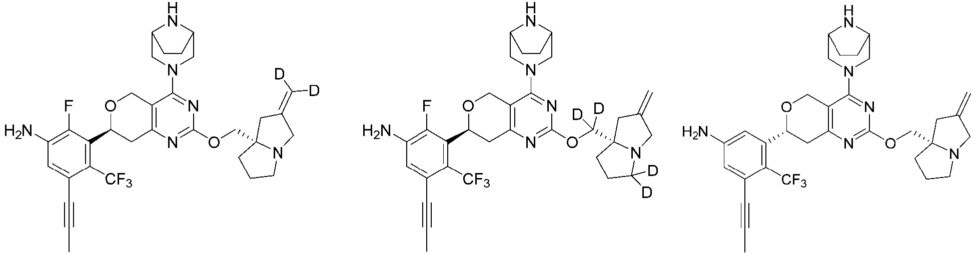

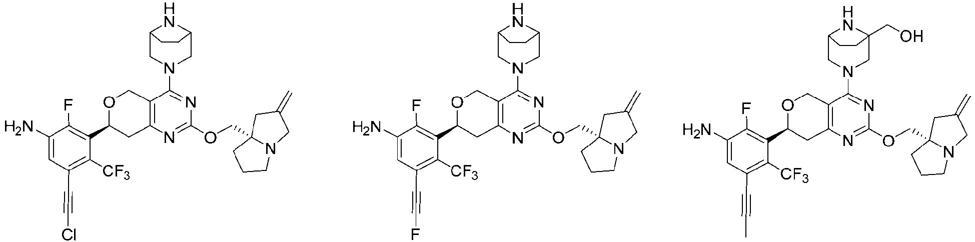

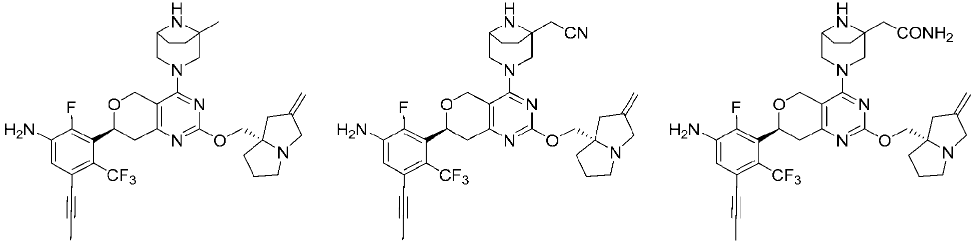

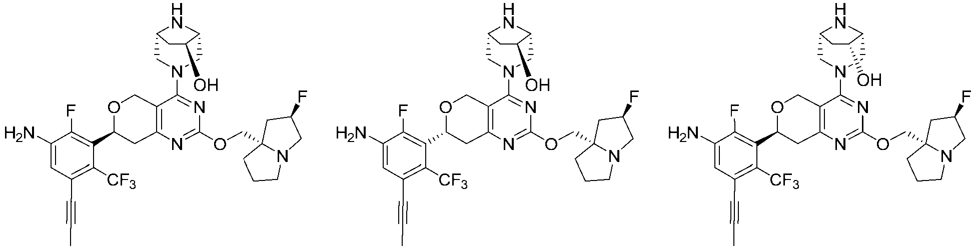

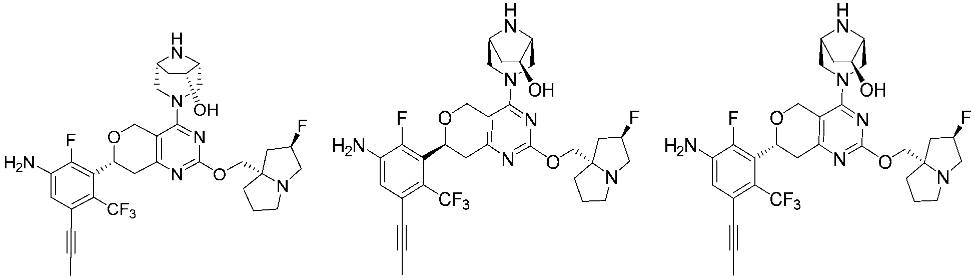

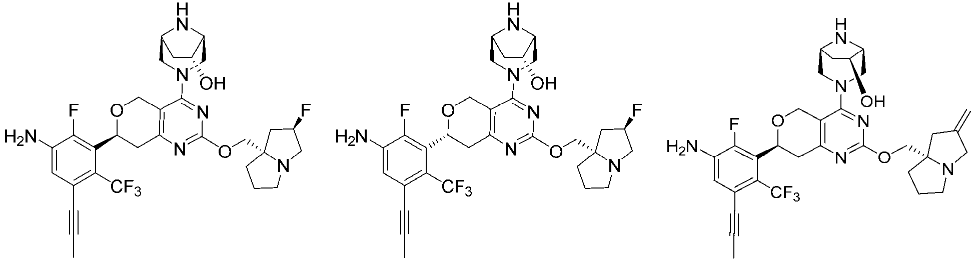

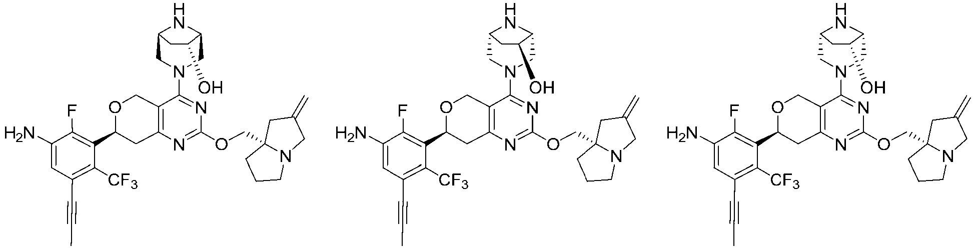

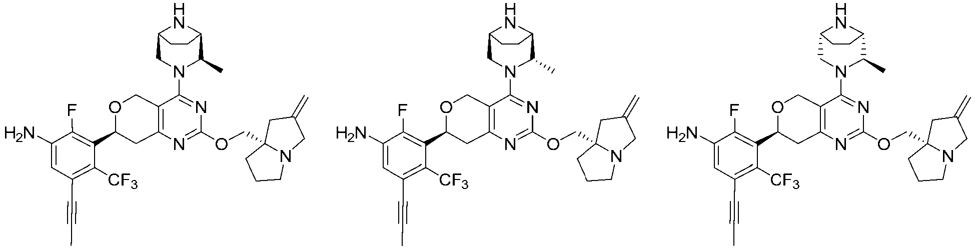

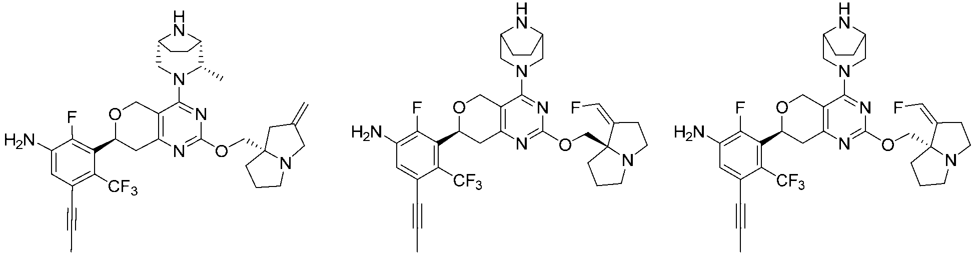

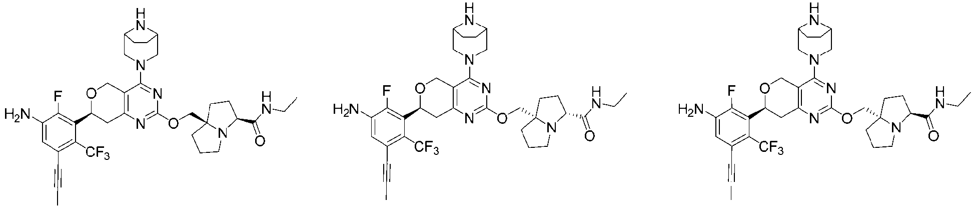

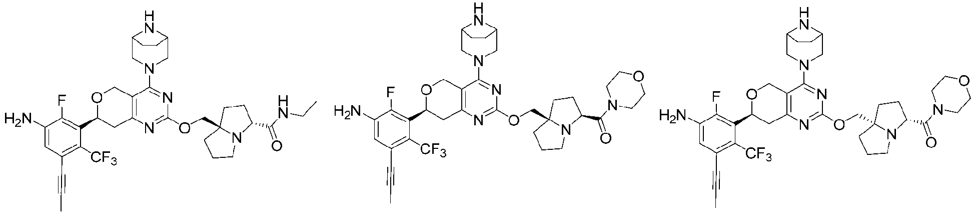

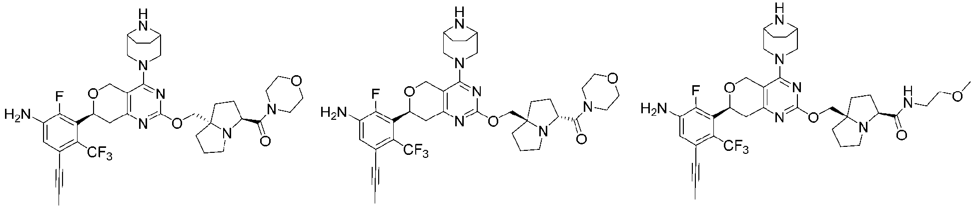

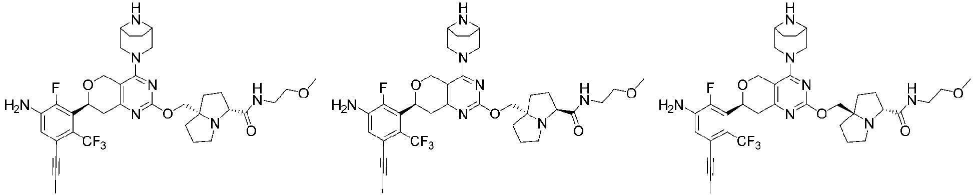

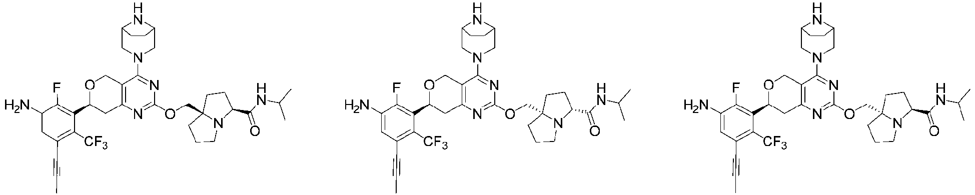

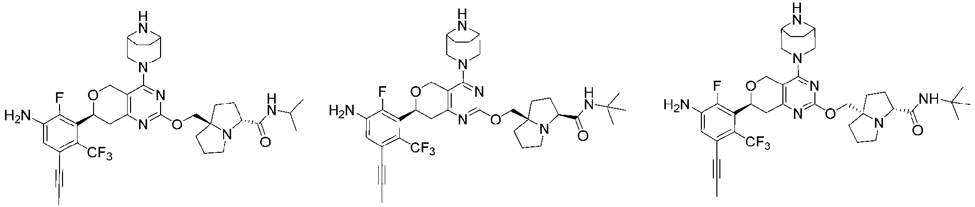

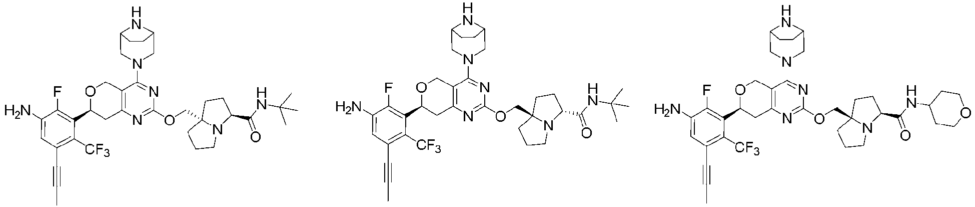

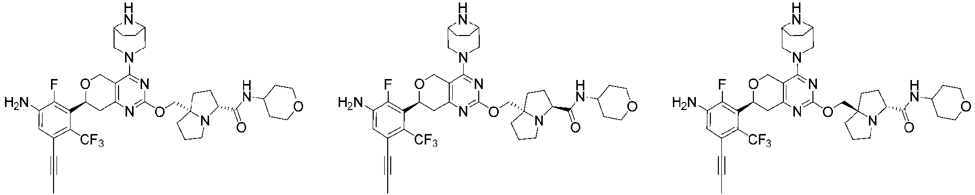

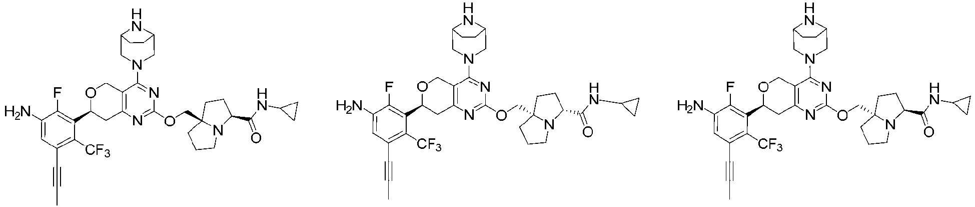

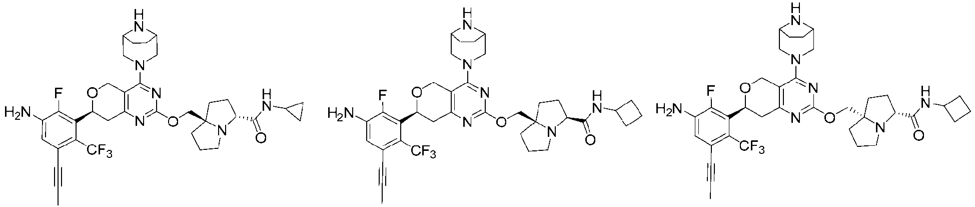

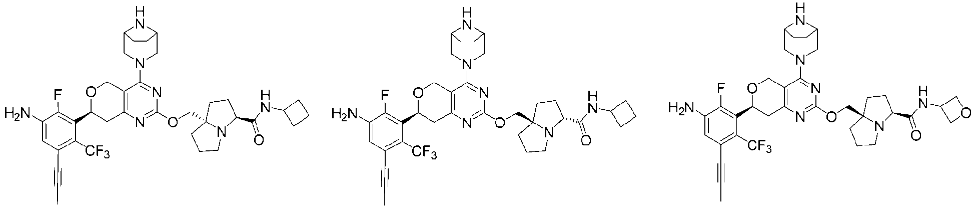

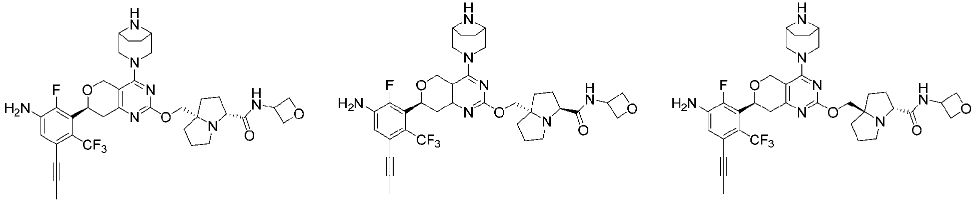

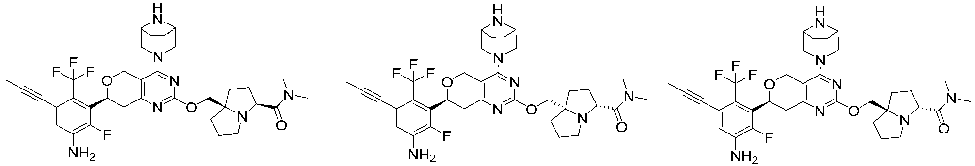

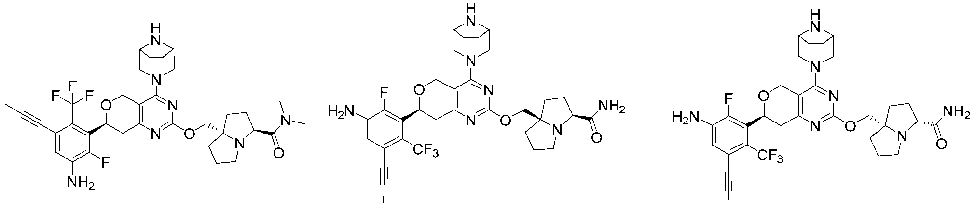

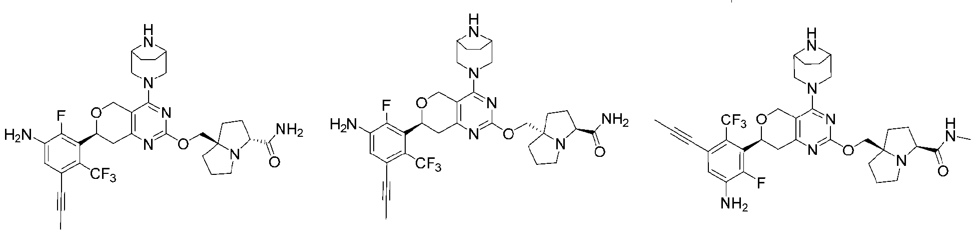

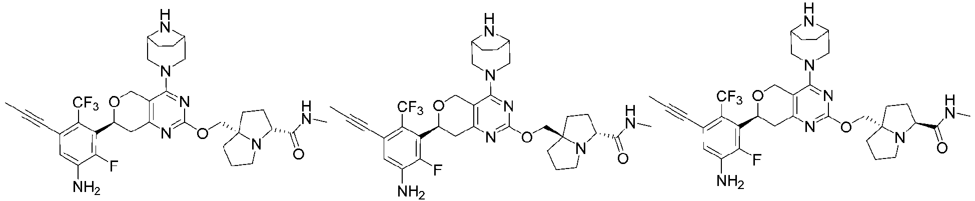

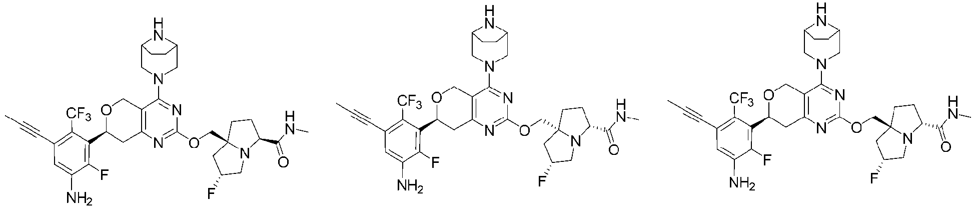

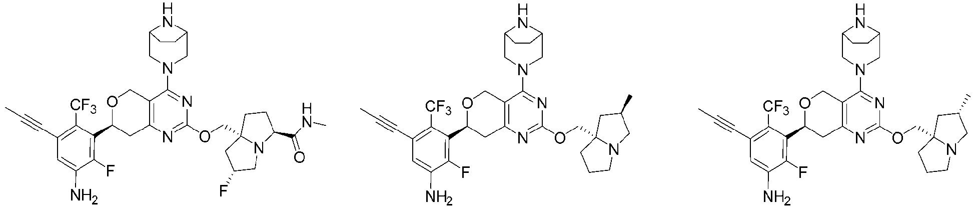

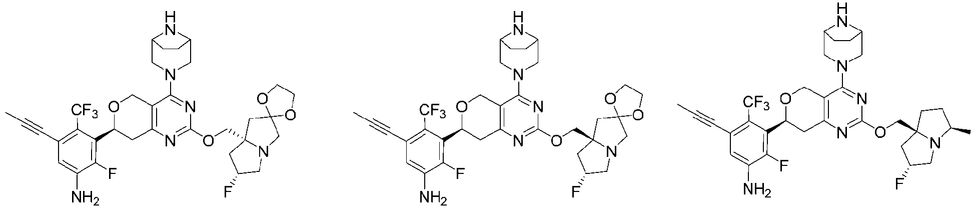

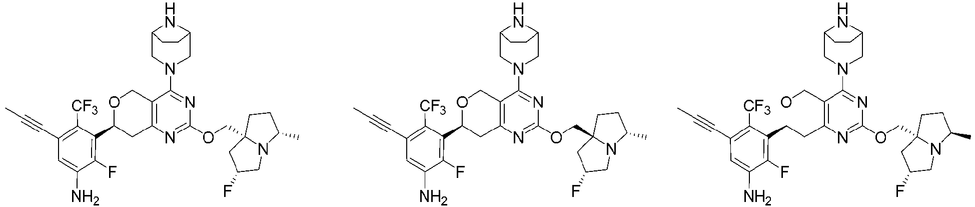

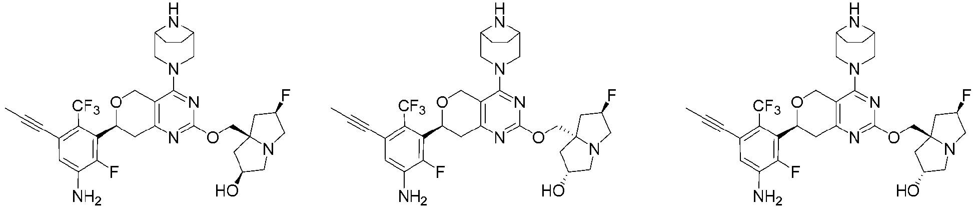

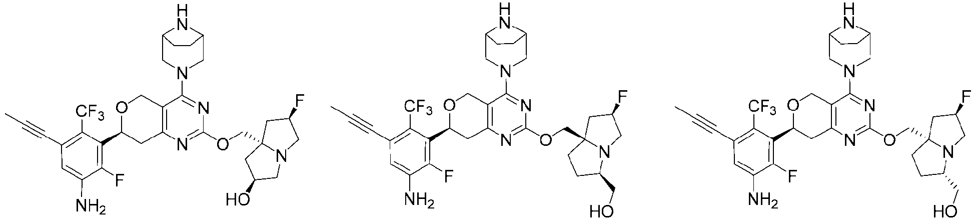

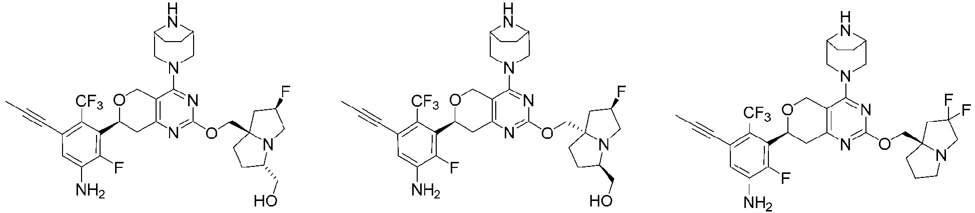

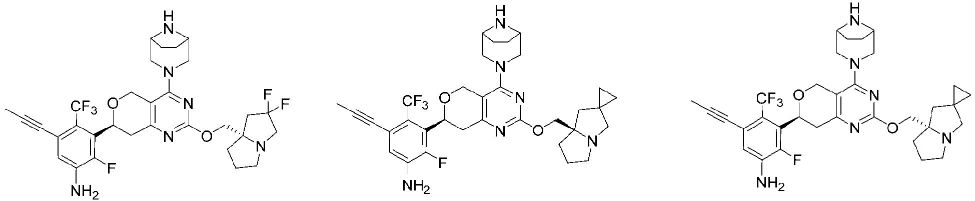

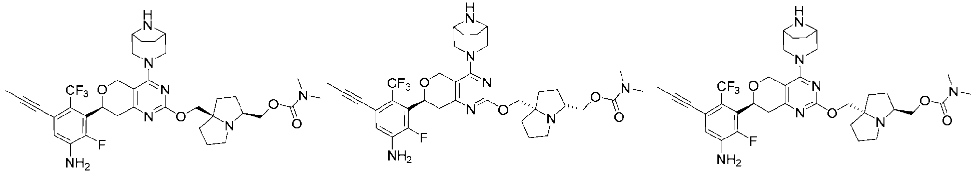

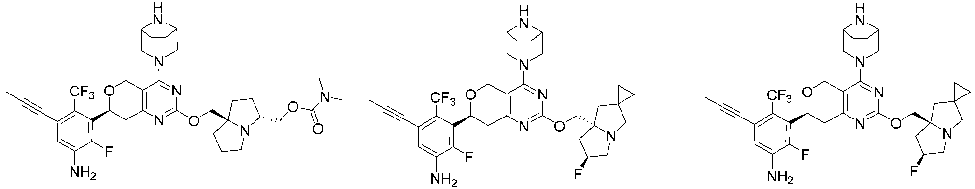

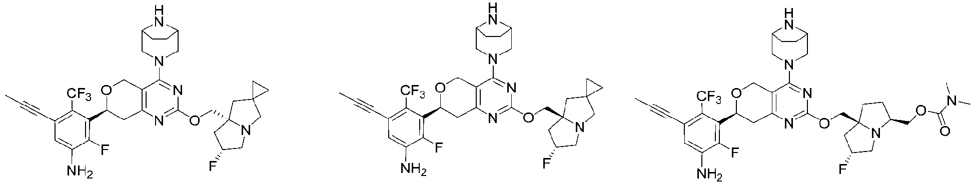

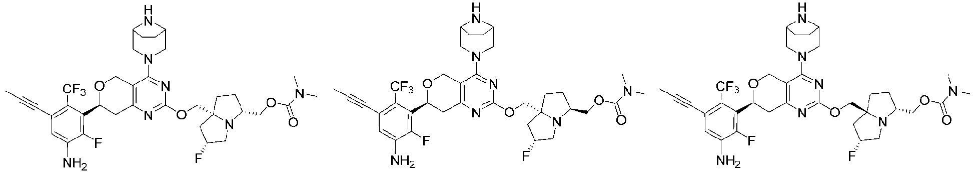

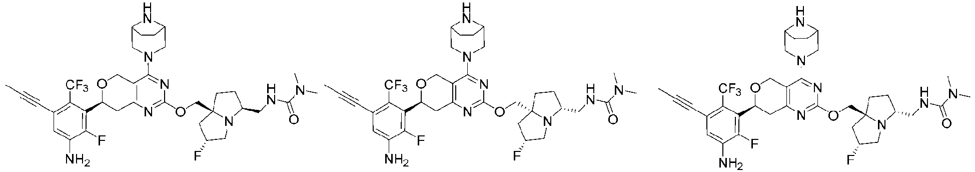

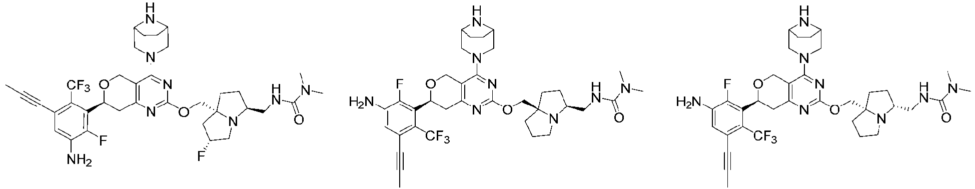

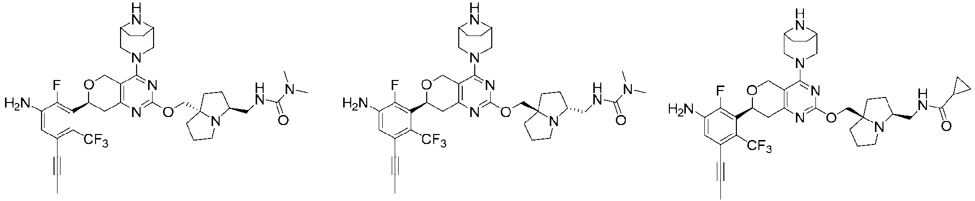

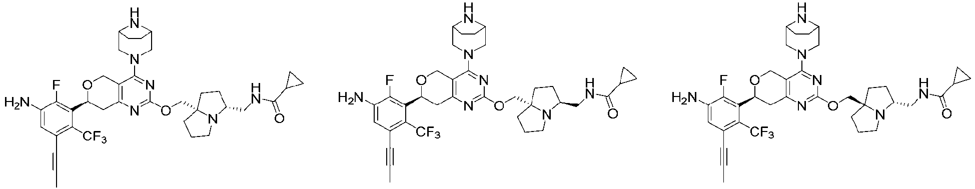

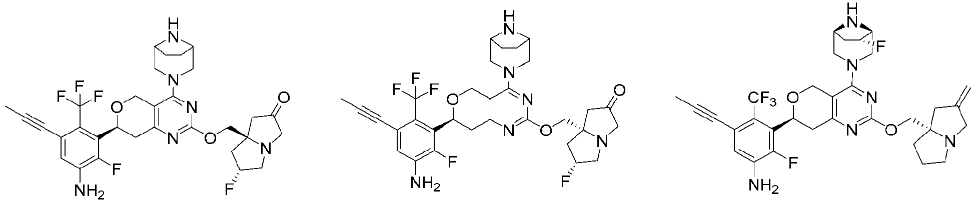

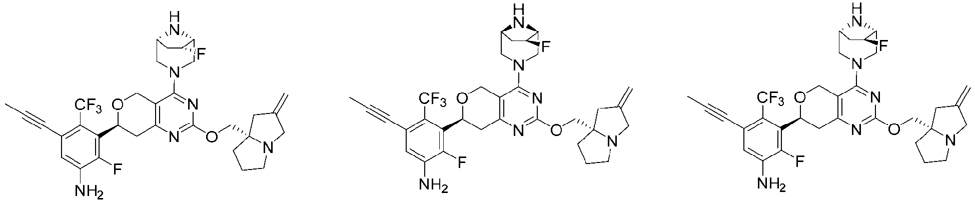

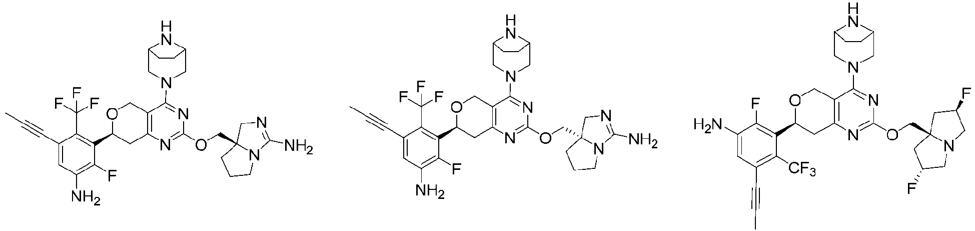

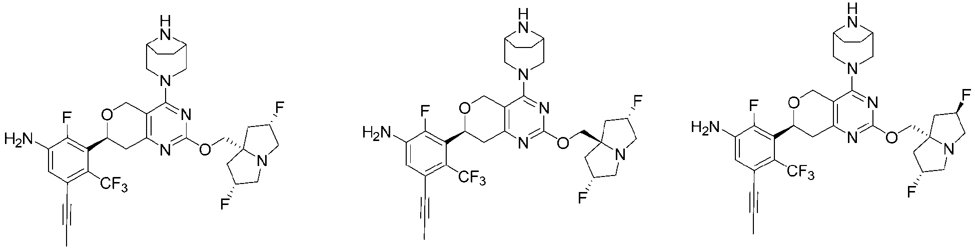

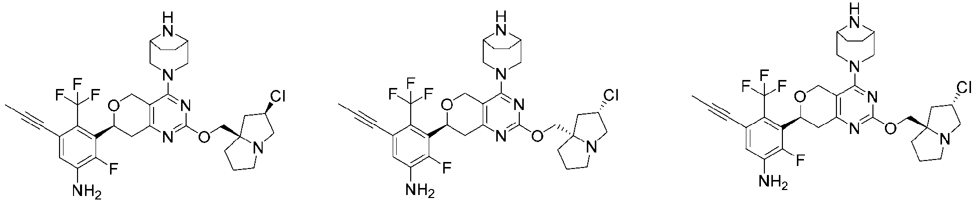

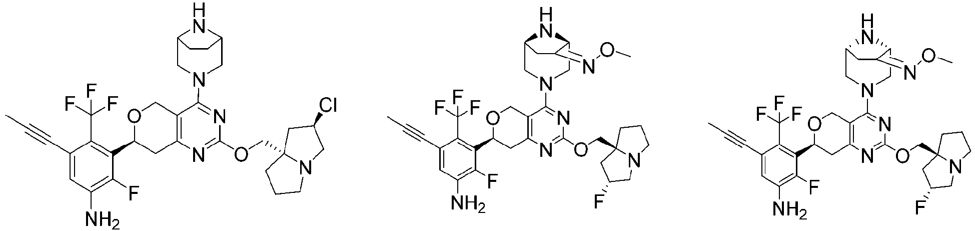

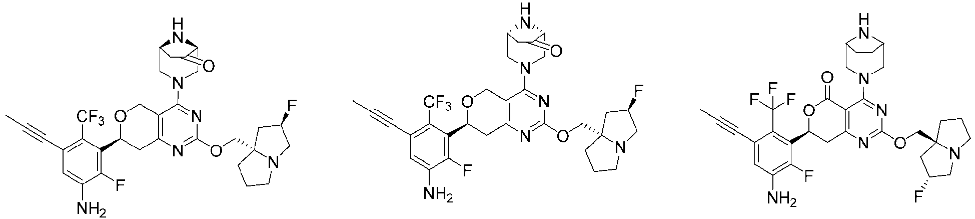

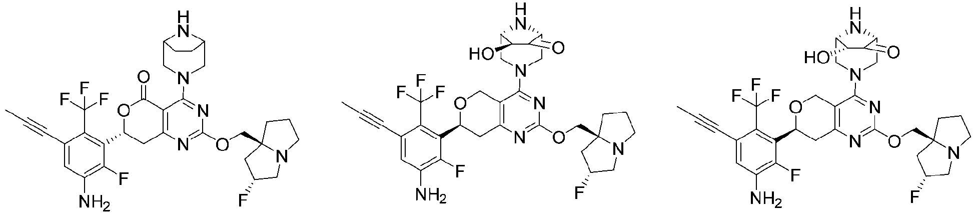

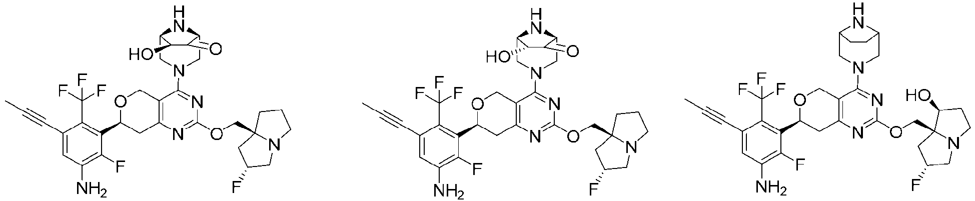

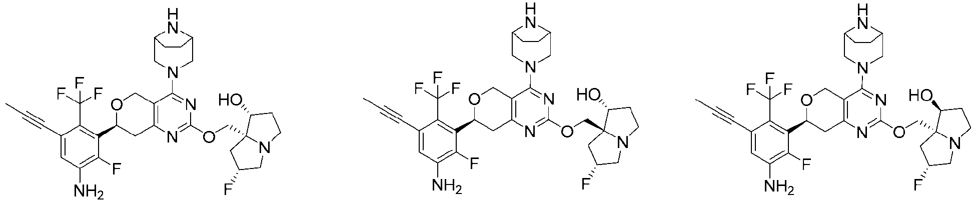

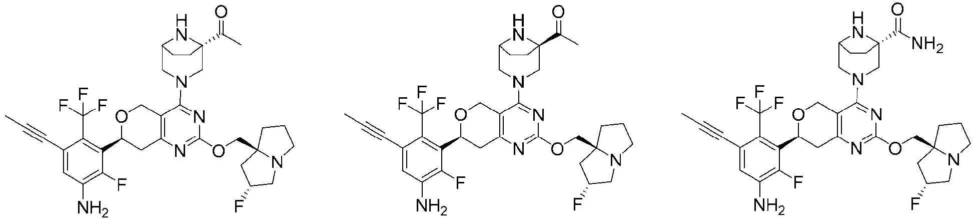

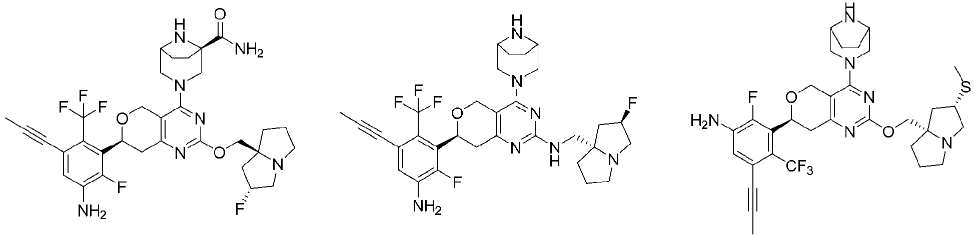

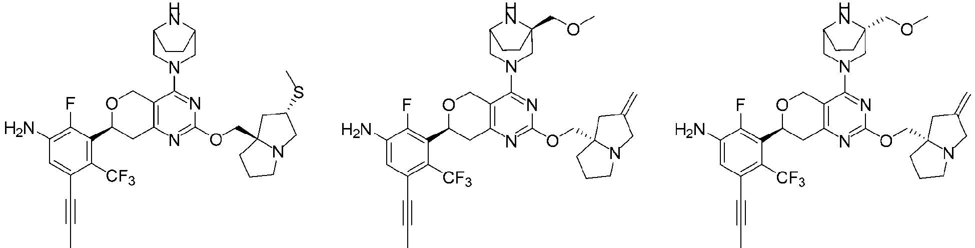

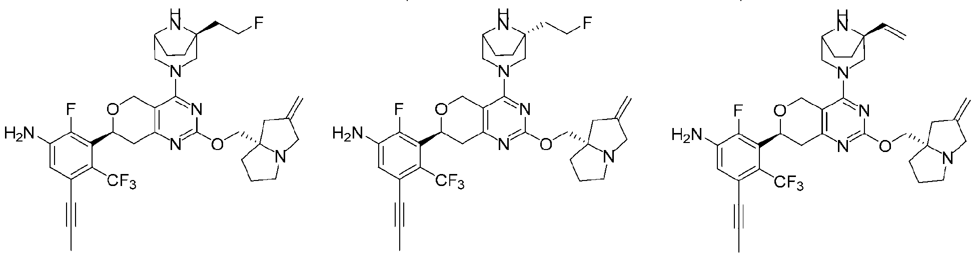

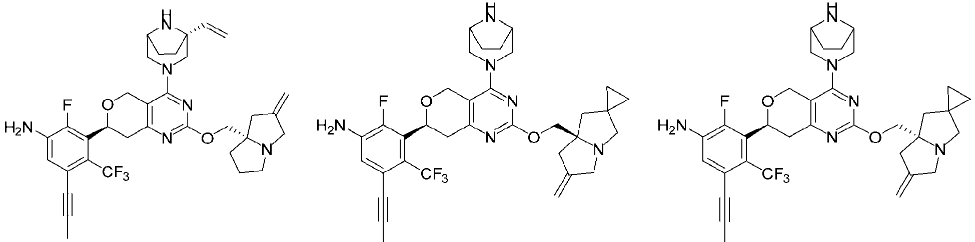

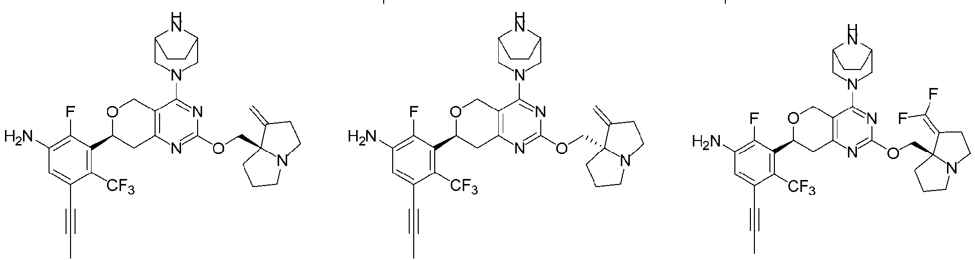

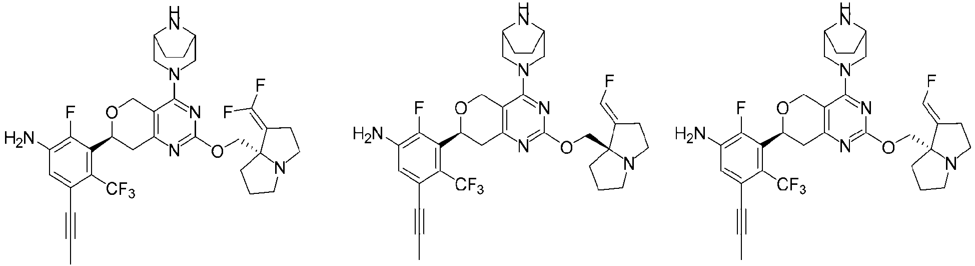

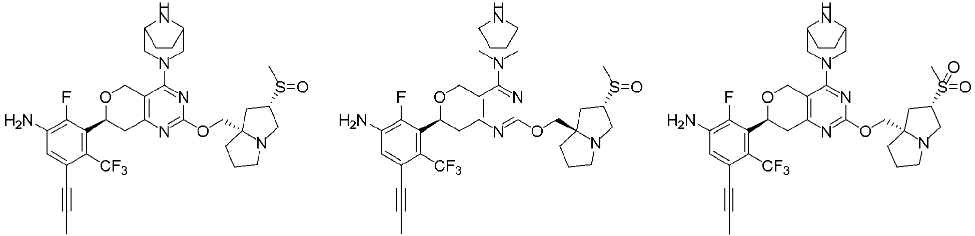

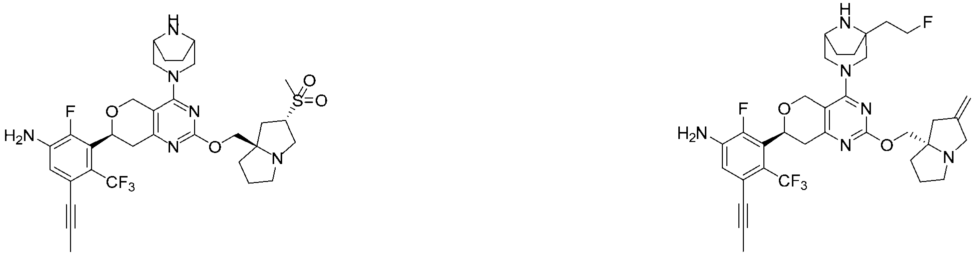

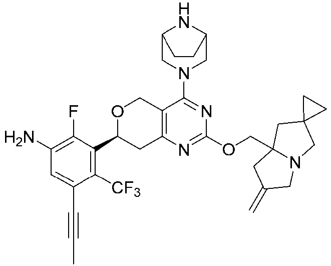

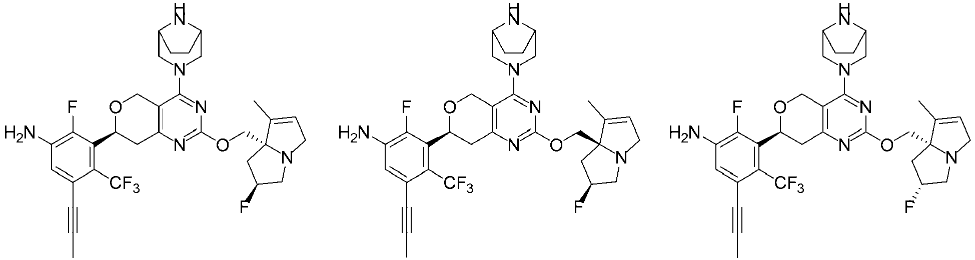

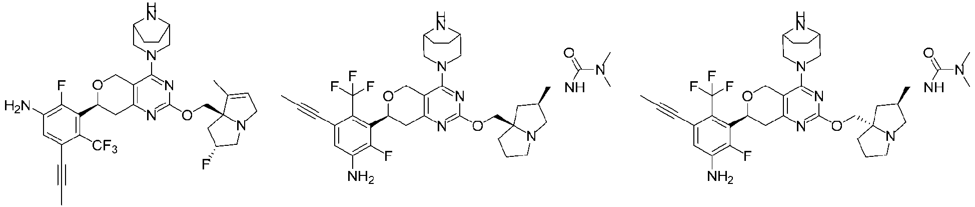

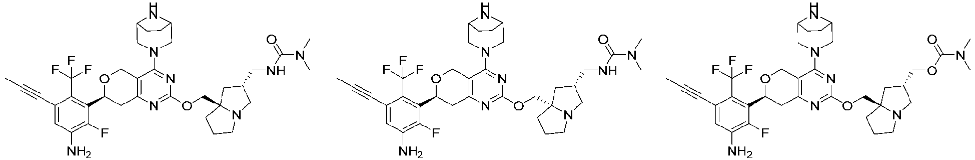

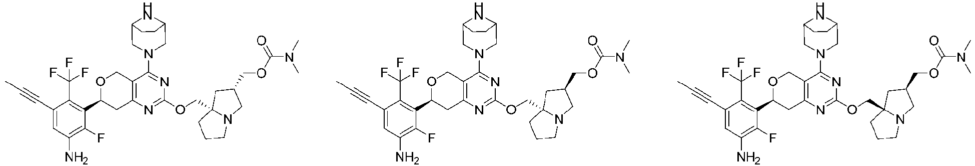

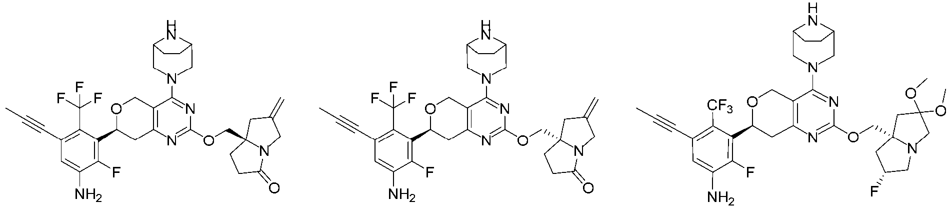

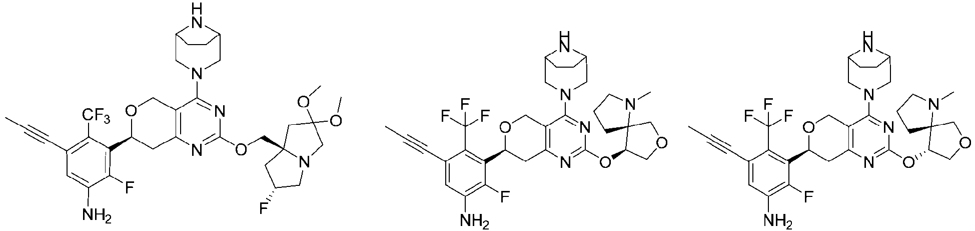

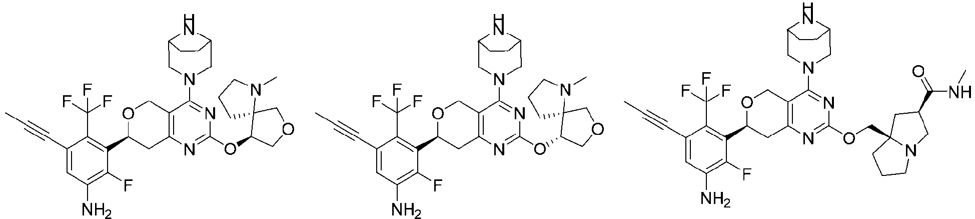

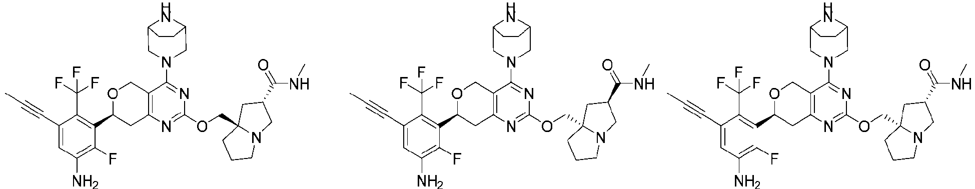

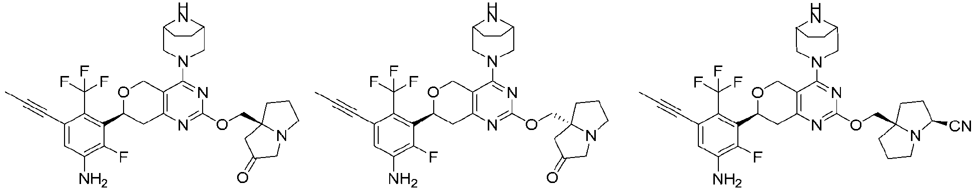

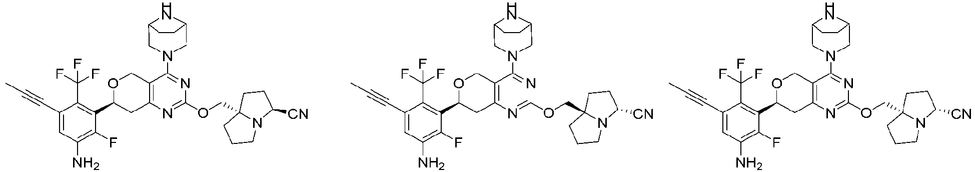

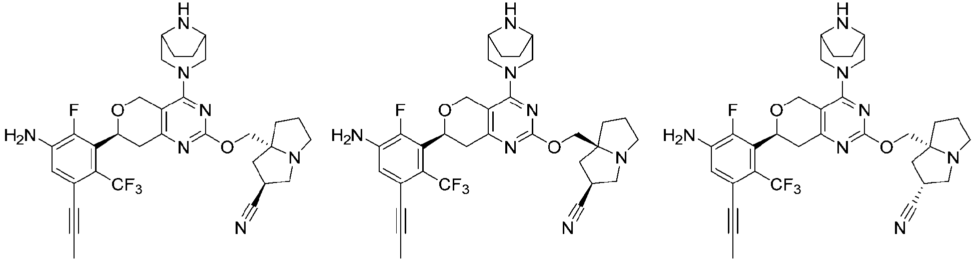

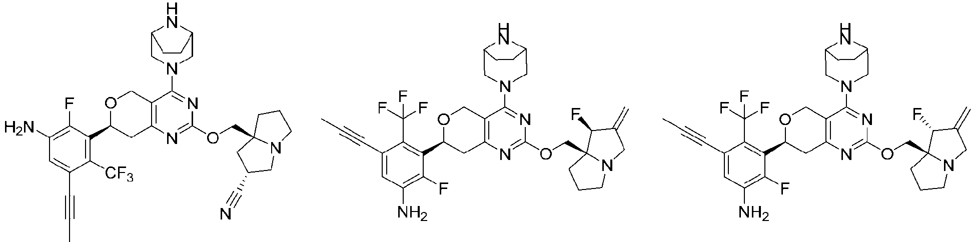

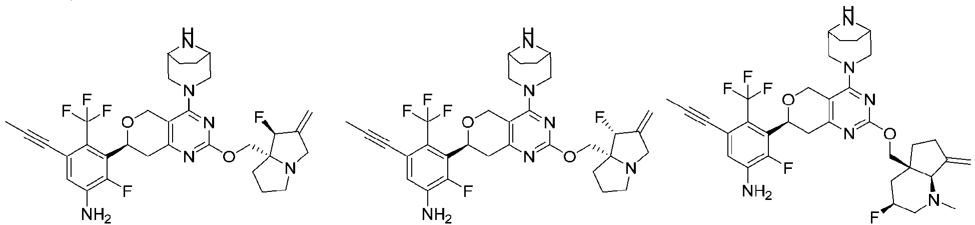

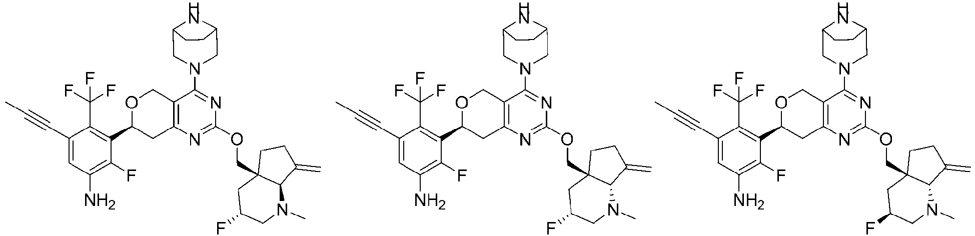

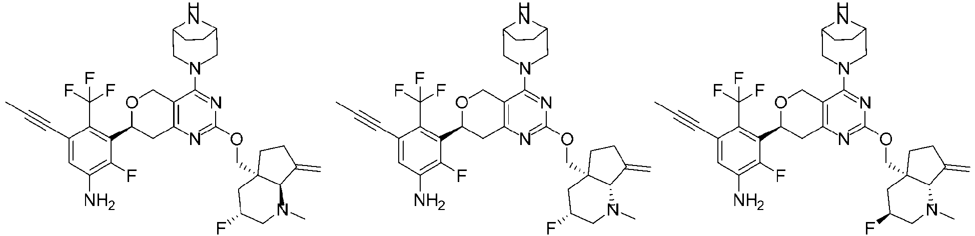

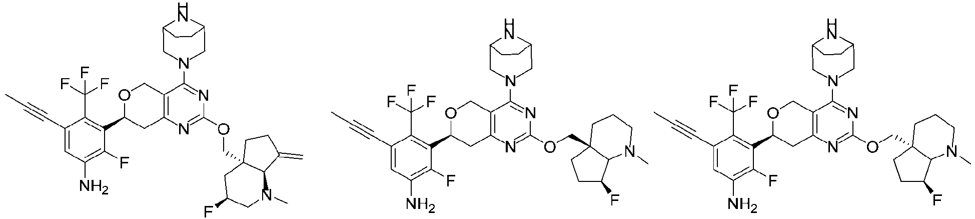

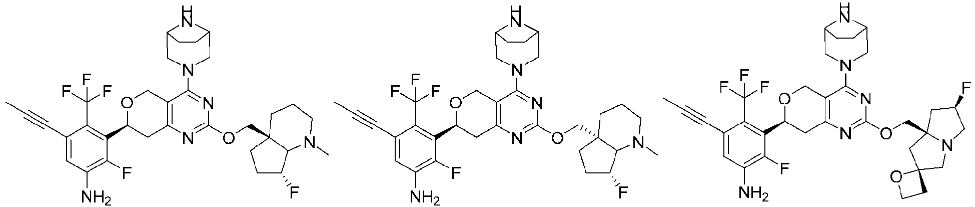

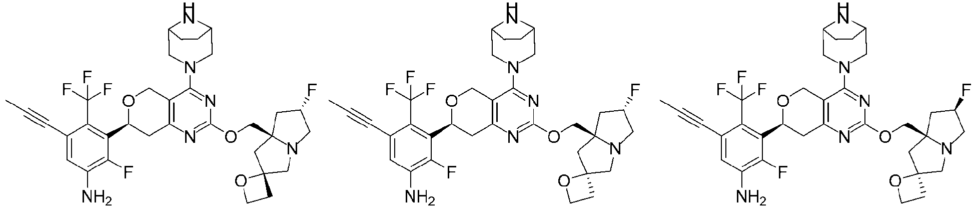

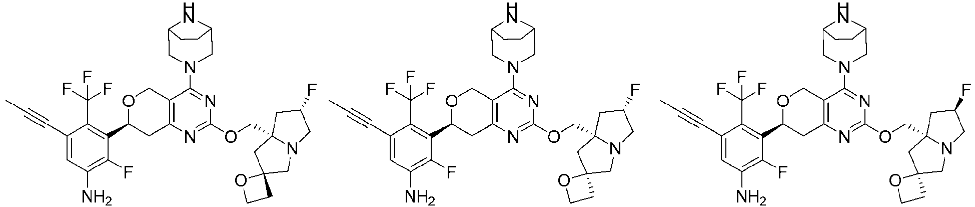

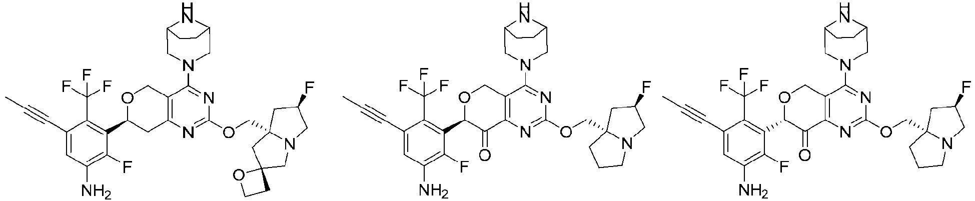

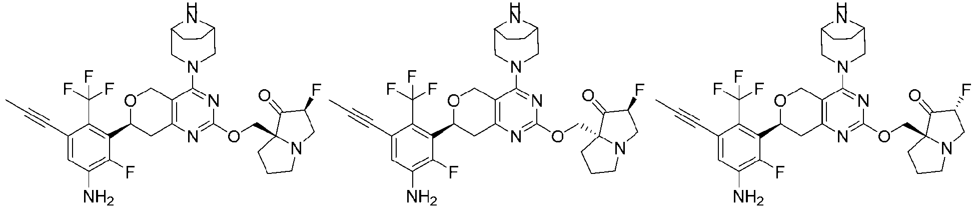

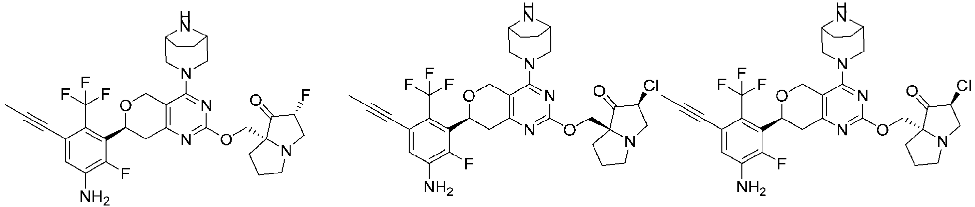

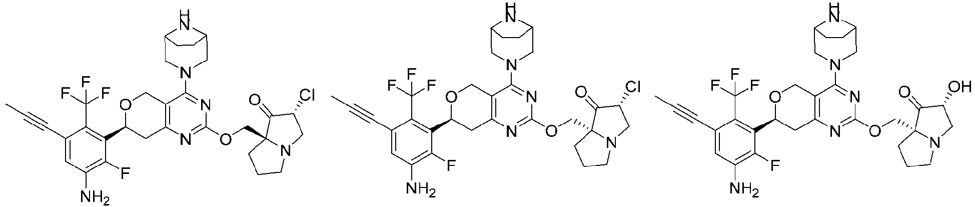

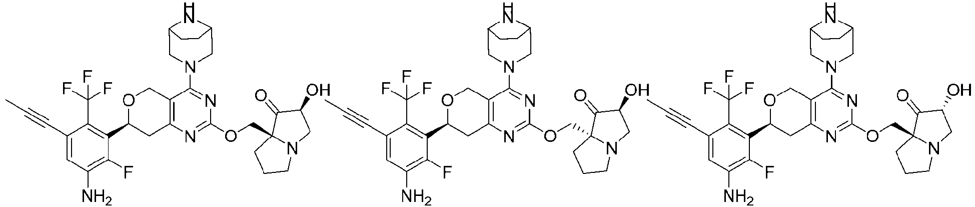

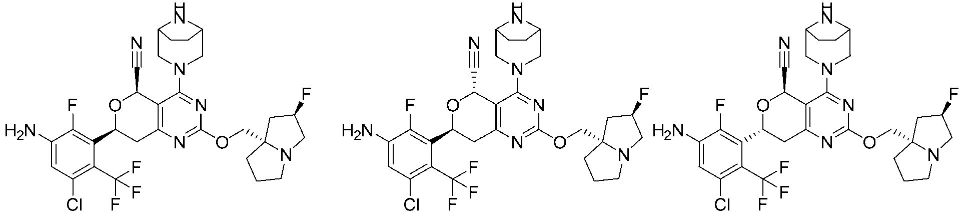

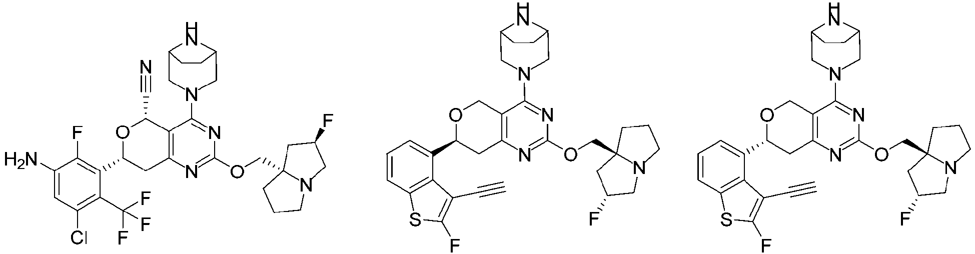

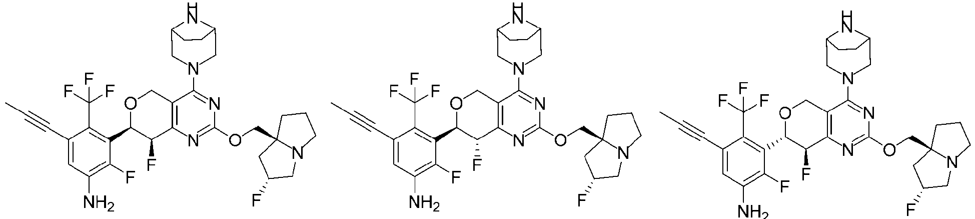

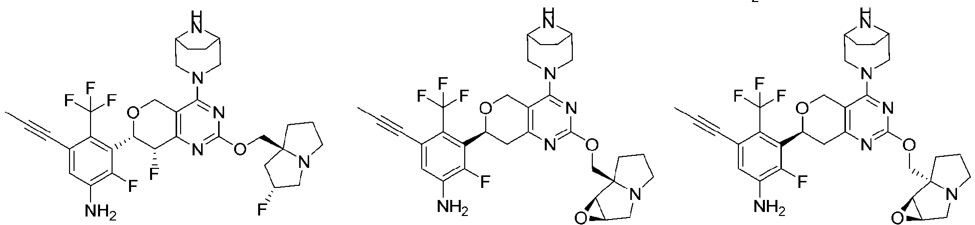

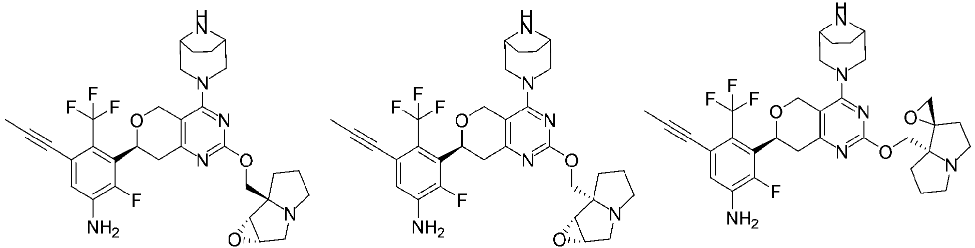

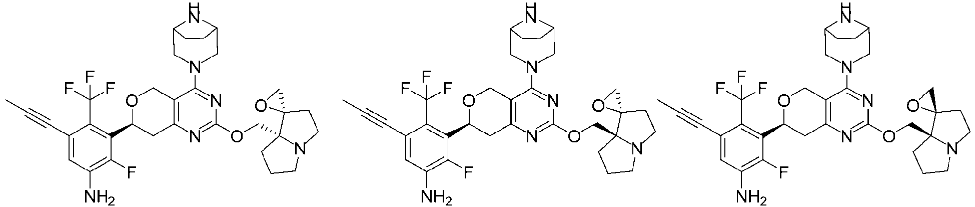

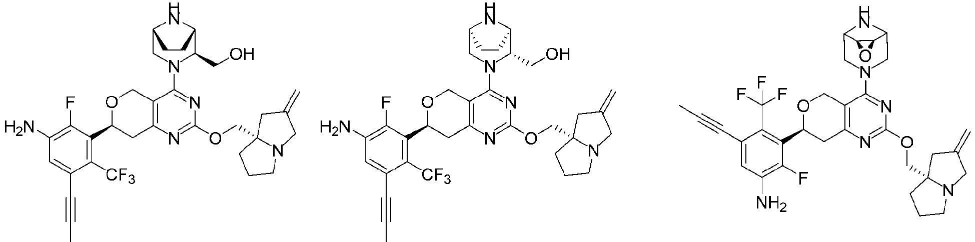

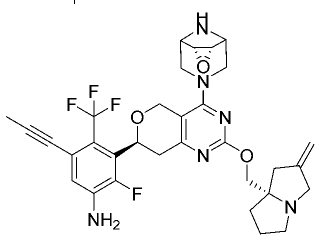

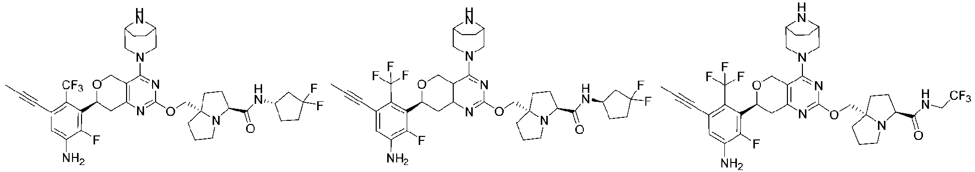

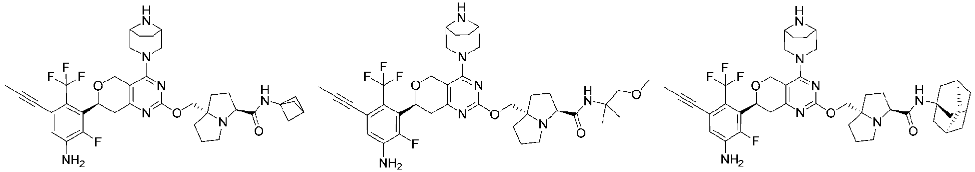

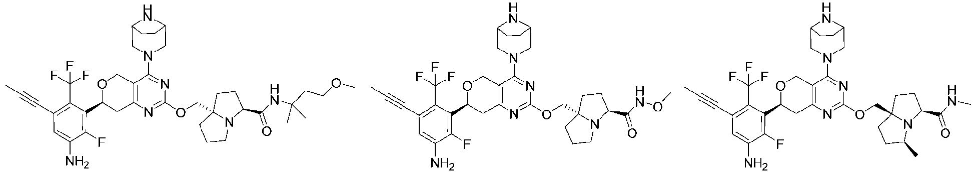

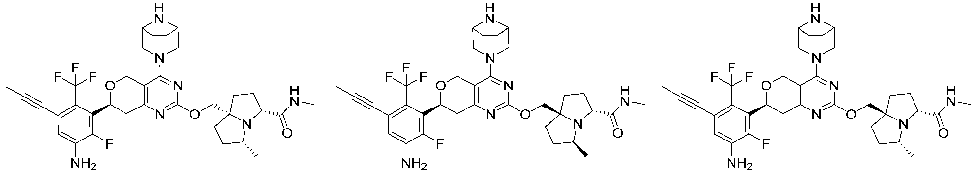

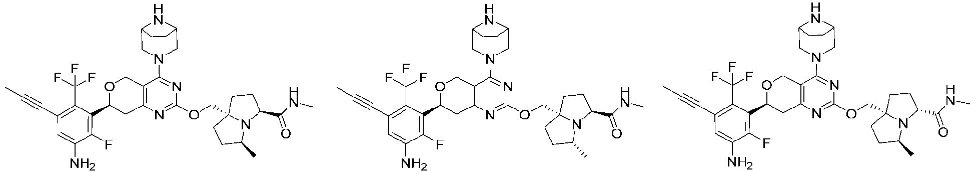

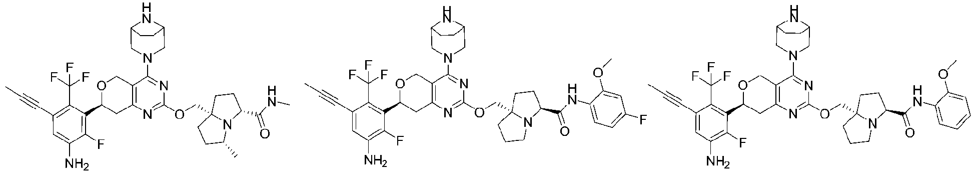

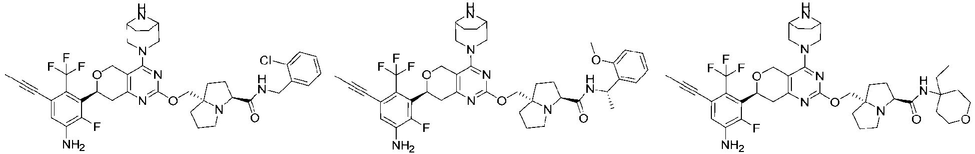

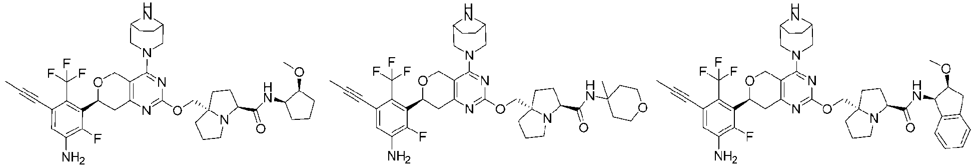

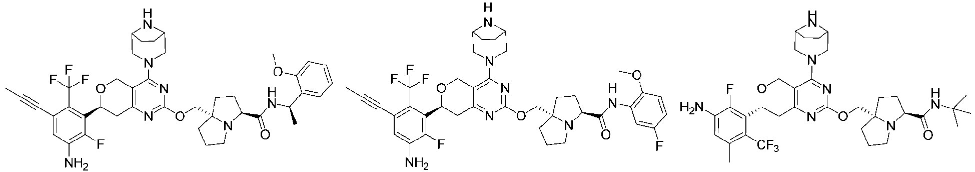

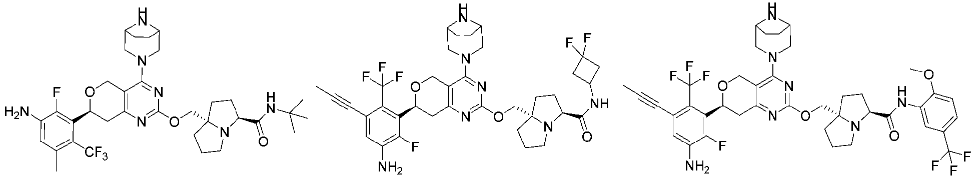

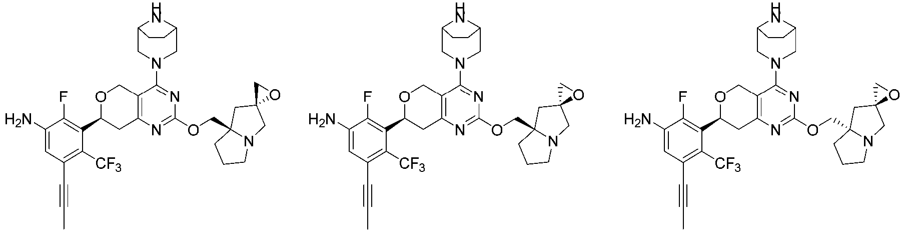

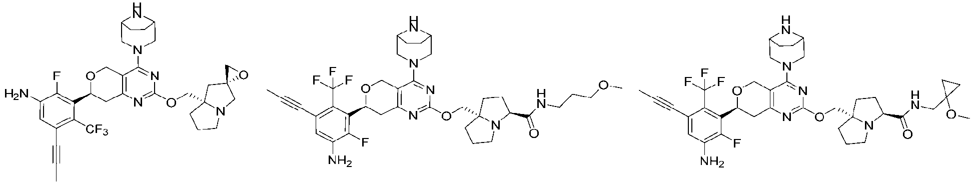

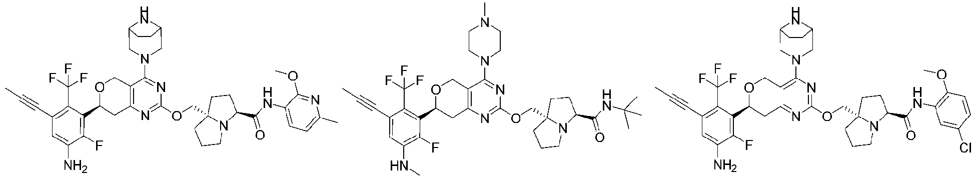

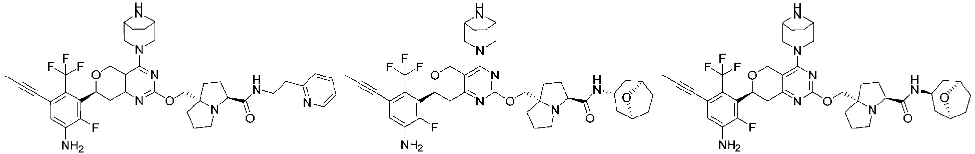

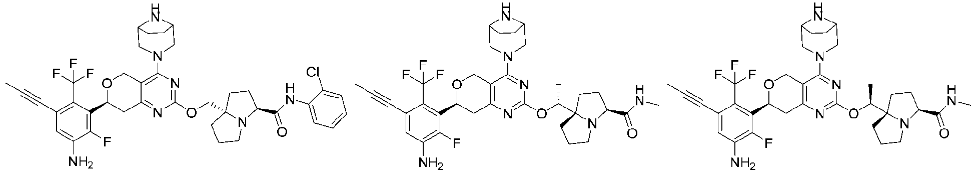

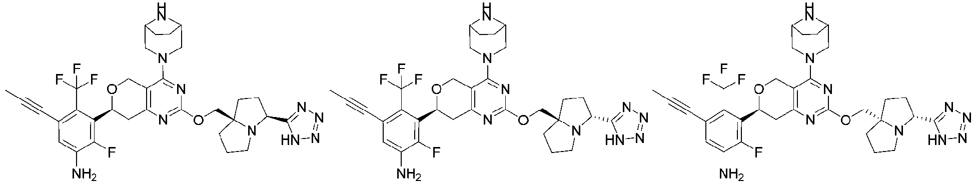

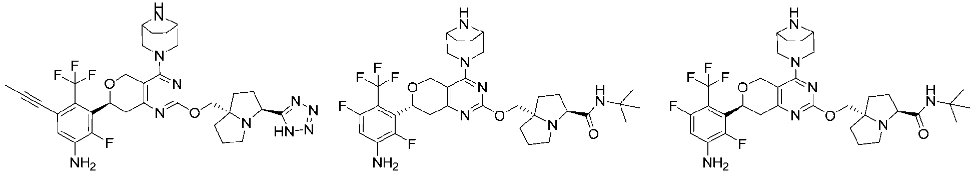

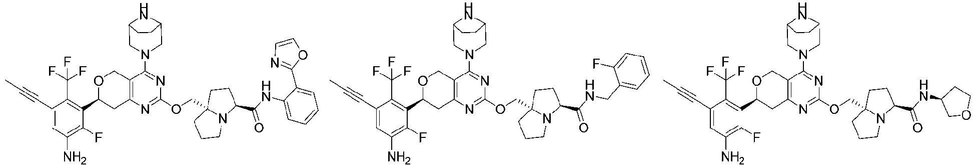

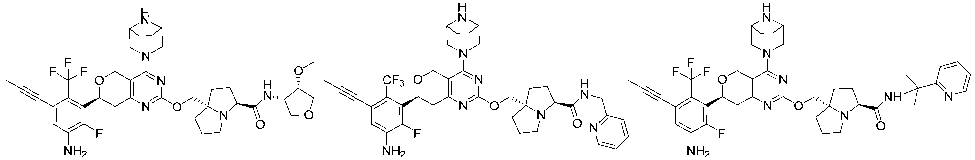

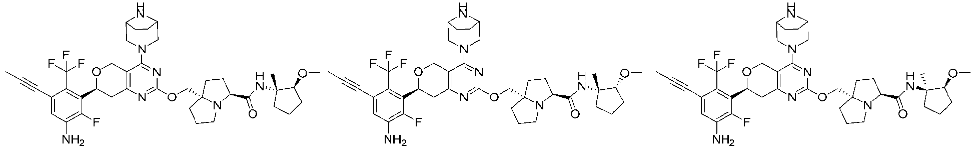

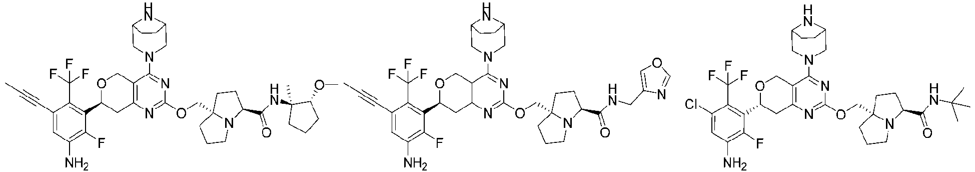

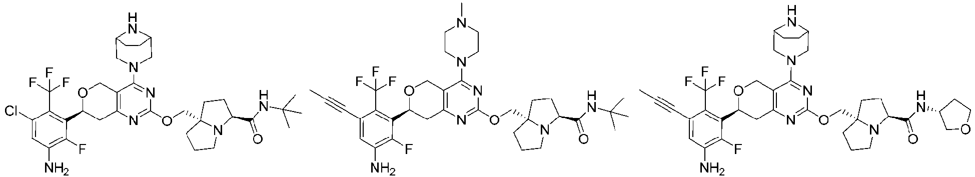

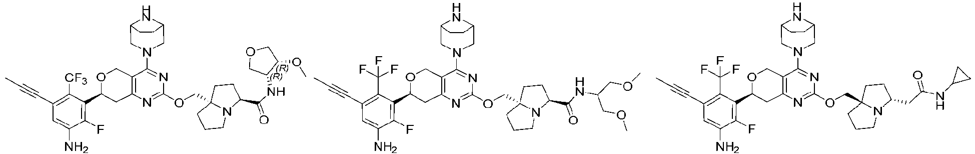

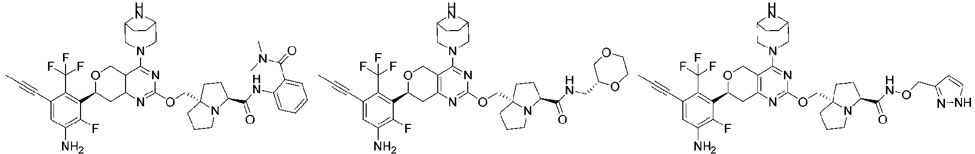

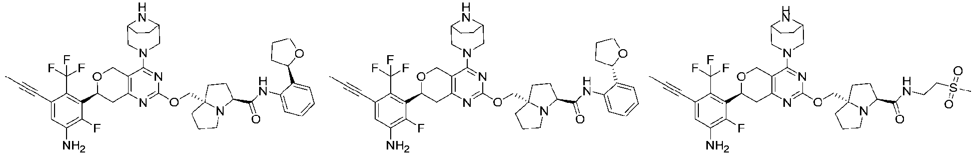

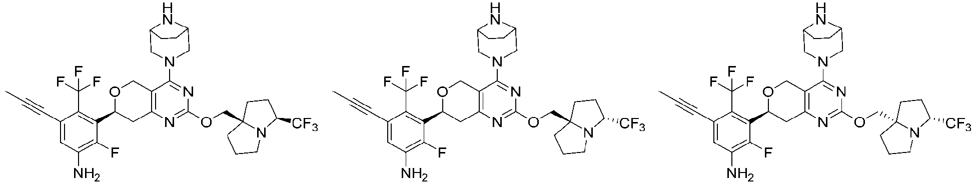

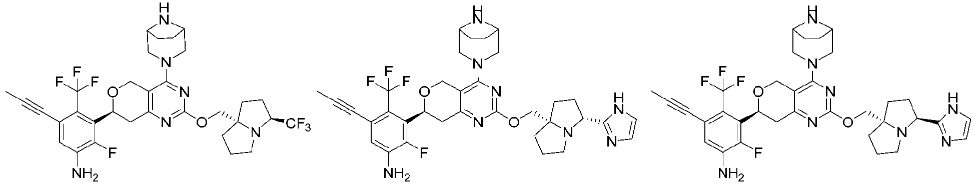

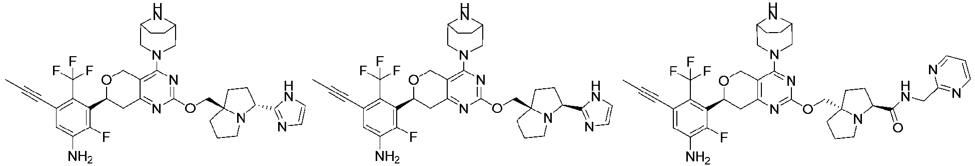

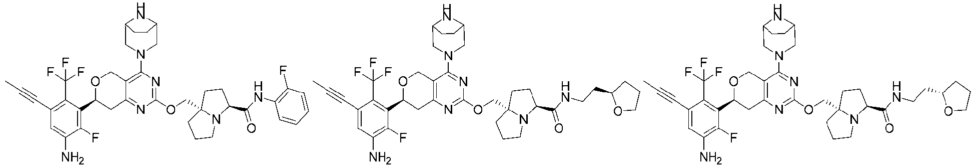

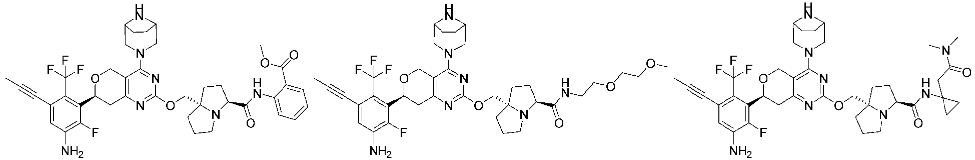

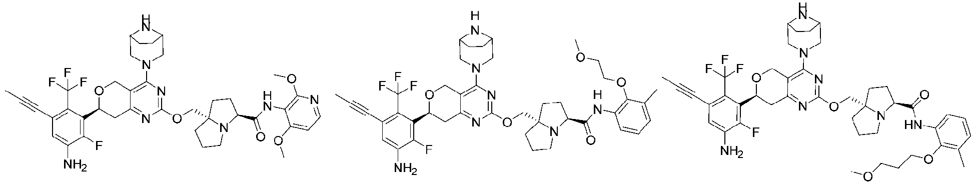

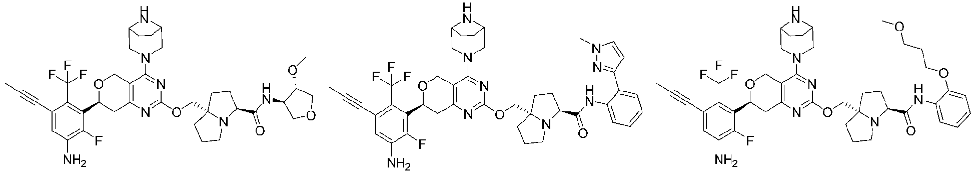

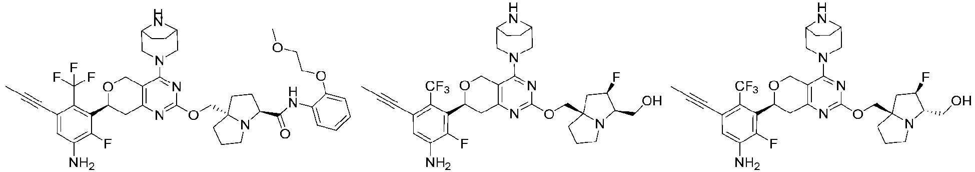

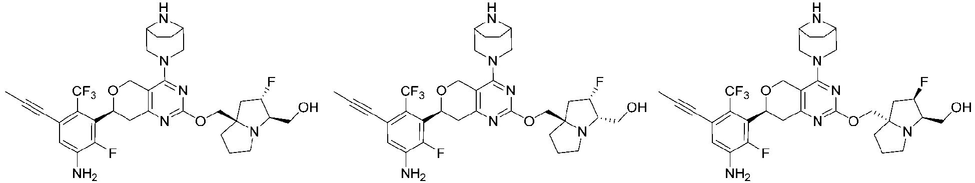

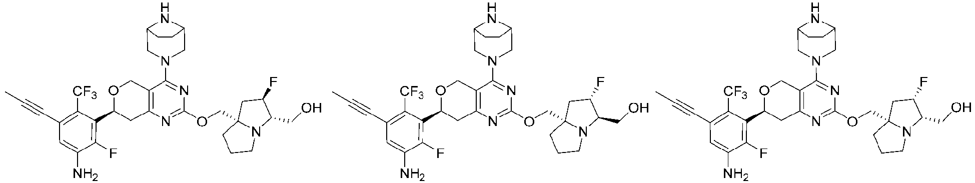

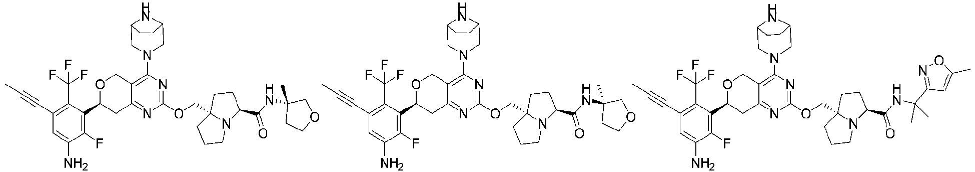

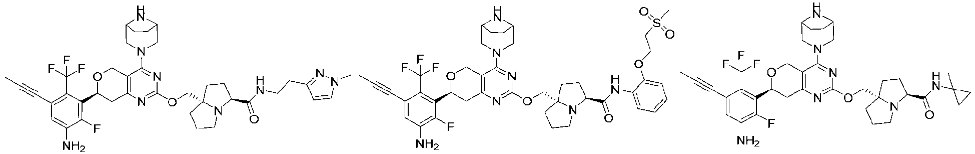

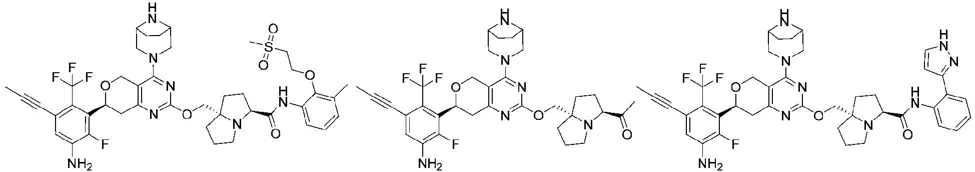

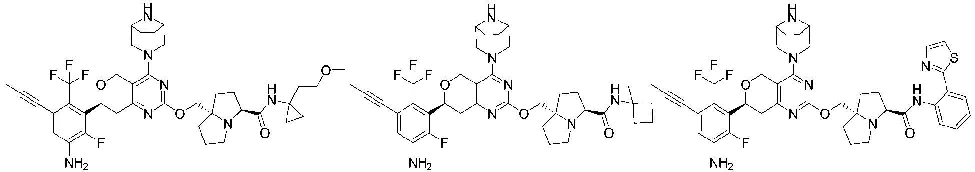

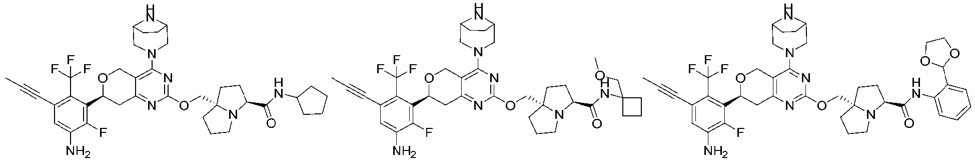

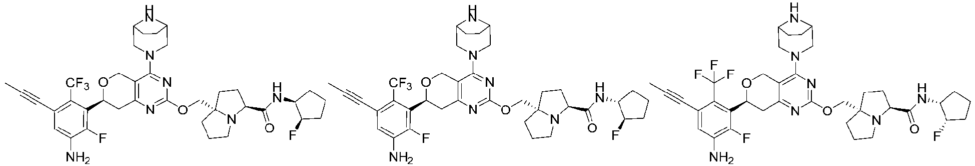

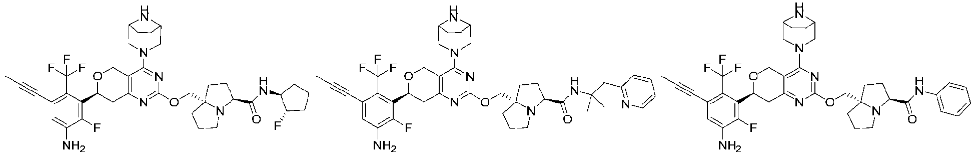

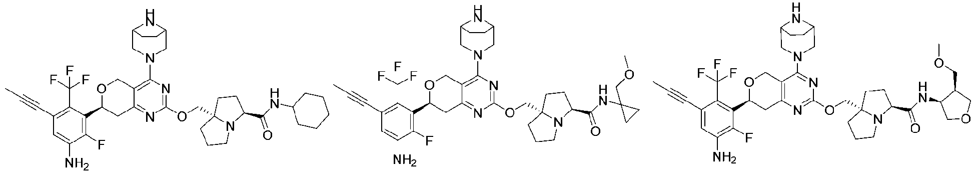

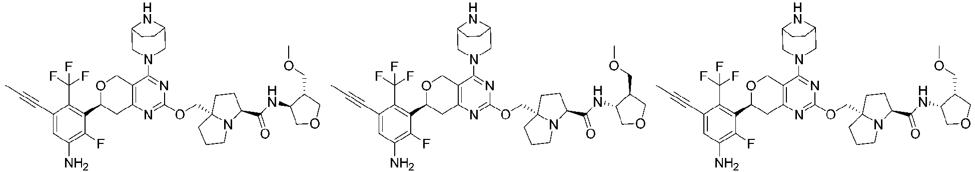

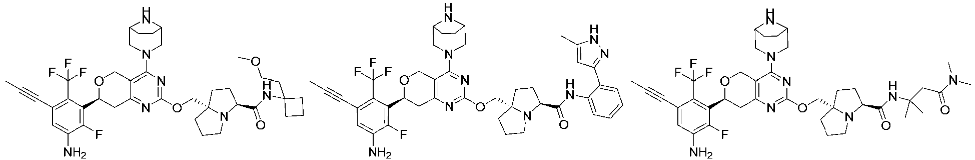

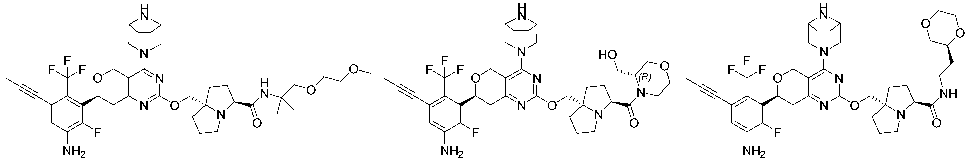

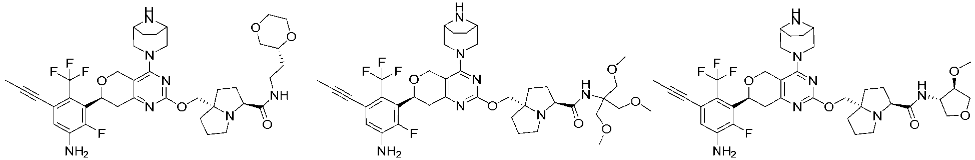

The present disclosure also provides the use of the above-mentioned compound, stereoisomers thereof or pharmaceutically acceptable salts thereof in the manufacture of a medicament for treating a KRAS mutation-associated disease or condition.

The present disclosure also provides the use of the above-mentioned compound, stereoisomers thereof or pharmaceutically acceptable salts thereof in the manufacture of a medicament for treating a KRAS mutation-associated disease or condition.

The present disclosure also provides the use of the above-mentioned compound, stereoisomers thereof or pharmaceutically acceptable salts thereof for treating a KRAS mutation-associated disease or condition.

The present disclosure also provides the above-mentioned compound, stereoisomers thereof or pharmaceutically acceptable salts thereof for use in the treatment of a KRAS mutation-associated disease or condition.

The present disclosure also provides a method for treating a KRAS mutation-associated disease or condition, comprising administering the above-mentioned compound, stereoisomers thereof or pharmaceutically acceptable salts thereof to a subject in need thereof.

In some embodiments of the present disclosure, the KRAS mutation is a KRAS^{G12D} mutation.

The present disclosure also provides the following biological test method:

### Test Method 1. GP2D Cell p-ERK Inhibition Test

### 1. Purpose

Using the HTRF method, compounds that can effectively inhibit p-ERK in GP2D cells with KRAS^{G12D} mutation were screened out.

### 2. Experimental Procedure

1). GP2D cells were inoculated in a transparent 96-well cell culture plate with 80 µL of cell suspension per well (containing 8,000 cells per well). The plate was placed in a CO₂ incubator and incubated overnight at 37°C.
2). 2 µL of compound was added to 78 µL of cell culture medium, and mixed thoroughly; then 20 µL of compound solution was added to the corresponding wells in the cell plate. The cell plate was returned to the CO₂ incubator and incubated for additional 1 hour.
3). After incubation, the cell supernatant was discarded and 50 µL of 1X cell lysis buffer was added to each well. The plate was incubated at room temperature with shaking for 30 minutes.
4). Phospho-ERK1/2 Eu Cryptate antibody and Phospho-ERK1/2 d2 antibody were subject to 20-folded dilution with the detection buffer.
5). 16 µL of cell lysate supernatant was transferred to each well in a new 384-well white microplate. Additional 2 µL of Phospho-ERK1/2 Eu Cryptate antibody dilution and 2 µL of Phospho-ERK1/2 d2 antibody dilution were added, and the plate was incubated at room temperature for at least 4 hours.
6). After incubation, a multi-label analyzer was used to read the HTRF with excitation: 320nm, emission: 615nm, 665nm;
7). IC₅₀ of the test compound was calculated.

### Test Method 2. AGS Cell p-ERK Inhibition Test

### 1. Purpose

Using the HTRF method, compounds that can effectively inhibit p-ERK in AGS cells with KRAS^{G12D} mutation were screened.

### 2. Experimental Procedure

1). AGS cells were inoculated in a transparent 96-well cell culture plate, with 80 µL of cell suspension per well (containing 10,000 cells per well). The plate was placed in a CO₂ incubator and incubated overnight at 37°C.
2). After incubation, the cell supernatant was discarded and 80 µL of culture medium containing 0.02% serum was added to each well. The cell plate was placed in a CO₂ incubator and incubated overnight at 37°C.
3). 2 µL of compound was added to 78 µL of cell culture medium, and mixed thoroughly; then 20 µL of compound solution was added to the corresponding well in the cell plate. The cell plate was returned to the CO₂ incubator and incubated for another 3 hours.
4). After incubation, the cell supernatant was discarded and 50 µL of 1X cell lysis buffer was added to each well. The plate was incubated at room temperature with shaking for 30 minutes.
5). Phospho-ERK1/2 Eu Cryptate antibody and Phospho-ERK1/2 d2 antibody were subject to 20-folded dilution with the detection buffer.
6). 16 µL of cell lysate supernatant was transferred to each well in a new 384-well white microplate. 2 µL of Phospho-ERK1/2 Eu Cryptate antibody dilution and 2 µL of Phospho-ERK1/2 d2 antibody dilution were then added, and incubated at room temperature for at least 4 hours.
7). After incubation, a multi-label analyzer was used to read the HTRF with excitation: 320nm, emission: 615nm, 665nm;
8). IC₅₀ of the test compound was calculated.

### Test Method 3. Anti-cell Proliferation Effect of compounds in Tumor Cell Line AsPC-1

### Purpose of the experiment

In this study, the inhibitory effect of compounds on cell proliferation was investigated by detecting their effects on in vitro cell activity in the KRAS^{G12D} mutant tumor cell line AsPC-1.

### Experimental Materials

Cell line: AsPC-1; Tumor type: Pancreatic cancer; Growth characteristics: Adherent growth; Culture method: RPMI 1640 + 10% FBS
Ultra Low Cluster-96 well plate (Corning-7007)
Greiner CELLSTAR 96-well Plate (#655090)
Promega CellTiter-Glo 3D Luminescent Cell Viability Assay Kit (Promega-G9683)
2104-10 EnVision plate reader, PerkinElmer
RPMI 1640, DMEM, PBS (phosphate buffered saline), FBS (fetal bovine serum), antibiotic-antimycotic (antibiotic-antifungal drug), L-glutamine (L-Gln), DMSO (dimethyl sulfoxide)

### Experimental methods and steps

### Cell culture

Tumor cell lines were cultured in a 37°C, 5% CO₂ incubator according to the culture conditions indicated in the culture methods. Cells were passaged regularly and cells in the logarithmic growth phase were used for plating.

### Cell plating

Cells were stained with trypan blue and viable cells were counted.

The cell concentration was adjusted to an appropriate level.

### Cell line: AsPC-1; Density (per well): 7000 cells.

135 µL of cell suspension was added to each well of a ULA culture plate, and the same volume of culture medium without cells was added to the blank control well.

Immediately after plating, the ULA culture plate was centrifuged at room temperature for 10 minutes at 1000 rpm. Note: after centrifugation, it should be careful to avoid any unnecessary agitation during subsequent operations.

The culture plate was incubated overnight in an incubator at 37°C, 5% CO₂, and 100% relative humidity.

### Preparation of 10X compound working solution and compound treatment of cells (Day 1)

After preparing the 10X compound working solution (DMSO 10X working solution), 15 µL of the 10X compound working solution was added to each well of a ULA culture plate. 15 µL of a DMSO-cell culture medium mixture was added to the vehicle control and blank control.

The 96-well cell plate was returned to the incubator and cultured for 120 hours.

The cell sphere formation was observed every day until the end of the experiment.

### CellTiter-Glo luminescent cell viability assay (day 5)

The following steps were performed according to the instructions of the Promega CellTiter-Glo 3D Luminescent Cell Viability Assay Kit (Promega # G9683).

150 µL (equal to the volume of cell culture medium in each well) of CellTiter-Glo 3D Reagent was added to each well. The cell plate was wrapped with aluminum foil to keep it in the dark.

The culture plate was shaken on an orbital shaker for 5 minutes.

The mixture in the well was carefully pipetted up and down 10 times to mix evenly. Ensure that the cell spheroids were fully dissociated before proceeding to the next step.

The solution in the ULA culture plate was then transferred to a black bottom culture plate (#655090) and placed at room temperature for 25 minutes to stabilize the luminescent signal.

Luminescent signals were detected on a 2104 EnVision plate reader.

### Data Analysis

The inhibition rate (IR) of the test compound was calculated by the following formula: IR (%) = (1 - (RLU compound - RLU blank control) / (RLU vehicle control - RLU blank control)) * 100%. The inhibition rate at different compound concentrations was calculated in Excel. GraphPad Prism software was then used to plot the inhibition curves and calculate relevant parameters, including minimum inhibition rate, maximum inhibition rate, and IC₅₀.

### Test Method 4. Pharmacokinetic Study of the Test compounds in CD-1 Mice after Oral and Intravenous Administration

### Purpose of the experiment

The *in vivo* pharmacokinetics were tested in CD-1 mice administrated with the compound orally and intravenously.

### Experimental procedures

The test compound was mixed with a 5% DMSO + 95% (10% HP-β-CD) aqueous solution, vortexed, and sonicated to prepare a 0.5 mg/mL clear solution (intravenous) or a 3 mg/mL clear solution (oral). The solution was then filtered through a microporous filter for later use. 7- to 10-week-old male CD-1 mice were selected and administered the candidate compound solution intravenously or orally. Whole blood was collected at defined intervals to prepare plasmas. The drug concentrations were analyzed by LC-MS/MS, and pharmacokinetic parameters were calculated using Phoenix WinNonlin software (Pharsight, USA).

### Technical Effects

The compound of the present disclosure has good cell proliferation inhibitory activity on KRAS^{G12D} mutant cells and significant inhibitory effect on p-ERK in KRAS^{G12D} mutant cells.

### Related definitions

Unless otherwise specified, the following terms and phrases used herein are intended to have the following meanings. A specific term or phrase should not be considered indefinite or unclear in the absence of a particular definition, but should be understood in the conventional sense. When a trade name appears herein, it is intended to refer to its corresponding commodity or active ingredient thereof.

The term "pharmaceutically acceptable" as used herein is intended to refer to those compounds, materials, compositions, and/or dosage forms, which are, within the scope of sound medical judgment, suitable for use in contact with the tissues of human beings and animals without excessive toxicity, irritation, allergic reaction, or other problems or complications, commensurate with a reasonable benefit/risk ratio.

The term "pharmaceutically acceptable salt" means a salt of compounds disclosed herein that is prepared by reacting the compound having a specific substituent disclosed herein with a relatively non-toxic acid or base. When compounds disclosed herein contain a relatively acidic functional group, a base addition salt can be obtained by bringing the compound into contact with a sufficient amount of base in a pure solution or a suitable inert solvent. When compounds disclosed herein contain a relatively basic functional group, an acid addition salt can be obtained by bringing the compound into contact with a sufficient amount of acid in a pure solution or a suitable inert solvent. Certain specific compounds disclosed herein contain both basic and acidic functional groups and can be converted to any base or acid addition salt.

The pharmaceutically acceptable salt disclosed herein can be prepared from the parent compound that contains an acidic or basic moiety by conventional chemical methods. Generally, such salt can be prepared by reacting the free acid or base form of the compound with a stoichiometric amount of an appropriate base or acid in water or an organic solvent or a mixture thereof.

Compounds disclosed herein may be present in a specific geometric or stereoisomeric form. The present disclosure contemplates all such compounds, including cis and trans isomers, (-)- and (+)-enantiomers, (*R*)*-* and (*S*)-enantiomers, diastereoisomer, (*D*)-isomer, (*L*)-isomer, and a racemic mixture and other mixtures, for example, a mixture enriched in enantiomer or diastereoisomer, all of which are encompassed within the scope disclosed herein. The substituent such as alkyl may have an additional asymmetric carbon atom. All these isomers and mixtures thereof are encompassed within the scope disclosed herein.

Compounds disclosed herein may contain an unnatural proportion of atomic isotopes at one or more of the atoms that make up the compounds. For example, a compound may be labeled with a radioisotope such as tritium (³H), iodine-125 (¹²⁵I) or C-14(¹⁴C). For another example, hydrogen can be replaced by heavy hydrogen to form a deuterated drug. The bond between deuterium and carbon is more robust than that between ordinary hydrogen and carbon. Compared with undeuterated drugs, deuterated drugs have advantages of reduced toxic side effects, increased drug stability, enhanced efficacy, and prolonged biological half-life of drugs. All changes in the isotopic composition of compounds disclosed herein, regardless of radioactivity, are included within the scope of the present disclosure.

The term "optional" or "optionally" means that the subsequent event or condition may occur but not requisite, that the term includes the instance in which the event or condition occurs and the instance in which the event or condition does not occur.

The term "substituted" means that one or more than one hydrogen atoms on a specific atom are substituted by a substituent, including deuterium and hydrogen variants, as long as the valence of the specific atom is normal and the substituted compound is stable. When the substituent is oxo (i.e., =O), it means two hydrogen atoms are substituted. The term "optionally substituted" means an atom can be substituted by a substituent or not, unless otherwise specified, the species and number of the substituent may be arbitrary so long as being chemically achievable.

When any variable (such as R) occurs in the constitution or structure of the compound more than once, the definition of the variable at each occurrence is independent. Thus, for example, if a group is substituted by 0-2 R, the group can be optionally substituted by up to two R, wherein the definition of R at each occurrence is independent. Moreover, a combination of the substituent and/or the variant thereof is allowed only when the combination results in a stable compound.

When the number of a linking group is 0, such as -(CRR)₀-, it means that the linking group is a single bond.

When one of variables is a single bond, it means that the two groups linked by the single bond are connected directly. For example, when L in A-L-Z represents a single bond, the structure of A-L-Z is actually A-Z.

When an enumerated linking group does not indicate its linking direction, its linking direction is arbitrary. For example, when the linking group L in 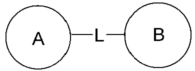 is -M-W-, the -M-W- can be linked to the ring A and the ring B in the same direction as the reading order from left to right to constitute 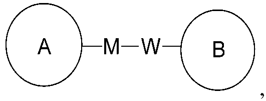 or can be linked to the ring A and the ring B in the reverse direction as the reading order from left to right to constitute 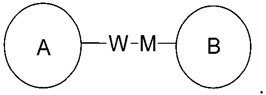 A combination of the linking groups, substituents and/or variants thereof is allowed only when such combination can result in a stable compound.

Unless otherwise specified, when a group has one or more connectable sites, any one or more sites of the group can be connected to other groups through chemical bonds. Where the connection position of the chemical bond is variable, and there is H atom(s) at a connectable site(s), when the connectable site(s) having H atom(s) is connected to the chemical bond, the number of H atom(s) at this site will correspondingly decrease as the number of the connected chemical bond increases, and the group will become a group of corresponding valence. The chemical bond between the site and other groups can be represented by a straight solid bond ( 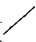 ), a straight dashed bond ( 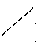 ), or a wavy line ( 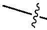 ). For example, the straight solid bond in -OCH₃ indicates that the group is connected to other groups through the oxygen atom in the group; the straight dashed bond in 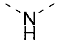 indicates that the group is connected to other groups through two ends of the nitrogen atom in the group; the wavy line in 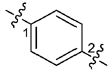 indicates that the group is connected to other groups through the 1- and 2-carbon atoms in the phenyl group; 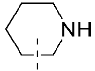 indicates that any connectable site on the piperidinyl group can be connected to other groups through one chemical bond, including at least four connection ways, 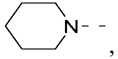 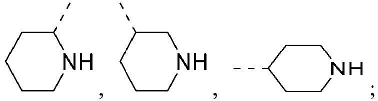 even if a H atom is drawn on -N-, 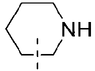 still includes the connection way of 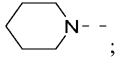 it's just that when one chemical bond is connected, the H at this site will be reduced by one, and the group will become the corresponding monovalent piperidinyl group.

Unless otherwise specified, a wedged solid bond ( 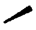 ) and a wedged dashed bond ( 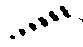 ) indicate the absolute configuration of a stereocenter; a straight solid bond ( 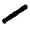 ) and a straight dashed bond ( 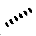 ) indicate the relative configuration of a stereocenter; a wavy line ( 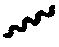 ) indicates a wedged solid bond ( 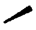 ) or a wedged dashed bond ( 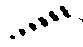 ); or a wavy line ( 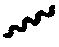 ) indicates a straight solid bond ( 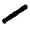 ) or a straight dashed bond ( 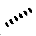 ).

Unless otherwise specified, when a double bond structure exists in a compound, such as a carbon-carbon double bond, a carbon-nitrogen double bond, and a nitrogen-nitrogen double bond, and each atom on the double bond is connected to two different substituents (in a double bond involving a nitrogen atom, a lone pair of electrons on the nitrogen atom is regarded as a substituent connected thereto), if the atom on the double bond and its substituent in the compound are represented by 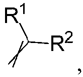 it represents a mixture of the two isomers of the compound.

Unless otherwise specified, the term "tautomer" or "tautomeric form" means that at room temperature, isomers with different functional groups are in dynamic equilibrium and can be rapidly converted to each other. If tautomerism is possible (such as in solution), chemical equilibrium of tautomerism can be achieved. For example, proton tautomers (also called prototropic tautomers) include interconversions through proton migration, such as keto-enol isomerization and imine-enamine isomerization. Valence isomers are tautomers that interconvert by reorganization of some of the bonding electrons. A specific example of keto-enol tautomerization is the interconversion between pentane-2,4-dione and 4-hydroxypent-3-en-2-one.

Unless otherwise indicated, the terms "enriched in one isomer", "isomerically enriched", "enriched in one enantiomer" or "enantiomerically enriched" mean that the content of one isomer or enantiomer is less than 100%, and the content of that isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise indicated, the term "isomer excess" or "enantiomeric excess" refers to the difference between the relative percentages of two isomers or two enantiomers. For example, if the content of one isomer or enantiomer is 90% and the content of the other isomer or enantiomer is 10%, the isomer or enantiomeric excess (ee value) is 80%.

Unless otherwise specified, Cₙ₋ₙ₊ₘ or Cₙ-Cₙ₊ₘ includes any specific case of n to n+m carbons, for example, C₁₋₁₂ includes C₁, C₂, C₃, C₄, C₅, C₆, C₇, C₈, C₉, C₁₀, C₁₁ and C₁₂, also includes any range from n to n+m, for example, C₁₋₁₂ includes C₁₋₃, C₁₋₆, C₁₋₉, C₃₋₆, C₃₋₉, C₃₋₁₂, C₆₋₉, C₆₋₁₂ and C₉₋₁₂, etc.; similarly, n membered to n+m membered indicates that the number of atoms on a ring is n to n+m, for example, 3-12 membered ring includes 3-membered ring, 4-membered ring, 5-membered ring, 6-membered ring, 7-membered ring, 8-membered ring, 9-membered ring, 10-membered ring, 11-membered ring, and 12-membered ring, also includes any range from n to n+m, for example, 3-12 membered ring includes 3-6 membered ring, 3-9 membered ring, 5-6 membered ring, 5-7 membered ring, 6-7 membered ring, 6-8 membered ring, and 6-10 membered ring, and the like.

Unless otherwise specified, the term "halo" or "halogen", by itself or as part of another substituent, means a fluorine, chlorine, bromine, or iodine atom.

Unless otherwise specified, the term "C₁₋₃ alkyl" is used to represent a linear or branched saturated hydrocarbon group consisting of 1 to 3 carbon atoms. The C₁₋₃ alkyl includes C₁₋₂ alkyl, C₂₋₃ alkyl, etc. It may be monovalent (such as methyl), divalent (such as methylene) or multivalent (such as methenyl). Examples of the C₁₋₃ alkyl include, but are not limited to, methyl (Me), ethyl (Et), propyl (including n-propyl and isopropyl), and the like.

Unless otherwise specified, the term "C₁₋₆ alkyl" is used to refer to a linear or branched saturated hydrocarbon group consisting of 1 to 6 carbon atoms. The C₁₋₆ alkyl group includes C₁₋₅, C₁₋₄, C₁₋₃, C₁₋₂, C₂₋₆, C₂₋₄, C₆, and C₅ alkyl groups, etc.; and can be monovalent (such as methyl), divalent (such as methylene), or polyvalent (such as methine). Examples of C₁₋₆ alkyl groups include, but are not limited to, methyl (Me), ethyl (Et), propyl (including n-propyl and isopropyl), butyl (including n-butyl, isobutyl, s-butyl, and t-butyl), pentyl (including n-pentyl, isopentyl, and neopentyl), hexyl, etc.

Unless otherwise specified, the term "C₁₋₃ alkoxy" refers to an alkyl group containing 1 to 3 carbon atoms that is attached to the rest of a molecule via an oxygen atom. The C₁₋₃ alkoxy group includes C₁₋₂, C₂₋₃, C₃ and C₂ alkoxy groups, etc. Examples of C₁₋₃ alkoxy groups include, but are not limited to, methoxy, ethoxy, propoxy (including n-propoxy and isopropoxy), and the like.

Unless otherwise specified, the term "C₁₋₃ alkylamino" refers to -NH-C₁₋₃ alkyl, including C₁₋₂, C₃, and C₂ alkylamino, etc. Examples of C₁₋₃ alkylamino include, but are not limited to, -NHCH₃, -NHCH₂CH₃, - NHCH₂CH₂CH₃, -NHCH₂(CH₃)₂, etc.

Unless otherwise specified, the term "di-C₁₋₃ alkylamino" refers to -N(C₁₋₃ alkyl)₂, including di-C₁₋₂, di-C₃, and di-C₂ alkylamino, etc. Examples of di-C₁₋₃ alkylamino include, but are not limited to, -N(CH₃)₂ and - N(CH₃)CH₂CH₃.

Unless otherwise specified, "C₂₋₄ alkenyl" is used to refer to a linear or branched hydrocarbon group consisting of 2 to 4 carbon atoms containing at least one carbon-carbon double bond, which may be located at any position in the group. The C₂₋₄ alkenyl group includes C₂₋₃, C₄, C₃, and C₂ alkenyl groups, etc., and the C₂₋₄ alkenyl group may be monovalent, divalent, or polyvalent. Examples of C₂₋₄ alkenyl groups include, but are not limited to, ethenyl, propenyl, butenyl, butadienyl, and the like. Unless otherwise specified, "C₂₋₃ alkenyl" is used to refer to a linear or branched hydrocarbon group consisting of 2 to 3 carbon atoms containing at least one carbon-carbon double bond, which may be located at any position in the group. The C₂₋₃ alkenyl group includes C₃ and C₂ alkenyl groups, and the C₂₋₃ alkenyl group may be monovalent, divalent, or polyvalent. Examples of C₂₋₃ alkenyl groups include, but are not limited to, ethenyl, propenyl, and the like.

Unless otherwise specified, "C₂₋₄ alkynyl" refers to a linear or branched hydrocarbon group consisting of 2 to 4 carbon atoms containing at least one carbon-carbon triple bond, which may be located at any position within the group. Examples of C₂₋₄ alkynyl groups include C₂₋₃, C₄, C₃, and C₂ alkynyl groups, etc. These groups may be monovalent, divalent, or polyvalent. Examples of C₂₋₄ alkynyl groups include, but are not limited to, ethynyl, propynyl, butynyl, and the like.

Unless otherwise specified, "C₃₋₅ cycloalkyl" refers to a saturated cyclic hydrocarbon group consisting of 3 to 5 carbon atoms, which is a monocyclic ring system. Such C₃₋₅ cycloalkyl groups include C₃₋₄ and C₄₋₅ cycloalkyl groups, etc., and may be monovalent, divalent, or polyvalent. Examples of C₃₋₅ cycloalkyl groups include, but are not limited to, cyclopropyl, cyclobutyl, and cyclopentyl, etc.

Unless otherwise specified, "C₃₋₁₀ cycloalkyl" refers to a saturated cyclic hydrocarbon group consisting of 3 to 10 carbon atoms, including monocyclic, bicyclic, and tricyclic ring systems, wherein bicyclic and tricyclic ring systems include spirocyclic, fused, and bridged rings. The C₃₋₁₀ cycloalkyl group includes C₃₋₈, C₃₋₆, C₃₋₅, C₄₋₁₀, C₄₋₈, C₄₋₆, C₄₋₅, C₅₋₈, or C₅₋₆, etc.; and can be monovalent, divalent, or polyvalent. Examples of C₃₋₁₀ cycloalkyl groups include, but are not limited to, cyclopropyl, cyclobutyl, cyclopentyl, cyclohexyl, cycloheptyl, norbornyl, [2.2.2]bicyclooctane, and the like.

Unless otherwise specified, "C₃₋₁₀ carbocyclyl" refers to a saturated or partially saturated cyclic hydrocarbon group consisting of 3 to 10 carbon atoms, including monocyclic, bicyclic, and tricyclic ring systems, wherein bicyclic and tricyclic ring systems include spirocyclic, fused, and bridged rings. The C₃₋₁₀ carbocyclyl includes C₃₋₈, C₃₋₆, C₃₋₅, C₄₋₁₀, C₄₋₈, C₄₋₆, C₄₋₅, C₅₋₈, or C₅₋₆, etc.; and can be monovalent, divalent, or polyvalent. Examples of C₃₋₁₀ carbocyclyl groups include, but are not limited to, cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), cycloheptyl (C₇), cycloheptenyl (C₇), cycloheptadienyl (C₇), cycloheptatrienyl (C₇), cyclooctyl (C₈), cyclooctenyl (C₈), bicyclo[2.2.1]heptyl (C₇), bicyclo[2.2.2]octyl (C₈), cyclononyl (C₉), cyclononenyl (C₉), cyclodecyl (C₁₀), cyclodecenyl (C₁₀), octahydro-1H-indenyl (C₉), decalinyl (C₁₀) or spiro[4.5]decyl (C₁₀).

Unless otherwise specified, the term "3- to 5-membered heterocycloalkyl" by itself or in combination with other terms refers to a saturated monocyclic group consisting of 3 to 5 ring atoms, 1, 2, 3 or 4 of which are heteroatoms independently selected from O, S and N, and the rest are carbon atoms, wherein the carbon atoms are optionally oxidized (i.e., C(O)), the nitrogen atoms are optionally quaternized, and the nitrogen and sulfur heteroatoms are optionally oxidized (i.e., NO and S(O)ₚ, where p is 1 or 2). In addition, with respect to such "3-to 5-membered heterocycloalkyl", a heteroatom may occupy the position at which the heterocycloalkyl is attached to the rest of the molecule. Such 3- to 5-membered heterocycloalkyls include 4-5 membered, 4 membered, and 5 membered heterocycloalkyls, etc. Examples of 3- to 5-membered heterocycloalkyl groups include, but are not limited to, azetidinyl, oxetanyl, thietanyl, pyrrolidinyl, pyrazolidinyl, imidazolidinyl, tetrahydrothiophenyl (including tetrahydrothiophen-2-yl and tetrahydrothiophen-3-yl, etc.), or tetrahydrofuranyl (including tetrahydrofuran-2-yl, etc.), etc.

Unless otherwise specified, the term "3- to 6-membered heterocycloalkyl" by itself or in combination with other terms refers to a saturated cyclic group consisting of 3 to 6 ring atoms, 1, 2, 3 or 4 of which are heteroatoms independently selected from O, S and N, and the rest are carbon atoms, wherein the carbon atoms are optionally oxidized (i.e., C(O)), the nitrogen atoms are optionally quaternized, and the nitrogen and sulfur heteroatoms are optionally oxidized (i.e., NO and S(O)ₚ, where p is 1 or 2). It includes monocyclic and bicyclic ring systems, wherein bicyclic ring systems include spirocyclic, fused and bridged rings. In addition, with respect to the "3- to 6-membered heterocycloalkyl", a heteroatom may occupy the position at which the heterocycloalkyl is connected to the rest of the molecule. The 3- to 6-membered heterocycloalkyl includes 4- to 6-membered, 5-to 6-membered, 4 membered, 5 membered and 6 membered heterocycloalkyls, etc. Examples of 3- to 6-membered heterocycloalkyl groups include, but are not limited to, azetidinyl, oxetanyl, thietanyl, pyrrolidinyl, pyrazolidinyl, imidazolidinyl, tetrahydrothiophenyl (including tetrahydrothiophen-2-yl and tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl and 3-piperidinyl, etc.), piperazinyl (including 1-piperazinyl and 2-piperazinyl, etc.), morpholinyl (including 3-morpholinyl and 4-morpholinyl, etc.), dioxanyl, dithianyl, isoxazolidinyl, isothiazolidinyl, 1,2-oxazinyl, 1,2-thiazinyl or hexahydropyridazinyl, etc.

Unless otherwise specified, the term "3- to 10-membered heterocycloalkyl" by itself or in combination with other terms refers to a saturated cyclic group consisting of 3 to 10 ring atoms, wherein 1, 2, 3 or 4 of the ring atoms are heteroatoms independently selected from O, S and N, and the rest are carbon atoms, wherein the carbon atoms are optionally oxidized (i.e., C(O)), the nitrogen atoms are optionally quaternized, and the nitrogen and sulfur heteroatoms are optionally oxidized (i.e., NO and S(O)ₚ, where p is 1 or 2). This includes monocyclic, bicyclic and tricyclic ring systems, wherein bicyclic and tricyclic ring systems include spirocyclic, fused and bridged rings. In addition, with respect to the term "3- to 10-membered heterocycloalkyl", a heteroatom may occupy the position at which the heterocycloalkyl group is attached to the rest of the molecule. The 3- to 10-membered heterocycloalkyl group includes 3- to 8-membered, 3- to 6-membered, 3- to 5-membered, 4- to 6-membered, 5- to 6-membered, 4 membered, 5 membered and 6 membered heterocycloalkyl groups, etc. Examples of 3- to 10-membered heterocycloalkyl groups include, but are not limited to, azetidinyl, oxetanyl, thietanyl, pyrrolidinyl, pyrazolidinyl, imidazolidinyl, tetrahydrothiophenyl (including tetrahydrothiophen-2-yl and tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl and 3-piperidinyl, etc.), piperazinyl (including 1-piperazinyl and 2-piperazinyl, etc.), morpholinyl (including 3-morpholinyl and 4-morpholinyl, etc.), dioxanyl, dithianyl, isoxazolidinyl, isothiazolidinyl, hexahydropyridazinyl, homopiperazinyl, homopiperidinyl or dioxepanyl, etc.

Unless otherwise specified, the terms "C₆₋₁₀ aromatic ring" and "C₆₋₁₀ aryl" are used interchangeably herein. The term "C₆₋₁₀ aromatic ring" or "C₆₋₁₀ aryl" refers to a cyclic hydrocarbon group composed of 6 to 10 carbon atoms and having a conjugated π electron system. It can be a monocyclic ring, a fused bicyclic ring, or a fused tricyclic ring system, wherein each ring is aromatic. It can be monovalent, divalent, or polyvalent. C₆₋₁₀ aryl groups include C₆₋₉, C₉, C₁₉, and C₆ aryl groups. Examples of C₆₋₁₀ aryl groups include, but are not limited to, phenyl and naphthyl (including 1-naphthyl and 2-naphthyl, etc.).

Unless otherwise specified, the terms "5- to 10-membered heteroaromatic ring" and "5- to 10-membered heteroaryl" may be used interchangeably. The term "5- to 10-membered heteroaryl" means a cyclic group having a conjugated pi electron system and consisting of 5 to 10 ring atoms, in which 1, 2, 3 or 4 ring atoms are heteroatoms independently selected from O, S and N, and the rest is carbon atoms. It may be a monocyclic, fused bicyclic or fused tricyclic ring system, wherein each ring is aromatic, and wherein the nitrogen atom is optionally quaternized and the nitrogen and sulfur heteroatoms are optionally oxidized (i.e., NO and S(O)ₚ, p is 1 or 2). A 5- to 10-membered heteroaryl can be attached to the rest of a molecule through a heteroatom or a carbon atom. The 5- to 10-membered heteroaryl group includes 5- to 8-membered, 5- to 7-membered, 5- to 6-membered, 5 membered and 6 membered heteroaryl groups, etc. Examples of the 5- to 10-membered heteroaryl include, but are not limited to, pyrrolyl (including N-pyrrolyl, 2-pyrrolyl, 3-pyrrolyl, and the like), pyrazolyl (including 2-pyrazolyl and 3-pyrazolyl, and the like), imidazolyl (including N-imidazolyl, 2-imidazolyl, 4-imidazolyl, and 5-imidazolyl, and the like), oxazolyl (including 2-oxazolyl, 4-oxazolyl, and 5-oxazolyl, and the like), triazolyl (1H-1,2,3-triazolyl, 2H-1,2,3-triazolyl, 1H-1,2,4-triazolyl and 4H-1,2,4-triazolyl, and the like), tetrazolyl, isoxazolyl (3-isoxazolyl, 4-isoxazolyl and 5-isoxazolyl, and the like), thiazolyl (including 2-thiazolyl, 4-thiazolyl and 5-thiazolyl, and the like), furyl (including 2-furyl and 3-furyl, and the like), thienyl (including 2-thienyl and 3-thienyl, and the like), pyridyl (including 2-pyridyl, 3-pyridyl and 4-pyridyl, and the like), pyrazinyl, pyrimidinyl (including 2-pyrimidinyl and 4-pyrimidinyl, and the like), benzothiazolyl (including 5-benzothiazolyl, and the like), purinyl, benzimidazolyl (including 2-benzimidazolyl, and the like), benzoxazolyl, indolyl (including 5-indolyl, and the like), isoquinolyl (including 1-isoquinolyl, 5-isoquinolyl, and the like), quinoxalinyl (including 2-quinoxalinyl, 5-quinoxalinyl, and the like) or quinolyl (including 3-quinolyl, 6-quinolyl, and the like).

Unless otherwise specified, the terms "5- to 6-membered heteroaromatic ring" and "5- to 6-membered heteroaryl" are used interchangeably herein. The term "5- to 6-membered heteroaryl" refers to a monocyclic group consisting of 5 to 6 ring atoms with a conjugated π electron system, wherein 1, 2, 3 or 4 of the ring atoms are heteroatoms independently selected from O, S and N, and the rest are carbon atoms. The nitrogen atom is optionally quaternized, and the nitrogen and sulfur heteroatoms are optionally oxidized (i.e., NO and S(O)ₚ, where p is 1 or 2). The 5- to 6-membered heteroaryl group may be attached to the rest of the molecule via a heteroatom or a carbon atom. The 5- to 6-membered heteroaryl group includes both 5-membered and 6-membered heteroaryl groups. Examples of the 5- to 6-membered heteroaryl group include, but are not limited to, pyrrolyl (including *N*-pyrrolyl, 2-pyrrolyl and 3-pyrrolyl, etc.), pyrazolyl (including 2-pyrazolyl and 3-pyrazolyl, etc.), imidazolyl (including N-imidazolyl, 2-imidazolyl, 4-imidazolyl and 5-imidazolyl, etc.), oxazolyl (including 2-oxazolyl, 4-oxazolyl and 5-oxazolyl, etc.), triazolyl (1*H*-1,2,3-triazolyl, 2*H*-1,2,3-triazolyl, 1*H-*1,2,4-triazolyl and 4*H*-1,2,4-triazolyl, etc.), tetrazolyl, isoxazolyl (3-isoxazolyl, 4-isoxazolyl and 5-isoxazolyl, etc.), thiazolyl (including 2-thiazolyl, 4-thiazolyl and 5-thiazolyl, etc.), furyl (including 2-furyl and 3-furyl, etc.), thienyl (including 2-thienyl and 3-thienyl, etc.), pyridyl (including 2-pyridyl, 3-pyridyl and 4-pyridyl, etc.), pyrazinyl or pyrimidinyl (including 2-pyrimidinyl and 4-pyrimidinyl, etc.).

The compounds of the present disclosure can be prepared by a variety of synthetic methods well known to those skilled in the art, including the specific embodiments listed below, embodiments formed by combining them with other chemical synthesis methods, and equivalents well known to those skilled in the art. Alternative embodiments include but are not limited to the examples of the present disclosure.

The structures of the compounds of the present disclosure can be confirmed using conventional methods well known to those skilled in the art. If the present disclosure relates to the absolute configuration of a compound, such absolute configuration can be confirmed using conventional techniques in the art. For example, single crystal X-ray diffraction (SXRD) is performed by collecting diffraction intensity data from a cultured single crystal using a Bruker D8 venture diffractometer, using CuKα radiation as light source and a φ/ω scanning mode. After collecting relevant data, the crystal structure can be further analyzed using a direct method (Shelxs97) to confirm the absolute configuration.

The solvents used in the present disclosure are commercially available. The present disclosure uses the following abbreviations: NaOH represents sodium hydroxide; DMF represents N,N-dimethylformamide; THF represents tetrahydrofuran; 2-MeTHF represents 2-methyltetrahydrofuran; DCM represents dioxane; EA represents ethyl acetate; DIPEA represents N,N-diisopropylethylamine; DCM represents dichloromethane; m-CPBA represents m-chloroperbenzoic acid; Boc₂O represents di-tert-butyl carbonic anhydride; LiAlH₄ represents lithium aluminum tetrahydride; MNO₂ represents manganese dioxide; NBS represents N-bromosuccinimide; TMP represents trimethylolpropane; n-BuLi represents n-butyllithium; TFA represents trifluoroacetic acid; Xphos Pd G4 represents: (SP-4-3)-[dicyclohexyl[2',4',6'-tri(isopropyl)[1,1'-biphenyl]-2-yl]phosphine](methanesulfonic acid)[2'-(methylamino)[1,1'-biphenyl]-2-yl]palladium; AgNO₃ represents silver nitrate; NCS represents N-chlorosuccinimide.

Compounds were named according to conventional nomenclature in the art or using ChemDraw^{®} software. Commercially available compounds were named according to the supplier's catalog name.

### DETAILED DESCRIPTION

The present disclosure is described in detail below by way of examples, but the examples are not intended to limit the present disclosure in any way. While the present disclosure has been described in detail herein, and specific embodiments thereof have been disclosed, it will be apparent to those skilled in the art that various changes and modifications can be made to the specific embodiments of the present disclosure without departing from the spirit and scope of the present disclosure.

### Example 1

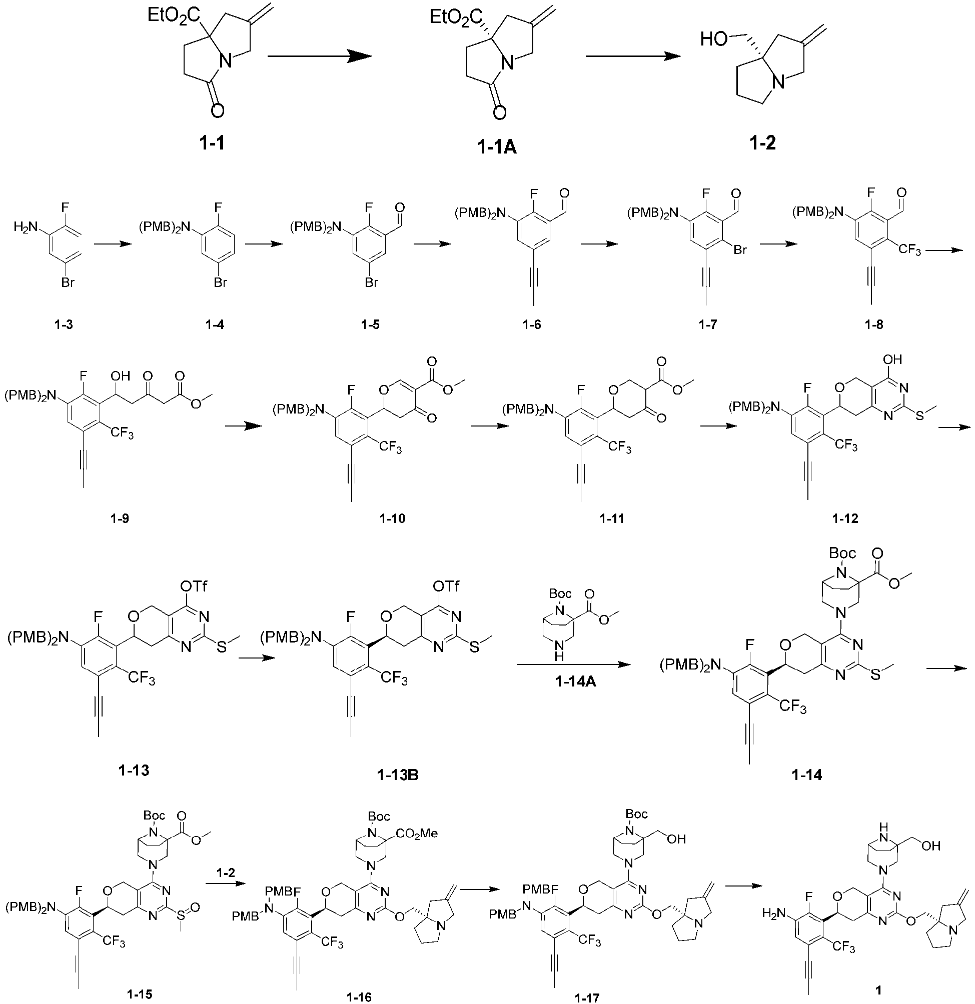

### Step 1

Compound 1-1 was subjected to preparative supercritical liquid chromatography (SFC) (Chromatographic column: ChiralPak IH, 250*50mm, 10µm; mobile phase: A: supercritical carbon dioxide, B: [0.1% ammonia-ethanol]; B%: 20%-20%, run time 3.7min) to obtain compound **1-1A.** SFC analysis method (Chromatographic column: Chiralpak IH-3, 100×4.6mm I.D., 3µm; mobile phase: A (supercritical carbon dioxide) and B (ethanol, containing 0.1% isopropylamine); gradient: B% = 10-50%, 4 min; flow rate: 3.4 mL/min; wavelength: 220nm; pressure: 2000psi). Compound 1-1A: Rt = 1.489 min and ee value of 98.82%. ¹H NMR (400 MHz, CDCl₃) δ = 4.99 - 4.86 (m, 2H), 4.26 - 3.95 (m, 3H), 3.59 (m, 1H), 3.00 - 2.88 (m, 1H), 2.87 - 2.12 (m, 4H), 1.91 (s, 1H), 1.20 - 1.08 (m, 3H).

### Step 2

Lithium aluminum tetrahydride (1.55 g, 40.15 mmol) was dissolved in anhydrous tetrahydrofuran (30 mL). The mixture was cooled to 0°C. Under nitrogen, a solution of compound **1-1A** (2.8 g, 13.38 mmol) in anhydrous tetrahydrofuran (20 mL) was added. The reaction was reacted at 70°C for 1 hour. At 0°C, 1.5 mL of water was added to the reaction, followed by 1.5 mL of 15% NaOH solution and 4.5 mL of water. The mixture was stirred for 20 minutes. The reaction mixture was filtered. The filter cake was washed with 10 mL of tetrahydrofuran, and the filtrate was concentrated to obtain compound **1-2.** ¹H NMR (400 MHz, CDCl₃) δ = 4.99 - 4.86 (m, 2H), 4.26 - 3.95 (m, 3H), 3.59 (m, 1H), 3.00 - 2.88 (m, 1H), 2.74 - 2.27 (m, 4H), 1.91 (s, 1H), 1.20 - 1.08 (m, 3H).

### Step 3

Compound **1-3** (480 g, 2.53 mol) was weighed and DMF (2500 mL) was added. 4-methoxybenzyl chloride (5.18 mol, 702.79 mL), potassium carbonate (872.82 g, 6.32 mol), and potassium iodide (419.35 g, 2.53 mol) were then added. The mixture was reacted at 65°C for 2 hours. Water (1000 mL) was added, and the mixture was extracted with ethyl acetate (1000 mL x 3). The organic phase was concentrated under reduced pressure to obtain compound **1-4.** MS m/z = 430.0 [M+H]⁺.

### Step 4

Compound 2,2,6,6-tetramethylpiperidine (220.59 g, 1.56 mol, 265.13 mL) was weighed, and THF (3000 mL) was added. n-Butyllithium (2.5 M, 499.73 mL) was added at -5°C, and the mixture was stirred for 0.5 h. The temperature was then lowered to -60°C and compound **1-4** (280 g, 624.67 mmol) was added. The mixture was stirred for 0.5 hours, and finally, DMF (228.28 g, 3.12 mol, 240.30 mL) was added. The mixture was reacted for additional 0.5 hours. The reaction mixture was poured into water (1000 mL), and the pH was adjusted to 7 with 1N hydrochloric acid. The mixture was extracted with ethyl acetate (1000 mL x 3). The organic phase was concentrated under reduced pressure and separated by column chromatography (petroleum ether:ethyl acetate = 10:1) to obtain compound **1-5.**

### Step 5

Compounds **1-5** (370 g, 807.30 mmol) was weighed. Toluene (1500 mL), dichlorobis(di-tert-butyl-(4-dimethylaminophenyl)phosphine)palladium (2.86 g, 4.04 mmol, 2.86 mL), and tributyl(1-propynyl)tin (265.69 g, 807.30 mmol) were added. The mixture was reacted at 120°C for 2 hours under nitrogen. The reaction solution was concentrated under reduced pressure and separated by column chromatography (petroleum ether:ethyl acetate = 5:1) to obtain compound **1-6.** MS m/z = 418.1 [M+H]⁺.

### Step 6

Compound **1-6** (450 g, 970.13 mmol) was weighed and DMF (100 mL) was added. N-bromosuccinimide (189.93 g, 1.07 mol) was added and the mixture was allowed to react at 25°C for 2 hours. Additional N-bromosuccinimide (17.27 g, 97.01 mmol) was added and the mixture was reacted for additional 3 hours. The reaction solution was concentrated under reduced pressure and separated by column chromatography (petroleum ether:ethyl acetate = 5:1) to obtain compound **1-7.** MS m/z = 496.0 [M+H]⁺.

### Step 7

Compound **1-7** (55 g, 110.81 mmol) was weighed and DMF (300 mL) was added. Methyl fluorosulfonyldifluoroacetate (42.57 g, 221.61 mmol, 28.19 mL) and cuprous iodide (42.21 g, 221.61 mmol) were added and the reaction mixture was reacted at 110°C under nitrogen for 2 hours. The mixture was quenched by adding 500 mL of water and extracted with ethyl acetate (600 mL x 3). The extracted organic phases were combined and washed sequentially with water (800 mL x 2) and saturated brine (800 mL), dried over anhydrous sodium sulfate, filtered, and concentrated. Compound **1-8** was obtained by column chromatography (petroleum ether:ethyl acetate = 10:1). MS m/z = 485.9 [M+H]⁺.

### Step 8

To a solution of sodium hydride (6.34 g, 158.61 mmol, 60% purity) in tetrahydrofuran (350 mL) at 0°C, methyl acetoacetate (158.61 mmol, 17.10 mL) was added dropwise. The mixture was allowed to react for 15 minutes. After cooling to -20°C, n-butyllithium (2.5 M, 63.44 mL) was added dropwise. After the addition was completed, the mixture was stirred for additional 15 min. A solution of compound **1-8** (35 g, 72.10 mmol) in tetrahydrofuran (350 mL) was then added. The mixture was allowed to react for 0.5 hours. The reaction was quenched by adding 200 mL of saturated ammonium chloride solution. The mixture was extracted with ethyl acetate (300 mL x 2). The extracted organic phases were combined and washed with saturated brine (400 mL), dried over anhydrous sodium sulfate, filtered, and concentrated under reduced pressure. Compound **1-9** was obtained by column chromatography (petroleum ether:ethyl acetate = 10:1 to 1:1). MS m/z =624.2 [M+Na]⁺.

### Step 9

Compound **1-9** (38 g, 63.17 mmol) was weighed and dichloromethane (300 mL) was added, followed by N,N-dimethylformamide dimethyl acetal (9.03 g, 75.80 mmol). The mixture was reacted at 25°C for 16 hours. The mixture was cooled to 0°C, and boron trifluoride etherate (10.76 g, 75.80 mmol, 9.32 mL) was added. The system was stirred at 0°C for additional 1 hour. 200 mL of saturated sodium bicarbonate solution was added to the mixture. The organic phase was separated. The aqueous phase was extracted with 200 mL of dichloromethane. The extracted organic phases were combined and washed with 250 mL of saturated brine, dried over anhydrous sodium sulfate, filtered, and concentrated under reduced pressure. Separation by column chromatography (petroleum ether:ethyl acetate = 10:1 to 1:1) afforded compound **1-10.** MS m/z = 612.1 [M+H]⁺.

### Step 10

Compound **1-10** (30 g, 49.05 mmol) was weighed, and tetrahydrofuran (300 mL) was added, followed by lithium tri-sec-butylborohydride (1 M, 53.96 mL) at -60°C. The mixture was reacted at -60°C for 1 hour, quenched with 200 mL of water, and extracted with ethyl acetate (300 mL x 2). The extracted organic phases were combined and washed with 300 mL of saturated brine, dried over anhydrous sodium sulfate, filtered, and concentrated under reduced pressure. Compound **1-11** was obtained by column chromatography (petroleum ether:ethyl acetate = 10:1 to 5:1). MS m/z = 614.1 [M+H]⁺.

### Step 11

Compound **1-11** (20 g, 32.59 mmol) was weighed and ethanol (200 mL) was added. 2-methyl-2-thioisourea sulfate (27.22 g, 97.78 mmol) and sodium carbonate (6.91 g, 65.19 mmol) were then added and reacted at 50°C for 13 hours. The reaction mixture was concentrated to dryness. 40 mL of water was added, and the mixture was extracted with ethyl acetate (50 mL x 2). The extracted organic phases were combined and washed with 60 mL of saturated brine and dried over anhydrous sodium sulfate. The mixture was filtered and concentrated under reduced pressure to obtain compound **1-12.** MS m/z = 654.3 [M+H]⁺.

### Step 12

Compound **1-12** (21 g, 32.13 mmol) was weighed and DMF (200 mL) was added. N,N-diisopropylethylamine (12.46 g, 96.38 mmol, 16.79 mL) and N-phenylbis(trifluoromethanesulfonyl)imide (13.77 g, 38.55 mmol) were then added. The mixture was reacted at 25°C for 1 hour. 300 mL of water was added to the mixture, and the mixture was extracted with ethyl acetate (300 mL x 3). The mixture was washed sequentially with water (2 x 400 mL) and saturated brine (400 mL), dried over anhydrous sodium sulfate, filtered, and concentrated under reduced pressure. Compound **1-13** was obtained by column chromatography (petroleum ether:ethyl acetate = 10:1). Preparative SFC separation (Chromatographic column: DAICEL CHIRALPAK IG (250mm*50mm, 10µm); mobile phase: [supercritical carbon dioxide-ethanol (0.1% ammonia)]; ethanol (0.1% ammonia) percentage: 25%-25%) afforded compound **1-13B.** Chiral SFC analysis (Chromatographic column: ChiralPak IG-3 (100mm*4.6mm, 3µm); mobile phase: [supercritical carbon dioxide-ethanol (0.05% diethylamine)]; ethanol (0.05% diethylamine) percentage: 5%-40%) revealed compound **1-13B** with an Rt of 3.055 minutes and an ee value of 99%.

### Step 13

Compound **1-13B** (200 mg, 254.53 µmol) and compound **1-14A** (174.74 mg) were added to N,N-dimethylformamide (2 mL), followed by N,N-diisopropylethylamine (131.58 mg, 1.02 mmol). The resulting reaction mixture was heated to 105°C under nitrogen and stirred for 1 hour. The reaction mixture was concentrated under reduced pressure to obtain a crude product. The crude product was purified by column chromatography (petroleum ether:ethyl acetate = 5:1) to obtain compound **1-14.** MS m/z = 906.3 [M+H]⁺.

### Step 14

Compound **1-14** (120 mg, 132.45 µmol) was dissolved in dichloromethane (2 mL), followed by the addition of m-chloroperbenzoic acid (29.58 mg, 145.70 µmol, 85% purity). The resulting reaction mixture was stirred at 20°C under nitrogen for 2 hours. The reaction mixture was diluted with 30 mL of dichloromethane and then washed with 5 mL of saturated sodium bicarbonate solution and 5 mL of saturated brine. The organic phase was dried and concentrated under reduced pressure to yield compound **1-15.** MS m/z = 922.7 [M+H]⁺.

### Step 15

Sodium tert-butoxide (23.97 mg, 249.46 µmol) and compound **1-2** (38.22 mg, 249.46 µmol) were added to tetrahydrofuran (1.5 mL) and stirred at 20°C for 0.5 hours. A solution of compound **1-15** (115 mg, 124.73 µmol) in tetrahydrofuran (1 mL) was then added to the mixture and stirred for additional hour. The reaction mixture was adjusted to pH 7 with 0.5M hydrochloric acid, followed by the addition of 20 mL of ethyl acetate and 10 mL of water. The mixture was dissolved with stirring. The aqueous layer was separated. The organic phase was washed with 2 mL of saturated brine, dried over anhydrous sodium sulfate, and filtered. The filtrate was concentrated under reduced pressure to yield compound **1-16.** MS m/z = 1011.5 [M+H]⁺.

### Step 16

Compound **1-16** (120.00 mg, 118.68 µmol) was dissolved in tetrahydrofuran (2 mL). The resulting solution was cooled to 0°C, and lithium aluminum tetrahydride (1 M, 118.68 µL) was added dropwise. The reaction was stirred for 0.5 hours. The reaction solution was carefully quenched with 0.2 mL of water, followed by the addition of 0.5 g of anhydrous sodium sulfate and stirring for 2 minutes. The mixture was filtered through a pad of Celite, and the filter cake was rinsed with 20 mL of tetrahydrofuran. The filtrate was collected and concentrated under reduced pressure to yield compound **1-17.** MS (ESI) m/z = 983.4 [M+H]⁺.

### Step 17

Compound **1-17** (120 mg, 122.06 µmol) was added to trifluoroacetic acid (2 mL) and stirred at 20°C for 2 hours. The reaction solution was concentrated under reduced pressure to obtain a crude product. The crude product was separated by high-performance liquid chromatography (HPLC column: Xtimate C18 150*40mm*5µm; mobile phase: [water (0.05% hydrochloric acid)-acetonitrile]; (acetonitrile): 10%-40%) and concentrated under reduced pressure to obtain the hydrochloride salt of compound 1. MS m/z = 643.3 [M+H]⁺.

### Example 2

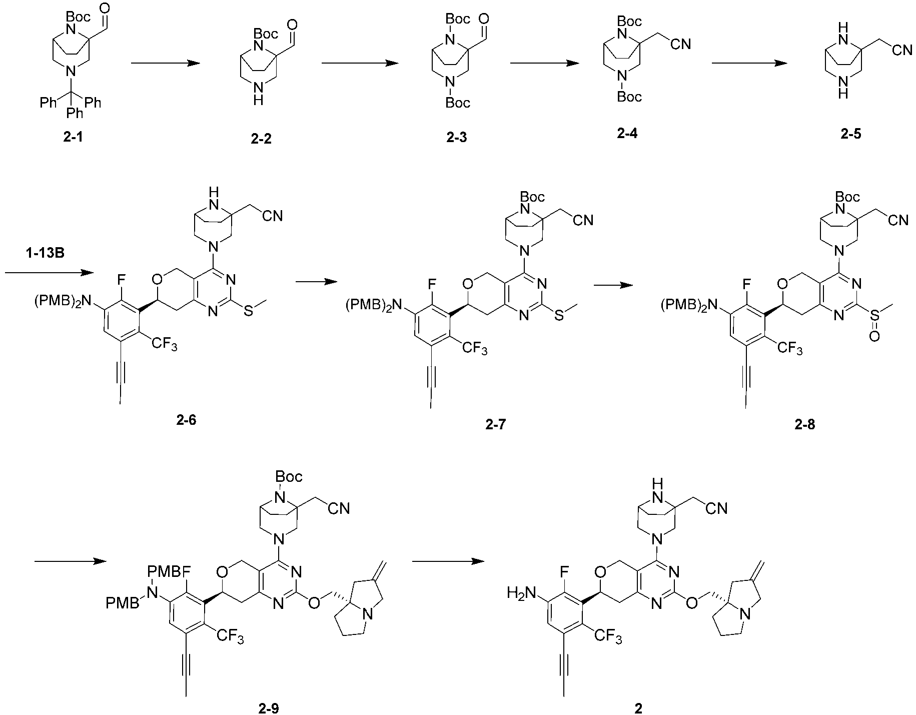

### Step 1

Compound **2-1** (2.3 g, 4.77 mmol) was dissolved in dioxane (30 mL), followed by the addition of hydrochloric acid (1 M, 14.30 mL). The resulting reaction mixture was stirred at 20°C under nitrogen for 1 hour. The reaction mixture was concentrated under reduced pressure, and 20 mL of water and 50 mL of ethyl acetate were added to the residue and stirred until fully dissolved. The aqueous phase was separated. The organic phase was washed with 10 mL of 0.5 M hydrochloric acid. The aqueous phases were combined and adjusted to pH 10 with 20% sodium carbonate solution, and then extracted with dichloromethane (20 mL x 2). The organic phases were combined concentrated under reduced pressure to yield compound **2-2.**

### Step 2

To compound **2-2** (1.2 g, 4.77 mmol) was added 20 mL of dichloromethane and then Boc₂O (2.08 g, 9.53 mmol). The mixture was stirred at 20°C for 1 hour. The reaction solution was concentrated under reduced pressure to obtain a crude product. The crude product was purified by column chromatography (petroleum ether:ethyl acetate = 5:1) to afford compound **2-3.** ¹H NMR (400 MHz, CDCl₃) δ: 9.55 - 9.47 (m, 1H), 4.40-4.00 (m, 2H), 3.95 - 3.55 (m, 1H), 3.30 - 2.90 (m, 2H), 2.25 - 1.91 (m, 2H), 1.78 (s, 2H), 1.47 (s, 18H).

### Step 3

Potassium tert-butoxide (830.68 mg, 7.40 mmol) was added to ethylene glycol dimethyl ether (25 mL). The resulting mixture was cooled to -78°C, followed by the addition of p-toluenesulfonylmethyl isocyanide (794.91 mg, 4.07 mmol). After the addition, the reaction was stirred for 30 minutes. A solution of compound **2-3** (1.26 g, 3.70 mmol) in ethylene glycol dimethyl ether (25 mL) was then added dropwise. After the addition, the mixture was stirred for 30 minutes. The cooling bath was removed. The mixture was warmed to room temperature (20°C) and stirred for 30 minutes. Finally, 40 mL of methanol was added, and the resulting reaction mixture was heated to 90°C and stirred for 1 hour. The reaction mixture was concentrated under reduced pressure to obtain a crude product. The crude product was purified by column chromatography (petroleum ether:ethyl acetate = 3:1) to obtain compound **2-4.** ¹H NMR (400MHz, CDCl₃) δ: 4.22 (s, 1H), 3.65 - 3.55 (m, 2H), 3.30-2.62 (m, 4H), 1.99 - 1.89 (m, 2H), 1.65 - 1.30 (m, 20H).

### Step 4

Compound **2-4** (375 mg, 1.07 mmol) was dissolved in dichloromethane (2 mL), followed by the addition of trifluoroacetic acid (1.22 g, 10.67 mmol). The resulting reaction mixture was stirred at 20°C under nitrogen for 1 hour. The reaction mixture was concentrated under reduced pressure, and 20 mL of ethyl acetate was added to the residue. Then, 1 mL of a 4M hydrogen chloride/ethyl acetate solution was added, and the mixture was concentrated under reduced pressure to obtain the hydrochloride salt of compound **2-5.**

### Step 5

Compound **1-13B** (350 mg, 445.44 µmol) and the hydrochloride salt of compound **2-5** (299.51 mg) were added to N,N-dimethylformamide (3 mL), followed by N,N-diisopropylethylamine (460.56 mg, 3.56 mmol). Under nitrogen, the mixture was heated to 105°C with stirring for 1 hour. The reaction mixture was concentrated under reduced pressure to obtain a crude product, which was dissolved in 50 mL of ethyl acetate and washed with saturated brine (10 mL x 2). The organic phase was dried and concentrated under reduced pressure to obtain compound **2-6.** MS m/z = 787.2 [M+H]⁺.

### Step 6

Compound **2-6** (368 mg, 467.67 µmol) and N,N-diisopropylethylamine (120.89 mg, 935.34 µmol) were added to dichloromethane (3 mL), followed by Boc₂O (153.10 mg, 701.51 µmol). The resulting reaction mixture was stirred at 20°C under nitrogen for 3 hours. The reaction mixture was concentrated under reduced pressure to obtain a crude product. The crude product was purified by column chromatography (petroleum ether:ethyl acetate = 3:1) to obtain compound **2-7.** MS m/z = 887.4 [M+H]⁺.

### Step 7

Compound **2-7** (220 mg, 248.03 µmol) was dissolved in dichloromethane (2 mL), followed by the addition of m-chloroperbenzoic acid (50.35 mg, 248.03 µmol, 85% purity). The resulting reaction mixture was stirred at 20°C under nitrogen for 2 hours. The reaction mixture was concentrated under reduced pressure to yield compound **2-8.** MS m/z = 903.6 [M+H]⁺.

### Step 8

Compound **1-2** (75.68 mg, 493.91 µmol) was dissolved in THF (2 mL), followed by the addition of sodium tert-butoxide (47.47 mg, 493.91 µmol). Under nitrogen, the reaction was stirred at 20°C for 1 hour. A solution of compound **2-8** (223 mg, 246.96 µmol) in tetrahydrofuran (1 mL) was then added. After the addition, the reaction was stirred at 20°C for 0.5 hours. The reaction solution was dissolved in 10 mL of ethyl acetate and washed with 5 mL of saturated brine. The organic phase was dried, and filtered. The filtrate was concentrated under reduced pressure to yield compound **2-9.** MS m/z = 992.5 [M+H]⁺.

### Step 9

Trifluoroacetic acid (2 mL) was added to compound **2-9** (214 mg, 215.70 µmol), and the resulting reaction mixture was stirred at 20°C for 1 hour. The reaction mixture was concentrated under reduced pressure to obtain a crude product. The crude product was separated by preparative HPLC (Chromatographic column: Xtimate C18 150*40mm*5µm; mobile phase: [water (0.05% hydrochloric acid)-acetonitrile]; (acetonitrile): 10%-40%) and concentrated under reduced pressure to obtain the hydrochloride salt of compound 2. MS m/z = 652.2 [M+H]⁺.

### Example 3

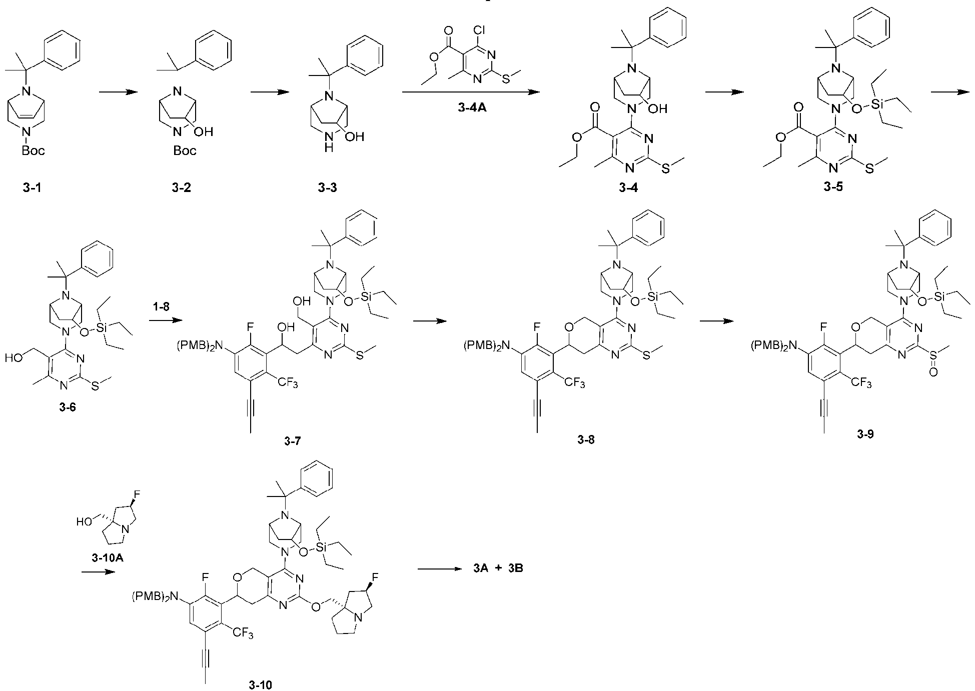

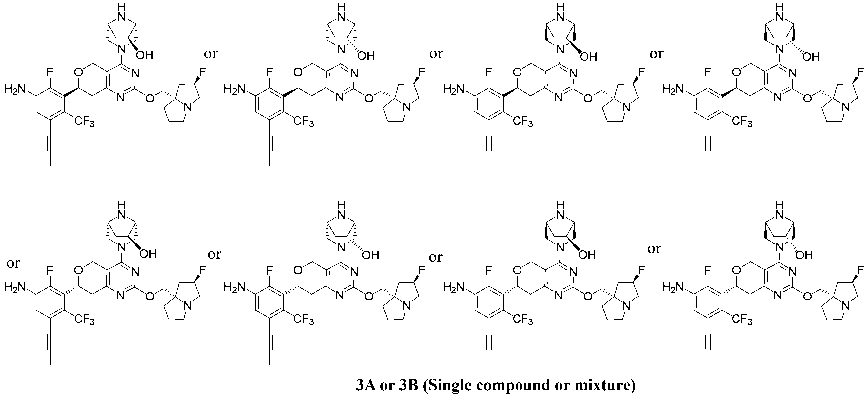

### Step 1

Borane solution in tetrahydrofuran (1 M, 21 mL) was slowly added dropwise to a solution of compound **3-1** (1.7 g, 5.18 mmol) in anhydrous THF (20 mL) at 0°C under nitrogen. The mixture was then stirred at 25°C for 12 hours. A 5% NaOH solution (26.31 mmol, 21 mL) was then added dropwise at 0°C, followed by dropwise addition of hydrogen peroxide (4.88 g, 43.04 mmol, 4.14 mL, 30% purity). The mixture was allowed to react at 25°C for 2 hours. The reaction mixture was quenched by the slow addition of 50 mL of saturated sodium sulfite solution and extracted with 50 mL of ethyl acetate. The organic phase was washed with 50 mL of brine, dried over anhydrous sodium sulfate, and filtered. The filtrate was concentrated under reduced pressure to obtain a crude product. The crude product was separated by preparative thin-layer chromatography (petroleum ether:ethyl acetate = 3:1) to afford compound **3-2.** MS m/z = 347.2 [M+H]⁺.

### Step 2

To a solution of compound **3-2** (1 g, 2.89 mmol) in anhydrous dioxane (10 mL) was added a hydrochloric acid/dioxane solution (4 M, 10 mL) and the mixture was reacted at 20°C for 0.5 h. The reaction solution was concentrated under reduced pressure to obtain the hydrochloride salt of compound **3-3.**

### Step 3

Compound **3-4A** (750 mg, 3.04 mmol) was dissolved in anhydrous DCM (10 mL). Then, triethylamine (584 mg, 5.77 mmol) and the hydrochloride salt of compound **3-3** (921 mg) were added and the reaction was reacted at 20°C for 1 hour. The resulting dichloromethane solution of compound **3-4** was used directly in the next step. MS m/z = 457.2 [M+1]⁺.

### Step 4

To 10 mL of the dichloromethane solution of compound **3-4** obtained in step 3, triethylsilyl chloride (871 mg, 5.78 mmol) and imidazole (590 mg, 8.67 mmol) were added, respectively. The mixture was allowed to react at 25°C for 12 hours. The reaction solution was diluted with 10 mL of water and extracted with 10 mL of dichloromethane. The organic phase was washed with 10 mL of brine, dried over anhydrous sodium sulfate, and filtered. The filtrate was concentrated under reduced pressure to obtain a crude product. The crude product was separated by column chromatography (petroleum ether:ethyl acetate = 50:1) to obtain compound **3-5.** MS m/z = 571.3 [M+1]⁺.

### Step 5

To a solution of compound **3-5** (1.43 g, 2.50 mmol) in THF (20 mL) was slowly added a solution of lithium aluminum hydride (2.5 M, 2.00 mL) in tetrahydrofuran at 0°C. The reaction was allowed to proceed at 25°C for 5 hours. Ethyl acetate (10 mL) was then added dropwise to quench the reaction. The resulting suspension was filtered, and the filtrate was concentrated under reduced pressure to yield a crude product. The crude product was purified by column chromatography (petroleum ether:ethyl acetate = 20:1) to yield compound **3-6.** MS m/z = 529.3 [M+1]⁺.

### Step 6

The compound tetramethylpiperidine (460 mg, 3.26 mmol) was dissolved in anhydrous tetrahydrofuran (1.0 mL) and cooled to -40°C under nitrogen, n-Butyllithium (2.5 M, 1.3 mL) was then added dropwise. The reaction was stirred for 0.5 hours. Compound **3-6** (430 mg, 813 µmol) was dissolved in anhydrous tetrahydrofuran (0.5 mL) and added dropwise to the reaction flask at -60°C. After completion of the addition, the reaction was stirred for 0.5 hours. Finally, a solution of compound **1-8** (486 mg, 1.00 mmol) in anhydrous tetrahydrofuran (0.5 mL) was added to the reaction at -60°C. The mixture was then warmed to 20°C and stirred for 2.5 hours. The reaction was quenched by the addition of 20 mL of water and extracted with ethyl acetate (20 mL). The organic phase was washed with 20 mL of brine, dried over anhydrous sodium sulfate, and filtered. The filtrate was concentrated under reduced pressure to obtain a crude product. The crude product was purified by column chromatography (petroleum ether:ethyl acetate = 5:1) and separated by preparative thin-layer chromatography (petroleum ether:ethyl acetate = 3:1) to obtain compound 3-7. MS m/z = 1014.5 [M+H]⁺.

### Step 7

Compound **3-7** (70 mg, 69.0 µmol) was dissolved in anhydrous toluene (1.5 mL), and cyanomethylenetri-n-butylphosphine (150 mg, 621.50 µmol) was added. After nitrogen replacement, the reaction was stirred at 110°C for 12 hours. The reaction solution was cooled and concentrated under reduced pressure to obtain a crude product. The crude product was purified by preparative thin-layer chromatography (petroleum ether:ethyl acetate = 5:1) to afford compound **3-8.** MS m/z = 996.5 [M+H]⁺.

### Step 8

Compound **3-8** (22.5 mg, 22.58 µmol) was dissolved in anhydrous dichloromethane (0.5 mL). Meta-chloroperbenzoic acid (5 mg, 24.63 µmol, 85% purity) was added and stirred at 25°C for 12 hours. The reaction mixture was quenched by adding 4 mL of saturated sodium sulfite solution and extracted with dichloromethane (20 mL x 3). The organic phase was washed with 20 mL of brine, dried over anhydrous sodium sulfate, and filtered. The filtrate was concentrated under reduced pressure to obtain a crude product. The crude product was purified by preparative thin-layer chromatography (petroleum ether:ethyl acetate = 5:1) to obtain compound **3-9.** MS m/z = 1012.6 [M+H]⁺.

### Step 9

Compound **3-9** (19 mg, 18.8 µmol) was dissolved in anhydrous toluene (0.5 mL). Sodium tert-butoxide (7.22 mg, 75.1 µmol), 4Å molecular sieves (10 mg), and compound **3-10A** (12 mg, 75.08 µmol) were added. The reaction was stirred at 100°C for 12 hours. The reaction solution was cooled, and filtered. The filtrate was concentrated under reduced pressure to obtain a crude product. The crude product was purified by preparative thin-layer chromatography (ethyl acetate:methanol = 10:1) to obtain compound **3-10.** MS m/z = 1107.7 [M+H]⁺.

### Step 10

Compound **3-10** (17 mg, 15.35 µmol) was dissolved in trifluoroacetic acid (5 mL) and reacted at 20°C for 12 hours. The reaction solution was concentrated under reduced pressure to obtain a crude product. The crude product was separated by preparative high-performance liquid chromatography (Chromatographic column: Phenomenex luna C18 150*25mm*10µm; mobile phase: [water (0.075% trifluoroacetic acid)-acetonitrile]; acetonitrile: 18%-48%) to obtain the trifluoroacetic acid salts of compounds **3A** and **3B.**

Trifluoroacetate salt of compound **3A** (single compound or mixture), MS m/z = 635.3 [M+H]⁺. ¹H NMR (CD₃OD, 400 MHz) δ 6.94 (d, *J* = 8.50 Hz, 1 H), 5.71 - 5.50 (m, 1 H), 5.17 (br dd, *J =* 10.94, 4.06 Hz, 1 H), 4.80 (br d, *J =* 13.63 Hz, 2 H), 4.62 - 4.48 (m, 3 H), 4.17 - 3.73 (m, 7 H), 3.54 - 3.36 (m, 3 H), 3.31 - 3.25 (m, 1 H), 2.93 (br dd, *J =* 18.01, 3.75 Hz, 1 H), 2.77 - 2.48 (m, 3 H), 2.46 - 2.28 (m, 3 H), 2.26 - 2.12 (m, 1 H), 2.04 (s, 3 H), 1.96 (dd, *J* = 14.01, 4.13 Hz, 1 H).

Trifluoroacetate salt of compound **3B** (single compound or mixture), MS m/z = 635.2 [M+H]⁺. ¹H NMR (CD₃OD, 400 MHz) δ 6.93 (d, *J =* 8.50 Hz, 1 H), 5.69 - 5.49 (m, 1 H), 5.30 (d, *J =* 13.88 Hz, 1 H), 5.18 (br dd, *J* = 11.19, 3.56 Hz, 1 H), 4.87 - 4.78 (m, 2 H), 4.69 - 4.50 (m, 3 H), 4.29 (br d, *J =* 13.26 Hz, 1 H), 4.20 - 3.87 (m, 6 H), 3.63 (br d, *J* = 14.51 Hz, 1 H), 3.53 - 3.42 (m, 1 H), 3.35 (br s, 1 H), 2.93 (br dd, *J* = 17.70, 3.56 Hz, 1 H), 2.77 - 2.51 (m, 3 H), 2.47 - 2.28 (m, 3 H), 2.26 - 2.12 (m, 1 H), 2.04 (s, 3 H), 1.83 (dd, *J =* 13.88, 4.25 Hz, 1 H).

### Example 4

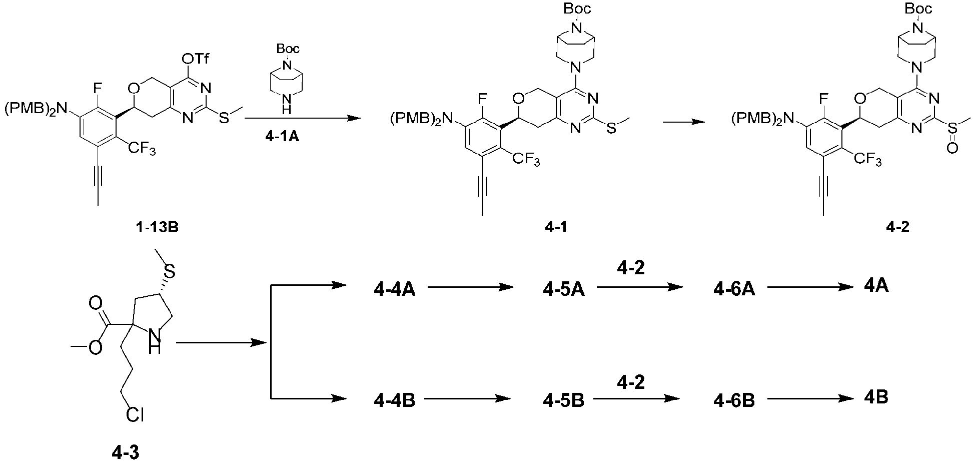

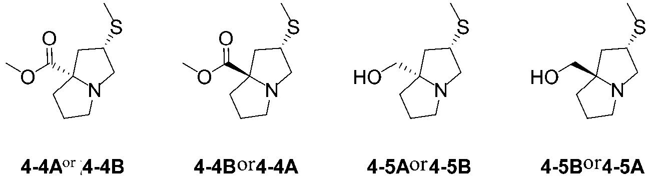

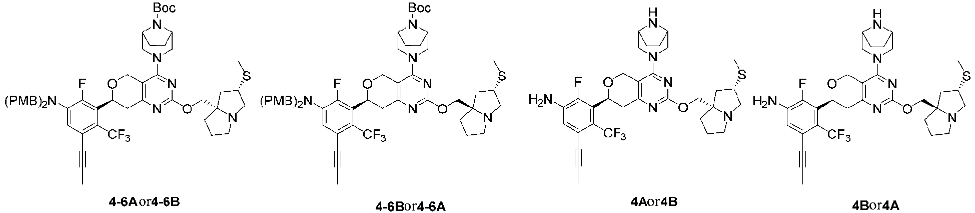

### Step 1

Compound **1-13B** (240 mg, 305.44 µmol) and compound **4-1A** (97.26 mg, 458.16 µmol) were dissolved in DMF (5 mL). DIPEA (916.33 µmol, 159.61 µL) was added and stirred at 100°C for 1 hour. The mixture was extracted with 30 mL of ethyl acetate. The organic phase was washed with 50 mL of brine, dried over anhydrous sodium sulfate, and filtered. The filtrate was concentrated under reduced pressure to obtain a crude product. The crude product was separated by preparative thin-layer chromatography (petroleum ether:ethyl acetate = 5:1) to obtain compound **4-1.** MS m/z = 848.5 [M+H]⁺.

### Step 2

Compound **4-1** (200 mg, 235.86 µmol) was dissolved in dichloromethane (5 mL). Meta-chloroperbenzoic acid (40.70 mg, 235.86 µmol, 85% purity) was added and stirred at 25°C for 1 hour. The reaction solution was extracted with 5 mL of dichloromethane. The organic phase was washed with 10 mL of saturated sodium bicarbonate, dried over anhydrous sodium sulfate, and filtered. The filtrate was concentrated under reduced pressure to obtain a crude product. The crude product was separated by preparative thin-layer chromatography (dichloromethane:methanol = 10:1) to obtain compound **4-2.** MS m/z = 864.3 [M+H]⁺.

### Step 3

Compound **4-3** (610 mg, hydrochloride) was dissolved in acetonitrile (10 mL), and potassium carbonate (1.46 g, 10.6 mmol) and potassium iodide (35.1 mg, 212 µmol) were added. The reaction mixture was reacted at 25°C for 12 hours. The reaction mixture was filtered and the filtrate was concentrated under reduced pressure. The crude product was purified by column chromatography (petroleum ether:ethyl acetate = 5:1) and then separated by preparative thin-layer chromatography (petroleum ether:ethyl acetate = 1:1) to obtain compounds **4-4A** and **4-4B.** Compound **4-4A** (petroleum ether:ethyl acetate = 1:1, R_{f} = 0.21) had an MS m/z of 216.0 [M+1]⁺, and compound **4-4B** (petroleum ether:ethyl acetate = 1:1, R_{f}= 0.12) had an MS m/z of 216.1 [M+1]⁺.

Compound **4-4A:** ¹H NMR (400 MHz, CDCl₃) δ ppm 3.75 (s, 3H), 3.54 (dd, *J* = 6.5, 9.0 Hz, 1H), 3.33 (tt, *J =* 6.4, 10.6 Hz, 1H), 3.13 (td, *J =* 6.4, 10.8 Hz, 1H), 2.86 - 2.75 (m, 2H), 2.52 (t, *J =* 9.6 Hz, 1H), 2.30 - 2.18 (m, 1H), 2.15 (s, 3H), 1.99 - 1.81 (m, 3H), 1.61 (dd, *J =* 11.3, 12.6 Hz, 1H); compound **4-4B:** ¹H NMR (400 MHz, CDCl₃) δ ppm 3.67 (s, 3H), 3.28 - 3.08 (m, 2H), 3.06 - 2.92 (m, 2H), 2.56 (td, *J =* 7.4, 9.5 Hz, 1H), 2.37 - 2.17 (m, 2H), 2.09 (dd, *J* = 6.9, 13.2 Hz, 1H), 2.04 (s, 3H), 1.79 - 1.69 (m, 2H), 1.67 - 1.56 (m, 1H).

### Step 4

Compound **4-4A** (102 mg, 474 µmol) was dissolved in THF (5.0 mL). Lithium aluminum tetrahydride (2.5 M, 0.3 mL) was added dropwise at 0°C. The reaction mixture was warmed to 25°C and allowed to react for 1 hour. Water (0.03 mL), 15% aqueous sodium hydroxide (0.03 mL), and water (0.1 mL) were then added dropwise at 0°C, followed by stirring for 0.5 hours. The mixture was filtered. The filter cake was washed with 10 mL of ethyl acetate, and the filtrate was concentrated under reduced pressure to yield compound **4-5A.** MS m/z = 188.1 [M+1]⁺.

### Step 5

Compound **4-2** (120 mg, 139 µmol), compound **4-5A** (89 mg, 475 µmol), 4Å molecular sieves (120 mg), and sodium tert-butoxide (80 mg, 832 µmol) were added to toluene (15 mL) and heated to 100°C for 6 hours. The reaction mixture was filtered. The filter cake was washed with 10 mL of ethyl acetate, and the filtrate was concentrated under reduced pressure to obtain a crude product. The crude product was purified by preparative thin-layer chromatography (dichloromethane:methanol = 10:1) to obtain compound **4-6A,** MS m/z = 987.4 [M+1]⁺.

### Step 6

Compound **4-6A** (81 mg, 82.0 µmol) was dissolved in dichloromethane (10.0 mL), and trifluoroacetic acid (3.07 g, 26.9 mmol, 2 mL) was added. The reaction mixture was reacted at 25°C for 12 hours. The reaction mixture was concentrated under reduced pressure to obtain a crude product. The crude product was purified by preparative HPLC (Chromatographic column: Phenomenex luna C18 150*25mm*10µm; mobile phase: [water (0.075% trifluoroacetic acid)-acetonitrile]; acetonitrile: 15%-45%) to obtain the trifluoroacetate salt of compound **4A.** MS m/z = 647.3 [M+1]⁺. ¹H NMR (400 MHz, CD₃OD) δ ppm 6.94 (d, *J =* 8.5 Hz, 1H), 5.21 (br dd, *J* = 3.9, 11.1 Hz, 1H), 4.79 - 4.49 (m, 4H), 4.47 - 4.32 (m, 1H), 4.24 - 4.02 (m, 3H), 3.86 (br d, *J =* 13.9 Hz, 1H), 3.76 - 3.65 (m, 2H), 3.65 - 3.55 (m, 1H), 3.54 - 3.46 (m, 1H), 3.41 - 3.35 (m, 1H), 3.20 - 3.08 (m, 1H), 3.01 - 2.89 (m, 1H), 2.71 (br dd, *J =* 6.5, 13.4 Hz, 1H), 2.41 - 2.19 (m, 8H), 2.19 - 1.90 (m, 8H).

### Step 7

Compound **4-4B** (51 mg, 237 µmol) was dissolved in THF (3.0 mL). Lithium aluminum tetrahydride (2.5 M, 0.15 mL) was added dropwise at 0°C. The reaction mixture was warmed to 25°C and allowed to react for 1 hour. Water (0.02 mL), 15% aqueous NaOH (0.02 mL), and water (0.06 mL) were then added dropwise at 0°C, followed by stirring for 0.5 hours. The mixture was filtered. The filter cake was washed with 10 mL of ethyl acetate, and the filtrate was concentrated under reduced pressure to yield compound **4-5B.** MS m/z = 188.2 [M+1]⁺.

### Step 8

Compound **4-2** (100 mg, 116 µmol), compound **4-5B** (41 mg, 219 µmol), 4Å molecular sieves (70 mg), and sodium tert-butoxide (80 mg, 832 µmol) were added to toluene (15 mL) and heated to 100°C for 6 hours. The reaction mixture was filtered. The filter cake was washed with 10 mL of ethyl acetate, and the filtrate was concentrated under reduced pressure to obtain a crude product. The crude product was purified by preparative thin-layer chromatography (dichloromethane:methanol = 20: 1) to obtain compound **4-6B,** MS m/z = 987.4 [M+1]⁺.

### Step 9

Compound **4-6B** (70 mg, 70.9 µmol) was dissolved in dichloromethane (10.0 mL), and trifluoroacetic acid (3.07 g, 26.9 mmol, 2 mL) was added. The reaction mixture was reacted at 25°C for 12 hours. The reaction mixture was concentrated under reduced pressure to obtain a crude product. The crude product was purified by preparative HPLC (Chromatographic column: Phenomenex luna C18 150*25mm*10µm; mobile phase: [water (0.075% trifluoroacetic acid)-acetonitrile]; acetonitrile: 15%-45%) to obtain the trifluoroacetate salt of compound 4B. MS m/z = 647.3 [M+1]⁺. ¹H NMR (400 MHz, CD₃OD) δ ppm 6.94 (d, *J* = 8.5 Hz, 1H), 5.21 (br dd, *J* = 4.1, 10.6 Hz, 1H), 4.78 - 4.64 (m, 4H), 4.54 (br d, *J =* 11.8 Hz, 1H), 4.42 - 4.32 (m, 1H), 4.21 - 4.11 (m, 2H), 3.88 - 3.54 (m, 6H), 3.40 - 3.34 (m, 2H), 2.96 (br dd, *J* = 4.0, 17.9 Hz, 1H), 2.55 - 2.37 (m, 2H), 2.36 - 1.89 (m, 14H).

### Example 5

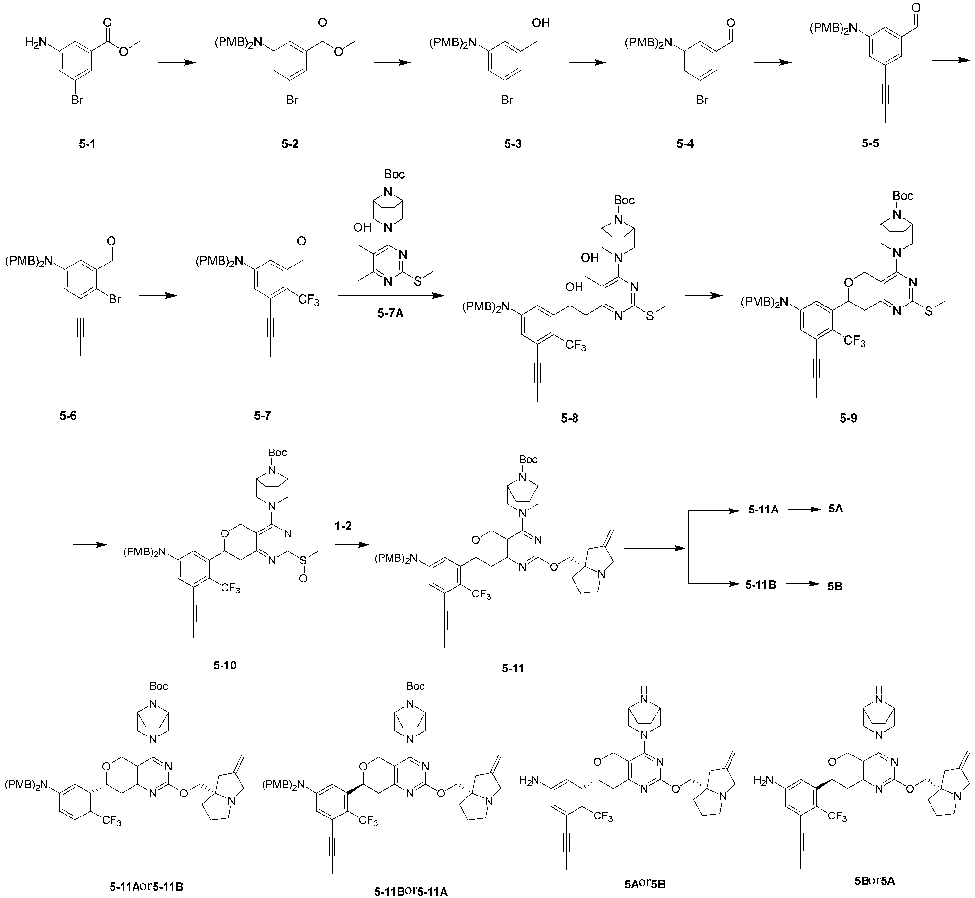

### Step 1

Compound 5-1 (10.0 g, 43.5 mmol) was dissolved in DMF (4 mL), followed by the addition of potassium carbonate (15.0 g, 109 mmol) and p-methoxybenzyl chloride (16.3 g, 104 mmol, 14.2 mL). The reaction mixture was allowed to react at 80°C for 12 hours. After the reaction mixture was cooled to room temperature, 200 mL of ethyl acetate was added to the reaction mixture. The mixture was washed with 200 mL of water and 100 mL of saturated brine, dried over anhydrous sodium sulfate, filtered, and concentrated under reduced pressure to obtain a crude product. The crude product was purified by column chromatography (petroleum ether:ethyl acetate = 30:1) to obtain compound **5-2.** ¹H NMR (400 MHz, CDCl₃) δ ppm 7.48 (t, *J =* 1.31 Hz, 1H), 7.41 (dd, *J =* 2.38, 1.13 Hz, 1H), 7.13 (d, *J =* 8.50 Hz, 4H), 7.06 - 7.02 (m, 1H), 6.92 - 6.83 (m, 4H), 4.56 (s, 4 H), 3.86 (s, 3 H) 3.81 (s, 6 H).

### Step 2

Compound **5-2** (17.5 g, 37.2 mmol) was dissolved in anhydrous THF (200 mL). LiAlH₄ (2.5 M, 30 mL) was then added at 0°C, followed by reaction at 25°C for 1 hour. The reaction mixture was cooled to room temperature, and 2.85 mL of H₂O, 2.85 mL of 15% aqueous NaOH, and 8.6 mL of H₂O were slowly added dropwise under a nitrogen stream. The resulting solid was filtered, and the filtrate was concentrated under reduced pressure to yield compound **5-3.** ¹H NMR (400 MHz, CDCl₃) δ ppm 7.03 (d, *J =* 8.63 Hz, 4H), 6.82 - 6.67 (m, 6H), 6.57 (s, 1H), 4.44 (s, 6H), 3.71 (s, 6H).

### Step 3

Compound **5-3** (15.0 g, 33.9 mmol) was dissolved in THF (150 mL). The atmosphere was replaced with nitrogen three times, and MnO₂ (60.0 g, 690 mmol) was added. After reacting at 75°C for 12 hours, the reaction solution was cooled, and filtered. The filtrate was concentrated under reduced pressure to obtain a crude product. The crude product was purified by column chromatography (petroleum ether:ethyl acetate = 20:1) to obtain compound **5-4.** ¹H NMR (400 MHz, CDCl₃) δ ppm 9.79 (s, 1H), 7.16 - 7.09 (m, 6H), 6.92 - 6.86 (m, 5H), 4.59 (s, 4H), 3.81 (s, 6H).

### Step 4

Compound **5-4** (7.20 g, 16.4 mmol), 1-propynyltri-n-butyltin (5.38 g, 16.4 mmol), and dichlorobis(di-tert-butyl-(4-dimethylaminophenyl)phosphine)palladium (II) (57.9 mg, 81.8 µmol) were dissolved in anhydrous toluene (170 mL). The atmosphere was purged with nitrogen three times, and the mixture was reacted at 110°C for 12 hours. The reaction solution was concentrated under reduced pressure to obtain a crude product. The crude product was purified by column chromatography (petroleum ether:ethyl acetate = 10:1) to obtain compound 5-5. MS m/z = 400.3 [M+H]⁺, ¹H NMR (400 MHz, CDCl₃) δ ppm 9.82 (s, 1H), 7.21 (s, 1H), 7.18 - 7.15 (m, 1H), 7.12 (d, *J* = 8.63 Hz, 4H), 7.03 - 7.01 (m, 1H), 6.87 (d, *J =* 8.63 Hz, 4H), 4.58 (s, 4H), 3.81 (s, 6H), 2.02 (s, 3H).

### Step 5

Compound **5-5** (4.60 g, 11.5 mmol) was dissolved in anhydrous DMF (50 mL), and NBS (2.25 g, 12.7 mmol) was added. The mixture was allowed to react at room temperature for 0.5 h. Water (150 mL) was added to the organic phase, and the mixture was extracted three times with ethyl acetate (50 mL). The organic phase was washed three times with saturated brine (50 mL), dried over anhydrous sodium sulfate, and filtered. The filtrate was concentrated under reduced pressure to obtain a crude product. The crude product was purified by column chromatography (petroleum ether:ethyl acetate = 10:1) to obtain compound **5-6.** MS m/z = 478.2 [M+H]⁺. ¹H NMR (400 MHz, CDCl₃) δ ppm 10.31 (s, 1H), 7.24 (d, *J =* 3.38 Hz, 1H), 7.10 (d, *J* = 8.63 Hz, 4H), 7.05 (d, *J* = 3.38 Hz, 1H), 6.89 - 6.83 (m, 4H), 4.55 (s, 4H), 3.80 (s, 6H), 2.09 (s, 3H).

### Step 6

Compound **5-6** (5.30 g, 11.1 mmol), cuprous iodide (4.22 g, 22.2 mmol), and methyl 2,2-difluoro-2-fluorosulfonylacetate (8.09 g, 42.1 mmol) were dissolved in DMF (50 mL). The atmosphere was purged with nitrogen three times and the mixture was reacted at 110°C for 2.5 hours. The reaction solution was cooled, filtered through celite, and water (150 mL) was added to the organic phase. The mixture was extracted three times with 150 mL of ethyl acetate. The organic phase was washed three times with saturated brine (150 mL), dried over anhydrous sodium sulfate, and filtered. The filtrate was concentrated under reduced pressure to obtain a crude product. The crude product was purified by column chromatography (petroleum ether:ethyl acetate = 20:1) to afford compound **5-7.** MS m/z = 468.2 [M+H]⁺. ¹H NMR (400 MHz, CDCl₃) δ ppm 10.30 (q, *J =* 2.71 Hz, 1H), 7.25 (d, *J =* 2.75 Hz, 1H), 7.10 (d, *J =* 8.63 Hz, 4H), 7.00 (d, *J =* 2.88 Hz, 1H), 6.90 - 6.85 (m, 4H), 4.60 (s, 4H), 3.81 (s, 6H), 2.05 (d, *J=* 4.13 Hz, 3H).

### Step 7

TMP (2.45 g, 17.4 mmol, 2.94 mL) was dissolved in THF (20 mL). The atmosphere was purged with nitrogen three times. The temperature was lowered to -40°C, and n-BuLi (2.5 M, 6.71 mL) was slowly added dropwise. After complete addition, the mixture was allowed to react at -40°C for 30 minutes. The reaction system was then cooled to -60°C, and a solution of **5-7A** (2.20 g, 5.78 mmol) in THF (20 mL) was slowly added dropwise to the reaction mixture. After reacting at -40°C for 15 minutes, compound **5-7** (3.94 g, 6.94 mmol) was added portionwise to the reaction mixture, and the mixture was reacted at room temperature for 2 hours. The reaction mixture was quenched with saturated aqueous ammonium chloride (100 mL) and extracted twice with ethyl acetate (100 mL). The organic phase was washed twice with saturated brine (100 mL), dried over anhydrous sodium sulfate, and filtered. The filtrate was concentrated under reduced pressure to obtain a crude product. The crude product was purified by column chromatography (petroleum ether: ethyl acetate = 2:1) to obtain compound **5-8.** MS m/z = 848.4 [M+H]⁺. ¹H NMR (400 MHz, CDCl₃) δ ppm 7.15 - 6.99 (m, 5H), 6.79 (d, *J =* 8.63 Hz, 4H), 6.75 (br d, *J* = 2.38 Hz, 1H), 5.39 (br d, *J =* 8.63 Hz, 1H), 4.84 - 4.40 (m, 5H), 4.38 - 4.12 (m, 5H), 3.72 (s, 6H), 3.42 - 3.11 (m, 2H), 3.06 - 2.86 (m, 1H), 2.70 - 2.51 (m, 1H), 2.40 (s, 3H), 2.36 - 2.23 (m, 1H), 1.94 (s, 2H), 1.85 - 1.74 (m, 3H), 1.66 (br d, *J* = 9.13 Hz, 1H), 1.42 (s, 9H).

### Step 8

Compound **5-8** (324 mg, 390 µmol) was dissolved in anhydrous toluene (23 mL), and tributyl cyanomethylene phosphate (1.30 g, 5.40 mmol) was added. The atmosphere was then purged with nitrogen three times, and the mixture was reacted at 110°C for 12 hours. The reaction solution was concentrated under reduced pressure to obtain a crude product. The crude product was purified by column chromatography (petroleum ether:ethyl acetate = 10:1) and separated by preparative high-performance liquid chromatography (Phenomenex luna C18 150*25mm*10µm column; mobile phase: [water (0.05% hydrochloric acid)-acetonitrile]; (acetonitrile): 55%-85%), and concentrated under reduced pressure to obtain the hydrochloride salt of compound 5-9. MS m/z = 830.5 [M+H]⁺.

### Step 9

Compound **5-9** (324 mg, hydrochloride) was dissolved in anhydrous dichloromethane (3 mL), and m-chloroperbenzoic acid (83.2 mg, 410 µmol, 85% purity) was added. The mixture was allowed to react at room temperature for 0.5 hours. The reaction solution was concentrated under reduced pressure. Sodium bicarbonate (10 mL) and sodium sulfite (10 mL) were added for quenching. The mixture was extracted twice with DCM (50 mL), washed with 50 mL of saturated brine, dried over anhydrous sodium sulfate, and filtered. The filtrate was concentrated under reduced pressure to obtain compound **5-10.** MS m/z = 846.5 [M+H]⁺.

### Step 10

Compound **5-10** (280 mg, 331 µmol) was dissolved in anhydrous toluene (20 mL). 4Å molecular sieves (150 mg, 2.34 mmol), compound **1-2** (203 mg, 1.32 mmol), and sodium tert-butoxide (127 mg, 1.32 mmol) were added, and the mixture was reacted at 100°C for 12 hours. The reaction mixture was filtered and concentrated under reduced pressure to obtain a crude product. The crude product was purified by column chromatography (dichloromethane:methanol = 20:1) and separated by preparative high-performance liquid chromatography (Phenomenex luna C18 150*30mm*10µm; mobile phase: [water (0.225% formic acid)-acetonitrile]; (acetonitrile): 48%-78%). The mixture was then concentrated under reduced pressure to obtain the formate salt of compound 5-11. MS m/z = 935.7 [M+H]⁺. The residue was then subjected to preparative SFC separation (chiral column: DAICEL CHIRALCEL OD (250mm*30mm, 10µm); mobile phase: [supercritical carbon dioxide-acetonitrile/isopropanol (0.1% ammonia)]; acetonitrile/isopropanol (0.1% ammonia): 45%-45%), and concentrated under reduced pressure to obtain compounds **5-11A** and **5-11B.** After analytical SFC (chiral column: DAICEL CHIRALCEL OD-3 (50mm*4.6mm, 3µm); mobile phase: [supercritical carbon dioxide-methanol (0.05% diethylamine)]; methanol (0.05% diethylamine)%: 40%), compound **5-11A** had Rt of 0.657 minutes, and ee value of 99%; MS m/z = 935.6[M+H]⁺; compound **5-11B** had Rt of 1.848 minutes, and ee value of 99%, MS m/z = 935.5[M+H]⁺.

### Step 11

Compound **5-11A** (105 mg, 112 µmol) was dissolved in TFA (1 mL) and reacted at 50°C for 2 hours. The reaction solution was concentrated under reduced pressure to obtain a crude product, which was separated by preparative HPLC (Chromatographic column: Phenomenex luna C18 150*25mm*10µm; mobile phase: [water (0.075% trifluoroacetic acid)-acetonitrile]; acetonitrile: 12%-42%) and lyophilized to obtain the trifluoroacetate salt of compound **5A.** MS m/z = 595.4 [M+H]⁺. ¹H NMR (400 MHz, CD₃OD) δ ppm 6.96 (d, *J* = 2.4 Hz, 1H), 6.75 (d, *J* = 2.4 Hz, 1H), 5.34 - 5.28 (m, 2H), 5.11 (dd, *J* = 2.8, 10.8 Hz, 1H), 4.99 - 4.92 (m, 1H), 4.82 - 4.75 (m, 1H), 4.57 (d, *J =* 1.6 Hz, 2H), 4.44 - 4.30 (m, 2H), 4.21 - 4.11 (m, 2H), 3.96 - 3.83 (m, 2H), 3.81 - 3.66 (m, 2H), 3.33 (br s, 1H), 3.24 (td, *J* = 7.2, 11.6 Hz, 1H), 3.05 (br s, 1H), 3.03 - 2.98 (m, 1H), 2.87 - 2.74 (m, 2H), 2.41 - 2.32 (m, 1H), 2.30 - 2.19 (m, 2H), 2.18 - 2.05 (m, 4H), 2.05 - 1.93 (m, 4H).

Compound **5-11B** (110 mg, 118 µmol) was dissolved in TFA (1 mL) and reacted at 50°C for 2 hours. The reaction solution was concentrated under reduced pressure to obtain a crude product, which was separated by preparative HPLC (Chromatographic column: Phenomenex luna C18 150*25mm*10µm; mobile phase: [water (0.075% trifluoroacetic acid)-acetonitrile]; acetonitrile: 12%-42%) and lyophilized to obtain the trifluoroacetate salt of compound **5B.** MS m/z = 595.4 [M+H]⁺. ¹H NMR (400 MHz, CD₃OD) δ ppm 6.97 (d, *J* = 2.4 Hz, 1H), 6.76 (d, *J =* 2.4 Hz, 1H), 5.30 (br d, *J =* 8.0 Hz, 2H), 5.11 (br dd, *J =* 10.8, 2.8 Hz, 1H), 4.98 - 4.94 (m, 1H), 4.84 - 4.77 (m, 1H), 4.66 - 4.55 (m, 2H), 4.46 (br d, *J =* 14.0 Hz, 1H), 4.34 (br d, *J =* 14.0 Hz, 1H), 4.17 (br d, *J* = 16.0 Hz, 2H), 3.96 - 3.85 (m, 2H), 3.82 - 3.68 (m, 2H), 3.37 (br d, *J =* 14.0 Hz, 1H), 3.23 (td, *J =* 7.2, 11.6 Hz, 1H), 3.03 (br d, *J =* 16.4 Hz, 2H), 2.89 - 2.75 (m, 2H), 2.42 - 2.33 (m, 1H), 2.28 - 2.20 (m, 2H), 2.20 - 2.09 (m, 4H), 2.06 - 1.93 (m, 4H).

### Example 7

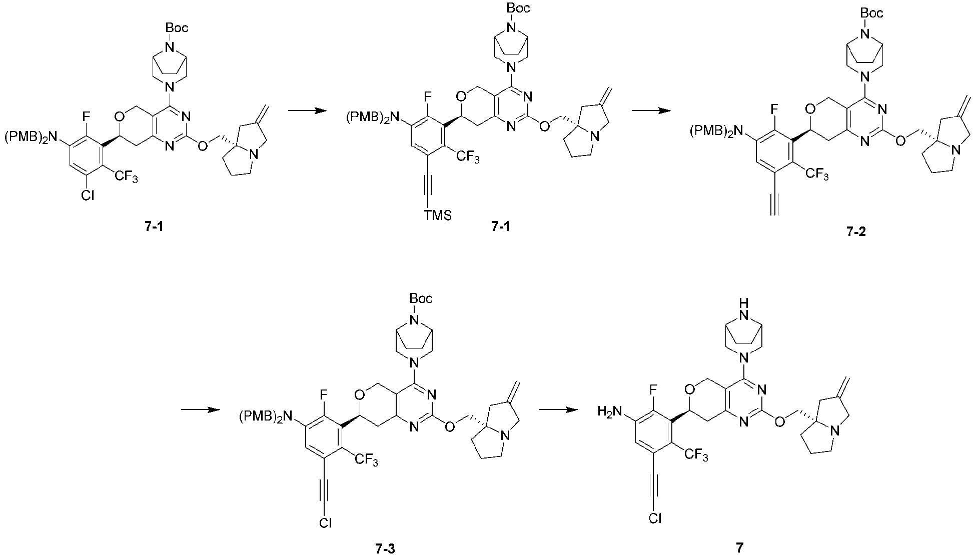

### Step 1

Compound **7-1** (1.20 g, 1.26 mmol) was dissolved in anhydrous toluene (12.0 mL), followed by the addition of tributyl (trimethylsilylethynyl)tin (2.94 g, 7.58 mmol) and dichlorobis[di-tert-butyl-(4-dimethylaminophenyl)phosphine]palladium (II) (537 mg, 758 µmol). The reaction was allowed to proceed at 110°C for 20 hours. The reaction solution was concentrated under reduced pressure to obtain a crude product. The crude product was purified by column chromatography (petroleum ether:ethyl acetate = 2:1) to obtain compound **7-1.** MS m/z = 1011.6 [M+1]⁺.

### Step 2

Compound **7-2** (1.00 g, 989 µmol) was dissolved in anhydrous tetrahydrofuran (10.0 mL), followed by the addition of tetrabutylammonium fluoride (1 M, 989 µL). The mixture was allowed to react at 25°C for 4 hours. 30.0 mL of water was added to the reaction solution, and the mixture was extracted with ethyl acetate (150 mL). The organic phase was dried and concentrated under reduced pressure to obtain a crude product. The crude product was purified by column chromatography (petroleum ether: ethyl acetate = 1:1) to obtain compound **7-2.** MS m/z = 939.6 [M+1]⁺.

### Step 3

Compound **7-2** (310 mg, 330.12 µmol) was dissolved in anhydrous acetone (5 mL), followed by the addition of AgNO₃ (350 mg, 2.06 mmol) and NCS (220.41 mg, 1.65 mmol). The reaction was allowed to proceed at 25°C for 10 hours. The reaction solution was concentrated under reduced pressure to obtain a crude product. The crude product was purified by preparative thin-layer chromatography (dichloromethane:methanol = 10:1) to obtain compound **7-3.** MS m/z = 973.4 [M+1]⁺.

### Step 4

Compound **7-3** (12.0 mg, 12.3 µmol) was dissolved in dichloromethane (1 mL), and trifluoroacetic acid (153 mg, 1.35 mmol, 0.1 mL) was added. The mixture was reacted at 25°C for 1 hour. The reaction solution was concentrated under reduced pressure to obtain a crude product. The crude product was separated by preparative HPLC (Chromatographic column: Phenomenex luna C18 150*25mm*10µm; mobile phase: [water (0.075% trifluoroacetic acid)-acetonitrile]; acetonitrile: 18%-48%) and lyophilized to obtain the trifluoroacetate salt of compound 7. MS m/z = 633.2[M+1]⁺. ¹H NMR (400 MHz, CD₃OD) δ ppm 7.00 (d, *J =* 8.5 Hz, 1H), 5.33 - 5.30 (m, 2H), 5.21 - 5.18 (m, 1H), 4.85 (s, 4H), 4.76 - 4.71 (m, 1H), 4.54 (s, 2H), 4.35 - 4.30 (m, 2H), 4.17 - 4.12 (m, 2H), 3.93 (d, *J =* 14.0 Hz, 1H), 3.86 - 3.70 (m, 2H), 3.68 - 3.64 (m, 1H), 3.07 - 2.88 (m, 2H), 2.82 - 2.73 (m, 1H), 2.37 - 2.29 (m, 1H), 2.28 - 2.18 (m, 2H), 2.17 - 2.05 (m, 4H), 2.01 - 1.92 (m, 1H).

### Example 8

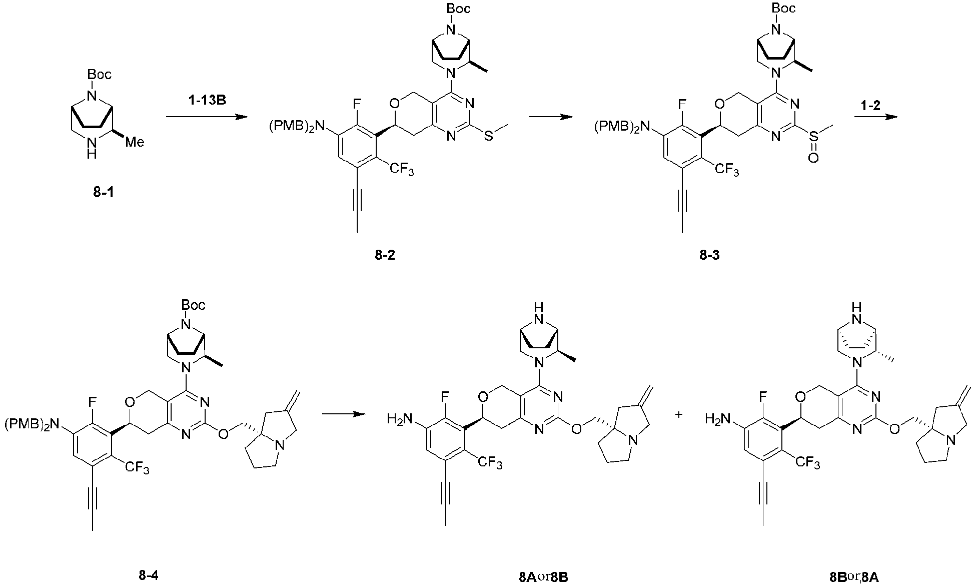

### Step 1

Compound **1-13B** (100 mg, 127 µmol), compound **8-1** (63.4 mg, 280 µmol), and triethylamine (509 µmol, 70.9 µL) were dissolved in DMF (1.00 mL). The atmosphere was purged with nitrogen three times and the mixture was reacted at 50°C for 6 hours. 10.0 mL of water was added to the reaction solution, and the mixture was extracted three times with ethyl acetate (20.0 mL). The organic phase was washed with saturated brine (50.0 mL), dried over anhydrous sodium sulfate, filtered, and concentrated under reduced pressure to obtain a crude product. The crude product was purified by preparative thin-layer chromatography (petroleum ether:ethyl acetate = 3:1) to obtain compound **8-2.** MS m/z = 862.4 [M+H]⁺.

### Step 2

Compound **8-2** (60.0 mg, 69.6 µmol) was dissolved in anhydrous dichloromethane (1.00 mL). Meta-chloroperbenzoic acid (15.5 mg, 76.6 µmol, 85.0% purity) was added portionwise at 0°C. The mixture was allowed to react at room temperature for 1 hour. 10.0 mL of sodium bicarbonate solution and 10.0 mL of sodium sulfite solution were added to the reaction solution, and the mixture was extracted three times with dichloromethane (30.0 mL). The organic phase was washed with saturated brine (50.0 mL), dried over anhydrous sodium sulfate, filtered, and concentrated under reduced pressure to obtain a crude product. The crude product was purified by preparative thin-layer chromatography (petroleum ether:ethyl acetate = 2:1) to obtain compound **8-3.** MS m/z = 878.3 [M+H]⁺.

### Step 3

Compound **8-3** (35.0 mg, 39.9 µmol) was dissolved in anhydrous toluene (1.00 mL). Compound **1-2** (12.2 mg, 79.7 µmol), sodium tert-butoxide (15.3 mg, 159 µmol), and 4Å molecular sieves (18.0 mg) were added. After replacing the atmosphere with nitrogen, the mixture was reacted at 100°C for 6 hours. The reaction solution was concentrated under reduced pressure to obtain a crude product. The crude product was purified by preparative thin-layer chromatography (pure ethyl acetate) to obtain compound **8-4.** MS m/z = 967.4 [M+H]⁺.

### Step 4

Compound **8-4** (30.0 mg, 31.0 µmol) was dissolved in anhydrous dichloromethane (0.5 mL), and trifluoroacetic acid (1.35 mmol, 100 µL) was added. The mixture was allowed to react at room temperature for 2 hours. The reaction solution was concentrated under reduced pressure to obtain a crude product, which was then separated by preparative high-performance liquid chromatography (Phenomenex luna C18 150*25mm*10µm column; mobile phase: [water (0.075% trifluoroacetic acid)-acetonitrile]; acetonitrile: 16%-46%). After lyophilization, the trifluoroacetic acid salts of compounds **8A** and **8B** were obtained.

Trifluoroacetate salt of compound **8A:** MS m/z = 627.3 [M+H]⁺, ¹H NMR (400 MHz, CD₃OD) δ ppm 6.92 (d, *J* = 8.5 Hz, 1H), 5.31 (br d, *J* = 8.5 Hz, 2H), 5.19 (br dd, *J* = 3.6, 11.8 Hz, 1H), 4.97 - 4.93 (m, 1H), 4.73 (br d, *J* = 14.6 Hz, 1H), 4.65 - 4.45 (m, 2H), 4.34 (br d, *J* = 14.4 Hz, 1H), 4.14 (br d, *J* = 5.5 Hz, 1H), 4.03 - 3.89 (m, 3H), 3.81 - 3.70 (m, 1H), 3.42 - 3.34 (m, 1H), 3.29 - 3.23 (m, 1H), 3.22 - 3.15 (m, 1H), 3.07 - 2.91 (m, 3H), 2.81 (br d, *J* = 16.0 Hz, 1H), 2.36 (br dd, *J =* 5.9, 11.4 Hz, 2H), 2.31 - 2.10 (m, 5H), 2.06 - 1.93 (m, 4H), 1.15 (d, *J* = 6.0 Hz, 3H).

Trifluoroacetic acid salt of compound **8B,** MS m/z = 627.3 [M+H]⁺, ¹H NMR (400 MHz, CD₃OD) δ ppm 6.93 (d, *J =* 8.5 Hz, 1H), 5.31 (br d, *J =* 8.0 Hz, 2H), 5.10 - 4.99 (m, 2H), 4.75 (s, 1H), 4.57 (s, 2H), 4.33 (br d, *J* = 14.3 Hz, 1H), 4.21 - 4.09 (m, 1H), 3.98 - 3.84 (m, 3H), 3.82 - 3.71 (m, 1H), 3.45 (br dd, *J* = 11.2, 17.7 Hz, 1H), 3.28 - 3.18 (m, 2H), 3.15 - 3.01 (m, 2H), 2.92 - 2.77 (m, 2H), 2.40 - 2.11 (m, 7H), 2.03 - 1.92 (m, 4H), 1.02 (d, *J* = 6.3 Hz, 3H).

### Example 9

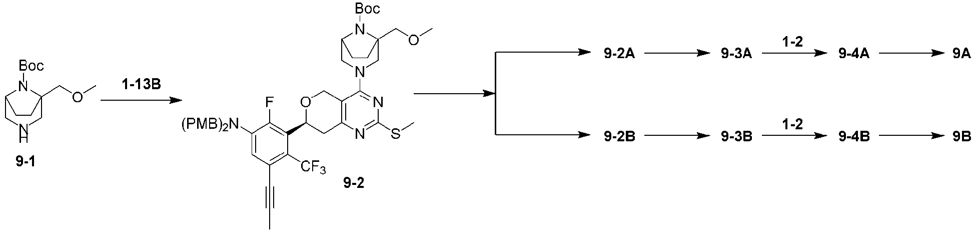

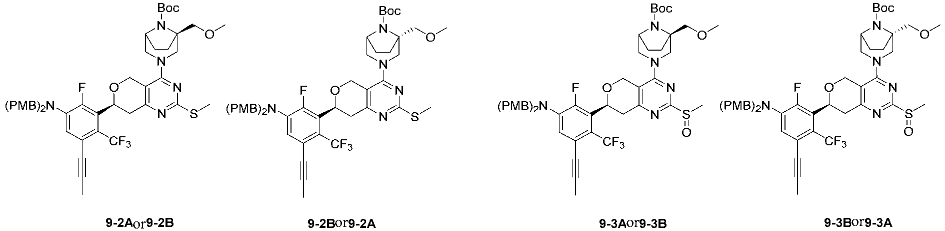

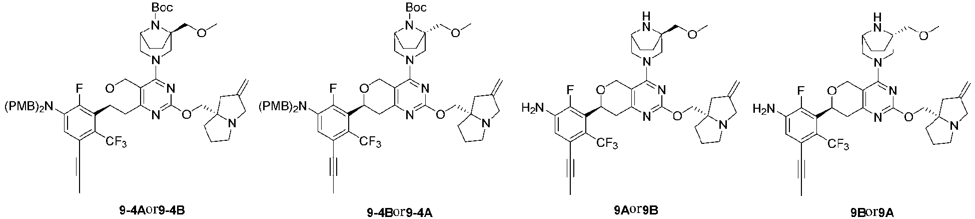

### Step 1

Compound **1-13B** (49.0 mg, 62.4 µmol) and compound **9-1** (48.0 mg, 187 µmol) were dissolved in dichloromethane (1.00 mL). *N,N-*diisopropylethylamine (54.3 µL) was added, and the reaction mixture was reacted at 25°C for 12 hours. The reaction mixture was concentrated under reduced pressure to obtain a crude product. The crude product was purified by column chromatography (petroleum ether:ethyl acetate = 10:1) to obtain compound **9-2.** MS m/z = 892.4 [M+1]⁺. Preparative SFC separation was then performed (chiral column: (s,s) WHELK-O1 (250mm*30mm,10µm); mobile phase: [supercritical carbon dioxide-acetonitrile/isopropanol (0.1% ammonia)]; acetonitrile/isopropanol (0.1% ammonia): 50%-50%). After concentration under reduced pressure, compounds **9-2A** and **9-2B** were obtained. Analytical SFC: (chiral column: (s,s) WHELK-O1 (50mm*4.6mm,3.5µm); mobile phase: [supercritical carbon dioxide-isopropanol (0.05% diethylamine)]; isopropanol (0.05% diethylamine)%: 40%), compound **9-2A,** Rt = 1.768 minutes, 99% ee; MS m/z = 892.4 [M+H]⁺; compound **9-2B,** Rt = 2.286 min, 99% ee, MS m/z = 892.4 [M+H]⁺.

### Step 2

Compound **9-2A** (30.0 mg, 33.6 µmol) was dissolved in dichloromethane (1.00 mL), and m-chloroperbenzoic acid (8.19 mg, 40.4 µmol, 85.0% purity) was added. The reaction mixture was reacted at 25°C for 2 hours. The reaction mixture was quenched with saturated sodium sulfite solution. The organic phase was collected and concentrated under reduced pressure to obtain a crude product. The crude product was purified by column chromatography (petroleum ether:ethyl acetate = 1:1) to obtain compound **9-3A.** m/z = 908.4 [M+1]⁺.

Referring to step 2, compound **9-2B** was used as a starting material in place of compound **9-2A** to obtain compound **9-3B.** m/z = 908.4 [M+1]⁺.

### Step 3

Compound **9-3A** (12.0 mg, 13.2 µmol), compound **1-2** (6.07 mg, 39.7 µmol), sodium tert-butoxide (3.81 mg, 39.7 µmol), and 4Å molecular sieves (12.0 mg) were added to toluene (1.00 mL). The reaction mixture was reacted at 110°C for 12 hours. The reaction mixture was cooled to room temperature, and filtered. The filtrate was concentrated to obtain a crude product. The crude product was purified by column chromatography (dichloromethane:methanol = 10:1) to obtain compound **9-4A.** MS m/z = 997.6 [M+1]⁺.

Referring to step 3, compound **9-3B** was used as a starting material in place of compound **9-3A** to obtain compound **9-4B.** MS m/z = 997.6 [M+1]⁺.

### Step 4

Compound **9-4A** (10.0 mg, 10.0 µmol) was dissolved in dichloromethane (1.00 mL), and trifluoroacetic acid (0.30 mL) was added. The reaction mixture was reacted at 25°C for 1 hour. The reaction mixture was concentrated under reduced pressure to obtain a crude product. The crude product was purified by preparative HPLC (Chromatographic column: Phenomenex luna C18 150*25mm*10µm; mobile phase: [water (0.075% trifluoroacetic acid)-acetonitrile]; acetonitrile: 15%-45%) to obtain the trifluoroacetate salt of **9A**. MS m/z = 657.4 [M+1]⁺, ¹H NMR (400 MHz, CD₃OD) δ ppm 6.97 - 6.92 (m, 1H), 5.33 (br d, *J =* 7.8 Hz, 2H), 5.28 - 5.17 (m, 1H), 4.76 - 4.72 (m, 1H), 4.56 (s, 2H), 4.41 - 4.28 (m, 2H), 4.22 - 4.15 (m, 1H), 3.99 - 3.91 (m, 1H), 3.79 - 3.63 (m, 5H), 3.52 - 3.48 (m, 3H), 3.35 (br d, *J =* 1.8 Hz, 1H), 3.31 - 3.23 (m, 3H), 3.09 - 2.79 (m, 3H), 2.40 - 2.10 (m, 6H), 2.04 (s, 3H), 2.01 - 1.88 (m, 2H).

Compound **9-4B** (13.0 mg, 13.0 µmol) was dissolved in dichloromethane (1.00 mL), and trifluoroacetic acid (0.30 mL) was added. The reaction mixture was reacted at 25°C for 1 hour. The reaction mixture was concentrated under reduced pressure to obtain a crude product. The crude product was purified by preparative high-performance liquid chromatography (Chromatographic column: Phenomenex luna C18 150*25mm*10µm; mobile phase: [water (0.075% trifluoroacetic acid)-acetonitrile]; acetonitrile: 15%-45%) to obtain the trifluoroacetate salt **of 9B.** MS m/z = 657.5 [M+1]⁺, ¹H NMR (400 MHz, CD₃OD) δ ppm 6.94 (d, *J =* 8.5 Hz, 1H), 5.33 (br d, *J =* 7.6 Hz, 2H), 5.26 - 5.18 (m, 1H), 4.78 - 4.73 (m, 1H), 4.57 (s, 2H), 4.39 - 4.28 (m, 2H), 4.17 - 4.12 (m, 1H), 3.94 (br d, *J =* 14.4 Hz, 1H), 3.85 - 3.63 (m, 5H), 3.50 (s, 3H), 3.41 - 3.34 (m, 2H), 3.31 - 3.21 (m, 2H), 3.09 - 2.78 (m, 3H), 2.41 - 2.12 (m, 6H), 2.04 (s, 3H), 2.02 - 1.91 (m, 2H).

### Example 10

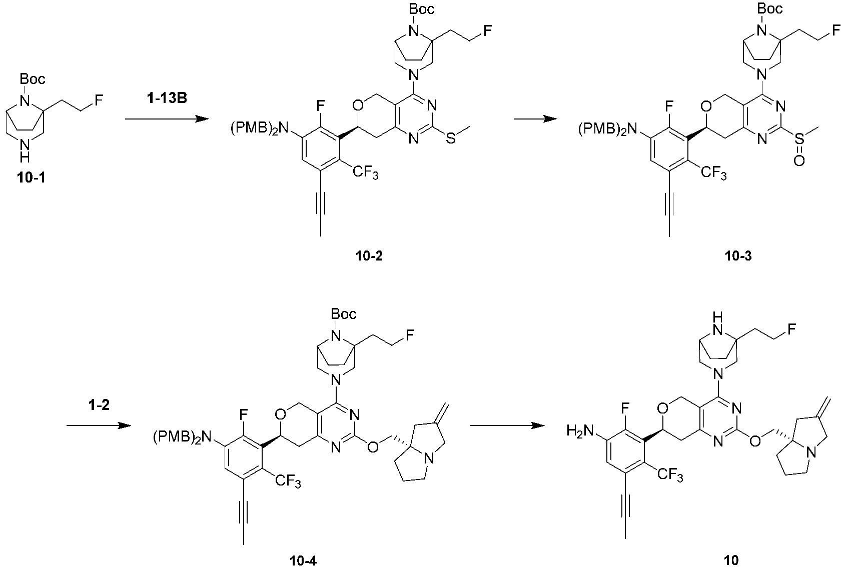

### Step 1

Compound **1-13B** (49.0 mg, 62.4 µmol) and compound **10-1** (48.0 mg, 187 µmol) were dissolved in dichloromethane (1.00 mL). N,N-diisopropylethylamine (40.3 mg, 312 µmol, 54.3 µL) was added, and the reaction mixture was reacted at 25°C for 12 hours. The reaction mixture was directly concentrated to obtain a crude product. The crude product was purified by column chromatography (petroleum ether:ethyl acetate = 5:1) to obtain compound **10-2.** MS m/z = 894.4 [M+1]⁺.

### Step 2

Compound **10-2** (50.0 mg, 55.9 µmol) was dissolved in dichloromethane (1.00 mL), and m-chloroperbenzoic acid (13.6 mg, 67.1 µmol, 85.0% purity) was added. The reaction mixture was reacted at 25°C for 1 hour. The reaction mixture was quenched with saturated sodium sulfite solution, and the aqueous phase was extracted with dichloromethane (10.0 mL x 2). The organic phase was collected and concentrated to obtain a crude product. The crude product was purified by column chromatography (petroleum ether:ethyl acetate = 1:1) to obtain compound **10-3.** m/z = 910.3 [M+1]⁺.

### Step 3

Compound **10-3** (30.0 mg, 32.9 µmol), compound **1-2** (15.2 mg, 98.9 µmol), sodium tert-butoxide (9.50 mg, 98.9 µmol), and 4Å molecular sieves (30.0 mg) were added to toluene (1.00 mL). The reaction mixture was reacted at 110°C for 12 hours. The reaction mixture was cooled to room temperature, and filtered. The filtrate was concentrated to obtain a crude product. The crude product was purified by column chromatography (dichloromethane:methanol = 10:1) to obtain compound **10-4.** MS m/z = 999.6 [M+1]⁺.

### Step 4

Compound **10-4** (25.0 mg, 25.0 µmol) was dissolved in dichloromethane (1.00 mL), and trifluoroacetic acid (0.30 mL) was added. The reaction mixture was allowed to react at 25°C for 1 hour. The reaction mixture was concentrated to obtain a crude product. The crude product was then purified by preparative HPLC (Chromatographic column: Phenomenex luna C18 150*25mm*10µm; mobile phase: [water (0.075% trifluoroacetic acid)-acetonitrile]; acetonitrile: 12%-42%) to obtain the trifluoroacetate salt of **10.** MS m/z = 659.3 [M+1]⁺, ¹H NMR (400 MHz, CD₃OD) δ ppm 6.92 (d, *J* = 8.5 Hz, 1H), 5.54 - 5.52 (m, 1H), 5.31 (br d, *J* = 7.6 Hz, 2H), 5.23 - 5.13 (m, 1H), 4.94 - 4.90 (m, 1H), 4.85 - 4.63 (m, 5H), 4.62 - 4.25 (m, 4H), 4.23 - 4.11 (m, 1H), 3.97 - 3.67 (m, 4H), 3.40 - 3.33 (m, 1H), 3.28 - 3.22 (m, 1H), 3.10 - 2.89 (m, 2H), 2.80 (br d, *J =* 15.9 Hz, 1H), 2.44 - 2.33 (m, 2H), 2.27 - 2.11 (m, 5H), 2.02 (s, 3H), 1.98 - 1.91 (m, 1H).

### Example 11

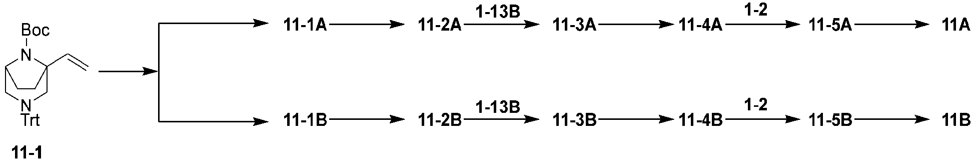

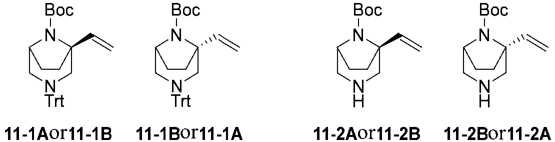

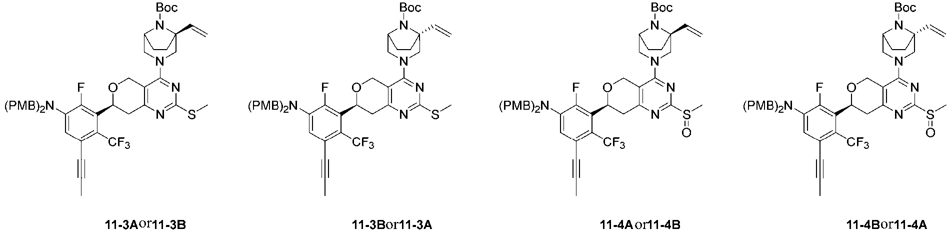

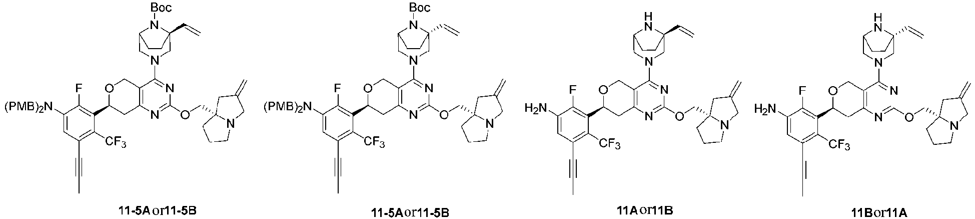

### Step 1

Compound **11-1** was subjected to preparative SFC separation (chiral column: DAICEL CHIRALPAK AD (250mm*30mm, 10µm; mobile phase: [supercritical carbon dioxide - acetonitrile / isopropanol (0.1% ammonia)]; acetonitrile / isopropanol (0.1% ammonia): 30%-30%). After concentration under reduced pressure, compounds **11-1A** and **11-1B** were obtained. Analytical SFC: (chiral column: DAICEL CHIRALPAK AD (50mm*4.6mm, 3µm); mobile phase: [supercritical carbon dioxide-isopropanol (0.05% diethylamine)]; isopropanol (0.05% diethylamine)%: 5%-40%), compound **11-1A,** Rt = 1.308 minutes, 99% ee; MS m/z = 503.2 [M+Na]⁺; compound **11-1B,** Rt = 1.499 min, 99% ee, MS m/z = 503.2 [M+Na]⁺.

### Step 2

Compound **11-1A** (238 mg, 495 µmol) was dissolved in acetic acid (4.00 mL), reacted at 25°C for 12 hours, and then concentrated under reduced pressure to remove acetic acid to obtain the acetate salt of compound **11-2A.**

Referring to step 2, compound **11-1B** was used as a raw material in place of compound **11-1A** to obtain the acetate salt of compound **11-2B.**

### Step 3

Compound **1-13B** (200 mg, 254 µmol) and compound **11-2A** (182 mg, acetate salt) were dissolved in dichloromethane (3.00 mL). *N,N*-diisopropylethylamine (1.27 mmol, 222 µL) was added, and the reaction mixture was reacted at 25°C for 12 hours. The reaction mixture was directly concentrated to obtain a crude product. The crude product was purified by column chromatography (petroleum ether:ethyl acetate = 5:1) to obtain compound **11-3A.** MS m/z = 874.4 [M+1]⁺.

Referring to step 3, the acetate salt of compound **11-2B** was used as a starting material in place of the acetate salt of compound **11-2A** to obtain compound **11-3B.** MS m/z = 874.4 [M+1]⁺.

### Step 4

Compound **11-3A** (180 mg, 206 µmol) was dissolved in dichloromethane (3.00 mL), and m-chloroperbenzoic acid (43.9 mg, 216 µmol, 85.0% purity) was added. The reaction mixture was reacted at 25°C for 2 hours. 10.0 mL of sodium bicarbonate solution and 10.0 mL of sodium sulfite solution were added to the reaction mixture. The aqueous phase was extracted with dichloromethane (30.0 mL x 2). The organic phase was collected and concentrated to obtain a crude product. The crude product was purified by column chromatography (petroleum ether:ethyl acetate = 1:1) to obtain compound **11-4A.**

Referring to step 4, compound **11-3B** was used as a starting material in place of compound **11-3A** to obtain compound **11-4B.**

### Step 5

Compound **11-4A** (140 mg, 157 µmol), compound **1-2** (48.2 mg, 315 µmol), sodium tert-butoxide (60.5 mg, 629 µmol), and 4Å molecular sieves (70.0 mg) were added to toluene (3.00 mL). The reaction mixture was reacted at 110°C for 12 hours. The reaction mixture was cooled to room temperature, and filtered. The filtrate was concentrated to obtain a crude product. The crude product was purified by column chromatography (petroleum ether:ethyl acetate = 1:2) to obtain compound **11-5A.** MS m/z = 979.5 [M+1]⁺.

Referring to step 4, compound **11-4B** was used as a starting material in place of compound **11-4A** to obtain compound **11-5B.** MS m/z = 979.5 [M+1]⁺.

### Step 6

Compound **11-5A** (72.0 mg, 73.5 µmol) was dissolved in dichloromethane (1.00 mL), and trifluoroacetic acid (0.20 mL) was added. The reaction mixture was allowed to react at 25°C for 2 hours. The reaction mixture was concentrated to obtain a crude product. The crude product was then purified by preparative HPLC (Chromatographic column: Phenomenex luna C18 150*25mm*10µm; mobile phase: [water (0.075% trifluoroacetic acid)-acetonitrile]; acetonitrile: 16%-46%) to obtain the trifluoroacetate salt of **11A.** MS m/z = 639.3 [M+H]⁺, ¹H NMR (400 MHz, CD₃OD) δ ppm 6.92 (d, *J =* 8.6 Hz, 1H), 6.15 (dd, *J =* 11.2, 17.7 Hz, 1H), 5.61 - 5.46 (m, 2H), 5.31 (br d, *J =* 7.5 Hz, 2H), 5.25 - 5.17 (m, 1H), 5.09 - 4.97 (m, 1H), 4.77 - 4.72 (m, 2H), 4.55 (s, 2H), 4.39 - 4.22 (m, 3H), 3.98 - 3.70 (m, 3H), 3.55 (d, *J* = 13.6 Hz, 1H), 3.28 - 3.21 (m, 2H), 3.09 - 2.90 (m, 2H), 2.79 (br d, *J =* 16.3 Hz, 1H), 2.40 - 2.05 (m, 8H), 2.02 (s, 3H).

Compound **11-5B** (64.0 mg, 65.4 µmol) was dissolved in dichloromethane (1.00 mL), and trifluoroacetic acid (0.20 mL) was added. The reaction mixture was allowed to react at 25°C for 2 hours. The reaction mixture was concentrated to obtain a crude product. The crude product was then purified by preparative HPLC (Chromatographic column: Phenomenex luna C18 150*25mm*10µm; mobile phase: [water (0.075% trifluoroacetic acid)-acetonitrile]; acetonitrile: 16%-46%) to obtain the trifluoroacetate salt of **11B.** MS m/z = 639.3 [M+H]⁺, ¹H NMR (400 MHz, CD₃OD) δ ppm 6.92 (d, *J* = 8.5 Hz, 1H), 6.18 (dd, *J* = 11.2, 17.7 Hz, 1H), 5.66 - 5.48 (m, 2H), 5.31 (br d, *J =* 7.4 Hz, 2H), 5.20 (br dd, *J =* 4.1, 10.9 Hz, 1H), 4.95 - 4.91 (m, 1H), 4.78 - 4.71 (m, 2H), 4.55 (s, 2H), 4.45 - 4.16 (m, 3H), 3.98 - 3.62 (m, 4H), 3.27 - 3.22 (m, 2H), 3.09 - 2.88 (m, 2H), 2.79 (br d, *J* = 16.3 Hz, 1H), 2.56 - 2.03 (m, 8H), 2.02 (s, 3H).

### Example 12

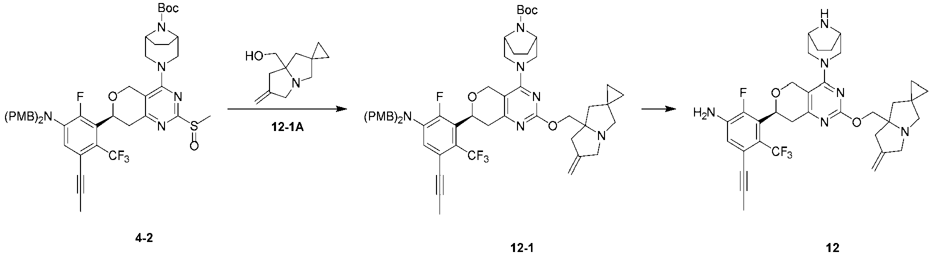

### Step 1

Compound **4-2** (100 mg, 115 µmol), compound **12-1A** (83.0 mg, 463 µmol), sodium tert-butoxide (44.5 mg, 463 µmol), and 4Å molecular sieves (100.0 mg) were added to toluene (5.00 mL). The reaction mixture was reacted at 110°C for 12 hours. The reaction mixture was cooled to room temperature, and filtered. The filtrate was concentrated to obtain a crude product. The crude product was purified by column chromatography (dichloromethane:methanol = 10:1) to obtain compound **12-1.** MS m/z = 979.5 [M+1]⁺.

### Step 2

Compound **12-1** (90.0 mg, 91.9 µmol) was dissolved in dichloromethane (1.00 mL), and trifluoroacetic acid (0.30 mL) was added. The reaction mixture was allowed to react at 25°C for 2 hours. The reaction mixture was concentrated to obtain a crude product. The crude product was then purified by preparative HPLC (Chromatographic column: Phenomenex luna C18 150*25mm*10µm; mobile phase: [water (0.075% trifluoroacetic acid)-acetonitrile]; acetonitrile: 16%-46%) to obtain the trifluoroacetate salt of 12. MS m/z = 639.4 [M+H]⁺, ¹H NMR (400 MHz, D₂O) δ ppm 7.12 (d, *J =* 8.5 Hz, 1H), 5.33 - 5.26 (m, 2H), 5.05 - 4.94 (m, 2H), 4.36 - 4.19 (m, 4H), 3.99 - 3.87 (m, 3H), 3.62 - 3.25 (m, 5H), 3.21 - 2.85 (m, 4H), 2.31 - 2.07 (m, 6H), 2.06 - 2.01 (m, 3H), 1.96 - 1.86 (m, 1H), 0.87 - 0.72 (m, 4H).

### Example 13

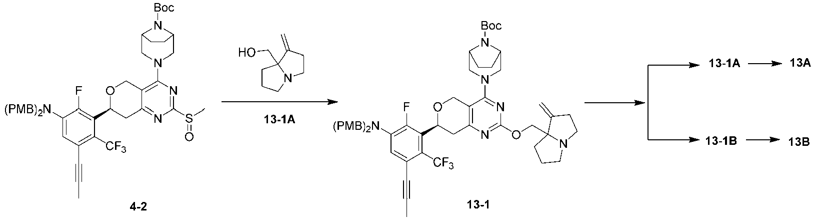

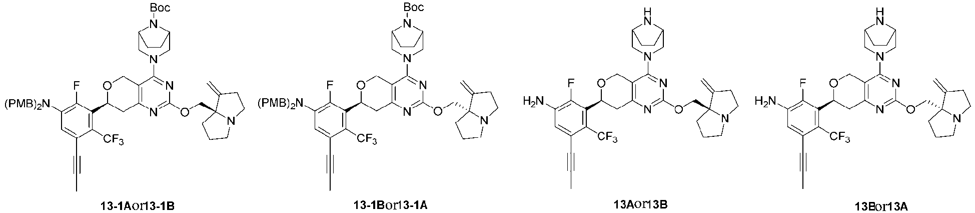

### Step 1

Compound **4-2** (50.0 mg, 57.9 µmol), compound **13-1A** (26.6 mg, 174 µmol), sodium tert-butoxide (27.8 mg, 289 µmol), and 4 Å molecular sieves (50.0 mg) were added to toluene (2.00 mL). The reaction mixture was reacted at 110°C for 12 hours. The reaction mixture was cooled to room temperature, and filtered. The filtrate was concentrated to obtain a crude product. The crude product was purified by column chromatography (dichloromethane:methanol = 10:1) to obtain compound **13-1.** MS m/z = 953.5 [M+1]⁺. Compound **13-1** was subjected to preparative SFC separation (chiral column: (s,s) WHELK-O1 (250mm*30mm,10µm); mobile phase: [supercritical carbon dioxide - acetonitrile / isopropanol (0.1% ammonia)]; acetonitrile / isopropanol (0.1% ammonia): 38%-38%) and concentrated under reduced pressure to obtain compounds **13-1A** and **13-1B.** Analytical SFC: (chiral column: (s,s) WHELK-O1 (50mm*4.6mm,3.5µm); mobile phase: [supercritical carbon dioxide-isopropanol (0.05% diethylamine)]; isopropanol (0.05% diethylamine)%: 40%), compound **13-1A,** Rt=1.495min, 99% ee; MS m/z = 953.5 [M+H]⁺; compound **13-1B,** Rt=1.716min, ee value 95%, MS m/z = 953.5 [M+H]⁺.

### Step 2

Compound **13-1A** (17.0 mg, 17.8 µmol) was dissolved in dichloromethane (1.00 mL), and trifluoroacetic acid (0.30 mL) was added. The reaction mixture was allowed to react at 25°C for 1 hour. The reaction mixture was concentrated to obtain a crude product. The crude product was then purified by preparative HPLC (Chromatographic column: Phenomenex luna C18 150*25mm*10µm; mobile phase: [water (0.075% trifluoroacetic acid)-acetonitrile]; acetonitrile: 10%-40%) to obtain the trifluoroacetate salt of **13A.** MS m/z =613.4 [M+H]⁺, ¹H NMR (400 MHz, CD₃OD) δ ppm 6.92 (d, *J =* 8.5 Hz, 1H), 5.41 - 5.27 (m, 2H), 5.18 (br dd, *J* = 3.8, 10.8 Hz, 1H), 4.77 - 4.70 (m, 1H), 4.68 - 4.50 (m, 2H), 4.32 (br d, *J=* 14.4 Hz, 1H), 4.22 - 4.10 (m, 2H), 3.88 - 3.63 (m, 4H), 3.48 - 3.32 (m, 4H), 3.27 (br d, *J =* 12.1 Hz, 1H), 3.03 - 2.88 (m, 3H), 2.53 - 2.40 (m, 1H), 2.32 - 2.09 (m, 6H), 2.04 - 2.01 (m, 3H), 2.01 - 1.93 (m, 1H).

Compound **13-1B** (20.0 mg, 21.0 µmol) was dissolved in dichloromethane (1.00 mL), and trifluoroacetic acid (0.30 mL) was added. The reaction mixture was allowed to react at 25°C for 1 hour. The reaction mixture was concentrated to obtain a crude product. The crude product was then purified by preparative HPLC (Chromatographic column: Phenomenex luna C18 150*25mm*10µm; mobile phase: [water (0.075% trifluoroacetic acid)-acetonitrile]; acetonitrile: 10%-40%) to obtain the trifluoroacetate salt of **13B.** MS m/z =613.5 [M+H]⁺, ¹H NMR (400 MHz, CD₃OD) δ ppm 6.94 (d, *J =* 8.5 Hz, 1H), 5.44 - 5.30 (m, 2H), 5.20 (dd, J = 3.8, 11.3 Hz, 1H), 4.79 - 4.73 (m, 1H), 4.70 - 4.56 (m, 2H), 4.39 (br d, *J =* 14.0 Hz, 1H), 4.22 - 4.13 (m, 2H), 3.88 - 3.69 (m, 4H), 3.47 - 3.34 (m, 4H), 3.32 - 3.27 (m, 1H), 3.01 - 2.94 (m, 3H), 2.53 - 2.40 (m, 1H), 2.32 - 2.12 (m, 6H), 2.04 (s, 3H), 2.02 - 1.94 (m, 1H).

### Example 14

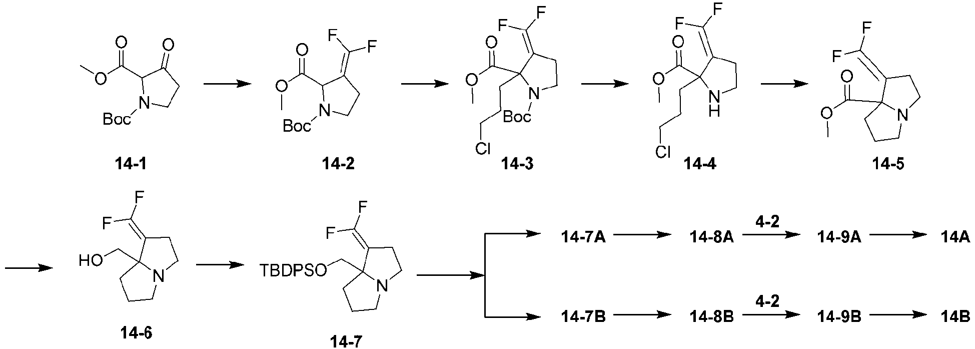

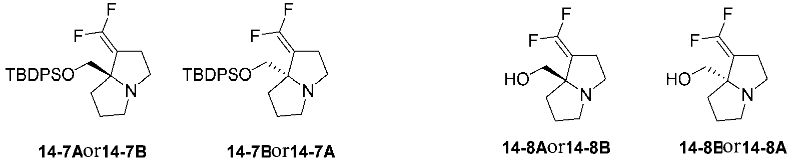

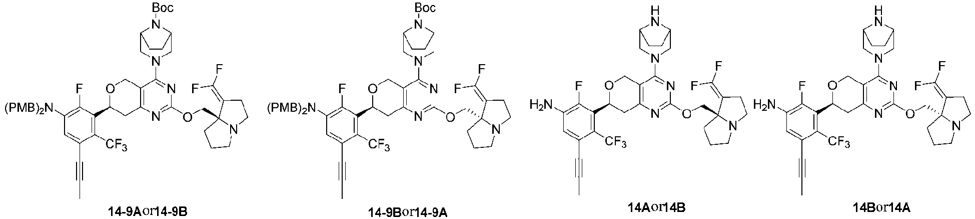

### Step 1

Compound **14-1** (5.00 g, 20.6 mmol) was dissolved in DMF (50.0 mL), followed by the addition of triphenylphosphonium difluoroacetate (19.0 g, 53.4 mmol). The reaction mixture was reacted at 80°C for 2 hours. 300 mL of water was added to the reaction solution, and the mixture was extracted three times with ethyl acetate (200 mL). The organic phase was washed with saturated brine (200 mL), dried over anhydrous sodium sulfate, filtered, and concentrated to obtain a crude product. The crude product was purified by column chromatography (petroleum ether:ethyl acetate = 100:1 to 3:1) to obtain compound **14-2.** ¹H NMR (400 MHz, CDCl₃) 5.00 - 4.76 (m, 1H), 3.82 - 3.65 (m, 4H), 3.61 - 3.45 (m, 1H), 2.71 - 2.55 (m, 2H), 1.49 - 1.38 (m, 9H).

### Step 2

Compound **14-2** (2.80 g, 10.1 mmol) was dissolved in tetrahydrofuran (3.00 mL), and lithium diisopropylamide (2.00 M, 10.1 mL) was added. The mixture was reacted at -60°C for 1 hour. 1-Chloro-3-iodopropane (10.3 g, 50.5 mmol, 1.55 mL) was then added and the mixture was reacted at -60°C for 1 hour, followed by 12 hours at 25°C. 50.0 mL of ammonium chloride solution was added to the reaction solution, and the mixture was extracted three times with ethyl acetate (50.0 mL). The organic phase was washed with saturated brine (100 mL), dried over anhydrous sodium sulfate, filtered, and concentrated to obtain a crude product. The crude product was purified by column chromatography (petroleum ether:ethyl acetate = 50:1-10:1) to obtain compound **14-3.** MS m/z = 376.1 [M+Na]⁺.

### Step 3

Compound **14-3** (3.20 g, 9.04 mmol) was dissolved in acetonitrile (1.00 mL) and hydrochloric acid/dioxane (2.00 M, 5.00 mL) was added. The mixture was reacted at 25°C for 12 hours. The mixture was concentrated under reduced pressure to remove the solvent, obtaining the hydrochloride salt of compound **14-4.** MS m/z = 254.1 [M+H]⁺.

### Step 4

Compound **14-4** (2.50 g, hydrochloride salt) was dissolved in acetonitrile (25.0 mL). Potassium carbonate (5.95 g, 43.1 mmol) and potassium iodide (143 mg, 862 µmol) were added. The mixture was reacted at 25°C for 12 hours. The mixture was filtered and concentrated to obtain a crude product. The crude product was purified by column chromatography (petroleum ether:ethyl acetate = 50:1-20:1) to obtain compound **14-5.** MS m/z = 218 [M+H]⁺.

### Step 5

Compound **14-5** (300 mg, 1.38 mmol) was dissolved in THF (3.00 mL) and lithium aluminum tetrahydride was added (2.50 M, 1.10 mL). The mixture was then reacted at 0°C for 1 hour. To the reaction solution, 0.11 mL of water, 0.11 mL of 15% sodium hydroxide solution, and 0.33 mL of water were added. The reaction solution was filtered and concentrated to obtain compound **14-6.** MS m/z = 190.1[M+H]⁺.

### Step 6

Compound **14-6** (300 mg, 1.59 mmol) was dissolved in anhydrous dichloromethane (3.00 mL). Imidazole (432 mg, 6.34 mmol), 4-dimethylaminopyridine (19.37 mg, 159 µmol), and tert-butyldiphenylsilyl chloride (872 mg, 3.17 mmol, 812 µL) were added and reacted at 25°C for 12 hours. The reaction solution was concentrated to obtain a crude product. The crude product was separated by preparative HPLC (Chromatographic column: Phenomenex luna C18 150*25mm*10µm; mobile phase: [water (0.075% trifluoroacetic acid)-acetonitrile]; acetonitrile: 25%-55%) to obtain compound **14-7.** MS m/z = 428.3 [M+H]⁺. Compound 14-7 was subjected to preparative SFC separation (chiral column: DAICEL CHIRALCEL OX (250mm*50mm, 10µm); mobile phase: [supercritical carbon dioxide -methanol (0.1% ammonia)]; methanol (0.1% ammonia): 10%-10%) and concentrated under reduced pressure to obtain compounds **14-7A** and **14-7B.** Analytical SFC: (chiral column: DAICEL CHIRALCEL OX (50mm*4.6mm, 3µm); mobile phase: [supercritical carbon dioxide-methanol (0.05% diethylamine)]; methanol (0.05% diethylamine)%: 5%-40%), compound **14-7A,** Rt=1.165min, 99% ee; MS m/z = 428.2 [M+H]⁺; compound **14-7B,** Rt=1.233min, ee value 95%, MS m/z = 428.2 [M+H]⁺.

### Step 7

Compound **14-7A** (126 mg, 295 µmol) was dissolved in dioxane (2.00 mL) and hydrochloric acid (12 M, 0.50 mL) was added. The mixture was reacted at 95°C for 12 hours, and then dissolved by adding 2 mL of water. The mixture was extracted with ethyl acetate (10 mL), and the aqueous phase was lyophilized to obtain the hydrochloride salt of compound **14-8A.** MS m/z = 190.1 [M+H]⁺.

Referring to step 7, compound **14-7B** was used as a starting material in place of compound **14-7A** to obtain the hydrochloride salt of compound **14-8B.** MS m/z = 190.1 [M+H]⁺.

### Step 8

Compound **4-2** (120 mg, 139 µmol), compound **14-8A** (62.7 mg, hydrochloride), sodium tert-butoxide (66.7 mg, 695 µmol), and 4Å molecular sieves (30.0 mg) were added to toluene (3.00 mL). The reaction solution was reacted at 100°C for 6 hours. The reaction solution was cooled to room temperature, and filtered. The filtrate was concentrated to obtain a crude product. The crude product was purified by thin-layer chromatography (pure ethyl acetate) to obtain compound **14-9A.** MS m/z = 989.7 [M+1]⁺.

Referring to step 8, compound **14-8B** was used as a starting material in place of compound **14-8A** to obtain compound **14-9B.** MS m/z = 989.6 [M+H]⁺.

### Step 9

Compound **14-9A** (63.0 mg, 63.7 µmol) was dissolved in dichloromethane (4.00 mL), and trifluoroacetic acid (1.00 mL) was added. The reaction mixture was allowed to react at 25°C for 12 hours. The reaction mixture was concentrated to obtain a crude product. The crude product was then purified by preparative HPLC (Chromatographic column: Phenomenex luna C18 150*25mm*10µm; mobile phase: [water (0.075% trifluoroacetic acid)-acetonitrile]; acetonitrile: 16%-46%) to obtain the trifluoroacetate salt of **14A.** MS m/z = 649.2 [M+H]⁺, ¹H NMR (400 MHz, MeOD) δ ppm 6.92 (d, *J =* 8.5 Hz, 1H), 5.19 (br dd, J= 3.9, 11.1 Hz, 1H), 4.96 - 4.90 (m, 1H), 4.84 - 4.78 (m, 1H), 4.77 - 4.70 (m, 2H), 4.66 - 4.58 (m, 1H), 4.32 (br d, *J =* 14.0 Hz, 1H), 4.15 (br dd, *J =* 3.0, 13.6 Hz, 2H), 3.97 - 3.63 (m, 4H), 3.57 - 3.39 (m, 2H), 3.36 - 3.33 (m, 1H), 3.09 - 2.87 (m, 3H), 2.49 - 2.05 (m, 7H), 2.04 - 1.93 (m, 4H).

Compound **14-9B** (88.0 mg, 89.0 µmol) was dissolved in dichloromethane (4.00 mL), and trifluoroacetic acid (1.00 mL) was added. The reaction mixture was allowed to react at 25°C for 12 hours. The reaction mixture was concentrated to obtain a crude product. The crude product was then purified by preparative HPLC (Chromatographic column: Phenomenex luna C18 150*25mm*10µm; mobile phase: [water (0.075% trifluoroacetic acid)-acetonitrile]; acetonitrile: 16%-46%) to obtain the trifluoroacetate salt of **14B.** MS m/z = 649.2 [M+H]⁺, ¹H NMR (400 MHz, MeOD) δ ppm 6.92 (d, *J =* 8.5 Hz, 1H), 5.19 (br dd, *J =* 4.1, 11.3 Hz, 1H), 4.93 - 4.88 (m, 2H), 4.80 - 4.70 (m, 2H), 4.59 (d, *J =* 12.4 Hz, 1H), 4.30 (br d, *J =* 14.1 Hz, 1H), 4.15 (br dd, *J* = 2.1, 14.3 Hz, 2H), 3.95 - 3.62 (m, 4H), 3.58 - 3.38 (m, 2H), 3.30 - 3.24 (m, 1H), 3.09 - 2.87 (m, 3H), 2.47 - 2.07 (m, 7H), 2.05 - 1.94 (m, 4H).

### Example 15

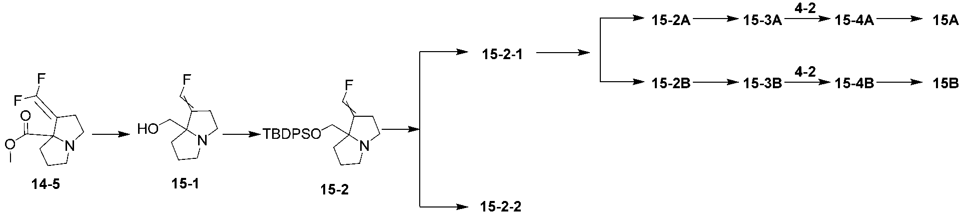

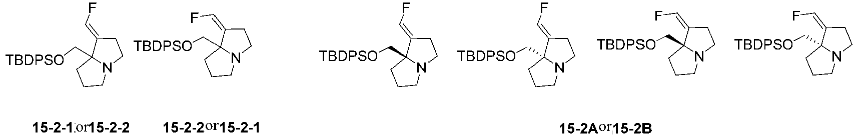

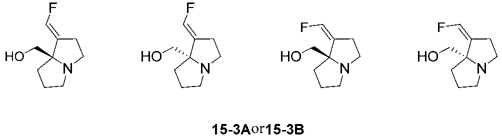

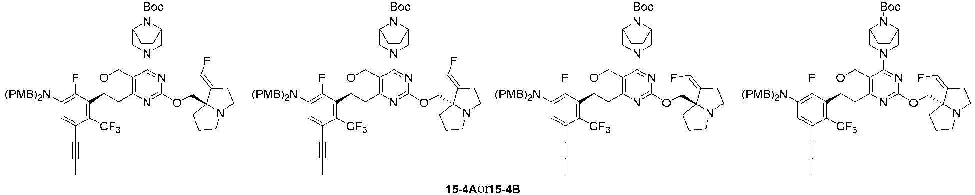

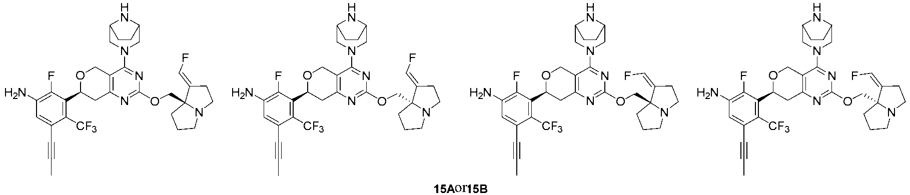

### Step 1

Compound **14-5** (500 mg, 2.30 mmol) was dissolved in 2-MeTHF (5.00 mL) and sodium bis(2-methoxyethoxy) aluminum hydride (2.66 g, 9.21 mmol, 2.57 mL, 70% purity) was added. The mixture was reacted for 2.5 hours at 10°C and then at room temperature for 12 hours. 10.0 mL of water was added to the reaction mixture, which was concentrated to remove dimethyltetrahydrofuran and lyophilized to obtain compound **15-1.** MS m/z = 172.1[M+H]⁺.

### Step 2

Compound **15-1** (261 mg, 1.52 mmol) was dissolved in anhydrous dichloromethane (3.00 mL). Imidazole (415 mg, 6.10 mmol), 4-dimethylaminopyridine (18.6 mg, 152 µmol), and tert-butyldiphenylsilyl chloride (838 mg, 3.05 mmol, 780 µL) were added and reacted at 45°C for 12 hours. The reaction solution was concentrated to obtain a crude product. The crude product was separated by preparative HPLC (Chromatographic column: Phenomenex luna C18 150*25mm*10µm; mobile phase: [water (0.075% trifluoroacetic acid)-acetonitrile]; acetonitrile: 35%-65%) to obtain compound **15-2.** MS m/z = 410.3 [M+H]⁺. Compound **15-2** was subjected to preparative SFC separation (chiral column: DAICEL CHIRALPAK IG (250mm*50mm, 10µm); mobile phase: [supercritical dichloromethane -methanol (0.1% ammonia)]; methanol (0.1% ammonia): 18%-18%) and concentrated under reduced pressure to give compounds **15-2-1** and **15-2-2.** Analytical SFC: (chiral column: Chiralcel OX-3 (50mm*4.6mm, 3µm); mobile phase: [supercritical carbon dioxide-ethanol (0.05% diethylamine)]; ethanol (0.05% diethylamine)%: 5%-40%), compound **15-2-1,** Rt=1.18min, 99% ee; MS m/z = 410.2[M+H]⁺; compound **15-2-2,** Rt=1.46min, 97% ee, MS m/z = 410.2 [M+H]⁺.

Compound **15-2-1** was subjected to chiral preparative HPLC separation (chiral column: DAICEL CHIRALCEL OX (250mm*50mm, 10µm); mobile phase: [n-hexane - ethanol (0.1% ammonia)]; ethanol (0.1% ammonia): 10%-10%), and concentrated under reduced pressure to give compounds **15-2A** and **15-2B.** Analytical SFC: (chiral column: Chiralcel OX-3 (50mm*4.6mm, 3µm); mobile phase: [supercritical carbon dioxide-ethanol (0.05% diethylamine)]; ethanol (0.05% diethylamine)%: 5%-40%), compound **15-2A,** Rt=1.18min, 99% ee; MS m/z = 410.2[M+H]⁺; compound **15-2B,** Rt=1.21min, 99% ee, MS m/z = 410.2 [M+H]⁺.

### Step 3

Compound **15-2A** (160 mg, 391 µmol) was dissolved in dioxane (4.00 mL) and hydrochloric acid (12 M, 1.00 mL) was added. The mixture was allowed to react at 95°C for 12 hours. The mixture was dissolved by adding 5 mL of water, and extracted with ethyl acetate (3.0 mL). The aqueous phase was lyophilized to obtain the hydrochloride salt of compound **15-3A.** MS m/z = 172.1 [M+H]⁺.

Referring to step 3, compound **15-2B** was used as a starting material in place of compound 15-2A to obtain the hydrochloride salt of compound **15-3B.** MS m/z = 172.1 [M+H]⁺.

### Step 4

Compound **4-2** (100 mg, 116 µmol), compound **15-3A** (48.1 mg, hydrochloride salt), sodium tert-butoxide (55.6 mg, 579 µmol), and 4Å molecular sieves (50.0 mg) were added to toluene (3.00 mL). The reaction solution was reacted at 100°C for 6 hours. The reaction solution was cooled to room temperature, and filtered. The filtrate was concentrated to obtain a crude product. The crude product was purified by preparative thin-layer chromatography (pure ethyl acetate) to obtain compound **15-4A.** MS m/z = 971.4 [M+1]⁺.

Referring to step 4, the hydrochloride of compound **15-3B** was used as a starting material in place of the hydrochloride of compound **15-3A** to obtain compound **15-4B.** MS m/z = 971.4 [M+H]⁺.

### Step 5

Compound **15-4A** (71.0 mg, 73.1 µmol) was dissolved in dichloromethane (5.00 mL), and trifluoroacetic acid (1.00 mL) was added. The reaction mixture was reacted at 25°C for 12 hours. The reaction mixture was concentrated to obtain a crude product. The crude product was then purified by preparative HPLC (Chromatographic column: Waters Xbridge 150*25mm*5µm; mobile phase: [water (0.1% ammonia)-acetonitrile]; acetonitrile: 35%-65%) to obtain compound **15A.** MS m/z = 631.3 [M+H]⁺, ¹H NMR (400 MHz, MeOD) δ ppm 6.99 - 6.71 (m, 2H), 5.14 (br dd, *J* = 4.1, 11.5 Hz, 1H), 4.81 - 4.76 (m, 2H), 4.20 - 4.09 (m, 3H), 3.61 - 3.38 (m, 4H), 3.26 - 3.00 (m, 4H), 2.90 - 2.58 (m, 5H), 2.25 - 2.10 (m, 1H), 2.07 - 1.60 (m, 10H).

Compound **15-4B** (61.0 mg, 62.8 µmol) was dissolved in dichloromethane (5.00 mL), and trifluoroacetic acid (1.00 mL) was added. The reaction mixture was reacted at 25°C for 12 hours. The reaction mixture was directly concentrated to obtain a crude product. The crude product was purified by preparative HPLC (Chromatographic column: Phenomenex luna C18 150*25mm*10µm; mobile phase: [water (0.075% trifluoroacetic acid)-acetonitrile]; acetonitrile: 10%-40%) to obtain the trifluoroacetate salt of compound 15B. MS m/z = 631.3 [M+H]⁺, ¹H NMR (400 MHz, MeOD) δ ppm 7.27 - 6.84 (m, 2H), 5.19 (br dd, *J =* 3.5, 11.0 Hz, 1H), 4.79 - 4.50 (m, 4H), 4.34 (br d, *J =* 14.3 Hz, 1H), 4.15 (br dd, *J =* 2.9, 14.6 Hz, 2H), 3.91 - 3.61 (m, 4H), 3.49 - 3.33 (m, 3H), 3.30 - 3.24 (m, 1H), 3.17 - 2.87 (m, 3H), 2.54 - 2.05 (m, 7H), 2.04 - 1.92 (m, 4H).

### Example 16

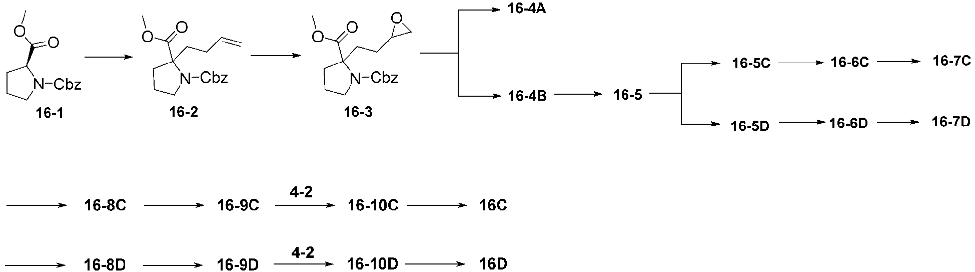

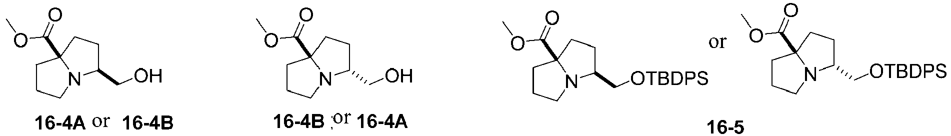

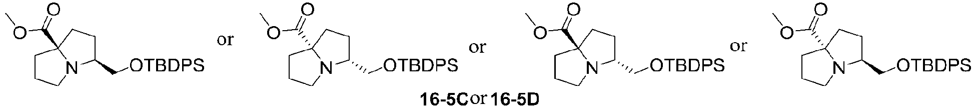

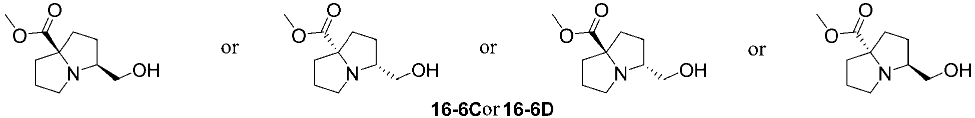

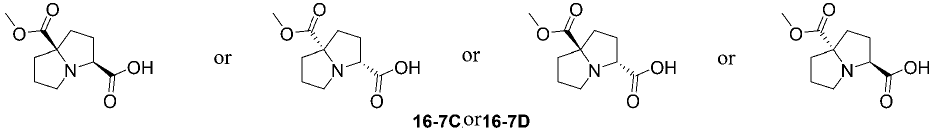

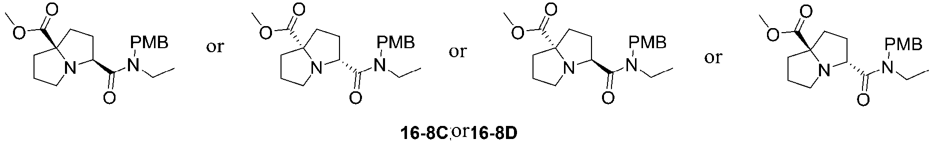

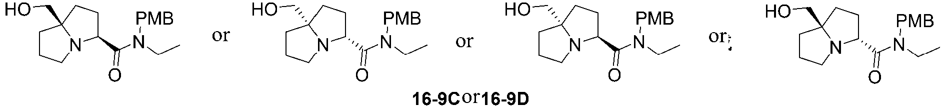

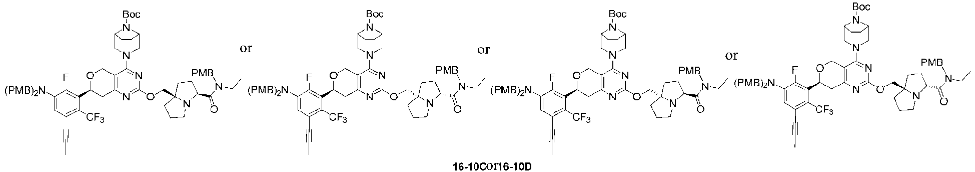

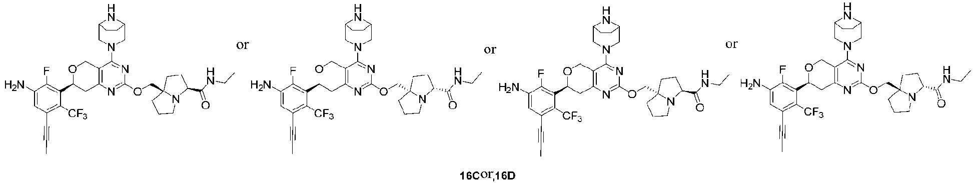

### Step 1

Under nitrogen, a solution of lithium bistrimethylsilylamide in tetrahydrofuran (569.72 mL, 569.72 mmol, 1M) was slowly added dropwise to a solution of compound **16-1** (100.00 g, 379.81 mmol) in tetrahydrofuran (1000 mL) at -70°C. The mixture was reacted at -70°C for 1 hour. 4-Bromo-1-butene (128.19 g, 949.53 mmol) was added dropwise to the reaction mixture at -70°C, and the mixture was warmed to 20°C with stirring for 12 hours. The reaction was quenched by the addition of 1000 mL of saturated aqueous ammonium chloride. The mixture was extracted with ethyl acetate (700 mL x 3), dried over anhydrous sodium sulfate, and filtered. The filtrate was concentrated under reduced pressure, and separated by column chromatography (petroleum ether:ethyl acetate = 9:1) to afford compound **16-2,** MS m/z = 318.0 [M+H]⁺.

### Step 2

Compound **16-2** (96.00 g, 302.48 mmol) was dissolved in dichloromethane (1000 mL), and m-chloroperbenzoic acid (135.10 g, 665.45 mmol, 85% purity) was added. The reaction was stirred at 20°C under nitrogen for 12 hours. The reaction was quenched by the addition of saturated aqueous sodium sulfite (1500 mL) and washed with saturated aqueous sodium bicarbonate (1000 mL). The organic phase was separated, dried over anhydrous sodium sulfate, and filtered. The filtrate was concentrated under reduced pressure, and separated by column chromatography (petroleum ether:ethyl acetate = 6:1) to obtain compound 16-3. MS m/z = 334.1 [M+H]⁺.

### Step 3

To a solution of compound **16-3** (79.00 g, 236.97 mmol) in methanol (1500 mL) was added palladium/carbon (16.64 g, 156.40 mmol, 10% purity). The mixture was reacted at 20°C under a hydrogen atmosphere (15 psi) for 16 hours. The filtrate was filtered, concentrated under reduced pressure, and separated by column chromatography (dichloromethane:methanol = 20:1 to 9:1) to afford compounds **16-4A** (developing solvent: dichloromethane:methanol = 15:1, Rf = 0.5) and **16-4B** (developing solvent: dichloromethane:methanol=15:1, Rf=0.3), respectively. Compound **16-4A:** MS m/z = 200.0 [M+H]⁺. ¹H NMR (400 MHz, CDCl₃) δ ppm 3.71 (s, 3H), 3.60 (dd, *J =* 12.0 Hz, 4.0 Hz,1H), 3.41 (dd, *J =* 12.0 Hz, 4.0 Hz,1H), 3.13 - 3.05 (m, 1H), 2.99 - 2.94 (m, 1H), 2.73 - 2.67 (m, 1H), 2.36 - 2.31 (m, 1H), 2.24 - 2.20 (m, 1H), 1.93 - 1.79 (m, 5H), 1.74 - 1.66 (m, 1H). Compound **16-4B:** MS m/z = 200.0 [M+H]⁺. ¹H NMR (400 MHz, CDCl₃) δ ppm 3.90 (dd, *J =* 12.0 Hz, 8.0 Hz,1H), 3.79 (dd, *J =* 12.0 Hz, 5.2 Hz,1H), 3.73 (s, 3H), 3.49 - 3.42 (m, 1H), 3.11 - 3.07 (m, 1H), 2.76 - 2.69 (m, 1H), 2.58 - 2.52 (m, 1H), 2.29 - 2.21 (m, 1H), 1.89 - 1.80 (m, 4H), 1.67 - 1.54 (m, 2H).

### Step 4

Compound **16-4B** (21.00 g, 105.40 mmol) was dissolved in dichloromethane (200 mL). Imidazole (15.07 g, 221.33 mmol) was added at 0°C and stirred for 10 minutes. Tert-butyldiphenylsilyl chloride (37.66 g, 137.02 mmol) was then added. The mixture was warmed to 20°C and stirred for 7 hours. The mixture was filtered, concentrated under reduced pressure, and separated by column chromatography (petroleum ether:ethyl acetate = 8:1) to afford compound **16-5,** MS m/z = 438.0 [M+H]⁺. Then Chiral preparative HPLC (Chromatographic column: Regis (S,S)Whelk-O 1, 25 x 250 mm 10 µm; mobile phase: A: n-hexane, B: ethanol; B%: 2%) afforded compounds **16-5C** and **16-5D.** SFC analysis method (Chromatographic column: Regis (s,s) WHELK-01 (4.6 mmI.D *150 mmL, 5 µm); mobile phase: [supercritical carbon dioxide-methanol (0.05% diethylamine)]; gradient: methanol (0.05% diethylamine)%: 5%-40%, 4 min), compound **16-5C,** Rt=3.640 minutes, ee value 97.06%, MS m/z = 438.0 [M+H]⁺; compound **16-5D,** Rt=3.826 minutes, ee value 97.88%, MS m/z = 438.0 [M+H]⁺.

### Step 5

Compound **16-5C** (12.00 g, 27.42 mmol) was dissolved in a hydrogen chloride/1,4-dioxane solution (100 mL, 4 M) and stirred at 50°C for 16 hours. The reaction solution was concentrated under reduced pressure and separated by column chromatography (dichloromethane:methanol = 7:1) to afford compound **16-6C.** MS m/z = 200.0 [M+H]⁺.

Referring to step 5, compound **16-5D** was used as a starting material in place of compound **16-5C** to obtain compound **16-6D.** MS m/z = 200.0 [M+H]⁺.

### Step 6

Compound **16-6C** (1.00 g, 5.02 mmol) was dissolved in 10 mL of a mixed solvent (acetonitrile:water = 100:0.75). Chromium trioxide (150.56 mg, 1.51 mmol) and periodic acid (2.86 g, 12.55 mmol) were added at 0°C. The mixture was warmed to 20°C and the reaction was stirred under nitrogen for 16 hours. The reaction solution was filtered, and the filtrate was concentrated under reduced pressure to obtain compound **16-7C.** MS m/z = 214.0 [M+H]⁺.

Referring to step 6, compound **16-6D** was used as a starting material in place of compound **16-6C** to obtain compound **16-7D.** MS m/z = 214.0 [M+H]⁺.

### Step 7

Compound **16-7C** (0.20 g, 0.94 mmol) was dissolved in N,N-dimethylformamide (5 mL). N-ethyl-4-methoxybenzylamine (154.98 mg, 0.94 mmol), N,N-diisopropylethylamine (606.12 mg, 4.69 mmol), 2-(7-azobenzotriazole)-N,N,N',N'-tetramethyluronium hexafluorophosphate were added in sequence. The reaction mixture was stirred at room temperature for 1 hour. Water (50 mL) was added to the reaction solution, and the mixture was extracted with ethyl acetate (20 mL x 3). The organic phases were combined, washed with saturated brine (50 mL), dried over anhydrous sodium sulfate, and filtered. The filtrate was concentrated under reduced pressure, and separated by column chromatography (petroleum ether:ethyl acetate = 2:1) to obtain compound **16-8C.** MS m/z = 361.0 [M+H]⁺.

Referring to step 7, compound **16-7D** was used as a starting material in place of compound 16-7C to obtain compound **16-8D.** MS m/z = 361.0 [M+H]⁺.

### Step 8

Compound **16-8C** (150 mg, 0.42 mmol) was dissolved in anhydrous methanol (3 mL). The mixture was cooled to 0°C and sodium borohydride (50 mg, 1.25 mmol) and sodium methoxide/methanol solution (7.49 mg, 41.62 µmol, 30% purity) were added sequentially. The reaction was stirred at room temperature for 8 hours. The reaction was quenched with a saturated aqueous solution of ammonium chloride (10 mL) and extracted with ethyl acetate (20 mL x 3). The extracted organic phases were combined and washed with saturated brine (20 mL), dried over anhydrous sodium sulfate, and filtered. The filtrate was concentrated under reduced pressure, and separated by column chromatography (petroleum ether:ethyl acetate = 1:1) to obtain compound **16-9C.** MS m/z = 333.3 [M+H]⁺.

Referring to step 8, compound **16-8D** was used as a starting material in place of compound **16-8C** to obtain compound **16-9D.** MS m/z = 333.3 [M+H]⁺.

### Step 9

Compound **16-9C** (0.10 g, 0.30 mmol) was dissolved in anhydrous tetrahydrofuran (3 mL) and cooled to 0°C. Sodium hydride (60.16 mg, 1.50 mmol, 60% purity) was added. After stirring at 0°C for half an hour, compound **4-2** (264.70 mg, 0.30 mmol) was added and the reaction was stirred at room temperature for 1 hour. The mixture was quenched with a saturated aqueous solution of ammonium chloride (10 mL) and extracted with ethyl acetate (20 mL x 3). The extracted organic phases were combined and washed with saturated brine (20 mL), dried over anhydrous sodium sulfate, and filtered. The filtrate was concentrated under reduced pressure, and separated by column chromatography (petroleum ether:ethyl acetate = 2:1) to obtain compound **16-10C.** MS m/z = 1132.5 [M+H]⁺.

Referring to step 9, compound **16-9D** was used as a starting material in place of compound **16-9C** to obtain compound **16-10D.** MS m/z = 1132.5 [M+H]⁺.

### Step 11

Compound **16-10C** (0.20 g, 0.18 mmol) was added to trifluoroacetic acid (4 mL) and stirred at 60°C for 1 hour. The reaction solution was concentrated under reduced pressure to obtain a crude product. The crude product was separated by preparative HPLC (Chromatographic column: Waters Xbridge, 250 x 19 mm, 5 mm; mobile phase: [water (0.1% ammonia)-acetonitrile]; gradient: (acetonitrile): 55%-70%) and lyophilized to obtain compound **16C.** MS m/z = 672.5 [M+H]⁺. ¹H NMR (400 MHz, CD₃OD) δ ppm 6.90 (d, *J =* 8.0 Hz, 1H), 5.16 - 5.14 (m, 1H), 4.81 - 4.78 (m, 1H), 4.67 - 4.63 (m, 1H), 4.16 - 4.13 (m, 2H), 4.09 - 4.08 (m, 1H), 3.75 - 3.72 (m, 1H), 3.54 - 3.51 (m, 3H), 3.43 - 3.40 (m, 1H), 3.28 - 3.17 (m, 3H), 3.06 - 3.03 (m, 1H), 2.91 - 2.83 (m, 2H), 2.78 - 2.72 (m, 1H), 2.12 - 2.08 (m, 2H), 2.02 - 1.91 (m, 11H), 1.76 - 1.66 (m, 2H), 1.14 (t, *J =* 8.0 Hz, 3H).

Compound **16-10D** (0.16 g, 0.14 mmol) was added to trifluoroacetic acid (4 mL) and stirred at 60°C for 1 hour. The reaction solution was concentrated under reduced pressure to obtain a crude product. The crude product was separated by preparative HPLC (Chromatographic column: Waters Xbridge, 250 x 19 mm, 5 mm; mobile phase: [water (0.1% ammonia)-acetonitrile]; gradient: (acetonitrile): 55%-70%) and lyophilized to obtain compound **16D.** MS m/z = 672.5 [M+H]⁺. ¹H NMR (400 MHz, CD₃OD) δ ppm 6.90 (d, *J =* 8.0 Hz, 1H), 5.17 - 5.12 (m, 1H), 4.84 - 4.78 (m, 1H), 4.66 - 4.63 (m, 1H), 4.18 - 4.12 (m, 2H), 4.05 - 4.03 (m, 1H), 3.74 - 3.71 (m, 1H), 3.53 - 3.50 (m, 3H), 3.43 - 3.40 (m, 1H), 3.28 - 3.17 (m, 3H), 3.05 - 3.02 (m, 1H), 2.92 - 2.82 (m, 2H), 2.78 - 2.71 (m, 1H), 2.13 - 2.07 (m, 2H), 2.01-1.82 (m, 11H), 1.76 - 1.66 (m, 2H), 1.14 (t, *J* = 8.0 Hz, 3H).

**16C** and **16D** were confirmed to have structures as follows:

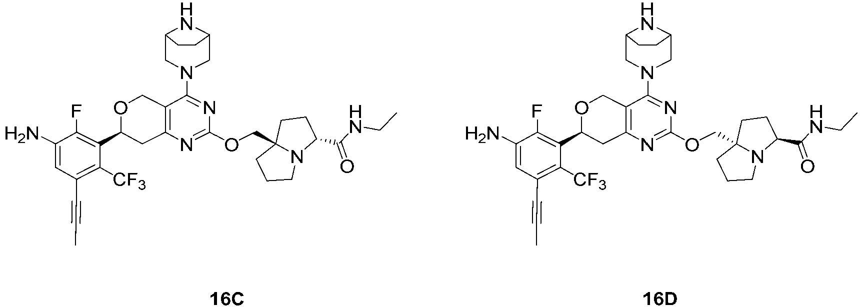

### Example 17

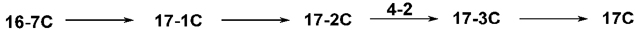

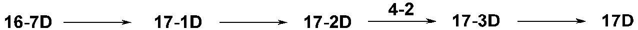

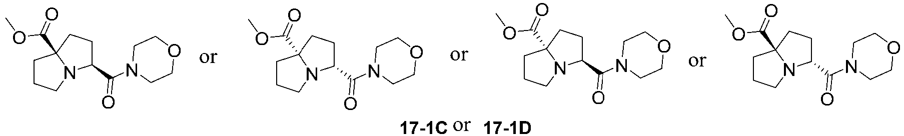

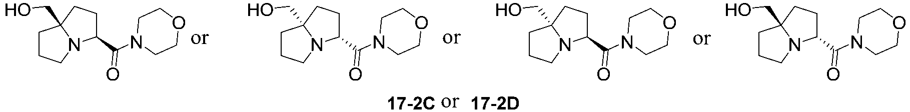

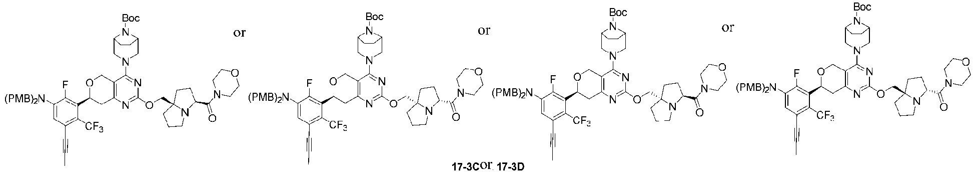

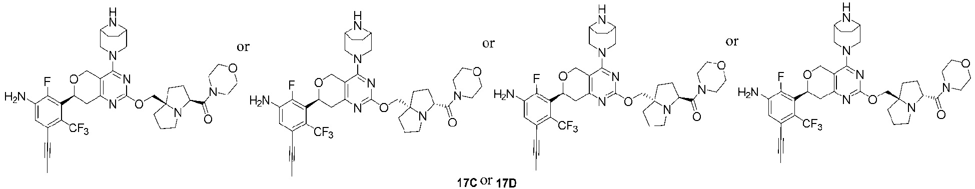

### Step 1

Compound **16-7C** (0.90 g, 4.22 mmol) was dissolved in N,N-dimethylformamide (10 mL). N,N-diisopropylethylamine (2.73 g, 21.10 mmol), N,N,N',N'-tetramethyl-O-(7-azabenzotriazol-1-yl)uronium hexafluorophosphate (3.21 g, 8.44 mmol), and morpholine (735.43 mg, 8.44 mmol) were added sequentially. The mixture was reacted at 25°C for 12 hours. Water (10 mL) was added to the reaction solution, which was then extracted with ethyl acetate (20 mL x 3). The extracted organic phases were combined and washed with saturated brine (10 mL), dried over anhydrous sodium sulfate, and filtered. The filtrate was concentrated under reduced pressure, and separated by column chromatography (dichloromethane:methanol = 20:1) to afford compound **17-1C.** MS m/z = 283.2 [M+H]⁺.

Referring to step 1, compound **16-7D** was used as a starting material in place of compound **16-7C** to obtain compound **17-1D.** MS m/z = 283.2 [M+H]⁺.

### Step 2

Compound **17-1C** (20.00 mg, 70.84 µmol) was dissolved in methanol (0.5 mL). Sodium methoxide (38.27 µg, 0.71 µmol) and sodium borohydride (8.04 mg, 212.51 µmol) were added at 0°C and reacted at 25°C for 16 hours. The reaction was quenched by the addition of saturated ammonium chloride (10 mL). The product was extracted with ethyl acetate (20 mL), dried over anhydrous sodium sulfate, filtered, and concentrated under reduced pressure to obtain compound **17-2C.** MS m/z = 255.3 [M+H]⁺.

Referring to step 2, compound **17-1D** was used as a starting material in place of compound **17-1C** to obtain compound **17-2D.** MS m/z = 255.3 [M+H]⁺.

### Step 3

Compound **17-2C** (66.00 mg, 259.51 µmol) was dissolved in tetrahydrofuran (10 mL). Sodium hydride (31.14 mg, 1.30 mmol, 60% purity) was added at 0°C and stirred for 0.5 hours. Finally, compound **4-2** (228.36 mg, 259.51 µmol) was added and allowed to react at 25°C for 2 hours. The reaction was quenched by the addition of water (10 mL), and the mixture was extracted with ethyl acetate (20 mL). The mixture was dried over anhydrous sodium sulfate, and filtered. The filtrate was concentrated under reduced pressure, and separated by column chromatography (petroleum ether:ethyl acetate = 0:1) to afford compound **17-3C.** MS m/z = 1054.9 [M+H]⁺.

Referring to step 3, compound **17-2D** was used as a starting material in place of compound **17-2C** to obtain compound **17-3D.** MS m/z = 1054.9 [M+H]⁺.

### Step 4

Compound **17-3C** (0.20 g, 189.72 µmol) was dissolved in dichloromethane (4 mL), and trifluoroacetic acid (2 mL) was added. The mixture was reacted at 25°C for 0.5 h. The reaction solution was concentrated under reduced pressure to obtain a crude product. The crude product was separated by preparative HPLC (Chromatographic column: Waters SunFire, 250 x 19 mm, 5 µm; mobile phase: [water (0.1% formic acid)-acetonitrile]; gradient: (acetonitrile): 40% - 75%) and lyophilized to obtain the formate salt of compound **17C.** MS m/z = 714.6 [M+H]⁺. ¹H NMR (400 MHz, CD₃OD) δ ppm 8.29 (s, 1H), 6.91 (d, *J =* 8.0 Hz, 1H), 5.18 (dd, *J =* 8.0, 4.0 Hz, 1H), 4.82 (s, 1H), 4.71 (d, *J =* 16.0 Hz, 1H), 4.30 - 4.22 (m, 3H), 4.12 (d, *J =* 12.0 Hz, 2H), 4.04 (dd, *J =* 12.0, 4.0 Hz, 1H), 3.78 - 3.57 (m, 9H), 3.52 - 3.46 (m, 1H), 3.25 (d, *J =* 12.0 Hz, 2H), 2.94 - 2.87 (m, 2H), 2.76 - 2.70 (m, 1H), 2.31 - 2.24 (m, 2H), 2.12 - 2.09 (m, 3H), 2.02 (s, 3H), 1.99 - 1.83 (m, 6H), 1.74 - 1.66 (m, 1H).

Compound **17-3D** (0.10 g, 94.86 µmol) was dissolved in dichloromethane (2 mL), and trifluoroacetic acid (1 mL) was added. The mixture was reacted at 25°C for 0.5 h. The reaction solution was concentrated under reduced pressure to obtain a crude product. The crude product was separated by preparative HPLC (Chromatographic column: Waters Xbridge, 250*19 mm, 5 mm; mobile phase: [water (0.1% ammonia)-acetonitrile]; gradient: (acetonitrile): 55%-70%) and lyophilized to obtain compound **17D.** MS m/z = 714.6 [M+H]⁺. ¹H NMR (400 MHz, CD₃OD) δ ppm 6.90 (d, *J =* 8.0 Hz, 1H), 5.14 (dd, *J =* 12.0,4.0 Hz, 1H), 4.80 (d, *J* = 12.0 Hz, 1H), 4.65 (d, *J* = 12.0 Hz, 1H), 4.37 (d, *J =* 8.0 Hz, 1H), 4.11 - 4.05 (m, 2H), 3.80 - 3.76 (m, 1H), 3.74 - 3.60 (m, 4H), 3.58 - 3.48 (m, 6H), 3.42 - 3.39 (m, 2H), 3.21 (dd, *J =* 16.0, 12.0 Hz, 1H), 3.03 (d, *J =* 12 Hz, 1H), 2.87 - 2.76 (m, 2H), 2.58 - 2.52 (m, 1H), 2.22 - 2.11 (m, 1H), 2.01 - 1.98 (m, 5H), 1.94 - 1.88 (m, 1H), 1.85 - 1.80 (m, 4H), 1.79-1.66 (m, 3H), 1.61-1.54 (m, 1H).

Compound **17C** and **17D** were confirmed to have structures as follows:

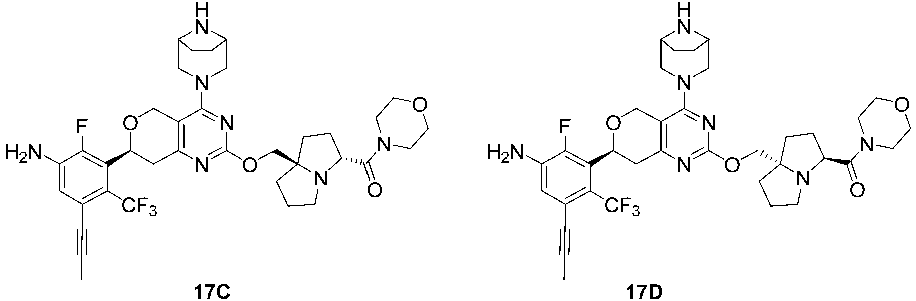

### Example 18

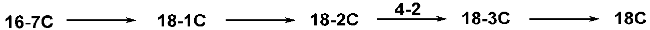

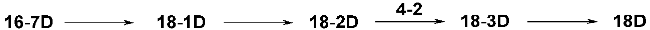

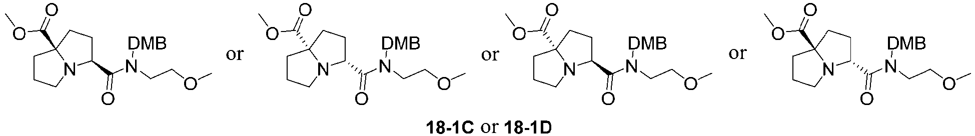

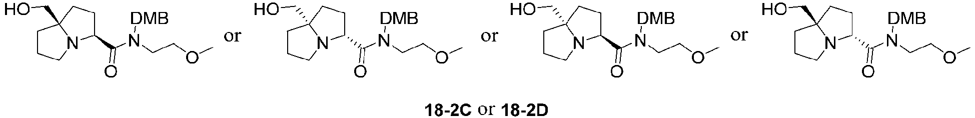

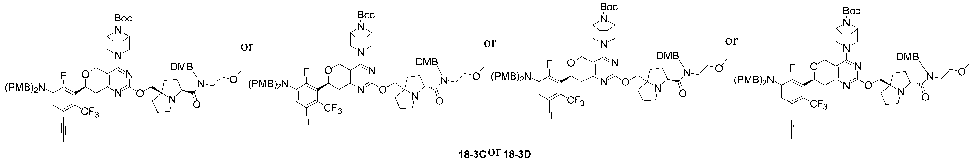

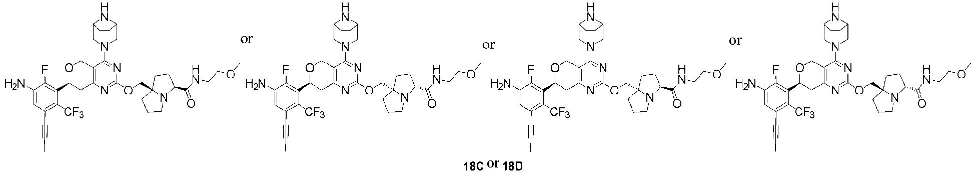

### Step 1

Compound **16-7C** (0.15 g, 703.47 µmol) was dissolved in dichloromethane (10 mL). N,N-diisopropylethylamine (3.52 mmol, 612.65 µL), N-(2,4-dimethoxybenzyl)-2-methoxyethylamine (316.96 mg, 1.41 mmol), and N,N,N',N'-tetramethyl-O-(7-azabenzotriazol-1-yl)uronium hexafluorophosphate (534.96 mg, 1.41 mmol) were added. The mixture was reacted at 25°C for 1 hour. The organic phase was concentrated under reduced pressure and separated by column chromatography (petroleum ether:ethyl acetate = 3:1) to obtain compound **18-1C.** MS m/z = 421.4 [M+H]⁺.

Referring to step 1, compound **16-7D** was used as a starting material in place of compound **16-7C** to obtain compound **18-1D.** MS m/z = 421.4 [M+H]⁺.

### Step 2

Compound **18-1C** (0.27 g, 642.10 µmol) was dissolved in tetrahydrofuran (3 mL), and sodium borohydride (485.84 mg, 12.84 mmol) and lithium chloride (27.22 mg, 642.10 µmol) were added. The mixture was reacted at 50°C for 16 hours. After cooling to room temperature, the reaction was quenched with water (3 mL). The mixture was extracted with ethyl acetate (5 mL x 3). The organic phases were combined and concentrated under reduced pressure to obtain compound **18-2C.** MS m/z = 393.3 [M+H]⁺.

Referring to step 2, compound **18-1D** was used as a starting material in place of compound **18-1C** to obtain compound **18-2D.** MS m/z = 393.3 [M+H]⁺.

### Step 3

Compound **18-2C** (0.18 g, 321.03 µmol) was dissolved in tetrahydrofuran (5 mL) and cooled to 0°C. Sodium hydride (17.12 mg, 428.04 µmol, 60% purity) was added and reacted at 0°C for 0.5 hours. Compound **4-2** (188.33 mg, 214.02 µmol) was added and reacted at 25°C for 0.5 hours. The reaction was quenched with saturated aqueous ammonium chloride (15 mL) and extracted with ethyl acetate (5 mL x 3). The organic phases were combined, concentrated under reduced pressure, and separated by column chromatography (petroleum ether:ethyl acetate = 1:1) to afford compound **18-3C.** MS m/z = 1192.8 [M+H]⁺.

Referring to step 3, compound **18-2D** was used as a starting material in place of compound **18-2C** to obtain compound **18-3D.** MS m/z = 1192.8 [M+H]⁺.

### Step 4

Compound **18-3C** (0.20 g, 167.74 µmol) was dissolved in trifluoroacetic acid (10 mL) and reacted at 50°C for 0.5 h. The organic phase was concentrated under reduced pressure to obtain a crude product, which was then separated by preparative HPLC (Chromatographic column: Waters Xbridge, 250 x 19 mm, 5 mm; mobile phase: water (0.1% ammonia)-acetonitrile; gradient: acetonitrile: 55%-70%). After lyophilization, compound **18C** was obtained. MS m/z = 702.6 [M+H]⁺. ¹H NMR (400 MHz, CD₃OD) δ ppm 6.90 (d, *J* = 8.0 Hz, 1H), 5.15 (dd, *J =* 12.0, 4.0 Hz, 1H), 4.78 (s, 1H), 4.65 (d, *J =* 12.0 Hz, 1H), 4.17 - 4.06 (m, 3H), 3.77 (dd, *J =* 8.0, 4.0 Hz, 1H), 3.53 (d, *J =* 12.0 Hz, 3H), 3.49 - 3.36 (m, 5H), 3.34 (s, 3H), 3.21 (dd, *J =* 16.0, 12.0 Hz, 1H), 3.04 (d, *J = 12.0* Hz, 1H), 2.95 - 2.91 (m, 1H), 2.85 (dd, *J =* 16.0, 4.0 Hz, 1H), 2.78 - 2.69 (m, 1H), 2.13 - 2.06 (m, 2H), 2.04 - 1.96 (m, 5H), 1.94 - 1.89 (m, 4H), 1.85 - 1.82 (m, 2H), 1.76 - 1.66 (m, 2H).

Compound **18-3D** (0.15 g, 125.80 µmol) was weighed. Dichloromethane (2.5 mL) and trifluoroacetic acid (2.5 mL) were added, and the mixture was reacted at 25°C for 0.5 h. The organic phase was concentrated under reduced pressure, and the crude product was separated by preparative HPLC (Chromatographic column: Waters Xbridge, 250 x 19 mm, 5 mm; mobile phase: [water (0.1% ammonia)-acetonitrile]; gradient: (acetonitrile): 55%-70%). After lyophilization, compound **18D** was obtained. MS m/z = 702.6 [M+H]⁺. ¹H NMR (400 MHz, CD₃OD) δ ppm 6.90 (d, *J =* 8.0 Hz, 1H), 5.15 (dd, *J =* 12.0, 4.0 Hz, 1H), 4.78 (s, 1H), 4.65 (d, *J =* 12.0 Hz, 1H), 4.15 *(t, J =* 12.0 Hz, 2H), 4.05 (d, *J =* 12.0 Hz, 1H), 3.78 - 3.75 (m, 1H), 3.53 (d, *J =* 8.0 Hz, 3H), 3.49 - 3.36 (m, 5H), 3.34 (s, 3H), 3.21 (dd, *J =* 16.0, 12.0 Hz, 1H), 3.04 (d, *J =* 12.0 Hz, 1H), 2.95 - 2.90 (m, 1H), 2.84 (dd, *J =* 16.0, 4.0 Hz, 1H), 2.76 - 2.70 (m, 1H), 2.15 - 2.07 (m, 2H), 2.01 - 1.94 (m, 5H),1.95 - 1.87 (m, 4H), 1.86-1.80 (m, 2H), 1.76-1.66 (m, 2H).

Compounds **18C** and **18D** were confirmed to have structures as follows:

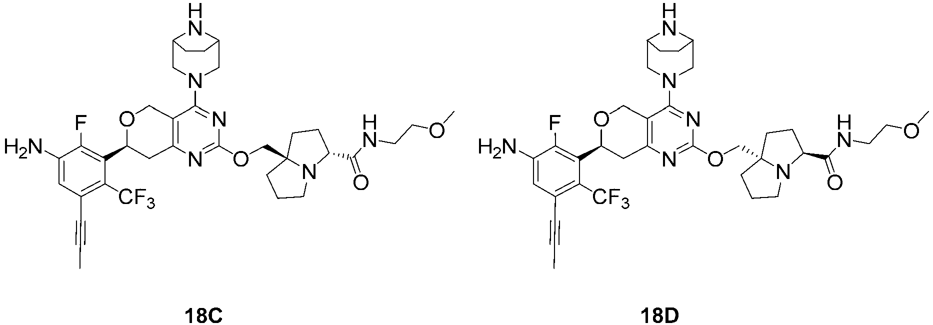

### Example 19

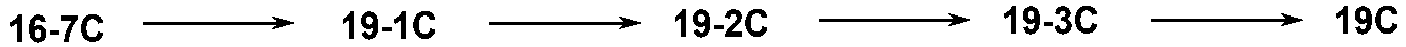

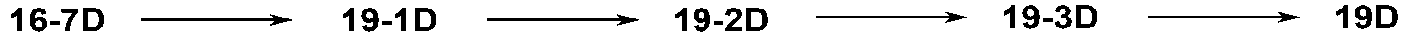

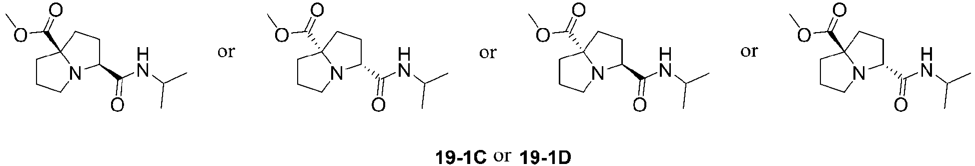

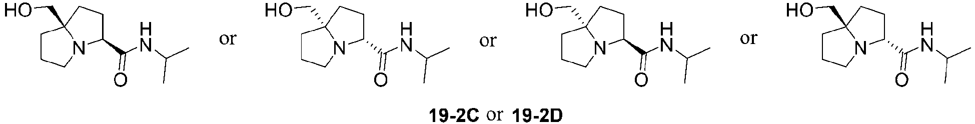

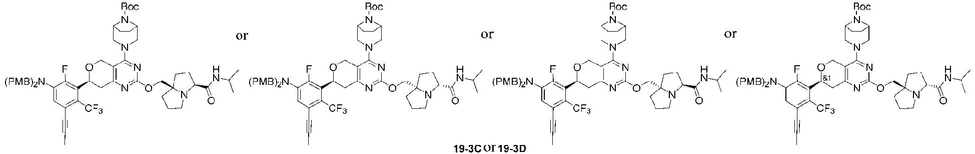

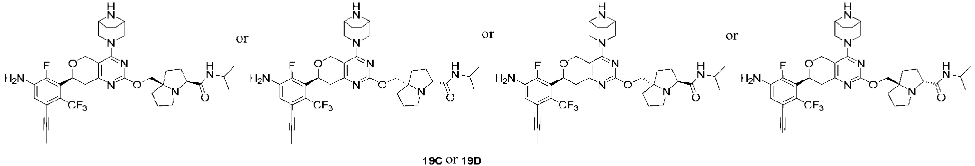

### Step 1

Compound **16-7C** (0.40 g, 1.86 mol) was weighed and dichloromethane (6 mL) was added. Also added were isopropylamine (166.33 mg, 2.81 mmol), 2-(7-azabenzotriazole)-N,N,N',N'-tetramethyluronium hexafluorophosphate (1.43 g, 3.75 mmol), and N,N-diisopropylethylamine (0.73 g, 5.63 mol). The mixture was reacted at 25°C for 3 hours. Water (10 mL) was added, and the mixture was extracted with dichloromethane (10 mL x 3). The organic phase was concentrated under reduced pressure to obtain compound **19-1C.** MS m/z = 255.0 [M+H]⁺.

Referring to step 1, compound **16-7D** was used as a starting material in place of compound **16-7C** to obtain compound **19-1D.** MS m/z = 255.0 [M+H]⁺.

### Step 2

Compound **19-1C** (0.10 g, 0.39 mmol) was weighed and anhydrous tetrahydrofuran (3 mL) was added under nitrogen. The mixture was cooled to 0°C, and a solution of lithium aluminum tetrahydride in tetrahydrofuran (1.18 mmol, 0.47 ml, 2.5 M) was added. The mixture was reacted at 25°C for 15 minutes. At 0°C, 0.2 mL of water was added to the reaction solution, and the reaction was quenched by adding 0.2 mL of 15% NaOH solution. The reaction was stirred for 10 minutes, and filtered. The filter cake was washed with 5 mL of tetrahydrofuran. The filtrate was concentrated to obtain compound **19-2C.** MS m/z = 227.1 [M+H]⁺

Referring to step 2, compound **19-1D** was used as a starting material in place of compound **19-1C** to obtain compound **19-2D.** MS m/z = 227.1 [M+H]⁺.

### Step 3

Compound **19-2C** (50.0 mg, 0.22 mmol) was weighed and anhydrous tetrahydrofuran (5 mL) was added. Sodium hydride (15.91 mg, 0.66 mmol, 60%) was added under ice bath at 0°C. The mixture was stirred at 25°C for 30 minutes, and compound **4-2** (0.20 g, 0.22 mmol) was added. The reaction mixture was allowed to react at 25°C for 1 hour. The reaction mixture was quenched with water (5 mL) and extracted with ethyl acetate (5 mL x 3). The organic phase was concentrated under reduced pressure and separated by column chromatography (dichloromethane:methanol = 20:1) to afford compound **19-3C.** MS m/z = 1026.8 [M+H]⁺.

Referring to step 3, compound **19-2D** was used as a starting material in place of compound **19-2C** to obtain compound **19-3D.** MS m/z = 1026.8 [M+H]⁺.

### Step 4

Compound **19-3C** (50 mg, 0.05 mmol) was dissolved in dichloromethane (2 mL), and trifluoroacetic acid (1 mL) was added. The reaction mixture was allowed to react at 25°C for 0.5 h. The reaction mixture was concentrated under reduced pressure to obtain a crude product. The crude product was separated by preparative HPLC (Chromatographic column: Waters SunFire, 250 x 19 mm, 5 µm; mobile phase: [water (0.05% formic acid)-acetonitrile]; (acetonitrile): 45%-75%) and lyophilized to obtain the formate salt of compound 19C. MS m/z = 686.5 [M+H]⁺. ¹H NMR (400 MHz, CD₃OD) δ ppm 8.45 (s, 1.52 H), 6.91 (d, *J =* 8.4 Hz, 1 H), 5.20 - 5.16 (m, 1 H), 4.84 - 4.79 (m, 1 H), 4.70 (d, *J =* 13.7 Hz, 1 H), 4.33 (d, *J =* 11.2 Hz, 1 H), 4.26 - 4.10 (m, 2 H), 4.08 - 3.97 (m, 4 H), 3.76 - 3.73 (m, 1 H), 3.58 (d, *J* = 13.4 Hz, 1 H), 3.27 - 3.21 (m, 3 H), 3.03 - 3.01 (m, 1 H), 2.93 - 2.88 (m, 1 H), 2.26 - 2.06 (m, 8 H), 2.01 - 1.94 (m, 5 H), 1.92 - 1.87 (m, 2 H), 1.17 (dd, *J =* 6.6, 1.5 Hz, 6 H).

Compound **19-3D** (50 mg, 0.05 mmol) was dissolved in dichloromethane (2 mL), and trifluoroacetic acid (1 mL) was added. The reaction mixture was reacted at 25°C for 0.5 h. The reaction mixture was concentrated under reduced pressure to obtain a crude product. The crude product was separated by preparative HPLC (Chromatographic column: Waters SunFire, 250 x 19 mm, 5 µm; mobile phase: [water (0.05% formic acid)-acetonitrile]; (acetonitrile): 45%-75%) and lyophilized to obtain the formate salt of compound **19D.** MS m/z = 686.5 [M+H]⁺. ¹H NMR (400 MHz, CD₃OD) δ ppm 8.36 (s, 1.59 H), 6.81 *(d, J=* 8.4 Hz, 1 H), 5.10 - 5.07 (m, 1 H), 4.81 - 4.78 (m, 1 H), 4.61 (d, *J* = 13.6 Hz, 1 H), 4.22 - 4.08 (m, 3 H), 3.94 - 3.88 (m, 4 H), 3.63 (d, *J =* 13.6 Hz, 1 H), 3.48 (d, *J =* 13.2 Hz, 1 H), 3.17 - 3.03 (m, 3 H), 2.91 - 2.78 (m, 2 H), 2.15 - 1.99 (m, 6 H), 1.98 - 1.92 (m, 6 H), 1.84 - 1.76 (m, 3 H), 1.07 (d, *J =* 6.6 Hz, 6 H).

Compounds **19C** and **19D** were confirmed to have structures as follows:

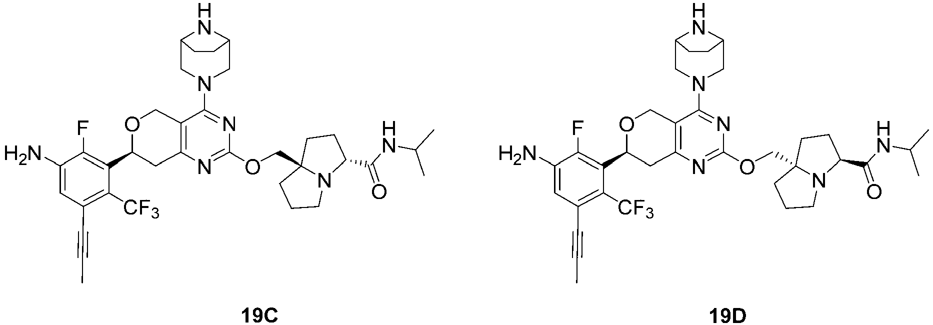

### Example 20

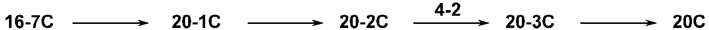

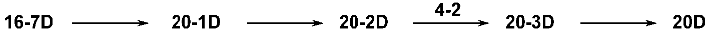

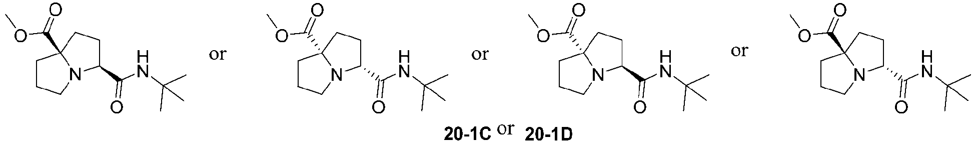

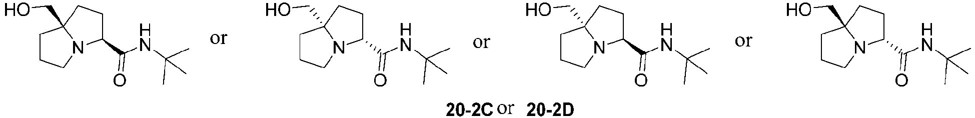

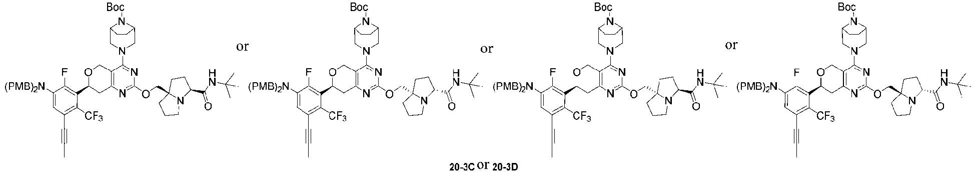

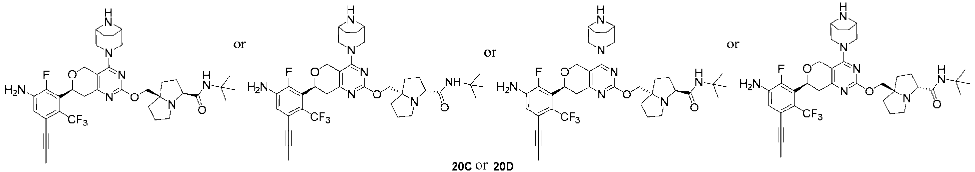

### Step 1

Compound **16-7C** (100 mg, 468.98 µmol) was dissolved in N,N-dimethylformamide (2 mL). 2-Chloro-1-methylpyridinium iodide (179.72 mg, 703.47 µmol), triethylamine (142.37 mg, 1.41 mmol), and tert-butylamine (51.45 mg, 703.47 µmol) were added and reacted at 25°C for 2 hours. Water (10 mL) was added, and the mixture was extracted with ethyl acetate (10 mL * 3). The extracted organic phases were combined and washed with saturated brine (10 mL), dried over anhydrous sodium sulfate, filtered, and concentrated under reduced pressure. Separation by column chromatography (petroleum ether:ethyl acetate = 1:1) afforded compound **20-1C.** MS m/z = 269.0 [M+H]⁺.

Referring to step 1, compound **16-7D** was used as a starting material in place of compound **16-7C** to obtain compound **20-1D.** MS m/z = 269.0 [M+H]⁺.

### Step 2

Compound **20-1C** (300 mg, 1.12 mmol) was dissolved in anhydrous tetrahydrofuran (5 mL) and cooled to 0°C under nitrogen. A 2M solution of lithium borohydride in tetrahydrofuran (1.12 mL) was added dropwise, and the mixture was heated to 50°C with stirring for 8 hours. The reaction was quenched by adding 3 mL of water, and filtered. The filtrate was concentrated under reduced pressure to obtain a crude product. The crude product was separated by column chromatography (dichloromethane:methanol = 10:1) to obtain compound **20-2C.** MS m/z = 241.1 [M+H]⁺.

Referring to step 2, compound **20-1D** was used as a starting material in place of compound **20-1C** to obtain compound **20-2D.** MS m/z = 241.1 [M+H]⁺.

### Step 3

Compound **20-2C** (81.90 mg, 340.93 µmol) was dissolved in anhydrous tetrahydrofuran (5 mL) and cooled to 0°C. Sodium hydride (20.50 mg, 853.75 µmol, 60% purity) was slowly added, and the mixture was warmed to 25°C with stirring for 0.5 hours. Compound **4-2** (200 mg, 227.28 µmol) was then added and allowed to react at 25°C for 2 hours. The reaction was quenched by adding 3 mL of water and extracted with ethyl acetate (5 mL x 3). The extracted organic phases were combined and washed with saturated brine (10 mL), dried over anhydrous sodium sulfate, filtered, and concentrated under reduced pressure. Separation by column chromatography (petroleum ether:ethyl acetate = 1:1) afforded compound **20-3C.** MS m/z = 1040.8 [M+H]⁺.

Referring to step 3, compound **20-2D** was used as a starting material in place of compound **20-2C** to obtain compound **20-3D.** MS m/z = 1040.8 [M+H]⁺.

### Step 4

Compound **20-3C** (100 mg, 96.14 µmol) was dissolved in anhydrous dichloromethane (3 mL), and trifluoroacetic acid (1 mL) was added. The reaction mixture was reacted at 25°C for 2 hours. The reaction mixture was concentrated under reduced pressure to obtain a crude product. The crude product was separated by preparative HPLC (Chromatographic column: Xtimate C18 150*40 mm*5 µm; mobile phase: [water (0.05% formic acid)-acetonitrile]; acetonitrile: 10%-40%) and lyophilized to obtain the formate salt of compound 20C. MS m/z = 700.6 [M+H]⁺, ¹H NMR (400 MHz, CD₃OD) δ ppm 8.51 (s, 1H), 6.91 *(d, J =* 8.4 Hz, 1H), 5.20 - 5.15 (m, 1H), 4.82 (d, *J* = 13.6 Hz, 1H), 4.69 (d, *J =* 13.6 Hz, 1H), 4.27 - 4.14 (m, 3H), 3.88 (s, 3H), 3.68 (d, *J =* 13.2 Hz, 1H), 3.54 (d, *J* = 13.2 Hz, 1H), 3.28 - 3.15 (m, 2H), 3.10 - 3.04 (m, 1H), 2.95 - 2.86 (m, 2H), 2.21 - 2.12 (m, 3H), 2.02 - 1.76 (m, 12H), 1.36 (s, 9H).

Compound **20-3D** (150 mg, 144.21 µmol) was dissolved in dichloromethane (4.5 mL), and trifluoroacetic acid (1.5 mL) was added. The reaction mixture was allowed to react at 25°C for 2 hours. The reaction mixture was concentrated under reduced pressure to obtain a crude product. The crude product was separated by preparative HPLC (Chromatographic column: Xtimate C18 150*40mm*5µm; mobile phase: [water (0.1% ammonium bicarbonate)-acetonitrile]; (acetonitrile): 35%-53%) and lyophilized to obtain compound 20D. MS m/z = 700.6 [M+H]⁺, ¹H NMR (400 MHz, CD₃OD) δ ppm 6.90 (d, *J* = 8.4 Hz, 1H), 5.17 - 5.12 (m, 1H), 4.80 (d, *J* = 13.6 Hz, 1H), 4.65 (d, *J =* 13.6 Hz, 1H), 4.18 - 4.12 (m, 2H), 4.05 (d, *J =* 16.0 Hz, 1H), 3.71 - 3.68 (m, 1H), 3.54 - 3.51 (m, 3H), 3.43 - 3.40 (m, 1H), 3.25 - 3.18 (m, 1H), 3.04 (d, *J* = 16.0 Hz, 1H), 2.93 - 2.71 (m, 3H), 2.14 - 2.09 (m, 2H), 2.06 - 2.01 (m, 4H), 1.92-1.83 (m, 7H), 1.74 - 1.96 (m, 2H), 1.35 (s, 9H).

Compounds **20C** and **20D** were confirmed to have structures as follows:

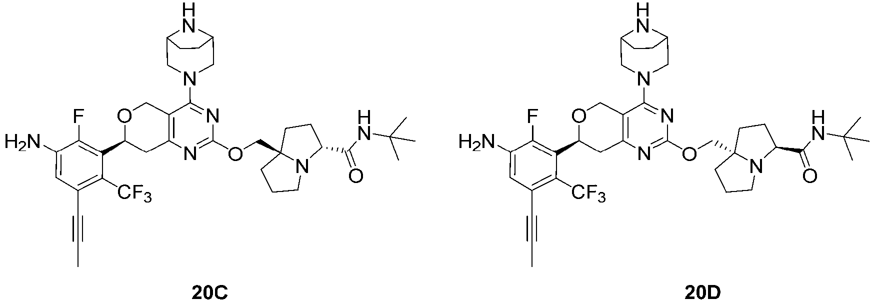

### Example 21

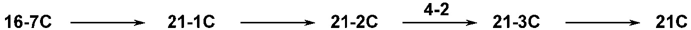

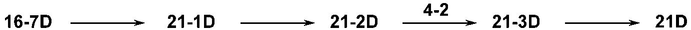

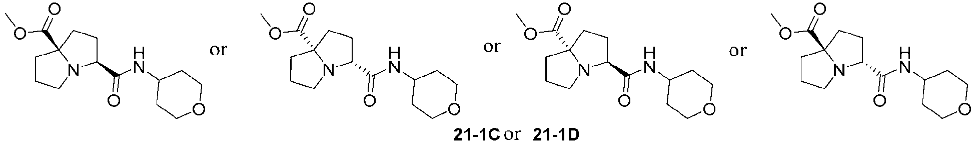

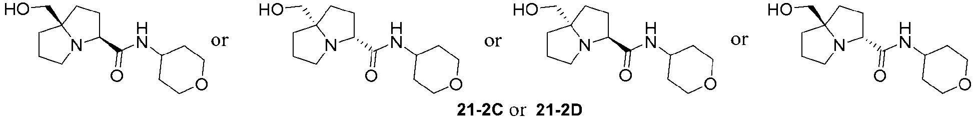

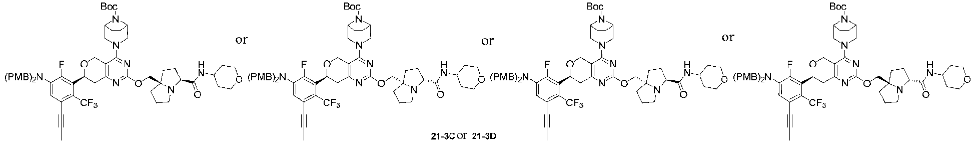

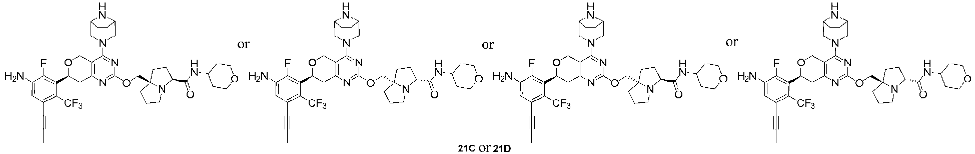

### Step 1

Compound **16-7C** (150 mg, 703.47 µmol) was dissolved in N,N-dimethylformamide (2 mL). 4-aminotetrahydrofuran (106.73 mg, 1.06 mmol), N-methylimidazole (173.27 mg, 2.11 µmol), and tetramethylchlorouronium hexafluorophosphate (296.07 mg, 1.06 mmol) were added and reacted at 25°C for 3 hours. Water (10 mL) was added, and the mixture was extracted with ethyl acetate (10 mL * 3). The extracted organic phases were combined and washed with saturated brine (5 mL), dried over anhydrous sodium sulfate, filtered, and concentrated under reduced pressure. Separation by column chromatography (dichloromethane:methanol = 20:1) afforded compound **21-1C.** MS m/z = 297.1 [M+H]⁺.

Referring to step 1, compound **16-7D** was used as a starting material in place of compound **16-7C** to obtain compound **21-1D.** MS m/z = 297.1 [M+H]⁺.

### Step 2

Compound **21-1C** (300 mg, 1.01 mmol) was dissolved in anhydrous methanol (5 mL). Sodium methoxide (21.90 mg, 404.91 µmol) and sodium borohydride (191.50 mg, 5.06 mmol) were added and reacted at 50°C for 24 hours. The reaction was quenched by the addition of 3 mL of saturated aqueous ammonium chloride. The mixture was filtered, and the filtrate was concentrated under reduced pressure to obtain a crude product. Separation by column chromatography (dichloromethane:methanol = 10:1) afforded compound **21-2C.** MS m/z = 269.2 268.5 [M+H]⁺.

Referring to step 2, compound **21-1D** was used as a starting material in place of compound **21-1C** to obtain compound **21-2D.** MS m/z = 269.2 [M+H]⁺.

### Step 3

Compound **21-2C** (100 mg, 372.65 µmol) was dissolved in anhydrous tetrahydrofuran (5 mL), and cooled to 0°C. Sodium hydride (44.70 mg, 1.12 mmol, 60% purity) was slowly added. The mixture was warmed to 25°C and stirred for 0.5 hours. Compound **4-2** (163.90 mg, 186.32 µmol) was then added and allowed to react at 25°C for 2 hours. The reaction was quenched by adding 3 mL of water and extracted with ethyl acetate (5 mL x 3). The extracted organic phases were combined and washed with saturated brine (10 mL), dried over anhydrous sodium sulfate, filtered, and concentrated under reduced pressure. Separation by Separation by column chromatography (dichloromethane:methanol = 20:1) afforded compound **21-3C.** MS m/z = 1068.5 [M+H]⁺.

Referring to step 3, compound **21-2D** was used as a starting material in place of compound **21-2C** to obtain compound **21-3D.** MS m/z = 1068.5 [M+H]⁺.

### Step 4

Compound **21-3C** (114 mg, 106.72 µmol) was dissolved in anhydrous dichloromethane (0.9 mL), and trifluoroacetic acid (0.3 mL) was added. The mixture was reacted at 25°C for 1 hour. The reaction solution was concentrated under reduced pressure to obtain a crude product. The crude product was separated by preparative HPLC (Chromatographic column: Xtimate C18 150*40mm*5µm; mobile phase: [water (0.05% formic acid)-acetonitrile]; (acetonitrile): 10%-60%) and lyophilized to obtain the formate salt of compound **21C.** MS m/z = 728.5 [M+H]⁺, ¹H NMR (400 MHz, DMSO-*d₆*) δ ppm 8.22 (s, 1.38H), 7.97 (d, *J =* 7.7 Hz, 1H), 6.88 (d, *J =* 8.4 Hz, 1H), 6.02 (s, 2H), 5.05 - 5.01 (m, 1H), 4.75 (d, *J =* 13.7 Hz, 1H), 4.57 (d, *J =* 13.7 Hz, 1H), 3.94 - 3.90 (m, 3H), 3.83 - 3.79 (m, 3H), 3.64 (s, 2H), 3.54 - 3.51 (m, 1H), 3.44 - 3.41 (m, 1H), 3.35 - 3.30 (m, 4H), 3.09 - 3.02 (m, 1H), 2.98 (d, *J=* 12.5 Hz, 1H), 2.82 - 2.77 (m, 1H), 2.75 - 2.70 (m, 1H), 2.66 - 2.59 (m, 1H), 2.03 (s, 3H), 1.97 - 1.80 (m, 6H), 1.80 - 1.52 (m, 8H), 1.47 - 1.37 (m, 2H).

Compound **21-3D** (200 mg, 187.23 µmol) was dissolved in anhydrous dichloromethane (5 mL), and trifluoroacetic acid (5 mL) was added. The mixture was reacted at 25°C for 1 hour. The reaction solution was concentrated under reduced pressure to obtain a crude product. The crude product was separated by preparative HPLC (Chromatographic column: Xtimate C18 150*40mm*5µm; mobile phase: [water (0.05% formic acid)-acetonitrile]; (acetonitrile): 10%-60%) and lyophilized to obtain the formate salt of compound **21D.** MS m/z = 728.5 [M+H]⁺, ¹H NMR (400 MHz, DMSO-*d₆*) δ ppm 8.14 (s, 0.56H), 7.96 (d, *J =* 7.8 Hz, 1H), 6.88 (d, *J =* 8.3 Hz, 1H), 6.01 (s, 2H), 5.04 - 5.00 (m, 1H), 4.74 (d, *J =* 13.7 Hz, 1H), 4.54 (d, *J =* 13.7 Hz, 1H), 4.05 - 3.92 (m, 4H), 3.85 - 3.79 (m, 4H), 3.64 - 3.51 (m, 2H), 3.46 (d, *J =* 13.3 Hz, 1H), 3.40 - 3.35 (m, 3H), 3.14 - 3.06 (m, 2H), 2.87 - 2.68 (m, 2H), 2.67 - 2.52 (m, 1H), 2.03 (s, 3H), 1.91 - 1.53 (m, 14H), 1.46 - 1.38 (m, 2H).

Compounds **21C** and **21D** were confirmed to have structures as follows:

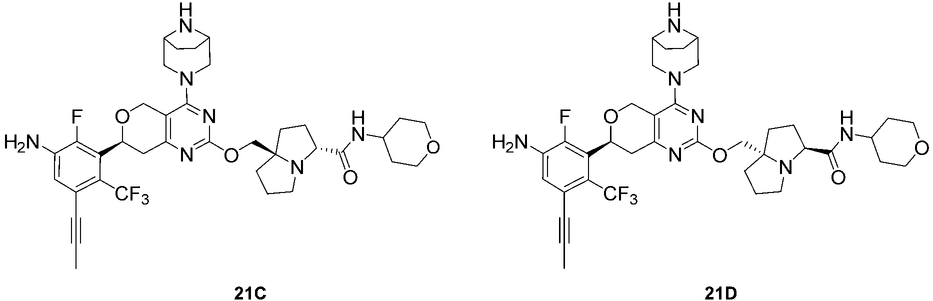

### Example 22

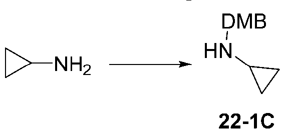

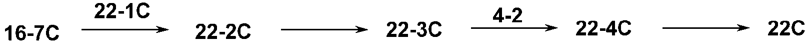

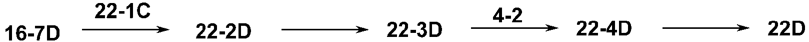

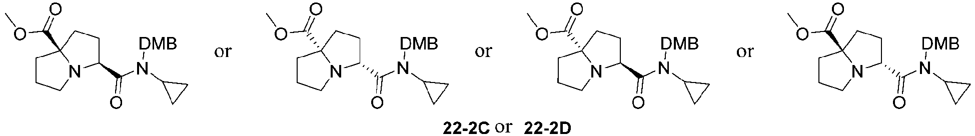

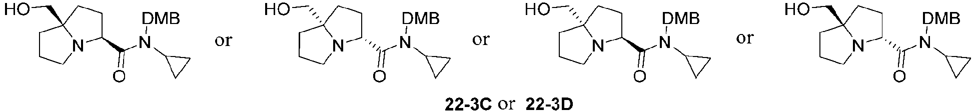

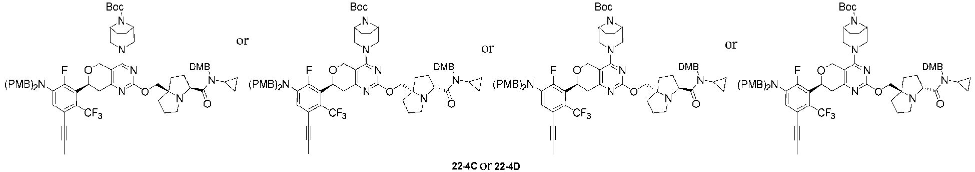

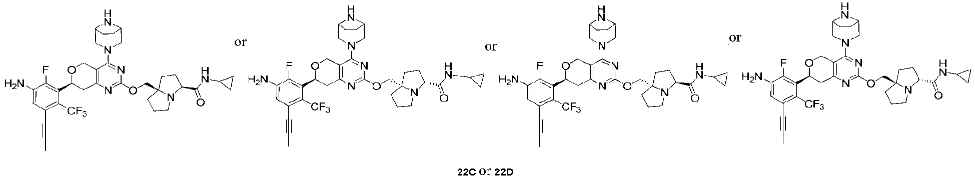

### Step 1

2,4-Dimethoxybenzaldehyde (500 mg, 3.01 mmol) was added to anhydrous tetrahydrofuran (5 mL), followed by cyclopropylamine (6.02 mmol, 416.97 µL) and glacial acetic acid (18.00 mg, 300.89 µmol). The mixture was reacted at 25°C for 2 hours. Sodium borohydride (567.20 mg, 9.03 mmol) was added to the reaction system, and the mixture was stirred at 25°C for 16 hours. The mixture was quenched with water (5 mL) and extracted with dichloromethane (10 mL x 3). The extracted organic phases were combined and washed with saturated brine (10 mL), dried over anhydrous sodium sulfate, filtered, and concentrated under reduced pressure to yield compound **22-1C.** MS m/z = 208.2 [M+H]⁺.

### Step 2

Compound **16-7C** (140 mg, 656.56 µmol) was dissolved in anhydrous dichloromethane (6 mL). N,N-diisopropylethylamine (3.28 mmol, 571.80 µL), compound **22-1C** (176.90 mg, 853.54 mmol), and N,N,N',N'-tetramethyl-O-(7-azabenzotriazol-1-yl)uronium hexafluorophosphate (499.31 mg, 1313.14 µmol) were added. The reaction was allowed to proceed at 25°C for 16 hours. The mixture was quenched by the addition of water (5 mL) and extracted with ethyl acetate (10 mL x 3). The extracted organic phases were combined and washed with saturated brine (10 mL), dried over anhydrous sodium sulfate, and filtered. The filtrate was concentrated under reduced pressure, and separated by column chromatography (petroleum ether:ethyl acetate = 3:1) to provide compound **22-2C.** MS m/z = 403.2 [M+H]⁺.

Referring to step 2, compound **16-7D** was used as a starting material in place of compound **16-7C** to obtain compound **22-2D.** MS m/z = 403.2 [M+H]⁺.

### Step 3

Compound **22-2C** (170 mg, 422.38 µmol) was dissolved in anhydrous tetrahydrofuran (3 mL) and anhydrous methanol (3 mL). Sodium borohydride (79.90 mg, 2.11 mmol) and lithium chloride (17.90 mg, 422.38 µmol) were added and reacted at 50°C for 2 hours. The mixture was quenched by the addition of water (0.5 mL), and filtered. The filtrate was concentrated under reduced pressure to obtain compound **22-3C.** MS m/z = 375.2 [M+H]⁺.

Referring to step 3, compound **22-2D** was used as a starting material in place of compound **22-2C** to obtain compound **22-3D.** MS m/z = 375.2 [M+H]⁺.

### Step 4

Compound **22-3C** (104 mg, 277.72 µmol) was dissolved in anhydrous tetrahydrofuran (5 mL), and sodium hydride (33.30 mg, 833.17 µmol, 60% purity) was added. The mixture was reacted at 0°C for 0.5 hours. Compound **4-2** (150.00 mg, 170.46 µmol) was then added and the mixture was reacted at 25°C for 1 hour. The mixture was quenched by the addition of saturated aqueous ammonium chloride (10 mL) and extracted with ethyl acetate (10 mL x 3). The extracted organic phases were combined and washed with saturated brine (10 mL), dried over anhydrous sodium sulfate, and filtered. The filtrate was concentrated under reduced pressure, and separated by column chromatography (petroleum ether:ethyl acetate = 1:1) to afford compound **22-4C.** MS m/z = 1174.5 [M+H]⁺.

Referring to step 4, compound **22-3D** was used as a starting material in place of compound **22-3C** to obtain compound **22-4D.** MS m/z = 1174.5 [M+H]⁺.

### Step 5

Compound **22-4C** (150.00 mg, 127.73 µmol) was dissolved in trifluoroacetic acid (5 mL) and reacted at 50°C for 0.5 h. The reaction solution was concentrated under reduced pressure to obtain a crude product, which was separated by preparative HPLC (Chromatographic column: Xtimate C18 150*40mm*5µm; mobile phase: [water (0.1% ammonia)-acetonitrile]; gradient: (acetonitrile): 35%-60%) and lyophilized to obtain compound **22C.** MS m/z = 684.6 [M+H]⁺. ¹H NMR (400 MHz, CD₃OD) δ ppm 6.90 (d, *J =* 8.4 Hz, 1H), 5.14 *(dd, J= 11.6* Hz, 4.8 Hz, 1H), 4.79 (d, *J = 13.6* Hz, 1H), 4.64 (d, *J =* 13.6 Hz, 1H), 4.14 - 4.04 (m, 3H), 3.68 (dd, *J =* 8.0 Hz, 5.2 Hz, 1H), 3.52 - 3.49 (m, 3H), 3.42 - 3.38 (m, 1H), 3.21 (dd, *J =* 17.6 Hz, 11.2 Hz, 1H), 3.03 (d, *J= 12.4* Hz, 1H), 2.88 - 2.84 (m, 2H), 2.73 - 2.65 (m, 2H), 2.11 - 2.05 (m, 2H), 2.01 - 1.97 (m, 5H), 1.94 - 1.81 (m, 6H), 1.76 - 1.64 (m, 2H), 0.75 - 0.71 (m, 2H), 0.53 - 0.48 (m, 2H).

Compound **22-4D** (100.00 mg, 85.16 µmol) was dissolved in trifluoroacetic acid (3 mL) and reacted at 50°C for 0.5 h. The reaction solution was concentrated under reduced pressure to obtain a crude product, which was separated by preparative high-performance liquid chromatography (Chromatographic column: Xtimate C18 150*40mm*5µm; mobile phase: [water (0.1% ammonia)-acetonitrile]; gradient: (acetonitrile): 35%-70%) and lyophilized to obtain compound **22D.** MS m/z = 684.5 [M+H]⁺. ¹H NMR (400 MHz, CD₃OD) δ ppm 6.90 (d, *J =* 8.4 Hz, 1H), 5.14 (dd, *J =* 11.6, 4.8 Hz, 1H), 4.79 (d, *J =* 13.6 Hz, 1H), 4.64 (d, *J =* 13.6 Hz, 1H), 4.16 - 4.12 (m, 2H), 4.03 (d, *J =* 10.4 Hz, 1H), 3.68 (dd, *J =* 8.0 Hz, 5.2 Hz, 1H), 3.53 - 3.50 (m, 3H), 3.42 - 3.39 (m, 1H), 3.21 (dd, *J =* 17.6 Hz, 11.2 Hz, 1H), 3.03 (d, *J =* 12.4 Hz, 1H), 2.88 - 2.81 (m, 2H), 2.78 - 2.66 (m, 2H), 2.12 - 2.06 (m, 2H), 2.01 - 1.97 (m, 5H), 1.95 - 1.81 (m, 6H), 1.77 - 1.66 (m, 2H), 0.76 - 0.72 (m, 2H), 0.52 - 0.48 (m, 2H).

Compounds **22C** and **22D** were confirmed to have structures as follows:

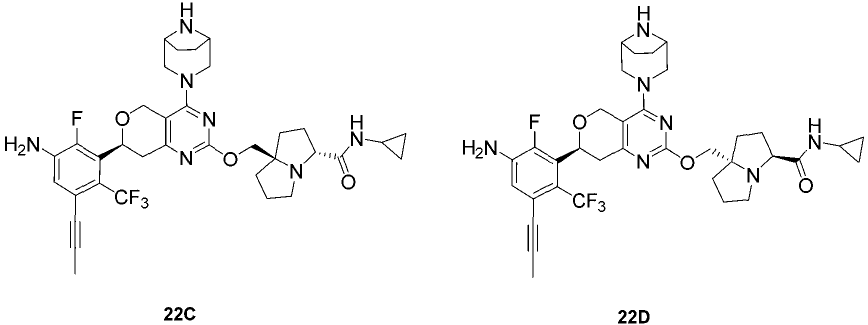

### Example 23

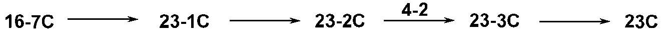

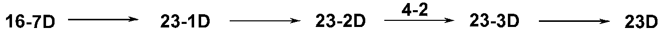

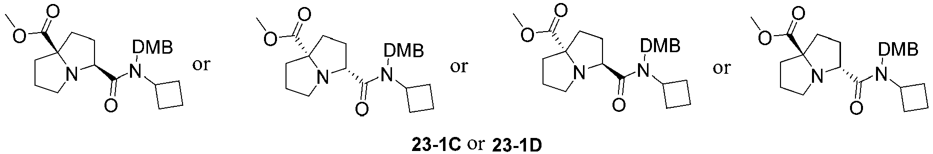

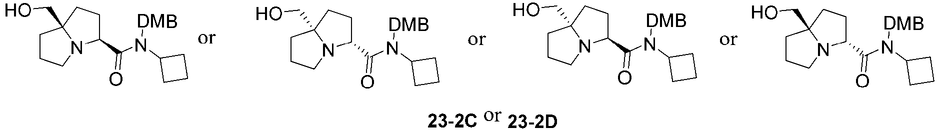

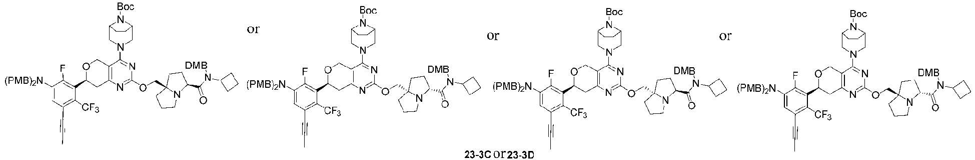

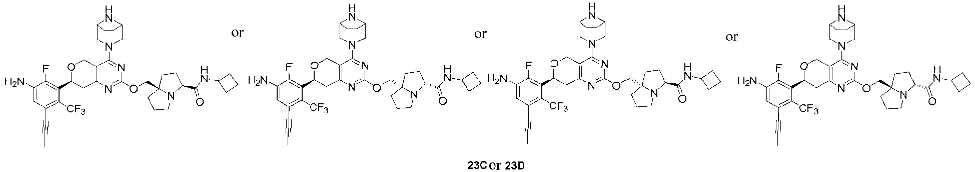

### Step 1

**C** ompound **16-7C** (0.20 g, 937.96 µmol) was dissolved in dimethylformamide (2 mL). N,N-diisopropylethylamine (606.12 mg, 4.69 mmol), N,N,N',N'-tetramethyl-O-(7-azabenzotriazol-1-yl)uronium hexafluorophosphate (713.28 mg, 1.88 mmol), and N-[(2,4-dimethoxyphenyl)methyl]cyclobutanamine (207.56 mg, 937.96 µmol) were added sequentially. The mixture was reacted at 25°C for 12 hours. Water (10 mL) was added to the reaction solution, which was extracted with ethyl acetate (20 mL x 3). The mixture was concentrated under reduced pressure and separated by column chromatography (petroleum ether:ethyl acetate = 5:1) to obtain compound **23-1C.** MS m/z = 417.4 [M+H]⁺

Referring to step 1, compound **16-7D** was used as a starting material in place of compound **16-7C** to obtain compound **23-1D.** MS m/z = 417.4 [M+H]⁺.

### Step 2

Compound **23-1C** (0.20 g, 480.18 µmol) was dissolved in methanol (2 mL). Sodium methoxide (2.59 mg, 48.02 µmol) and sodium borohydride (90.83 mg, 2.40 mmol) were added at 0°C and reacted at 25°C for 16 hours. The reaction was quenched by adding saturated ammonium chloride (10 mL), and the mixture was extracted with ethyl acetate (20 mL). The mixture was dried over anhydrous sodium sulfate, filtered, and concentrated under reduced pressure to obtain compound **23-2C.** MS m/z = 389.3 [M+H]⁺

Referring to step 2, compound **23-1D** was used as a starting material in place of compound **23-1C** to obtain compound **23-2D.** MS m/z = 389.3 [M+H]⁺.

### Step 3

Compound **23-2C** (50.00 mg, 128.70 µmol) was dissolved in tetrahydrofuran (1 mL). Sodium hydride (51.48 mg, 1.29 mmol, 60% purity) was added at 0°C and stirred for 0.5 h. Finally, compound **4-2** (113.25 mg, 128.70 µmol) was added and the mixture was allowed to react at 25°C for 2 h. The reaction was quenched by adding water (10 mL), and the mixture was extracted with ethyl acetate (20 mL). The mixture was concentrated under reduced pressure and separated by column chromatography (petroleum ether:ethyl acetate = 0:1) to obtain compound **23-3C.** MS m/z = 1188.7 [M+H]⁺.

Referring to step 3, compound **23-2D** was used as a starting material in place of compound **23-2C** to obtain compound **23-3D.** MS m/z = 1188.7 [M+H]⁺.

### Step 4

Compound **23-3C** (17 mg, 14.31 µmol) was dissolved in dichloromethane (1 mL), and trifluoroacetic acid (0.3 mL) was added. The mixture was reacted at 25°C for 0.5 h. The reaction solution was concentrated under reduced pressure to obtain a crude product. The crude product was separated by preparative HPLC (Chromatographic column: Waters Xbridge, 250 x 19 mm, 5 mm; mobile phase: [water (0.1% ammonia)-acetonitrile]; gradient: (acetonitrile): 55%-70%) and lyophilized to obtain compound 23C. MS m/z = 698.5 [M+H]⁺. ¹H NMR (400 MHz, CD₃OD) δ ppm 6.90 (d, *J =* 8.0 Hz, 1H), 5.15 (dd, *J =* 12.0, 4.0 Hz, 1H), 4.84 - 4.79 (m, 1H), 4.62 (d, *J =* 16.0 Hz, 1H), 4.34 - 4.26 (m, 1H), 4.16 - 4.06 (m, 3H), 3.74 - 3.70 (m, 1H), 3.51 - 3.48 (m, 3H), 3.44 - 3.41 (m, 1H), 3.25 - 3.13 (m, 1H), 3.04 (d, *J* = 12.0 Hz, 1H), 2.89 - 2.82 (m, 2H), 2.76 - 2.70 (m, 1H), 2.32 - 2.26 (m, 2H), 2.12 - 2.06 (m, 2H), 2.04 - 1.89 (m, 11H), 1.85 - 1.83 (m, 2H), 1.77 - 1.71 (m, 4H).

Compound **23-3D** (40 mg, 33.66 µmol) was dissolved in dichloromethane (1 mL), and trifluoroacetic acid (1 mL) was added. The mixture was reacted at 25°C for 0.5 h. The reaction solution was concentrated under reduced pressure to obtain a crude product. The crude product was separated by preparative HPLC (Waters Xbridge, 250 x 19 mm, 5 mm; mobile phase: [water (0.1% ammonia)-acetonitrile]; gradient: (acetonitrile): 55%-70%) and lyophilized to obtain compound **23D.** MS m/z = 698.5 [M+H]⁺. ¹H NMR (400 MHz, CD₃OD) δ ppm 6.90 (d, *J* = 8.0 Hz, 1H), 5.15 (dd, *J =* 12.0, 4.0 Hz, 1H), 4.83 - 4.78 (m, 1H), 4.65 (d, *J =* 16.0 Hz, 1H), 4.34 - 4.26 (m, 1H), 4.18 - 4.12 (m, 2H), 4.04 (d, *J =* 8.0 Hz, 1 H), 3.73 - 3.69 (m, 1H), 3.53 (d, *J =* 12.0 Hz, 3H), 3.43 - 3.41 (d, *J =* 8.0 Hz, 1H), 3.25 - 3.18 (m, 1H), 3.04 *(d, J=* 12.0 Hz, 1H), 2.91 - 2.82 (m, 2H), 2.76-2.70 (m, 1H), 2.33 - 2.26 (m, 2H), 2.11 - 2.07 (m, 2H), 2.01 - 1.90 (m, 11H), 1.85 - 1.83 (m, 2H), 1.78-1.68 (m, 4H).

Compounds **23C** and **23D** were confirmed to have structures as follows:

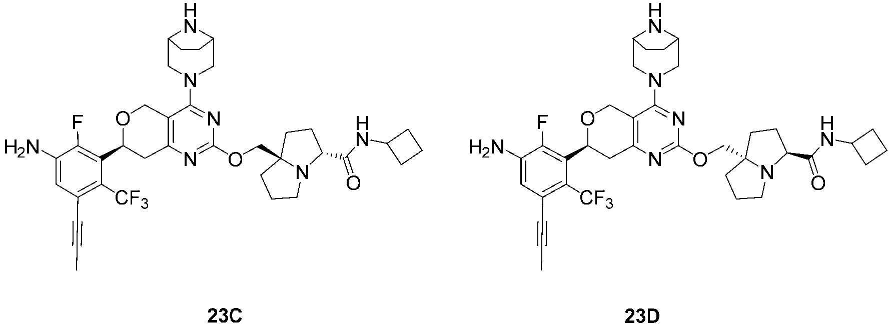

### Example 24

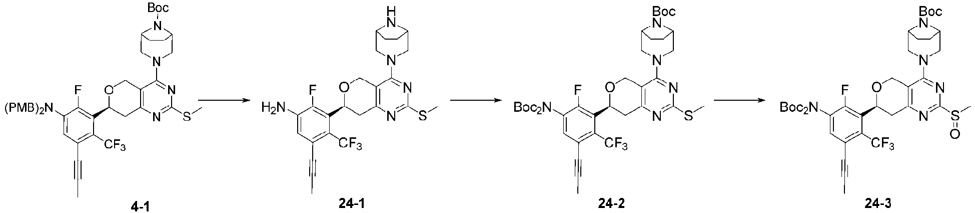

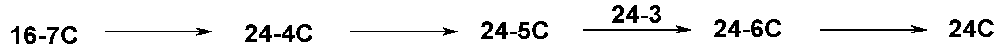

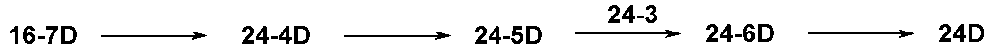

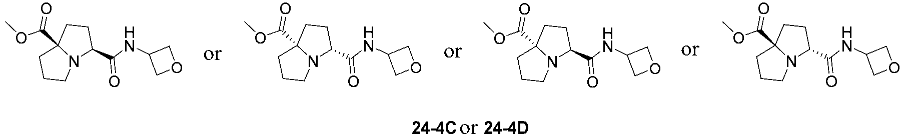

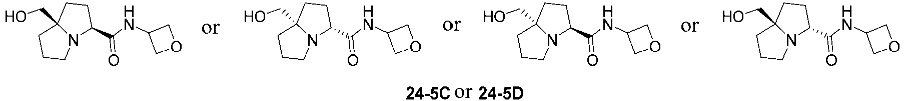

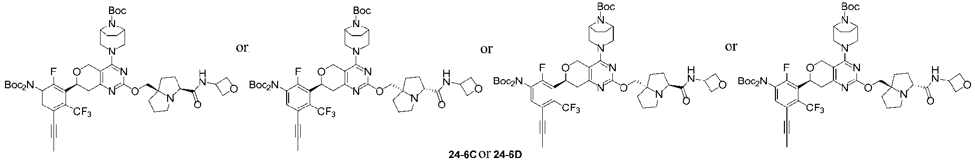

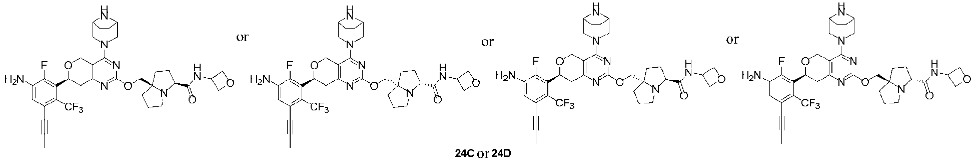

### Step 1

Compound **4-1** (280 mg, 0.33 mmol) was dissolved in dichloromethane (3 mL), and trifluoroacetic acid (376 mg, 3.30 mmol) was added. The resulting reaction solution was stirred at 25°C under nitrogen for 3 hours. The reaction solution was concentrated under reduced pressure, and 3 mL of ethyl acetate was added to the residue. A hydrogen chloride/ethyl acetate solution (4 M, 1 mL) was then added. The reaction was stirred at 25°C for 1 hour, and the mixture was concentrated under reduced pressure to obtain the hydrochloride salt of compound **24-1.**

### Step 2

The hydrochloride salt of compound **24-1** (160 mg) and triethylamine (159.50 mg, 1.58 mol) were added to dichloromethane (5 mL). Di-tert-butyl dicarbonate (1.38 g, 6.30 mmol) and 4-dimethylaminopyridine (38.51 mg, 0.33 mmol) were then added. The resulting reaction mixture was stirred at 25°C under nitrogen for 12 hours. The reaction mixture was concentrated under reduced pressure to obtain a crude product. The crude product was purified by column chromatography (petroleum ether:ethyl acetate = 5:1) to obtain compound 24-2. MS m/z = 808.6 [M+H]⁺.

### Step 3

Compound **24-2** (240 mg, 0.30 mmol) was dissolved in tetrahydrofuran (5 mL), and m-chloroperbenzoic acid (61.52 mg, 0.35 mmol, 85% purity) was added. The resulting reaction mixture was stirred at 25°C under nitrogen for 0.5 hours. The reaction mixture was concentrated under reduced pressure to obtain compound **24-3.** MS m/z = 824.5 [M+H]⁺.

### Step 4

Compound **16-7C** (180 mg, 0.84 mmol) was dissolved in N,N-dimethylformamide (4 mL). Benzotriazole-N,N,N',N'-tetramethyluronium hexafluorophosphate (641.95 mg, 1.69 mmol) and N,N-diisopropylethylamine (218.42 mg, 1.69 mmol) were added, and the reaction mixture was stirred at 25°C for 30 minutes. 3-Oxetaneamine hydrochloride (92.09 mg, 1.26 mmol) was added to the mixture, and the resulting mixture was stirred at 25°C for 16 hours. The reaction mixture was diluted with water (20 mL) and extracted with ethyl acetate (50 mL). The organic phase was washed with saturated brine, dried over anhydrous sodium sulfate, filtered, and concentrated under reduced pressure. Separation by column chromatography (dichloromethane:methanol = 20:1) provided compound **24-4C.** MS m/z = 269.18 [M+H]⁺.

Referring to step 4, compound **16-7D** was used as a starting material in place of compound 16-7C to obtain compound **24-4D.** MS m/z = 269.18 [M+H]⁺.

### Step 5

Compound **24-4C** (100 mg, 0.37 mmol) was dissolved in tetrahydrofuran (5 mL), and sodium borohydride (69.98 mg, 1.85 mmol) and lithium chloride (78.42 mg, 1.85 mmol) were added. The mixture was reacted at 25°C under nitrogen for 12 hours. The reaction mixture was diluted with water (10 mL) and extracted with ethyl acetate (30 mL). The organic phase was washed with saturated brine, dried over anhydrous sodium sulfate, filtered, and concentrated under reduced pressure. Separation by column chromatography (dichloromethane:methanol = 10:1) afforded compound **24-5C.** MS m/z = 241.22 [M+H]⁺.

Referring to step 5, compound **24-4D** was used as a starting material in place of compound **24-4C** to obtain compound **24-5D.** MS m/z = 241.22 [M+H]⁺.

### Step 6

Compound **24-5C** (70 mg, 0.29 mmol) was dissolved in tetrahydrofuran (3 mL), followed by the addition of sodium hydride (12 mg, 0.29 mmol, 60% purity). Under nitrogen, the reaction was stirred at 25°C for 0.5 hours. A solution of compound **24-3** (210 mg, 0.29 mmol) in tetrahydrofuran (1 mL) was then added. After the addition, the reaction was stirred at 25°C for 2 hours. The reaction solution was dissolved in 10 mL of ethyl acetate and washed with saturated brine (5 mL). The organic phase was dried, and filtered. The filtrate was concentrated under reduced pressure. Separation by column chromatography (dichloromethane:methanol = 20:1) afforded compound **24-6C.** MS m/z = 900.6 [M-100+1]⁺.

Referring to step 6, compound **24-5D** was used as a starting material in place of compound 24-5C to obtain compound **24-6D.** MS m/z = 900.6 [M-100+1]⁺.

### Step 7

Compound **24-6C** (10 mg, 10.00 µmol) was dissolved in dichloromethane (3 mL), followed by the addition of zinc bromide (5 mg, 20.00 µmol). The mixture was stirred at 25°C under nitrogen for 2 hours. The reaction solution was concentrated under reduced pressure to obtain a crude product. The crude product was separated by preparative HPLC (Chromatographic column: Waters SunFire, 250×19 mm, 5 µm; mobile phase: [water (0.1% formic acid)-acetonitrile]; (acetonitrile): 10%-40%) and lyophilized to obtain the formate salt of compound **24C.** MS m/z = 700.4 [M+H]⁺, ¹H NMR (400 MHz, CD₃OD) δ ppm 8.39 (s, 2H), 6.92 (d, *J=* 8.4 Hz, 1H), 5.19 (dd, *J* = 11.2, 4.4 Hz, 1H), 4.94 *(q, J =* 6.4 Hz, 1H), 4.88 *(d, J=* 6.8 Hz, 3H), 4.72 *(d, J=* 13.8 Hz, 1H), 4.59 (q, *J =* 5.8 Hz, 2H), 4.41 (d, *J =* 6.8 Hz, 3H), 4.27 (d, *J =* 13.8 Hz, 1H), 4.11 (d, *J =* 12.8 Hz, 2H), 3.78 (d, *J = 13.6* Hz, 1H), 3.65 (d, *J =* 13.2 Hz, 1H), 3.49 - 3.36 (m, 1H), 3.27 (d, *J =* 11.6 Hz, 2H), 3.13 (s, 1H), 2.93 (dd, *J =* 18.2, 4.4 Hz, 1H), 2.37 - 2.19 (m, 5H), 2.14 - 2.05 (m, 4H), 2.03 - 1.99 (m, 4H), 1.97 - 1.91 (m, 2H).

Compound **24-6D** (30 mg, 30.00 µmol) was dissolved in dichloromethane (3 mL), followed by the addition of zinc bromide (14 mg, 30.00 µmol). Under nitrogen, the mixture was stirred at 25°C for 2 hours. The reaction solution was concentrated under reduced pressure to obtain a crude product. The crude product was separated by preparative HPLC (Chromatographic column: Waters SunFire, 250×19 mm, 5 µm; mobile phase: [water (0.1% formic acid)-acetonitrile]; (acetonitrile): 10%-40%) and lyophilized to obtain the formate salt of compound **24D.** MS m/z = 700.5 [M+H]⁺. ¹H NMR (400 MHz, CD₃OD) δ ppm 8.46 (s, 3H), 6.92 *(d, J=* 8.4 Hz, 1H), 5.19 (dd, *J =* 11.0, 4.0 Hz, 1H), 4.98 - 4.92 (m, 1H), 4.91 - 4.89 (m, 2H), 4.72 (d, *J =* 13.6 Hz, 2H), 4.60 - 4.57 (m, 2H), 4.37 - 4.23 (m, 3H), 4.21 - 4.15 (m, 1H), 4.07 (d, *J =* 12.6 Hz, 2H), 3.76 (d, *J =* 14.6 Hz, 1H), 3.63 (d, *J* = 13.6 Hz, 1H), 3.27 (d, *J =* 13.2 Hz, 3H), 3.01 - 2.96 (m, 1H), 2.95 - 2.86 (m, 1H), 2.33 - 2.13 (m, 5H), 2.13 - 2.03 (m, 4H), 2.02 (s, 3H), 1.99 - 1.85 (m, 3H).

Compounds 24C and 24D were confirmed to have structures as follows:

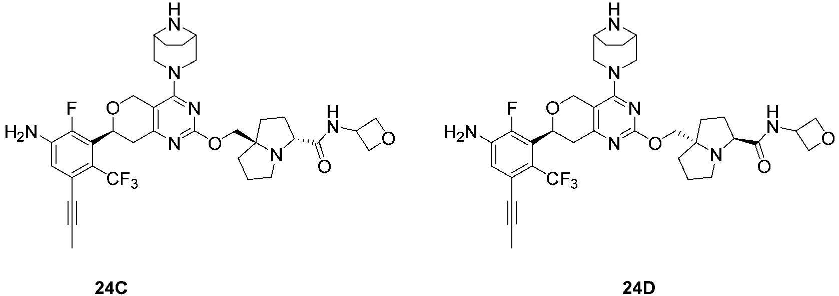

### Example 25

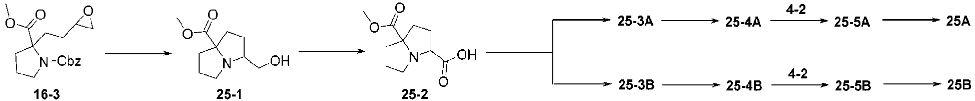

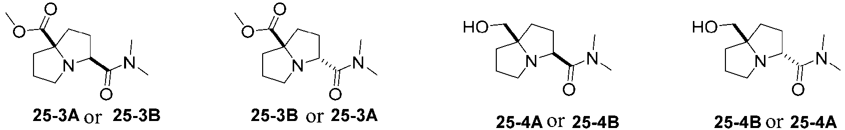

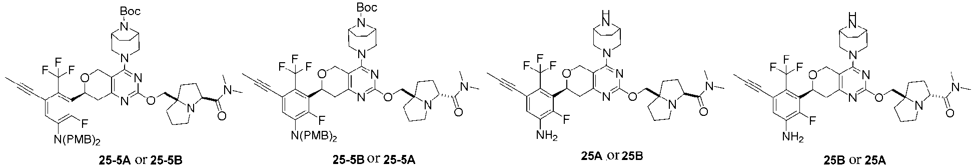

### Step 1

Compound **16-3** (8.00 g, 24.00 mmol) was dissolved in methanol (30 mL), and 10% palladium on carbon (1.5 g) was added. The reaction mixture was stirred at 25°C for 24 hours under a hydrogen atmosphere. The reaction mixture was filtered and the filtrate was concentrated to obtain compound **25-1.** MS m/z = 200.2 [M+H]⁺

### Step 2

Periodic acid (12.30 g, 53.95 mmol) was dissolved in acetonitrile (0.075% water) (50.0 mL). Chromium trioxide (647.40 mg, 6.47 mmol) was slowly added at 0°C and stirred for 10 minutes. Compound **25-1** was dissolved in acetonitrile (50.0 mL) and slowly added to the mixed solution at 0°C. After addition, the mixture was slowly warmed to room temperature and stirred for 10 hours. The mixture was filtered and the filtrate was concentrated under reduced pressure to obtain compound **25-2.** MS m/z = 214.2 [M+H]⁺

### Step 3

Compound **25-2** (0.90 g, 4.22 mmol) was dissolved in N,N-dimethylformamide (20 mL). Benzotriazole-N,N,N',N'-tetramethyluronium hexafluorophosphate (2.41 g, 6.33 mmol) and N,N-diisopropylethylamine (2.73 g, 21.10 mmol) were added, and the reaction mixture was stirred at room temperature for 30 minutes. Dimethylamine hydrochloride (1.72 g, 21.10 mmol) was added to the above mixture, and the resulting mixture was stirred at room temperature for 16 hours. The reaction mixture was diluted with water (100 mL) and extracted with dichloromethane (35 mL). The organic phase was washed with saturated brine (10 mL), dried over anhydrous sodium sulfate, filtered, and concentrated under reduced pressure to obtain a crude product. The crude product was subjected to preparative high performance liquid chromatography separation (Chromatographic column: Waters Xbridge, 250*19mm, 5mm; mobile phase: [water (0.1% ammonia water)-acetonitrile]; gradient: (acetonitrile): 10%-40%) to obtain compound **25-3A** and compound **25-3B.** LCMS analysis method (Chromatographic column: Waters Xbridge C18, (50mm×4.6mm×3.5µm); mobile phase: [A: water (0.1% ammonia) B: acetonitrile]; gradient: B%: 0-10%, 0.2 min; 20-95%, 1.8 min; 95-95%, 0.7 min; 95-10%, 0.1 min; 10-10%, 0.7 min) Rt = 1.780 min for compound **25-3A,** MS m/z = 241.2 [M+H]⁺, and Rt = 1.833 min for compound **25-3B,** MS m/z = 241.2 [M+H]⁺.

### Step 4

Compound **25-3A** (0.13 g, 540.99 µmol) was dissolved in methanol (5 mL). Sodium borohydride (61.40 mg, 1.62 mmol) and 30% sodium methoxide in methanol (2.92 mg, 54.10 µmol) were added at 0°C. The reaction mixture was stirred at 25°C for 16 hours. After completion, the reaction was quenched with saturated ammonium chloride solution (10 mL) and extracted with dichloromethane (10 mL). The organic phase was washed with saturated brine (5 mL), dried over anhydrous sodium sulfate, filtered, and concentrated under reduced pressure. Separation by column chromatography (dichloromethane:methanol = 10:1) afforded compound **25-4A.** MS m/z = 213.1 [M+H]⁺.

Referring to step 4, compound **25-3B** was used as a starting material in place of compound **25-3A** to obtain compound **25-4B.** MS m/z = 213.1 [M+H]⁺.

### Step 5

Compound **25-4A** (50.00 mg, 235.53 µmol) was dissolved in tetrahydrofuran (2 mL) and cooled to 0°C. NaH (16.96 mg, 706.59 µmol, 60% purity) was added and stirred for 1 hour. Compound **4-2** (50.00 mg, 235.53 µmol) was added to the above mixture, and the resulting mixture was stirred at 25°C for 1 hour. The resulting mixture was diluted with water (5 mL) and extracted with ethyl acetate (20 mL). The organic phase was washed with saturated brine (2 mL), dried over anhydrous sodium sulfate, filtered, and concentrated under reduced pressure. Separation by column chromatography (dichloromethane:methanol = 10:1) afforded compound **25-5A.** MS m/z = 1012.8 [M+H]⁺.

Referring to step 5, compound **25-4B** was used as a starting material in place of compound **25-4A** to obtain compound **25-5B.** MS m/z = 1012.8 [M+H]⁺.

### Step 6

Compound **25-5A** (0.14 g, 138.32 µmol) was dissolved in trifluoroacetic acid (2 mL) and stirred at 25°C for 1 hour. The reaction mixture was concentrated to remove TFA. The crude product was separated by preparative HPLC (Chromatographic column: Waters Xbridge, 250 x 19 mm, 5 mm; [water (0.1% ammonia)-acetonitrile]; gradient: acetonitrile: 30%-70%). Mobile phases A: water (0.1% ammonia) and B: (acetonitrile); gradient: B% = 30%-70%). After lyophilization, compound **25A** was obtained. MS m/z = 672.6 [M+H]⁺. ¹H NMR (400 MHz, CD₃OD) δ ppm 6.90 (d, *J =* 8.0 Hz, 1H), 5.13 - 5.09 (m, 1H), 4.78 - 4.67 (m, 2H), 4.54 (d, *J =* 8.0 Hz, 1H), 4.35 - 4.28 (m, 1H), 4.26 - 4.23 (m, 1H), 4.13 - 4.09 (m, 1H), 3.91 - 3.88 (m, 1H), 3.27 - 3.19 (m, 2H), 3.16 - 3.10 (m, 4H), 2.94 - 2.88 (m, 4H), 2.79 - 2.66 (m, 4H), 2.22 - 2.15 (m, 2H), 2.11 - 1.89 (m, 11H), 1.78 - 1.75 (m, 1H), 1.72 - 1.66 (m, 1H).

Compound **25-5B** (60.00 mg, 59.28 µmol) was dissolved in trifluoroacetic acid (1 mL) and stirred at 25°C for 1 hour. The reaction mixture was concentrated to remove TFA. The crude product was separated by preparative HPLC (Chromatographic column: Waters Xbridge, 250 x 19 mm, 5 mm; [water (0.1% ammonia)-acetonitrile]; gradient: acetonitrile: 30%-70%). Mobile phases A: water (0.1% ammonia) and B: (acetonitrile); gradient: B% = 30%-70%). After lyophilization, compound **25B** was obtained. MS m/z = 672.1 [M+H]⁺. ¹H NMR (400 MHz, CD₃OD) δ ppm 6.90 (d, *J =* 8.0 Hz, 1H), 5.13 - 5.10 (m, 1H), 4.78 - 4.67 (m, 2H), 4.52 - 4.47 (m, 1H), 4.32 - 4.28 (m, 1H), 4.26 - 4.21 (m, 1H), 4.15 - 4.10 (m, 1H), 3.91 - 3.87 (m, 1H), 3.27 - 3.22 (m, 2H), 3.15 - 3.13 (m, 3H), 2.93 - 2.92 (m, 3H), 2.80 - 2.72 (m, 4H), 2.67 - 2.66 (m, 1H), 2.54 - 2.50 (m, 1H), 2.18 - 2.04 (m, 3H), 2.02 - 1.89 (m, 7H), 1.83 - 1.74 (m, 4H), 1.62 - 1.55 (m, 1H).

### Example 26

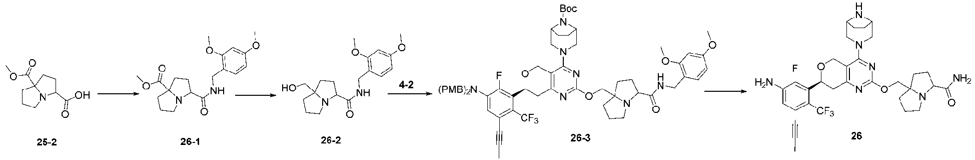

### Step 1

Compound **25-2** (1.00 g, 4.69 mmol) was dissolved in N,N-dimethylformamide (20 mL). N,N-diisopropylethylamine (2.12 g, 16.41 mmol), N,N,N',N'-tetramethyl-O-(7-azabenzotriazol-1-yl)uronium hexafluorophosphate (2.67 g, 7.03 mmol), and 2,4-dimethoxybenzylamine (1.02 g, 6.10 mmol) were added sequentially. The mixture was reacted at 25°C for 2 hours. Water (50 mL) was added to the reaction solution, which was then extracted with ethyl acetate (40 mL x 3). The mixture was concentrated under reduced pressure and separated by column chromatography (petroleum ether:ethyl acetate = 2:3) to obtain compound **26-1.** MS m/z = 363.5 [M+H]⁺.

### Step 2

Compound **26-1** (50.00 mg, 137.96 µmol) was dissolved in ethanol (1 mL), and tetrahydrofuran (0.6 mL), lithium chloride (11.70 mg, 275.92 µmol), and sodium borohydride (10.44 mg, 275.92 µmol) were added sequentially. The mixture was reacted at 50°C for 3 h. The mixture was cooled to room temperature, and DCM (20 mL) was added. The mixture was washed with saturated brine (3 mL x 3), dried over anhydrous sodium sulfate, filtered, and concentrated under reduced pressure to obtain compound **26-2.** MS m/z = 335.3 [M+H]⁺.

### Step 3

Compound **26-2** (30.00 mg, 89.71 µmol) was dissolved in tetrahydrofuran (3 mL). Sodium tert-butoxide (43.11 mg, 44.85 µmol) was added, and stirred for 0.5 hours, and finally compound **4-2** (77.51 mg, 89.71 µmol) was added. The reaction mixture was stirred at 25°C for 1 hour. Water (3 mL) was added to quench the reaction, and the mixture was extracted with ethyl acetate (20 mL). The mixture was concentrated under reduced pressure and separated by column chromatography (petroleum ether:ethyl acetate = 1:4) to afford compound **26-3.** MS m/z = 1134.6 [M+H]⁺.

### Step 4

Compound **26-3** (50.00 mg, 44.08 µmol) was dissolved in trifluoroacetic acid (1 mL) and reacted at 70°C for 1 hour. The reaction solution was concentrated under reduced pressure to obtain a crude product. The crude product was separated by preparative HPLC (Chromatographic column: Waters SunFire, 250*19 mm, 5 µm; mobile phase: [water (0.1% formic acid)-acetonitrile]; gradient: (acetonitrile): 40%-75%) and lyophilized to obtain the formate salt of compound **26.** MS m/z = 644.1 [M+H]⁺. ¹H NMR (400 MHz, CD₃OD) δ ppm 8.38 (brs, 1H), 6.91 (d, *J =* 8.5 Hz, 1H), 5.20 - 5.12 (m, 1H), 4.82 - 4.66 (m, 4H), 4.47 (d, *J =* 6.4 Hz, 1H), 4.27 (q, *J* = 10.8 Hz, 2H), 3.58 (d, *J =* 12.5 Hz, 1H), 3.52 - 3.41 (m, 1H), 3.26 - 3.15 (m, 4H), 2.91 - 2.77 (m, 2H), 2.38 - 2.28 (m, 2H), 2.20 - 2.09 (m, 2H), 2.07 - 1.93 (m, 9H), 1.86 - 1.71 (m, 2H).

### Example 27

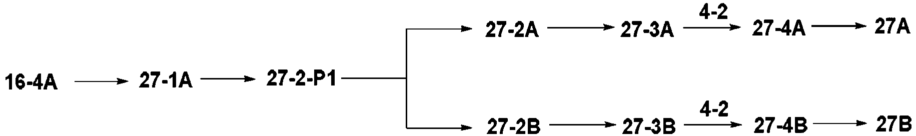

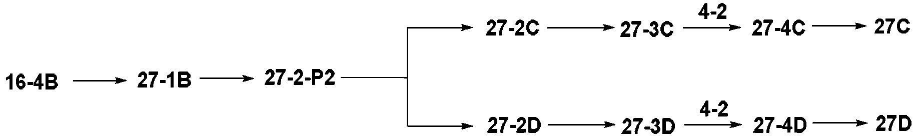

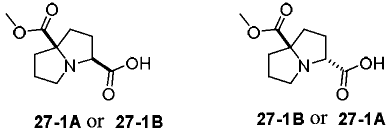

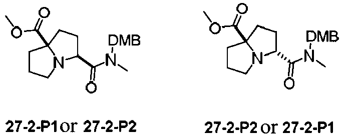

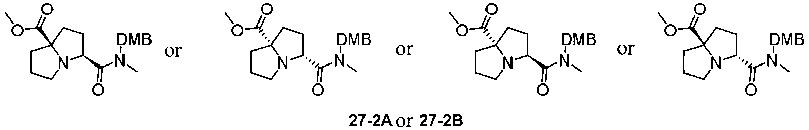

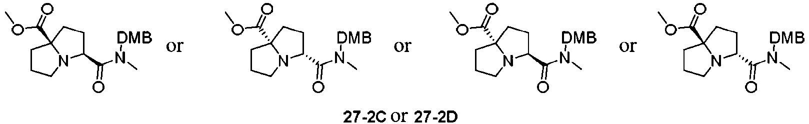

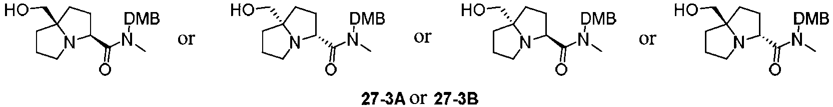

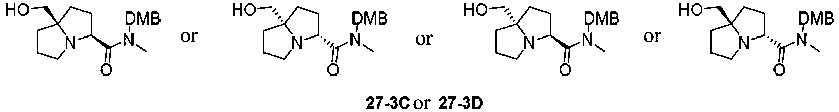

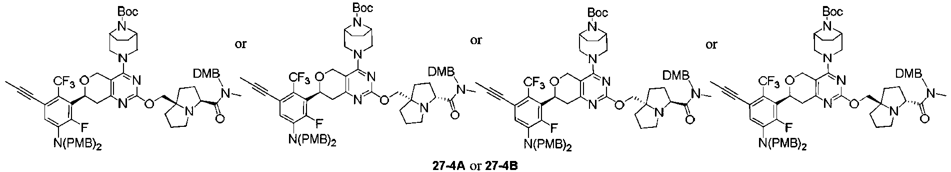

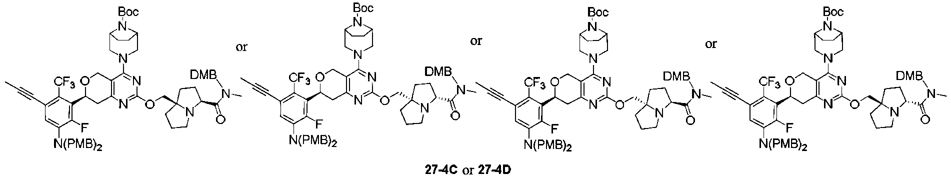

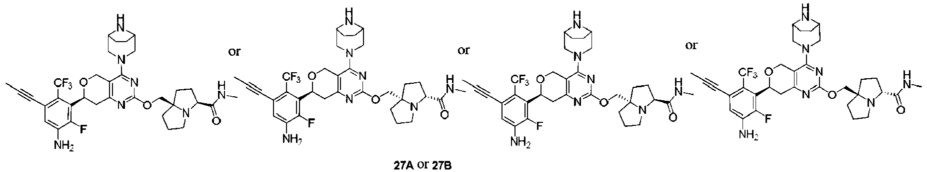

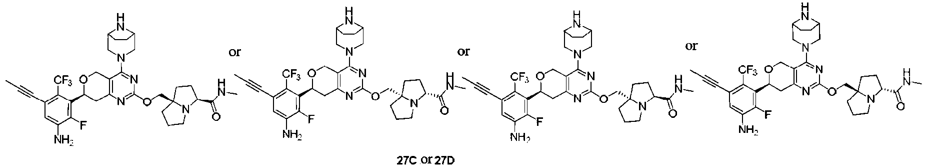

### Step 1

Periodic acid (1.71 g, 7.54 mmol) was dissolved in 15 mL of a mixed solvent (acetonitrile:water = 100:0.75). Chromium trioxide (89.54 mg, 0.90 mmol) was added at 0°C and stirred for 15 minutes. Compound **16-4A** (0.60 g, 3.02 mmol) was dissolved in acetonitrile (15 mL) and slowly added to the above mixed solution at 0°C. After addition, the mixture was slowly warmed to room temperature and stirred for 16 hours. The reaction mixture is filtered, and the filtrate is concentrated under reduced pressure to yield compound **27-1A.** MS m/z = 214.0 [M+H]⁺.

Referring to step 1, compound **16-4B** was used as a starting material in place of compound **16-4A** to obtain compound **27-1B.** MS m/z = 214.0 [M+H]⁺.

### Step 2

Compound **27-1A** (0.20 g, 0.94 mmol) was dissolved in N,N-dimethylformamide (5 mL). 2,4-Dimethoxy-N-methylbenzylamine (254.92 mg 1.41 mmol), N,N-diisopropylethylamine (605.63 mg 4.69 mmol), and 2-(7-azobenzotriazole)-N,N,N',N'-tetramethyluronium hexafluorophosphate (606.57 mg 1.60 mml) were added sequentially. The mixture was reacted at room temperature for 1 hour. 25 mL of water was added to the reaction solution, and the mixture was extracted with ethyl acetate (15 mL x 3). The organic phases were combined, washed sequentially with water (10 mL x 2) and saturated brine (10 mL), dried over anhydrous sodium sulfate, filtered, and concentrated. Separation by column chromatography (dichloromethane:methanol = 20:1) gave compound **27-2-P1,** MS m/z = 377.1 [M+H]⁺.

Compound **27-2-P1** was subjected to preparative SFC separation (Chromatographic column: DAICEL CHIRALPAK^{®} AS-10, 25*250mm 10 µm; mobile phase: A: supercritical carbon dioxide, B: [0.05% ammonia methanol-ethanol]; B%: 80%-20%) to obtain compound **27-2A** and compound **27-2B.** After analytical SFC (Chromatographic column: DAICEL CHIRALPAK^{®}AS-10 (4.6 mmI.D *150 mmL, 5 µm); mobile phase: [A: supercritical carbon dioxide, B: ethanol (0.05% diethylamine)]; gradient: B%: 5%-40%, 4 min), compound **27-2A** had Rt of 2.824 min, ee value 99.05%, MS m/z = 377.1 [M+H]⁺; compound **27-2B** had Rt of 3.327 min, ee value 99.10%, MS m/z = 377.1 [M+H]⁺.

Referring to step 2, compound **27-1B** was used as a starting material in place of compound **27-1A** to obtain compound **27-2-P2.** MS m/z = 377.1 [M+H]⁺.

Compound **27-2-P2** was subjected to preparative SFC separation (Chromatographic column: DAICEL CHIRALPAK^{®} AS-10, 25*250mm 10 µm; mobile phase: A: supercritical carbon dioxide, B: [0.05% ammonia methanol-ethanol]; B%: 80%-20%) to obtain compound **27-2C** and compound **27-2D.** After analytical SFC (Chromatographic column: DAICEL CHIRALPAK^{®}AS-10 (4.6 mmI.D*150 mmL, 5 µm); mobile phase: [A: supercritical carbon dioxide, B: ethanol (0.05% diethylamine)]; gradient: B%: 5%-40%, 4 min), compound **27-2C** had Rt of 2.891 min, ee value 95.80%, MS m/z =377.1 [M+H]⁺; compound **27-2D** had Rt of 3.327 min, ee value 96.34%, MS m/z = 377.1 [M+H]⁺.

### Step 3

Compound **27-2A** (80.00 mg, 0.21 mmol) was dissolved in methanol (5 mL). Sodium methoxide-methanol solution (7.66 mg, 0.04 mmol, 30% purity) and sodium borohydride (24.2 mg, 0.63 mmol) were added, and the reaction was stirred at 25°C for 16 hours. The mixture was quenched by the addition of 3 mL of saturated ammonium chloride solution and extracted with dichloromethane (5 mL x 3). The extracted organic phases were combined and washed with 5 mL of saturated brine, dried over anhydrous sodium sulfate, and filtered. The filtrate was concentrated under reduced pressure, and separated by column chromatography (dichloromethane:ethyl acetate = 20:1) to obtain compound **27-3A.** MS m/z = 349.1 [M+H]⁺.

Referring to step 3, compound **27-2B** was used as a starting material in place of compound **27-2A** to obtain compound **27-3B.** MS m/z = 349.1 [M+H]⁺.

Referring to step 3, compound **27-2C** was used as a starting material in place of compound **27-2A** to obtain compound **27-3C.** MS m/z = 349.1 [M+H]⁺.

Referring to step 3, compound **27-2D** was used as a starting material in place of compound **27-2A** to obtain compound 27-3D. MS m/z = 349.1 [M+H]⁺.

### Step 4

Compound **27-3A** (60.00 mg, 0.17 mmol) was dissolved in tetrahydrofuran (5 mL). Sodium hydride (34.48 mg, 0.85 mmol, 60% purity) was added at 0°C and stirred for 0.5 hours. A solution of compound **4-2** (164.56 mg, 0.19 mmol) in tetrahydrofuran (1 mL) was then added to the system, and the mixture was warmed to 25°C and stirred for additional 1 hour. The reaction was quenched by the addition of 6 mL of saturated aqueous ammonium chloride. The mixture was extracted with ethyl acetate (5 mL x 2). The extracted organic phases were combined and washed with 5 mL of saturated brine, dried over anhydrous sodium sulfate, and filtered. The filtrate was concentrated under reduced pressure, and separated by column chromatography (dichloromethane:methanol = 20:1) to afford compound **27-4A.** MS m/z = 1148.5 [M+H]⁺.

Referring to step 4, compound **27-3B** was used as a starting material in place of compound **27-3A** to obtain compound **27-4B.** MS m/z = 1148.5 [M+H]⁺.

Referring to step 4, compound 27-3C was used as a starting material in place of compound **27-3A** to obtain compound **27-4C.** MS m/z = 1148.5 [M+H]⁺.

Referring to step 4, compound 27-3D was used as a starting material in place of compound **27-3A** to obtain compound **27-4D.** MS m/z = 1148.5 [M+H]⁺.

### Step 5

Compound **27-4A**(0.10 g, 87.15 µmol) was dissolved in trifluoroacetic acid (3 mL) and stirred at 25°C for 1 hour. The reaction solution was concentrated under reduced pressure to obtain a crude product. The crude product was separated by preparative HPLC (Chromatographic column: Waters Xbridge, 250 x 19 mm, 5 µm; mobile phase: [water (0.1% ammonia)-acetonitrile]; (acetonitrile): 50%-70%) and lyophilized to obtain compound **27A.** MS m/z = 658.6 [M+H]⁺. ¹H NMR (400 MHz, CD₃OD) δ ppm 6.90 (d, *J =* 8.0 Hz, 1H), 5.18 - 5.10 (m, 1H), 4.78 (s, 1H), 4.65 (d, *J* = 12.0 Hz, 1H), 4.23 (d, *J =* 12.0 Hz, 1H), 4.16 - 4.07 (m, 2H), 3.57 - 3.48 (m, 3H), 3.45 - 3.38 (m, 1H), 3.29 - 3.17 (m, 2H), 3.10 - 3.01 (m, 2H), 2.89 - 2.81 (m, 1H), 2.73 (s, 3H), 2.67 - 2.60 (m, 1H), 2.31 - 2.23 (m, 1H), 2.15 - 2.08 (m, 1H), 2.04 - 1.96 (m, 4H), 1.95 - 1.87 (m, 4H), 1.86 - 1.79 (m, 2H), 1.76 - 1.65 (m, 3H).

Compound **27-4B** (80.00 mg, 69.71 µmol) was dissolved in trifluoroacetic acid (3 mL) and stirred at 25°C for 1 hour. The reaction solution was concentrated under reduced pressure to obtain a crude product. The crude product was separated by preparative HPLC (Chromatographic column: Waters Xbridge, 250*19 mm, 5 µm; mobile phase: [water (0.1% ammonia)-acetonitrile]; (acetonitrile): 50%-70%) and lyophilized to obtain compound **27B.** MS m/z = 658.5 [M+H]⁺. ¹H NMR (400 MHz, CD₃OD) δ ppm 6.90 (d, *J =* 8.0 Hz, 1H), 5.21 - 5.09 (m, 1H), 4.80 (d, *J =* 12.0 Hz, 1H), 4.65 (d, *J=* 12.0 Hz, 1H), 4.25 (d, *J = 9.0* Hz, 1H), 4.16 - 4.06 (m, 2H), 3.57 - 3.48 (m, 3H), 3.44 - 3.38 (m, 1H), 3.30 - 3.15 (m, 2H), 3.10 - 3.01 (m, 2H), 2.89 - 2.80 (m, 1H), 2.73 (s, 3H), 2.68 - 2.60 (m, 1H), 2.31 - 2.21 (m, 1H), 2.19 - 2.09 (m, 1H), 2.01 - 1.96 (m, 4H), 1.94 - 1.78 (m, 6H), 1.77 - 1.64 (m, 3H).

Compound **27-4C** (90.00 mg, 78.36 µmol) was dissolved in trifluoroacetic acid (3 mL) and stirred at 25°C for 1 hour. The reaction solution was concentrated under reduced pressure to obtain a crude product. The crude product was separated by preparative HPLC (Chromatographic column: Waters Xbridge, 250*19 mm, 5 µm; mobile phase: [water (0.1% ammonia)-acetonitrile]; (acetonitrile): 50%-70%) and lyophilized to obtain compound **27C.** MS m/z = 658.3 [M+H]⁺. ¹H NMR (400 MHz, CD₃OD) δ ppm 6.90 (d, *J =* 8.0 Hz, 1H), 5.19 - 5.10 (m, 1H), 4.79 (d, *J=* 12.0 Hz, 1H), 4.65 (d, *J=* 12.0 Hz, 1H), 4.18 - 4.10 (m, 2H), 4.10 - 4.05 (m, 1H), 3.76 - 3.71 (m, 1H), 3.57 - 3.48 (m, 3H), 3.45 - 3.38 (m, 1H), 3.26 - 3.16 (m, 1H), 3.04 (d, *J =* 12.7 Hz, 1H), 2.92 - 2.81 (m, 2H), 2.75 (s, 3H), 2.75 - 2.68 (m, 1H), 2.14 - 2.05 (m, 2H), 2.04 - 1.97 (m, 5H), 1.96 - 1.80 (m, 6H), 1.76 - 1.64 (m, 2H).

Compound **27-4D** (95.00 mg, 82.28 µmol) was dissolved in trifluoroacetic acid (3 mL) and stirred at 25°C for 1 hour. The reaction solution was concentrated under reduced pressure to obtain a crude product. The crude product was separated by preparative HPLC (Chromatographic column: Waters Xbridge, 250*19 mm, 5 µm; mobile phase: [water (0.1% ammonia)-acetonitrile]; (acetonitrile): 50%-70%) and lyophilized to obtain compound **27D.** MS m/z = 658.4 [M+H]⁺. ¹H NMR (400 MHz, CD₃OD) δ ppm 6.90 (d, *J =* 8.0 Hz, 1H), 5.18 - 5.11 (m, 1H), 4.79 (d, *J =* 12.0 Hz, 1H), 4.64 *(d, J =* 12.0 Hz, 1H), 4.19 - 4.10 (m, 2H), 4.07 - 4.01 (m, 1H), 3.76 - 3.71 (m, 1H), 3.56 - 3.48 (m, 3H), 3.45 - 3.38 (m, 1H), 3.26 - 3.17 (m, 1H), 3.03 (d, *J =* 12.6 Hz, 1H), 2.92 - 2.81 (m, 2H), 2.75 (s, 3H), 2.75 - 2.69 (m, 1H), 2.15 - 2.06 (m, 2H), 2.05 - 1.97 (m, 5H), 1.96 - 1.80 (m, 6H), 1.76 - 1.65 (m, 2H).

Compounds **27C** and **27D** were confirmed to have structures as follows:

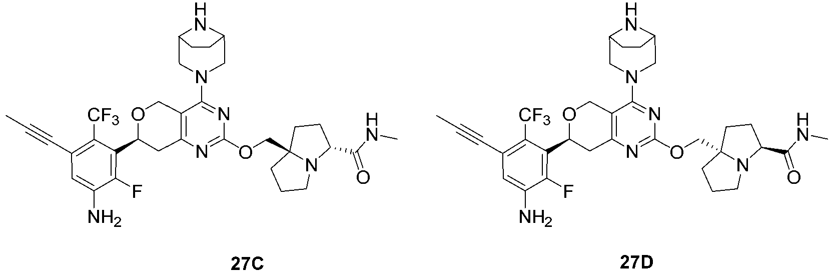

### Example 28

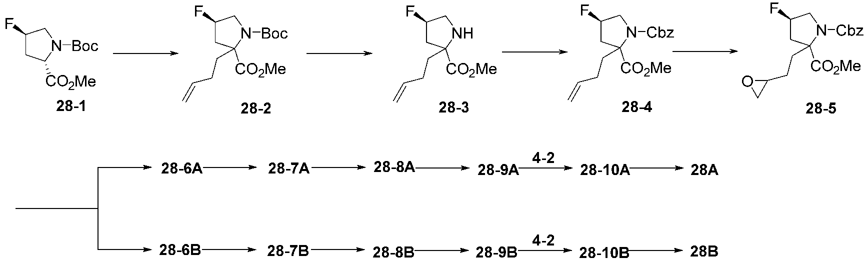

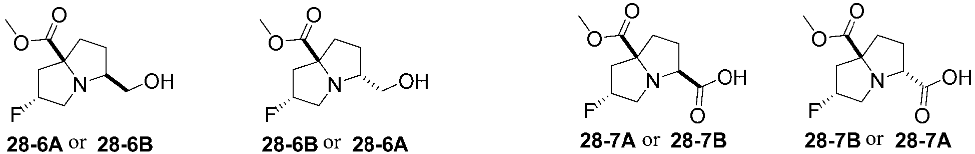

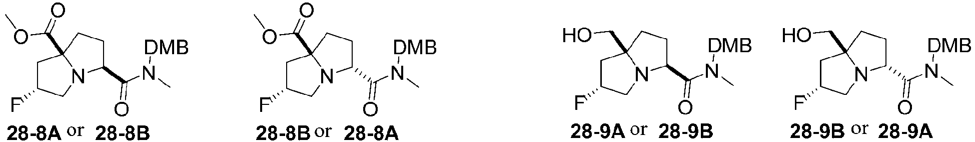

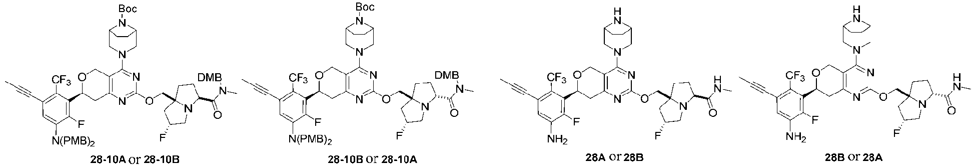

### Step 1

Compound **28-1** (1 g, 4.04 mmol) was weighed and anhydrous tetrahydrofuran (10 mL) was added under nitrogen. The mixture was cooled to -76°C in a dry ice bath, and lithium bis(trimethylsilyl)amide (12.12 mL, 12.12 mmol, 1 M) was slowly added. The mixture was stirred at -76°C for 40 minutes. A solution of 4-bromobutene (654.48 mg, 4.84 mmol) in anhydrous tetrahydrofuran (5 mL) was added, and stirred for additional 60 minutes. The reaction was quenched by the addition of saturated aqueous ammonium chloride (10 mL), and extracted with ethyl acetate (15 mL x 3). The extracted organic phases were combined and washed with saturated brine (20 mL), dried over anhydrous sodium sulfate, filtered, and concentrated under reduced pressure. Separation by column chromatography (ethyl acetate:petroleum ether = 1:7) afforded compound **28-2.** MS m/z = 302.2 [M+H]⁺

### Step 2

Compound 28-2 (500 mg, 1.66 mmol) was weighed. Dichloromethane (3 mL) and hydrochloric acid/1,4-dioxane solution (3 mL) were added, and the mixture was reacted at room temperature for 30 minutes. The reaction solution was directly concentrated to obtain the hydrochloride salt of compound 28-3. MS m/z = 202.1 [M+H]⁺

### Step 3

Compound 28-3 (500 mg, hydrochloride salt) was weighed and anhydrous tetrahydrofuran (10 mL) was added. Sodium hydride (148.80 mg, 3.72 mmol, 60% purity) was added under an ice bath at 0°C. The mixture was stirred at 0°C for 1 hour. Benzyl chloroformate (634.59 mg, 3.72 mmol) was added portionwise and stirred at 40°C for 16 hours. The reaction was quenched with water (15 mL) and extracted with ethyl acetate (20 mL x 3). The extracted organic phases were combined and washed with saturated brine (20 mL), dried over anhydrous sodium sulfate, filtered, and concentrated under reduced pressure. Separation by column chromatography (ethyl acetate:petroleum ether = 1:8) afforded compound **28-4** with MS m/z = 336.2 [M+H]⁺.

### Step 4

Compound **28-4** (300 mg, 0.89 mmol) was weighed and dichloromethane (5 mL) was added. m-Chloroperbenzoic acid (307.17 mg, 1.78 mmol) was added, and the mixture was stirred at room temperature for 2 hours. Saturated aqueous sodium sulfite solution (1 mL) was added to the reaction solution, and stirred for 5 minutes. Water (10 mL) was added. The mixture was extracted with dichloromethane (15 mL * 3). The organic phases were combined, washed with saturated brine (20 mL), dried over anhydrous sodium sulfate, filtered, and concentrated under reduced pressure. Separation by column chromatography (ethyl acetate:petroleum ether = 1:6) gave compound **28-5.** MS m/z = 352.2 [M+H]⁺.

### Step 5

Compound **28-5** (1 g, 2.85 mmol) was weighed and anhydrous methanol (10 mL) was added. 10% palladium on carbon (100 mg) was added and the atmosphere was replaced with hydrogen three times. The mixture was stirred at room temperature for 1 hour. The reaction solution was directly filtered, the filter cake was washed with methanol (10 mL), and the filtrate was concentrated under reduced pressure. Separation by column chromatography (dichloromethane:methanol = 50:1) gave compound **28-6A** (developing solvent: DCM/MeOH = 15:1, Rf = 0.5), MS m/z = 218.1 [M+H]⁺, and compound **28-6B** (developing solvent: DCM/MeOH = 15:1, Rf = 0.3), MS m/z = 218.1 [M+H]⁺.

Compound **28-6A:** ¹H NMR (400 MHz, DMSO-*d₆*) δ ppm 5.39 - 5.21 (m, 1 H), 4.37 (d, *J* = 8.0 Hz, 1H), 3.58 (s, 3H), 3.39 - 3.35 (m, 1H), 3.29 - 3.21 (m, 2H), 2.99 - 2.86 (m, 1H), 2.82 - 2.77 (m, 1H), 2.61 - 2.52 (m, 1H), 2.09 - 1.91 (m, 3H), 1.84 - 1.78 (m, 1H), 1.68 - 1.60 (m, 1H).

Compound **28-6B:** ¹H NMR (400 MHz, DMSO-*d₆*) δ ppm 5.27 - 5.10 (m, 1H), 4.64 (d, *J* = 8.0 Hz, 1H), 3.59 (s, 3H), 3.58 - 3.54 (m, 1H), 3.51 - 3.46 (m, 1H), 3.25 - 3.18 (m, 1H), 3.09 - 2.95 (m, 2H), 2.69 - 2.60 (m, 1H), 2.02 - 1.70 (m, 4H), 1.59 - 1.48 (m, 1H).

### Step 6

Compound **28-6A** (200.00 mg, 920.65 mmol) was weighed. Acetonitrile (2 mL) and water (0.014 mL) were added. The mixture was cooled to 0°C, and periodic acid (662.87 mg, 2.30 mmol) and chromium trioxide (27.62 mg, 1.84 mmol) were added. The reaction was allowed to proceed at room temperature for 4 hours. The reaction mixture was filtered. The filter cake was washed with 10 mL of dichloromethane, and the filtrate was concentrated to obtain compound **28-7A** (MS m/z = 232.1 [M+H]⁺).

Referring to step 6, compound **28-6B** was used as a starting material in place of compound **28-6A** to obtain compound **28-7B.** MS m/z = 232.1 [M+H]⁺.

### Step 7

Compound **28-7A** (120 mg, 518.99 µmol) was dissolved in N,N-dimethylformamide (3 mL). N-methyl-3,4-dimethylbenzylamine (92.94 mg, 622.79 µmol), 2-(7-azabenzotriazole)-N,N,N',N'-tetramethyluronium hexafluorophosphate (394.67 mg, 1.04 mmol), and N,N-diisopropylethylamine (201.22 mg, 1.56 mmol) were added and reacted at 25°C for 4 hours. The mixture was extracted with ethyl acetate (20 mL x 2). The extracted organic phases were combined and washed with saturated brine (20 mL), dried over anhydrous sodium sulfate, filtered, and concentrated under reduced pressure. Compound **28-8A** was obtained by column chromatography (petroleum ether:ethyl acetate = 10:1 to 1:1). MS m/z = 395.2 [M+H]⁺.

Referring to step 7, compound **28-7B** was used as a starting material in place of compound **28-7A** to obtain compound **28-8B.** MS m/z = 395.2 [M+H]⁺.

### Step 8

Compound **28-8A** (20.00 mg, 55.18 µmol) was dissolved in methanol (0.5 mL), and sodium borohydride (4.18 mg, 110.36 µmol) and sodium methoxide (29.81 µg, 0.55 µmol) were added. The reaction was allowed to proceed at 25°C for 4 hours. The mixture was extracted with ethyl acetate (20 mL x 2). The extracted organic phases were combined and washed with saturated brine (20 mL), dried over anhydrous sodium sulfate, filtered, and concentrated under reduced pressure to obtain compound **28-9A.** MS m/z = 367.3 [M+H]⁺.

Referring to step 8, compound **28-8B** was used as a starting material in place of compound **28-8A** to obtain compound **28-9B.** MS m/z = 367.3 [M+H]⁺.

### Step 9

Compound **28-9A** (20 mg, 54.58 µmol) was dissolved in anhydrous tetrahydrofuran (0.5 mL), and cooled to 0°C. Sodium hydride (2.62 mg, 109.16 µmol, 60% purity) was added under nitrogen. The mixture was allowed to react at room temperature for 1 hour. The reaction was quenched by the addition of 5 mL of saturated ammonium chloride solution and extracted with ethyl acetate (10 mL x 2). The extracted organic phases were combined and washed with saturated brine (10 mL), dried over anhydrous sodium sulfate, filtered, and concentrated under reduced pressure. Purification by preparative thin-layer chromatography (dichloromethane:methanol = 20:1) afforded compound **28-10A.** MS m/z = 1166.5 [M+H]⁺.

Referring to step 9, compound **28-9B** was used as a starting material in place of compound **28-9A** to obtain compound **28-10B.** MS m/z = 1166.5 [M+H]⁺.

### Step 10

Compound **28-10A** (20 mg, 17.63 µmol) was added to dichloromethane (1 mL) and trifluoroacetic acid (0.1 mL) and the reaction was stirred at 20°C for 2 hours. The reaction solution was concentrated under reduced pressure to obtain a crude product. The crude product was separated by preparative HPLC (Chromatographic column: Xtimate C18 150*40mm*5µm; mobile phase: [water (0.05% formic acid)-acetonitrile]; gradient: (acetonitrile): 10%-40%) and lyophilized to obtain the formate salt of compound **28A.** MS m/z = 676.5 [M+H]⁺. ¹H NMR (400 MHz, CD₃OD) δ ppm 8.52 (brs, 1H), 6.91 (d, *J =* 8.0 Hz, 1H), 5.49 - 5.27 (m, 1H), 5.17 (dd, *J =* 11.6, 4.5 Hz, 1H), 4.82 - 4.79 (m, 1H), 4.69 (d, *J =* 16.0 Hz, 1H), 4.39 (d, *J = 12.0* Hz, 1H), 4.30 - 4.19 (m, 2H), 3.91 (s, 2H), 3.71 - 3.64 (m, 1H), 3.59 - 3.42 (m, 2H), 3.29 - 3.12 (m, 3H), 3.04 - 2.85 (m, 2H), 2.73 (s, 3H), 2.44 - 2.29 (m, 2H), 2.23 - 2.15 (m, 2H), 2.07 - 1.98 (m, 6H), 1.97 - 1.75 (m, 3H).

Compound **28-10B** (20 mg, 17.63 µmol) was added to dichloromethane (1 mL) and trifluoroacetic acid (0.1 mL) and the reaction was stirred at 20°C for 2 hours. The reaction solution was concentrated under reduced pressure to obtain a crude product. The crude product was separated by preparative HPLC (Chromatographic column: Xtimate C18 150*40mm*5µm; mobile phase: [water (0.05% hydrochloric acid)-acetonitrile]; gradient: (acetonitrile): 10%-40%) and lyophilized to obtain the hydrochloride salt of compound **28B.** MS m/z = 676.5 [M+H]⁺. ¹H NMR (400 MHz, CD₃OD) δ ppm 6.90 (d, *J =* 8.0 Hz, 1H), 5.42 - 5.25 (m, 1H), 5.18 - 5.11 (m, 1H), 4.79 (d, *J =* 13.5 Hz, 1H), 4.64 (d, *J =* 16.0 Hz, 1H), 4.23 (d, *J =* 8.0 Hz, 1H), 4.20 - 4.11 (m, 2H), 3.75 (t, *J =* 5.6 Hz, 1H), 3.52 (d, *J =* 12.0 Hz, 3H), 3.42 (d, *J =* 12.0 Hz, 1H), 3.26 - 3.12 (m, 2H), 3.10 - 2.99 (m, 2H), 2.88 - 2.80 (m, 1H), 2.73 (s, 3H), 2.45 - 2.35 (m, 1H), 2.24 - 2.07 (m, 2H), 2.06 - 1.96 (m, 7H), 1.87 - 1.77 (m, 2H), 1.68 (t, *J =* 8.9 Hz, 1H).

### Example 29

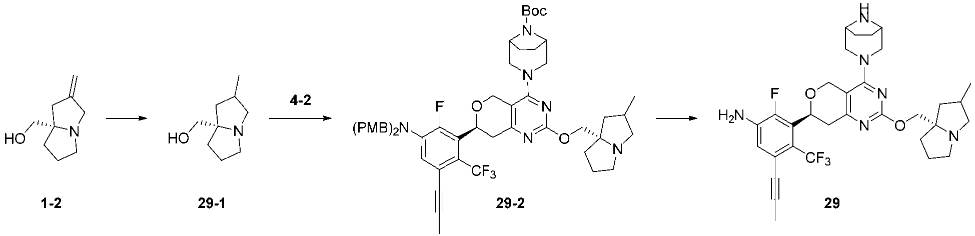

### Step 1

To a solution of compound **1-2** (50.00 mg, 326.33 µmol) in methanol (2 mL) was added palladium/carbon (16.64 mg, 10% purity), and the mixture was reacted under a hydrogen atmosphere (15 psi) at 20°C for 16 h. The reaction mixture was filtered. The filtrate was concentrated to dryness by nitrogen blowing to obtain compound **29-1.** MS m/z = 156.15 [M+H]⁺

### Step 2

Potassium tert-butoxide (257.54 mg, 2.68 mmol) was added to a solution of compound 29-1 (52.00 mg, 334.97 µmol) in tetrahydrofuran (4 ml) at 0°C and stirred for 1 hour. Compound 4-2 (347.28 mg, 401.97 µmol) was added to the above mixture, and the resulting mixture was stirred at 25°C for 1 hour. 5 mL of saturated aqueous ammonium chloride was added to quench the reaction. The mixture was extracted with ethyl acetate (2 mL*2), dried over anhydrous sodium sulfate, and filtered. The filtrate was concentrated under reduced pressure, and separated by column chromatography (petroleum ether:ethyl acetate = 3:1) to obtain compound **29-2.** MS m/z **=** 955.2 [M+H]⁺

### Step 3

Compound **29-2** (0.11 g, 115.17 µmol) was dissolved in trifluoroacetic acid (1 mL). The mixture was stirred at 25°C for 1 hour. The reaction mixture was concentrated under reduced pressure to obtain a crude product. The crude product was separated by preparative high-performance liquid chromatography (Chromatographic column: Waters Xbridge, 250*19 mm, 5 µm; mobile phase: [water (0.1% formic acid)-acetonitrile]; gradient: (acetonitrile): 45%-75%) to obtain the formate salt of compound **29.** MS m/z = 615.2 [M+H]⁺. ¹H NMR (400 MHz, CD₃OD) δ ppm 8.53 (s, 0.71H), 6.93 (d, *J* = 12.0 Hz, 1H), 5.18 - 5.15 (m, 1H), 4.80 - 4.73 (m, 3H), 4.62 - 4.60 (m, 1H), 4.49 - 4.43 (m, 3H), 3.78 - 3.72 (m, 1H), 3.52 - 3.36 (m, 2H), 3.30 - 3.17 (m, 2H), 3.02 - 2.99 (m, 1H), 2.91- 2.87 (m, 2H), 2.83 - 2.75 (m, 2H), 2.62 - 2.42 (m, 1H), 2.35 - 2.26 (m, 1H), 2.21 - 2.18 (m, 3H), 2.10 - 2.06 (m, 2H), 2.04 (m, 5H), 1.90 - 1.70 (m, 1H), 1.20 - 1.15 (m, 3H).

### Example 30

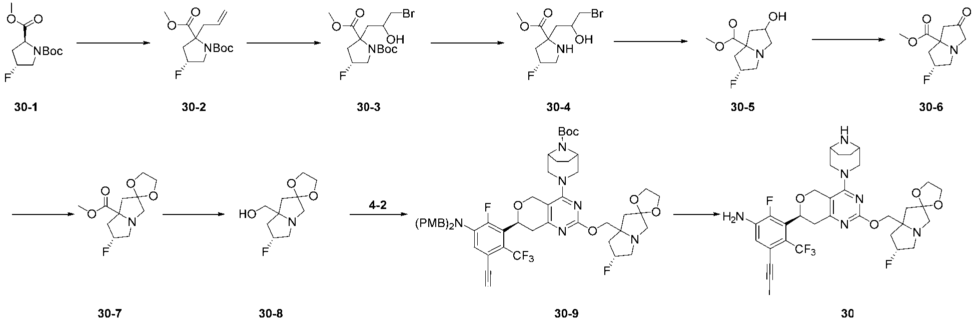

### Step 1

Compound **30-1** (10 g, 40.44 mmol) was dissolved in tetrahydrofuran (100 mL), and cooled to -78°C. Lithium hexamethyldisilazide (1 M, 52.58 mmol, 52.58 mL) was then added and stirred at -78°C for 0.5 h. Allyl bromide (5.87 g, 48.53 mmol) was added to the reaction mixture, which was then stirred at room temperature for 2 hours. Saturated ammonium chloride solution (100 mL) was added, and the mixture was extracted with ethyl acetate (50 mL x 3). The organic phases were combined, concentrated under reduced pressure, and separated by column chromatography (petroleum ether:ethyl acetate = 10:1) to obtain compound **30-2.** MS m/z = 288.2 [M+H]⁺

### Step 2

Compound **30-2** (200 mg, 696.07 µmol) was dissolved in tetrahydrofuran (1 mL). Water (1 mL) was added and stirred thoroughly. N-bromosuccinimide (123.89 mg, 696.07 µmol) was added in three batches. Saturated sodium bicarbonate aqueous solution (3 mL) was added, and the mixture was extracted with ethyl acetate (5 mL x 3). The mixture was dried over sodium sulfate, and filtered. The filtrate was concentrated under reduced pressure, and separated by column chromatography (petroleum ether:ethyl acetate = 4:1) to obtain compound **30-3.** MS m/z = 384.2 [M+H]⁺

### Step 3

Compound **30-3** (400 mg, 1.04 mmol) was dissolved in dichloromethane (3 mL), and cooled to 0°C. Trifluoroacetic acid (1 mL) was added, and the mixture was reacted at room temperature for 2 hours. The reaction solution was concentrated under reduced pressure to obtain compound **30-4.** MS m/z = 284.2 [M+H]⁺

### Step 4

Compound **30-4** (295.78 mg, 1.04 mmol) was dissolved in acetonitrile (4 mL). Potassium carbonate (719.38 mg, 5.21 mmol) was added and the mixture was heated to 80°C and stirred for 3 hours. After cooling to room temperature, the mixture was filtered, concentrated, and separated by column chromatography (dichloromethane:methanol = 10:1) to obtain compound **30-5.** MS m/z = 204.3 [M+H]⁺

### Step 5

Compound **30-5** (500 mg, 2.46 mmol) was dissolved in chloromethane. Dess-Martin periodinane (3.13 g, 7.38 mmol) was added at 0°C and stirred at room temperature for 2 hours. Saturated aqueous sodium bicarbonate (10 mL) was added to quench the reaction, and the mixture was filtered and extracted with ethyl acetate (5 mL x 3). The mixture was dried over anhydrous sodium sulfate, concentrated under reduced pressure, and separated by column chromatography (dichloromethane:methanol = 10:1) to give compound **30-6.** MS m/z = 202.1 [M+H]⁺

### Step 6

Compound **30-6** (240 mg, 1.19 mmol) was dissolved in toluene (5 mL), and ethylene glycol (0.37 g, 5.96 mmol) was added. p-Toluenesulfonic acid monohydrate (22.69 mg, 0.12 mmol) was added, heated to 90°C and stirred for 16 hours. After cooling to room temperature, saturated sodium bicarbonate aqueous solution (5 mL) was added. The mixture was extracted with ethyl acetate (5 mL * 3). The organic phases were combined, dried over anhydrous sodium sulfate, filtered, and concentrated under reduced pressure to obtain compound **30-**7. MS m/z = 246.1 [M+H]⁺.

### Step 7

Compound **30-7** (100 mg, 0.41 mmol) was dissolved in tetrahydrofuran (5 mL), and cooled to 0°C. Lithium aluminum tetrahydride (15.48 mg, 0.41 mmol) was added, and the mixture was slowly returned to room temperature and stirred for 1 hour. Water (0.02 mL), 15% aqueous sodium hydroxide solution (0.02 mL), and water (0.06 mL) were added sequentially to quench the reaction. The mixture was stirred at room temperature for 0.5 hour, filtered, and concentrated under reduced pressure to obtain compound **30-8.** MS m/z = 218.2 [M+H]⁺.

### Step 8

Compound 30-8 (60 mg, 0.28 mmol) was dissolved in anhydrous tetrahydrofuran (1 mL), and cooled to 0°C. Sodium hydride (55.23 mg, 1.38 mmol, 60% purity) was added. After stirring at 0°C for half an hour, compound **4-2** (264.70 mg, 0.30 mmol) was added and stirred at room temperature for 2 hours. The reaction was quenched with saturated aqueous ammonium chloride (5 mL) and extracted with ethyl acetate (5 mL x 3). The organic phases were combined, dried over anhydrous sodium sulfate, and filtered. The filtrate was concentrated under reduced pressure, and separated by column chromatography (petroleum ether:ethyl acetate = 1:1) to obtain compound **30-9.** MS m/z = 1017.7 [M+H]⁺.

### Step 9

Compound **30-9** (0.10 g, 0.098 mmol) was added to 10 mL of a mixed solvent (trifluoroacetic acid:dichloromethane = 1:3) at 0°C and stirred at room temperature for 4 hours. The reaction solution was concentrated under reduced pressure to obtain a crude product. The crude product was separated by preparative HPLC (Waters Xbridge, 250 x 19 mm, 5 µm; mobile phase: [water (0.1% ammonia)-acetonitrile]; gradient: (acetonitrile): 55%-70%) and concentrated under reduced pressure to obtain compound **30.** MS m/z = 677.5 [M+H]⁺. ¹H NMR (400 MHz, CD₃OD) *δ* ppm 6.96 (d, *J =* 8.0 Hz, 1H), 5.42 - 5.28 (m, 1H), 5.20 - 5.17 (m, 1H), 4.85 - 4.47 (m, 2H), 4.62 - 4.60 (m, 1H), 4.46 - 4.41 (m, 1H), 4.35 - 4.27 (m, 2H), 4.03 - 3.92 (m, 4H), 3.55 - 3.38 (m, 3H), 3.31 - 3.18 (m, 3H), 3.08 - 2.99 (m, 1H), 2.92 - 2.84 (m, 3H), 2.59 - 2.49 (M, 1H), 2.43 - 2.35 (m, 1H), 2.31 - 2.16 (m, 2H), 2.12 - 2.01 (m, 7H).

### Example 31

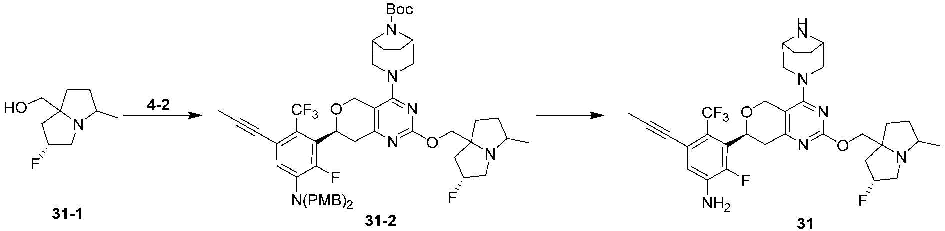

### Step 1

Compound **31-1** (20 mg, 57.87 µmol) was dissolved in anhydrous tetrahydrofuran (3 mL), cooled to 0°C, and sodium hydride (4.63 mg, 115.75 µmol, 60% purity) was added. The mixture was stirred at 0°C for half an hour. Compound **4-2** (50 mg, 57.87 µmol) was added, and the mixture was stirred at room temperature for 1 hour. The reaction was quenched by adding a saturated aqueous solution of ammonium chloride (10 mL). The mixture was extracted with ethyl acetate (20 mL x 3). The extracted organic phases were combined and washed with saturated brine (20 mL), dried over anhydrous sodium sulfate, and filtered. The filtrate was concentrated under reduced pressure, and separated by column chromatography (petroleum ether:ethyl acetate = 2:1) to obtain compound 31-2. MS m/z = 973.7 [M+H]⁺.

### Step 2

Compound **31-2** (40 mg, 41.11 µmol) was added to trifluoroacetic acid (4 mL) and stirred at room temperature for 1 hour. The reaction solution was concentrated under reduced pressure to obtain a crude product. The crude product was separated by preparative high performance liquid chromatography (Chromatographic column: Waters Xbridge, 250 * 19 mm, 5 µm; mobile phase: [water (0.1% ammonia)-acetonitrile]; gradient: (acetonitrile): 55%-70%). After drying, compound **31** was obtained. MS m/z = 633.5 [M+H]⁺. ¹H NMR (400 MHz, CD₃OD) δ ppm 6.91 (d, *J =* 8.0 Hz, 1H), 5.47 - 5.45 (m, 0.5H), 5.34 - 5.31 (m, 0.5H), 5.17 (dd, *J =* 8.0 Hz, 4.0 Hz, 1H), 4.80 (s, 1H), 4.67 *(d, J=* 12.0 Hz, 1H), 4.57 (s, 1H), 4.25 (d, *J* = 12.0 Hz, 2H), 4.12 (d, *J =* 12.0 Hz, 1H), 3.89 - 3.85 (m, 2H), 3.69-3.62 (m, 1H), 3.58 - 3.53 (m, 1H), 3.47 - 3.37 (m, 1H), 3.21 - 3.15 (m, 1H), 3.07 - 2.83 (m, 3H), 2.47 (dd, *J =* 20.0 Hz, 16.0 Hz, 1H), 2.25 - 2.15 (m, 2H), 2.08 - 1.93 (m, 7H), 1.88 - 1.70 (m, 3H), 1.16 (d, *J =* 8.0 Hz, 3H).

### Example 32

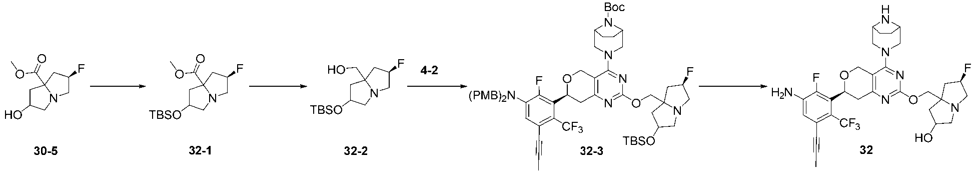

### Step 1

Compound **30-5** (110.00 mg, 0.54 mmol) was weighed. Dichloromethane (5 mL) was added. TertButyldimethylsilyl chloride (162.76 mg, 1.08 mmol) and imidazole (110.16 mg, 1.62 mmol) were added, and the mixture was reacted at room temperature for 16 hours. Water (10 mL) was added and the mixture was extracted with dichloromethane (10 mL * 3). The organic phase was concentrated under reduced pressure and separated by column chromatography (petroleum ether:ethyl acetate = 3:1) to obtain compound **32-1,** MS m/z = 318.2 [M+H]⁺.

### Step 2

Compound **32-1** (85 mg, 0.27 mmol) was weighed and anhydrous tetrahydrofuran (3 mL) was added under nitrogen. The mixture was cooled to 0°C, and lithium aluminum tetrahydride (0.54 mmol, 0.22 ml, 2.5 M) was added. The mixture was allowed to react at room temperature for 15 minutes. 0.2 mL of water was added to the reaction solution at 0°C, and the reaction was quenched by adding 0.2 mL of 15% sodium hydroxide solution. The mixture was stirred for 10 minutes, and filtered. The filter cake was washed with 5 mL of tetrahydrofuran. The filtrate was concentrated to obtain compound **32-2.** MS m/z = 290.2 [M+H]⁺

### Step 3

Compound **32-2** (64 mg, 0.22 mmol) was weighed and anhydrous tetrahydrofuran (5 mL) was added. Sodium hydride (15.91 mg, 0.66 mmol, 60% purity) was added under ice bath, and the mixture was stirred at room temperature for 30 minutes. Compound **4-2** (195.41 mg, 0.22 mmol) was added and reacted at room temperature for 1 hour. Water (5 mL) was added to quench the reaction, and the mixture was extracted with ethyl acetate (5 mL * 3). The organic phase was concentrated under reduced pressure and separated by column chromatography (dichloromethane:methanol = 20:1) to obtain compound **32-3.** MS m/z = 1089.5 [M+H]⁺.

### Step 4

Compound **32-3** (50 mg, 0.05 mmol) was weighed. Dichloromethane (2 mL) and trifluoroacetic acid (1 mL) were added, and the mixture was stirred at room temperature for 0.5 hours. The reaction mixture was concentrated under reduced pressure to obtain a crude product, which was then separated by preparative HPLC (Chromatographic column: Waters Xbridge, 250 x 19 mm, 5 µm; mobile phase: [water (0.1% hydrochloric acid)-acetonitrile]; gradient: (acetonitrile): 10%-40%) to afford the hydrochloride salt of compound **32.** MS m/z = 635.3 [M+H]⁺. ¹H NMR (400 MHz, CD₃OD) *δ* ppm 6.94 (d, *J =* 8.0 Hz, 1H), 5.57 (d, *J =* 48.0 Hz, 1H), 5.23 (d, *J =* 12.0 Hz, 1H), 5.0 - 5.04 (m, 1H), 4.99 - 4.90 (m, 4H), 4.76 (t, *J =* 4.0 Hz, 1H), 4.23 (d, *J =* 28.0 Hz, 2H), 4.12 - 3.95 (m, 3H), 3.73 - 3.51 (m, 4H), 3.35 - 3.43 (m, 1H), 3.07 - 3.03 (m, 1H), 2.85 - 2.76 (m, 1H), 2.67 - 2.41 (m, 3H), 2.22 - 2.05 (m, 4H), 2.02 (s, 3H).

### Example 33

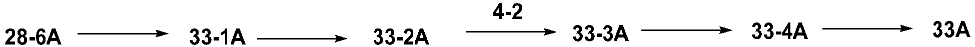

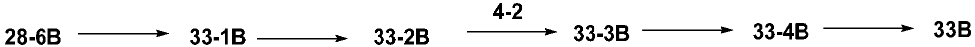

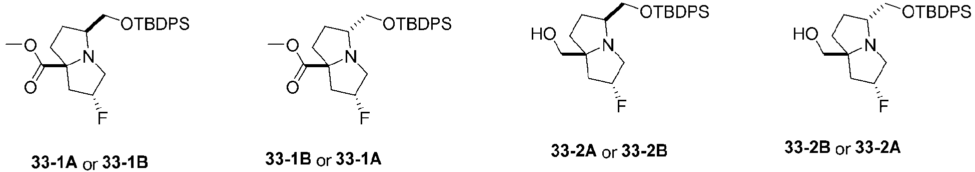

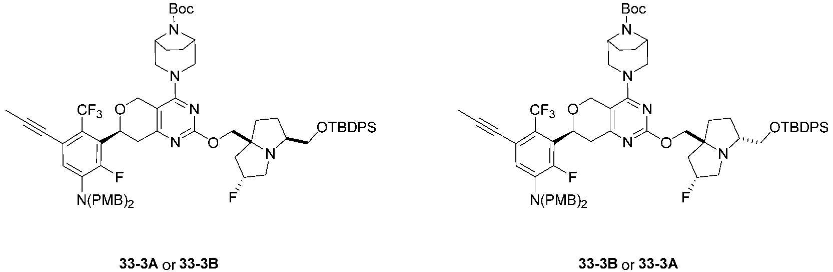

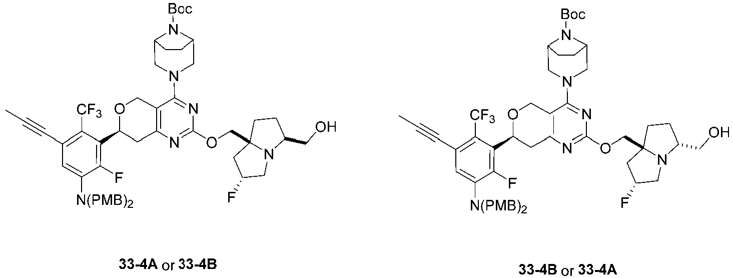

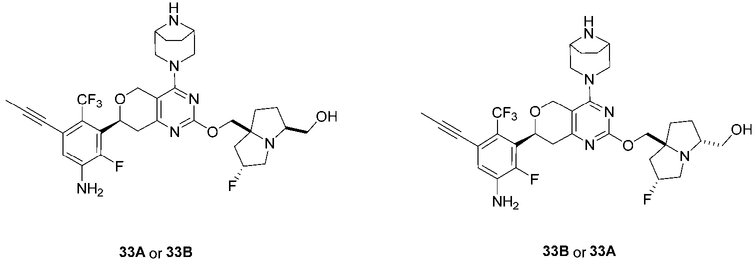

### Step 1

At 0°C, tert-butyldiphenylsilyl chloride (1.50 g, 4.83 mmol) was added dropwise to a solution of compound **28-6A** (700 mg, 3.22 mmol) and imidazole (658.09 mg, 9.67 mmol) in dichloromethane (20 mL). The mixture was warmed to 20°C and stirred for 4 hours. The reaction was quenched by adding 30 mL of saturated aqueous ammonium chloride. The mixture was extracted with dichloromethane (15 mL x 2), dried over anhydrous sodium sulfate, and filtered. The filtrate was concentrated under reduced pressure, and separated by column chromatography (petroleum ether:ethyl acetate = 5:1) to afford compound **33-1A.** MS m/z = 456.3 [M+H]⁺.

Referring to step 1, compound **28-6B** was used as a starting material in place of compound **28-6A** to obtain compound **33-1B.** MS m/z = 456.3 [M+H]⁺.

### Step 2

Lithium aluminum tetrahydride (37.58 mg, 0.99 mmol) was slowly added to a solution of compound **33-1A** (300 mg, 0.66 mmol) in tetrahydrofuran (5 mL) at 0°C. The reaction was stirred at 0°C for 0.5 hours. The reaction was quenched by the addition of 100 mg of sodium sulfate decahydrate. The quenched reaction solution was filtered, and the filtrate was concentrated under reduced pressure and separated by column chromatography (petroleum ether:ethyl acetate = 3:1) to obtain compound **33-2A.** MS m/z = 428.3 [M+H]⁺.

Referring to step 2, compound **33-1B** was used as a starting material in place of compound **33-1A** to obtain compound **33-2B.** MS m/z = 428.3 [M+H]⁺.

### Step 3

Sodium hydride (46.84 mg, 1.17 mmol, 60% purity) was slowly added to a solution of compound 33-**2A** (100.00 mg, 0.23 mmol) in tetrahydrofuran (5 mL) at 0°C. The reaction was stirred at 0°C for 0.5 h. A solution of compound **4-2** (205.49 mg, 0.24 mmol) in tetrahydrofuran (1 mL) was then added to the system, and the mixture was warmed to 25°C and stirred for additional 1 h. The reaction was quenched by adding saturated aqueous ammonium chloride (6 mL) and extracted with ethyl acetate (6 mL x 2). The extracted organic phases were combined and washed with 5 mL of saturated brine, dried over anhydrous sodium sulfate, filtered, and concentrated under reduced pressure. Separation by column chromatography (petroleum ether:ethyl acetate = 3:1) afforded compound **33-3A.** MS m/z = 1227.5 [M+H]⁺.

Referring to step 3, compound **33-2B** was used as a starting material in place of compound **33-2A** to obtain compound **33-3B.** MS m/z = 1227.5 [M+H]⁺.

### Step 4

To a solution of compound **33-3A** (220 mg, 0.18 mmol) in tetrahydrofuran (4 mL) was added a solution of tetrabutylammonium fluoride in tetrahydrofuran (0.54 mL, 0.54 mmol, 1 M) at 20°C. The reaction was stirred at 20°C for 4 hours. To the system ethyl acetate (20 mL) was added and the mixture was washed with water (10 mL x 3). The organic phase was dried over anhydrous sodium sulfate, filtered, and concentrated under reduced pressure. Separation by column chromatography (dichloromethane:methanol = 20:1) afforded compound **33-4A.** MS m/z = 989.7 [M+H]⁺.

Referring to step 4, compound **33-3B** was used as a starting material in place of compound **33-3A** to obtain compound **33-4B.** MS m/z = 989.7 [M+H]⁺.

### Step 5

Compound **33-4A** (100 mg, 0.10 mmol) was added to trifluoroacetic acid (3 mL) and stirred at 25°C for 1 hour. The reaction solution was concentrated under reduced pressure to obtain a crude product. The crude product was separated by preparative HPLC (Chromatographic column: Waters Xbridge, 250 x 19 mm, 5 µm; mobile phase: [water (0.1% ammonia)-acetonitrile]; (acetonitrile): 40%-70%) and dried to obtain compound **33A.** MS m/z = 649.5 [M+H]⁺. ¹H NMR (400 MHz, CD₃OD) δ ppm 6.90 (d, *J =* 8.0 Hz, 1H), 5.36 (dt, *J =* 56.0, 4.0 Hz, 1H), 5.14 (dd, *J =* 12.0, 4.0 Hz, 1H), 4.81 - 4.77 (m, 1H), 4.66 - 4.62 (m, 1H), 4.23 - 4.13 (m, 2H), 4.11 - 4.04 (m, 1H), 3.59 - 3.40 (m, 7H), 3.25 - 3.16 (m, 1H), 3.08 - 2.92 (m, 2H), 2.91 - 2.80 (m, 2H), 2.49 - 2.38 (m, 1H), 2.24 - 2.16 (m, 1H), 2.09 - 1.99 (m, 5H), 1.96 - 1.77 (m, 4H), 1.74 - 1.63 (m, 2H).

Compound **33-4B** (140 mg, 0.14 mmol) was added to trifluoroacetic acid (5 mL) and stirred at 25°C for 1 hour. The reaction solution was concentrated under reduced pressure to obtain a crude product. The crude product was separated by preparative HPLC (Chromatographic column: Waters Xbridge, 250 x 19 mm, 5 µm; mobile phase: [water (0.1% ammonia)-acetonitrile]; (acetonitrile): 40%-70%) and dried to obtain compound 33B. MS m/z = 649.5 [M+H]⁺. ¹H NMR (400 MHz, CD₃OD) δ ppm 6.90 (d, *J =* 8.0 Hz, 1H), 5.26 (dt, *J* = 52.0, 4.0 Hz, 1H), 5.14 (dd, *J =* 8.0, 4.0 Hz, 1H), 4.82 - 4.77 (m, 1H), 4.68 - 4.61 (m, 1H), 4.35 - 4.29 (m, 1H), 4.22 - 4.15 (m, 2H), 3.84 - 3.77 (m, 1H), 3.73 - 3.67 (m, 1H), 3.57 - 3.49 (m, 3H), 3.46 - 3.34 (m, 2H), 3.25 - 3.02 (m, 4H), 2.89 - 2.81 (m, 1H), 2.49 - 2.39 (m, 1H), 2.08 - 1.92 (m, 6H), 1.91 - 1.79 (m, 4H), 1.77 - 1.64 (m, 2H).

### Example 34

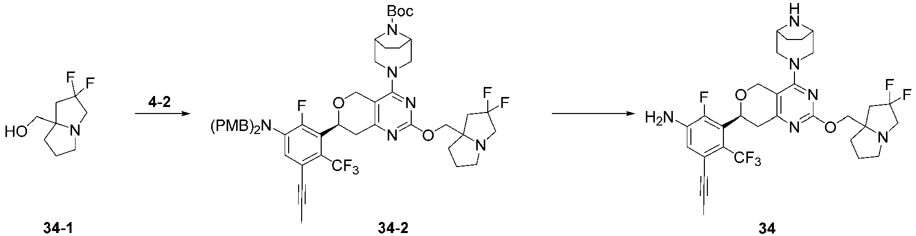

### Step 1

Compound **34-1** (40 mg, 0.23 mmol) was dissolved in anhydrous tetrahydrofuran (5 mL), and cooled to 0°C. Sodium hydride (9.03 mg, 0.23 mmol, 60% purity) was added. After stirring at 0°C for half an hour, compound **4-2** (195.03 mg, 0.23 mmol) was added and stirred at room temperature for 1 hour. The reaction was quenched by adding saturated aqueous ammonium chloride solution (10 mL), and extracted with ethyl acetate (20 mL * 3). The extracted organic phases were combined and washed with saturated brine (20 mL), dried over anhydrous sodium sulfate, and filtered. The filtrate was concentrated under reduced pressure, and separated by column chromatography (petroleum ether: ethyl acetate = 1:1) to obtain compound **34-2.** MS m/z = 977.5 [M+H]⁺.

### Step 4

Compound **34-2** (100 mg, 0.10 mmol) was added to 10 mL of a mixed solvent (trifluoroacetic acid:dichloromethane = 1:3) at 0°C and stirred at room temperature for 4 hours. The reaction solution was concentrated under reduced pressure to obtain a crude product. The crude product was separated by preparative HPLC (Waters Xbridge, 250 x 19 mm, 5 µm; mobile phase: [water (0.1% ammonium bicarbonate)-acetonitrile]; gradient: (acetonitrile): 50%-70%) and concentrated under reduced pressure to obtain compound **34.** MS m/z = 637.5 [M+H]⁺. ¹H NMR (400 MHz, CD₃OD) δ ppm 6.93 (d, *J =* 8.0 Hz, 1H), 5.20 - 5.16 (m, 1H), 4.85 - 4.82 (m, 1H), 4.72 - 4.69 (m, 1H), 4.24 - 4.11 (m, 3H), 3.93 - 3.88 (m, 2H), 3.71 - 3.66 (m, 1H), 3.59 - 3.54 (m, 1H), 3.47 - 3.38 (m, 1H), 3.30 - 3.31 (m, 4H), 2.92 - 2.83 (m, 2H), 2.62 - 2.50 (m, 1H), 2.37 - 2.27 (m, 1H), 2.22 - 2.12 (m, 2H), 2.05 - 1.98 (m, 6H), 1.94 - 1.85 (m, 3H).

### Example 35

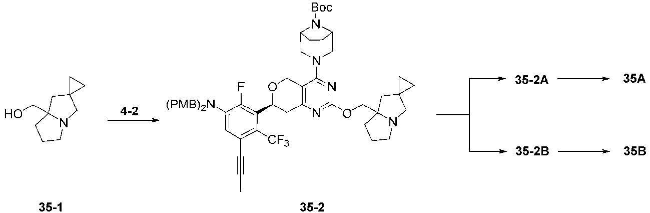

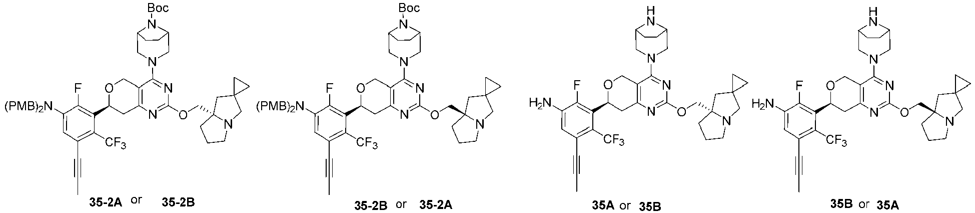

### Step 1

Compound **35-1** (48.40 mg, 289.37 µmol) was dissolved in tetrahydrofuran (5 mL). Sodium hydride (41.66 mg, 1.74 mmol, 60% purity) was then added. Under nitrogen, the reaction was stirred at 25°C for 0.5 hours. A solution of compound **4-2** (250 mg, 289.37 µmol) in tetrahydrofuran (2 mL) was then added. After the addition, the reaction was stirred at 25°C for 2 hours. The reaction solution was dissolved in 10 mL of ethyl acetate, washed with 100 mL of saturated brine, dried over anhydrous sodium sulfate, and filtered. The filtrate was concentrated under reduced pressure. The crude product was purified by column chromatography (dichloromethane:methanol = 30:1) to obtain compound **35-2.** MS m/z = 967.8 [M+H]⁺. Next, preparative supercritical liquid chromatography (SFC) separation (Chromatographic column: DAICEL CHIRALPAK^{®}IA, 250*40mm 10 µm; mobile phase: A: supercritical carbon dioxide, B: ethanol (0.2% 7 Mol / L ammonia in methanol); B%: 55%) was performed to obtain compounds **35-2A** and **35-2B.** Analytical SFC method (Chromatographic column: DAICEL CHIRALPAK^{®}IA (100*3mm 3µm); mobile phase: [A: supercritical carbon dioxide B: ethanol (0.1% diethylamine)]; gradient B%: 40%, 8 min), compound **35-2A,** Rt = 1.847 minutes, ee value 100%, MS m / z = 967.8 [M+H]⁺; compound **35-2B,** Rt = 2.858 minutes, ee value 97.95%, MS m/z = 967.8 [M+H]⁺.

### Step 2

Compound **35-2A** (70 mg, 72.38 µmol) was dissolved in dichloromethane (1 mL), and trifluoroacetic acid (1 mL) was added. The reaction mixture was stirred at 25°C under nitrogen for 2 hours. The reaction solution was concentrated under reduced pressure to obtain a crude product. The crude product was separated by preparative HPLC (Chromatographic column: Waters Xbridge, 250*19 mm, 5 µm; mobile phase: [water (0.1% hydrochloric acid)-acetonitrile]; gradient: (acetonitrile): 10%-40%) and dried to obtain the hydrochloride salt of compound **35A.** MS m/z = 627.5 [M+H]⁺. ¹H NMR (400 MHz, CD₃OD) δ ppm 6.96 (d, *J =* 8.0 Hz, 1H), 5.26 - 5.24 (m, 1H), 4.98 - 4.86 (m, 3H), 4.83 - 4.70 (m, 2H), 4.27 - 4.21 (m, 2H), 4.05 - 3.94 (m, 2H), 3.77 - 3.60 (m, 3H), 3.51 - 3.41 (m, 2H), 3.23 - 3.20 (m, 1H), 3.08 - 3.05 (m, 1H), 2.47 - 2.09 (m, 10H), 2.04 (s, 3H), 0.94 - 0.81 (m, 4H).

Compound **35-2B** (73 mg, 75.48 µmol) was dissolved in dichloromethane (1 mL), and trifluoroacetic acid (1.49 g, 13.07 mmol) was added. The mixture was stirred at 25°C under nitrogen for 2 hours. The reaction solution was concentrated under reduced pressure to obtain a crude product. The crude product was separated by preparative HPLC (Chromatographic column: Waters Xbridge, 250*19 mm, 5 µm; mobile phase: [water (0.1% hydrochloric acid)-acetonitrile]; gradient: (acetonitrile): 10%-40%) and dried to obtain the hydrochloride salt of compound **35B.** MS m/z = 627.5 [M+H]⁺. ¹H NMR (400 MHz, CD₃OD) δ ppm 6.94 (d, *J =* 8.0 Hz, 1H), 5.24 - 5.12 (m, 1H), 4.99 - 4.96 (m, 3H), 4.73 - 4.70 (m, 2H), 4.27 - 4.19 (m, 2H), 4.05 - 3.94 (m, 2H), 3.74 - 3.54 (m, 3H), 3.48 - 3.35 (m, 2H), 3.27 - 3.24 (m, 1H), 3.08 - 3.04 (m, 1H), 2.47 - 2.12 (m, 10H), 2.02 (s, 3H), 0.93 - 0.78 (m, 4H).

### Example 36

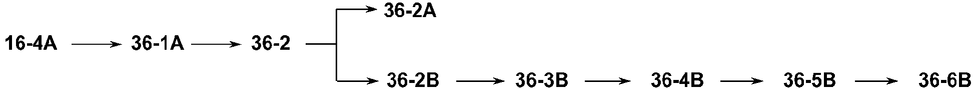

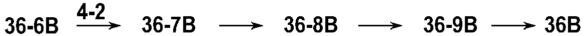

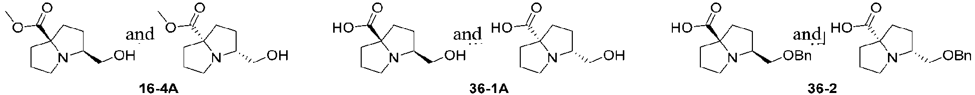

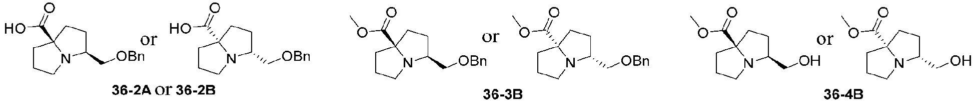

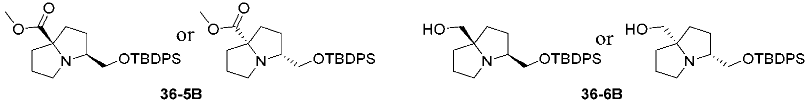

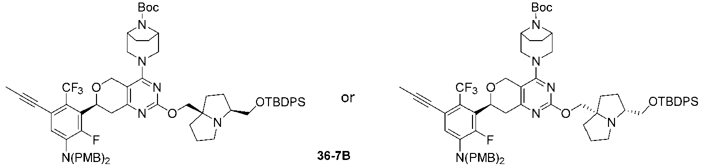

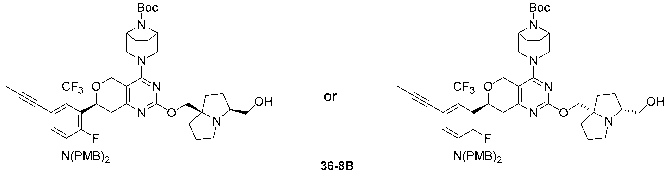

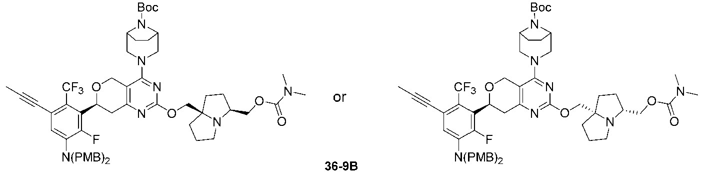

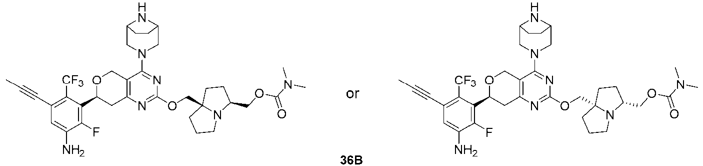

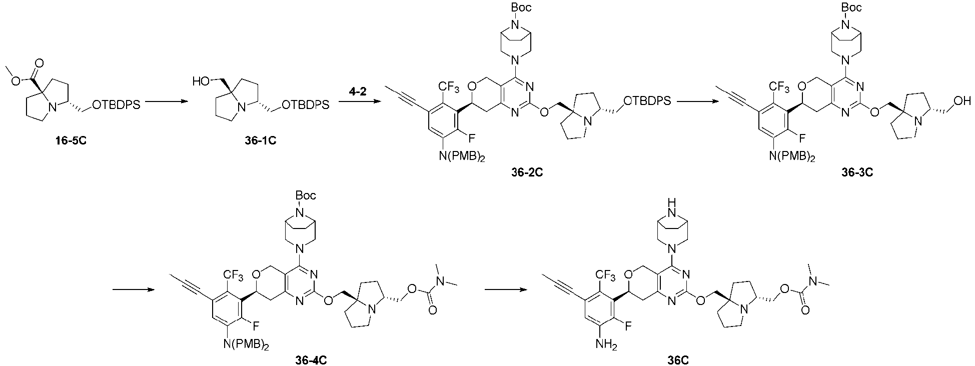

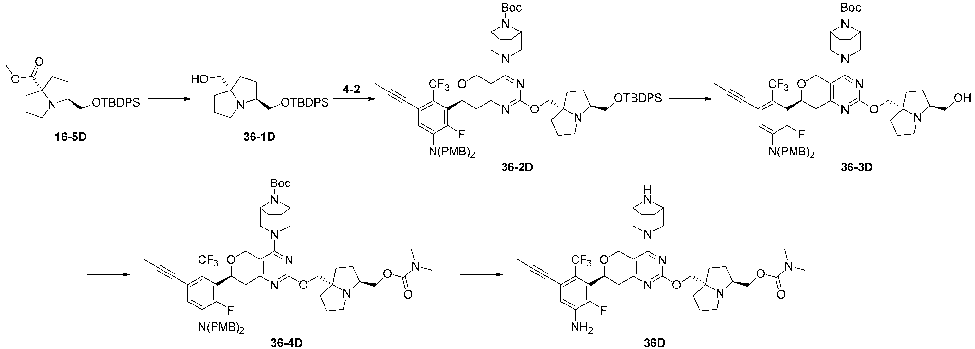

### Step 1

Compound **16-4A** (2 g, 10.04 mmol) was dissolved in tetrahydrofuran (5 mL) and water (5 mL), and lithium hydroxide (480.78 mg, 20.08 mmol) was added. The mixture was stirred at 50°C for 16 hours. The reaction solution was concentrated under reduced pressure to obtain compound **36-1A.** MS m/z = 186.0 [M+H]⁺.

### Step 2

Compound **36-1A** (300 mg, 1.12 mmol) was dissolved in N,N-dimethylformamide. Cesium carbonate (2.11 g, 6.48 mmol) and benzyl bromide (738.73 mg, 4.32 mmol) were added and stirred at 25°C for 8 hours. The reaction solution was concentrated under reduced pressure to obtain a crude product. The crude product was separated by preparative high-performance liquid chromatography (Chromatographic column: Xtimate C18 150*40 mm*5 µm;mobile phase: [water (0.05% hydrochloric acid)-acetonitrile]; (acetonitrile): 5%-40%) and concentrated under reduced pressure to obtain the formate salt of compound 36-2. Next, preparative SFC separation (Chromatographic column: UniChiral CND-5H, 20 x 250 mm; mobile phase: [supercritical carbon dioxide - methanol]; gradient: methanol: 30%) afforded compounds **36-2A** and **36-2B.** Analytical SFC method (Chromatographic column: UniChiral CND-5H, 20 x 250 mm; mobile phase: [supercritical carbon dioxide - methanol]; gradient: methanol: 30%). Compounds **36-2A,** Rt = 2.968 min, ee 99.02%, MS m/z = 276.2 [M+H]⁺; compound **36-2B,** Rt = 3.287 min, ee 99.08%, MS m/z = 276.2 [M+H]⁺.

### Step 3

Compound **36-2B** (700 mg, 2.54 mmol) was dissolved in dichloromethane (10 mL), and N,N-dimethylformamide (18.58 mg, 254.23 µmol) was added. The mixture was cooled to 0°C. Thionyl chloride (1.21 g, 10.17 mmol) was then slowly added, and the mixture was allowed to react at 25°C for 2 hours. The reaction was quenched by the addition of 3 mL of methanol, and the mixture was extracted with ethyl acetate (10 mL x 3). The extracted organic phases were combined and washed with saturated brine (10 mL), dried over anhydrous sodium sulfate, filtered, and concentrated under reduced pressure. Separation by column chromatography (petroleum ether:ethyl acetate = 5:1) afforded compound **36-3B.** MS m/z = 290.2 [M+H]⁺.

### Step 4

Compound **36-3B** (500 mg, 1.73 mmol) was dissolved in methanol (10 mL). Palladium on carbon (200 mg) was added, and the mixture was reacted at 25°C under a hydrogen atmosphere (15 psi) for 4 hours. The mixture was filtered. The filter cake was washed with methanol (20 mL), and the filtrate was concentrated under reduced pressure to obtain a crude product. Column chromatography separation (dichloromethane:methanol = 10:1) gave compound **36-4B.** MS m/z = 200.0 [M+H]⁺.

### Step 5

Compound **36-4B** (300 mg, 1.51 mmol) was dissolved in dichloromethane (10 mL). Imidazole (307.51 mg, 4.52 mmol) and tert-butyldiphenylsilyl chloride (620.77 mg, 2.26 mmol) were added. The mixture was reacted at 25°C for 4 hours. The mixture was concentrated under reduced pressure. Separation by column chromatography (petroleum ether:ethyl acetate = 10:1) afforded compound **36-5B.** MS m/z = 438.1 [M+H]⁺.

### Step 6

Compound **36-5B** (250 mg, 571.24 µmol) was dissolved in anhydrous tetrahydrofuran (5 mL) and cooled to -78°C under nitrogen. Lithium aluminum tetrahydride (43.36 mg, 1.14 mmol) was added and the reaction was stirred at -78°C for additional 1 hour. Water (2 mL) was added to quench the reaction, and the mixture was filtered. The filter cake was washed with acetonitrile (10 mL), and the filtrate was concentrated under reduced pressure to obtain a crude product. Column chromatography separation (dichloromethane:methanol = 10:1) gave compound **36-6B.** MS m/z = 410.1 [M+H]⁺.

### Step 7

Compound **36-6B** (209.48 mg, 511.39 µmol) was dissolved in tetrahydrofuran (5 mL) and cooled to 0°C. Sodium hydride (40.86 mg, 1.02 mmol, 60% purity) was added portionwise, and the mixture was warmed to 25°C with stirring for 0.5 hours. Compound **4-2** (300 mg, 340.93 µmol) was added, and the mixture was reacted at 25°C for 2 hours. The reaction was quenched by adding water (3 mL) and extracted with ethyl acetate (5 mL x 3). The organic phases were combined, washed with saturated brine (10 mL), dried over anhydrous sodium sulfate, filtered, and concentrated under reduced pressure. Separation by column chromatography (petroleum ether:ethyl acetate = 1:1) afforded compound **36-7B.** MS m/z = 1209.9 [M+H]⁺.

### Step 8

Compound **36-7B** (180 mg, 148.82 µmol) was dissolved in tetrahydrofuran (3 mL), and tetrabutylammonium fluoride (83.19 mg, 297.65 µmol) was added. The mixture was reacted at 25°C for 4 hours. The mixture was concentrated under reduced pressure. Separation by column chromatography (dichloromethane:methanol = 5:1) afforded compound **36-8B.** MS m/z = 971.7 [M+H]⁺.

### Step 9

Compound **36-8B** (100 mg, 102.98 µmol) was dissolved in tetrahydrofuran (3 mL), and N,N'-carbonyldiimidazole (50.09 mg, 308.93 µmol) was added. The mixture was stirred at 25°C for 0.5 hours. Dimethylamine (9.29 mg, 205.95 µmol) was added, and the mixture was reacted at 25°C with stirring for additional 2 hours. The mixture was concentrated under reduced pressure. Separation by column chromatography (petroleum ether:ethyl acetate = 1:1) afforded compound **36-9B.** MS m/z = 1042.7 [M+H]⁺.

### Step 10

Compound **36-9B** (80 mg, 76.76 µmol) was dissolved in dichloromethane (3 mL). Trifluoroacetic acid (1 mL) was added, and the mixture was stirred at 25°C for 2 hours. The reaction solution was concentrated under reduced pressure to obtain a crude product. The crude product was separated by preparative HPLC (Chromatographic column: Xtimate C18 150*40 mm * 5 µm; mobile phase: [water (0.05% formic acid)-acetonitrile]; (acetonitrile): 10%-40%) and dried to obtain the formate salt of compound **36B.** MS m/z = 702.6 [M+H]⁺. ¹H NMR (400 MHz, CD₃OD) δ ppm 8.52 (s, 0.38H), 6.91 (d, *J* = 8.0 Hz, 1H), 5.17 (dd, *J* = 12.0 Hz, 8.0 Hz, 1H), 4.81 (d, *J* = 12.0 Hz, 1H), 4.69 (d, *J* = 12.0 Hz, 1H), 4.23 - 4.14 (m, 4H), 4.05 (dd, *J* = 12.0 Hz, 8.0 Hz, 1H), 3.96 (s, 2H), 3.70 (d, *J* = 12.0 Hz, 1H), 3.57 (d, *J* = 16.0 Hz, 1H), 3.27 - 3.14 (m, 4H), 3.01 - 2.85 (m, 8H), 2.26 - 2.18 (m, 2H), 2.08 - 1.90 (m, 10H), 1.88 - 1.75 (m, 3H).

### Step 11

Compound **16-5C** (100 mg, 228.50 µmol) was dissolved in anhydrous tetrahydrofuran (2 mL) and cooled to -78°C under nitrogen. Lithium aluminum tetrahydride (17.34 mg, 456.00 µmol) was added and the reaction was stirred at -78°C for 1 hour. Water (2 mL) was added to quench the reaction, and the mixture was filtered. The filter cake was washed with acetonitrile (10 mL), and the filtrate was concentrated under reduced pressure to obtain a crude product. Column chromatography separation (dichloromethane:methanol = 10:1) gave compound **36-1C.** MS m/z = 410.2 [M+H]⁺.

Referring to step 11, compound **16-5D** was used as a starting material in place of compound **16-5C** to obtain compound **36-1D.** MS m/z = 410.2 [M+H]⁺.

### Step 12

Compound **36-1C** (104.74 mg, 255.70 µmol) was dissolved in tetrahydrofuran (3 mL), and cooled to 0°C. Sodium hydride (20.00 mg, 510.00 µmol, 60% purity) was added portionwise. The mixture was warmed to 25°C and stirred for 0.5 hours. Compound **4-2** (150 mg, 170.47 µmol) was added and the mixture was reacted at 25°C with stirring for additional 2 hours. Water (2 mL) was added to quench the reaction, and the mixture was extracted with ethyl acetate (5 mL * 3). The extracted organic phases were combined, washed with saturated brine (10 mL), dried over anhydrous sodium sulfate, filtered, and concentrated under reduced pressure. Column chromatography separation (petroleum ether:ethyl acetate = 1:1) gave compound **36-2C.** MS m/z = 1209.9 [M+H]⁺.

Referring to step 12, compound **36-1D** was used as a starting material in place of compound **36-1C** to obtain compound **36-2D.** MS m/z = 1209.9 [M+H]⁺.

### Step 13

Compound **36-2C** (90 mg, 74.41 µmol) was dissolved in tetrahydrofuran (3 mL). Tetrabutylammonium fluoride (41.60 mg, 148.83 µmol) was added, and the mixture was stirred at 25°C for 4 hours. The mixture was concentrated under reduced pressure and separated by column chromatography (dichloromethane:methanol =5:1) to obtain compound **36-3C.** MS m/z = 971.7 [M+H]⁺.

Referring to step 13, compound **36-2D** was used as a starting material in place of compound **36-2C** to obtain compound **36-3D.** MS m/z = 971.7 [M+H]⁺.

### Step 14

Compound **36-3C** (200 mg, 205.96 µmol) was dissolved in tetrahydrofuran (3 mL), and N,N'-carbonyldiimidazole (100.18 mg, 617.86 µmol) was added. The mixture was stirred at 25°C for 0.5 hours. Dimethylamine (18.58 mg, 411.9 µmol) was added, and the mixture was stirred at 25°C for additional 2 hours. The mixture was concentrated under reduced pressure and separated by column chromatography (petroleum ether:ethyl acetate = 1:1) to obtain compound **36-4C.** MS m/z = 1042.7 [M+H]⁺.

Referring to step 14, compound **36-3D** was used as a starting material in place of compound **36-3C** to obtain compound **36-4D.** MS m/z = 1042.7 [M+H]⁺.

### Step 15

Compound **36-4C** (80 mg, 76.76 µmol) was dissolved in dichloromethane (3 mL), and trifluoroacetic acid (1 mL) was added. The mixture was stirred at 25°C for 2 hours. The reaction solution was concentrated under reduced pressure to obtain a crude product. The crude product was separated by preparative HPLC (Chromatographic column: Xtimate C18 150*40mm*5µm; mobile phase: [water (0.05% formic acid)-acetonitrile]; (acetonitrile): 10%-40%) and concentrated under reduced pressure to obtain the formate salt of compound **36C.** MS m/z = 702.3 [M+H]⁺. ¹H NMR (400 MHz, CD₃OD) *δ* ppm 8.53 (s, 0.24 H), 6.90 (d, *J* = 12.0 Hz, 1H), 5.15 (dd, *J* = 8.0 Hz, 4.0 Hz, 1H), 4.79 (s, 1H), 4.67 (d, *J =* 16.0 Hz, 1H), 4.40 - 4.31 (m, 3H), 4.26 (t, *J* = 8.0 Hz, 1H), 4.18 (d, *J* = 12.0, 1H), 3.83 (s, 1H), 3.71 (s, 2H), 3.61 (d, *J* = 12.0 Hz, 1H), 3.48 (d, *J* = 12.0 Hz, 1H), 3.23 (t, *J* = 8.0 Hz, 2H), 3.11 (d, *J* = 16.0 Hz, 2H), 2.96 - 2.84 (m, 7H), 2.27 (dd, *J* = 12.0 Hz, 8.0 Hz, 1H), 2.18 - 2.03 (m, 4H), 2.01 (s, 3H), 1.98 - 1.90 (m, 5H), 1.87 - 1.73 (m, 2H).

Referring to step 15, compound **36-4D** was used as a starting material in place of compound **36-4C** to obtain the formate salt of compound **36D.** MS m/z = 702.3 [M+H]⁺. ¹H NMR (400 MHz, CD₃OD) *δ* ppm 8.51 (s, 0.60 H), 6.91 (d, *J* = 8.0 Hz, 1H), 5.16 (dd, *J* = 8.0 Hz, 4.0 Hz, 1H), 4.81 (s, 1H), 4.69 (d, *J* = 12.0 Hz, 1H), 4.44 - 4.40 (m, 2H), 4.37 - 4.31 (m, 2H), 4.22 (d, *J* = 12.0 Hz, 1H), 3.95 (s, 1H), 3.83 (s, 2H), 3.66 (d, *J= 12.0* Hz, 1H), 3.53 (d, *J* = 12.0 Hz, 1H), 3.34 - 3.33 (m, 1H), 3.29 - 3.15 (m, 3H), 2.97 - 2.86 (m, 7H), 2.32 - 2.20 (m, 2H), 2.16 - 2.08 (m, 3H), 2.03 - 1.90 (m, 9H), 1.87 - 1.79 (m, 1H).

### Example 37

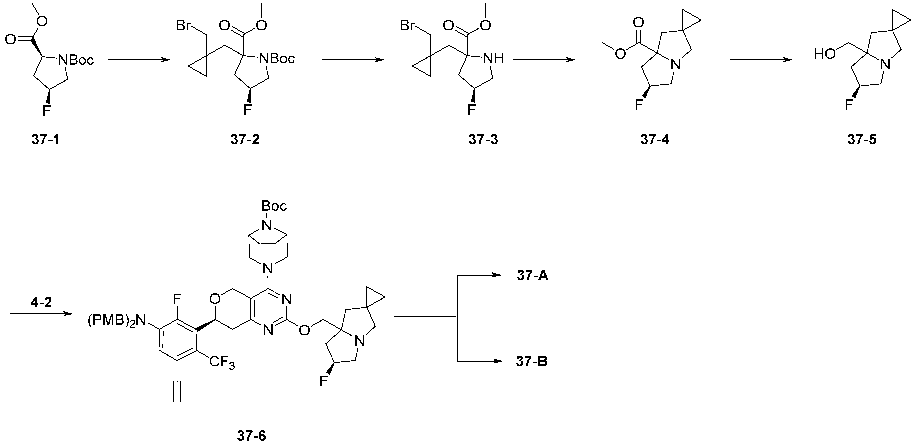

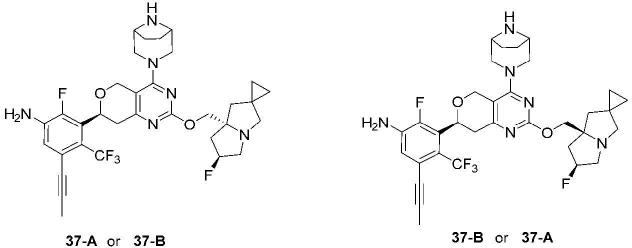

### Step 1

Under nitrogen, lithium bistrimethylsilylamide (5.02 g, 30 mmol, 1M in THF) was slowly added dropwise to a solution of compound **37-1** (5 g, 20.22 mmol) in tetrahydrofuran (50 mL) at -70°C. The mixture was allowed to react at -70°C for 1 hour. 1,1-Bis-bromomethylcyclopropane (9.1 g, 39.93 mmol) was added dropwise to the reaction solution at -70°C, and the mixture was warmed to 25°C with stirring for 12 hours. The reaction was quenched by the addition of saturated aqueous ammonium chloride (50 mL). The mixture was extracted with ethyl acetate (100 mL x 3), dried over anhydrous sodium sulfate, and filtered. The filtrate was concentrated under reduced pressure, and separated by column chromatography (petroleum ether:ethyl acetate = 5:1) to afford compound **37-2.** MS m/z = 294.12 [M-Boc+H]⁺.

### Step 2

Compound **37-2** (1.00 g, 2.54 mmol) was dissolved in dichloromethane (10 mL), and a hydrogen chloride/ethyl acetate solution (4 M, 5 mL) was added. The mixture was stirred at 25°C for 2 hours. The reaction solution was concentrated under reduced pressure to obtain the hydrochloride salt of compound **37-3.** MS m/z = 294.10 [M+H]⁺.

### Step 3

Compound **37-3** (839.78 mg, hydrochloride) was dissolved in acetonitrile (10 mL), and potassium carbonate (2.21 g, 15.99 mmol) was added. The mixture was stirred at 25°C for 12 hours. The reaction solution was filtered, and the filtrate was concentrated under reduced pressure to obtain compound **37-4.** MS m/z = 214.19 [M+H]⁺.

### Step 4

Compound **37-4** (570 mg, 2.67 mmol) was dissolved in tetrahydrofuran (5 mL). Lithium aluminum hydride (202.90 mg, 5.35 mmol) was added under nitrogen. The reaction mixture was allowed to react at 25°C for 2 hours under nitrogen. The reaction mixture was diluted with water (10 mL) and extracted with ethyl acetate (30 mL). The organic phase was washed with saturated brine, dried over anhydrous sodium sulfate, filtered, and concentrated under reduced pressure to yield compound **37-5.** MS m/z = 186.24 [M+H]⁺.

### Step 5

Compound **37-5** (100 mg, 0.53 mmol) was dissolved in tetrahydrofuran (10 mL), and then sodium hydride (110 mg, 4.58 mmol, 60% purity) was added. Under nitrogen protection, the reaction was stirred at 25°C for 0.5 hours, and then a solution of compound **4-2** (300 mg, 0.34 mmol) in tetrahydrofuran (1 mL) was added. After the addition was completed, the reaction was stirred at 25°C for 2 hours. The reaction solution was diluted with ethyl acetate (20 mL) and then washed with saturated brine (10 mL). The organic phase was dried and then filtered. The filtrate was concentrated under reduced pressure and purified by column chromatography (dichloromethane:methanol = 20:1) to give compound **37-6.** MS m/z = 985.8 [M+H]⁺.

### Step 6

Compound **37-6** (250 mg, 0.25 mmol) was dissolved in dichloromethane (10 mL), and trifluoroacetic acid (2 mL) was added. The mixture was stirred at 25°C for 2 hours. The reaction solution was concentrated under reduced pressure, and the crude product was separated by preparative high-performance liquid chromatography (Chromatographic column: Waters Xbridge, 250*19 mm, 5 µm; mobile phase: [water (0.1% hydrochloric acid)-acetonitrile]; gradient: (acetonitrile): 10%-40%) and dried to obtain the hydrochloride salts of compounds **37-A** and **37-B.** HPLC analysis method (Chromatographic column: Waters Xbridge C18, (50mm×4.6mm×3.5µm); mobile phase: [A: water (0.1% ammonium bicarbonate) B: acetonitrile]; gradient: B%: 0-0%, 1.5 min; 0-85%, 4.0 min; 85-85%, 4.49 min; 85-0%, 0.01 min), Rt = 5.868 min for compound **37-A** and Rt = 6.003 min for compound **37-B.** Hydrochloride salt of compound **37-A:** MS m/z = 645.5 [M+H]⁺. ¹H NMR (400 MHz, CD₃OD) δ ppm 7.02 (d, *J* = 8.0 Hz, 1H), 5.61 (d, *J* = 52.0 Hz, 1H), 5.27 - 5.25 (m, 1H), 5.06 - 4.98 (m, 3H), 4.82 - 4.74 (m, 2H), 4.7 - 4.19 (m, 3H), 4.03 (s, 2H), 3.95 - 3.92 (m, 1H), 3.77 - 3.63 (m, 2H), 3.48 - 3.36 (m, 1H), 3.13 - 3.09 (m, 2H), 2.87 - 2.57 (m, 3H), 2.22 - 2.03 (m, 7H), 1.89 - 1.86 (m, 1H), 0.98 - 0.94 (m, 2H), 0.79 - 0.75 (m, 2H).

Hydrochloride salt of compound **37-B:** MS m/z = 645.5 [M+H]⁺. ¹H NMR (400 MHz, CD₃OD) δ ppm 6.95 (d, *J* = 8.0 Hz, 1H), 5.73 - 5.60 (m, 1H), 5.26 - 5.24 (m, 1H), 5.12 - 4.98 (m, 5H), 4.36 - 4.20 (m, 3H), 4.02 (s, 2H), 3.89 - 3.85 (m, 1H), 3.69 - 3.66 (m, 1H), 3.57 - 3.54 (m, 1H), 3.40 (m, 1H), 3.13 - 2.67 (m, 5H), 2.30 - 2.13 (m, 5H), 2.02 (s, 3H), 0.91-0.88 (m, 4H).

### Example 38

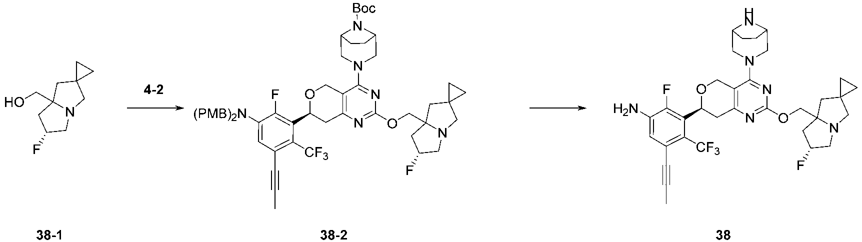

### Step 1

Compound **38-1** (100 mg, 0.54 mmol) was dissolved in tetrahydrofuran (5 mL), and then sodium hydride (65 mg, 2.70 mmol, 60% purity) was added. Under nitrogen protection, the reaction was stirred at 25°C for 0.5 hours. Then, a solution of compound **4-2** (373 mg, 0.44 mmol) in tetrahydrofuran (1 mL) was added. After the addition was completed, the reaction was stirred at 25°C for 2 hours. The reaction solution was dissolved in ethyl acetate (20 mL), and then washed with saturated brine (10 mL). The organic phase was dried and then filtered. The filtrate was concentrated under reduced pressure and purified by column chromatography (dichloromethane:methanol = 20:1) to give compound **38-2.** MS m/z = 985.8 [M+H]⁺.

### Step 2

Compound **38-2** (180 mg, 0.18 mmol) was dissolved in dichloromethane (10 mL), followed by the addition of trifluoroacetic acid (0.5 mL). The mixture was stirred at 25°C for 2 hours. The reaction solution was concentrated under reduced pressure to obtain a crude product. The crude product was separated by preparative HPLC (Chromatographic column: Waters Xbridge, 250 x 19 mm, 5 µm; mobile phase: [water (0.1% hydrochloric acid)-acetonitrile]; gradient: (acetonitrile): 10%-40%) and dried to obtain the hydrochloride salt of compound **38.** MS m/z = 645.5 [M+H]⁺. ¹H NMR (400 MHz, CD₃OD) δ ppm 6.95 (d, *J* = 8.0 Hz, 1H), 5.62 (d, *J =* 52.0 Hz, 1H), 5.23 (d, *J* = 8.0 Hz, 1H), 5.04 - 4.87 (m, 5H), 4.30 - 4.11 (m, 3H), 4.07 - 3.93 (m, 3H), 3.78 - 3.58 (m, 1H), 3.64 - 3.55 (m, 1H), 3.46 - 3.39 (m, 1H), 3.15 (d, *J* = 12.0 Hz, 1H), 3.06 (d, *J* = 16.0 Hz, 1H), 2.86 - 2.79 (m, 2H), 2.71 - 2.57 (m, 1H), 2.15 (s, 3H), 2.06 - 1.99 (m, 4H), 1.90 (d, *J* = 12.0 Hz, 1H), 0.99 - 0.92 (m, 2H), 0.85 - 0.69 (m, 2H).

### Example 39

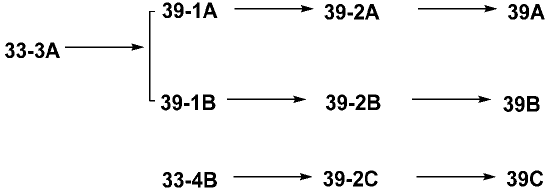

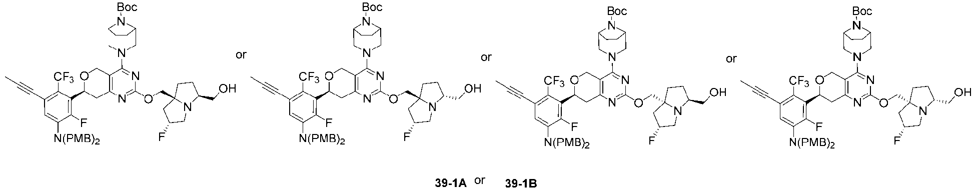

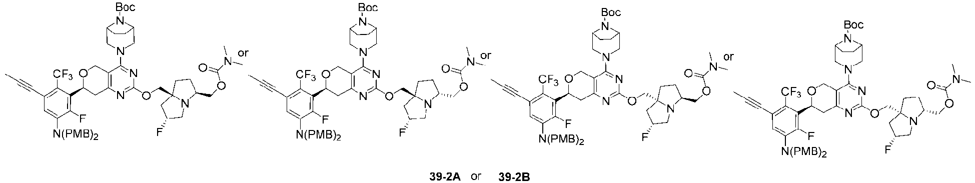

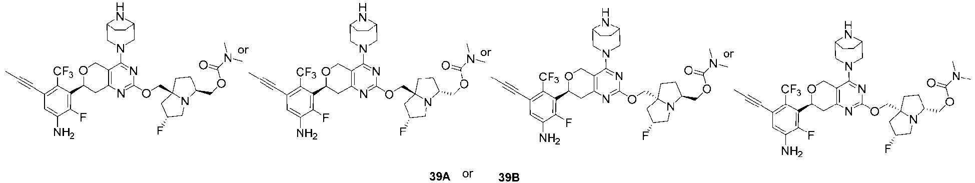

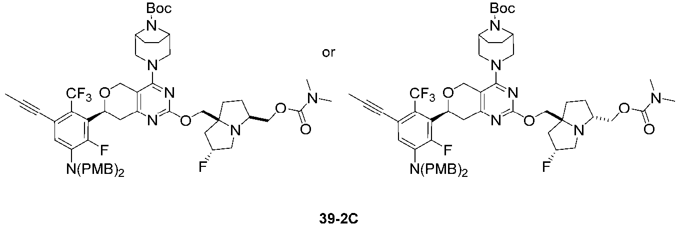

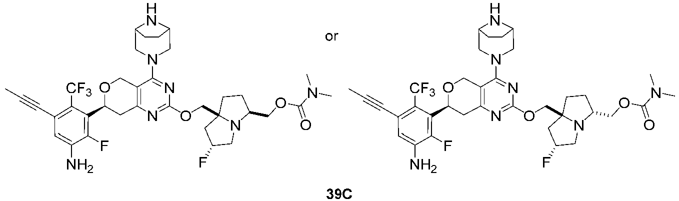

### Step 1

Compound **33-3A** (20 mg, 16.29 µmol) was added to tetrabutylammonium fluoride (0.5 mL, 1 M in tetrahydrofuran) and stirred at room temperature for 2 hours. The mixture was extracted with ethyl acetate (3 mL x 3). The extracted organic phases were combined and washed with saturated brine (10 mL), dried over anhydrous sodium sulfate, filtered, and concentrated under reduced pressure. Purification by preparative thin-layer chromatography (dichloromethane:methanol = 20:1) afforded compounds **39-1A** and **39-1B.** Compound **39-1A,** MS m/z = 989.7 [M+H]⁺, (dichloromethane:methanol = 20:1, Rf = 0.18). Compound **39-1B,** MS m/z = 989.7 [M+H]⁺, (dichloromethane:methanol = 20:1, Rf = 0.14).

### Step 2

Compound **39-1A** (30 mg, 30.33 µmol) was dissolved in anhydrous tetrahydrofuran (2 mL). N,N'-Carbonyldiimidazole (14.75 mg, 90.99 µmol) was added at room temperature and allowed to react for 30 minutes at room temperature. Dimethylamine (2.05 mg, 45.49 µmol) was then added and allowed to react for 3 hours at room temperature. The reaction was quenched by the addition of saturated ammonium chloride solution (5 mL) and extracted with ethyl acetate (10 mL x 2). The extracted organic phases were combined and washed with saturated brine (10 mL), dried over anhydrous sodium sulfate, filtered, and concentrated under reduced pressure. Purification by preparative thin-layer chromatography (petroleum ether:ethyl acetate = 2:1) afforded compound **39-2A.** MS m/z = 1060.7 [M+H]⁺.

Referring to step 2, compound **39-1B** was used as a starting material in place of compound **39-1A** to obtain compound **39-2B.** MS m/z = 1060.7 [M+H]⁺.

Referring to step 2, compound **33-4B** was used as a starting material in place of compound **39-1A** to obtain compound **39-2C.** MS m/z = 1060.7 [M+H]⁺.

### Step 3

Compound **39-2A** (20 mg, 17.63 µmol) was dissolved in dichloromethane (1 mL), and trifluoroacetic acid (0.5 mL) was added. The mixture was stirred at 20°C for 2 hours. The reaction solution was concentrated under reduced pressure to obtain a crude product. The crude product was separated by preparative HPLC (Chromatographic column: Xtimate C18 150*40mm*5µm; mobile phase: [water (0.1% formic acid)-acetonitrile]; gradient: (acetonitrile): 20%-40%) and concentrated under reduced pressure to obtain the formate salt of compound **39A.** MS m/z = 720.5 [M+H]⁺. ¹H NMR (400 MHz, CD₃OD) δ ppm 8.51 (s, 2.87H), 6.91 (d, *J* = 8.0 Hz, 1H), 5.445.11 (m, 2H), 4.81 - 4.79 (m, 1H), 4.69 (d, *J =* 12.0 Hz, 1H), 4.20 (d, *J =* 12.0 Hz, 1H), 4.11 (s, 2H), 4.02 - 3.93 (m, 3H), 3.88 - 3.84 (m, 1H), 3.71 (d, *J* = 12.0 Hz, 1H), 3.55 (d, *J* = 12.0 Hz, 1H), 3.30 - 3.15 (m, 4H), 3.06 - 2.84 (m, 8H), 2.28 - 2.09 (m, 4H), 2.07 - 1.86 (m, 9H).

Compound **39-2B** (20 mg, 17.63 µmol) was dissolved in dichloromethane (1 mL), and trifluoroacetic acid (0.5 mL) was added. The mixture was stirred at 20°C for 2 hours. The reaction solution was concentrated under reduced pressure to obtain a crude product. The crude product was separated by preparative HPLC (Chromatographic column: Xtimate C18 150*40mm*5µm; mobile phase: [water (0.1% formic acid)-acetonitrile]; gradient: (acetonitrile): 20%-40%) and concentrated under reduced pressure to obtain the formate salt of compound **39B.** MS m/z = 720.5 [M+H]⁺. ¹H NMR (400 MHz, CD₃OD) δ ppm 8.51 (s, 2.90H), 6.91 (d, *J* = 8.0 Hz, 1H), 5.48 - 5.28 (m, 1H), 5.23 - 5.09 (m, 1H), 4.81 - 4.79 (s, 1H), 4.69 (d, *J* = 12.0 Hz, 1H), 4.25 (d, *J = 12.0* Hz, 1H), 4.20 - 4.09 (m, 2H), 4.08 - 4.04 (m, 1H), 4.01-3.96 (m, 1H), 3.94 - 3.88 (m, 1H), 3.71 (d, *J* = 16.0 Hz, 1H), 3.59 (d, *J =* 16.0 Hz, 1H), 3.50 - 3.41 (m, 1H), 3.29 - 3.19 (m, 3H), 3.08 - 2.84 (m, 9H), 2.47 - 2.38 (m, 1H), 2.28 - 2.19 (m, 2H), 2.09 - 2.00 (m, 5H), 1.99 - 1.79 (m, 4H), 1.76 - 1.66 (m, 1H).

Compound **39-2C** (200 mg, 188.65 µmol) was dissolved in trifluoroacetic acid (5 mL) and reacted at 25°C for 1 hour. The mixture was concentrated under reduced pressure to obtain a crude product, which was then separated by preparative high-performance liquid chromatography (Chromatographic column: Waters Xbridge, 250 x 19 mm, 5 µm; mobile phase: [water (0.1% ammonia)-acetonitrile]; gradient: 50% to 70% (acetonitrile)) to afford compound **39C.** MS m/z = 720.5 [M+H]⁺. ¹H NMR (400 MHz, CD₃OD) δ ppm 6.90 (d, *J* = 8.0 Hz, 1H), 5.34 - 5.20 (m, 1H), 5.16 - 5.12 (dd, *J* = 8.0 Hz, 4.0 Hz, 1H), 4.82 - 4.78 (m, 1H), 4.65 (d, *J* = 12.0 Hz, 1H), 4.34 - 4.19 (m, 5H), 3.57 - 3.41 (m, 5H), 3.24 - 3.10 (m, 3H), 3.06 - 2.92 (m, 7H), 2.85 (dd, *J =* 16.0 Hz, 4.0 Hz, 1H), 2.49 - 2.40 (m, 1H), 2.11 - 1.98 (m, 6H), 1.92 - 1.79 (m, 4H), 1.76 - 1.65 (m, 2H).

### Example 40

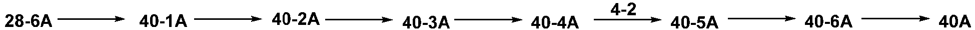

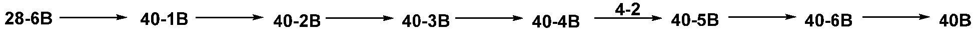

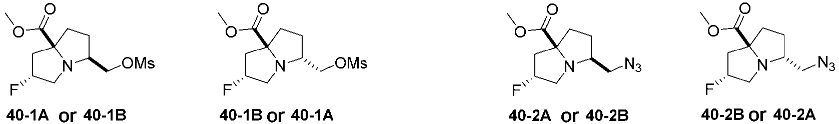

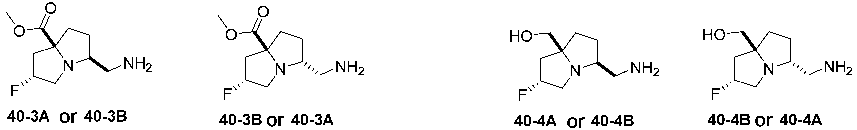

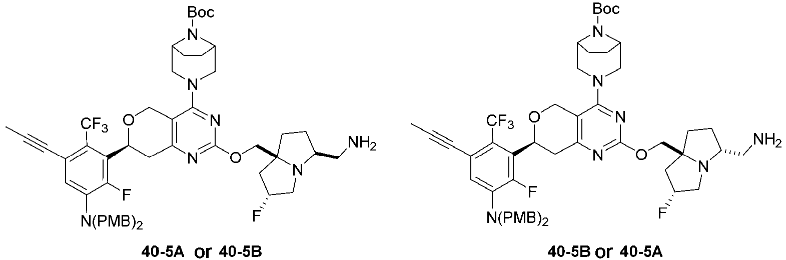

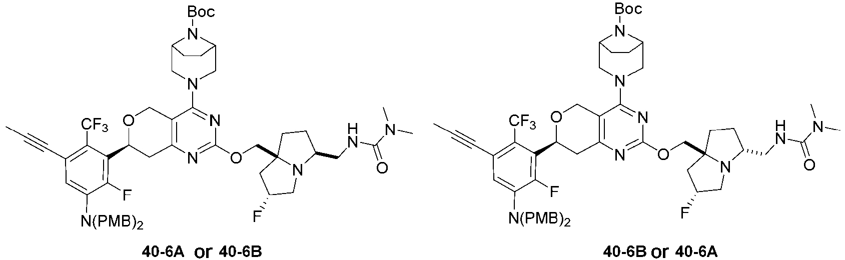

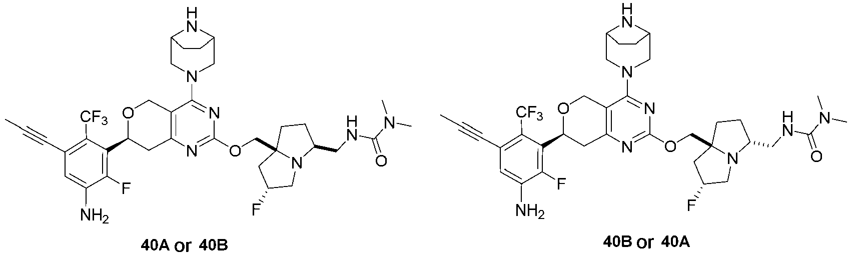

### Step 1

Compound **28-6A** (420 mg, 1.93 mmol) was weighed and dichloromethane (10 mL) was added. Triethylamine (586.91 mg, 5.80 mmol) and methanesulfonyl chloride (332.21 mg, 2.90 mmol) were added, and the mixture was reacted at 25°C for 2 hours. Saturated aqueous sodium bicarbonate (15 mL) was added, and the mixture was extracted with ethyl acetate (5 mL x 3). The mixture was dried over anhydrous sodium sulfate, filtered, and concentrated under reduced pressure to obtain compound **40-1A.** MS m/z = 296.1 [M+H]⁺.

Referring to step 1, compound **28-6B** was used as a starting material in place of compound **28-6A** to obtain compound **40-1B.** MS m/z = 296.1 [M+H]⁺

### Step 2

Compound **40-1A** (405 mg, 1.37 mmol) was weighed and N,N-dimethylformamide (10 mL) and sodium azide (178.30 mg, 2.74 mmol) were added. The mixture was reacted at 80°C for 16 hours. The organic phase was concentrated under reduced pressure to obtain compound **40-2A.** MS m/z = 243.1 [M+H]⁺.

Referring to step 2, compound **40-1B** was used as a starting material in place of compound **40-1A** to obtain compound **40-2B.** MS m/z = 243.1 [M+H]⁺.

### Step 3

Compound **40-2A** (250 mg, 1.03 mmol) was weighed and methanol (10 mL) was added. Palladium on carbon (109.82 mg, 1.03 mmol) was added and the mixture was reacted at 25°C for 16 hours under a hydrogen atmosphere (15 psi). The reaction mixture was filtered. The filter cake was washed with 10 mL of methanol, and the filtrate was concentrated to obtain compound **40-3A.** MS m/z = 217.2 [M+H]⁺.

Referring to step 3, compound **40-2B** was used as a starting material in place of compound **40-2A** to obtain compound **40-3B.** MS m/z = 217.2 [M+H]⁺.

### Step 4

Compound **40-3A** (180 mg, 832.36 µmol) was weighed and dissolved in tetrahydrofuran (10 mL). The resulting solution was cooled to 0°C, and lithium aluminum tetrahydride (1 M, 2.5 mL) was added dropwise. The mixture was stirred for 2 hours. The reaction solution was carefully quenched with 0.1 mL of water, followed by the addition of 0.5 g of anhydrous sodium sulfate and stirring for 2 minutes. The mixture was filtered through a pad of Celite, and the filter cake was rinsed with 20 mL of tetrahydrofuran. The filtrate was collected and concentrated under reduced pressure to yield compound **40-4A.** MS m/z = 189.2 [M+H]⁺.

Referring to step 4, compound **40-3B** was used as a starting material in place of compound **40-3A** to obtain compound **40-4B.** MS m/z = 189.2 [M+H]⁺.

### Step 5

Compound **40-4A** (105 mg, 557.79 µmol) was weighed. N,N-dimethylformamide (3 mL) was added. Sodium hydride (66.93 mg, 1.67 mmol, 60% purity) was added, and the mixture was reacted at 0°C for 0.5 h. Compound **4-2** (490.83 mg, 557.79 µmol) was added, and the mixture was reacted at 25°C for 1 h. Saturated aqueous ammonium chloride solution was added. (15mL). The mixture was extracted with ethyl acetate (5mL * 3). The organic phase was concentrated under reduced pressure, and separated by column chromatography (dichloromethane:methanol =10:1) to obtain compound **40-5A.** MS m/z = 988.7 [M+H]⁺.

Referring to step 5, compound **40-4B** was used as a starting material in place of compound **40-4A** to obtain compound **40-5B.** MS m/z = 988.7 [M+H]⁺.

### Step 6

Compound **40-5A** (140 mg, 141.69 µmol) was weighed and tetrahydrofuran (10 mL) was added. N,N'-carbonyldiimidazole (45.95 mg, 283.37 µmol) was added and reacted at 25°C for 1 hour. A solution of dimethylamine in tetrahydrofuran (2 M, 14.74 µL) was added and reacted at 25°C for 2 hours. The organic phase was concentrated under reduced pressure and separated using a reverse-phase column (Flash Spherical C18, water:acetonitrile = 3:2) to obtain compound **40-6A.** MS m/z = 1059.7 [M+H]⁺.

Referring to step 6, compound **40-5B** was used as a starting material in place of compound **40-5A** to obtain compound **40-6B.** MS m/z = 1059.7 [M+H]⁺.

### Step 7

Compound **40-6A** (50 mg, 47.21 µmol) was weighed. Dichloromethane (3 mL) and trifluoroacetic acid (1 mL) were added, and the mixture was reacted at 25°C for 0.5 hours. The organic phase was concentrated under reduced pressure to obtain a crude product, which was then separated by preparative HPLC (Chromatographic column: Waters SunFire, 250 x 19 mm, 5 µm; mobile phase: [water (0.1% ammonia)-acetonitrile]; gradient: (acetonitrile): 40% - 75%) to afford compound **40A.** MS m/z = 719.5 [M+H]⁺. ¹H NMR (400 MHz, CD₃OD) δ ppm 6.90 (d, *J* = 8.0 Hz, 1H), 5.36 - 5.03 (m, 2H), 4.81 - 4.77 (m, 2H), 4.68 - 4.55 (m, 2H), 4.27-4.16 (m, 2H), 3.94 - 3.89 (m, 1H), 3.58- 3.42 (m, 5H), 3.23 - 3.10 (m, 2H), 3.06 (d, *J* = 12.0 Hz, 1H), 3.00 - 2.91 (m, 1H), 2.88 (s, 6H), 2.85 - 2.82 (m, 1H), 2.21 - 2.13 (m, 2H), 2.04 - 1.99 (m, 5H), 1.87 - 1.60 (m, 6H).

Compound **40-6B** (50 mg, 47.21 µmol) was weighed. Dichloromethane (3 mL) and trifluoroacetic acid (1 mL) were added, and the mixture was reacted at 25°C for 0.5 h. The organic phase was concentrated under reduced pressure to obtain a crude product, which was then separated by preparative HPLC (Chromatographic column: Waters SunFire, 250 x 19 mm, 5 µm; mobile phase: [water (0.1% formic acid)-acetonitrile]; gradient: (acetonitrile): 40%-75%) to obtain the formate salt of compound **40B.** MS m/z = 719.5 [M+H]⁺. ¹H NMR (400 MHz, CD₃OD) δ ppm 8.47 (s, 1.3H), 6.95 - 6.82 (m, 1H), 5.48 - 5.35 (m, 1H), 5.21 - 5.07 (m, 2H), 4.76 - 4.70 (m, 1H), 4.66 - 4.51 (m, 2H), 4.46 - 4.32 (m, 2H), 4.25 - 4.06 (m, 2H), 4.04 - 3.99 (m, 2H), 3.64 - 3.56 (m, 1H), 3.52 - 3.46 (m, 1H), 3.43 - 3.34 (m, 2H), 3.30 - 3.23 (m, 2H), 3.11 - 3.05 (m, 1H), 2.94 - 2.89 (m, 6H), 2.62 - 2.16 (m, 4H), 2.08 - 1.97 (m, 7H), 1.94 - 1.89 (m, 2H).

### Example 41

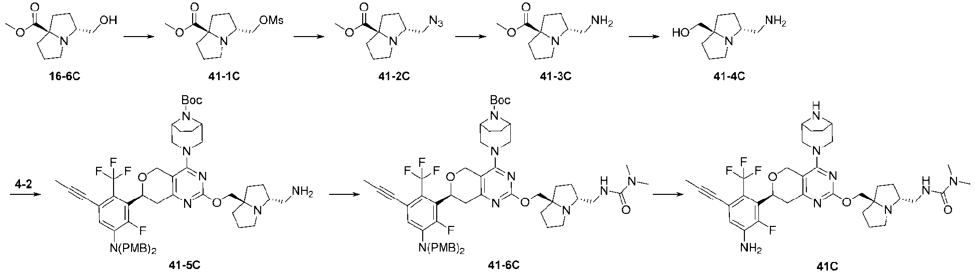

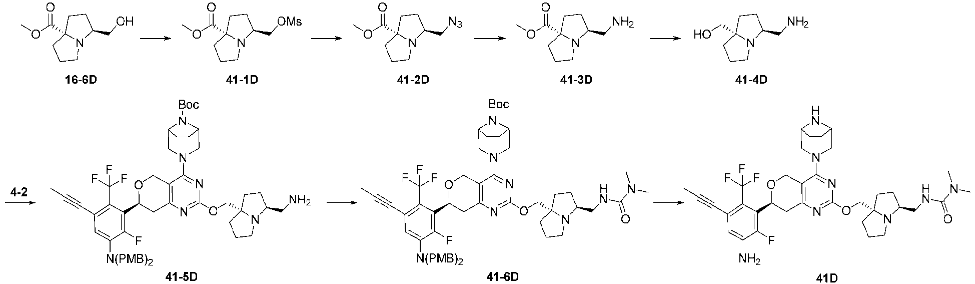

### Step 1

Compound **16-6C** (216 mg, 1.08 mmol) and triethylamine (438.79 mg, 4.34 mmol) were dissolved in dichloromethane (3 mL), and methanesulfonyl chloride (248.37 mg, 2.17 mmol) was added at 0°C. The reaction mixture was reacted at 0°C for 4 hours. The mixture was quenched with water (10 mL) and extracted with ethyl acetate (10 mL x 3). The extracted organic phases were combined and washed with saturated brine (10 mL), dried over anhydrous sodium sulfate, filtered, and concentrated under reduced pressure to obtain compound **41-1C.** MS m/z = 278.0 [M+H]⁺.

Referring to step 1, compound **16-6D** was used as a starting material in place of compound **16-5C to** obtain compound **41-1D.** MS m/z = 278.0 [M+H]⁺.

### Step 2

Compound **41-1C** (300.7 mg, 1.08 mmol) was dissolved in N,N-dimethylformamide (5 mL). Sodium azide (211.46 mg, 3.25 mmol) was added. The mixture was heated to 80°C and stirred for 16 hours. The mixture was filtered, and the filtrate was concentrated under reduced pressure to obtain crude compound **41-2C.** MS m/z = 225.0 [M+H]⁺.

Referring to step 2, compound **41-1D** was used as a starting material in place of compound **41-1C** to obtain compound **41-2D.** MS m/z = 225.0 [M+H]⁺.

### Step 3

Compound **41-2C** (243.0 mg, crude) was dissolved in methanol (5 mL). Palladium on carbon (230.6 mg, 216.71 mmol, 10% purity) was added portionwise. The mixture was stirred at 25°C under a hydrogen atmosphere for 16 hours. The mixture was filtered. The filter cake was washed with methanol (15 mL x 3), and concentrated under reduced pressure to obtain crude compound **41-3C.** MS m/z = 199.0 [M+H]⁺.

Referring to step 3, compound **41-2D** was used as a starting material in place of compound **41-2C** to obtain compound **41-3D.** MS m/z = 199.0 [M+H]⁺.

### Step 4

Compound **41-3C** (214.8 mg, crude) was dissolved in anhydrous tetrahydrofuran (5 mL). The atmosphere was replaced with nitrogen, and the temperature was lowered to -78°C. A solution of lithium aluminum hydride in tetrahydrofuran (866.73 µL, 2.17 mmol, 2.5 M) was slowly added, and the reaction was stirred at -78°C for 1 hour. The reaction was quenched by adding water (0.3 mL), and filtered. The filter cake was washed with ethyl acetate (15 mL x 3). The mixture was concentrated under reduced pressure to obtain crude compound **41-4C.** MS m/z = 171.0 [M+H]⁺.

Referring to step 4, compound **41-3D** was used as a starting material in place of compound **41-3C** to obtain compound **41-4D.** MS m/z = 171.0 [M+H]⁺.

### Step 5

Compound **41-4C** (184.5 mg, crude) was dissolved in tetrahydrofuran (5 mL), and cooled to 0°C. Sodium hydride (86.69 mg, 2.17 mmol, 60% purity) was slowly added. The mixture was stirred at 0°C for 0.5 hours. Compound **4-2** (1.14 g, 1.30 mmol) was added, and the mixture was stirred at 0°C for 1 hour. Water (0.5 mL) was added to quench the reaction, and the mixture was extracted with dichloromethane (15 mL * 3). The organic phases were combined and concentrated under reduced pressure. The mixture was then purified by C18 reverse-phase column chromatography (acetonitrile:water = 2:3) to obtain compound **41-5C.** MS m/z = 970.0 [M+H]⁺.

Referring to step 5, compound **41-4D** was used as a starting material in place of compound **41-4C** to obtain compound **41-5D.** MS m/z = 970.0 [M+H]⁺.

### Step 6

Compound **41-5C** (183 mg, 188.64 µmol) was dissolved in tetrahydrofuran (3 mL). N,N'-carbonyldiimidazole (61.18 mg, 377.28 mmol) was added at 25°C and the mixture was stirred for 2 hours. A solution of dimethylamine in tetrahydrofuran (141.49 µL, 282.99 µmol, 2 M) was added and the mixture was reacted at 25°C with stirring for 2 hours. Water (5 mL) was added to quench the reaction. The mixture was extracted with dichloromethane (10 mL*3). The organic phases were combined, washed with saturated brine (10 mL), dried over anhydrous sodium sulfate, and filtered. The filtrate was concentrated under reduced pressure, and purified by C18 reverse phase column chromatography (acetonitrile:water = 2:3) to obtain compound **41-6C.** MS m/z = 1041.8 [M+H]⁺.

Referring to step 6, compound **41-5D** was used as a starting material in place of compound 41-5C to obtain compound **41-6D.** MS m/z = 1041.8 [M+H]⁺.

### Step 7

Compound **41-6C** (90 mg, 86.44 µmol) was dissolved in anhydrous dichloromethane (1.5 mL). Trifluoroacetic acid (0.5 mL) was added and the mixture was stirred at 25°C for 2 hours. The reaction solution was concentrated under reduced pressure to obtain a crude product. The crude product was separated by preparative HPLC (Chromatographic column: Xbridge C18 250*19 mm*5 µm; mobile phase: [water (0.05% ammonia)-acetonitrile]; (acetonitrile): 45%-70%) and dried to obtain compound **41C.** MS m/z = 701.5 [M+H]⁺. ¹H NMR (400 MHz, DMSO-*d₆*) δ ppm 6.88 (d, *J* = 8.0 Hz, 1H), 6.18 (t, *J* = 4.0 Hz, 1H), 6.01 (s, 2H), 5.02 (dd, *J* = 12.0 Hz, 4.0 Hz, 1H), 4.74 (d, *J* = 16.0 Hz, 1H), 4.54 (d, *J* = 16.0 Hz, 1H), 3.92 - 3.83 (m, 3H), 3.39 - 3.32 (m, 5H), 3.23 (d, *J* = 12.0 Hz, 1H), 3.17 - 3.10 (m, 1H), 3.06 - 2.99 (m, 2H), 2.86 (d, *J =* 12.0 Hz, 1H), 2.81 - 2.75 (m, 8H), 2.63 - 2.57 (m, 1H), 2.03 (s, 3H), 2.00 - 1.97 (m, 1H), 1.81 - 1.75 (m, 1H), 1.70 - 1.51 (m, 9H), 1.47 - 1.41 (m, 1H).

Compound **41-6D** (30 mg, 28.81 µmol) was dissolved in dichloromethane (1.5 mL). Trifluoroacetic acid (0.5 mL) was added, and the mixture was stirred at 25°C for 2 hours. The reaction solution was concentrated under reduced pressure to obtain a crude product. The crude product was separated by preparative HPLC (Chromatographic column: Waters SunFire, 250*19 mm, 5µm; mobile phase: [water (0.1% formic acid)-acetonitrile]; gradient: (acetonitrile): 40%-75%) and dried to obtain the formate salt of compound **41D.** MS m/z = 701.5 [M+H]⁺. ¹H NMR (400 MHz, CD ₃ CN) δ ppm 8.10 (s, 0.13H), 6.91 (d, *J =* 8.0 Hz, 1H), 5.14 (dd, *J* = 12.0 Hz, 4.0 Hz, 1H), 4.76 (d, *J* = 12.0 Hz, 1H), 4.66 (d, *J* = 16.0 Hz, 1H), 4.47 (d, *J =* 12.0 Hz, 1H), 4.39 (d, *J* = 12.0 Hz, 1H), 4.10 - 4.07 (m, 3H), 4.00 - 3.94 (m, 1H), 3.71 (d, *J =* 16.0 Hz, 1H), 3.60 - 3.56 (m, 2H), 3.49 (dd, *J* = 16.0 Hz, 8.0 Hz, 1H), 3.42 - 3.32 (m, 2H), 3.24 - 3.09 (m, 2H), 2.87 (dd, *J* = 16.0 Hz, 4.0 Hz, 1H), 2.79 (s, 6H), 2.20 - 2.15 (m, 4H), 2.12 - 2.03 (m, 9H), 1.90 - 1.83 (m, 2H).

### Example 42

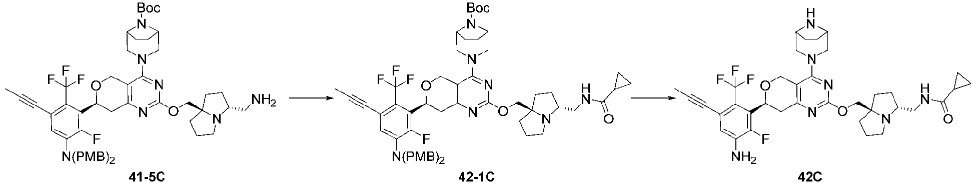

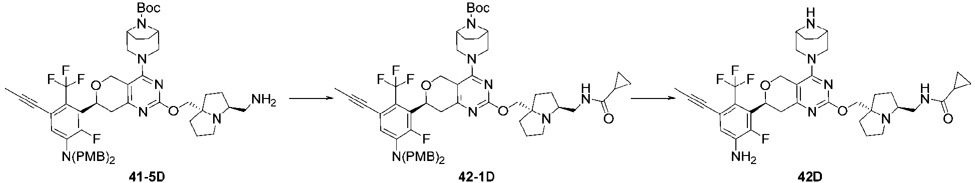

### Step 1

C yclopropanecarboxylic acid (6.66 mg, 77.31 µmol) was dissolved in dichloromethane (2 mL), and N,N-diisopropylethylamine (19.98 mg, 154.62 µmol) and 2-(7-azobenzotriazole)-N,N,N',N'-tetramethyluronium hexafluorophosphate (29.4 mg, 77.31 µmol) were added in sequence. The reaction was stirred at room temperature for 0.5 hours. Compound **41-5C** was added and stirred at room temperature for 2 hours. Water (5 mL) was added to quench the reaction. The mixture was extracted with dichloromethane (10 mL * 3). The extracted organic phases were combined and washed with saturated brine (50 mL), dried over anhydrous sodium sulfate, and filtered. The filtrate was concentrated under reduced pressure, and separated by column chromatography (dichloromethane:methanol = 15:1) to obtain compound **42-1C.** MS m/z = 1038.8 [M+H]⁺.

Referring to step 1, compound **41-5D** was used as a starting material in place of compound **41-5C** to obtain compound **42-1D.** MS m/z = 1038.8 [M+H]⁺.

### Step 2

Compound **42-1C** (25 mg, 24.08 µmol) was dissolved in dichloromethane (1.5 mL). Trifluoroacetic acid (0.5 mL) was added, and the mixture was stirred at 25°C for 2 hours. The reaction solution was concentrated under reduced pressure to obtain a crude product. The crude product was separated by preparative HPLC (Chromatographic column: SunFire C18 150*40mm*5µm; mobile phase: [water (0.1% formic acid)-acetonitrile]; (acetonitrile): 6%-36%) and dried to obtain compound **42C.** MS m/z = 698.5 [M+H]⁺, ¹H NMR (400 MHz, CD₃OD) δ ppm 6.90 (d, *J* = 8.0 Hz, 1H), 5.14 (dd, *J* = 12.0 Hz, 4.0 Hz, 1H), 4.77 (s, 1H), 4.65 (d, *J* = 12.0 Hz, 1H), 4.33 (d, *J= 12.0* Hz, 1H), 4.12 (d, *J* = 12.0 Hz, 2H), 3.57 - 3.51 (m, 3H), 3.43 (d, *J* = 12.0 Hz, 1H), 3.25 - 3.17 (m, 2H), 3.05 (d, *J* = 16.0 Hz, 2H), 2.96 (dd, *J* = 12.0 Hz, 4.0 Hz, 1H), 2.90 - 2.82 (m, 2H), 2.62 - 2.58 (m, 1H), 2.04 - 1.98 (m, 6H), 1.92 - 1.83 (m, 6H), 1.76 - 1.71 (m, 2H), 1.62 - 1.56 (m, 2H), 0.85 - 0.81 (m, 2H), 0.75 - 0.72 (m, 2H).

Compound **42-1D** (25 mg, 24.08 µmol) was dissolved in dichloromethane (1.5 mL), and trifluoroacetic acid (0.5 mL) was added. The reaction mixture was allowed to react at 25°C for 2 hours. The reaction mixture was concentrated under reduced pressure to obtain a crude product. The crude product was separated by preparative HPLC (Chromatographic column: SunFire C18 150*40mm*5µm; mobile phase: [water (0.1% formic acid)-acetonitrile]; (acetonitrile): 10%-36%) and dried to obtain the formate salt of compound **42D.** MS m/z = 698.5 [M+H]⁺, ¹H NMR (400 MHz, CD₃OD) δ ppm 8.49 (s, 2.55H), 6.92 (d, *J =* 8.0 Hz, 1H), 5.18 (dd, *J* = 12.0 Hz, 4.0 Hz, 1H), 4.84 (s, 1H), 4.71 (d, *J* = 12.0 Hz, 1H), 4.46 (d, *J* = 12.0 Hz, 1H), 4.38 (d, *J* = 12.0 Hz, 1H), 4.25 (d, *J* = 16.0 Hz, 1H), 3.96 - 3.86 (m, 3H), 3.73 - 3.67 (m, 2H), 3.58 (d, *J* = 16.0 Hz, 1H), 3.52 - 3.47 (m, 2H), 3.28 - 3.20 (m, 3H), 2.91 (dd, *J* = 20.0 Hz, 4.0 Hz, 1H), 2.34 - 2.30 (m, 1H), 2.26 - 2.14 (m, 4H), 2.08 - 1.95 (m, 9H), 1.89 - 1.85 (m, 1H), 1.61 - 1.55 (m, 1H), 0.89 - 0.85 (m, 2H), 0.81 - 0.76 (m, 2H).

### Example 43

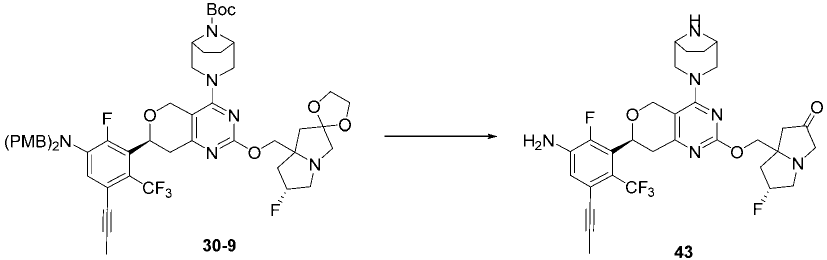

### Step 1

Compound **30-9** (100 mg, 0.10 mmol) was dissolved in anhydrous tetrahydrofuran (3 mL). Water (1.5 mL) and concentrated hydrochloric acid (1.5 mL) were added sequentially at room temperature, and the mixture was heated to 75°C and stirred for 32 hours. The reaction solution was concentrated under reduced pressure to obtain a crude product. The crude product was separated by preparative HPLC (Chromatographic column: Waters Xbridge, 250*19 mm, 5µm; mobile phase: [water (0.1% ammonium bicarbonate)-acetonitrile]; gradient: (acetonitrile): 50%-70%) and concentrated under reduced pressure to obtain compound **43.** MS m/z = 633.4 [M+H]⁺. ¹H NMR (400 MHz, CD₃OD) *δ* ppm 6.96 (d, *J* = 8.0 Hz, 1H), 5.45 - 5.30 (m, 1H), 5.22 - 5.18 (m, 1H), 4.87 - 4.84 (m, 1H), 4.73 - 4.70 (m, 1H), 4.40 - 4.32 (m, 2H), 4.23 - 4.18 (m, 1H), 3.79 - 3.73 (m, 2H), 3.66 - 3.50 (m, 4H), 3.32 - 3.24 (m, 2H), 3.17 - 3.04 (m, 2H), 2.94 - 2.88 (m, 1H), 2.77 - 2.71 (m, 1H), 2.62 - 2.57 (m, 1H), 2.50 - 2.47 (m, 1H), 2.43 - 2.34 (m, 1H), 2.16 - 2.11 (m, 1H), 2.07 (s, 3H), 1.97 - 1.91 (m, 2H), 1.85 - 1.80 (m, 1H).

### Example 44

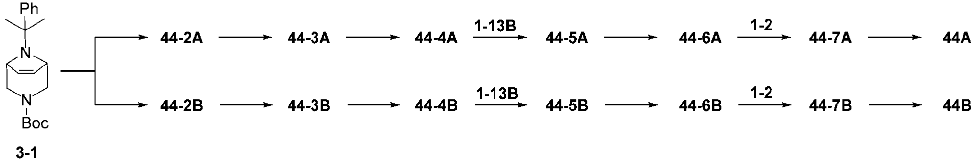

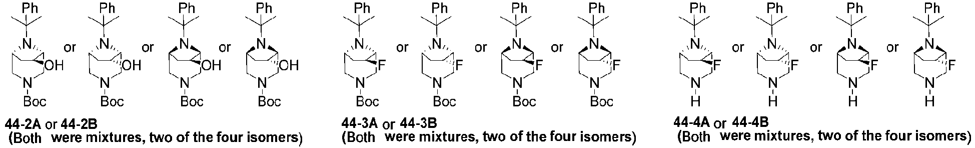

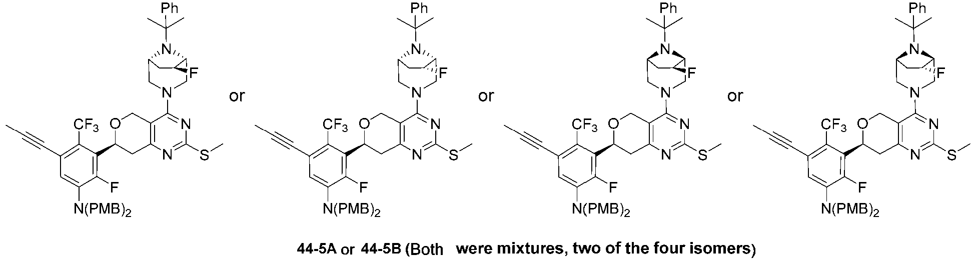

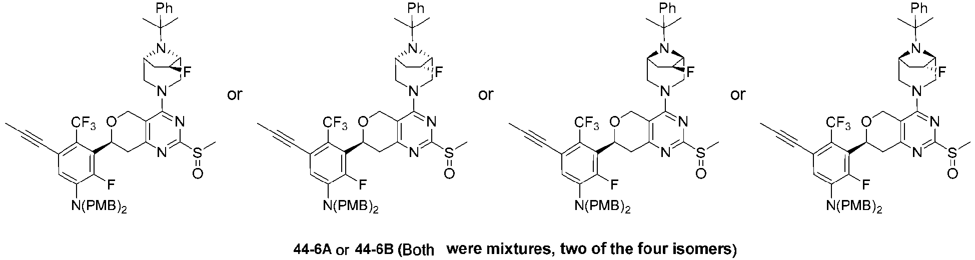

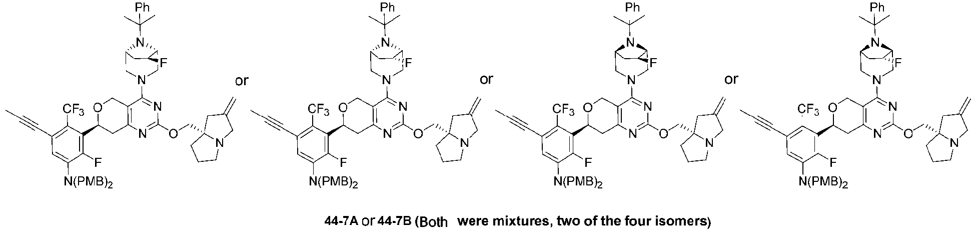

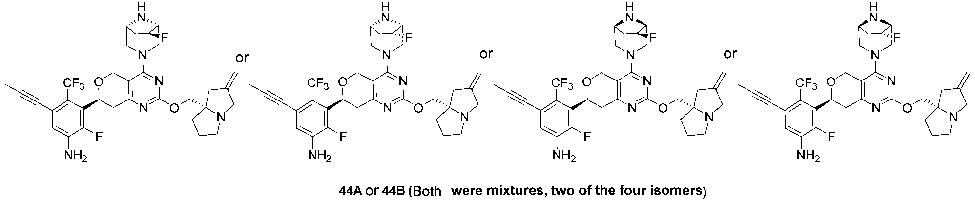

### Step 1

Compound **3-1** (4 g, 12.18 mmol) was dissolved in tetrahydrofuran (40 mL), and borane in tetrahydrofuran (3.14 g, 36.54 mmol, 1 M) was added. The mixture was stirred at 60°C for 1.5 hours. 2M sodium hydroxide solution (1.95 g, 48.71 mmol) and hydrogen peroxide (6.90 g, 60.89 mmol) were added, and the mixture was stirred at room temperature for 3 hours. The reaction was quenched by adding water (50 mL), and extracted with ethyl acetate (30 mL x 3). The organic phases were combined, concentrated under reduced pressure, and separated by column chromatography (petroleum ether:ethyl acetate = 3:1) to provide compounds **44-2A** and **44-2B.** Compound **44-2A** (developing solvent: petroleum ether:ethyl acetate = 5:1, Rf = 0.5), MS m/z = 347.2 [M+H]⁺ compound **44-2B** (developing solvent: petroleum ether:ethyl acetate = 5:1, Rf = 0.3), MS m/z = 347.2 [M+H]⁺.

### Step 2

Compound **44-2A** (1 g, 2.89 mmol) was dissolved in dichloromethane (30 mL), and diethylaminosulfur trifluoride (1.4 g, 8.66 mmol) was added. The mixture was stirred at 0°C for 1 hour. The reaction was quenched with saturated aqueous sodium bicarbonate (50 mL), and extracted with dichloromethane (20 mL x 3). The organic phases were combined, concentrated under reduced pressure, and separated by column chromatography (petroleum ether:ethyl acetate = 10:1) to obtain compound **44-3A.** MS m/z = 349.1 [M+H]⁺.

Referring to step 2, compound **44-2B** was used as a starting material in place of compound **44-2A** to obtain compound **44-3B.** MS m/z = 349.1 [M+H]+.

### Step 3

Compound **44-3A** (200 mg, 574.0 µmol) was dissolved in methanol (2 mL), and dilute hydrochloric acid (2 mL, 1 M) was added. The mixture was stirred at 25°C for 1 hour. The mixture was concentrated under reduced pressure to obtain compound **44-4A.** MS m/z = 249.2 [M+H]⁺.

Referring to step 3, compound **44-3B** was used as a starting material in place of compound **44-3A** to obtain compound **44-4B.** MS m/z = 249.2 [M+H]+.

### Step 4

Compound **44-4A** (140 mg, 563.75 µmol) was dissolved in N,N-dimethylformamide (2 mL) and N,N-diisopropylethylamine (72.86 mg, 563.75 µmol) and **1-13B** (443 mg, 563.75 µmol) were added at room temperature. The mixture was heated to 100°C and stirred for 1 hour. Water (2 mL) was added to quench the reaction, followed by extraction with ethyl acetate (5 mL x 3). The extracted organic phases were combined and concentrated under reduced pressure and separated by column chromatography (petroleum ether:ethyl acetate = 2:1) to afford compound **44-5A.** MS m/z = 884.5 [M+H]⁺.

Referring to step 4, compound **44-4B** was used as a starting material in place of compound **44-4A** to obtain compound **44-5B.** MS m/z = 884.5 [M+H]+.

### Step 5

Compound **44-5A** (260 mg, 294.11 µmol) was dissolved in dichloromethane (5 mL), and m-chloroperbenzoic acid (50.75 mg, 294.11 µmol, 85% purity) was added. The mixture was stirred at room temperature for 2 hours. Saturated aqueous sodium sulfite solution (2 mL) was added to quench the reaction, and the mixture was extracted with dichloromethane (5 mL * 3). The organic phases were combined, washed with saturated aqueous sodium bicarbonate solution (5 mL), dried over anhydrous sodium sulfate, and filtered. The filtrate was concentrated under reduced pressure, and separated by column chromatography (petroleum ether:ethyl acetate = 5:1) to obtain compound **44-6A.** MS m/z = 900.6 [M+H]⁺.

Referring to step 5, compound **44-5B** was used as a starting material in place of compound **44-5A** to obtain compound **44-6B.** MS m/z = 900.6 [M+H]+.

### Step 6

Compound (S)-(2-methylenetetrahydro-1H-pyrrolin-7A(5H)-yl)methanol (66.4 mg, 433.3 µmol) was dissolved in anhydrous tetrahydrofuran (5 mL). Sodium hydride (20.8 mg, 866.66 µmol, 60% purity) was added at 0°C and stirred for 0.5 hours. Compound **44-6A** (260 mg, 289.0 µmol) was added. The mixture was warmed to 25°C and stirred for 2 hours. The reaction was quenched by the addition of water (5 mL). The product was extracted with ethyl acetate (5 mL x 3 L), dried over anhydrous sodium sulfate, and filtered. The filtrate was concentrated under reduced pressure, and separated by column chromatography (dichloromethane:methanol = 10:1) to afford compound **44-7A.** MS m/z = 989.7 [M+H]⁺.

Referring to step 6, compound **44-6B** was used as a starting material in place of compound **44-6A** to obtain compound **44-7B.** MS m/z = 989.7 [M+H]+.

### Step 7

Compound **44-7A** (150 mg, 151.65 µmol) was dissolved in dichloromethane (5 mL), and trifluoroacetic acid (3 mL) was added. The mixture was stirred at 25°C for 0.5 hours. The reaction solution was concentrated under reduced pressure to obtain a crude product. The crude product was separated by preparative HPLC (Chromatographic column: Waters SunFire, 250*19 mm, 5 µm; mobile phase: [water (0.1% ammonia)-acetonitrile]-acetonitrile]; gradient: (acetonitrile): 30%-55%) and dried to obtain compound **44A.** MS m/z = 631.5 [M+H]⁺. ¹H NMR (400 MHz, CD₃OD) *δ* ppm 6.92 (d, *J =* 8.4 Hz, 1H), 5.63 - 5.34 (m, 1H), 5.30 (d, *J* = 6.9 Hz, 2H), 5.22 - 5.12 (m, 1H), 4.76 (d, *J* = 13.7 Hz, 1H), 4.71 (d, *J* = 13.7 Hz, 1H), 4.58 - 4.50 (m, 2H), 4.34 (d, *J* = 13.8 Hz, 1H), 4.12 (dd, *J* = 45.8, 14.6 Hz, 2H), 3.92 (t, *J =* 12.8 Hz, 2H), 3.78 - 3.51 (m, 3H), 3.30 - 3.15 (m, 3H), 3.08 - 2.99 (m, 1H), 2.91 (d, *J* = 20.9 Hz, 1H), 2.78 (d, *J* = 16.0 Hz, 1H), 2.44 - 2.07 (m, 6H), 2.02 (s, 3H).

Compound **44-7B** (80 mg, 80.88 µmol) was dissolved in dichloromethane (2 mL). Trifluoroacetic acid (1 mL) was added, and the mixture was stirred at 25°C for 0.5 h. The reaction solution was concentrated under reduced pressure to obtain a crude product. The crude product was separated by preparative HPLC (Chromatographic column: Waters SunFire, 250*19 mm, 5 µm; mobile phase: [Water (0.1% formic acid)-acetonitrile]; gradient: (acetonitrile): 40%-75%), and dried to give the formate salt of compound 44B. MS m/z = 631.5 [M+H]⁺. ¹H NMR (400 MHz, CD₃OD) *δ* ppm 8.37 (s, 2H), 6.91 (d, J = 8.5 Hz, 1H), 5.40 - 5.18 (m, 3H), 5.17 - 5.12 (m, 1H), 4.91 (d, J = 13.6 Hz, 1H), 4.73 - 4.55 (m, 1H), 4.53 - 4.45 (m, 2H), 4.33 (d, J = 14.3 Hz, 1H), 4.17 - 3.48 (m, 7H), 3.28 - 3.11 (m, 3H), 3.06 - 2.97 (m, 1H), 2.93 - 2.84 (m, 1H), 2.77 (d, J = 16.0 Hz, 1H), 2.55 - 2.30 (m, 2H), 2.25 - 2.06 (m, 3H), 2.03 - 1.81 (m, 4H).

### Example 45

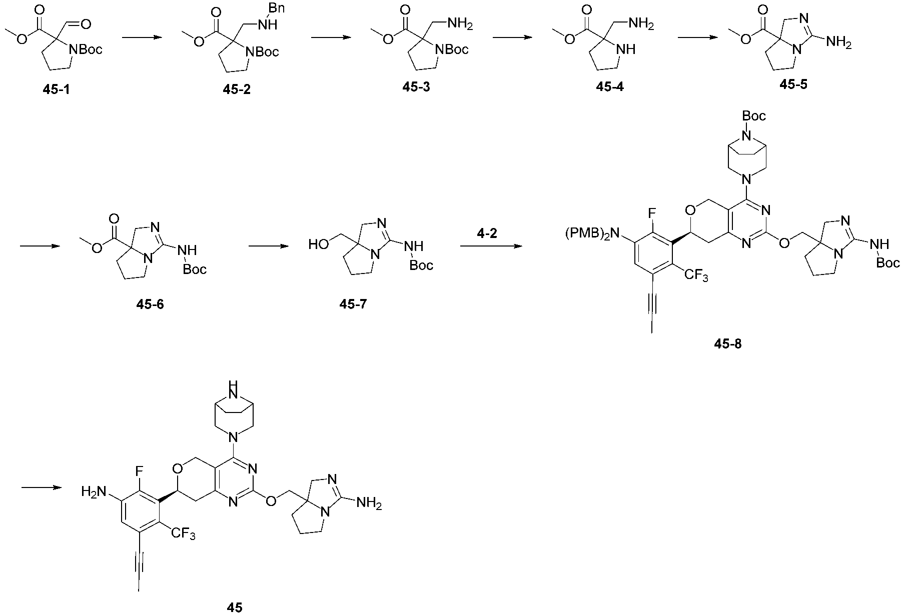

### Step 1

Compound **45-1** (2 g, 7.77 mmol) was dissolved in methanol (20 mL). Benzylamine (832.96 mg, 7.77 mmol), sodium cyanoborohydride (977 mg, 15.55 mmol), and glacial acetic acid (46.68 mg, 0.78 mmol) were added and stirred at room temperature for 16 hours. Water (10 mL) was added to quench the reaction. The product was extracted with ethyl acetate (10 mL * 3), dried over anhydrous sodium sulfate, and filtered. The filtrate was concentrated under reduced pressure, and separated by column chromatography (dichloromethane:methanol = 40:1) to give compound **45-2.** MS m/z = 349.0 [M+H]⁺.

### Step 2

To a solution of compound **45-2** (1.78 g, 5.11 mmol) in methanol (20 mL) was added palladium on carbon (1.5 g, 10% purity). The mixture was reacted at 20°C under a hydrogen atmosphere (15 psi) for 16 hours. The mixture was filtered, and the filtrate was concentrated under reduced pressure to afford compound **45-3.** MS m/z = 259.0 [M+H]⁺.

### Step 3

Compound **45-3** (1 g, 3.87 mmol) was dissolved in a hydrogen chloride/ethyl acetate solution (10 mL, 4 M) and stirred at 25°C for 16 hours. The mixture was concentrated under reduced pressure to obtain compound **45-4.** MS m/z = 159.0 [M+H]⁺.

### Step 4

Compound **45-4** (600 mg, 3.79 mmol) was dissolved in methanol (10 mL), and cyanogen bromide (803.46 mg, 7.59 mmol) and potassium carbonate (1.05 g, 7.59 mmol) were added. The reaction mixture was stirred at 50°C for 16 hours. After filtration, the reaction solution was concentrated under reduced pressure to obtain compound **45-5.** MS m/z = 184.1 [M+H]⁺.

### Step 5

Compound **45-5** (600 mg, 3.27 mmol) was dissolved in dichloromethane (10 mL). Triethylamine (662.79 mg, 6.55 mmol), di-tert-butyl dicarbonate (2.86 g, 13.10 mmol), and 4-dimethylaminopyridine (200.05 mg, 1.64 mmol) were added. The mixture was stirred at 25°C for 16 hours. The reaction solution was concentrated under reduced pressure and separated by column chromatography (dichloromethane:methanol = 40:1) to obtain compound **45-6.** MS m/z = 284.1 [M+H]⁺.

### Step 6

Compound **45-6** (270 mg, 0.95 mmol) was dissolved in anhydrous methanol (3 mL), and cooled to 0°C. Sodium borohydride (33.33 mg, 0.83 mmol) and a solution of sodium methoxide in methanol (17.16 mg, 95.3 µmol, 30%) were added, and the mixture was stirred at room temperature for 8 hours. A saturated aqueous solution of ammonium chloride (10 mL) was added to quench the reaction, and the product was extracted with ethyl acetate (10 mL * 3). The organic phases were combined, washed with saturated brine (20 mL), dried over anhydrous sodium sulfate, and filtered. The filtrate was concentrated under reduced pressure, and separated by column chromatography (petroleum ether:ethyl acetate = 1:1) to obtain compound **45-7.** MS m/z = 256.1 [M+H]⁺.

### Step 7

Compound **45-7** (100 mg, 0.39 mmol) was dissolved in anhydrous tetrahydrofuran (3 mL), and cooled to 0°C. Sodium hydride (31.33 mg, 0.78 mmol, 60% purity) was added. After stirring at 0°C for half an hour, compound **4-2** (344.66 mg, 0.39 mmol) was added and stirred at room temperature for 1 hour. The mixture was quenched with a saturated aqueous solution of ammonium chloride (10 mL), and extracted with ethyl acetate (20 mL * 3). The extracted organic phases were combined and washed with saturated brine (20 mL), dried over anhydrous sodium sulfate, and filtered. The filtrate was concentrated under reduced pressure, and separated by column chromatography (petroleum ether:ethyl acetate = 2:1) to obtain compound **45-8.** MS m/z = 1055.5 [M+H]⁺.

### Step 8

Compound **45-8** (100 mg, 0.94 mmol) was added to trifluoroacetic acid (4 mL) and stirred at 25°C for 1 hour. The reaction solution was concentrated under reduced pressure to obtain a crude product. The crude product was separated by preparative high performance liquid chromatography (Chromatographic column: Xbridge C18 250*19mm, 5µm; mobile phase: [water (0.1% ammonia)-acetonitrile]; (acetonitrile): 55%-80%), and dried to obtain compound **45.** MS m/z = 615.3[M+H]⁺. ¹H NMR (400 MHz, CD₃OD) δ ppm 7.00 (d, *J* = 8.0 Hz, 1H), 5.24 - 5.21 (m, 1H), 5.00 - 4.96 (m, 2H), 4.84 (s, 1H), 4.63 - 4.58 (m, 2H), 4.28-4.21 (m, 2H), 4.11 - 3.92 (m, 3H), 3.75 - 3.72 (m, 1H), 3.66 - 3.56 (m, 2H), 3.49 - 3.35 (m, 2H), 3.05 - 3.00 (m, 1H), 2.26 - 2.15 (m, 6H), 2.02 (m, 5H).

### Example 46

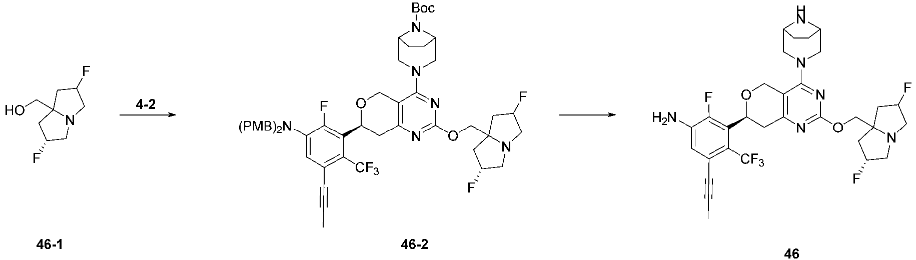

### Step 1

Compound **46-1** (50 mg, 0.28 mmol) was dissolved in tetrahydrofuran (5 mL), and sodium hydride (20 mg, 0.84 mmol, 60% purity) was added. Under nitrogen protection, the reaction was stirred at 25°C for 0.5 hours. Then, a solution of compound **4-2** (243 mg, 0.28 mmol) in tetrahydrofuran (1 mL) was added. After the addition was completed, the reaction was stirred at 25°C for 2 hours. The reaction solution was dissolved in ethyl acetate (10 mL), then washed with saturated brine (5 mL). The organic phase was dried and filtered. The filtrate was concentrated under reduced pressure and purified by column chromatography (dichloromethane:methanol = 20:1) to give compound **46-2.** MS m/z = 977.7 [M+H]⁺.

### Step 4

Compound **46-2** (50 mg, 51.00 µmol) was dissolved in dichloromethane (3 mL), followed by the addition of trifluoroacetic acid (1 mL). Under nitrogen, the reaction mixture was stirred at 25°C for 2 hours. The reaction solution was concentrated under reduced pressure to obtain a crude product. The crude product was separated by preparative HPLC (Chromatographic column: Waters Xbridge, 250*19 mm, 5 µm; mobile phase: [water (0.1% hydrochloric acid)-acetonitrile]; gradient: (acetonitrile): 10%-40%) and dried to obtain the hydrochloride salt of compound **46.** MS m/z = 637.5 [M+H]⁺. ¹H NMR (400 MHz, CD₃OD) *δ* ppm 6.93 (d, *J* = 8.0 Hz, 1H), 5.40 - 5.35 (m, 1H), 5.28 - 5.25 (m, 1H), 5.20 - 5.14 (m, 1H), 4.82 - 4.79 (m, 1H), 4.71 - 4.65 (m, 1H), 4.30 (d, *J* = 8.0 Hz, 1H), 4.27 - 4.18 (m, 2H), 3.98 - 3.79 (m, 2H), 3.64 (d, *J* = 12.0 Hz, 1H), 3.56 (d, *J =* 12.0 Hz, 1H), 3.52 - 3.41 (m, 2H), 3.26-3.24 (m, 1H), 3.23 - 3.08 (m, 3H), 2.91-2.83 (m, 1H), 2.52 - 2.43 (m, 1H), 2.35 - 2.32 (m, 1H), 2.26 - 2.12 (m, 3H), 2.04 (s, 3H), 1.97 - 1.94 (m, 1H), 1.88 - 1.79 (m, 1H), 1.65 - 1.59 (m, 1H).

### Example 47

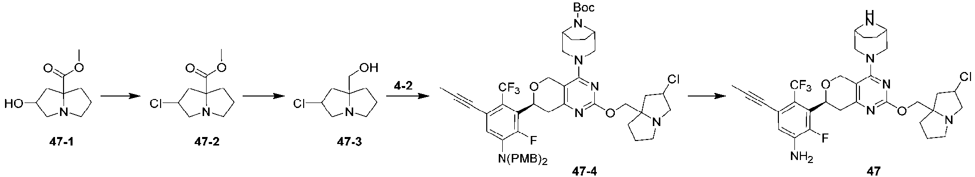

### Step 1

Compound **47-1** (150 mg, 809.85 µmol) was dissolved in thionyl chloride (5 mL), heated to 80°C and refluxed with stirring for 2 hours. After returning to room temperature, the mixture was concentrated under reduced pressure to obtain crude compound **47-2.** MS m/z = 204.1 [M+H]⁺.

### Step 2

Compound **47-2** (40 mg, crude product) was dissolved in anhydrous methanol (3 mL) and the mixture was cooled to 0°C. Sodium borohydride (22.29 mg, 392.8 µmol) and sodium methoxide / methanol solution (7.49 mg, 41.62 µmol, 30%) were added sequentially, and the mixture was stirred at room temperature for 8 hours. The reaction was quenched by the addition of a saturated aqueous solution of ammonium chloride (10 mL). The mixture was extracted with ethyl acetate (20 mL x 3). The organic phases were combined, washed with saturated brine (20 mL), dried over anhydrous sodium sulfate, and filtered. The filtrate was concentrated under reduced pressure, and separated by column chromatography (petroleum ether:ethyl acetate = 1:1) to obtain compound **47-3.** MS m/z = 176.1 [M+H]⁺.

### Step 3

Compound **47-3** (19.96 mg, 113.64 µmol) was dissolved in anhydrous tetrahydrofuran (3 mL), and the mixture was cooled to 0°C. Sodium hydride (4.55 mg, 113.64 µmol, 60%) was added. The mixture was stirred at 0°C for half an hour. Compound **4-2** (50 mg, 56.82 µmol) was added, and the reaction was stirred at room temperature for 1 hour. The reaction was quenched by the addition of a saturated aqueous solution of ammonium chloride (10 mL). The mixture was extracted with ethyl acetate (20 mL x 3). The extracted organic phases were combined and washed with saturated brine (20 mL), dried over anhydrous sodium sulfate, and filtered. The filtrate was concentrated under reduced pressure, and separated by column chromatography (petroleum ether:ethyl acetate = 2:1) to obtain compound **47-4.** MS m/z = 975.7 [M+H]⁺.

### Step 4

Compound **47-4** (40 mg, 41.00 µmol) was added to trifluoroacetic acid (4 mL) and stirred at room temperature for 1 hour. The reaction solution was concentrated under reduced pressure to obtain a crude product. The crude product was separated by preparative HPLC (Chromatographic column: Waters Xbridge, 250 x 19 mm, 5 µm; mobile phase: [water (0.1% ammonia)-acetonitrile]; gradient: (acetonitrile): 55%-70%) and dried to obtain compound **47.** MS m/z = 635.5 [M+H]⁺. ¹H NMR (400 MHz, CD₃OD) δ ppm 6.90 (d, *J* = 8.0 Hz, 1H), 5.15 (dd, *J* = 12.0 Hz, 4.0 Hz, 1H), 4.80 (d, *J* = 16.0 Hz, 1H), 4.66 (d, *J* = 16.0 Hz, 1H), 4.62 - 4.55 (m, 2H), 4.15 (d, *J* = 16.0 Hz, 1H), 4.11 - 4.02 (m, 2H), 3.65 (s, 2H), 3.58 - 3.43 (m, 3H), 3.26 - 3.19 (m, 1H), 3.10 - 2.98 (m, 3H), 2.91 - 2.83 (m, 2H), 2.67 - 2.62 (m, 1H), 2.07 - 1.94 (m, 8H), 1.93 - 1.85 (m, 3H).

### Example 48

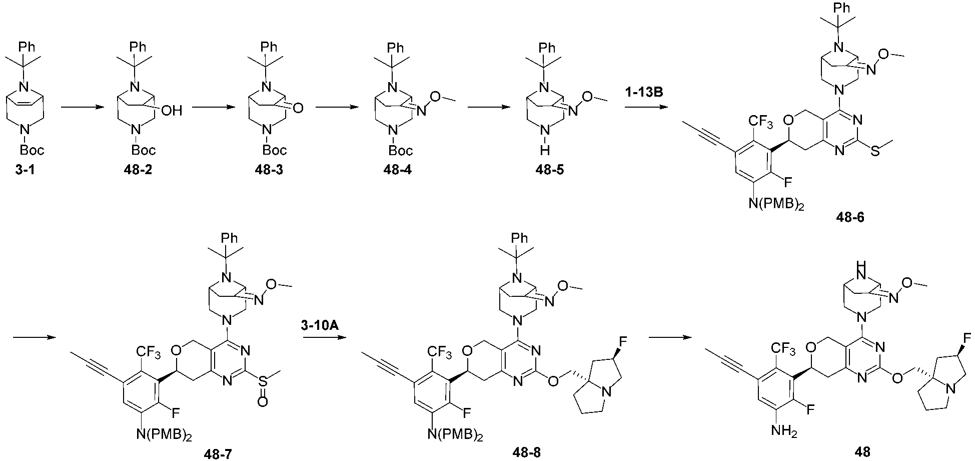

### Step 1

Compound **3-1** (500 mg, 1.52 mmol) was dissolved in tetrahydrofuran (10 mL), and boron trifluoride etherate solution (6.08 mL, 6.08 mmol, 1 M) was added at 0°C. The mixture was heated to 25°C and stirred for 8 hours. The reaction was quenched with 5% sodium hydroxide solution (5 mL) at 0°C and stirred for additional 1 hour. The product was extracted with ethyl acetate (20 mL * 3). The organic phases were combined, washed with saturated brine (20 mL), dried over anhydrous sodium sulfate, and filtered. The filtrate was concentrated under reduced pressure, and separated by column chromatography (petroleum ether:ethyl acetate = 2:1) to obtain compound **48-2.** MS m/z = 347.3 [M+H]⁺.

### Step 2

Compound **48-2** (220 mg, 634.99 µmol) was dissolved in dichloromethane (10 mL), and cooled to 0°C. Dess - Martin periodinane (404 mg, 952.48 µmol) was added. The mixture was stirred at 0°C for 1 hour. Saturated ammonium chloride solution (10 mL) was added to quench the reaction, and the product was extracted with ethyl acetate (20 mL * 3). The organic phases were combined, washed with saturated brine (20 mL), dried over anhydrous sodium sulfate, and filtered. The filtrate was concentrated under reduced pressure, and separated by column chromatography (petroleum ether:ethyl acetate = 5:1) to obtain compound **48-3.** MS m/z = 345.3 [M+H]⁺.

### Step 3

Compound **48-3** (100 mg, 290.32 µmol) was dissolved in anhydrous ethanol (2 mL). Methoxyamine hydrochloride (29.1 mg, 348.38 µmol) and pyridine (34.5 mg, 435.48 µmol) were added. The mixture was stirred at 25°C for 2 hours. The mixture was concentrated under reduced pressure, and dichloromethane (10 mL) was added to dissolve the mixture. The mixture was washed with 2M aqueous hydrochloric acid solution (10 mL) and saturated brine (10 mL) in sequence, dried over anhydrous sodium sulfate, filtered, and concentrated under reduced pressure to obtain compound **48-4.** MS m/z = 374.4 [M+H]⁺.

### Step 4

Compound **48-4** (80 mg, 214.20 µmol) was dissolved in a hydrochloric acid - ethyl acetate solution (4 M, 2 mL) and stirred at 25°C for 1 hour. The mixture was concentrated under reduced pressure to obtain compound **48-5.** MS m/z = 274.2 [M+H]⁺.

### Step 5

Compound **48-5** (50 mg, 182.90 µmol) was dissolved in N,N-dimethylformamide (2 mL). **1-13B** (143.7 mg, 182.90 µmol) and N,N-diisopropylethylamine (118.2 mg, 914.50 µmol) were added, and the mixture was heated to 100°C and stirred for 2 hours. After returning to room temperature, water (5 mL) was added to quench the reaction. The mixture was extracted with ethyl acetate (10 mL x 3). The extracted organic phases were combined and washed with saturated brine (5 mL), dried over anhydrous sodium sulfate, filtered, and concentrated under reduced pressure. Column chromatography (petroleum ether:ethyl acetate = 10:1) afforded compound **48-6.** MS m/z = 909.6 [M+H]⁺.

### Step 6

Compound **48-6** (135 mg, 148.51 µmol) was dissolved in anhydrous tetrahydrofuran (2 mL), and m-chloroperbenzoic acid (33.2 mg, 163.36 µmol) was added. The mixture was stirred at 25°C for 1 hour. The mixture was concentrated under reduced pressure to obtain crude compound **48-7.** MS m/z = 925.5 [M+H]⁺.

### Step 7

Compound **3-10A** (25.6 mg, 160.53 µmol) was dissolved in anhydrous tetrahydrofuran (2 mL). Sodium hydride (6.4 mg, 160.53 µmol, 60%) was added at 0°C and the mixture was heated to 25°C with stirring for 0.5 hours. Compound **48-7** (135 mg, crude) was added and the mixture was stirred at 25°C for additional hour. The reaction was quenched by adding 5 mL of water and extracted with ethyl acetate (10 mL x 3). The extracted organic phases were combined and washed with saturated brine (10 mL), dried over anhydrous sodium sulfate, filtered, and concentrated under reduced pressure. Column chromatography (dichloromethane:methanol = 10:1) afforded compound **48-8.** MS m/z = 1020.8 [M+H]⁺.

### Step 8

Compound **48-8** (60 mg, 58.82 µmol) was dissolved in dichloromethane (1.5 mL). Trifluoroacetic acid (0.5 mL) was added, and the reaction was stirred at 25°C for 2 hours. The mixture was concentrated under reduced pressure to obtain a crude product. The crude product was separated by preparative HPLC (Chromatographic column: Xtimate C18 150*40mm, 5µm; mobile phase: [water (0.05% formic acid)-acetonitrile]; (acetonitrile): 10%-50%) and dried to obtain the formate salt of compound **48.** MS m/z = 662.5 [M+H]⁺. ¹H NMR (400 MHz, CD₃OD) δ ppm 8.44 (s, 0.41H), 6.91 (d, *J* = 8.0 Hz, 1H), 5.47 (d, *J* = 52.0 Hz, 1H), 5.16 - 5.11 (m, 1H), 4.77 - 4.72 (m, 1H), 4.66 - 4.55 (m, 1H), 4.40 (dd, *J* = 12.0 Hz, 4.0 Hz, 1H), 4.31 (d, *J* = 12.0 Hz, 1H), 4.14 - 4.00 (m, 1H), 3.88 - 3.77 (m, 4H), 3.76 - 3.63 (m, 5H), 3.55 - 3.42 (m, 1H), 3.27 - 3.04 (m, 3H), 2.87 (dd, *J* = 20.0 Hz, 4.0 Hz, 1H), 2.72 - 2.54 (m, 2H), 2.49 - 2.38 (m, 2H), 2.29 - 2.16 (m, 3H), 2.09 - 2.01 (m, 4H).

### Example 49

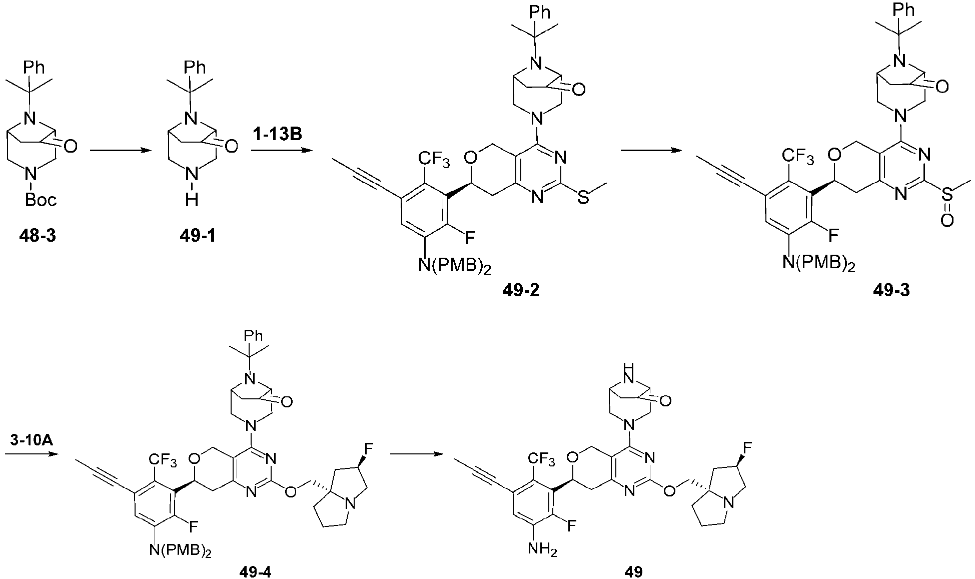

### Step 1

Compound **48-3** (60 mg, 174.19 µmol) was dissolved in dichloromethane (5 mL), and hydrochloric acid / ethyl acetate solution (5 mL, 4 M) was added at 25°C. The mixture was stirred for 2 hours, and concentrated under reduced pressure to obtain compound **49-1.** MS m/z = 245.2 [M+H]⁺.

### Step 2

Compound **49-1** (40 mg, 163.71 µmol) was dissolved in anhydrous N,N-dimethylformamide (5 mL). N,N-diisopropylethylamine (105.8 mg, 818.56 µmol) and **1-13B** (128.6 mg, 163.71 µmol) were added at 25°C. The mixture was heated to 80°C and stirred for 1 hour. After returning to room temperature, the reaction was quenched with saturated aqueous ammonium chloride (10 mL). The mixture was extracted with ethyl acetate (20 mL x 3). The extracted organic phases were combined and washed with saturated brine (20 mL), dried over anhydrous sodium sulfate, filtered, and concentrated under reduced pressure. Column chromatography (petroleum ether:ethyl acetate = 2:1) afforded compound **49-2.** MS m/z = 880.7 [M+H]⁺.

### Step 3

Compound **49-2** (120 mg, 136.36 µmol) was dissolved in dichloromethane (5 mL), and m-chloroperbenzoic acid (47.1 mg, 272.73 µmol) was added. The mixture was stirred at 25°C for 1 hour. A saturated aqueous solution of ammonium chloride (10 mL) was added to quench the reaction, and the product was extracted with ethyl acetate (20 mL * 3). The organic phases were combined, washed with saturated brine (20 mL), dried over anhydrous sodium sulfate, and filtered. The filtrate was concentrated under reduced pressure, and separated by column chromatography (petroleum ether:ethyl acetate = 1:1) to obtain compound **49-3.** MS m/z = 896.3 [M+H]⁺.

### Step 4

Compound **3-10A** (41.9 mg, 263.16 µmol) was dissolved in anhydrous tetrahydrofuran (3 mL). Sodium hydride (5.8 mg, 394.74 µmol, 60%) was added at 0°C, and the mixture was stirred for half an hour. Compound **49-3** (120 mg, 133.88 µmol) was then added and the mixture was stirred at room temperature for 1 hour. The mixture was quenched by adding a saturated aqueous solution of ammonium chloride (10 mL) and extracted with ethyl acetate (20 mL x 3). The organic phases were combined, washed with saturated brine (20 mL), dried over anhydrous sodium sulfate, and filtered. The filtrate was concentrated under reduced pressure, and separated by column chromatography (petroleum ether:ethyl acetate = 1:1) to obtain compound **49-4.** MS m/z = 991.8 [M+H]⁺.

### Step 5

Compound **49-4** (120 mg, 121.08 µmol) was added to trifluoroacetic acid (4 mL) and stirred at 25°C for 1 hour. The reaction solution was concentrated under reduced pressure to obtain a crude product. The crude product was separated by preparative HPLC (Chromatographic column: Waters Xbridge, 250*19 mm, 5 µm; mobile phase: [water (0.1% ammonia)-acetonitrile]; gradient: (acetonitrile): 55%-70%) and dried to obtain compound **49.** MS m/z = 633.5 [M+H]⁺. ¹H NMR (400 MHz, CD₃OD) δ ppm 6.91 (d, *J* = 8.0 Hz, 1H), 5.45 (d, *J* = 52.0 Hz, 1H), 5.15 (dd, *J* = 12.0 Hz, 4.0 Hz, 1H), 4.80 - 4.75 (m, 2H), 4.58 (dd, *J* = 32.0 Hz, 16.0 Hz, 1H), 4.42 - 4.35 (m, 1H), 4.30 - 4.25 (m, 1H), 4.23 - 4.00 (m, 1H), 3.95 - 3.92 (m, 1H), 3.84 - 3.56 (m, 5H), 3.48 - 3.33 (m, 2H), 3.28 - 3.10 (m, 3H), 2.90 - 2.84 (m, 1H), 2.55 - 2.38 (m, 3H), 2.28 - 2.15 (m, 3H), 2.01 (s, 3H).

### Example 50

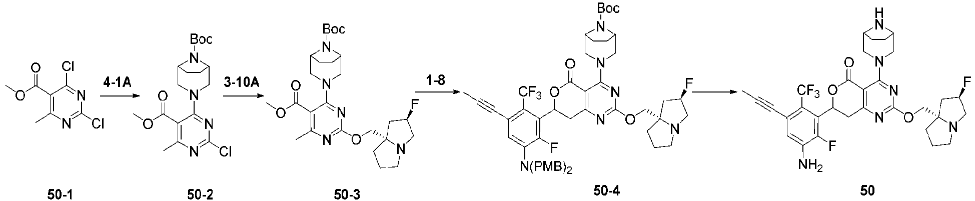

### Step 1

**50-1** (1.8 g, 8.14 mmol) was dissolved in ethanol (30 mL). N,N-diisopropylethylamine (2.1 g, 16.29 mmol, 2.84 mL) and compound **4-1A** (1.82 g, 8.55 mmol) were added. The mixture was stirred at 25°C for 1 hour. Water (10 mL) was added to quench the reaction, and the mixture was extracted with ethyl acetate (20 mL * 3). The organic phases after the extraction were combined, washed with saturated brine (10 mL * 2), dried over anhydrous sodium sulfate, filtered, and concentrated under reduced pressure. Column chromatography (dichloromethane:ethyl acetate = 10:1) obtained compound **50-2.** MS m/z = 397.1 [M+H]⁺.

### Step 2

Compound **50-2** (100 mg, 251.97 µmol) and compound **3-10A** (72.21 mg, 453.55 µmol) were dissolved in ultra-dry tetrahydrofuran (3 mL), and sodium hydride (29.02 mg, 1.21 mmol, 60% purity) was added at 0°C. The mixture was heated to 25°C and stirred for 2 hours. Water (2 mL) was added to quench the reaction, and the mixture was extracted with ethyl acetate (10 mL x 2). The extracted organic phases were combined and washed with saturated brine (5 mL x 2), dried over anhydrous sodium sulfate, filtered, and concentrated under reduced pressure. Column chromatography (petroleum ether:ethyl acetate = 1:2) afforded compound **50-3.** MS m/z = 520.5 [M+H]⁺.

### Step 3

Compound **50-3** (100 mg, 192.45 µmol) was dissolved in ultra-dry tetrahydrofuran (3 mL) and lithium diisopropylamide (24.74 mg, 230.94 µmol, 2.0 M tetrahydrofuran solution) was slowly added dropwise at -78°C. The mixture was stirred at -78°C for 40 minutes. **1-8** was dissolved in ultra-dry tetrahydrofuran (1 mL) and slowly added dropwise to the above system at -78°C. The mixture was heated to 0°C and stirred for 1 hour. The mixture was quenched by adding water (10 mL) and extracted with ethyl acetate (20 mL * 3). The organic phases after extraction were combined, washed with saturated brine (10 mL * 2), dried over anhydrous sodium sulfate, and filtered. The filtrate was concentrated under reduced pressure, and purified by preparative thin-layer chromatography (petroleum ether:ethyl acetate = 1:1) to obtain compound **50-4.** MS m/z = 973.7 [M+H]⁺.

### Step 4

Compound **50-4** (85 mg, 87.36 µmol) was dissolved in dichloromethane (3 mL). Trifluoroacetic acid (1 mL) was added, and the mixture was stirred at 25°C for 0.5 hours. The reaction solution was concentrated under reduced pressure to obtain a crude product. The crude product was separated by preparative HPLC (Chromatographic column: Waters SunFire, 250*19mm, 5 µm; mobile phase: [water (0.1% formic acid)-acetonitrile]; gradient: (acetonitrile): 40%-75%), and dried to give the formate salt of compound **50.** MS m/z = 633.5 [M+H]⁺. ¹H NMR (400 MHz, CD₃OD) δ ppm 8.48 (s, 0.50 H), 6.98 (d, *J* = 8.4 Hz, 1H), 5.99 (d, *J* = 12.8 Hz, 1H), 5.39 (d, *J =* 52.0 Hz, 1H), 4.98 - 4.95 (m, 1H), 4.39 - 4.26 (m, 2H), 4.08 (s, 1H), 3.96 (s, 1H), 3.76 - 3.72 (m, 1H), 3.63 - 3.53 (m, 2H), 3.49 - 3.34 (m, 4H), 3.18 - 3.12 (m, 1H), 3.02 (dd, *J* = 20.0 Hz, 4.0 Hz, 1H), 2.42 - 2.18 (m, 4H), 2.11 - 2.06 (m, 2H), 2.02 - 1.94 (m, 6H), 1.86 - 1.78 (m, 1H).

### Example 51

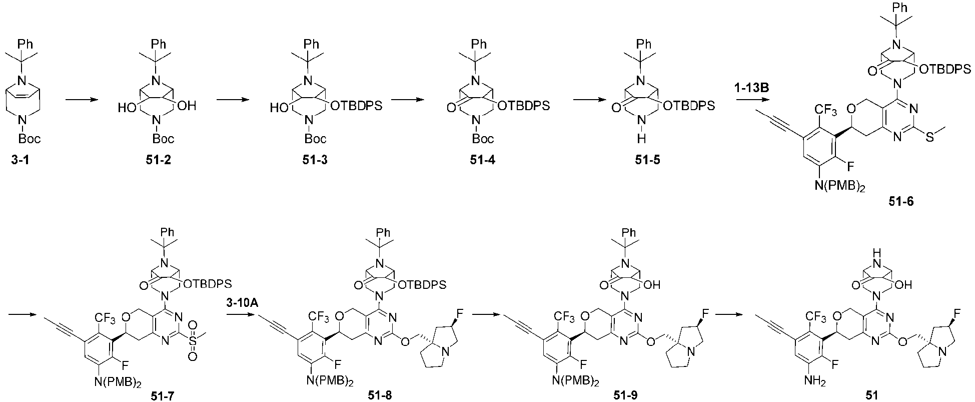

### Step 1

Compound **3-1** (1 g, 3.04 mmol) was dissolved in acetonitrile (10 mL) and water (2 mL). Potassium osmate dihydrate (26.70 mg, 91.34 µmol) and N-methylmorpholine oxide (713.36 mg, 6.09 mmol) were added, and the mixture was stirred at room temperature for 16 hours. The reaction was quenched with a saturated aqueous solution of ammonium chloride (10 mL). The mixture was extracted with ethyl acetate (20 mL x 3). The extracted organic phases were combined and washed with saturated brine (20 mL), dried over anhydrous sodium sulfate, and filtered. The filtrate was concentrated under reduced pressure, and separated by column chromatography (petroleum ether:ethyl acetate = 5:1) to provide compound **51-2.** MS m/z = 363.4 [M+H]⁺.

### Step 2

Compound **51-2** (600 mg, 1.66 mmol) was dissolved in dichloromethane (10 mL). Imidazole (338.07 mg, 4.97 mmol) and tert-butyldiphenylsilyl chloride (545.99 mg, 1.99 mmol) were added and stirred at room temperature for 3 hours. The reaction was quenched with a saturated aqueous solution of ammonium chloride (10 mL) and extracted with ethyl acetate (20 mL x 3). The extracted organic phases were combined and washed with saturated brine (20 mL), dried over anhydrous sodium sulfate, and filtered. The filtrate was concentrated under reduced pressure, and separated by column chromatography (petroleum ether:ethyl acetate = 10:1) to afford compound **51-3.** MS m/z = 601.6 [M+H]⁺.

### Step 3

Compound **51-3** (300 mg, 499.28 µmol) was dissolved in dichloromethane (10 mL), and cooled to 0°C. Dess-Martin periodinane (317.65 mg, 748.93 µmol) was added and stirred for 2 hours. A saturated aqueous solution of ammonium chloride (10 mL) was added to quench the reaction, and the mixture was extracted with ethyl acetate (20 mL * 3). The extracted organic phases were combined and washed with saturated brine (20 mL), dried over anhydrous sodium sulfate, and filtered. The filtrate was concentrated under reduced pressure, and separated by column chromatography (petroleum ether:ethyl acetate = 10:1) to obtain compound **51-4.** MS m/z = 599.5 [M+H]⁺.

### Step 4

Compound **51-4** (100 mg, 166.99 µmol) was dissolved in dichloromethane (10 mL). Zinc bromide (45.13 mg, 200.39 µmol) was added at room temperature and stirred at room temperature for 2 hours. The reaction solution was filtered, and the filter cake was washed three times with dichloromethane (20 mL). The filtrates were combined and concentrated under reduced pressure to obtain compound **51-5.** MS m/z = 499.5 [M+H]⁺.

### Step 5

Compound **51-5** (80 mg, 160.41 µmol) was dissolved in anhydrous N,N-dimethylformamide (5 mL). N,N-diisopropylethylamine (103.66 mg, 802.04 µmol) and **1-13B** (126.04 mg, 160.41 µmol) were added at room temperature. The mixture was heated to 80°C and stirred for 1 hour. A saturated aqueous solution of ammonium chloride (10 mL) was added to quench the reaction, and the mixture was extracted with ethyl acetate (20 mL * 3). The extracted organic phases were combined and washed with saturated brine (20 mL), dried over anhydrous sodium sulfate, and filtered. The filtrate was concentrated under reduced pressure, and separated by column chromatography (petroleum ether:ethyl acetate = 2:1) to obtain compound **51-6.** MS m/z = 1134.8 [M+H]⁺.

### Step 6

Compound **51-6** (120 mg, 105.78 µmol) was dissolved in dichloromethane (5 mL). m-Chloroperbenzoic acid (53.69 mg, 264.46 µmol) was added at room temperature and the mixture was stirred for 1 hour. A saturated aqueous solution of ammonium chloride (10 mL) was added to quench the reaction, and the mixture was extracted with ethyl acetate (20 mL * 3). The extracted organic phases were combined and washed with saturated brine (20 mL), dried over anhydrous sodium sulfate, and filtered. The filtrate was concentrated under reduced pressure, and separated by column chromatography (petroleum ether:ethyl acetate = 1:1) to obtain compound **51-7.** MS m/z = 1166.8 [M+H]⁺.

### Step 7

Compound **3-10A** (40.95 mg, 257.20 µmol) was dissolved in anhydrous tetrahydrofuran (3 mL), and cooled to 0°C. Sodium hydride (10.29 mg, 257.20 µmol, 60% purity) was added. The mixture was stirred at 0°C for half an hour. Compound **51-7** (120 mg, 102.88 µmol) was added, and the reaction was stirred at room temperature for 1 hour. The reaction was quenched by the addition of a saturated aqueous solution of ammonium chloride (10 mL). The mixture was extracted with ethyl acetate (20 mL x 3). The extracted organic phases were combined and washed with saturated brine (20 mL), dried over anhydrous sodium sulfate, and filtered. The filtrate was concentrated under reduced pressure, and separated by column chromatography (petroleum ether:ethyl acetate = 1:1) to obtain compound **51-8.** MS m/z = 1246.0 [M+H]⁺.

### Step 8

Compound **51-8** (85 mg, 68.25 µmol) was dissolved in anhydrous tetrahydrofuran (5 mL). Tetrabutylammonium fluoride (0.34 mL, 341.23 µmol) was added at room temperature, and the mixture was reacted by stirring for 1 hour. A saturated aqueous solution of ammonium chloride (10 mL) was added to quench the reaction, and the mixture was extracted with ethyl acetate (20 mL * 3). The extracted organic phases were combined and washed with saturated brine (20 mL), dried over anhydrous sodium sulfate, and filtered. The filtrate was concentrated under reduced pressure, and separated by column chromatography (petroleum ether:ethyl acetate = 1:1) to obtain compound **51-9.** MS m/z = 1007.8 [M+H]⁺.

### Step 9

Compound **51-9** (80 mg, 79.44 µmol) was added to trifluoroacetic acid (4 mL) and stirred at room temperature for 1 hour. The reaction solution was concentrated under reduced pressure to obtain a crude product. The crude product was separated by preparative HPLC (Chromatographic column: Waters Xbridge, 250 x 19 mm, 5 µm; mobile phase: [water (0.1% ammonia)-acetonitrile]; gradient: (acetonitrile): 55%-70%) and dried to obtain compound **51.** MS m/z = 649.5 [M+H]⁺. ¹H NMR (400 MHz, CD₃OD) δ ppm 6.96 (d, *J* = 8.0 Hz, 1H), 5.60 (d, *J* = 52.0 Hz, 1H), 5.42 - 5.30 (m, 1H), 5.23 - 5.13 (m, 1H), 4.96 - 4.90 (m, 2H), 4.28 - 3.82 (m, 7H), 3.75 - 3.60 (m, 1H), 3.48 - 3.38 (m, 2H), 3.13 - 3.07 (m, 1H), 2.86 - 2.70 (m, 1H), 2.67 - 2.53 (m, 2H), 2.34 - 2.23 (m, 4H), 2.06 - 2.02 (m, 5H).

### Example 52

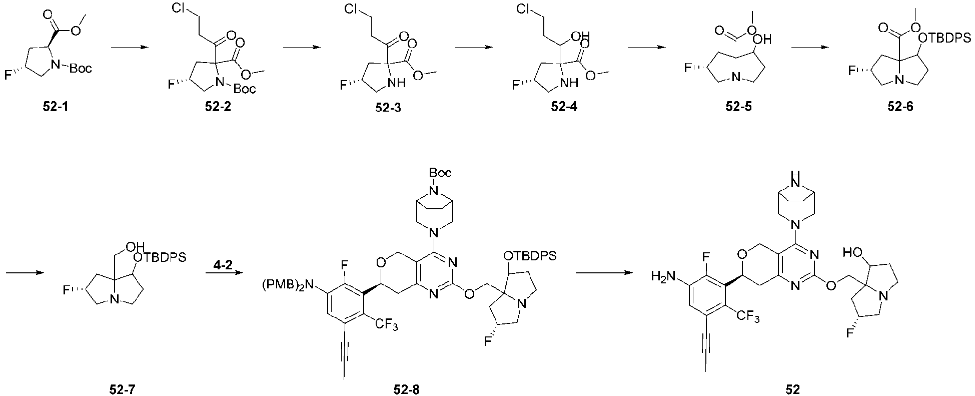

### Step 1

Compound **52-1** (500 mg, 2.02 mmol) was weighed and anhydrous tetrahydrofuran (15 mL) was added. The mixture was purged with nitrogen and cooled to -70°C in a dry ice acetone bath. Lithium bis(trimethylsilyl)amide (2.02 mL, 2.02 mmol, 1 M solution in tetrahydrofuran) was added and stirred at -70°C for 30 minutes. A solution of chloropropionyl chloride (384.81 mg, 3.03 mmol) in anhydrous tetrahydrofuran (2 mL) was added and stirred at -70°C for 15 minutes. The reaction was quenched by adding saturated aqueous ammonium chloride (10 mL). The mixture was extracted with ethyl acetate (20mL*3). The organic phases were combined and concentrated under reduced pressure. The crude product was separated by column chromatography (petroleum ether:ethyl acetate = 3:1) to obtain compound **52-2.** MS m/z = 338.1 [M+H]⁺.

### Step 2

Compound **52-2** (50 mg, 0.15 mmol) was weighed and dichloromethane (3 mL) was added. The temperature was lowered to 0°C, and trifluoroacetic acid (0.5 mL) was added. The mixture was allowed to react at room temperature for 15 minutes. The reaction solution was directly concentrated to obtain compound **52-3.** MS m/z = 238.1 [M+H]⁺

### Step 3

Compound **52-3** (50 mg, 0.22 mmol) was weighed and methanol (3 mL) was added. Sodium borohydride (41.80 mg, 1.10 mmol) was added under ice-cooling and stirred at room temperature for 1 hour. The reaction was quenched by adding 1N hydrochloric acid (1 mL). The reaction solution was directly concentrated under reduced pressure and separated by column chromatography (dichloromethane:methanol = 30:1) to obtain compound **52-4.** MS m/z = 240.1 [M+H]⁺.

### Step 4

Compound **52-4** (100 mg, 0.42 mmol) was weighed and acetonitrile (4 mL) was added. Potassium carbonate (115.92 mg, 0.84 mmol) was added at room temperature and stirred at 60°C for 16 hours. The reaction solution was directly concentrated under reduced pressure and separated by column chromatography (dichloromethane:methanol = 20:1) to obtain compound **52-5.** MS m/z = 204.2 [M+H]⁺.

### Step 5

Compound **52-5** (50 mg, 0.25 mmol) was weighed, and dichloromethane (3 mL) was added. tert-Butyldiphenylsilyl chloride (137.43 mg, 0.50 mmol) and imidazole (68 mg, 1.00 mmol) were added, and 4-dimethylaminopyridine (15.86 mg, 0.13 mmol) was then added. The mixture was stirred at 40°C for 16 hours. The reaction mixture was diluted with water (10 ml) and extracted with dichloromethane (10 ml x 3). The organic phase was dried and concentrated under reduced pressure. Column chromatography (dichloromethane:methanol = 30:1) afforded compound **52-6.** MS m/z = 442.2 [M+H]⁺.

### Step 6

Compound **52-6** (50 mg, 0.11 mmol) was weighed and anhydrous tetrahydrofuran (3 mL) was added under nitrogen. The mixture was cooled to 0°C, and lithium aluminum tetrahydride (0.10 mL, 0.22 mmol, 2.5 M solution in tetrahydrofuran) was added. The reaction was allowed to react at room temperature for 15 minutes. Water (0.2 mL) was added to the reaction mixture at 0°C, and 15% sodium hydroxide solution (0.2 mL) was added to quench the reaction. The mixture was stirred for 10 minutes, and filtered. The filter cake was washed with tetrahydrofuran (5 mL). The filtrate was concentrated to obtain compound 52-7. MS m/z = 414.2 [M+H]⁺.

### Step 7

Compound **52-7** (90 mg, 0.22 mmol) was weighed and anhydrous tetrahydrofuran (5 mL) was added. 60% sodium hydride (15.91 mg, 0.66 mmol) was added under ice-cooling. The mixture was stirred at room temperature for 30 minutes. Compound **4-2** (195.41 mg, 0.22 mmol) was added and allowed to react at room temperature for 1 hour. The reaction mixture was quenched with water (5 mL) and extracted with ethyl acetate (10 mL x 3). The organic phase was concentrated under reduced pressure and separated by column chromatography (dichloromethane:methanol = 20:1) to obtain compound **52-8.** MS m/z = 1213.5 [M+H]⁺.

### Step 8

Compound **52-8** (60 mg, 0.05 mmol) was weighed and dichloromethane (2 mL) was added. Trifluoroacetic acid (1 mL) was added and the mixture was stirred at room temperature for 0.5 hours. The reaction solution was concentrated under reduced pressure to obtain a crude product, which was then separated by preparative HPLC (column: Waters Xbridge, 250*19 mm, 5 µm; mobile phase: [water (0.1% formic acid)-acetonitrile]; gradient: (acetonitrile): 10%-40%) to afford the formate salt of compound **52.** MS m/z = 635.5 [M+H]⁺. ¹H NMR (400 MHz, CD₃OD) *δ* ppm 8.39 (s, 0.91H), 6.91 (d, *J* = 8.0 Hz, 1H), 5.47 - 5.27 (m, 1H), 5.22 - 5.14 (m, 1H), 4.75 - 4.50 (m, 2H), 4.44 - 4.39 (m, 1H), 4.31 - 4.20 (m, 2H), 4.10 (d, *J* = 8.0 Hz, 2H), 3.92 - 3.76 (m, 2H), 3.65 - 3.58 (m, 2H), 3.45 - 3.35 (m, 1H), 3.30 - 3.15 (m, 3H), 3.02 - 2.88 (m, 2H), 2.61 (t, *J* = 16.0 Hz, 1H), 2.34 - 2.03 (m, 5H), 2.02 (s, 3H), 2.00 - 1.86 (m, 2H).

### Example 53

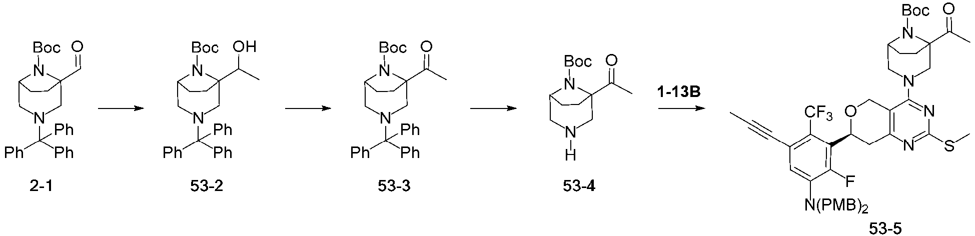

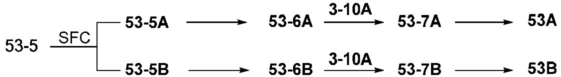

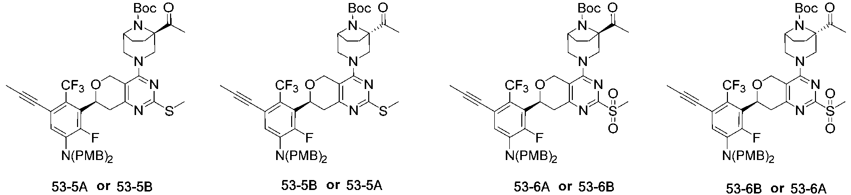

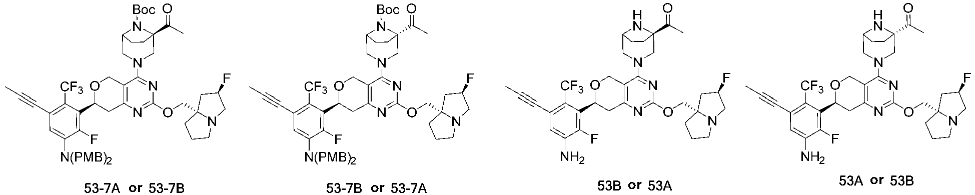

### Step 1

Compound **2-1** (200 mg, 414.41 µmol) was dissolved in tetrahydrofuran (10 mL). Methylmagnesium bromide solution (186 µL, 1.86 mmol, 1 M solution in tetrahydrofuran) was added dropwise at -60°C under nitrogen protection. After the addition was completed, the temperature was slowly raised to room temperature and the mixture was stirred for 1 hour. A saturated aqueous solution of ammonium chloride (10 mL) was added to quench the reaction, and the product was extracted with ethyl acetate (20 mL * 3). The extracted organic phases were combined and washed with saturated brine (20 mL), dried over anhydrous sodium sulfate, and filtered. The filtrate was concentrated under reduced pressure, and separated by column chromatography (petroleum ether:ethyl acetate = 10:1) to obtain compound **53-2.** MS m/z = 499.4 [M+H]⁺.

### Step 2

Compound **53-2** (230 mg, 461.24 µmol) was dissolved in dichloromethane (10 mL), and cooled to 0°C. Dess-Martin periodinane (391.26 mg, 922.48 µmol) was added. The reaction was stirred at 0°C for 2 hours. The reaction was quenched by the addition of a saturated aqueous solution of ammonium chloride (10 mL). The product was extracted with ethyl acetate (20 mL x 3). The extracted organic phases were combined and washed with saturated brine (20 mL), dried over anhydrous sodium sulfate, and filtered. The filtrate was concentrated under reduced pressure, and separated by column chromatography (petroleum ether:ethyl acetate = 10:1) to afford compound **53-3.** MS m/z = 497.4 [M+H]⁺.

### Step 3

Compound **53-3** (100 mg, 201.35 µmol) was dissolved in dichloromethane (5 mL). A hydrochloric acid/ethyl acetate solution (5 mL, 4 M) was added at room temperature and the reaction was stirred for 2 hours. The mixture was concentrated under reduced pressure to afford compound **53-4.** MS m/z = 255.2 [M+H]⁺.

### Step 4

Compound **53-4** (50 mg, 196.60 µmol) was dissolved in anhydrous N,N-dimethylformamide (5 mL). N,N-diisopropylethylamine (127.04 mg, 983.00 µmol) and **1-13B** (185.37 mg, 235.92 µmol) were added at room temperature. The mixture was heated to 80°C and the reaction was stirred for 1 hour. A saturated aqueous solution of ammonium chloride (10 mL) was added to quench the reaction, and the mixture was extracted with ethyl acetate (20 mL * 3). The organic phases after the extraction were combined, washed with saturated brine (20 mL), dried over anhydrous sodium sulfate, and filtered. The filtrate was concentrated under reduced pressure, and separated by column chromatography (petroleum ether:ethyl acetate = 2:1) to obtain compound **53-5.** Compound **53-5** was further subjected to preparative SFC separation (Chromatographic column: DAICEL CHIRALPAK^{®} IG-10, 25*250mm·10µm; mobile phase: A: supercritical carbon dioxide; B: ethanol + ammonia methanol (0.05%); B%: 40%) to give compound **53-5A** and compound **53-5B.** Analytical SFC method (Chromatographic column: DAICEL CHIRALPAK^{®} IG-10, 4.6 * 150 mm. 5 µm; mobile phase: A: supercritical carbon dioxide; mobile phase B: ethanol + diethylamine (0.05%); B%: 40%), compound **53-5A,** Rt = 3.202 minutes, MS m/z = 890.8 [M+H]⁺; compound **53-5B,** Rt = 7.309 minutes, MS m/z = 890.8 [M+H]⁺.

### Step 5

Compound **53-5A** (40 mg, 44.94 µmol) was dissolved in dichloromethane (5 mL). Metachloroperbenzoic acid (19.39 mg, 112.36 µmol) was added at room temperature and stirred for 1 hour. The reaction was quenched by the addition of a saturated aqueous solution of ammonium chloride (10 mL). The mixture was extracted with ethyl acetate (20 mL x 3). The extracted organic phases were combined and washed with saturated brine (20 mL), dried over anhydrous sodium sulfate, and filtered. The filtrate was concentrated under reduced pressure, and separated by column chromatography (petroleum ether:ethyl acetate = 1:1) to obtain compound **53-6A.** MS m/z = 922.7 [M+H]⁺.

### Step 6

Compound **3-10A** (17.27 mg, 108.46 µmol) was dissolved in anhydrous tetrahydrofuran (3 mL), and cooled to 0°C. Sodium hydride (4.34 mg, 108.46 µmol, 60% purity) was added. The mixture was stirred at 0°C for half an hour, followed by the addition of compound **53-6A** (40 mg, 43.38 µmol). The mixture was heated to 25°C and the reaction was stirred for 1 hour. The reaction was quenched by the addition of a saturated aqueous solution of ammonium chloride (10 mL). The mixture was extracted with ethyl acetate (20 mL x 3). The extracted organic phases were combined and washed with saturated brine (20 mL), dried over anhydrous sodium sulfate, and filtered. The filtrate was concentrated under reduced pressure, and separated by column chromatography (petroleum ether:ethyl acetate = 1:1) to afford compound **53-7A.** MS m/z = 1001.8 [M+H]⁺.

### Step 7

Compound **53-7A** (20 mg, 19.98 µmol) was added to trifluoroacetic acid (4 mL) and stirred at room temperature for 1 hour. The reaction solution was concentrated under reduced pressure to obtain a crude product. The crude product was separated by preparative HPLC (Chromatographic column: Waters Xbridge, 250 x 19 mm, 5 µm; mobile phase: water (0.1% formic acid)-acetonitrile; gradient: acetonitrile: 55%-70%), and dried to obtain the formate salt of compound **53A.** MS m/z = 661.5 [M+H]⁺. ¹H NMR (400 MHz, CD₃OD) δ ppm 8.31 (s, 1.56H), 6.92 (d, *J* = 8.0 Hz, 1H), 5.55 (d, *J* = 52.0 Hz, 1H), 5.20 (dd, *J =* 12.0 Hz, 4.0 Hz, 1H), 4.81 - 4.70 (m, 2H), 4.52 - 4.48 (m, 2H), 3.97 - 3.77 (m, 4H), 3.68 - 3.51 (m, 2H), 3.45 - 3.32 (m, 2H), 3.29 - 3.24 (m, 1H), 3.11 - 3.08 (m, 1H), 2.92 (dd, *J* = 20.0 Hz, 4.0 Hz, 1H), 2.72 - 2.66 (m, 1H), 2.59 - 2.44 (m, 2H), 2.38 (s, 3H), 2.34 - 2.28 (m, 2H), 2.21 - 2.07 (m, 3H), 2.02 (s, 3H), 1.96 - 1.78 (m, 2H).

### Step 8

Referring to step **5-7,** compound **53-5B** was used as a starting material in place of compound **53-5A** to finally give the formate salt of compound **53B.** MS m/z = 661.5 [M+H]⁺. ¹H NMR (400 MHz, CD₃OD) δ ppm 8.16 (s, 0.87H), 6.92 (d, *J* = 8.0 Hz, 1H), 5.57 (d, *J* = 52.0 Hz, 1H), 5.24 - 5.20 (m, 1H), 4.81 - 4.73 (m, 2H), 4.58 - 4.51 (m, 2H), 4.15 - 3.96 (m, 3H), 3.91 - 3.68 (m, 4H), 3.53 - 3.41 (m, 2H), 3.27 - 3.17 (m, 1H), 2.97 - 2.92 (m, 1H), 2.74 - 2.69 (m, 1H), 2.63 - 2.51 (m, 2H), 2.39 - 2.29 (m, 6H), 2.25 - 2.17 (m, 3H), 2.14 - 2.04 (m, 1H), 2.02 (s, 3H).

### Example 54

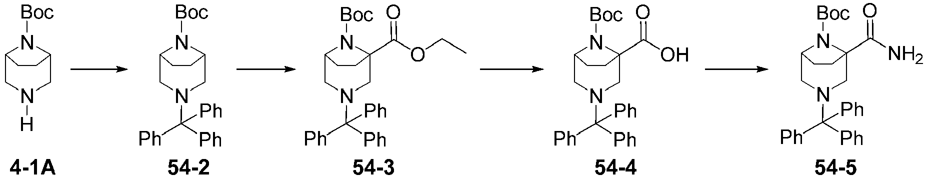

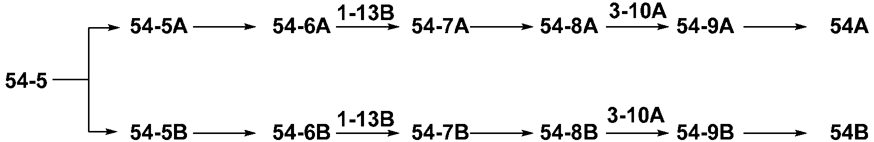

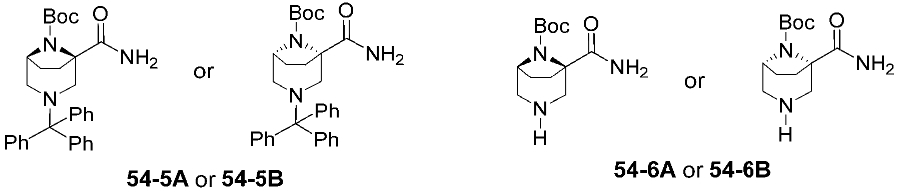

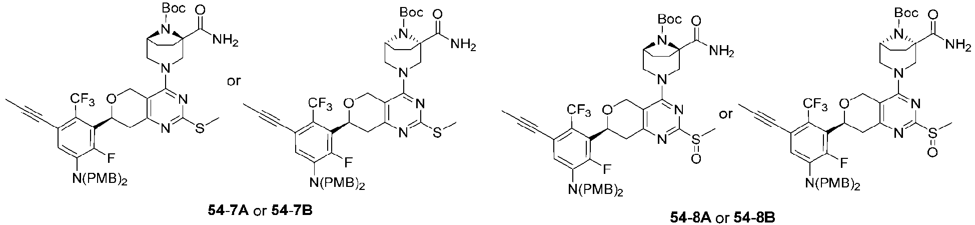

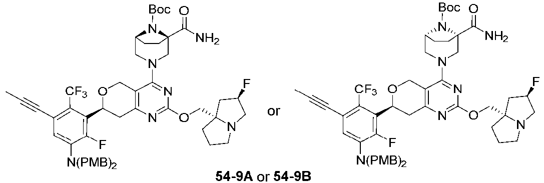

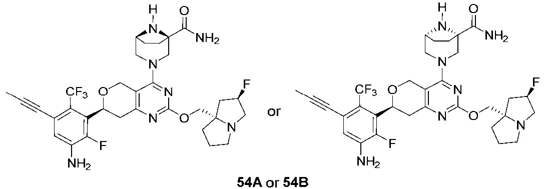

### Step 1

Compound **4-1A** (5 g, 23.55 mmol) was dissolved in acetonitrile (50 mL). Triethylamine (9.53 g, 94.21 mmol) and triphenylmethane (7.22 g, 25.91 mmol) were added, and the mixture was reacted at 25°C for 1 hour. The reaction solution was concentrated under reduced pressure, and the crude product was separated by column chromatography (petroleum ether:ethyl acetate = 3:1) to obtain compound **54-2.** ¹H NMR (400 MHz, CDCl₃) δ ppm 7.49-7.47 (m, 6H), 7.27-7.23 (m, 6H), 7.15-7.12 (m, 3H), 4.15 (s, 1H), 4.02 (s, 1H), 2.96 (d, *J* = 8.0 Hz, 2H), 2.38-2.33 (m, 2H), 2.00-1.97 (m, 2H), 1.83-1.72 (m, 2H), 1.25 (s, 9H).

### Step 2

Compound **54-2** (200 mg, 0.44 mmol) was dissolved in anhydrous ether (5 mL). N,N,N',N'-tetramethylethylenediamine (77 mg, 0.66 mmol) was added at -40°C and reacted for 5 minutes. Sec-butyllithium (0.858 mL, 0.66 mmol, 1.3M solution in n-hexane) was then added and reacted for 30 minutes at -40°C. The temperature was then lowered to -70°C, and ethyl chloroformate (96 mg, 0.88 mmol) was slowly added. The mixture was then reacted for 30 minutes at -70°C. After warming to room temperature, the reaction was quenched with saturated aqueous ammonium chloride (5 mL). The mixture was extracted with ethyl acetate (5 mL x 3). The organic phases were combined, concentrated under reduced pressure, and separated by column chromatography (petroleum ether:ethyl acetate = 5:1) to afford compound **54-3.** ¹H NMR (400 MHz, CDCl₃) δ ppm 7.48-7.46 (m, 6H), 7.27-7.23 (m, 6H), 7.15-7.11 (m, 3H), 4.20 (d, *J* = 4.0 Hz,1H), 4.12 (q, *J* = 8.0 Hz, 2H), 3.36 (d, *J* = 12.0 Hz, 1H), 2.92 (d, *J* = 12.0 Hz, 1H), 2.86-2.79 (m, 1H), 2.69-2.63 (m, 1H), 2.23-2.08 (m, 2H), 2.02 (d, *J* = 12.0 Hz, 1H), 1.86 (d, *J* = 12.0 Hz, 1H), 1.25 (t, *J* = 8.0 Hz, 3H), 1.15 (s, 9H).

### Step 3

Compound **54-3** (240 mg, 0.46 mmol) was dissolved in water (1 mL) and ethanol (2 mL). Potassium hydroxide (128 mg, 2.30 mmol) was added and the mixture was reacted at 110°C for 16 hours. The reaction was quenched by the addition of dilute hydrochloric acid (2 mL, 1 M). The mixture was extracted with ethyl acetate (5 mL x 3). The organic phases were combined, concentrated under reduced pressure, and separated by column chromatography (dichloromethane:methanol = 20:1) to afford compound **54-4.** ¹H NMR (400 MHz, DMSO-*d₆*) δ ppm 12.44 (s, 1H), 7.41-7.29 (m, 12H), 7.18-7.14 (m, 3H), 3.20 (d, *J =* 12.0 Hz, 1H), 2.79 (d, *J* = 12.0 Hz, 1H), 2.62-2.57 (m, 1H), 2.28-2.23 (m, 1H), 2.19-2.13 (m, 1H), 2.10-1.96 (m, 1H), 2.00 (m, 1H), 1.85 (d, *J* = 12.0 Hz, 1H), 1.62 (d, *J* = 8.0 Hz, 1H), 1.05 (s, 9H).

### Step 4

Compound **54-4** (120 mg, 0.24 mmol) was dissolved in N,N-dimethylformamide (2 mL). 1-(3-Dimethylaminopropyl)-3-ethylcarbodiimide hydrochloride (70 mg, 0.36 mmol) and 1 -hydroxybenzotriazole (55 mg, 0.36 mmol) were added and reacted at 25°C for 10 minutes. Aqueous ammonia (0.25 mL, 0.24 mmol) was added and the mixture was reacted at 25°C for 2 hours. The reaction was quenched by the addition of water (2 mL). The mixture was extracted with ethyl acetate (5 mL × 3). The organic phases were combined and concentrated under reduced pressure to yield compound **54-5.** MS m/z = 498.4 [M+H]⁺. Preparative SFC separation was then performed (Chromatographic column: DAICEL CHIRALPAK^{®} AS-10, 25*250mm 10 µm; mobile phase: A: supercritical carbon dioxide, B: [0.05% ammonia methanol-ethanol]; B%: 80%-20%) to obtain compounds **54-5A** and **54-5B.** Analytical SFC method (Chromatographic column: DAICEL CHIRALPAK^{®}AS-10 (4.6 mmI.D *150 mmL, 5 µm); Mobile phase: [supercritical carbon dioxide-ethanol (0.05% diethylamine)]; gradient: ethanol (0.05% diethylamine) 5%-40%, 4 min), compound **54-5A** had an Rt = 2.824 min, ee value of 97.05%, and MS m/z = 498.4 [M+H]⁺; and compound **54-5B** had an Rt = 3.327 min, an ee value of 96.10%, and an MS m/z = 498.4 [M+H]⁺.

### Step 5

Compound **54-5A** (0.036 g, 72.34 µmol) was dissolved in 1,4-dioxane (2 mL), and dilute hydrochloric acid (0.5 mL, 1 M) was added. The mixture was reacted at 25°C for 12 hours. The mixture was concentrated under reduced pressure to afford compound **54-6A.** MS m/z = 256.3 [M+H]⁺.

Referring to step 5, compound **54-5B** was used as a starting material in place of compound **54-5A** to obtain compound **54-6B.** MS m/z = 256.3 [M+H]⁺.

### Step 6

Compound **54-6A** (20 mg, 78.34 µmol) was dissolved in N,N-dimethylformamide (2 mL). N,N-diisopropylethylamine (30.37 mg, 235.01 µmol) and intermediate **1-13B** (73.86 mg, 94.00 µmol) were added. The mixture was reacted at 100°C for 1 hour. Water (2 mL) was added to quench the reaction, and ethyl acetate (5 mL * 3) was used for extraction. The organic phases were combined and concentrated under reduced pressure. The product was separated by column chromatography (petroleum ether:ethyl acetate = 2:1) to obtain compound **54-7A.** MS m/z = 891.3 [M+H]⁺.

Referring to step 6, compound **54-6B** was used as a starting material in place of compound **54-6A** to obtain compound **54-7B.** MS m/z = 891.3 [M+H]⁺

### Step 7

Compound **54-7A** (45 mg, 50.51 µmol) was dissolved in tetrahydrofuran (2 mL), and m-chloroperbenzoic acid (8.72 mg, 50.51 µmol, 85% purity) was added. The mixture was stirred at 20°C under nitrogen for 2 hours. Saturated aqueous sodium sulfite solution (2 mL) was added to quench the reaction, and the mixture was washed with saturated aqueous sodium bicarbonate solution (5 mL). The mixture was extracted with ethyl acetate (5 mL * 3). The organic phase was separated and dried over anhydrous sodium sulfate, and filtered. The filtrate was concentrated under reduced pressure, and separated by column chromatography (petroleum ether:ethyl acetate = 6:1) to obtain compound **54-8A.** MS m/z = 907.3 [M+H]⁺.

Referring to step 7, compound **54-7B** was used as a starting material in place of compound **54-7A** to obtain compound **54-8B.** MS m/z = 907.3 [M+H]⁺.

### Step 8

Compound **3-10A** (11.85 mg, 74.42 µmol) was dissolved in tetrahydrofuran (2 mL). Sodium hydride (3.57 mg, 148.85 µmol, 60% purity) was added at 0°C and stirred for 0.5 hour. **54-8A** (45.00 mg, 49.62 µmol) was added and reacted at 25°C for 2 hours. The reaction was quenched by adding water (5 mL). The product was extracted with ethyl acetate (5 mL x 3), dried over anhydrous sodium sulfate, and filtered. The filtrate was concentrated under reduced pressure, and separated by column chromatography (petroleum ether:ethyl acetate = 10:1) to afford compound **54-9A.** MS m/z = 1002.8 [M+H]⁺.

Referring to step 8, compound **54-8B** was used as a starting material in place of compound **54-8A** to obtain compound **54-9B.** MS m/z = 1002.8 [M+H]⁺.

### Step 9

Compound **54-9A** (48 mg, 47.90 µmol) was dissolved in dichloromethane (2 mL). Trifluoroacetic acid (1 mL) was added. The mixture was reacted at 25°C for 0.5 h. The reaction solution was concentrated under reduced pressure to obtain a crude product. The crude product was separated by preparative HPLC (Chromatographic column: Waters SunFire, 250*19 mm, 5 µm; mobile phase: [water (0.1% ammonia)-acetonitrile]-acetonitrile]; gradient: (acetonitrile): 40%-75%) and dried to obtain compound **54A.** MS m/z = 662.5 [M+H]⁺. ¹H NMR (400 MHz, CD₃OD) δ ppm 6.91 (d, *J =* 8.0 Hz, 1H), 5.52 (d, *J =* 52.0 Hz, 1H), 5.18 (dd, *J* = 12.0 Hz, 4.0 Hz, 1H), 4.88 (s, 1H), 4.76-4.69 (m, 2H), 4.50-4.40 (m, 2H), 3.80-3.76 (m, 2H), 3.69 (d, *J* = 4 Hz, 1H), 3.55-3.52 (m, 1H), 3.42-3.37 (m, 2H), 3.28-3.21 (m, 2H), 2.99 (d, *J* = 16.0 Hz, 1H), 2.90 (dd, *J* = 16.0 Hz, 4.0 Hz, 1H), 2.64-2.46 (m, 2H), 2.37-2.35 (m, 1H), 2.37-2.35 (m, 3H), 2.14-2.09 (m, 1H), 2.04-1.98 (m, 4H), 1.88-1.82 (m, 1H), 1.67-1.61 (m, 1H).

Compound **54-9B** (46 mg, 45.90 µmol) was dissolved in dichloromethane (2 mL). Trifluoroacetic acid (1 mL) was added. The mixture was reacted at 25°C for 0.5 h. The reaction solution was concentrated under reduced pressure to obtain a crude product. The crude product was separated by preparative HPLC (Chromatographic column: Waters Xbridge, 250 x 19 mm, 5 µm; mobile phase: water (0.1% formic acid)-acetonitrile; gradient: (acetonitrile): 55%-70%) and dried to obtain the formate salt of compound **54B.** MS m/z = 662.5 [M+H]⁺. ¹H NMR (400 MHz, CD₃OD) δ ppm 8.38 (s, 0.41H), 6.92 (d, *J =* 12.0 Hz, 1H), 5.51 (d, *J* = 52.0 Hz, 1H), 5.19 (dd, *J* = 12.0 Hz, 4.0 Hz, 1H), 4.69 (d, *J* = 12.0 Hz, 1H), 4.47-4.39 (m, 2H), 4.12 (d, *J* = 16.0 Hz, 1H), 3.98 (d, *J* = 12.0 Hz, 1H), 3.87-3.67 (m, 4H), 3.39-3.34 (m, 3H), 3.27-3.23 (m, 1H), 3.02 (d, *J* = 16.0 Hz, 1H), 2.91 (dd, *J* = 16.0 Hz, 4.0 Hz, 1H), 2.65-2.44 (m, 2H), 2.36-2.31 (m, 1H), 2.27-2.21 (m, 2H), 2.07-1.98 (m, 7H), 1.91-1.87 (m, 1H).

### Example 56

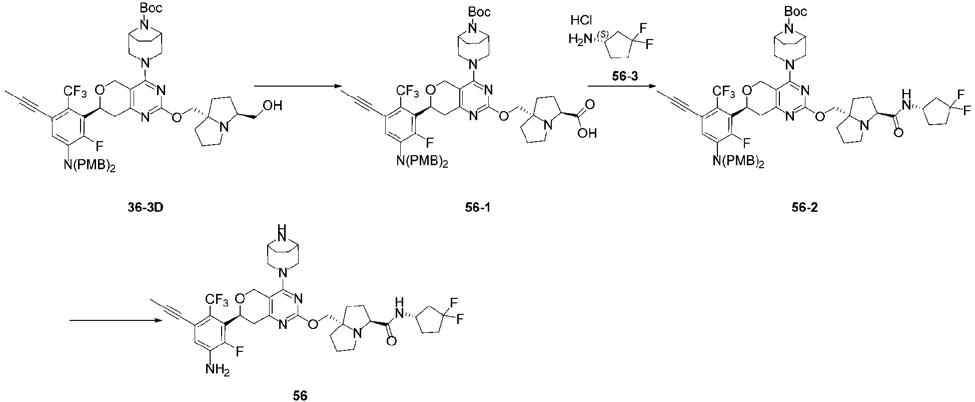

### Step 1

Compound **36-3D** (445 mg, 458.25 µmol) was weighed and N,N-dimethylformamide (13 mL) was added. Pyridinium dichromate (1.38 g, 3.67 mmol) was added and stirred at room temperature for 24 hours. Water (50 mL) was added and the mixture was extracted with ethyl acetate (50 mL x 3). The mixture was washed with saturated brine (150 mL x 3) and dried over anhydrous sodium sulfate. The organic phase was concentrated under reduced pressure to obtain compound **56-1,** which was used directly in the next reaction. MS m/z = 985.7 [M+H]⁺.

### Step 2

Compound **56-1** (200 mg, crude product) was weighed and dichloromethane (2.5 mL) was added. N,N,N,N-tetramethyl-O-(7-azabenzotriazol-1-yl)uronium hexafluorophosphate (154.40 mg, 406.08 µmol), diisopropylethylamine (78.72 mg, 609.12 µmol), and (S)-3,3-difluorocyclopentylamine hydrochloride (24.59 mg, 203.03 µmol) were added. The mixture was stirred at room temperature for 2 hours. Column chromatography (petroleum ether:ethyl acetate = 0:1) afforded **56-2.** MS m/z = 1088.7 [M+H]⁺.

### Step 3

Compound **56-2** (95 mg, 87.30 µmol) was weighed and dichloromethane (1 mL) was added. Trifluoroacetic acid (1 mL) was added and stirred at room temperature for 0.5 hours. The reaction solution was concentrated under reduced pressure to obtain a crude product, which was then separated by preparative HPLC (column: Waters Xbridge, 250*19 mm, 5 µm; mobile phase: [water (0.1% ammonia)-acetonitrile]; gradient: (acetonitrile): 30%-70%) to obtain compound **56.** MS m/z = 748.6 [M+H]⁺. ¹H NMR (400 MHz, CD₃OD) *δ* 6.90 (d, *J* = 8.0 Hz, 1H), 5.19 - 5.12 (m, 1H), 4.82 - 4.76 (m, 1H), 4.68 - 4.61 (m, 1H), 4.32 (p, *J* = 8.0 Hz, 1H), 4.20 - 4.10 (m, 2H), 4.05 (d, *J* = 8.0 Hz, 1H), 3.78 - 3.71 (m, 1H), 3.60 - 3.48 (m, 3H), 3.43 (d, *J* = 12.0 Hz, 1H), 3.27 - 3.13 (m, 1H), 3.05 (d, *J =* 12.0 Hz, 1H), 2.95 - 2.80 (m, 2H), 2.80 - 2.68 (m, 1H), 2.57 - 2.41 (m, 1H), 2.30 - 2.03 (m, 6H), 2.01 (s, 3H), 2.00 - 1.78 (m, 8H), 1.79 - 1.64 (m, 3H).

### Example 57

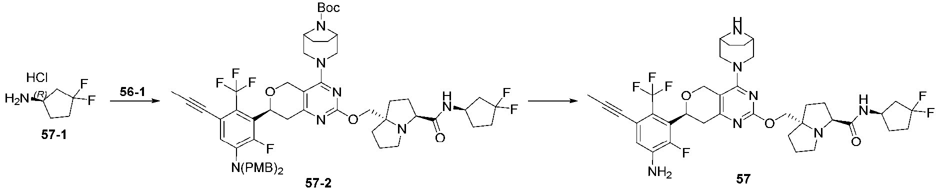

Referring to steps 2 to 3 of Example **56, 57-1** was used as a starting material in place of (S)-3,3-difluorocyclopentylamine hydrochloride to obtain crude compound 57. The crude product was separated by preparative HPLC (Chromatographic column: Waters Xbridge, 250*19 mm, 5 µm; mobile phase: [water (0.1% ammonia)-acetonitrile]; gradient: (acetonitrile): 49%-69%) and dried to afford compound 57. MS m/z = 748.5 [M+H]⁺. ¹H NMR (400 MHz, CD₃OD) δ ppm 6.90 (d, *J* = 8.0 Hz, 1H), 5.15 (dd, *J* = 12.0, 4.0 Hz, 1H), 4.79 (d, *J* = 12.0 Hz, 1H), 4.65 (d, *J* = 16.0 Hz, 1H), 4.34 - 4.27 (m, 1H), 4.15 (t, *J* = 12.0 Hz, 2H), 4.05 (d, *J* = 8.0 Hz, 1H), 3.76 - 3.72 (m, 1H), 3.55 - 3.51 (m, 3H), 3.42 (d, *J* = 12.0 Hz, 1H), 3.22 (dd, *J* = 16.0, 12.0 Hz, 1H), 3.05 (d, *J* = 12.0 Hz, 1H), 2.91 - 2.82 (m, 2H), 2.75 - 2.69 (m, 1H), 2.55 - 2.43 (m, 1H), 2.29 - 2.08 (m, 5H), 2.06 - 1.96 (m, 6H), 1.94 - 1.82 (m, 6H), 1.78 - 1.67 (m, 3H).

### Example 58

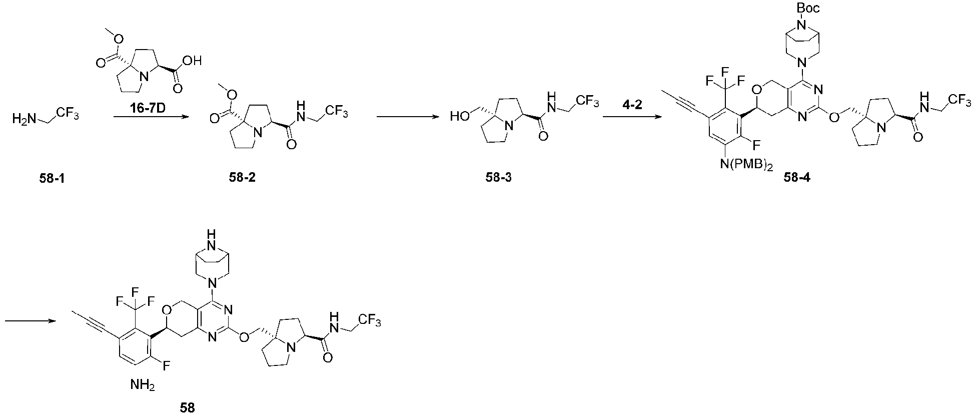

Referring to steps 7 to 10 of Example **16,** compound **58-1** was used as a starting material in place of *N-ethyl*-4-methoxybenzylamine to obtain crude compound **58.** The crude product was separated by preparative HPLC (Chromatographic column: Waters Xbridge, 250 x 19 mm, 5 µm; mobile phase: [water (0.1% ammonia)-acetonitrile]; gradient: (acetonitrile): 45%-70%) and dried to yield compound **58.** MS m/z = 726.5 [M+H]⁺. ¹H NMR (400 MHz, CD₃OD) δ ppm 6.90 (d, *J* = 8.0 Hz, 1H), 5.18 - 5.10 (m, 1H), 4.79 (d, *J* = 12.0 Hz, 1H), 4.65 (d, *J* = 12.0 Hz, 1H), 4.20 - 4.10 (m, 2H), 4.08 - 3.96 (m, 2H), 3.92 - 3.78 (m, 2H), 3.57 - 3.48 (m, 3H), 3.45 - 3.38 (m, 1H), 3.26 - 3.17 (m, 1H), 3.04 (d, *J* = 12.0 Hz, 1H), 2.96 - 2.89 (m, 1H), 2.88 - 2.81 (m, 1H), 2.75 - 2.67 (m, 1H), 2.15 - 1.97 (m, 7H), 1.97 - 1.78 (m, 6H), 1.77 - 1.64 (m, 2H).

### Example 59

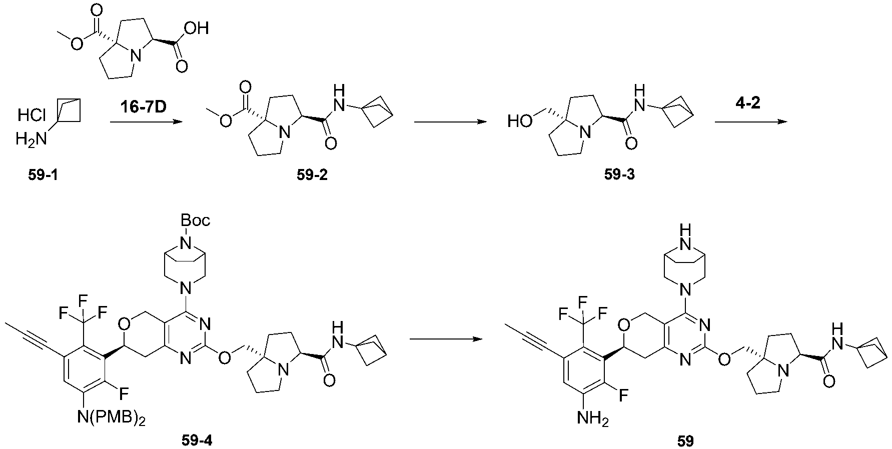

Referring to steps 7 to 10 of Example **16,** compound **59-1** was used as a starting material in place of *N*-ethyl-4-methoxybenzylamine to obtain crude compound **59.** The crude product was separated by preparative HPLC (Chromatographic column: Waters Xbridge, 250x19 mm, 5 µm; mobile phase: [water (0.1% ammonia)-acetonitrile]; gradient: (acetonitrile): 45%-70%) and dried to yield compound **59.** MS m/z = 710.5 [M+H]⁺. ¹H NMR (400 MHz, CD₃OD) δ ppm 6.90 (d, *J* = 8.0 Hz, 1H), 5.16 - 5.12 (m, 1H), 4.80 (d, *J* = 12.0 Hz, 1H), 4.65 (d, *J* = 12.0 Hz, 1H), 4.17 - 4.12 (m, 2H), 4.03 (d, *J* = 12.0 Hz, 1H), 3.69 - 3.65 (m, 1H), 3.56 - 3.51 (m, 3H), 3.43 (d, *J* = 12.0 Hz, 1H), 3.25 - 3.18 (m, 1H), 3.05 (d, *J* = 12.0 Hz, 1H), 2.93 - 2.82 (m, 2H), 2.78 - 2.72 (m, 1H), 2.11 - 2.05 (m, 8H), 2.04 - 1.98 (m, 5H), 1.96 - 1.82 (m, 7H), 1.75 - 1.67 (m, 2H).

### Example 62

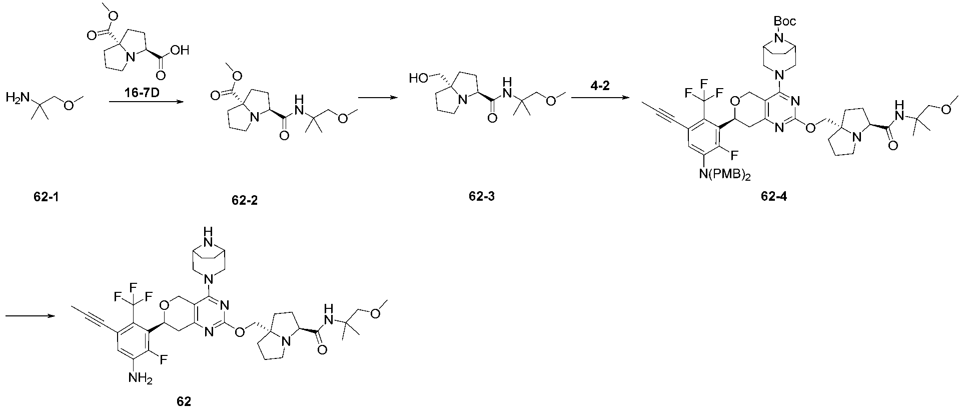

Referring to steps 7 to 10 of Example **16,** (1,1-dimethyl-2-methoxyethyl)amine was used as a starting material in place of *N*-ethyl-4-methoxybenzylamine to obtain crude compound **62.** The crude product was separated by preparative HPLC (Chromatographic column: Waters Xbridge, 250 x 19 mm, 5 µm; mobile phase: [water (0.1% ammonia)-acetonitrile]; gradient: (acetonitrile): 45%-70%) and dried to yield compound **62.** MS m/z = 730.5 [M+H]⁺. ¹H NMR (400 MHz, CD₃OD) δ ppm 6.90 (d, *J* = 8.0 Hz, 1H), 5.19 - 5.10 (m, 1H), 4.78 (s, 1H), 4.65 (d, *J* = 16.0 Hz, 1H), 4.21 - 4.09 (m, 2H), 4.05 (d, *J* = 12.0 Hz, 1H), 3.76 - 3.68 (m, 1H), 3.56 - 3.38 (m, 6H), 3.34 (s, 3H), 3.28 - 3.16 (m, 1H), 3.04 (d, *J* = 16.0 Hz, 1H), 3.00 - 2.94 (m, 1H), 2.88 - 2.80 (m, 1H), 2.78 - 2.67 (m, 1H), 2.17 - 1.96 (m, 6H), 1.95 - 1.79 (m, 7H), 1.76 - 1.64 (m, 2H), 1.32 (s, 6H).

### Example 63

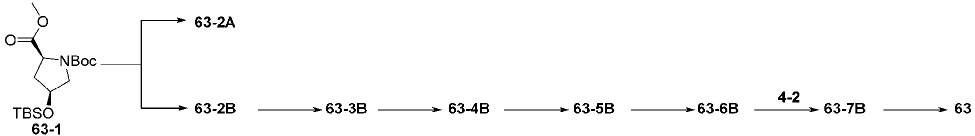

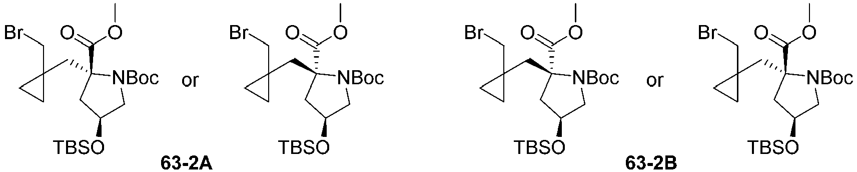

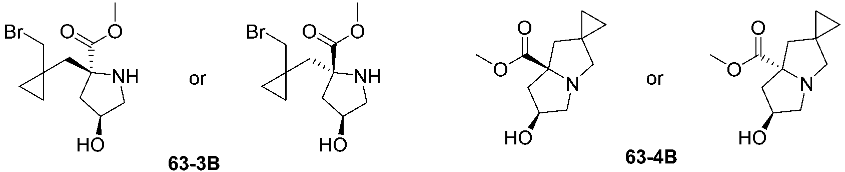

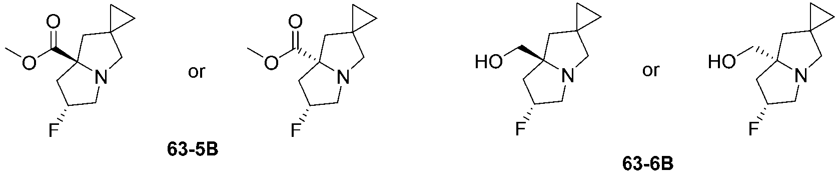

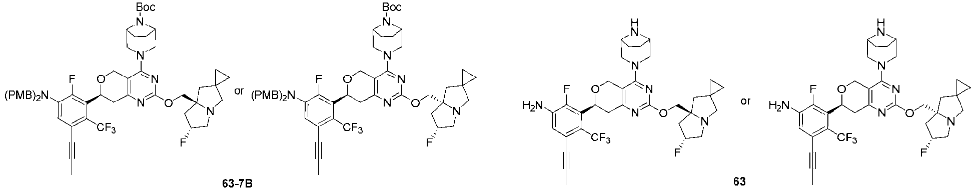

### Step 1

Under nitrogen, a solution of lithium bistrimethylsilylamide in tetrahydrofuran (3.49 g, 20.86 mmol) was slowly added dropwise to a solution of compound **63-1** (2.5 g, 6.95 mmol) in tetrahydrofuran (30 mL) at - 70°C and allowed to react at -70°C for 1 hour. 1,1-Bis-bromomethylcyclopropane (4.75 g, 20.86 mmol) was added dropwise to the reaction solution at -70°C, and the mixture was heated to 25°C with stirring for 12 hours. The reaction was quenched by the addition of saturated aqueous ammonium chloride (30 mL). The mixture was extracted with ethyl acetate (50 mL * 3), dried over anhydrous sodium sulfate, and filtered. The filtrate was concentrated under reduced pressure, and separated by column chromatography (petroleum ether:ethyl acetate = 5:1) to obtain compounds **63-2A** and **63-2B.** Compound **63-2A** (developing solvent: petroleum ether:ethyl acetate = 8:1, Rf = 0.45); compound **63-2B** (developing solvent: petroleum ether:ethyl acetate = 8:1, Rf = 0.5).

Compound **63-2A:** MS m/z = 507.9 [M+H]⁺. ¹H NMR (400 MHz, CDCl₃) δ ppm 4.46 - 4.38 (m, 1H), 3.71 -3.59 (m, 5H), 3.36 - 3.24 (m, 1H), 3.05 - 2.92 (m, 1H), 2.83 - 2.76 (m, 1H), 2.40 - 2.29 (m, 1H), 2.12 - 2.05 (m, 1H), 1.67 - 1.57 (m, 1H), 1.40 - 1.37 (m, 9H), 0.96 - 0.91 (m, 1H), 0.87 - 0.82 (m, 9H), 0.76 - 0.71 (m, 1H), 0.65 - 0.46 (m, 2H),0.18 - 0.00 (m, 6H).

Compound **63-2B:** MS m/z = 507.9 [M+H]⁺. ¹H NMR (400 MHz, CDCl₃) δ ppm 4.50 - 4.44 (m, 1H), 4.14 - 4.03 (m, 1H), 3.89 - 3.71 (m, 1H), 3.69 (m, 3H), 3.23 - 3.14 (m, 2H), 2.75 - 2.67 (m, 1H), 2.50 - 2.16 (m, 2H), 1.93 - 1.87(m, 1H), 1.45 - 1.42 (m, 9H), 0.87 (s, 9H), 0.85 - 0.51 (m, 4H), 0.05 (s, 6H).

### Step 2

Compound **63-2B** (200 mg, 0.39 mmol) was dissolved in dichloromethane (2 mL), and a hydrogen chloride/1,4-dioxane solution (2 mL, 4 M) was added. The mixture was stirred at 25°C for 15 hours. The reaction mixture was concentrated under reduced pressure to afford the hydrochloride salt of compound **63-3B.** MS m/z = 292.04 [M+H]⁺.

### Step 3

Compound **63-3B** (193 mg, hydrochloride) was dissolved in acetonitrile (2 mL), and potassium carbonate (270 mg, 1.95 mmol) was added. The mixture was stirred at 25°C for 12 hours. The reaction solution was filtered, concentrated under reduced pressure, and separated by column chromatography (dichloromethane:methanol = 10:1) to afford compound **63-4B.** MS m/z = 212.17 [M+H]⁺.

### Step 4

Compound **63-4B** (180 mg, 0.85 mmol) was dissolved in ultra-dry dichloromethane (5 mL). Diethylaminosulfur trifluoride (412.02 mg, 2.56 mmol) was added at -78°C and reacted under nitrogen at -78°C for 4 hours. The reaction solution was extracted with dichloromethane (30 mL) and water (10 mL). The organic phase was dried over anhydrous sodium sulfate, and filtered. The filtrate was concentrated under reduced pressure, and separated by column chromatography (dichloromethane:methanol = 10:1) to afford compound **63-5B.** MS m/z = 214.14 [M+H]⁺.

### Step 5

Compound **63-5B** (30 mg, 0.14 mmol) was dissolved in tetrahydrofuran (3 mL). Lithium aluminum hydride (10.69 mg, 0.28 mmol) was added under nitrogen. The mixture was reacted at 25°C for 0.5 h. The reaction mixture was diluted with water (10 mL) and extracted with ethyl acetate (30 mL). The organic phase was washed with saturated brine, dried over anhydrous sodium sulfate, filtered, and concentrated under reduced pressure to yield compound **63-6B.** MS m/z = 186.18 [M+H]⁺.

### Step 6

Compound **63-6B** (20 mg, 0.10 mmol) was dissolved in tetrahydrofuran (10 mL). Sodium hydride (5.18 mg, 0.21 mmol, 60% purity) was then added, and the reaction was stirred at 25°C for 0.5 hours under nitrogen protection. Then, a solution of compound **4-2** (93 mg, 0.10 mmol) in tetrahydrofuran (1 mL) was added. After the addition was completed, the reaction was stirred at 25°C for 2 hours. The reaction solution was dissolved in ethyl acetate (20 mL), and then washed with saturated brine (10 mL). The organic phase was dried over anhydrous sodium sulfate, and filtered. The filtrate was concentrated under reduced pressure. The crude product was purified by column chromatography (dichloromethane:methanol = 20:1) to give compound **63-7B.** MS m/z = 985.7 [M+H]⁺.

### Step 7

Compound **63-7B** (15 mg, 0.03 mmol) was dissolved in dichloromethane (1 mL), and trifluoroacetic acid (1 mL) was added. The mixture was stirred at 25°C for 2 hours. The reaction solution was concentrated under reduced pressure, and the crude product was separated by preparative HPLC (Chromatographic column: Waters Xbridge, 250*19mm, 5µm; mobile phase: [water (0.1% hydrochloric acid)-acetonitrile]; gradient: (acetonitrile): 10%-40%), and dried to give the hydrochloride salt of compound **63.** MS m/z = 645.5 [M+H]⁺. ¹H NMR (400 MHz, CD₃OD) δ ppm 6.94 (d, *J* = 8.0 Hz, 1H), 5.65 (d, *J* = 52.0 Hz, 1H), 5.27 - 5.16 (m, 1H), 5.00 - 4.87 (m, 5H), 4.27 - 4.20 (m, 2H), 4.05 - 3.87 (m, 4H), 3.68 - 3.65 (m, 1H), 3.59 - 3.53 (m, 2H), 3.47 - 3.37 (m, 1H), 3.07 - 3.03 (m, 1H), 2.81 - 2.64 (m, 3H), 2.23 - 2.09 (m, 4H), 2.02-1.97 (m, 4H), 0.88 (s, 4H).

### Example 64

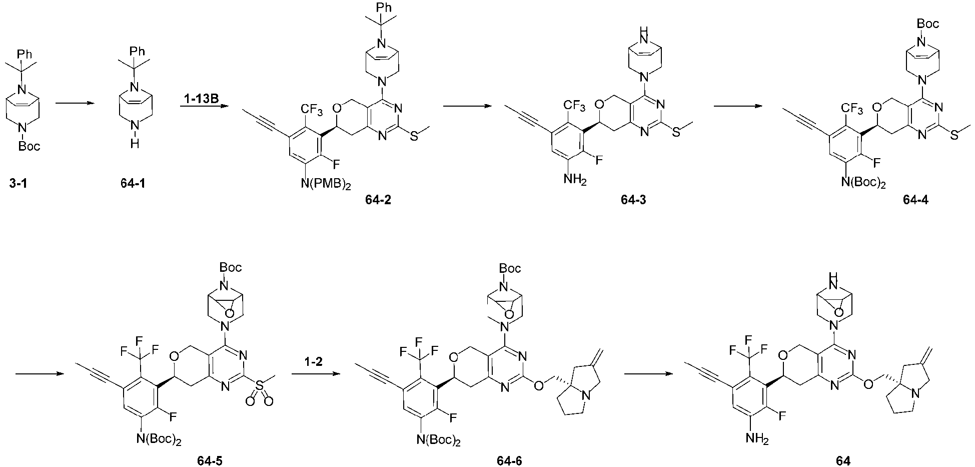

### Step 1

Compound **3-1** (2.0 g, 6.09 mmol) was dissolved in dioxane hydrochloride (20 mL, 4 M) and the mixture was stirred at 25°C for 1 hour. The reaction solution was concentrated under reduced pressure to obtain compound **64-1.** MS m/z = 229.2 [M+H]⁺.

### Step 2

Compound **64-1** (650 mg, 2.85 mmol) was dissolved in N,N-dimethylformamide (20 mL). Compound **1-13B** (2.03 g, 2.59 mmol) and N,N-diisopropylethylamine (1.0 g, 7.76 mmol) were added at room temperature. The mixture was heated to 100°C and stirred for 2 hours. The reaction was quenched by adding water (20 mL). The mixture was extracted with ethyl acetate (30 mL x 3). The extracted organic phases were combined and washed with saturated brine (30 mL), dried over anhydrous sodium sulfate, filtered, and concentrated under reduced pressure. Column chromatography (petroleum ether:ethyl acetate = 10:1) afforded compound **64-2.** MS m/z = 864.3 [M+H]⁺.

### Step 3

Compound **64-2** (1.34 g, 1.55 mmol) was dissolved in dichloromethane (18 mL). Trifluoroacetic acid (18 mL) was added at room temperature. The mixture was heated to 40°C and stirred for 48 hours. The reaction solution was concentrated under reduced pressure to obtain compound **64-3.** MS m/z = 506.3 [M+H]⁺.

### Step 4

Compound **64-3** (900 mg, 1.78 mmol) was dissolved in dichloromethane (10 mL). Triethylamine (900.74 mg, 8.90 mmol), 4-dimethylaminopyridine (43.50 mg, 356.06 µmol), and di-tert-butyl dicarbonate (1.36 g, 6.23 mmol) were added at room temperature. The mixture was stirred at room temperature for 16 hours. Water (10 mL) was added to quench the reaction, and the product was extracted with dichloromethane (15 mL * 3). The organic phases were combined and concentrated under reduced pressure. The product was separated by column chromatography (petroleum ether:ethyl acetate = 3:1) to obtain compound **64-4.** MS m/z = 806.4 [M+H]⁺.

### Step 5

Compound **64-4** (170 mg, 210.95 µmol) was dissolved in dichloromethane (3 mL). m-Chloroperbenzoic acid (214.13 mg, 1.05 mmol, 85% purity) was added at 25°C, and the mixture was heated to 35°C and stirred for 20 hours. The reaction was quenched with saturated sodium sulfite (5 mL) and extracted with dichloromethane (10 mL * 3). The organic phases were combined and concentrated under reduced pressure. The product was separated by column chromatography (petroleum ether:ethyl acetate = 1:1) to obtain compound **64-5.** MS m/z = 854.5 [M+H]⁺.

### Step 6

Compound **1-2** (20 mg, 130.53 µmol) was dissolved in anhydrous tetrahydrofuran (2 mL). Sodium hydride (7.83 mg, 195.80 µmol, 60%) was added at 0°C and the mixture was stirred for 0.5 hours. Compound 64-5 (111.46 mg, 130.53 µmol) was added, and the mixture was heated to 25°C and stirred for 1.5 hours. The reaction was quenched by adding water (5 mL), extracted with ethyl acetate (15 mL x 3), dried over anhydrous sodium sulfate, and filtered. The filtrate was concentrated under reduced pressure, and separated by column chromatography (dichloromethane:methanol = 10:1) to obtain compound **64-6.** MS m/z = 827.6 [M-100+H]⁺.

### Step 7

Compound **64-6** (45 mg, 48.54 µmol) was dissolved in dichloromethane (2 mL). Trifluoroacetic acid (0.4 mL) was added, and the mixture was stirred at room temperature for 1.5 hours. The reaction solution was concentrated under reduced pressure to obtain a crude product. The crude product was separated by preparative high performance liquid chromatography (Chromatographic column: SunFire prep c18, 250*19mm, 5µm; mobile phase: [water (0.1% ammonia)-acetonitrile]; gradient: (acetonitrile): 50%-80%). After drying, compound **64** was obtained. MS m/z = 627.31 [M+H]⁺. ¹H NMR (400 MHz, CD₃OD) δ ppm 6.90 (d, *J* = 8.0 Hz, 1H), 5.15 (dd, *J = 12.0* Hz, 4.0 Hz, 1H), 5.00 (s, 2H), 4.81 (s, 1H), 4.65 (d, *J* = 12.0 Hz, 1H), 4.43 - 4.37 (m, 1H), 4.17 - 4.11 (m, 2H), 3.75 (d, *J* = 12.0 Hz, 1H), 3.68 (d, *J* = 4.0 Hz, 1H), 3.61 - 3.56 (m, 2H), 3.53 - 3.47 (m, 2H), 3.37 - 3.36 (m, 1H), 3.24 - 3.13 (m, 4H), 2.84 (d, J = 16.0 Hz, 1H), 2.76 (d, *J* = 16.0 Hz, 2H), 2.46 (d, *J* = *16.0 Hz, 1H*), *2.14 -* 2.09 (m, 1H), 2.02 (s, 3H), 1.99 - 1.89 (m, 2H), 1.86 - 1.81 (m, 1H).

### Example 65

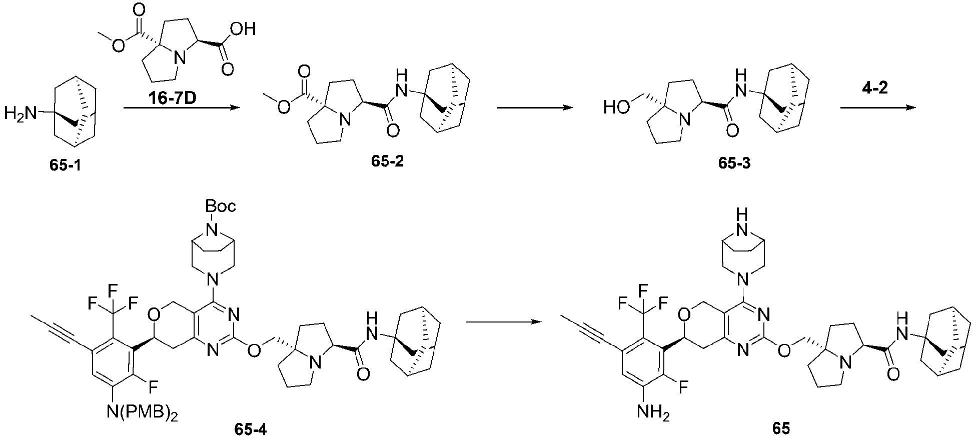

Referring to steps 7 to 10 of Example **16,** compound **65-1** was used as a starting material in place of *N*-ethyl-4-methoxybenzylamine to obtain crude compound **65.** The crude product was separated by preparative HPLC (Chromatographic column: Waters Xbridge, 250 x 19 mm, 5 µm; mobile phase: [water (0.1% ammonia)-acetonitrile]; gradient: (acetonitrile): 45%-72%), and dried to yield compound **65.** MS m/z = 778.6 [M+H]⁺. ¹H NMR (400 MHz, CD₃OD) δ ppm 6.90 (d, *J* = 8.0 Hz, 1H), 5.17 - 5.13 (m, 1H), 4.80 (d, *J=* 12.0 Hz, 1H), 4.65 (d, *J* = 12.0 Hz, 1H), 4.18 - 4.12 (m, 2H), 4.05 (d, *J* = 12.0 Hz, 1H), 3.69 (dd, *J* = 12.0 Hz, 4.0 Hz, 1H), 3.54 - 3.51 (m, 3H), 3.42 (d, *J =* 12.0 Hz, 1H), 3.22 (dd, *J* = 20.0 Hz, 12.0 Hz, 1H), 3.05 (d, *J* = 12.0 Hz, 1H), 2.96 - 2.91 (m, 1H), 2.85 (dd, *J* = 12.0 Hz, 4.0 Hz, 1H), 2.78 - 2.72 (m, 1H), 2.14 - 2.02 (m, 15H), 1.90 - 1.82 (m, 7H), 1.74 - 1.68 (m, 8H).

### Example 66

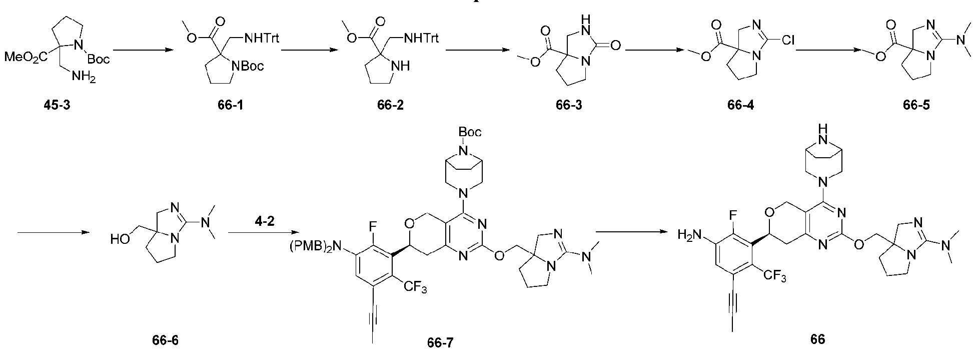

### Step 1

Compound **45-3** (4.2 g, 16.26 mmol) was weighed and dissolved in anhydrous tetrahydrofuran (20 mL). Triethylamine (1.65 g, 16.26 mmol, 2.27 mL) was added. Chlorotriphenylmethane (4.53 g, 16.26 mmol) was added under ice-cooling. The mixture was stirred at room temperature for 12 hours. The organic phase was concentrated under reduced pressure and separated by column chromatography (petroleum ether:ethyl acetate = 9:1) to obtain compound **66-1.** MS m/z = 501.4 [M+H]⁺.

### Step 2

Compound **66-1** (6.3 g, 12.58 mmol) was weighed and dissolved in anhydrous dichloromethane (20 mL). 2,6-Dimethylpyridine (2.70 g, 25.17 mmol, 2.92 mL) was added. Trimethylsilyl trifluoromethanesulfonate (4.20 g, 18.88 mmol) was added under ice-cooling. The mixture was reacted at 0°C for 2 h. Water (50 mL) was added and the mixture was extracted with dichloromethane (100 mL*3). The mixture was dried over anhydrous sodium sulfate and filtered. The organic phase was concentrated under reduced pressure and separated by column chromatography (petroleum ether:ethyl acetate = 1:1) to obtain compound **66-2.** MS m/z = 401.3 [M+H]⁺.

### Step 3

Compound **66-2** (3 g, 7.49 mmol) was weighed and dissolved in anhydrous tetrahydrofuran (15 mL), and triphosgene (2.22 g, 7.49 mmol) was added. The mixture was reacted at room temperature for 2 hours. The mixture was concentrated under reduced pressure and separated by column chromatography (dichloromethane:methanol = 10:1) to obtain a crude product. Secondary chromatography (Flash Spherical C18 column; [water (0.1% formic acid)-acetonitrile]; gradient: (acetonitrile): 0%-20%) afforded compound **66-3.** MS m/z = 185.1 [M+H]⁺.

### Step 4

Compound **66-3** (480 mg, 2.61 mmol) was weighed and dissolved in phosphorus oxychloride (5 mL) and reacted at 80°C for 2 hours. The mixture was concentrated under reduced pressure to obtain crude compound **66-4.** MS m/z = 203.1 [M+H]⁺.

### Step 5

Compound **66-4** (194 mg, crude product) was weighed and dissolved in acetonitrile (5 mL). Potassium carbonate (395.65 mg, 2.88 mmol) and dimethylamine (129.48 mg, 2.88 mmol, 2M solution in tetrahydrofuran) were added. The mixture was reacted at 80°C for 12 hours. The mixture was concentrated under reduced pressure and separated by column chromatography (dichloromethane:methanol = 5:1) to obtain compound **66-5.** MS m/z = 212.1 [M+H]⁺.

### Step 6

Compound **66-5** (70 mg, 331.34 µmol) was weighed and dissolved in methanol (2 mL). Lithium chloride (28.09 mg, 662.69 µmol) and sodium borohydride (25.07 mg, 662.69 µmol) were added and reacted at room temperature for 2 hours. The mixture was concentrated under reduced pressure and chromatographed (Flash Spherical C18 column; [water (0.1% formic acid)-acetonitrile]; gradient: (acetonitrile): 0 %-20%) to afford compound **66-6.** MS m/z = 184.3 [M+H]⁺.

### Step 7

Compound **66-6** (65 mg, 354.71 µmol) was weighed and anhydrous tetrahydrofuran (1 mL) was added. Sodium hydride (17.02 mg, 709.41 µmol, 60%) was added under ice-cooling. The mixture was stirred at room temperature for 30 minutes. Compound **4-2** (312.13 mg, 354.71 µmol) was added and allowed to react at room temperature for 1 hour. The reaction mixture was quenched with water (5 mL) and extracted with ethyl acetate (5 mL x 3). The organic phase was concentrated under reduced pressure and separated by column chromatography (dichloromethane:methanol = 20:1) to obtain compound **66-7.** MS m/z = 983.7 [M+H]⁺.

### Step 8

Compound **66-7** (45 mg, 45.77 µmol) was weighed. Dichloromethane (1 mL) and trifluoroacetic acid (1 mL) were added, and the mixture was stirred at room temperature for 0.5 hours. The reaction solution was concentrated under reduced pressure to obtain a crude product, which was then separated by preparative HPLC (Chromatographic column: SunFire prep C18, 250*19 mm, 5 µm; mobile phase: [water (0.1% formic acid)-acetonitrile]; gradient: (acetonitrile): 20%-70%) to obtain the formate salt of compound **66.** MS m/z = 643.5 [M+H]⁺. ¹H NMR (400 MHz, CD₃OD) *δ* ppm 8.51 (s, 1.28H), 6.93 (d, *J =* 8.0 Hz, 1H), 5.19 (dd, *J* = 12.0, 4.0 Hz, 1H), 4.86 - 4.82 (m, 1H), 4.72 (d, *J* = 12.0 Hz, 1H), 4.47 - 4.34 (m, 2H), 4.25 (t, *J* = 12.0 Hz, 1H), 4.11 - 4.01 (m, 2H), 3.89 (dd, *J=* 12.0, 4.0 Hz, 1H), 3.76 (d, *J* = 12.0 Hz, 1H), 3.70 - 3.59 (m, 2H), 3.58 - 3.50 (m, 2H), 3.30 - 3.23 (m, 2H), 3.15 - 3.06 (m, 6H), 2.91 (dd, *J* = 20.0, 4.0 Hz, 1H), 2.29 - 2.13 (m, 4H), 2.11 - 2.01 (m, 6H), 1.99 - 1.90 (m, 1H).

### Example 68

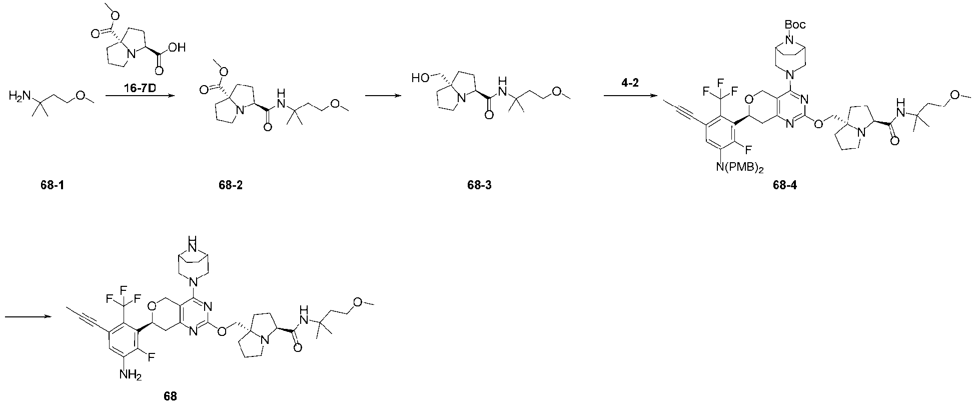

Referring to steps 7 to 10 of Example **16,** compound **68-1** was used as a starting material in place of N-ethyl-4-methoxybenzylamine to obtain crude compound **68.** The crude product was separated by preparative HPLC (Chromatographic column: Waters Xbridge, 250 x 19 mm, 5 µm; mobile phase: [water (0.1% ammonia)-acetonitrile]; gradient: (acetonitrile): 45%-70%) and dried to yield compound **68.** MS m/z = 744.6 [M+H]⁺. ¹H NMR (400 MHz, CD₃OD) δ ppm 6.90 (d, *J* = 8.0 Hz, 1H), 5.16 - 5.12 (m, 1H), 4.79 (d, *J* = 12.0 Hz, 1H), 4.65 (d, *J* = 12.0 Hz, 1H), 4.20 - 4.11 (m, 2H), 4.05 (d, *J* = 12.0 Hz, 1H), 3.72 - 3.67 (m, 1H), 3.55 - 3.46 (m, 5H), 3.44 - 3.39 (m, 1H), 3.31 (s, 3H), 3.26 - 3.17 (m, 1H), 3.04 (d, *J* = 12.0 Hz, 1H), 2.97 - 2.90 (m, 1H), 2.88 - 2.80 (m, 1H), 2.78 - 2.70 (m, 1H), 2.15 - 1.98 (m, 7H), 1.96 - 1.77 (m, 8H), 1.75 - 1.65 (m, 2H), 1.36 (d, *J* = 8.0 Hz, 6H).

### Example 69

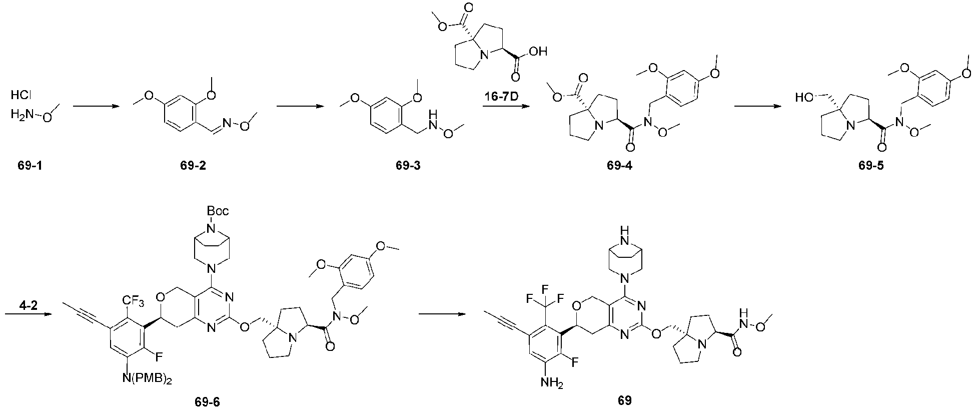

### Step 1

Compound **69-1** (1.7 g, 20.46 mmol) was dissolved in tetrahydrofuran (4 mL) and water (12 mL). 2,4-Dimethoxybenzaldehyde (2 g, 12.04 mmol) and sodium acetate (1.48 g, 18.05 mmol) were added sequentially. The mixture was stirred at 25°C for 3 hours. Water (10 mL) was added to quench the reaction, and the mixture was extracted with ethyl acetate (20 mL x 3). The mixture was concentrated under reduced pressure to obtain crude compound **69-2.** MS m/z = 196.1 [M+H]⁺.

### Step 2

Compound **69-2** (1.00 g, crude product) was dissolved in glacial acetic acid (8 mL). Sodium cyanoborohydride (644 mg, 10.25 mmol) was added, and the mixture was stirred at 25°C for 4 hours. 2 N sodium hydroxide solution was added to adjust the pH to 9, and the mixture was extracted with ethyl acetate (15 mL * 3). The extracted organic phases were combined and washed with saturated brine (15 mL), dried over anhydrous sodium sulfate, concentrated under reduced pressure, and separated by column chromatography (petroleum ether:ethyl acetate = 3:2) to obtain compound **69-3.** ¹H NMR (400 MHz, DMSO-*d₆*) δ ppm δ 7.17 (d, *J* = 8.0 Hz, 1H), 6.52 (d, *J =* 4.0 Hz, 1H), 6.50 - 6.43 (m, 2H), 3.84 (d, *J =* 4.0 Hz, 2H), 3.75 (d, *J =* 8.0 Hz, 6H), 3.37 (s, 3H).

### Step 3

Referring to steps 7 to 10 of Example **16, 69-3** was used as a starting material in place of N-ethyl-4-methoxybenzylamine to obtain crude compound **69.** The crude product was separated by preparative HPLC (Chromatographic column: Waters SunFire, 250 x 19 mm, 5 µm; mobile phase: [water (0.1% ammonia)-acetonitrile]; gradient: (acetonitrile): 30% - 75%) and dried to afford compound **69.** MS m/z = 674.5 [M+H]⁺. ¹H NMR (400 MHz, CD₃OD) δ ppm 6.90 (d, *J* = 8.0 Hz, 1H), 5.14 (dd, *J =* 12.0, 4.0 Hz, 1H), 4.79 (d, *J* = 12.0 Hz, 1H), 4.65 (d, *J* = 12.0 Hz, 1H), 4.17 - 4.12 (m, 2H), 4.04 (d, *J =* 12.0 Hz, 1H), 3.71 (s, 3H), 3.60 (t, *J* = 8.0 Hz, 1H), 3.57 - 3.49 (m, 3H), 3.42 (d, *J* = 12.0 Hz, 1H), 3.21 (dd, *J =* 20.0, 12.0 Hz, 1H), 3.04 (d, *J =* 16.0 Hz, 1H), 2.96 - 2.89 (m, 1H), 2.88 - 2.78 (m, 2H), 2.22 - 1.93 (m, 11H), 1.85 - 1.65 (m, 4H).

### Example 70

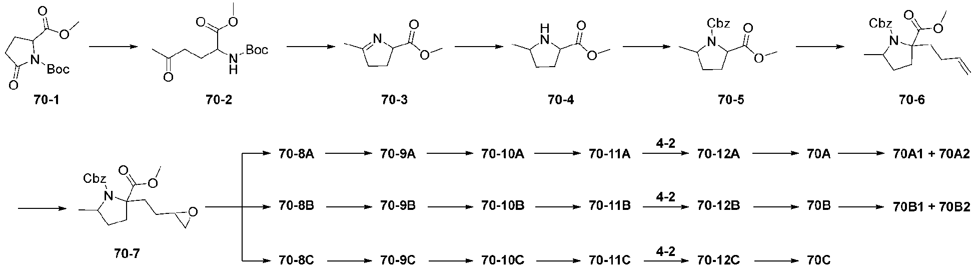

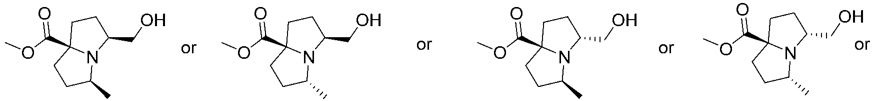

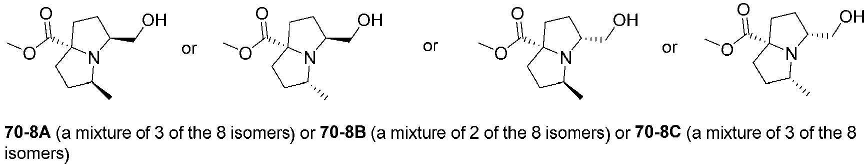

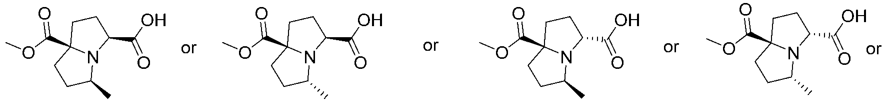

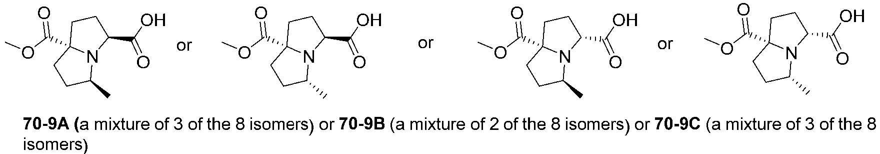

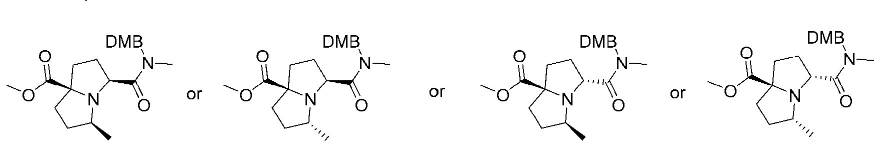

or 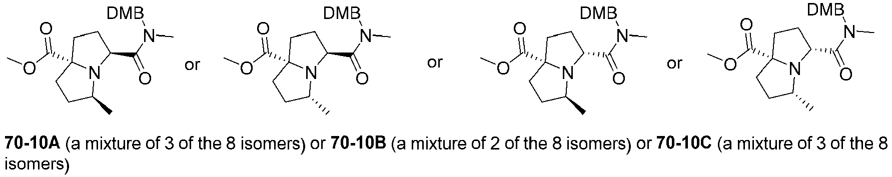 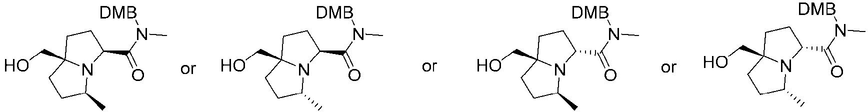 or

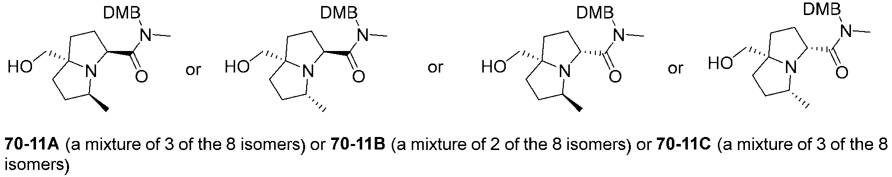

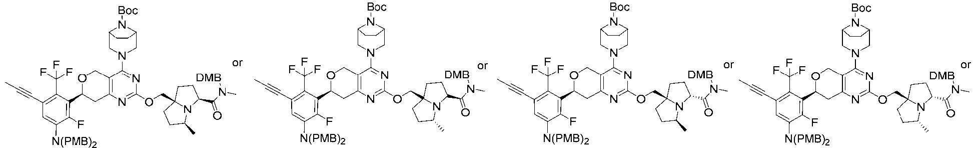

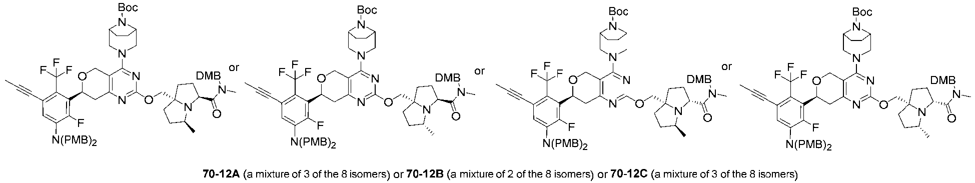

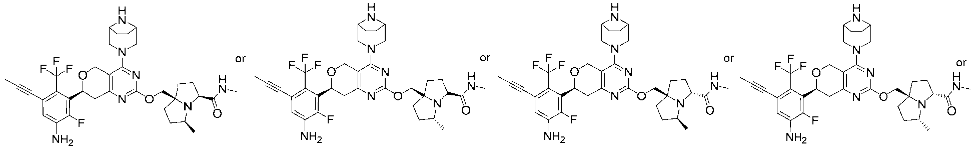

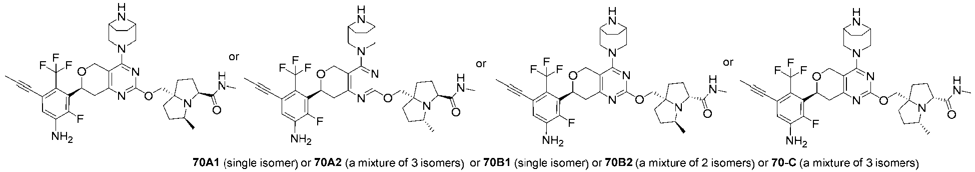

### Step 1

C ompound **70-1** (2.00 g, 8.22 mmol) was dissolved in tetrahydrofuran (15 mL). Methylmagnesium bromide solution (3.56 mL, 12.69 mmol, 3 M in tetrahydrofuran) was slowly added dropwise at 0°C. The mixture was heated to 25°C and stirred for 12 hours. The reaction was quenched by the addition of saturated ammonium chloride solution (10 mL) at 0°C. The mixture was extracted with ethyl acetate (20 mL × 3). The extracted organic phases were combined and washed with saturated brine (10 mL × 3), dried over anhydrous sodium sulfate, and filtered. The filtrate was concentrated under reduced pressure, and separated by column chromatography (petroleum ether:ethyl acetate = 8:1) to obtain compound **70-2.** MS m/z = 160.3 [M-Boc+H]⁺.

### Step 2

Compound **70-2** (4.00 g, 15.43 mmol) was dissolved in dichloromethane (15 mL), and trifluoroacetic acid (5 mL) was added. The mixture was stirred at 25°C for 16 hours. The reaction solution was concentrated under reduced pressure to obtain crude product **70-3.** MS m/z = 142.1 [M+H]⁺.

### Step 3

Compound **70-3** (120 mg, crude) was dissolved in anhydrous ethanol (5 mL), and palladium / carbon (12 mg, 10% purity) was added. The mixture was stirred at 25°C under a hydrogen atmosphere (15 psi) for 16 hours. The mixture was filtered, and the filtrate was concentrated under reduced pressure to obtain crude product **70-4.** MS m/z = 144.1 [M+H]⁺.

### Step 4

C ompound **70-4** (120 mg, 0.84 mmol) was dissolved in acetonitrile (15 mL). Potassium carbonate (173 mg, 1.26 mmol) was added. The temperature was lowered to 0°C, and benzyl chloroformate (172 mg, 1.01 mmol) was slowly added dropwise. The mixture was heated to 25°C and stirred for 2 hours. The reaction was quenched by adding ice water (1 mL). The mixture was extracted with dichloromethane (20 mL × 3). The extracted organic phases were combined and washed with saturated brine (10 mL × 3), dried over anhydrous sodium sulfate, and filtered. The filtrate was concentrated under reduced pressure, and separated by column chromatography (petroleum ether:ethyl acetate = 10:1) to afford compound **70-5.** MS m/z = 278.3 [M+H]⁺.

### Step 5

C ompound **70-5** (150 mg, 0.54 mmol) was dissolved in tetrahydrofuran (10 mL). Lithium bistrimethylsilylamide (0.65 mL, 0.65 mmol, 1 M solution in tetrahydrofuran) was added at 0°C and stirred for 30 minutes. 4-Bromo-1-butene (88 mg, 0.65 mmol) was then slowly added dropwise. The mixture was heated to 25°C and stirred for 6 hours. The reaction was quenched by the addition of saturated ammonium chloride solution (2 mL). The mixture was extracted with dichloromethane (20 mL × 3). The extracted organic phases were combined and washed with saturated brine (10 mL × 3), dried over anhydrous sodium sulfate, and filtered. The filtrate was concentrated under reduced pressure, and separated by column chromatography (petroleum ether:ethyl acetate = 30:1) to afford compound **70-6.** MS m/z = 332.2 [M+H]⁺.

### Step 6

C ompound **70-6** (500 mg, 1.51 mmol) was dissolved in dichloromethane (10 mL). m-Chlorobenzoic acid (781 mg, 4.53 mmol) was slowly added at 0°C. The mixture was heated to 25°C and stirred for 6 hours. The reaction was quenched by the addition of saturated sodium thiosulfate solution (10 mL). The mixture was extracted with dichloromethane (20 mL × 3). The extracted organic phases were combined and washed with saturated brine (10 mL × 3), dried over anhydrous sodium sulfate, and filtered. The filtrate was concentrated under reduced pressure, and separated by column chromatography (petroleum ether:ethyl acetate = 3:1) to obtain compound **70-7.** MS m/z = 348.2 [M+H]⁺.

### Step 7

Compound **70-7** (1.60 g, 4.61 mmol) was dissolved in anhydrous ethanol (10 mL). Palladium / carbon (100 mg, 10% purity) was added, and the mixture was stirred under a hydrogen atmosphere (15 psi) for 16 hours. The reaction solution was filtered. The filtrate was concentrated under reduced pressure and separated by column chromatography (dichloromethane:methanol = 20:1) to obtain compound **70-8A** (a mixture of any 3 of the 8 isomers) (dichloromethane:methanol = 15:1, R_{f} = 0.6), **70-8B** (a mixture of any 2 of the 8 isomers) (dichloromethane:methanol = 15:1, R_{f} = 0.5), and **70-8C** (a mixture of any 3 of the 8 isomers) (dichloromethane:methanol = 15:1, R_{f} = 0.2). MS m/z = 214.2 [M+H]⁺.

### Step 8

C ompound **70-8A** (180 mg, 843.99 µmol) was dissolved in 4 mL of a mixed solvent (acetonitrile:water= 100:0.75). Periodic acid (534.39 mg, 2.34 mmol) and chromium trioxide (28.13 mg, 281.33 µmol) were added at 0°C. The reaction mixture was heated to 25°C and stirred for 16 hours. The mixture was filtered and the filtrate was concentrated under reduced pressure to afford 70-9A. MS m/z = 228.1 [M+H]⁺.

Referring to step 8, compound **70-8B** or **70-8C** was used as a starting material in place of compound **70-8A** to obtain compound **70-9B,** MS m/z = 228.1 [M+H]⁺ or compound **70-9C,** MS m/z = 228.1 [M+H]⁺, respectively.

### Step 9

C ompound **70-9A** (200 mg, 0.80 mmol) was dissolved in N,N-dimethylformamide (4 mL). N,N-diisopropylethylamine (227 mg, 1.76 mmol), N,N,N',N'-tetramethyl-O-(7-azabenzotriazol-1-yl)uronium hexafluorophosphate (435 mg, 1.14 mmol) and 1-(3,4-dimethoxyphenyl)-N-methylmethanamine (160 mg, 0.80 mmol) were added in sequence. The reaction mixture was stirred at 25°C for 6 hours. The reaction solution was concentrated under reduced pressure and separated by column chromatography (dichloromethane:methanol = 20:1) to obtain compound **70-10A.** MS m/z = 391.3 [M+H]⁺.

Referring to step 9, compound **70-9B** or **70-9C** was used as a starting material in place of compound **70-9A** to obtain compound **70-10B,** MS m/z = 391.3 [M+H]⁺ or compound **70-10C,** MS m/z = 391.3 [M+H]⁺, respectively.

### Step 10

Compound **70-10A** (200.00 mg, 0.51 mmol) was dissolved in methanol (4 mL). Sodium methoxide (55 mg, 1.02 mmol) and sodium borohydride (194 mg, 5.12 mmol) were added at 0°C. The mixture was heated to 60°C and stirred for 16 hours. The reaction was quenched by the addition of sodium sulfate decahydrate (1.0 g). The mixture was filtered and concentrated under reduced pressure to obtain compound **70-11A.** MS m/z = 363.3 [M+H]⁺.

Referring to step 10, compound **70-10B** or **70-10C** was used as a starting material in place of compound **70-10A** to obtain compound **70-11B,** MS m/z = 363.3 [M+H]⁺ or compound **70-11C,** MS m/z = 363.3 [M+H]⁺, respectively.

### Step 11

Compound **70-11A** (40.28 mg, 111.12 µmol) was dissolved in anhydrous tetrahydrofuran (4 mL). Sodium hydride (4.44 mg, 185.19 µmol, 60%) was added at 0°C, and the mixture was heated to 25°C with stirring for 1 hour. Compound **4-2** (80 mg, 92.60 µmol) was added, and the mixture was reacted with stirring at 25°C for 4 hours. The reaction was quenched by the addition of water (10 mL), and the product was extracted with ethyl acetate (20 mL x 3), dried over anhydrous sodium sulfate, and filtered. The filtrate was concentrated under reduced pressure, and separated by column chromatography (100% ethyl acetate) to obtain compound **70-12A.** MS m/z = 1162.8 [M+H]⁺.

Referring to step 11, compound **70-11B** or **70-11C** was used as a starting material in place of compound **70-11A** to obtain compound **70-12B,** MS m/z = 1162.8 [M+H]⁺ or compound **70-12C,** MS m/z = 1162.8 [M+H]⁺.

### Step 12

Compound **70-12A** (30 mg, 25.81 µmol) was dissolved in dichloromethane (1 mL). Trifluoroacetic acid (1 mL) was added, and the reaction was stirred at 25°C for 1 hour. The reaction solution was concentrated under reduced pressure to obtain crude product **70A.** The crude product was separated by preparative HPLC (Chromatographic column: Waters SunFire, 250*19 mm, 5 µm; mobile phase: [water (0.1% formic acid)-acetonitrile]; gradient: (acetonitrile): 10%-35%). After drying, the formate salt of compounds **70A1** (single isomer) and the formate salt of **70A2** (a mixture of three isomers) were obtained, respectively. HPLC analysis method (Chromatographic column: Agilent InfinityLab Poroshell 120 EC-C18 4.6*100mm 2.7um; mobile phase A: 90% (5 mM potassium dihydrogen phosphate aqueous solution)-10% acetonitrile); mobile phase B: 90% acetonitrile - 10% water; gradient: B%: 0 - 85%, 9 minutes); compound **70A1,** Rt = 5.792 minutes; compound **70A2,** Rt = 6.000 minutes (peak 1) + 6.089 minutes (peak 2) + 11.8 minutes (peak 3).

Compound **70A1** (single isomer): MS m/z = 672.5 [M+H]⁺. ¹H NMR (400 MHz, CD₃OD) δ ppm 8.49 (s, 1H), 6.91 (d, *J* = 8.0 Hz, 1H), 5.17 (dd, *J =* 12.0, 4.0 Hz, 1H), 4.80 (s, 1H), 4.69 (d, *J =* 16.0 Hz, 1H), 4.24 - 4.16 (m, 2H), 4.11 (d, *J =* 12.0 Hz, 1H), 3.98 (s, 2H), 3.69 (d, *J =* 12.0 Hz, 1H), 3.55 (d, *J =* 16.0 Hz, 1H), 3.39 - 3.34 (m, 1H), 3.21 (dd, *J =* 16.0, 12.0 Hz, 2H), 2.96 - 2.84 (m, 2H), 2.74 (s, 3H), 2.38 - 2.30 (m, 1H), 2.22 (t, *J* = 12.0 Hz, 1H), 2.11 - 1.91 (m, 10H), 1.78 - 1.64 (m, 3H), 1.11 (d, *J =* 8.0 Hz, 3H). Compound **70A2** (mixture of three isomers): MS m/z = 672.5 [M+H]⁺. ¹H NMR (400 MHz, CD₃OD) δ ppm 8.46 (s, 2.98H), 7.01 - 6.84 (m, 1H), 5.18 - 5.06 (m, 2H), 4.80 - 4.68 (m, 1H), 4.63 - 4.51 (m, 1H), 4.23 - 3.99 (m, 6H), 3.87 - 3.71 (m, 1H), 3.64 - 3.50 (m, 1H), 3.38 - 3.35 (m, 1H), 3.15 - 3.09 (m, 1H), 2.95 - 2.90 (m, 1H), 2.76 - 2.75 (m, 3H), 2.37 - 2.28 (m, 1H), 2.12 - 1.93 (m, 11H), 1.77 - 1.66 (m, 3H), 1.13 - 1.10 (m, 3H).

Compound **70-12B** (40 mg, 34.41 µmol) was dissolved in dichloromethane (1 mL). Trifluoroacetic acid (1 mL) was added, and the mixture was stirred at 25°C for 1 hour. The reaction solution was concentrated under reduced pressure to obtain the crude product **70B.** The crude product was separated by preparative high performance liquid chromatography (Chromatographic column: Waters SunFire, 250*19mm, 5µm; mobile phase: [water (0.1% formic acid)-acetonitrile]; gradient: (acetonitrile): 12%-28%). After drying, the formate salt of compound **70B1** (single isomer) and the formate salt of **70B2** (a mixture of two isomers) were obtained, respectively. HPLC analysis method (Chromatographic column: Agilent InfinityLab Poroshell 120 EC-C18 4.6*100mm 2.7um; mobile phase A: 90% (5 mM potassium dihydrogen phosphate aqueous solution)-10% acetonitrile); mobile phase B: 90% acetonitrile - 10% water; gradient: B%: 0 - 85%, 9 minutes); compound **70B1,** Rt = 3.517 minutes; compound **70B2,** Rt = 3.524 minutes (peak 1) + 3.550 minutes (peak 2).

Compound **70B1** (single isomer): MS m/z= 672.5 [M+H]⁺. ¹H NMR (400 MHz, CD₃OD) δ ppm 8.48 (s, 1.21H), 6.91 (d, *J =* 8.0 Hz, 1H), 5.17 (dd, *J =* 12.0, 4.0 Hz, 1H), 4.82 (s, 1H), 4.71 (d, *J =* 16.0 Hz, 1H), 4.36 (d, *J =* 12.0 Hz, 1H), 4.19 (dd, *J =* 20.0, 12.0 Hz, 2H), 4.03 (s, 2H), 3.78 - 3.63 (m, 2H), 3.58 (d, *J =* 13.5 Hz, 1H), 3.37 (s, 1H), 3.24 (dd, *J =* 20.0, 12.0 Hz, 2H), 2.90 (dd, *J =* 16.0, 4.0 Hz, 1H), 2.73 (s, 3H), 2.29 - 2.14 (m, 3H), 2.02 (s, 5H), 1.97 - 1.90 (m, 1H), 1.85 (d, *J* = 12.0 Hz, 2H), 1.80 - 1.74 (m, 2H), 1.70 (dd, *J=* 12.0, 4.0 Hz, 1H), 1.54 (q, *J =* 12.0, 8.0 Hz, 1H), 1.14 (d, *J =* 8.0 Hz, 3H). Compound **70B2** (mixture of 2 isomers): MS m/z = 672.5 [M+H]⁺. ¹H NMR (400 MHz, CD₃OD) δ ppm 8.47 (s, 2H), 6.91 (d, *J =* 8.0 Hz, 1H), 5.19 - 5.16 (m, 1H), 4.82 (s, 1H), 4.73 - 4.68 (m, 1H), 4.38 - 4.13 (m, 3H), 4.05 (s, 2H), 3.76 - 3.57 (m, 3H), 3.39 - 3.34 (m, 1H), 3.26 - 3.20 (m, 2H), 2.93 - 2.86 (m, 1H), 2.74 - 2.73 (m, 3H), 2.28 - 2.18 (m, 2H), 2.10 - 1.95 (m, 7H), 1.89 - 1.66 (m, 5H), 1.59 - 1.49 (m, 1H), 1.15 - 1.10 (m, 3H).

Compound **70-12C** (28 mg, 24.09 µmol) was dissolved in dichloromethane (1 mL). Trifluoroacetic acid (1 mL) was added, and the mixture was stirred at 25°C for 0.5 h. The reaction solution was concentrated under reduced pressure to obtain a crude product, which was then separated by preparative high performance liquid chromatography (Chromatographic column: Xbridge-C18, 250*19mm, 5µm; mobile phase: [water (0.1% ammonia)-acetonitrile]; gradient: (acetonitrile): 35%-80%), and dried to give compound **70C** (a mixture of three isomers). MS m/z = 672.5 [M+H]⁺. ¹H NMR (400 MHz, CD₃OD) δ ppm 6.90 (d, *J* = 8.0 Hz, 1H), 5.14 (dd, *J* = 12.0, 4.0 Hz, 1H), 4.79 (d, *J* = 16.0 Hz, 1H), 4.64 (d, *J* = 12.0 Hz, 1H), 4.13 (d, *J* = 16.0 Hz, 1H), 4.07 - 4.00 (m, 2H), 3.66 - 3.64 (m, 1H), 3.55 - 3.48 (m, 3H), 3.43 - 3.40 (m, 1H), 3.24 - 3.14 (m, 2H), 3.05 - 3.02 (m, 1H), 2.87 - 2.81 (m, 1H), 2.73 (s, 3H), 2.13 - 1.98 (m, 9H), 1.92 - 1.89 (m, 1H), 1.85 - 1.80 (m, 2H), 1.74 - 1.63 (m, 3H), 1.03 (d, *J =* 4.0 Hz, 3H).

### Example 71

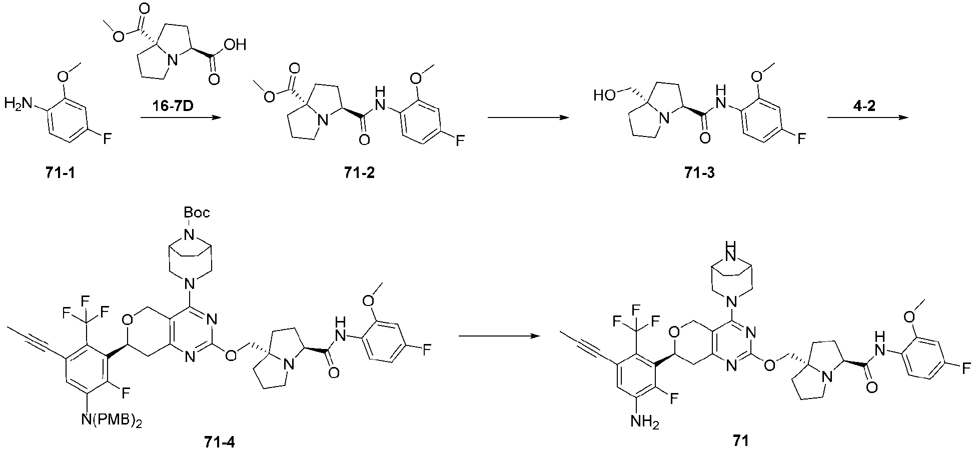

Referring to steps 7 to 10 of Example **16,** compound **71-1** was used as a starting material in place of N-ethyl-4-methoxybenzylamine to obtain crude compound **71.** The crude product was separated by preparative HPLC (Chromatographic column: Xbridge-C18 250 x 19 mm, 5 µm; mobile phase: [water (0.1% ammonia)-acetonitrile]; gradient: (acetonitrile): 60%-80%) and dried to yield compound **71.** MS m/z = 768.5 [M+H]⁺. ¹H NMR (400 MHz, CD₃OD) δ ppm 8.17 (dd, *J =* 8.0, 4.0 Hz, 1H), 6.90 (d, *J =* 12.0 Hz, 1H), 6.80 (dd, *J =* 12.0, 4.0 Hz, 1H), 6.65 (td, *J* = 8.0, 4.0 Hz, 1H), 5.10 (dd, *J =* 8.0, 4.0 Hz, 1H), 4.75 (d, *J* = 12.0 Hz, 1H), 4.60 (d, *J* = 12.0 Hz, 1H), 4.31 (d, *J =* 8.0 Hz, 1H), 4.20 (d, *J =* 12.0 Hz, 1H), 4.05 (d, *J =* 12.0 Hz, 1H), 3.69 (s, 3H), 3.49 - 3.40 (m, 4H), 3.23 - 3.16 (m, 1H), 3.13 - 3.06 (m, 1H), 2.93 (d, *J* = 16.0 Hz, 1H), 2.81 - 2.74 (m, 2H), 2.40 - 2.31 (m, 1H), 2.19 - 2.09 (m, 2H), 2.04 - 1.69 (m, 12H), 1.61 - 1.52 (m, 1H).

### Example 72

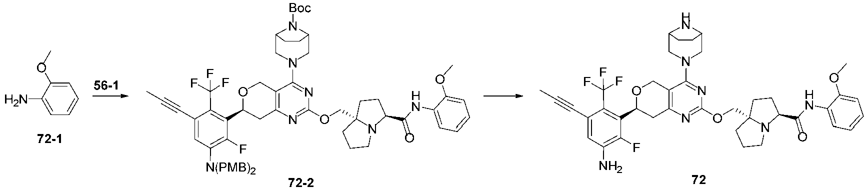

Referring to steps 2 to 3 of Example **56,** compound **72-1** was used as a starting material in place of (S)-3,3-difluorocyclopentylamine hydrochloride to obtain crude compound **72.** The crude product was separated by preparative HPLC (Chromatographic column: Waters Xbridge, 250*19 mm, 5 µm; mobile phase: [water (0.1% ammonia)-acetonitrile]; gradient: (acetonitrile): 55%-80%) and dried to yield compound **72.** MS m/z = 750.5 [M+H]⁺. ¹H NMR (400 MHz, CD₃OD) δ ppm 8.20 (d, *J* = 8.0 Hz, 1H), 7.09 - 7.04 (m, 1H), 6.97 (d, *J* = 8.0 Hz, 1H), 6.94 - 6.89 (m, 2H), 5.11 (dd, *J =* 12.0, 4.0 Hz, 1H), 4.74 (d, *J =* 12.0 Hz, 1H), 4.60 (d, *J =* 12.0 Hz, 1H), 4.32 (d, *J* = 12.0 Hz, 1H), 4.24 (d, *J* = 12.0 Hz, 1H), 4.00 (d, *J* = 12.0 Hz, 1H), 3.72 (s, 3H), 3.49 - 3.40 (m, 4H), 3.25 - 3.19 (m, 1H), 3.13 (dd, *J =* 16.0, 12.0 Hz, 1H), 2.89 (d, *J =* 12.0 Hz, 1H), 2.82 - 2.74 (m, 2H), 2.41 - 2.34 (m, 1H), 2.16 - 1.94 (m, 9H), 1.87 (t, *J =* 8.0 Hz, 1H), 1.82 - 1.77 (m, 2H), 1.75 - 1.69 (m, 2H), 1.58 - 1.53 (m, 1H).

### Example 73

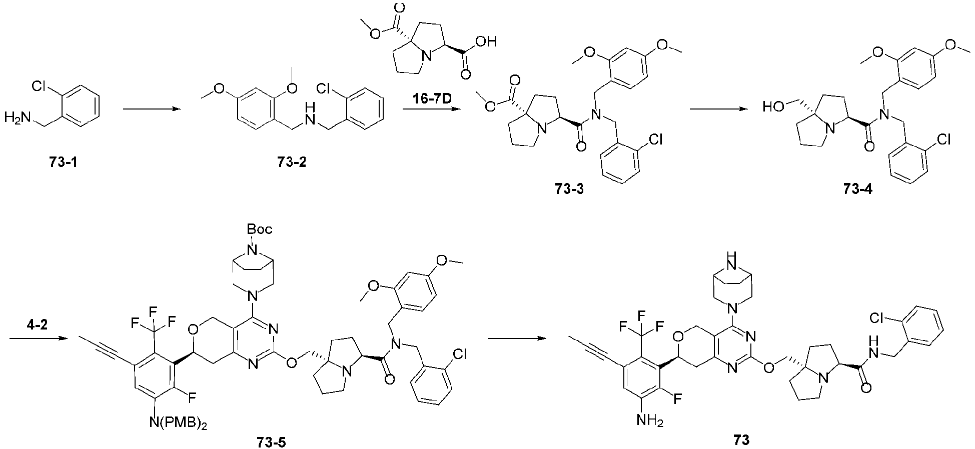

### Step 1

Compound **73-1** (1 g, 7.06 mmol) was dissolved in anhydrous methanol (10 mL). 2,4-dimethoxybenzaldehyde (1.41 g, 8.47 mmol), sodium cyanoborohydride (1.33 g, 21.19 mmol), and acetic acid (42.41 mg, 706.23 µmol) were added. The mixture was stirred at 25°C for 2 hours. The mixture was concentrated under reduced pressure and separated by column chromatography (methanol:dichloromethane = 1:20) to obtain compound **73-2.** MS m/z = 292.1 [M+H]⁺.

### Step 2

Referring to steps 7 to 10 of Example **16,** compound **73-2** was used as a starting material in place of N-ethyl-4-methoxybenzylamine to obtain crude compound **73.** The crude product was separated by preparative HPLC (Chromatographic column: Xtimate C18 150 x 40 mm x 5 µm; mobile phase: [water (0.1% ammonia)-acetonitrile]; gradient: (acetonitrile): 35%-70%) and dried to yield compound **73.** MS m/z = 768.5 [M+H]⁺. ¹H NMR (400 MHz, CD₃OD) δ ppm 7.41 (td, *J =* 8.0, 4.0 Hz, 2H), 7.31 - 7.24 (m, 2H), 6.90 (d, *J =* 8.0 Hz, 1H), 5.14 (dd, *J =* 12.0, 4.0 Hz, 1H), 4.79 (d, *J =* 12.0 Hz, 1H), 4.64 (d, *J =* 16.0 Hz, 1H), 4.54 (d, *J =* 16.0 Hz, 1H), 4.48 (d, *J* = 16.0 Hz, 1H), 4.19 - 4.10 (m, 2H), 4.05 (d, *J =* 8.0 Hz, 1H), 3.83 (dd, *J =* 8.0, 4.0 Hz, 1H), 3.53 - 3.50 (m, 3H), 3.41 (d, *J* = 12.0 Hz, 1H), 3.21 (dd, *J =* 16.0, 12.0 Hz, 1H), 3.03 (d, *J =* 16.0 Hz, 1H), 2.93 - 2.87 (m, 1H), 2.84 (dd, *J =* 16.0, 4.0 Hz, 1H), 2.78 - 2.72 (m, 1H), 2.15 - 2.06 (m, 2H), 2.06 - 1.98 (m, 5H), 1.97 - 1.86 (m, 4H), 1.85 - 1.79 (m, 2H), 1.76 - 1.65 (m, 2H).

### Example 74

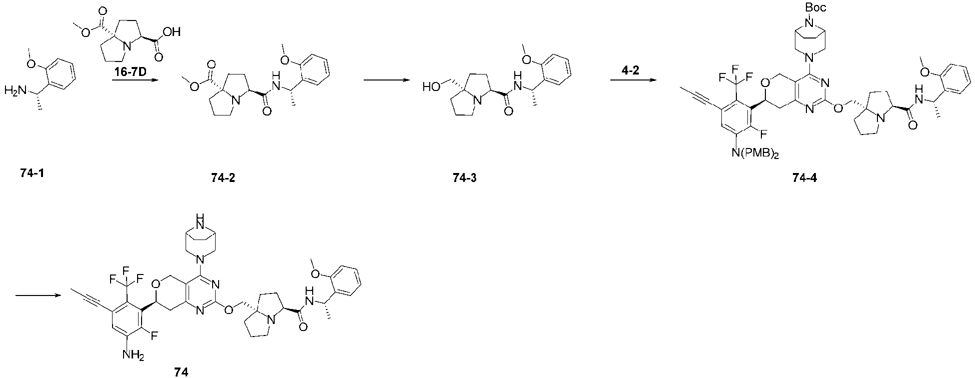

Referring to steps 7 to 10 of Example **16,** compound **74-1** was used as a starting material in place of N-ethyl-4-methoxybenzylamine to obtain crude compound 74. The crude product was separated by preparative HPLC (Chromatographic column: Waters Xbridge, 250 x 19 mm, 5 µm; mobile phase: [water (0.1% ammonia)-acetonitrile]; gradient: (acetonitrile): 45%-70%) and dried to yield compound **74.** MS m/z = 778.6 [M+H]⁺. ¹H NMR (400 MHz, CD₃OD) δ ppm 7.27 - 7.20 (m, 2H), 6.98 - 6.87 (m, 3H), 5.34 - 5.29 (m, 1H), 5.15 (dd, *J =* 12.0, 4.0 Hz, 1H), 4.79 (d, *J =* 16.0 Hz, 1H), 4.65 (d, *J =* 16.0 Hz, 1H), 4.24 - 4.03 (m, 3H), 3.85 (s, 3H), 3.83 - 3.77 (m, 1H), 3.61 - 3.39 (m, 4H), 3.27 - 3.10 (m, 2H), 3.08 - 2.95 (m, 2H), 2.90 - 2.74 (m, 2H), 2.16 - 2.05 (m, 2H), 2.02 (s, 3H), 1.98 - 1.80 (m, 7H), 1.78 - 1.66 (m, 2H), 1.42 (d, *J =* 4.0 Hz, 3H).

### Example 76

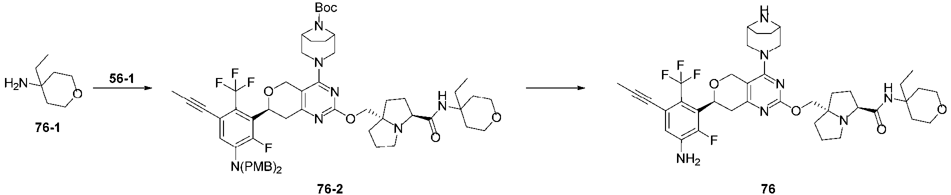

Referring to steps 2 to 3 of Example **56,** compound **76-1** was used as a starting material in place of (S)-3,3-difluorocyclopentylamine hydrochloride to obtain crude compound **76.** The crude product was separated by preparative HPLC (column: Waters Xbridge, 250*19 mm, 5 µm; mobile phase: [water (0.1% ammonia)-acetonitrile]; gradient: (acetonitrile): 45%-70%) and dried to yield compound **76.** MS m/z = 756.5 [M+H]⁺. ¹H NMR (400 MHz, CD₃OD) δ ppm 6.90 (d, *J* = 8.0 Hz, 1H), 5.15 (dd, *J* = 12.0, 4.0 Hz, 1H), 4.80 (d, *J* = 12.0 Hz, 1H), 4.65 (d, *J =* 12.0 Hz, 1H), 4.17 (t, *J =* 12.0 Hz, 2H), 4.08 (d, *J =* 12.0 Hz, 1H), 3.83 (dd, *J =* 8.0, 4.0 Hz, 1H), 3.78 - 3.71 (m, 2H), 3.68 - 3.63 (m, 2H), 3.54 (d, *J* = 12.0 Hz, 3H), 3.43 (d, *J =* 12.0 Hz, 1H), 3.22 (dd, *J =* 16.0, 12.0 Hz, 1H), 3.06 (d, *J =* 12.0 Hz, 1H), 3.02 - 2.95 (m, 1H), 2.88 - 2.79 (m, 2H), 2.20 - 2.06 (m, 4H), 2.05 - 1.83 (m, 12H), 1.77 - 1.68 (m, 3H), 1.63 - 1.53 (m, 2H), 0.86 (t, *J =* 8.0 Hz, 3H).

### Example 77

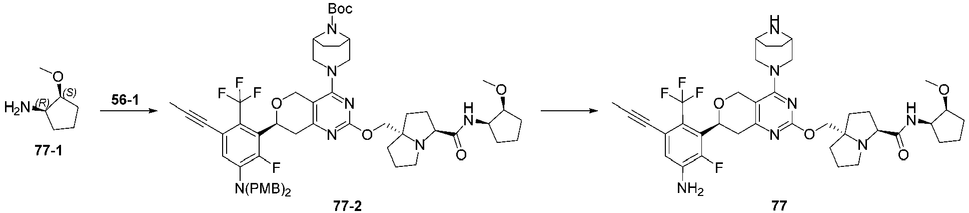

Referring to steps 2 to 3 of Example **56,** compound **77-1** was used as a starting material in place of (S)-3,3-difluorocyclopentylamine hydrochloride to obtain crude compound **77.** The crude product was separated by preparative HPLC (Chromatographic column: Waters Xbridge, 250*19 mm, 5 µm; mobile phase: [water (0.1% ammonia)-acetonitrile]; gradient: (acetonitrile): 45%-70%) and dried to yield compound **77.** MS m/z = 742.5 [M+H]⁺. ¹H NMR (400 MHz, CD₃OD) δ ppm 6.90 (d, *J =* 8.0 Hz, 1H), 5.15 (dd, *J =* 12.0, 4.0 Hz, 1H), 4.80 (d, *J* = 16.0 Hz, 1H), 4.66 (d, *J =* 12.0 Hz, 1H), 4.20 - 4.06 (m, 4H), 3.84 - 3.80 (m, 1H), 3.72 - 3.69 (m, 1H), 3.58 - 3.53 (m, 3H), 3.43 (d, *J =* 12.0 Hz, 1H), 3.30 (s, 3H), 3.22 (dd, *J =* 16.0, 12.0 Hz, 1H), 3.07 - 3.00 (m, 2H), 2.85 (dd, *J =* 20.0, 4.0 Hz, 1H), 2.78 - 2.71 (m, 1H), 2.17 - 2.10 (m, 2H), 2.07 - 1.88 (m, 10H), 1.88 - 1.80 (m, 3H), 1.79 - 1.70 (m, 4H), 1.65 - 1.56 (m, 2H).

### Example 78

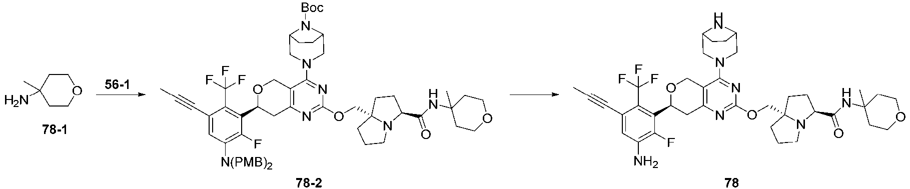

Referring to steps 2 to 3 of Example **56,** compound **78-1** was used as a starting material in place of (S)-3,3-difluorocyclopentylamine hydrochloride to obtain crude compound **78.** The crude product was separated by preparative HPLC (column: xbridge-C18, 250 x 19 mm, 5 µm; mobile phase: [water (0.1% ammonia)-acetonitrile]; gradient: (acetonitrile): 28%-36%) and dried to yield compound **78.** MS m/z = 742.5 [M+H]⁺. ¹H NMR (400 MHz, CD₃OD) δ ppm 6.90 (d, *J* = 8.0 Hz, 1H), 5.14 (dd, *J =* 12.0, 4.0 Hz, 1H), 4.79 (d, *J* = 12.0 Hz, 1H), 4.65 (d, *J* = 12.0 Hz, 1H), 4.19 - 4.13 (m, 2H), 4.06 (d, *J =* 8.0 Hz, 1H), 3.79 - 3.76 (m, 1H), 3.74 - 3.64 (m, 4H), 3.54 - 3.51 (m, 3H), 3.42 (d, *J =* 12.0 Hz, 1H), 3.21 (dd, *J =* 16.0, 12.0 Hz, 1H), 3.05 (d, *J =* 12.0 Hz, 1H), 2.98 - 2.94 (m, 1H), 2.85 (dd, *J =* 16.0, 4.0 Hz, 1H), 2.79 - 2.73 (m, 1H), 2.16 - 2.06 (m, 4H), 2.03 - 1.99 (m, 4H), 1.94 - 1.87 (m, 5H), 1.83 (s, 2H), 1.77 - 1.67 (m, 2H), 1.66 - 1.59 (m, 2H), 1.40 (s, 3H).

### Example 79

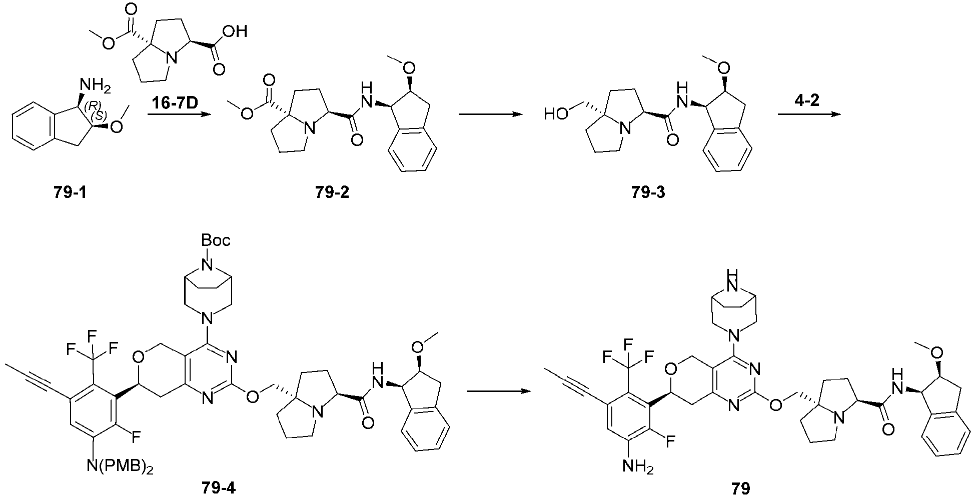

Referring to steps 7 to 10 of Example **16,** compound **79-1** was used as a starting material in place of N-ethyl-4-methoxybenzylamine to obtain crude compound **79.** The crude product was separated by preparative HPLC (Chromatographic column: Xtimate C18, 250 x 19 mm, 5 µm; mobile phase: [water (0.1% ammonia)-acetonitrile]; gradient: (acetonitrile): 40% - 90%) and dried to yield compound **79.** MS m/z = 790.5 [M+H]⁺. ¹H NMR (400 MHz, CD₃OD) δ ppm 7.23 - 7.08 (m, 4H), 6.90 (d, *J* = 12.0 Hz, 1H), 5.45 - 5.32 (m, 1H), 5.14 - 5.07 (m, 1H), 4.80 - 4.74 (m, 1H), 4.61 (t, *J =* 16.0 Hz, 1H), 4.19 - 3.83 (m, 5H), 3.51 - 3.40 (m, 3H), 3.38 (s, 3H), 3.24 - 2.99 (m, 5H), 2.85 - 2.43 (m, 3H), 2.20 - 2.12 (m, 2H), 2.07 - 1.85 (m, 9H), 1.80 - 1.54 (m, 4H).

### Example 80

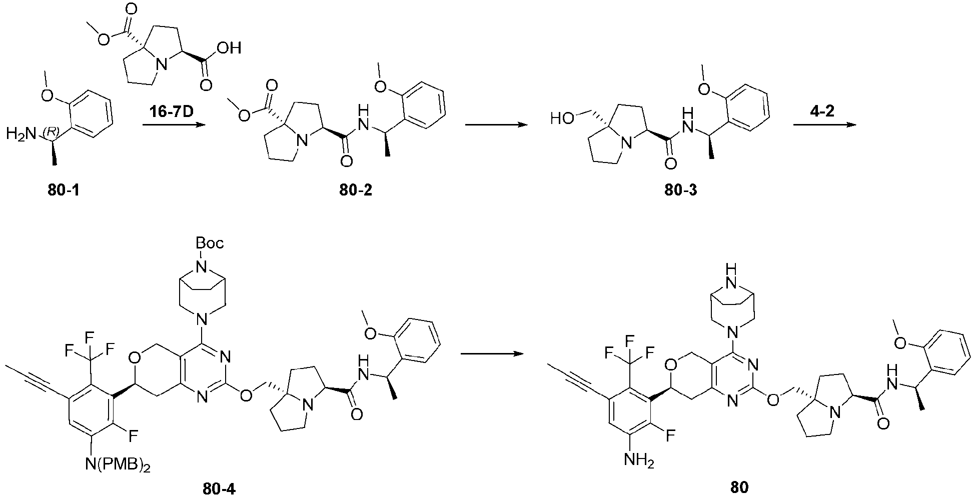

Referring to steps 7 to 10 of Example **16,** compound **80-1** was used as a starting material in place of N-ethyl-4-methoxybenzylamine to obtain crude compound **80.** The crude product was separated by preparative HPLC (column: Waters Xbridge, 250*19 mm, 5 µm; mobile phase: [water (0.1% ammonia)-acetonitrile]; gradient: (acetonitrile): 50%-70%) and dried to yield compound **80.** MS m/z = 778.5 [M+H]⁺. ¹H NMR (400 MHz, CD₃OD) δ ppm 7.32 (dd, *J =* 8.0, 4.0 Hz, 1H), 7.25 - 7.20 (m, 1H), 6.96 - 6.89 (m, 3H), 5.34 (q, *J =* 8.0 Hz, 1H), 5.14 (dd, *J* = 8.0, 4.0 Hz, 1H), 4.79 (d, *J =* 12.0 Hz, 1H), 4.65 (d, *J =* 16.0 Hz, 1H), 4.17 - 4.12 (m, 2H), 4.05 (d, *J* = 8.0 Hz, 1H), 3.85 (s, 3H), 3.80 (dd, *J =* 8.0, 4.0 Hz, 1H), 3.55 - 3.51 (m, 3H), 3.42 (d, *J =* 12.0 Hz, 1H), 3.21 (dd, *J* = 16.0, 12.0 Hz, 1H), 3.04 (d, *J =* 12.0 Hz, 1H), 2.84 (dd, *J =* 16.0, 4.0 Hz, 1H), 2.81 - 2.75 (m, 1H), 2.67 - 2.59 (m, 1H), 2.14 - 2.05 (m, 2H), 2.04 - 1.95 (m, 5H), 1.94 - 1.89 (m, 2H), 1.87 - 1.77 (m, 4H), 1.73 - 1.64 (m, 2H), 1.41 (d, *J =* 8.0 Hz, 3H).

### Example 81

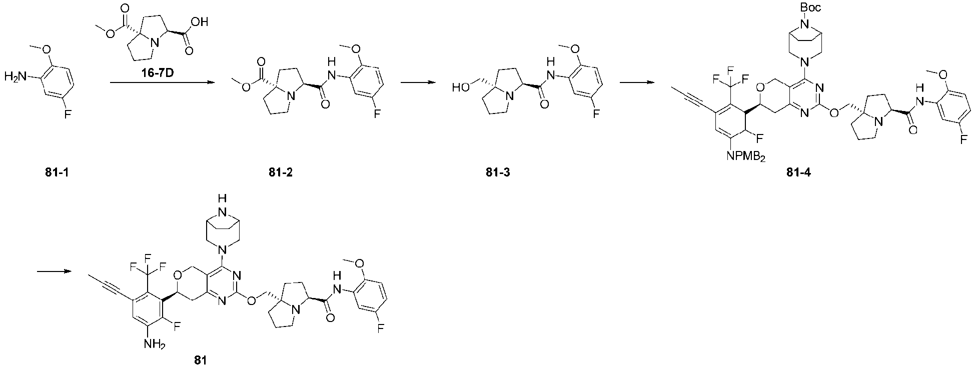

### Step 1

Referring to steps 7 to 10 of Example **16,** compound **81-1** was used as a starting material in place of N-ethyl-4-methoxybenzylamine to obtain crude compound **81.** The crude product was separated by preparative HPLC (Chromatographic column: Waters Xbridge, 250 x 19 mm, 5 µm; mobile phase: [water (0.1% ammonia)-acetonitrile]; gradient: (acetonitrile): 45%-70%) and dried to yield compound **81.** MS m/z = 768.5 [M+H]⁺. ¹H NMR (400 MHz, CD₃OD) δ ppm 7.98 (dd, *J =* 12.0, 4.0 Hz, 1H), 6.98 (dd, *J =* 8.0, 4.0 Hz, 1H), 6.90 (d, *J* = 8.0 Hz, 1H), 6.83 - 6.78 (m, 1H), 5.15 (dd, *J =* 12.0, 4.0 Hz, 1H), 4.81 - 4.79 (m, 1H), 4.66 (d, *J =* 12.0 Hz, 1H), 4.24 (d, *J* = 12.0 Hz, 1H), 4.17 - 4.10 (m, 2H), 4.03 (dd, *J =* 8.0, 4.0 Hz, 1H), 3.88 (s, 3H), 3.58 - 3.54 (m, 3H), 3.49 - 3.43 (m, 1H), 3.27 - 3.20 (m, 1H), 3.06 (d, *J =* 12.0 Hz, 1H), 3.01 - 2.98 (m, 1H), 2.86 (dd, *J =* 16.0, 4.0 Hz, 1H), 2.80 - 2.73 (m, 1H), 2.22 - 2.14 (m, 2H), 2.06 - 2.02 (m, 5H), 1.97 - 1.90 (m, 4H), 1.88 - 1.81 (s, 2H), 1.80 - 1.72 (m, 2H).

### Example 82

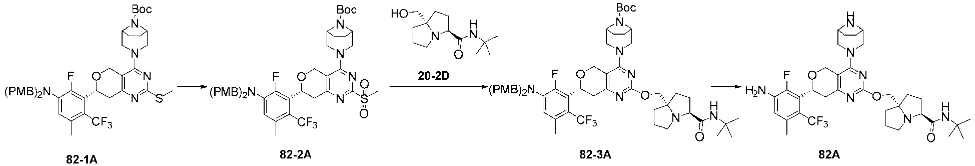

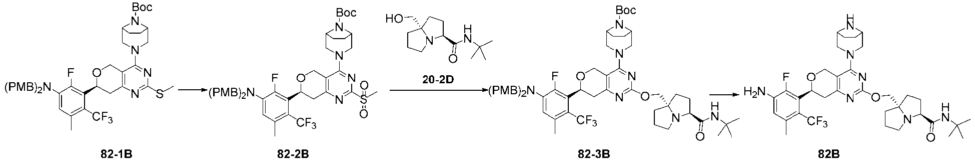

### Step 1

At 0°C, m-chloroperbenzoic acid (344.96 mg, 1.70 mmol, 85% purity) was slowly added to a solution of compound **82-1A** (700 mg, 0.85 mmol) in tetrahydrofuran (10 mL). The temperature was raised to 25°C and the reaction mixture was allowed to react for 1 hour. A 5% sodium thiosulfate solution (4 mL) was added to the reaction mixture, and the mixture was extracted with dichloromethane (10 mL x 2). The organic phases were combined, dried over anhydrous sodium sulfate, filtered, and concentrated. The crude product was purified by column chromatography (petroleum ether:ethyl acetate = 2:1) to obtain compound **82-2A.** MS m/z = 856.5 [M+H]⁺.

Referring to step 1, compound **82-1B** was used as a starting material in place of compound **82-1A** to obtain compound **82-2B.** MS m/z = 856.5 [M+H]⁺.

### Step 2

Sodium hydride (25.72 mg, 0.64 mmol, 60% purity) was slowly added to a solution of compound **20-2D** (30.89 mg, 0.13 mmol) in tetrahydrofuran (5 mL) at 0°C. The reaction was stirred at 0°C for 0.5 h. A solution of compound **82-2A** (110 mg, 0.13 mmol) in tetrahydrofuran (5 mL) was then added to the above system, and the mixture was warmed to 25°C and stirred for 1 h. The reaction was quenched by adding saturated aqueous ammonium chloride (5 mL) and extracted with ethyl acetate (6 mL x 2). The extracted organic phases were combined and washed with saturated brine (8 mL), dried over anhydrous sodium sulfate, filtered, and concentrated under reduced pressure. Column chromatography (dichloromethane:methanol = 20:1) afforded compound **82-3A.** MS m/z = 1016.7 [M+H]⁺.

Referring to step 2, compound **82-2B** was used as a starting material in place of compound **82-2A** to obtain compound **82-3B.** MS m/z = 1016.7 [M+H]⁺.

### Step 3

Compound **82-3A** (105 mg, 0.11 mmol) was added to trifluoroacetic acid (5 mL) and stirred at 25°C for 1 hour. The reaction solution was concentrated under reduced pressure, and the crude product was separated by preparative HPLC (Chromatographic column: Waters Xbridge, 250*19 mm, 5 µm; mobile phase: [water (0.1% ammonia)-acetonitrile]; (acetonitrile): 40%-70%) and dried to obtain compound **82A.** MS m/z = 676.5 [M+H]⁺. ¹H NMR (400 MHz, CD₃OD) δ ppm 6.68 (d, *J =* 8.0 Hz, 1H), 5.20 - 5.13 (m, 1H), 4.79 (d, *J =* 12.0 Hz, 1H), 4.65 (d, *J =* 12.0 Hz, 1H), 4.20 - 4.10 (m, 2H), 4.05 (d, *J =* 12.0 Hz, 1H), 3.73 - 3.67 (m, 1H), 3.56 - 3.47 (m, 3H), 3.45 - 3.39 (m, 1H), 3.29 - 3.20 (m, 1H), 3.04 (d, *J =* 16.0 Hz, 1H), 2.96 - 2.89 (m, 1H), 2.89 - 2.80 (m, 1H), 2.79 - 2.70 (m, 1H), 2.39 - 2.32 (m, 3H), 2.15 - 2.04 (m, 2H), 2.04 - 1.97 (m, 1H), 1.95 - 1.80 (m, 7H), 1.76 - 1.65 (m, 2H), 1.36 (s, 9H).

Compound **82-3B** (120 mg, 0.11 mmol) was added to trifluoroacetic acid (5 mL) and stirred at 25°C for 1 hour. The reaction solution was concentrated under reduced pressure, and the crude product was separated by preparative HPLC (Chromatographic column: Waters Xbridge, 250*19 mm, 5 µm; mobile phase: [water (0.1% ammonia)-acetonitrile]; (acetonitrile): 40%-70%). After drying, compound **82B** was obtained. MS m/z = 676.5 [M+H]⁺. ¹H NMR (400 MHz, CD₃OD) δ ppm 6.68 (d, *J =* 8.0 Hz, 1H), 5.21 - 5.13 (m, 1H), 4.80 (d, *J* = 12.0 Hz, 1H), 4.64 (d, *J* = 12.0 Hz, 1H), 4.19 - 4.10 (m, 2H), 4.07 (d, *J =* 8.0 Hz, 1H), 3.73 - 3.67 (m, 1H), 3.57 - 3.47 (m, 3H), 3.45 - 3.38 (m, 1H), 3.28 - 3.18 (m, 1H), 3.07 - 2.99 (m, 1H), 2.95 - 2.82 (m, 2H), 2.78 - 2.69 (m, 1H), 2.39 - 2.32 (m, 3H), 2.16 - 1.97 (m, 3H), 1.96 - 1.79 (m, 7H), 1.75 - 1.64 (m, 2H), 1.35 (s, 9H).

### Example 83

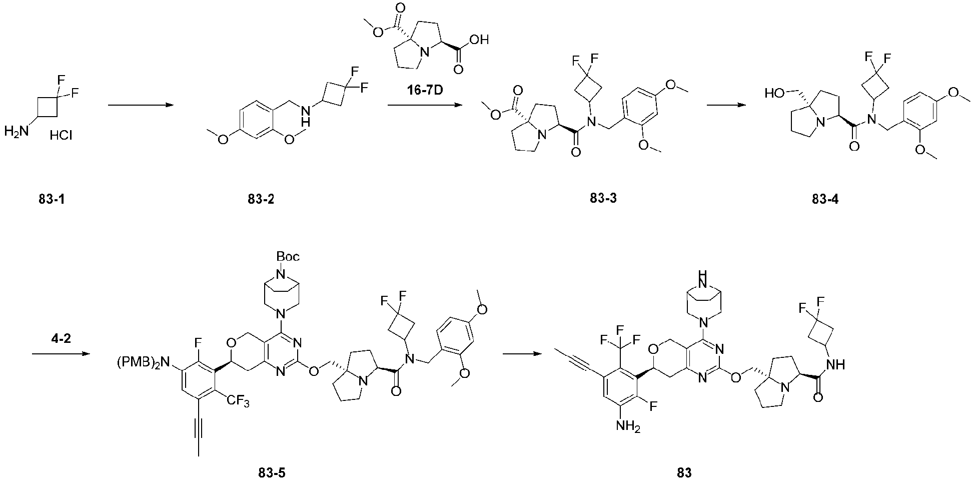

### Step 1

Compound **83-1** (100 mg, 696.56 µmol) was weighed and methanol (1 mL) was added. 2,4-Dimethoxybenzaldehyde (104.17 mg, 626.90 µmol) was added under ice-cooling. After stirring for 0.5 hours, sodium cyanoborohydride (87.55 mg, 1.39 mmol) was added and the reaction was stirred at room temperature for 15 hours. The mixture was quenched with water (3 mL) and extracted with ethyl acetate (2 mL x 3). The organic phase was washed with saturated brine (3 mL x 3), dried over anhydrous sodium sulfate, and concentrated under reduced pressure. The crude product was purified by column chromatography (dichloromethane:methanol = 15:1) to obtain **83-2.** MS m/z = 258.1 [M+H]⁺.

### Step 2

Referring to steps 7 to 10 of Example **16,** compound **83-2** was used as a starting material in place of N-ethyl-4-methoxybenzylamine to obtain crude compound **83.** The crude product was separated by preparative HPLC (Chromatographic column: Waters Xbridge, 250 x 19 mm, 5 µm; mobile phase: [water (0.1% ammonia)-acetonitrile]; gradient: (acetonitrile): 45%-70%) and dried to yield compound **83.** MS m/z = 734.5 [M+H]⁺. ¹H NMR (400 MHz, CD₃OD) δ ppm 6.90 (d, *J =* 8.0 Hz, 1H), 5.17 - 5.13 (m, 1H), 4.82 - 4.78 (m, 1H), 4.67 - 6.64 (m, 1H), 4.18 - 4.14 (m, 3H), 4.07 - 4.04 (m, 1H), 3.78 - 3.75 (m, 1H), 3.62 (s, 2H), 3.57 - 3.54 (m, 1H), 3.47 - 3.44 (m, 1H), 3.26 - 3.19 (m, 1H), 3.09 - 3.06 (m, 1H), 2.98 - 2.82 (m, 4H), 2.77 - 2.71 (m, 1H), 2.62 - 2.49 (m, 2H), 2.13 - 2.04 (m, 3H), 2.02 - 1.99 (m, 4H), 1.96 - 1.85 (m, 6H), 1.77 - 1.70 (m, 2H).

### Example 84

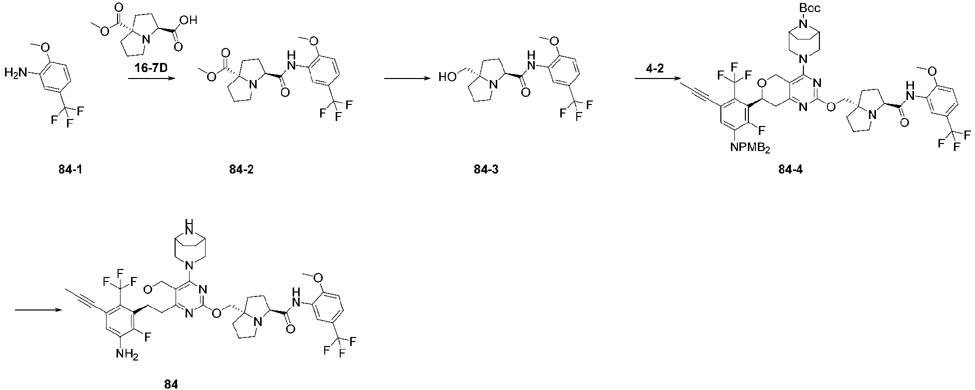

Referring to steps 7 to 10 of Example **16,** compound **84-1** was used as a starting material in place of N-ethyl-4-methoxybenzylamine to obtain crude compound **84.** The crude product was separated by preparative HPLC (Chromatographic column: Waters Xbridge, 250 x 19 mm, 5 µm; mobile phase: [water (0.1% ammonia)-acetonitrile]; gradient: (acetonitrile): 45%-70%) and dried to yield compound **84.** MS m/z = 818.5 [M+H]⁺. ¹H NMR (400 MHz, CD₃OD) *δ* ppm 8.49 (s, 1H), 7.41 (d, *J =* 8.0 Hz, 1H), 7.17 (d, *J =* 8.0 Hz, 1H), 6.90 (d, *J =* 8.0 Hz, 1H), 5.15 (dd, *J =* 12.0, 4.0 Hz, 1H), 4.82 - 4.79 (m, 1H), 4.66 (d, *J =* 12.0 Hz, 1H), 4.24 (d, *J =* 8.0 Hz, 1H), 4.17 - 4.03 (m, 3H), 3.97 (s, 3H), 3.58 - 3.52 (m, 3H), 3.46 - 3.41 (m, 1H), 3.27 - 3.19 (m, 1H), 3.10 - 2.96 (m, 2H), 2.90 - 2.74 (m, 2H), 2.28-2.13 (m, 2H), 2.11-1.98 (m, 6H), 1.97-1.91 (m, 3H), 1.87-1.68 (m, 4H).

### Example 86

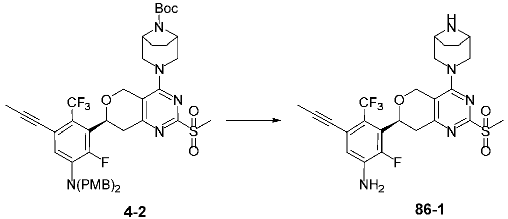

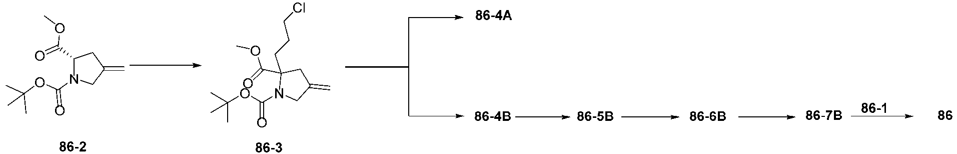

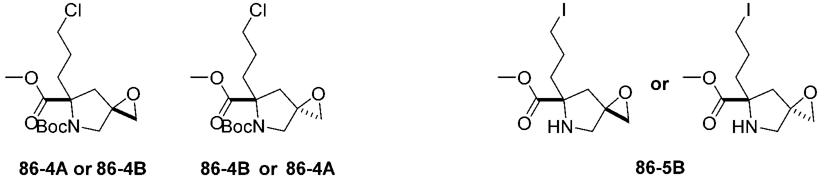

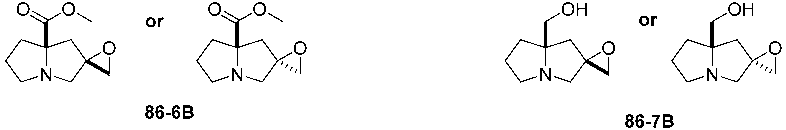

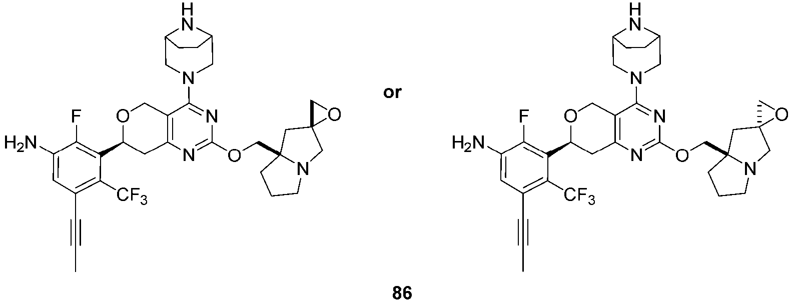

### Step 1

Compound **4-2** (100 mg, 113.64 µmol) was dissolved in dichloromethane (1 mL), and trifluoroacetic acid (0.2 mL) was added. The mixture was stirred at 25°C for 1 hour. The reaction solution was concentrated under reduced pressure, dissolved in dichloromethane (5 mL), and washed with saturated sodium bicarbonate solution (10 mL x 3). The organic phase was dried over anhydrous sodium sulfate, filtered, and concentrated under reduced pressure to obtain compound **86-1.** MS m/z = 540.4 [M+H]⁺.

### Step 2

Compound **86-2** (1 g, 4.15 mmol) was weighed and anhydrous tetrahydrofuran (15 mL) was added. The mixture was cooled to -70°C under nitrogen purge and then lithium hexamethyldisilazide (8.3 mL, 8.30 mmol, 1 M solution in tetrahydrofuran) was added. After stirring for 0.5 hours, 1-chloro-3-iodopropane (1.27 g, 6.22 mmol) was added and allowed to react at -70°C for two hours. The reaction was quenched by addition of saturated aqueous ammonium chloride (10 mL). The mixture was extracted with ethyl acetate (30 mL x 3) and separated by column chromatography (petroleum ether:ethyl acetate = 10:1) to afford compound **86-3.** MS m/z = 318.3 [M+H]⁺.

### Step 3

Compound **86-3** (40 mg, 126.18 µmol) was dissolved in dichloromethane (8 mL), and m-chloroperbenzoic acid (43.40 mg, 252.36 µmol) was added. The mixture was stirred at room temperature for 2 hours. Column chromatography (petroleum ether:ethyl acetate = 30:1) afforded compound **86-4A** (petroleum ether:ethyl acetate = 10:1, Rf = 0.45), MS m/z = 334.3 [M+H]+, and compound **86-4B** (petroleum ether:ethyl acetate = 10:1, Rf = 0.40). MS m/z =334.3 [M+H]⁺.

### Step 4

Compound **86-4B** (1 g, 3.00 mmol) was dissolved in methanol (20 mL). The mixture was cooled to 0°C and iodotrimethylsilane (899.14 mg, 4.49 mmol) was added. The mixture was reacted at 50°C for 2 hours. The reaction solution was directly concentrated and separated by column chromatography (dichloromethane:methanol = 1:10) to obtain compound **86-5B.** MS m/z = 326.1 [M+H]⁺.

### Step 5

Compound **86-5B** (100 mg, 429.18 µmol) was weighed and acetonitrile (3 mL) was added. Potassium carbonate (118.4 mg, 858.36 µmol) was added and reacted at 60°C for 4 hours. The reaction solution was directly concentrated and separated by column chromatography (dichloromethane:methanol = 10:1) to obtain compound **86-6B.** MS m/z = 198.2 [M+H]⁺.

### Step 6

Compound **86-6B** (100 mg, 0.51 mmol) was weighed and anhydrous tetrahydrofuran (3 mL) was added under nitrogen. The mixture was cooled to -40°C, and lithium aluminum tetrahydride (0.41 mL, 1.02 mmol, 2.5 M solution in tetrahydrofuran) was added. The mixture was allowed to react at -40°C for 15 minutes. Dichloromethane (5 mL) was added for dilution. At 0°C, water (0.2 mL) was added to the reaction mixture, and 15% sodium hydroxide solution (0.2 mL) was added to quench the reaction. The reaction mixture was stirred for 10 minutes, and filtered. The filter cake was washed with dichloromethane (10 mL). The filtrate was dried over anhydrous sodium sulfate and concentrated to obtain compound **86-7B.** MS m/z = 170.1 [M+H]⁺.

### Step 7

Compound **86-7B** (30 mg, 176.47 µmol) was dissolved in tetrahydrofuran (1 mL). Sodium hydride (10 mg, 60% purity) was added at 0°C and stirred at 25°C for 0.5 hours. Compound **86-1** (95 mg, 176.47 µmol) was then added and stirred at room temperature for 1 hour. The reaction was quenched with water (5 mL) and extracted with ethyl acetate (6 mL x 5). The organic phase was dried over anhydrous sodium sulfate, filtered, and concentrated under reduced pressure. The crude product was separated by preparative HPLC (column: Waters Xbridge, 250 x 19 mm, 5 µm; mobile phase: [water (0.1% ammonia)-acetonitrile]; gradient: (acetonitrile): 45%-70%), and dried to yield compound **86.** MS m/z = 629.4 [M+H]⁺. ¹H NMR (400 MHz, CD₃OD) δ ppm 6.90 (d, *J* = 8.0 Hz, 1H), 5.14 (dd, *J =* 12.0, 4.0 Hz, 1H), 4.79 (d, *J =* 16.0 Hz, 1H), 4.64 (d, *J =* 12.0 Hz, 1H), 4.29 - 4.26 (m, 1H), 4.24 - 4.10 (m, 2H), 3.52 - 3.48 (m, 2H), 3.47 - 3.38 (m, 1H), 3.27 - 3.18 (m, 1H), 3.18 - 3.08 (m, 2H), 3.04 (d, *J =* 12.0 Hz, 1H), 2.94 (d, *J =* 12.0 Hz, 1H), 2.91 - 2.79 (m, 3H), 2.77 - 2.71 (m, 1H), 2.23 - 2.19 (m, 1H), 2.17 - 2.07 (m, 1H), 2.04 - 1.99 (m, 6H), 1.97 - 1.75 (m, 5H), 1.74 - 1.59 (m, 1H).

### Example 87

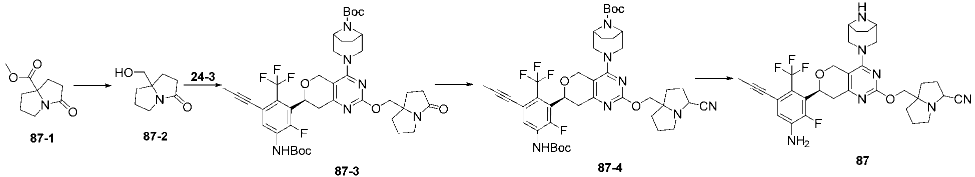

### Step 1

Compound **87-1** (800 mg, 4.37 mmol) was dissolved in anhydrous tetrahydrofuran (10 mL) and cooled to 0°C under nitrogen. Lithium borohydride (190.25 mg, 8.73 mmol) was then added. The mixture was heated to 25°C and stirred for 4 hours. The reaction was quenched by adding water (8 mL), and filtered. The filter cake was washed with acetonitrile (20 mL). The filtrate was concentrated under reduced pressure to obtain compound **87-2.** MS m/z = 156.1 [M+H]⁺.

### Step 2

Compound **87-2** (150 mg, 966.53 µmol) was dissolved in anhydrous tetrahydrofuran (8 mL), and cooled to 0°C. Sodium hydride (46.39 mg, 1.93 mmol, 60% purity) was slowly added. The mixture was heated to 25°C and the reaction was stirred for 0.5 hours. Compound **24-3** (489.69 mg, 676.57 µmol) was added, and the mixture was reacted at 25°C with stirring for 2 hours. Water (4 mL) was added to quench the reaction, and the mixture was extracted with ethyl acetate (5 mL x 3). The organic phases were combined, washed with saturated brine (10 mL), dried over anhydrous sodium sulfate, filtered, and concentrated under reduced pressure. Column chromatography (petroleum ether:ethyl acetate = 1:1) afforded compound **87-3.** MS m/z = 815.6 [M+H]⁺.

### Step 3

Compound **87-3** (500 mg, 613.60 µmol) was dissolved in anhydrous tetrahydrofuran (10 mL), and cooled to 0°C. LDBIPA (lithium diisobutylisopropylaluminum hydride) (10 mL, 0.44 M solution in tetrahydrofuran) was slowly added. The mixture was heated to 25°C and the reaction was stirred for 8 hours. After the disappearance of the starting material detected by thin layer chromatography (petroleum ether:ethyl acetate = 1:1), trimethylsilyl cyanide (304.36 mg, 3.07 mmol) was slowly added and the reaction was stirred at 25°C for additional 4 hours. The reaction was quenched by the addition of water (10 mL), and extracted with ethyl acetate (15 mL x 3). The organic phases were combined, washed with saturated brine (10 mL), dried over anhydrous sodium sulfate, filtered, and concentrated under reduced pressure. Column chromatography (petroleum ether:ethyl acetate = 10:1) afforded compound **87-4.** MS m/z = 826.6 [M+H]⁺.

### Step 4

Compound **87-4** (80 mg, 96.87 µmol) was dissolved in methanol (3 mL), and trimethylsilyl iodide (96.91 mg, 484.33 µmol) was added at room temperature. The mixture was heated to 50°C and stirred for 2 hours. The reaction solution was concentrated under reduced pressure to obtain a crude product. The crude product was separated by preparative HPLC (Chromatographic column: Xbridge-C18 250 * 19 mm, 5 µm; mobile phase: [water (0.1% ammonia)-acetonitrile]; gradient: (acetonitrile): 45%-85%) and concentrated under reduced pressure to obtain compound **87.** MS m/z = 626.5 [M+H]⁺. ¹H NMR (400 MHz, CD₃OD) δ ppm 6.90 (d, *J =* 8.0 Hz, 1H), 5.14 (dd, *J =* 12.0, 4.0 Hz, 1H), 4.79 (d, *J =* 16.0 Hz, 1H), 4.68 - 4.62 (m, 1H), 4.16 - 4.09 (m, 2H), 4.06 (d, *J* = 8.0 Hz, 1H), 3.61 - 3.51 (m, 3H), 3.46 - 3.39 (m, 1H), 3.24 - 3.16 (m, 1H), 3.09 - 3.02 (m, 2H), 2.87 - 2.80 (m, 1H), 2.43 - 1.93 (m, 11H), 1.91 - 1.79 (m, 4H), 1.75 - 1.67 (m, 2H).

### Example 88

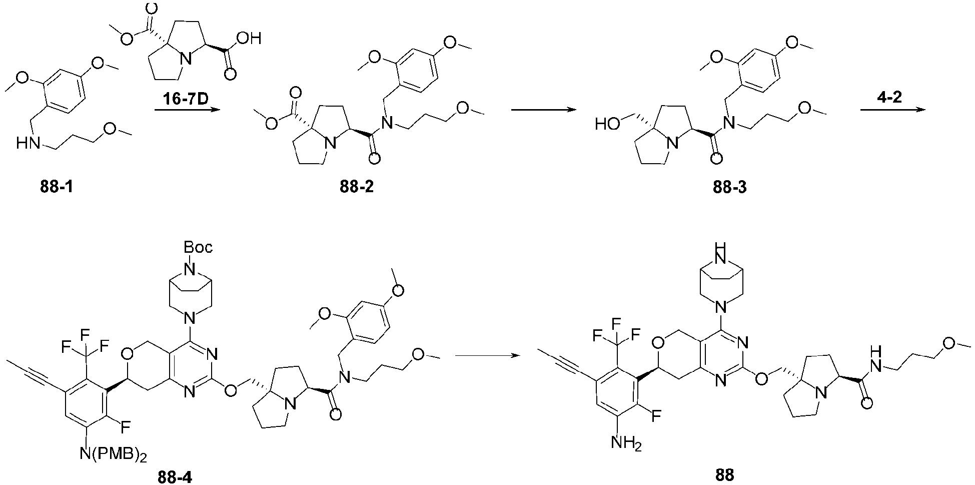

Referring to steps 7 to 10 of Example **16,** compound **88-1** was used as a starting material in place of N-ethyl-4-methoxybenzylamine to obtain crude compound **88.** The crude product was separated by preparative HPLC (Chromatographic column: Waters Xbridge, 250 x 19 mm, 5 µm; mobile phase: [water (0.1% ammonia)-acetonitrile]; gradient: (acetonitrile): 45%-70%) and dried to yield compound **88.** MS m/z = 716.5 [M+H]⁺. ¹H NMR (400 MHz, CD₃OD) δ ppm 6.90 (d, *J* = 8.0 Hz, 1H), 5.15 (dd, *J =* 12.0 Hz, 4.0 Hz, 1H), 4.80 (d, *J =* 16.0 Hz, 1H), 4.66 (d, *J* = 16.0 Hz, 1H), 4.17 (t, *J =* 12.0 Hz, 2H), 4.07 (d, *J =* 12.0 Hz, 1H), 3.79 - 3.76 (m, 1H), 3.63 (s, 2H), 3.56 (d, *J =* 12.0 Hz, 1H), 3.46 - 3.43 (m, 3H), 3.36 - 3.32 (m, 4H), 3.28 - 3.25 (m, 1H), 3.24 - 3.19 (m, 1H), 3.07 (d, *J =* 12.0 Hz, 1H), 2.96 - 2.93 (m, 1H), 2.85 (dd, *J =* 16.0, 4.4 Hz, 1H), 2.80 - 2.74 (m, 1H), 2.14 - 2.08 (m, 2H), 2.05 - 1.99 (m, 5H), 1.96 - 1.86 (m, 6H), 1.81 - 1.71 (m, 4H).

### Example 89

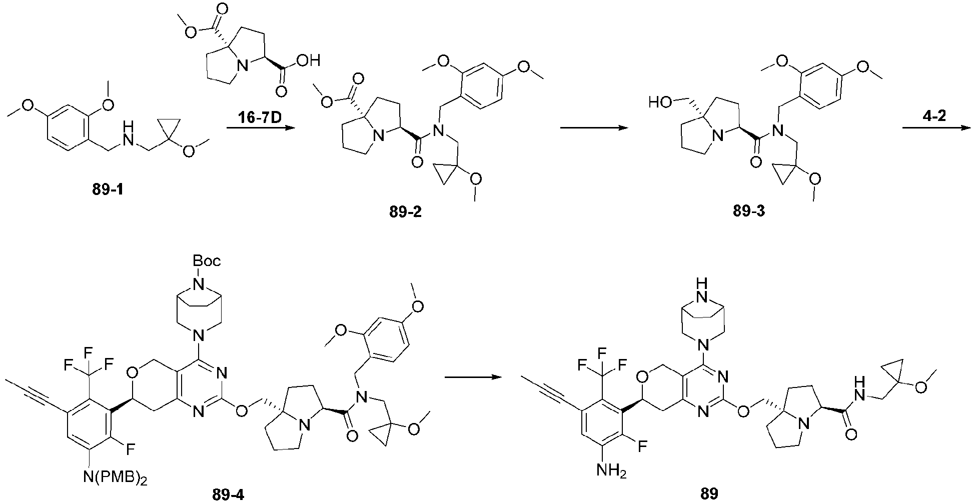

Referring to steps 7 to 10 of Example **16,** compound **89-1** was used as a starting material in place of N-ethyl-4-methoxybenzylamine to obtain crude compound **89.** The crude product was separated by preparative HPLC (Chromatographic column: Waters Xbridge, 250*30 mm, 5 µm; mobile phase: [water (0.1% ammonia)-acetonitrile]; gradient: (acetonitrile): 50%-80%) and dried to yield compound **89.** MS m/z = 728.5 [M+H]⁺. ¹H NMR (400 MHz, DMSO-*d₆*) δ ppm 8.05 (t, *J =* 4.0 Hz, 1H), 6.87 (d, *J =* 8.0 Hz, 1H), 6.02 (s, 2H), 5.02 (dd, *J* = 12.0, 4.0 Hz, 1H), 4.74 (d, *J =* 12.0 Hz, 1H), 4.54 (d, *J =* 16.0 Hz, 1H), 3.92 (t, *J =* 12.0 Hz, 2H), 3.84 (d, *J =* 8.0 Hz, 1H), 3.60 (t, *J =* 4.0 Hz, 1H), 3.42 - 3.37 (m, 3H), 3.25 - 3.20 (m, 2H), 3.19 (s, 3H), 3.03 (dd, *J =* 16.0, 8.0 Hz, 1H), 2.86 (d, *J =* 12.0 Hz, 1H), 2.80 - 2.72 (m, 2H), 2.67 - 2.63 (m, 1H), 2.03 (s, 3H), 2.01 - 1.94 (m, 1H), 1.93 - 1.71 (m, 8H), 1.65 - 1.48 (m, 4H), 0.68 - 0.58 (m, 2H), 0.56 - 0.48 (m, 2H).

### Example 90

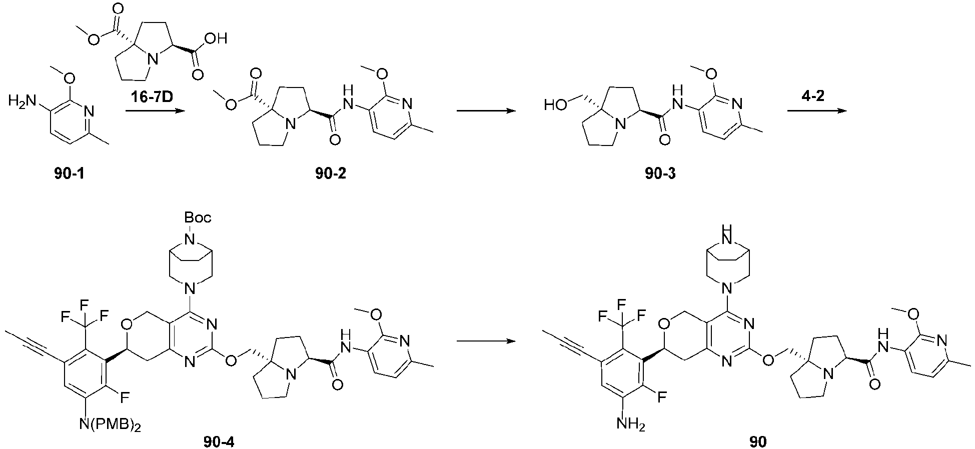

Referring to steps 7 to 10 of Example **16,** compound **90-1** was used as a starting material in place of N-ethyl-4-methoxybenzylamine to obtain crude compound **90.** The crude product was separated by preparative HPLC (Chromatographic column: Waters Xbridge, 250 x 19 mm, 5 µm; mobile phase: [water (0.1% ammonia)-acetonitrile]; gradient: (acetonitrile): 60%-90%) and dried to yield compound **90.** MS m/z = 765.6 [M+H]⁺. ¹H NMR (400 MHz, CD₃OD) δ ppm 8.20 (d, *J =* 8.0 Hz, 1H), 6.90 (d, *J =* 8.0 Hz, 1H), 6.77 (d, *J =* 4.0 Hz, 1H), 5.15 (dd, *J =* 12.0, 4.0 Hz, 1H), 4.81 (d, *J =* 16.0 Hz, 1H), 4.66 (d, *J =* 16.0 Hz, 1H), 4.23 (d, *J =* 12.0 Hz, 1H), 4.15 (d, *J* = 12.0 Hz, 1H), 4.10 (d, *J =* 12.0 Hz, 1H), 4.03 - 3.99 (m, 1H), 3.98 (s, 3H), 3.54 (d, *J* = 8.0 Hz, 3H), 3.43 (d, *J* = 12.0 Hz, 1H), 3.23 (dd, *J =* 16.0, 8.0 Hz, 1H), 3.08 - 2.96 (m, 2H), 2.86 (dd, *J =* 20.0, 4.0 Hz, 1H), 2.81 - 2.74 (m, 1H), 2.39 (s, 3H), 2.23 - 2.11 (m, 2H), 2.08 - 1.99 (m, 5H), 1.98 - 1.87 (m, 4H), 1.86 - 1.65 (m, 4H).

### Example 91

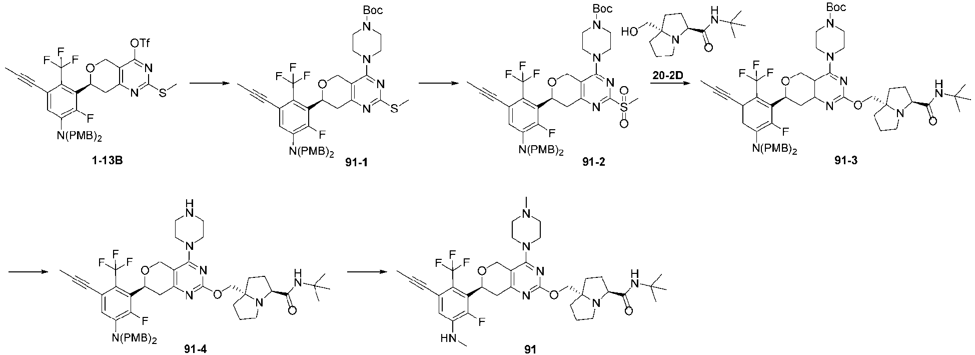

### Step 1

C ompound **1-13B** (300 mg, 381.80 µmol) was dissolved in N,N-dimethylformamide (10 mL). N,N-diisopropylethylamine (148.04 mg, 1.15 mmol) and 1-tert-butyloxycarbonylpiperazine (142.22 mg, 763.60 µmol) were added sequentially at room temperature. The mixture was heated to 50°C and stirred for 1 hour. The reaction was quenched by the addition of water (10 mL). The mixture was extracted with ethyl acetate (20 mL x 3). The extracted organic phases were combined and washed with saturated brine (10 mL), dried over anhydrous sodium sulfate, and filtered. The filtrate was concentrated under reduced pressure, and separated by column chromatography (petroleum ether:ethyl acetate = 5:1) to obtain compound **91-1.** MS m/z = 822.6 [M+H]⁺.

### Step 2

C ompound **91-1** (130 mg, 158.17 µmol) was dissolved in dichloromethane (10 mL), and m-chloroperbenzoic acid (68.24 mg, 395.42 µmol) was added. The mixture was stirred at 25°C for 1 hour. The reaction was quenched by adding water (10 mL), and extracted with ethyl acetate (20 mL x 3). The extracted organic phases were combined and washed with saturated brine (10 mL), dried over anhydrous sodium sulfate, and filtered. The filtrate was concentrated under reduced pressure, and separated by column chromatography (petroleum ether:ethyl acetate = 3:1) to obtain compound **91-2.** MS m/z = 854.6 [M+H]⁺.

### Step 3

Compound **20-2D** (28.15 mg, 117.11 µmol) was dissolved in anhydrous tetrahydrofuran (3 mL), and cooled to 0°C. Sodium hydride (14.05 mg, 351.32 µmol, 60%) was added. The mixture was stirred at 0°C for half an hour. Compound **91-2** (100 mg, 117.11 µmol) was added, and the mixture was heated to 25°C with stirring for 1 hour. The reaction was quenched by the addition of a saturated aqueous solution of ammonium chloride (10 mL). The mixture was extracted with ethyl acetate (20 mL x 3). The extracted organic phases were combined and washed with saturated brine (20 mL x 3), dried over anhydrous sodium sulfate, and filtered. The filtrate was concentrated under reduced pressure, and separated by column chromatography (100% ethyl acetate) to afford compound **91-3.** MS m/z = 1014.8 [M+H]⁺.

### Step 4

C ompound **91-3** (100 mg, 98.6 µmol) was dissolved in dichloromethane (10 mL), and zinc bromide (66.6 mg, 295.8 µmol) was added. The mixture was stirred at 25°C for 4 hours. The mixture was filtered. The filter cake was washed with dichloromethane (20 mL), and the filtrate was concentrated under reduced pressure to obtain crude compound **91-4.** MS m/z = 914.7 [M+H]⁺.

### Step 5

Compound **91-4** (30.00 mg, crude) was dissolved in methanol (3 mL). Paraformaldehyde (3.00 mg, 98.46 µmol) and acetic acid (187.6 µL, 3.28 µmol) were added, and the mixture was stirred at 25°C for 2 hours. Sodium cyanoborohydride (4.13 mg, 65.64 µmol) was added, and the mixture was stirred at 25°C for additional hour. The reaction solution was concentrated under reduced pressure to obtain a crude product, which was then separated by preparative HPLC (column: Waters Xbridge, 250*19 mm, 5 µm; mobile phase: [water (0.1% ammonia)-acetonitrile]; gradient: (acetonitrile): 50%-85%) and dried to yield compound **91.** MS m/z = 702.5 [M+H]⁺. ¹H NMR (400 MHz, CD₃OD) δ ppm 6.74 (d, *J =* 12.0 Hz, 1H), 5.17 (dd, *J =* 12.0, 4.0 Hz, 1H), 4.78 (d, *J =* 12.0 Hz, 1H), 4.62 (d, *J =* 12.0 Hz, 1H), 4.18 (d, *J =* 8.0 Hz, 1H), 4.05 (d, *J =* 12.0 Hz, 1H), 3.69 (dd, *J* = 8.0, 4.0 Hz, 1H), 3.61 - 3.53 (m, 2H), 3.48 - 3.40 (m, 2H), 3.22 (dd, *J =* 16.0, 12.0 Hz, 1H), 2.95 - 2.84 (m, 5H), 2.78 - 2.71 (m, 1H), 2.64 - 2.58 (m, 2H), 2.54 - 2.49 (m, 2H), 2.34 (s, 3H), 2.14 - 2.06 (m, 2H), 2.04 (s, 3H), 1.94 - 1.83 (m, 5H), 1.74 - 1.69 (m, 1H), 1.35 (s, 9H).

### Example 92

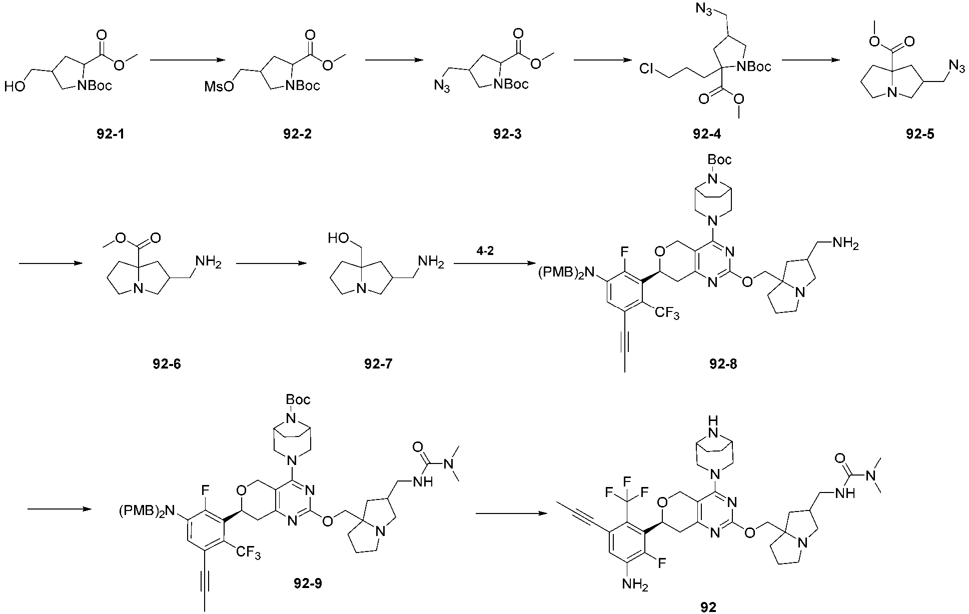

### Step 1

Compound **92-1** (600 mg, 2.31 mmol) was weighed and dichloromethane (6 mL) was added. Triethylamine (351.22 mg, 3.47 mmol) and methylsulfonyl chloride (530.13 mg, 4.63 mmol) were then added at room temperature. The mixture was allowed to react for 4 hours. Water (20 mL) was added, and the mixture was extracted with dichloromethane (20 mL × 3). The mixture was washed with saturated brine (10 mL × 3), and dried over anhydrous sodium sulfate. The organic phase was concentrated under reduced pressure to obtain compound **92-2.** MS m/z = 238.0 [M+H-Boc]⁺.

### Step 2

Compound **92-2** (500 mg, 1.48 mmol) was weighed and N,N-dimethylformamide (5 mL) was added. Sodium azide (289.03 mg, 4.45 mmol) was added at room temperature and allowed to react at 80°C for 4 hours. The reaction was quenched by adding water (15 mL) and extracted with ethyl acetate (20 mL x 3). The extracted organic phases were combined and washed with saturated brine (10 mL), dried over anhydrous sodium sulfate, filtered, and concentrated under reduced pressure. Column chromatography (dichloromethane:methanol = 1:1) afforded compound **92-3.** MS m/z = 185.1 [M+H-Boc]⁺.

### Step 3

Compound **92-3** (210 mg, 738.63 µmol) was weighed and tetrahydrofuran (3 mL) was added under nitrogen. The mixture was cooled to -78°C, and lithium bis(trimethylsilylamide) (247.18 mg, 1.48 mmol, 1 M solution in tetrahydrofuran) was added. The reaction was allowed to proceed at -78°C for 30 minutes. 1-Chloro-3-iodopropane (226.50 mg, 1.11 mmol) was added and the mixture was reacted at room temperature for 2 hours. Water (10 mL) was added, and the mixture was extracted with ethyl acetate (10 mL x 3). The organic phase was concentrated under reduced pressure and separated by column chromatography (petroleum ether:ethyl acetate = 10:1) to obtain compound **92-4.** MS m/z = 260.9 [M+H-Boc]+.

### Step 4

Compound **92-4** (800 mg, 1.83 mmol) was weighed and dichloromethane (10 mL) was added. Zinc bromide (1.24 g, 5.50 mmol) was added and the mixture was allowed to react at room temperature for 16 hours. Water (20 mL) was added and the mixture was extracted with dichloromethane (20 mL x 3). The organic phase was concentrated under reduced pressure and separated by column chromatography (petroleum ether:ethyl acetate = 2:1) to afford compound **92-5.** MS m/z = 225.2 [M+H]+.

### Step 5

To a solution of compound **92-5** (60 mg, 267.55 µmol) in tetrahydrofuran (2 mL) was added palladium / carbon (10 mg, 10% purity). The mixture was reacted at 20°C under a hydrogen atmosphere (15 psi) for 16 hours. The mixture was filtered, and the filtrate was concentrated to dryness by nitrogen blowing to afford compound **92-6.** MS m/z = 199.1 [M+H]⁺

### Step 6

To a solution of compound **92-6** (200 mg, 1.00 mmol) in tetrahydrofuran (5 mL) was added lithium aluminum tetrahydride (94.8 mg, 1.03 mmol) at 0°C and stirred at the same temperature for 0.3 h. The reaction was quenched with sodium sulfate decahydrate. The mixture was filtered, and concentrated under reduced pressure to afford compound **92-7.** MS m/z = 171.1 [M+H]⁺.

### Step 7

Compound **92-7** (8.71 mg, 51.14 µmol) was weighed and anhydrous tetrahydrofuran (1 mL) was added. Sodium hydride (1.23 mg, 51.14 µmol, 60% purity) was added under ice-cooling. The mixture was stirred at room temperature for 30 minutes, and compound **4-2** (30 mg, 34.09 µmol) was added. The reaction mixture was allowed to react at room temperature for 1 hour. The reaction mixture was quenched with water (10 mL) and extracted with ethyl acetate (10 mL x 3). The organic phase was dried over anhydrous sodium sulfate, concentrated under reduced pressure, and purified by preparative thin-layer chromatography (dichloromethane:methanol = 15:1) to obtain compound **92-8.** MS m/z = 970.7 [M+H]+.

### Step 8

Referring to steps 7 to 8 of Example **39, 92-8** was used as a starting material in place of **39-6B** to obtain crude compound **92.** The crude product was separated by preparative HPLC (column: Waters Xbridge, 250*19 mm, 5 µm; mobile phase: [water (0.1% ammonia)-acetonitrile]; (acetonitrile): 60%-90%) and dried to obtain compound **92.** MS m/z = 701.5 [M+H]⁺. ¹H NMR (400 MHz, CD₃OD) δ ppm 6.90 (d, *J =* 8.0 Hz, 1H), 5.16 - 5.12 (m, 1H), 4.81 - 4.78 (m, 1H), 4.65 (d, *J =* 16.0 Hz, 1H), 4.20 - 4.05 (m, 3H), 3.57 - 3.50 (m, 3H), 3.42 (d, *J =* 12.0 Hz, 1H), 3.28 - 3.16 (m, 4H), 3.08 - 2.99 (m, 2H), 2.88 (s, 6H), 2.85 - 2.78 (m, 2H), 2.59 - 2.51 (m, 1H), 2.40 (t, *J =* 8.0 Hz, 1H), 2.33 - 2.21 (m, 1H), 2.04 - 2.00 (m, 4H), 1.99 - 1.87 (m, 3H), 1.86 - 1.80 (m, 3H), 1.72 - 1.60 (m, 1H), 1.41 (t, *J =* 12.0 Hz, 1H).

### Example 93

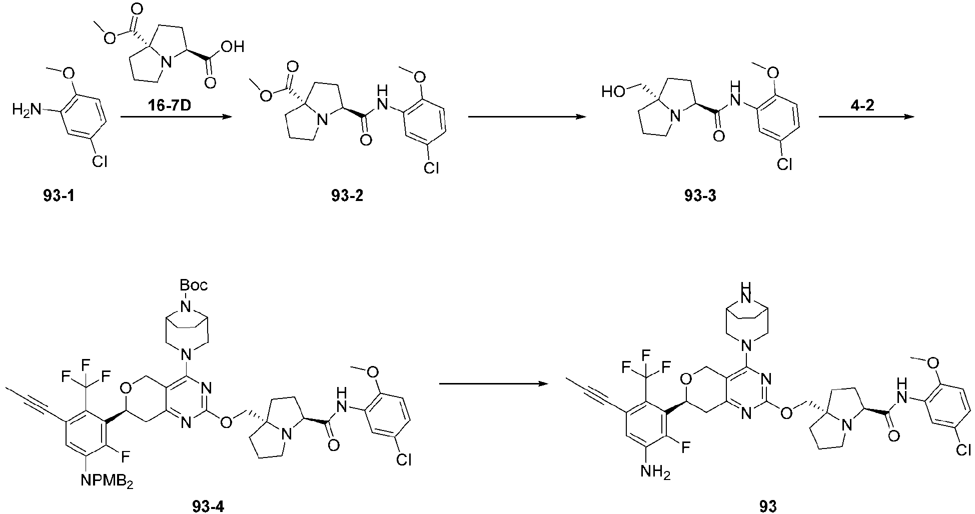

Referring to steps 7 to 10 of Example **16, 93-1** was used as a starting material in place of N-ethyl-4-methoxybenzylamine to obtain crude compound **93.** The crude product was separated by preparative HPLC (Chromatographic column: Waters Xbridge, 250 x 19 mm, 5 µm; mobile phase: [water (0.1% ammonia)-acetonitrile]; gradient: (acetonitrile): 10%-40%) and dried to yield compound **93.** MS m/z = 784.5 [M+H]⁺. ¹H NMR (400 MHz, CD₃OD) δ ppm δ 8.17 (d, *J* = 4.0 Hz, 1H), 7.08 (dd, *J =* 12.0, 4.0 Hz, 1H), 7.00 (d, *J =* 12.0 Hz, 1H), 6.90 (d, *J =* 8.0 Hz, 1H), 5.15 (dd, *J =* 12.0, 4.0 Hz, 1H), 4.78 (s, 1H), 4.66 (d, *J =* 16.0 Hz, 1H), 4.24 (d, *J* = 12.0 Hz, 1H), 4.17 - 4.09 (m, 2H), 4.02 (dd, *J =* 8.0, 4.0 Hz, 1H), 3.89 (s, 3H), 3.57 - 3.51 (m, 3H), 3.45 - 3.40 (m, 1H), 3.26 - 3.19 (m, 1H), 3.05 (d, *J =* 12.0 Hz, 1H), 3.01 - 2.96 (m, 1H), 2.86 (dd, *J =* 20.0, 4.0 Hz, 1H), 2.80 - 2.73 (m, 1H), 2.17 - 2.12 (m, 1H), 2.07 - 2.03 (m, 2H), 2.02 (s, 3H), 1.96 - 1.88 (m, 4H), 1.86 - 1.80 (m, 2H), 1.78 - 1.69 (m, 2H), 1.62 - 1.58 (m, 1H).

### Example 95

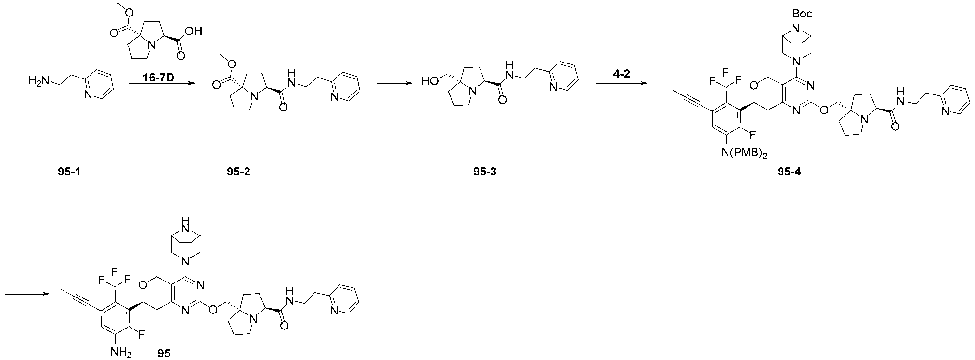

Referring to steps 7 to 10 of Example **16,** compound **95-1** was used as a starting material in place of N-ethyl-4-methoxybenzylamine to obtain crude compound **95.** The crude product was separated by preparative HPLC (Chromatographic column: Waters Xbridge, 250 x 19 mm, 5 µm; mobile phase: [water (0.1% ammonia)-acetonitrile]; gradient: (acetonitrile): 45%-70%) and dried to yield compound **95.** MS m/z = 749.5 [M+H]⁺. ¹H NMR (400 MHz, CD₃OD) δ ppm 8.51 - 8.43 (m, 1H), 7.81 - 7.71 (m, 1H), 7.38 - 7.32 (m, 1H), 7.30 - 7.23 (m, 1H), 6.90 (d, *J =* 8.0 Hz, 1H), 5.21 - 5.09 (m, 1H), 4.79 (d, *J =* 16.0 Hz, 1H), 4.64 (d, *J =* 16.0 Hz, 1H), 4.17 - 4.08 (m, 2H), 4.02 (d, *J* = 8.0Hz, 1H), 3.72 - 3.67 (m, 1H), 3.65 - 3.56 (m, 2H), 3.55 - 3.47 (m, 3H), 3.44 - 3.37 (m, 1H), 3.25 - 3.16 (m, 1H), 3.06 - 2.96 (m, 3H), 2.88 - 2.77 (m, 2H), 2.67 - 2.59 (m, 1H), 2.12 - 1.94 (m, 7H), 1.94 - 1.77 (m, 6H), 1.75 - 1.63 (m, 2H).

### Example 96

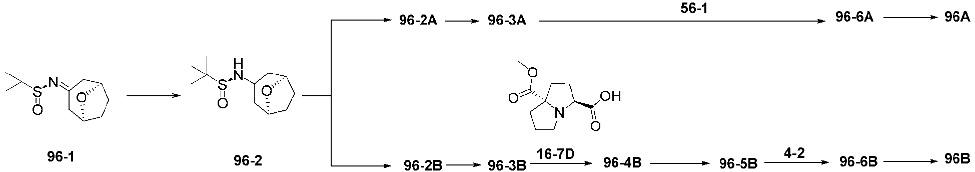

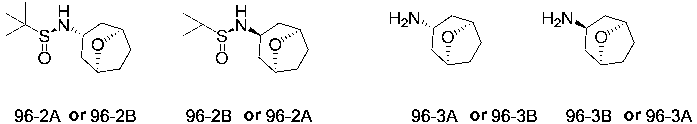

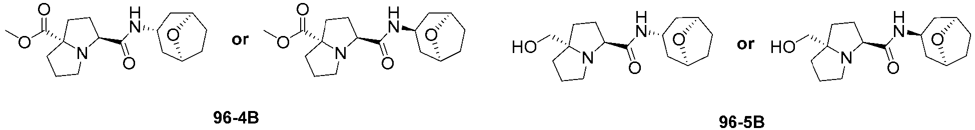

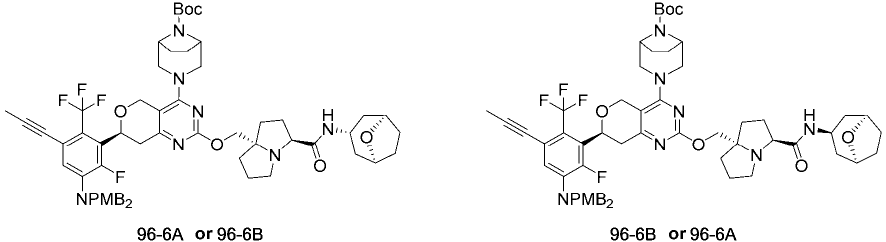

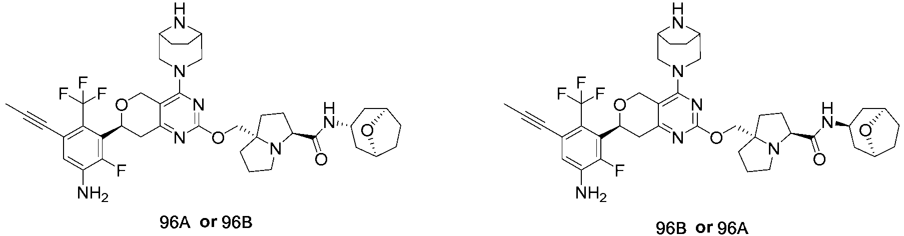

### Step 1

Compound **96-1** (3 g, 13.08 mmol) was weighed and methanol (30 mL) was added. Sodium borohydride (1.48 g, 39.24 mmol) was added and stirred at room temperature for 15 minutes. Column chromatography (dichloromethane:methanol = 10:1) afforded **96-2.** MS m/z = 232.2 [M+H]⁺. Preparative supercritical fluid chromatography (SFC) was then performed (column: DAICEL CHIRALPAK^{®} IC-10, 25*250 mm 10 µm; mobile phase: A: supercritical carbon dioxide, B: [0.05% ammonia methanol-methanol]; B%: 5%-40%) to afford compounds **96-2A** and **96-2B.** Analytical SFC method (chromatographic column: DAICEL CHIRALPAK^{®} IC-3 (100*3 mm 3 µm); mobile phase: [A: supercritical carbon dioxide B: methanol (0.05% diethylamine)]; gradient B%: 5%-40%, 4 min), compound **96-2A,** Rt = 2.817 minutes, ee value 100%, MS m/z = 232.2 [M+H]⁺; compound **96-2B,** Rt = 3.662 minutes, ee value 98.78%, MS m/z = 232.2 [M+H]⁺.

### Step 2

Compound **96-2A** (300 mg, 1.30 mmol) was weighed and a dioxane hydrochloride solution (10 mL, 4M) was added. The mixture was stirred at room temperature for 2 hours. The reaction mixture was concentrated under reduced pressure to afford the hydrochloride salt of **96-3A,** which was used directly in the next reaction. MS m/z = 128.1 [M+H]⁺.

Referring to step 2, compound **96-2B** was used as a starting material in place of compound **96-2A** to obtain the hydrochloride salt of compound **96-3B.** MS m/z = 128.1 [M+H]⁺.

### Step 3

Compound **56-1** (60 mg, 60.91 µmol) was weighed and dichloromethane (2 mL) was added. N,N,N,N-tetramethyl-O-(7-azabenzotriazol-1-yl)uronium hexafluorophosphate (39.37 mg, 103.55 µmol) and diisopropylethylamine (39.36 mg, 304.55 µmol) were also added. Compound **96-3A** (11.62 mg, 91.36 µmol) was then added and stirred at room temperature for 3 hours. Column chromatography (petroleum ether:ethyl acetate = 0:1) afforded compound **96-6A.** MS m/z = 1094.7 [M+H]⁺.

### Step 4

Compound **96-6A** (20 mg, 18.28 µmol) was weighed. Dichloromethane (0.5 mL) and trifluoroacetic acid (0.5 mL) were added, and the mixture was stirred at room temperature for 0.5 hours. The reaction solution was concentrated under reduced pressure to obtain a crude product, which was then separated by preparative HPLC (Chromatographic column: Waters Xbridge, 250*19 mm, 5 µm; mobile phase: [water (0.1% ammonia)-acetonitrile]; gradient: (acetonitrile): 30%-70%) to obtain compound **96A.** MS m/z = 754.5 [M+H]⁺. ¹H NMR (400 MHz, CD₃OD) δ 6.90 (d, *J =* 8.0 Hz, 1H), 5.14 (dd, *J =* 12.0, 4.0 Hz, 1H), 4.81 - 4.79 (m, 1H), 4.65 (d, *J =* 12.0 Hz, 1H), 4.40 (s, 2H), 4.24 - 4.12 (m, 3H), 4.04 (d, *J =* 12.0 Hz, 1H), 3.72 (dd, *J =* 8.0, 4.0 Hz, 1H), 3.57 - 3.50 (m, 3H), 3.42 (d, *J* = 12.0 Hz, 1H), 3.25 - 3.18 (m, 1H), 3.04 (d, *J* = 12.0 Hz, 1H), 2.92 - 2.80 (m, 2H), 2.77 - 2.70 (m, 1H), 2.11 - 2.06 (m, 2H), 2.03 - 1.99 (m, 4H), 1.98 - 1.67 (m, 15H), 1.62 - 1.56 (m, 2H).

### Step 5

Referring to steps 7 to 10 of Example **16, 96-3B** was used as a starting material in place of N-ethyl-4-methoxybenzylamine to obtain crude compound **96B.** The crude product was separated by preparative HPLC (Chromatographic column: Waters Xbridge, 250 x 19 mm, 5 µm; mobile phase: [water (0.1% ammonia)-acetonitrile]; gradient: (acetonitrile): 45%-70%) and dried to yield compound **96B.** MS m/z = 754.6 [M+H]⁺. ¹H NMR (400 MHz, CD₃OD) *δ* ppm 6.90 (d, *J =* 8.0 Hz, 1H), 5.15 (dd, *J =* 12.0, 4.0 Hz, 1H), 4.81 - 4.78 (m, 1H), 4.66 (d, *J* = 12.0 Hz, 1H), 4.34 (s, 2H), 4.20 - 4.13 (m, 2H), 4.09 (d, *J =* 12.0 Hz, 1H), 3.98 (t, *J =* 8.0 Hz, 1H), 3.80 - 3.76 (m, 1H), 3.61 - 3.51 (m, 3H), 3.44 (d, *J =* 12.0 Hz, 1H), 3.26 - 3.18 (m, 1H), 3.06 (d, *J =* 12.0 Hz, 1H), 2.96 - 2.82 (m, 2H), 2.75 - 2.66 (m, 1H), 2.23 - 2.08 (m, 7H), 2.04 - 1.99 (m, 5H), 1.98 - 1.86 (m, 8H), 1.74 - 1.69 (m, 3H).

### Example 97

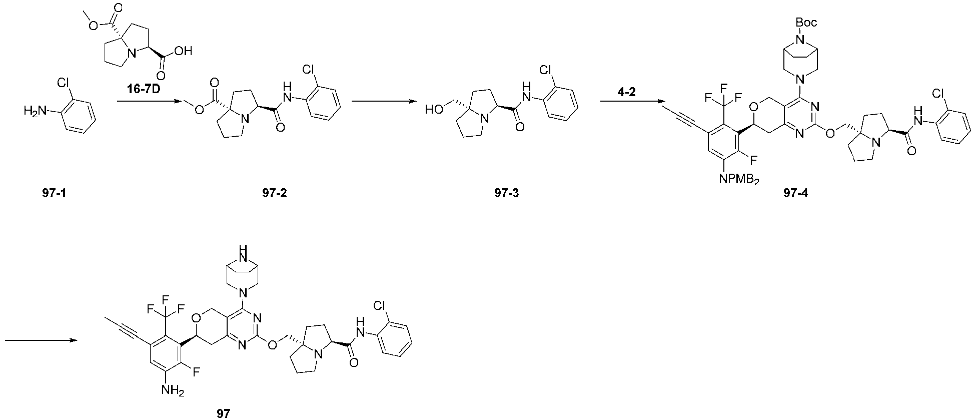

Referring to steps 7 to 10 of Example **16,** o-chloroaniline was used as a starting material in place of *N*-ethyl-4-methoxybenzylamine to obtain crude compound **97.** The crude product was separated by preparative HPLC (Chromatographic column: Waters Xbridge, 250 x 19 mm, 5 µm; mobile phase: water (0.1% ammonia)-acetonitrile; gradient: (acetonitrile): 45%-70%) and dried to yield compound **97.** MS m/z = 754.5 [M+H]⁺. ¹H NMR (400 MHz, CD₃OD) δ ppm 7.84 - 7.82 (m, 1H), 7.47 - 7.45 (m, 1H), 7.31 (td, *J =* 8.0, 4.0 Hz, 1H), 7.19 (td, *J =* 8.0, 4.0 Hz, 1H), 6.90 (d, *J =* 8.0 Hz, 1H), 5.15 (dd, *J =* 12.0, 4.0 Hz, 1H), 4.80 (d, *J =* 12.0 Hz, 1H), 4.66 (d, *J* = 16.0 Hz, 1H), 4.24 (d, *J =* 12.0 Hz, 1H), 4.18 - 4.09 (m, 2H), 4.06 - 4.02 (m, 1H), 3.60 - 3.51 (m, 3H), 3.47 - 3.40 (m, 1H), 3.22 (dd, *J =* 16.0, 12.0 Hz, 1H), 3.12 - 3.00 (m, 2H), 2.91 - 2.75 (m, 2H), 2.30 - 2.06 (m, 4H), 2.02 (s, 3H), 2.00 - 1.91 (m, 4H), 1.88 - 1.67 (m, 4H).

### Example 98

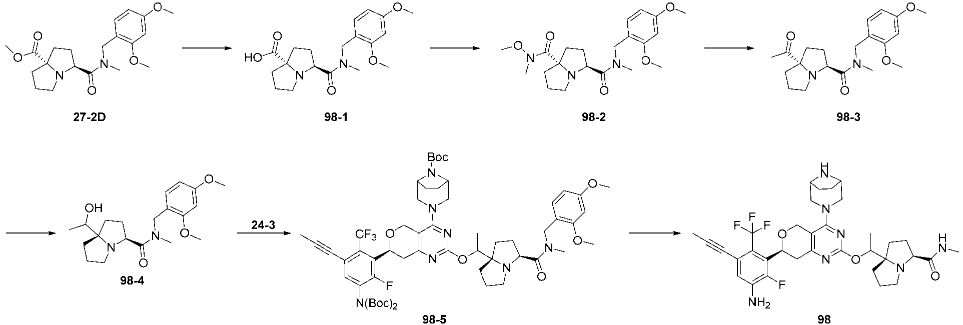

### Step 1

Compound **27-2D** (400 mg, 1.06 mmol) was dissolved in tetrahydrofuran (3 mL) and water (3 mL). Lithium hydroxide monohydrate (89.36 mg, 2.12 mmol) was added and the mixture was heated to 50°C with stirring for 4 hours. After returning to room temperature, the mixture was extracted with ethyl acetate (6 mL x 3). The combined organic phases were dried over anhydrous sodium sulfate, and filtered. The filtrate was concentrated under reduced pressure to obtain compound **98-1.** MS m/z = 361.2 [M-H]⁻.

### Step 2

Compound **98-1** (300 mg, 829 µmol) was dissolved in dichloromethane (10 mL). N,N-diisopropylethylamine (320.72 mg, 2.49 mmol) and N,N,N',N'-tetramethyl-O-(7-azabenzotriazol-1-yl)uronium hexafluorophosphate (377.9 mg, 994.8 µmol) were added sequentially. The mixture was stirred at 25°C for 0.5 hours. Dimethylhydroxylamine hydrochloride (96.96 mg, 994.8 mmol) was added, and the mixture was stirred at 25°C for additional 2 hours. The reaction was quenched by the addition of water (5 mL), and the mixture was extracted with ethyl acetate (10 mL x 3). The combined organic phases were dried over anhydrous sodium sulfate, filtered, and concentrated under reduced pressure. Column chromatography (petroleum ether:ethyl acetate = 1:1) gave compound **98-2,** MS m/z = 406.3 [M+H]⁺.

### Step 3

Compound **98-2** (150.00 mg, 370.16 µmol) was dissolved in anhydrous tetrahydrofuran (5 mL) and cooled to -78°C under nitrogen. A solution of methylmagnesium bromide in tetrahydrofuran (0.30 mL, 296.13 µmol, 1M) was slowly added dropwise. The reaction was stirred at -78°C for 2 hours. Water (4 mL) was added to quench the reaction, and the mixture was filtered. The filter cake was washed with acetonitrile (10 mL). The filtrate was concentrated under reduced pressure and separated by C18 reverse-phase column chromatography (mobile phase: [water (0.1% formic acid)-acetonitrile]; gradient: (acetonitrile): 0%-30%) to afford compound **98-3.** MS m/z = 361.3 [M+H]⁺.

### Step 4

Compound **98-3** (90 mg, 249.86 µmol) was dissolved in methanol (2 mL) and cooled to 0°C. Sodium borohydride (47.26 mg, 1.25 mmol) was added portionwise, and the mixture was heated to 25°C with stirring for 8 hours. The reaction was quenched by adding water (2 mL), and filtered. The filter cake was washed with acetonitrile (10 mL). The filtrate was concentrated under reduced pressure to obtain crude compound **98-4.** MS m/z = 363.3 [M+H]⁺.

### Step 5

Compound **98-4** (50 mg, crude product) was dissolved in anhydrous tetrahydrofuran (5 mL), and cooled to 0°C. Sodium hydride (11.05 mg, 0.28 mmol, 60% purity) was added. The mixture was heated to 25°C and the reaction was stirred for 0.5 hours. Compound **24-3** (94.92 mg, 110.48 µmol) was added, and the mixture was reacted at 25°C with stirring for additional 2 hours. The reaction was quenched by the addition of water (3 mL), and extracted with ethyl acetate (5 mL x 3). The organic phases were combined, dried over anhydrous sodium sulfate, filtered, and concentrated under reduced pressure. Column chromatography (petroleum ether:ethyl acetate = 1:1) afforded compound **98-5.** MS m/z = 1022.8 [M-Boc+H]⁺.

### Step 6

Compound **98-5** (60 mg, 53.50 µmol) was dissolved in dichloromethane (1.5 mL). Trifluoroacetic acid (0.5 mL) was added. The mixture was stirred at 25°C for 2 hours. The reaction solution was concentrated under reduced pressure to obtain a crude product. The crude product was separated by preparative high-performance liquid chromatography (Chromatographic column: Xbridge-C 18 250 x 19 mm, 5µm; mobile phase: [water (0.1% ammonia)-acetonitrile]; gradient: (acetonitrile): 30%-85%) and concentrated under reduced pressure to obtain compound **98.** MS m/z = 672.3 [M+H]⁺. ¹H NMR (400 MHz, CD₃OD) δ ppm 6.90 (d, *J =* 8.0 Hz, 1H), 5.17 - 5.12 (m, 1H), 4.78 (s, 1H), 4.66 (d, *J =* 16.0 Hz, 1H), 4.08 (dd, *J =* 20.0, 12.0 Hz, 1H), 3.58 - 3.49 (m, 3H), 3.45 - 3.36 (m, 1H), 3.29 - 3.14 (m, 3H), 3.08 - 3.02 (m, 1H), 3.01 - 2.89 (m, 1H), 2.88 - 2.81 (m, 1H), 2.69 (s, 1H), 2.68 - 2.62 (m, 1H), 2.60 (s, 2H), 2.32 - 2.20 (m, 1H), 2.19 - 2.13 (m, 1H), 2.01 (s, 3H), 2.00 - 1.88 (m, 2H), 1.86 - 1.74 (m, 5H), 1.72 - 1.66 (m, 2H), 1.64 - 1.55 (m, 1H), 1.33 (t, *J =* 8.0 Hz, 3H).

### Example 99

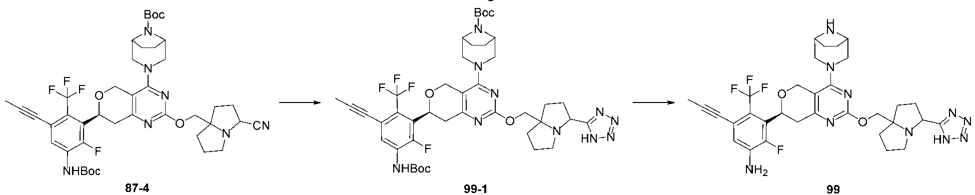

### Step 1

Compound **87-4** (100 mg, 121.08 µmol) was dissolved in N,N-dimethylformamide (2 mL). Sodium azide (78.72 mg, 1.21 mmol) and ammonium chloride (64.77 mg, 1.21 mmol) were added at 0°C. The mixture was heated to 70°C and stirred for 48 hours. The reaction was quenched by the addition of water (6 mL). The mixture was extracted with ethyl acetate (10 mL x 3). The extracted organic phases were combined and washed with saturated brine (10 mL), dried over anhydrous sodium sulfate, filtered, and concentrated under reduced pressure to obtain compound **99-1.** MS m/z = 869.7 [M+H]⁺.

### Step 2

Compound **99-1** (100 mg, 115.09 µmol) was dissolved in dichloromethane (3 mL), and trifluoroacetic acid (1 mL) was added. The mixture was stirred at 25°C for 2 hours. The reaction solution was concentrated under reduced pressure to obtain a crude product. The crude product was separated by preparative HPLC (Chromatographic column: Xbridge-C18 250 x 19 mm, 5 µm; mobile phase: [water (0.1% ammonia)-acetonitrile]; gradient: (acetonitrile): 10%-50%) and concentrated under reduced pressure to obtain compound **99.** MS m/z = 669.5 [M+H]⁺. ¹H NMR (400 MHz, CD₃OD) δ ppm δ 6.90 (d, *J* = 8.0 Hz, 1H), 5.15 (dd, *J* = 12.0, 4.0 Hz, 1H), 4.79 (s, 1H), 4.67 (dd, *J* = 12.0, 4.0 Hz, 1H), 4.44 - 4.36 (m, 2H), 4.23 - 4.17 (m, 2H), 3.89 (s, 2H), 3.66 (d, *J* = 16.0 Hz, 1H), 3.52 (dd, *J =* 16.0, 8.0 Hz, 1H), 3.24 - 3.14 (m, 4H), 2.86 - 2.77 (m, 1H), 2.54 - 2.46 (m, 1H), 2.42 - 2.34 (m, 2H), 2.19 - 1.83 (m, 12H).

### Example 100

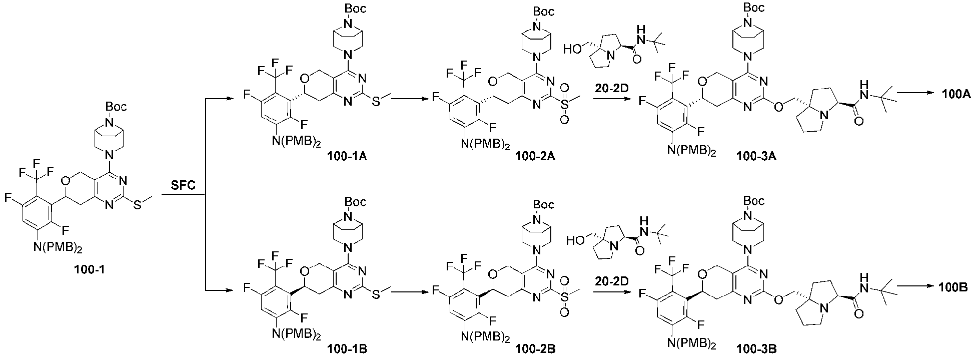

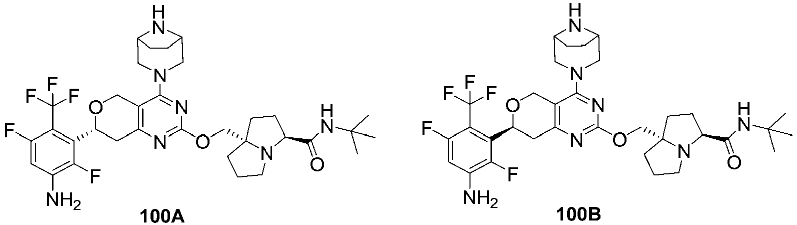

### Step 1

Compound **100-1** (390 mg, 471.41 µmol) was separated by preparative SFC (chromatographic column: Regis (S,S)Whelk-O 1, 25*250 mm 10 mm; mobile phase: A: supercritical carbon dioxide, B: 50% isopropanol + 50% acetonitrile + (0.05%) ammonia methanol, gradient: B%: 50%) to obtain compounds **100-1A** and **100-1B.** Analytical SFC method (column: DAICEL CHIRALPAK^{®} WHELK-O1 (4.6 mm*150 mm, 5 µm); mobile phase: [A: supercritical carbon dioxide; B: 50% isopropanol + 50% acetonitrile (0.05% diethylamine)]; gradient B%: 50%), compound **100-1A,** Rt = 7.180 minutes, MS m/z = 828.6 [M+H]⁺; compound **100-1B,** Rt = 9.250 minutes, MS m/z = 828.6 [M+H]⁺.

### Step 2

Compound **100-1A** (170 mg, 205.49 µmol) was dissolved in dichloromethane (2 mL), and m-chloroperbenzoic acid (91.71 mg, 451.75 µmol, 85%) was added. The mixture was stirred at 25°C for 1 hour. The reaction was quenched with saturated sodium sulfite (5 mL), and extracted with dichloromethane (5 mL x 3). The extracted organic phases were combined and washed with saturated brine (10 mL), dried over anhydrous sodium sulfate, filtered, and concentrated under reduced pressure. Column chromatography (petroleum ether:ethyl acetate = 1:1) afforded compound **100-2A.** MS m/z = 860.5 [M+H]⁺.

Referring to step 2, compound **100-1B** was used as a starting material in place of compound **100-1A** to obtain compound **100-2B.** MS m/z = 860.5 [M+H]⁺.

### Step 3

Compound **20-2D** (39.97 mg, 166.30 µmol) was dissolved in anhydrous tetrahydrofuran (2 mL). Sodium hydride (9.07 mg, 226.77 µmol, 60%) was added at 0°C and stirred for 0.5 hours. Compound **100-2A** (130 mg, 151.18 µmol) was added. The mixture was warmed to 25°C and stirred for 1.5 hours. The reaction was quenched by adding water (5 mL) and extracted with ethyl acetate (15 mL x 3). The extracted organic phases were combined and washed with saturated brine (15 mL), dried over anhydrous sodium sulfate, and filtered. The filtrate was concentrated under reduced pressure, and separated by column chromatography (petroleum ether:ethyl acetate = 1:1) to provide compound **100-3A.** MS m/z = 1020.7 [M+H]⁺.

Referring to step 3, compound **100-2B** was used as a starting material in place of compound **100-2A** to obtain compound **100-3B.** MS m/z = 1020.7 [M+H]⁺.

### Step 4

Compound **100-3A** (95 mg, 93.13 µmol) was dissolved in dichloromethane (2 mL), and trifluoroacetic acid (0.4 mL) was added. The mixture was stirred at 25°C for half an hour. The reaction solution was concentrated under reduced pressure to obtain a crude product. The crude product was separated by preparative HPLC (column: xbridge-C18, 250*19 mm, 5 µm; mobile phase: [water (0.1% ammonia)-acetonitrile]; gradient: (acetonitrile): 40%-80%) and dried to obtain compound **100A.** MS m/z = 680.5 [M+H]⁺. ¹H NMR (400 MHz, CD₃OD) δ ppm 6.60 - 6.55 (m, 1H), 5.13 (dd, *J* = 12.0, 4.0 Hz, 1H), 4.79 (s, 1H), 4.66 (d, *J* = 12.0 Hz, 1H), 4.18 - 4.13 (m, 2H), 4.04 (d, *J =* 12.0 Hz, 1H), 3.70 (dd, *J =* 8.0, 4.0 Hz, 1H), 3.54 - 3.51 (m, 3H), 3.42 (d, *J* = 12.0 Hz, 1H), 3.22 (dd, *J =* 16.0, 8.0 Hz, 1H), 3.04 (d, *J =* 12.0 Hz, 1H), 2.95 - 2.83 (m, 2H), 2.78 - 2.72 (m, 1H), 2.14 - 2.05 (m, 2H), 2.03 - 1.98 (m, 1H), 1.93 - 1.78 (m, 7H), 1.74 - 1.66 (m, 2H), 1.35 (s, 9H).

Compound **100-3B** (90 mg, 88.22 µmol) was dissolved in dichloromethane (2 mL), and trifluoroacetic acid (0.4 mL) was added. The mixture was stirred at 25°C for half an hour. The reaction solution was concentrated under reduced pressure to obtain a crude product. The crude product was separated by preparative HPLC (Chromatographic column: xbridge-C18, 250*19 mm, 5 µm; mobile phase: [water (0.1% ammonia)-acetonitrile]; gradient: (acetonitrile): 40%-80%) and dried to obtain compound **100B.** MS m/z = 680.5 [M+H]⁺. ¹H NMR (400 MHz, CD₃OD) δ ppm 6.61 - 6.56 (m, 1H), 5.14 (dd, *J =* 8.0, 4.0 Hz, 1H), 4.79 (s, 1H), 4.66 (d, *J* = 16.0 Hz, 1H), 4.19 - 4.13 (m, 2H), 4.05 (d, *J =* 8.0 Hz, 1H), 3.70 (dd, *J =* 12.0, 4.0 Hz, 1H), 3.54 - 3.51 (m, 3H), 3.42 (d, *J* = 12.0 Hz, 1H), 3.22 (dd, *J =* 20.0, 12.0 Hz, 1H), 3.05 (d, *J =* 12.0 Hz, 1H), 2.95 - 2.83 (m, 2H), 2.78 - 2.72 (m, 1H), 2.14 - 2.05 (m, 2H), 2.01 (t, *J* = 8.0 Hz, 1H), 1.93 - 1.78 (m, 7H), 1.75 - 1.67 (m, 2H), 1.36 (s, 9H).

### Example 101

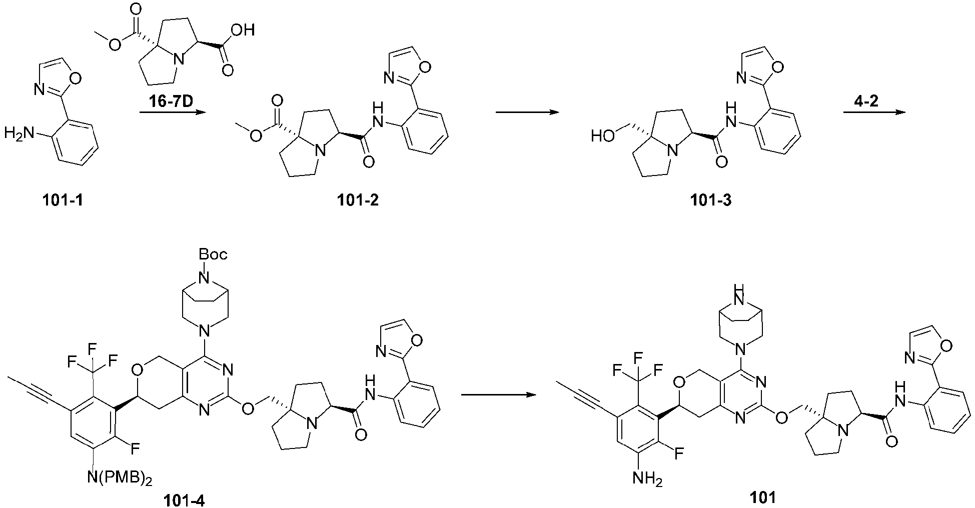

Referring to steps 7 to 10 of Example **16,** compound **101-1** was used as a starting material in place of N-ethyl-4-methoxybenzylamine to obtain crude compound 101. The crude product was separated by preparative HPLC (Chromatographic column: Waters Xbridge, 250 x 19 mm, 5 µm; mobile phase: [water (0.1% ammonia)-acetonitrile]; gradient: (acetonitrile): 65%-70%) and dried to afford compound **101.** MS m/z = 787.5 [M+H]⁺. ¹H NMR (400 MHz, CD₃OD) δ ppm 8.74 (d, *J =* 8.0 Hz, 1H), 8.04 (d, *J =* 8.0, 1H), 7.93 (s, 1H), 7.47 (t, *J =* 8.0 Hz, 1H), 7.37 (s, 1H), 7.22 (t, *J =* 8.0 Hz, 1H), 6.90 (d, *J =* 8.0 Hz, 1H), 5.05 (dd, *J =* 8.0, 4.0 Hz, 1H), 4.69 (d, *J* = 16.0 Hz, 1H), 4.56 (d, *J =* 16.0 Hz, 1H), 4.30 - 4.23 (m, 2H), 3.91 (d, *J =* 12.0 Hz, 1H), 3.53 (t, *J =* 8.0 Hz, 1H), 3.41 - 3.32 (m, 4H), 3.22 - 3.17 (m, 1H), 3.12 - 3.03 (m, 1H), 2.82 (d, *J =* 12.0 Hz, 1H), 2.79 - 2.68 (m, 2H), 2.49 - 2.40 (m, 1H), 2.23 - 2.15 (m, 2H), 2.13 - 2.02 (m, 3H), 2.01 (s, 3H), 1.85 - 1.75 (m, 3H), 1.72 - 1.63 (m, 2H), 1.57 - 1.50 (m, 1H).

### Example 102

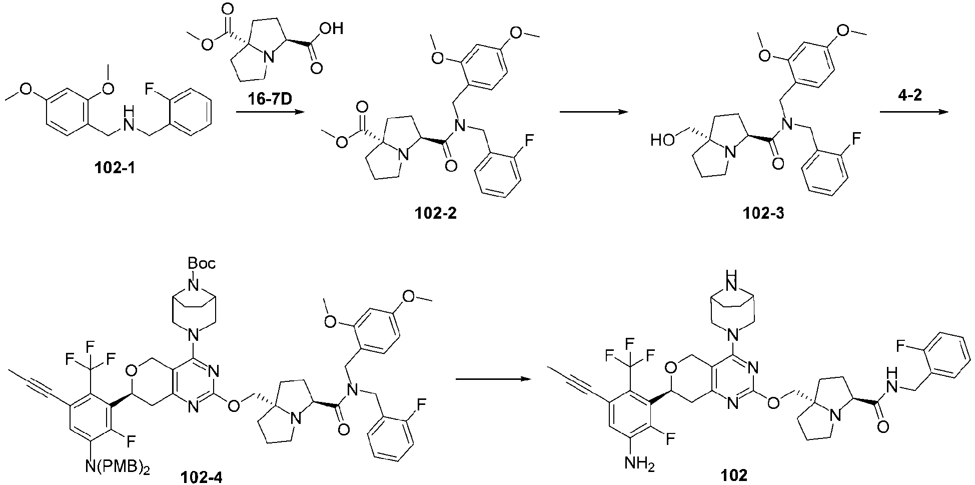

Referring to steps 7 to 10 of Example **16,** compound **102-1** was used as a starting material in place of N-ethyl-4-methoxybenzylamine to obtain crude compound **102.** The crude product was separated by preparative HPLC (Chromatographic column: Xtimate C18, 250*19 mm, 5 µm; mobile phase: [water (0.1% ammonia)-acetonitrile]; gradient: (acetonitrile): 20%-50%) and dried to yield compound **102.** MS m/z = 752.5 [M+H]⁺. ¹H NMR (400 MHz, CD₃OD) δ ppm 7.38 (t, *J =* 8.0 Hz, 1H), 7.33 - 7.27 (m, 1H), 7.14 (t, *J =* 8.0 Hz, 1H), 7.07 (t, *J =* 8.0 Hz, 1H), 6.90 (d, *J* = 8.0 Hz, 1H), 5.14 (dd, *J* = 12.0, 4.0 Hz, 1H), 4.79 (d, *J* = 12.0 Hz, 1H), 4.64 (d, *J =* 16.0 Hz, 1H), 4.50 (d, *J =* 16.0 Hz, 1H), 4.41 (d, *J* = 12.0 Hz, 1H), 4.17 - 4.11 (m, 2H), 4.04 (d, *J =* 8.0 Hz, 1H), 3.79 (dd, *J* = 8.0, 4.0 Hz, 1H), 3.53 - 3.50 (m, 3H), 3.41 (d, *J =* 12.0 Hz, 1H), 3.20 (dd, *J =* 16.0, 12.0 Hz, 1H), 3.03 (d, *J* = 16.0 Hz, 1H), 2.90 - 2.80 (m, 2H), 2.76 - 2.70 (m, 1H), 2.15 - 2.06 (m, 2H), 2.03 - 1.97 (m, 5H), 1.95 - 1.80 (m, 6H), 1.76 - 1.65 (m, 2H).

### Example 103

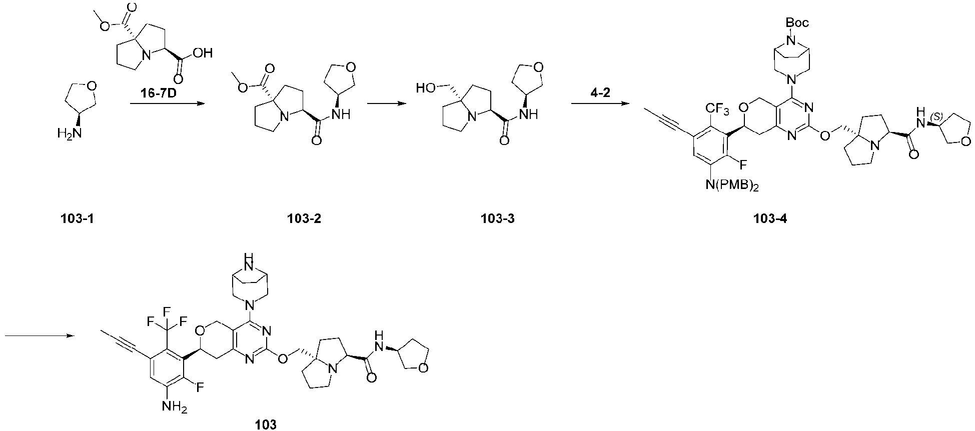

Referring to steps 7 to 10 of Example **16,** compound **103-1** was used as a starting material in place of *N*-ethyl-4-methoxybenzylamine to obtain crude compound **103.** The crude product was separated by preparative HPLC (Chromatographic column: Waters Xbridge, 250 x 19 mm, 5 µm; mobile phase: [water (0.1% ammonia)-acetonitrile]; gradient: (acetonitrile): 45%-70%) and dried to afford compound **103.** MS m/z = 714.5 [M+H]⁺. ¹H NMR (400 MHz, CD₃OD) δ ppm 6.90 (d, *J* = 8.0 Hz, 1H), 5.15 (d, *J* = 8.0 Hz, 1H), 4.81 - 4.78 (m, 1H), 4.65 (d, *J* = 12.0 Hz, 1H), 4.42 - 4.37 (m, 1H), 4.18 - 4.12 (m, 2H), 4.05 (d, *J* = 8.0 Hz, 1H), 3.97 - 3.72 (m, 4H), 3.66 - 3.59 (m, 1H), 3.55 - 3.40 (m, 3H), 3.26 - 3.17 (m, 2H), 3.05 (d, *J =* 16.0 Hz, 1H), 2.96 - 2.81 (m, 2H), 2.80 - 2.71 (m, 1H), 2.28 - 2.16 (m, 2H), 2.16 - 2.08 (m, 3H), 2.03 - 1.98 (m, 5H), 1.95 - 1.91 (m, 3H), 1.87 - 1.82 (m, 2H), 1.76 - 1.69 (m, 2H).

### Example 104

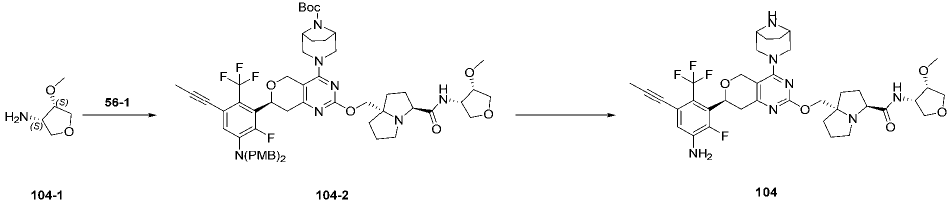

### Step 1

Compound **56-1** (60 mg, 60.91 µmol) was weighed and dichloromethane (2 mL) was added. N,N,N,N-tetramethyl-O-(7-azabenzotriazol-1-yl)uronium hexafluorophosphate (39.37 mg, 103.55 µmol), diisopropylethylamine (39.36 mg, 304.55 µmol), and compound **104-1** (14.27 mg, 121.82 µmol) were added and stirred at room temperature for 3 hours. Column chromatography (petroleum ether:ethyl acetate = 0:1) afforded compound **104-2.** MS m/z = 1084.7 [M+H]⁺.

### Step 2

Compound **104-2** (35 mg, 32.28 µmol) was weighed and dichloromethane (1 mL) was added. Trifluoroacetic acid (1 mL) was added and stirred at room temperature for 0.5 hours. The reaction solution was concentrated under reduced pressure to obtain a crude product, which was then separated by preparative HPLC (column: Waters Xbridge, 250*19 mm, 5 µm; mobile phase: [water (0.1% ammonia)-acetonitrile]; gradient: (acetonitrile): 30%-70%) to obtain compound 104. MS m/z = 744.5 [M+H]⁺. ¹H NMR (400 MHz, CD₃OD) δ ppm 6.90 (d, *J =* 8.0 Hz, 1H), 5.15 (dd, *J =* 12.0, 4.0 Hz, 1H), 4.80 - 4.77 (m, 1H), 4.65 (d, *J =* 12.0 Hz, 1H), 4.48 - 4.43 (m, 1H), 4.19 - 4.11 (m, 2H), 4.07 (d, *J =* 12.0 Hz, 1H), 4.00 - 3.96 (m, 2H), 3.93 - 3.90 (m, 1H), 3.89 - 3.82 (m, 2H), 3.60 - 3.51 (m, 4H), 3.46 - 3.40 (m, 1H), 3.37 (s, 3H), 3.25 - 3.18 (m, 1H), 3.05 (d, *J* = 12.0 Hz, 1H), 2.93 - 2.81 (m, 2H), 2.74 - 2.67 (m, 1H), 2.16 - 2.09 (m, 2H), 2.04 - 1.99 (m, 5H), 1.95 - 1.81 (m, 6H), 1.77 - 1.67 (m, 2H).

### Example 105

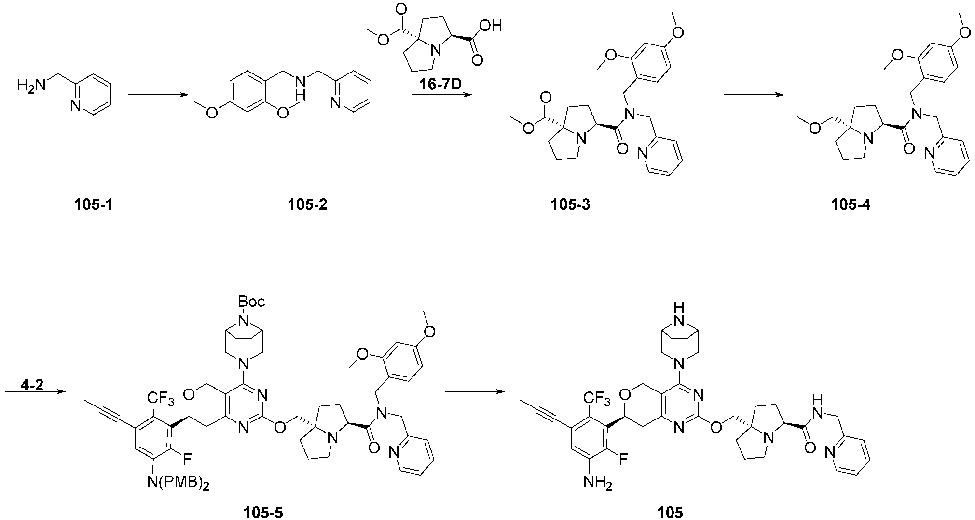

### Step 1

Compound **105-1** (500 mg, 4.62 mmol) was weighed and methanol (5 mL) was added. 2,4-dimethoxybenzaldehyde (768.32 mg, 4.62 mmol) was added under ice-cooling and stirred for 0.5 hours. Sodium cyanoborohydride (581.11 mg, 9.25 mmol) was then added and stirred at room temperature for 15 hours. The mixture was quenched with water (10 mL) and extracted with ethyl acetate (10 mL x 3). The organic phase was washed with saturated brine (10 mL x 3), dried over anhydrous sodium sulfate, and concentrated under reduced pressure. The crude product was purified by column chromatography (dichloromethane:methanol = 15:1) to obtain compound **105-2.** MS m/z = 259.17 [M+H]⁺.

### Step 2

Referring to steps 7 to 10 of Example **16,** compound **105-2** was used as a starting material in place of N-ethyl-4-methoxybenzylamine to obtain crude compound **105.** The crude product was separated by preparative HPLC (Chromatographic column: Waters Xbridge, 250 x 19 mm, 5 µm; mobile phase: water (0.1% ammonia)-acetonitrile; gradient: (acetonitrile): 45%-70%) and dried to afford compound **105.** MS m/z = 735.6 [M+H]⁺. ¹H NMR (400 MHz, CD₃OD) δ 8.49 - 8.47 (m, 1H), 7.83 - 7.78 (m, 1H), 7.43 (d, *J* = 8.0 Hz, 1H), 7.36 - 7.28 (m, 1H), 6.92 (d, *J* = 8.0 Hz, 1H), 5.16 (dd, *J=* 12.0, 4.0 Hz, 1H), 4.81 - 4.77 (m, 1H), 4.66 (d, *J* = 12.0 Hz, 1H), 4.55 (s, 2H), 4.22 - 4.06 (m, 3H), 3.89 - 3.85 (m, 1H), 3.58 - 3.42 (m, 4H), 3.28 - 3.14 (m, 1H), 3.06 (d, *J* = 12.0 Hz, 1H), 2.96 - 2.91 (m, 1H), 2.87 - 2.73 (m, 2H), 2.20 - 2.06 (m, 3H), 2.03 (s, 3H), 2.01 - 1.66 (m, 9H).

### Example 106

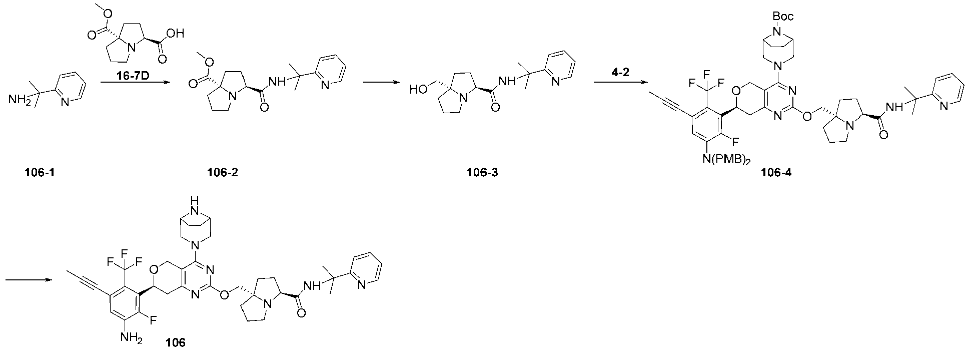

Referring to steps 7 to 10 of Example **16,** compound **106-1** was used as a starting material in place of N-ethyl-4-methoxybenzylamine to obtain crude compound **106.** The crude product was separated by preparative HPLC (Chromatographic column: Waters Xbridge, 250 x 19 mm, 5 µm; mobile phase: [water (0.1% ammonia)-acetonitrile]; gradient: (acetonitrile): 45%-70%) and dried to yield compound **106.** MS m/z = 763.5 [M+H]⁺. ¹H NMR (400 MHz, CD₃OD) δ ppm 8.52 - 8.42 (m, 1H), 7.81 - 7.71 (m, 1H), 7.51 (d, *J =* 8.0 Hz, 1H), 7.27 - 7.19 (m, 1H), 6.90 (d, *J =* 8.0 Hz, 1H), 5.20 - 5.10 (m, 1H), 4.78 (s, 1H), 4.65 (d, *J =* 12.0 Hz, 1H), 4.21 - 4.11 (m, 2H), 4.07 (d, *J* = 8.0 Hz, 1H), 3.87 - 3.81 (m, 1H), 3.58 - 3.49 (m, 3H), 3.45 - 3.38 (m, 1H), 3.27 - 3.18 (m, 1H), 3.07 - 3.01 (m, 1H), 3.01 - 2.95 (m, 1H), 2.89 - 2.81 (m, 1H), 2.77 - 2.69 (m, 1H), 2.14 - 1.98 (m, 6H), 1.97 - 1.80 (m, 7H), 1.76 - 1.63 (m, 8H).

### Example 107

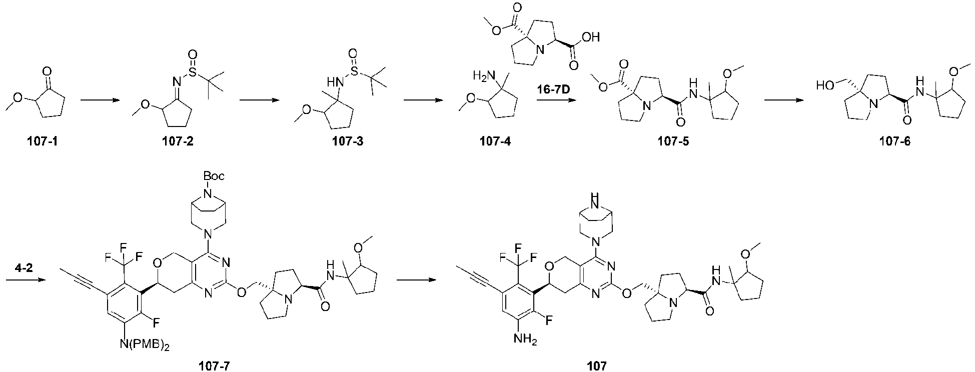

### Step 1

Compound **107-1** (2.2 g, 19.27 mmol) was dissolved in anhydrous tetrahydrofuran (25 mL), and tert-butylsulfenamide (2.34 g, 19.27 mmol) and tetraisopropyl orthotitanate (16.43 g, 57.82 mmol) were added. The reaction was stirred at 25°C for 3 hours. Water (10 mL) was added to quench the reaction, and the mixture was extracted with ethyl acetate (20 mL x 3). The organic phases were combined, dried over anhydrous sodium sulfate, and filtered. The filtrate was concentrated under reduced pressure, and separated by column chromatography (petroleum ether:ethyl acetate = 5:1) to obtain compound **107-2.** MS m/z = 218.1 [M+H]⁺.

### Step 2

Compound **107-2** (2.1 g, 9.66 mmol) was dissolved in a solution of methylmagnesium bromide in tetrahydrofuran (15 mL, 1M). The mixture was heated to 60°C and stirred for 16 hours. The reaction was quenched by the addition of a saturated aqueous ammonium chloride solution (10 mL). The mixture was extracted with ethyl acetate (20 mL x 3). The combined organic phases were dried over anhydrous sodium sulfate, and filtered. The filtrate was concentrated under reduced pressure, and separated by column chromatography (petroleum ether:ethyl acetate = 3:1) to afford compound **107-3.** MS m/z = 234.4 [M+H]⁺.

### Step 3

Compound **107-3** (340 mg, 1.46 mmol) was dissolved in a solution of hydrochloric acid in 1,4-dioxane (2 mL, 4 M) and stirred at 25°C for 3 hours. The mixture was concentrated under reduced pressure and separated by C18 reverse-phase column chromatography (mobile phase A: water (0.1% trifluoroacetic acid), mobile phase B: acetonitrile, gradient: 0-20% B) to obtain compound **107-4.** MS m/z = 130.2 [M+H]⁺.

### Step 4

Referring to steps 7 to 10 of Example **16, 107-4** was used as a starting material in place of N-ethyl-4-methoxybenzylamine to obtain crude compound **107.** The crude product was separated by preparative HPLC (Chromatographic column: sunfire-C18, 250 x 19 mm, 5 µm; mobile phase: [water (0.1% formic acid)-acetonitrile]; gradient: (acetonitrile): 25%-60%), and dried to give the formate salt of compound **107.** MS m/z = 756.6 [M+H]⁺. ¹H NMR (400 MHz, CD₃OD) δ ppm 8.47 (s, 1.82H), 6.88 (dd, *J* = 24.0, 8.0 Hz, 1H), 5.20 - 5.14 (m, 1H), 4.87 - 4.84 (m, 1H), 4.71 (d, *J* = 16.0 Hz,1H), 4.38 - 4.32 (m, 1H), 4.29 - 4.11 (m, 4H), 4.06 - 4.02 (m, 2H), 3.96 - 3.92 (m, 1H), 3.77 - 3.66 (m, 1H), 3.64 - 3.55 (m, 1H), 3.35 - 3.33 (m, 3H), 3.26 - 3.23 (m, 2H), 3.10 - 3.05 (m, 1H), 2.94 - 2.89 (m, 1H), 2.27 - 2.20 (m, 3H), 2.08 - 1.92 (m, 12H), 1.74 - 1.68 (m, 2H), 1.63 - 1.56 (m, 2H), 1.38 - 1.32 (m, 5H).

### Example 108

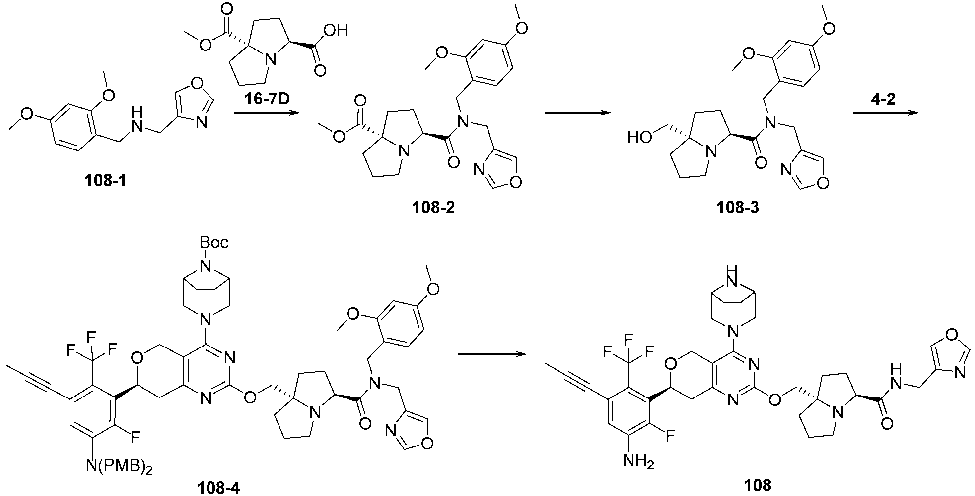

Referring to steps 7 to 10 of Example **16,** compound **108-1** was used as a starting material in place of *N*-ethyl-4-methoxybenzylamine to obtain crude compound **108.** The crude product was separated by preparative HPLC (Chromatographic column: Waters Xbridge, 250*30mm, 5 µm; mobile phase: [water (0.1% ammonia)-acetonitrile]; gradient: (acetonitrile): 50%-80%) and dried to yield compound **108.** MS m/z = 725.5 [M+H]⁺. ¹H NMR (400 MHz, CD₃OD) δ ppm 8.13 (s, 1H), 7.83 (s, 1H), 6.90 (d, *J =* 8.0 Hz, 1H), 5.14 (dd, *J* = 12.0, 4.0 Hz, 1H), 4.79 (d, *J =* 16.0 Hz, 1H), 4.65 (d, *J* = 16.0 Hz, 1H), 4.37 (d, *J =* 16.0 Hz, 1H), 4.31 (d, *J =* 16.0 Hz, 1H), 4.15 (t, *J* = 12.0 Hz, 2H), 4.05 (d, *J =* 8.0 Hz, 1H), 3.79 (dd, *J =* 8.0, 4.0 Hz, 1H), 3.56 - 3.51 (m, 3H), 3.43 (d, *J* = 12.0 Hz, 1H), 3.21 (dd, *J =* 20.0, 12.0 Hz, 1H), 3.05 (d, *J =* 16.0 Hz, 1H), 2.94 - 2.81 (m, 2H), 2.77 - 2.70 (m, 1H), 2.14 - 2.05 (m, 2H), 2.04 - 1.98 (m, 5H), 1.96 - 1.81 (m, 6H), 1.76 - 1.67 (m, 2H).

### Example 109

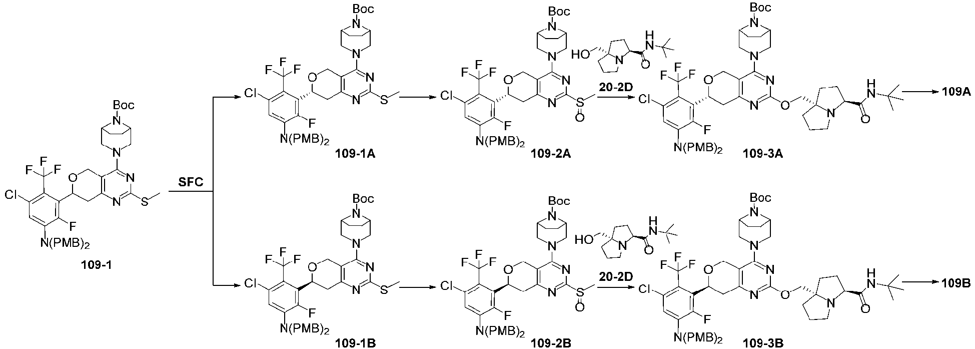

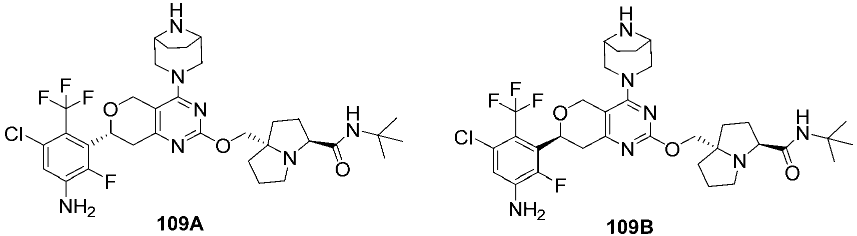

### Step 1

Compound **109-1** (500 mg, 592.90 µmol) was subjected to preparative SFC separation (Chromatographic column: Regis (S,S)Whelk-O 1, 25*250 mm 10 mm; mobile phase: A: supercritical carbon dioxide, B: 50% isopropanol + 50% acetonitrile + (0.05%) ammonia methanol; gradient: B %: 50%) to obtain compounds **109-1A** and **109-1B.** Analytical SFC method (Chromatographic column: DAICEL CHIRALPAK^{®} WHELK-O1 (4.6 mm*150 mm, 5 µm); mobile phase: [supercritical carbon dioxide - isopropanol 50% + acetonitrile 50% (0.05% diethylamine); gradient 50% (isopropanol 50% + acetonitrile 50% (0.05% diethylamine)), compound **109-1A,** Rt = 3.322 minutes, MS m/z = 844.5 [M+H]⁺; compound **109-1B,** Rt = 4.143 minutes, MS m/z = 844.5 [M+H]⁺.

### Step 2

Compound **109-1A** (210 mg, 249.02 µmol) was dissolved in dichloromethane (2 mL), and m-chloroperbenzoic acid (101.10 mg, 498.04 µmol, 85%) was added. The mixture was stirred at 25°C for 1 hour. The reaction was quenched with saturated sodium sulfite (5 mL), and extracted with dichloromethane (5 mL x 3). The extracted organic phases were combined and washed with saturated brine (10 mL), dried over anhydrous sodium sulfate, filtered, and concentrated under reduced pressure. Column chromatography (petroleum ether:ethyl acetate = 1:1) afforded compound **109-2A.** MS m/z = 860.5 [M+H]⁺.

Referring to step 2, compound **109-1B** was used as a starting material in place of compound **109-1A** to obtain compound **109-2B.** MS m/z = 860.5 [M+H]⁺.

### Step 3

Compound **20-2D** (35 mg, 145.63 µmol) was dissolved in anhydrous tetrahydrofuran (3 mL). Sodium hydride (5.82 mg, 145.63 µmol, 60%) was added at 0°C and stirred for 0.5 hours. Compound **109-2A** (125.29 mg, 145.63 µmol) was added and the mixture was heated to 25°C and stirred for 1.5 hours. Water (5 mL) was added to quench the reaction and the mixture was extracted with ethyl acetate (15 mL x 3). The organic phases were combined and washed with saturated brine (15 mL). The organic phase was dried over anhydrous sodium sulfate, and filtered. The filtrate was concentrated under reduced pressure, and separated by column chromatography (petroleum ether:ethyl acetate = 1:1) to obtain compound **109-3A.** MS m/z = 1036.8 [M+H]⁺.

Referring to step 3, compound **109-2B** was used as a starting material in place of compound **109-2A** to obtain compound **109-3B.** MS m/z = 1036.8 [M+H]⁺.

### Step 4

Compound **109-3A** (100 mg, 96.47 µmol) was dissolved in dichloromethane (3 mL), and trifluoroacetic acid (1 mL) was added. The reaction mixture was stirred at 25°C for 0.5 hours. The reaction solution was concentrated under reduced pressure to obtain a crude product. The crude product was separated by preparative HPLC (Chromatographic column: Xtimate C18 150*40mm, 5µm; mobile phase: [water (0.1% ammonia)-acetonitrile]; gradient: (acetonitrile): 50%-85%) and dried to obtain compound **109A.** MS m/z = 696.5 [M+H]⁺. ¹H NMR (400 MHz, CD₃OD) δ ppm 6.91 (d, *J* = 8.0 Hz, 1H), 5.21 (dd, *J =* 12.0, 4.0 Hz, 1H), 4.78 (s, 1H), 4.65 (d, *J =* 16.0 Hz, 1H), 4.18 - 4.12 (m, 2H), 4.05 (d, *J =* 12.0 Hz, 1H), 3.70 (dd, *J =* 8.0, 4.0 Hz, 1H), 3.53 - 3.50 (m, 3H), 3.42 (d, *J* = 12.0 Hz, 1H), 3.22 (dd, *J =* 16.0, 12.0 Hz, 1H), 3.04 (d, *J =* 16.0 Hz, 1H), 2.95 - 2.85 (m, 2H), 2.78 - 2.72 (m, 1H), 2.14 - 1.98 (m, 3H), 1.92 - 1.81 (m, 7H), 1.74 - 1.66 (m, 2H), 1.36 (s, 9H).

Compound **109-3B** (90 mg, 88.22 µmol) was dissolved in dichloromethane (3 mL), and trifluoroacetic acid (1 mL) was added. The reaction was stirred at 25°C for 0.5 hours. The reaction solution was concentrated under reduced pressure to obtain a crude product. The crude product was separated by preparative HPLC (Chromatographic column: Xbridge C18 150*40mm, 5µm; mobile phase: [water (0.1% ammonia)-acetonitrile]; gradient: (acetonitrile): 50%-90%) and dried to obtain compound **109B.** MS m/z = 696.5 [M+H]⁺. ¹H NMR (400 MHz, CD₃OD) δ ppm 6.91 (d, *J =* 8.0 Hz, 1H), 5.21 (dd, *J =* 12.0, 4.0 Hz, 1H), 4.78 (s, 1H), 4.65 (d, *J =* 16.0 Hz, 1H), 4.17 - 4.13 (m, 2H), 4.08 (d, *J =* 12.0 Hz, 1H), 3.70 (dd, *J =* 8.0, 4.0 Hz, 1H), 3.55 - 3.51 (m, 3H), 3.42 (d, *J* = 12.0 Hz, 1H), 3.21 (dd, *J* = 16.0, 12.0 Hz, 1H), 3.05 (d, *J =* 12.0 Hz, 1H), 2.95 - 2.86 (m, 2H), 2.78 - 2.72 (m, 1H), 2.15 - 1.99 (m, 3H), 1.94 - 1.80 (m, 7H), 1.76 - 1.65 (m, 2H), 1.36 (s, 9H).

### Example 110

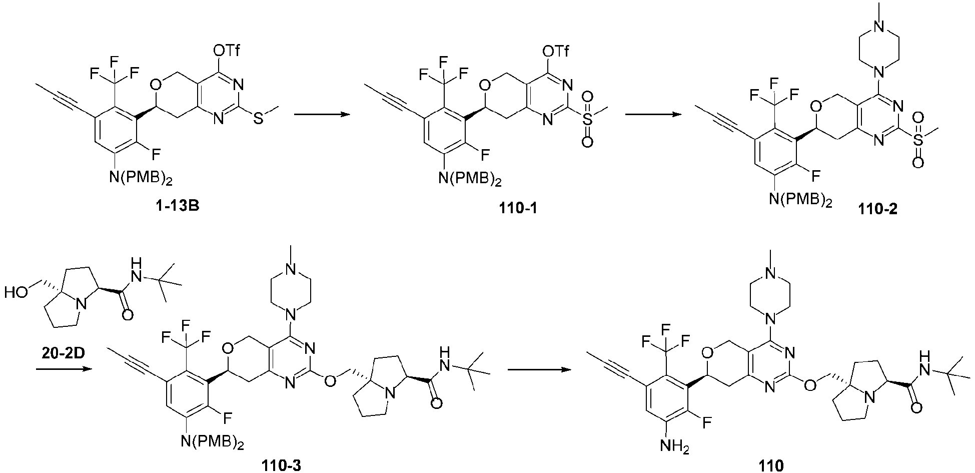

### Step 1

C ompound **1-13B** (200 mg, 254.53 µmol) was dissolved in dichloromethane (5 mL), and m-chloroperbenzoic acid (129.19 mg, 636.34 µmol) was added. The mixture was stirred at 25°C for 1 hour. The reaction was quenched by the addition of water (10 mL), and extracted with ethyl acetate (10 mL x 3). The extracted organic phases were combined and washed with saturated brine (10 mL), dried over anhydrous sodium sulfate, and filtered. The filtrate was concentrated under reduced pressure, and separated by column chromatography (petroleum ether:ethyl acetate = 5:1) to obtain compound **110-1.** MS m/z = 818.4 [M+H]⁺.

### Step 2

C ompound **110-1** (200 mg, 244.57 µmol) was dissolved in N,N-dimethylformamide (5 mL). N,N-diisopropylethylamine (94.83 mg, 733.72 µmol) and 1-methylpiperazine (48.99 mg, 489.15 µmol) were added sequentially at room temperature. The mixture was heated to 50°C and stirred for 1 hour. The reaction was quenched by the addition of water (10 mL). The mixture was extracted with ethyl acetate (20 mL x 3). The extracted organic phases were combined and washed with saturated brine (10 mL), dried over anhydrous sodium sulfate, and filtered. The filtrate was concentrated under reduced pressure, and separated by column chromatography (dichloromethane:methanol = 10:1) to obtain compound **110-2.** MS m/z = 768.3 [M+H]⁺.

### Step 3

Compound **20-2D** (46.95 mg, 195.36 µmol) was dissolved in anhydrous tetrahydrofuran (3 mL). Sodium hydride (15.63 mg, 390.71 µmol, 60%) was added at 0°C and the reaction was stirred at 0°C for half an hour. Compound **110-2** (100 mg, 130.24 µmol) was added, and the mixture was heated to 25°C with stirring for 1 hour. The reaction was quenched by the addition of a saturated aqueous solution of ammonium chloride (10 mL). The mixture was extracted with ethyl acetate (20 mL x 3). The extracted organic phases were combined and washed with saturated brine (20 mL), dried over anhydrous sodium sulfate, and filtered. The filtrate was concentrated under reduced pressure, and separated by column chromatography (dichloromethane:methanol = 20:1) to afford compound **110-3.** MS m/z = 928.7 [M+H]⁺.

### Step 4

Compound **110-3** (100 mg, 107.75 µmol) was dissolved in dichloromethane (5 mL), and trifluoroacetic acid (2 mL) was added. The reaction mixture was stirred at 25°C for 0.5 hours. The reaction solution was concentrated under reduced pressure to obtain a crude product. The crude product was separated by preparative HPLC (Chromatographic column: Waters Xbridge, 250*19 mm, 5 µm; mobile phase: [water (0.1% ammonia)-acetonitrile]; gradient: (acetonitrile): 40%-70%), and dried to obtain compound **110.** MS m/z = 688.5 [M+H]⁺. ¹H NMR (400 MHz, CD₃OD) δ ppm 6.91 (d, *J* = 8.0 Hz, 1H), 5.18 (dd, *J =* 12.0, 4.0 Hz, 1H), 4.79 (d, *J* = 12.0 Hz, 1H), 4.63 (d, *J* = 16.0 Hz, 1H), 4.24 (d, *J* = 12.0 Hz, 1H), 4.10 (d, *J* = 8.0 Hz, 1H), 3.85 - 3.74 (m, 1H), 3.63 - 3.56 (m, 2H), 3.48 - 3.43 (m, 2H), 3.24 (dd, *J* = 16.0, 12.0 Hz, 1H), 3.03 - 2.99 (m, 1H), 2.87 (dd, *J =* 20.0, 4.0 Hz, 2H), 2.67 - 2.58 (m, 2H), 2.57 - 2.49 (m, 2H), 2.35 (s, 3H), 2.21 - 2.13 (m, 2H), 2.02 (s, 3H), 1.97 - 1.88 (m, 5H), 1.80 - 1.75 (m, 1H), 1.36 (s, 9H).

### Example 111

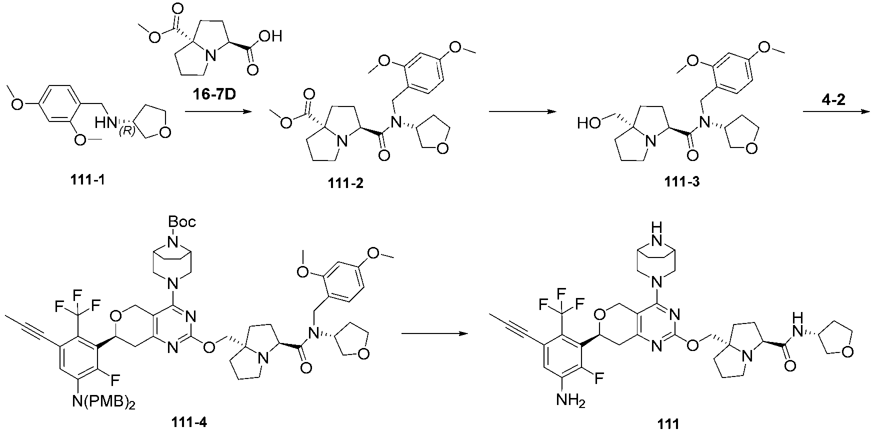

Referring to steps 7 to 10 of Example **16,** compound **111-1** was used as a starting material in place of N-ethyl-4-methoxybenzylamine to obtain crude compound **111.** The crude product was separated by preparative HPLC (column: Waters Xbridge, 250*19 mm, 5 µm; mobile phase: [water (0.1% ammonia)-acetonitrile]; gradient: (acetonitrile): 45%-60%) and dried to yield compound **111.** MS m/z = 714.5 [M+H]⁺. ¹H NMR (400 MHz, CD₃OD) δ ppm 6.90 (d, *J =* 8.0 Hz, 1H), 5.15 (dd, *J =* 12.0, 4.0 Hz, 1H), 4.80 (d, *J =* 12.0 Hz, 1H), 4.65 (d, *J* = 12.0 Hz, 1H), 4.41 - 4.36 (m, 1H), 4.18 - 4.13 (m, 2H), 4.05 (d, *J* = 8.0 Hz, 1H), 3.92 (q, *J* = 8.0 Hz, 1H), 3.88 - 3.75 (m, 3H), 3.64 (dd, *J =* 8.0, 4.0 Hz, 1H), 3.59 - 3.53 (m, 3H), 3.44 (d, *J =* 12.0 Hz, 1H), 3.22 (dd, *J =* 16.0, 12.0 Hz, 1H), 3.06 (d, *J =* 12.0 Hz, 1H), 2.93 - 2.82 (m, 2H), 2.78 - 2.71 (m, 1H), 2.27 - 2.17 (m, 1H), 2.15 - 2.07 (m, 2H), 2.05 - 1.97 (m, 5H), 1.95 - 1.82 (m, 7H), 1.77 - 1.68 (m, 2H).

### Example 112

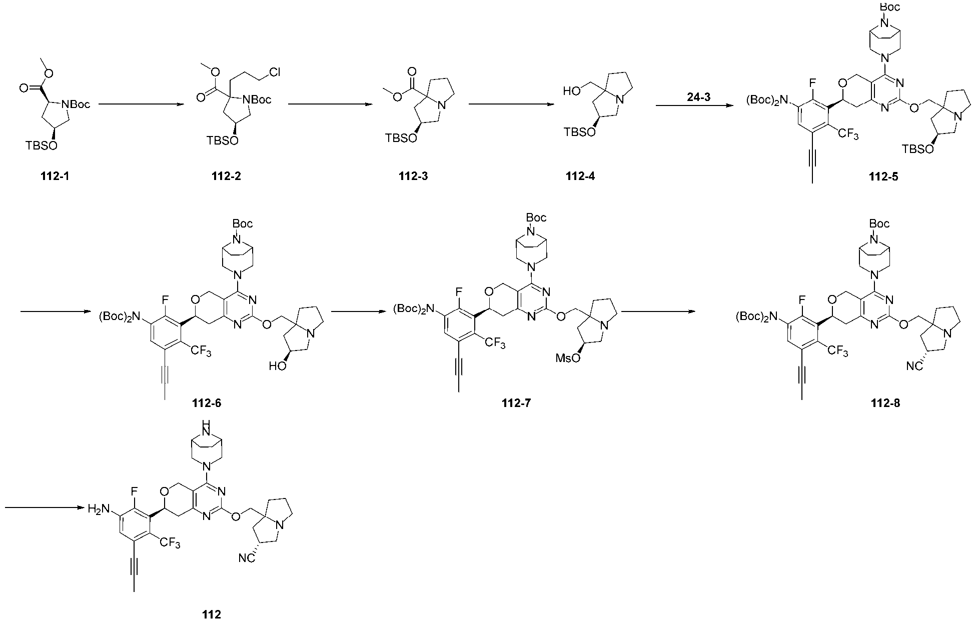

### Step 1

Compound **112-1** (1 g, 2.78 mmol) was weighed and anhydrous tetrahydrofuran (10 mL) was added. Lithium bis(trimethylsilyl)amide (930.79 mg, 5.56 mmol, 1 M tetrahydrofuran solution) was added at -78°C and stirred for 0.5 h. 1-Chloro-3-iodopropane (852.93 mg, 4.17 mmol) was added and allowed to react at room temperature for 3 h. 15 mL of water was added to quench the reaction and the mixture was extracted with ethyl acetate (20 mL*3). The organic phases were combined, washed with saturated brine (10 mL), dried over anhydrous sodium sulfate, filtered, and concentrated under reduced pressure. Column chromatography (petroleum ether:ethyl acetate = 1:1) afforded compound **112-2.** MS m/z = 436.2 [M+H]⁺.

### Step 2

Compound **112-2** (800 mg, 1.83 mmol) and zinc bromide (1.24 g, 5.50 mmol) were dissolved in dichloromethane (10 mL) and reacted at room temperature for 3 hours. The mixture was then concentrated under reduced pressure. The crude product was dissolved in acetonitrile (8 mL), and potassium carbonate (507.11 mg, 3.67 mmol) was added at room temperature. The mixture was reacted at room temperature for 3 hours. The mixture was concentrated under reduced pressure and separated by column chromatography (petroleum ether:ethyl acetate = 5:1) to obtain compound **112-3.** MS m/z = 300.2 [M+H]⁺.

### Step 3

Compound **112-3** (1.00 g, 3.34 mmol) was weighed and anhydrous tetrahydrofuran (10 mL) was added under nitrogen. The mixture was cooled to 0°C, and lithium aluminum tetrahydride (380.20 mg, 10.02 mmol, 2.5 M solution in tetrahydrofuran) was added. The mixture was reacted at 0°C for 1 hour. Dichloromethane (50 mL) was added to dilute the mixture. The reaction was quenched by adding water (0.4 mL) and 15% sodium hydroxide solution (0.4 mL) at 0°C. The mixture was stirred for 10 minutes, and filtered. The filter cake was washed with dichloromethane (10 mL), dried over anhydrous sodium sulfate, and filtered. The filtrate was concentrated to obtain compound **112-4.** MS m/z = 272.0 [M+H]⁺.

### Step 4

Compound **112-4** (145.45 mg, 535.78 µmol) was weighed and anhydrous tetrahydrofuran (2 mL) was added. Sodium hydride (12.86 mg, 535.78 µmol, 60% purity) was added under ice-cooling. The mixture was stirred at room temperature for 30 minutes. Compound **24-3** (300 mg, 357.19 µmol) was added and allowed to react at room temperature for 1 hour. The reaction mixture was quenched with water (10 mL) and extracted with ethyl acetate (20 mL × 3). The organic phase was dried over anhydrous sodium sulfate, concentrated under reduced pressure, and purified by preparative thin-layer chromatography (dichloromethane:methanol = 15:1) to obtain compound **112-5.** MS m/z = 1031.7 [M+H]⁺.

### Step 5

Compound **112-5** (130.00 mg, 126.06 µmol) was added to tetrabutylammonium fluoride (0.5 mL, 1M in THF) and stirred at room temperature for 2 hours. The reaction was quenched with water (5 mL) and extracted with ethyl acetate (3 mL x 3). The extracted organic phases were combined and washed with saturated brine (10 mL), dried over anhydrous sodium sulfate, filtered, and concentrated under reduced pressure. Purification by preparative thin-layer chromatography (dichloromethane:methanol = 15:1) afforded compound **112-6.** MS m/z = 817.6 [M+H-Boc]⁺.

### Step 7

Compound **112-6** (80.00 mg, 87.24 µmol) was weighed and dichloromethane (2 mL) was added. Triethylamine (26.48 mg, 261.72 µmol) and methylsulfonyl chloride (22.49 mg, 196.29 µmol) were then added at room temperature. The mixture was allowed to react at room temperature for 1 hour. Water (10 mL) was added, and the mixture was extracted with dichloromethane (10 mL x 3). The mixture was washed with saturated brine (10 mL x 3), and dried over anhydrous sodium sulfate. The organic phase was concentrated under reduced pressure to obtain compound **112-7.** The crude product was used directly in the next reaction. MS m/z = 895.6 [M+H-Boc]⁺.

### Step 8

Compound **112-7** (120.00 mg, crude product) was weighed and dichloromethane (2 mL) was added. Triethylamine (17.66 mg, 174.48 µmol) and sodium cyanide (14.99 mg, 130.86 µmol) were added at 0°C and reacted at 70°C for 4 hours. The reaction was quenched by adding saturated sodium carbonate (15 mL) and extracted with ethyl acetate (20 mL x 3). The extracted organic phases were combined and washed with saturated brine (10 mL), dried over anhydrous sodium sulfate, filtered, and concentrated under reduced pressure. Column chromatography (dichloromethane:methanol = 15:1) afforded compound **112-8.** MS m/z = 826.4 [M+H-Boc]⁺.

### Step 9

Compound **112-8** (20 mg, 21.60 µmol) was added to a solution of dichloromethane (1 mL) and trifluoroacetic acid (0.5 mL) and stirred at 20°C for 2 hours. The reaction solution was concentrated under reduced pressure to obtain a crude product, which was then separated by preparative HPLC (column: Waters Xbridge, 250*19 mm, 5 µm; mobile phase: [water (0.1% ammonia)-acetonitrile]; gradient: (acetonitrile): 20%-70%) to afford compound **112.** MS m/z = 626.4 [M+H]⁺. ¹H NMR (400 MHz, CD₃OD) δ 6.90 (d, *J* = 8.0 Hz, 1H), 5.21 - 5.09 (m, 1H), 4.81 - 4.78 (m, 1H), 4.65 (d, *J* = 12.0 Hz, 1H), 4.32 - 3.97 (m, 3H), 3.61 - 3.50 (m, 3H), 3.50 - 3.39 (m, 1H), 3.30 - 3.20 (m, 2H), 3.19 - 3.16 (m, 2H), 3.15 - 3.10 (m, 1H), 3.06 (d, *J* = 12.0 Hz, 1H), 2.88 - 2.83 (m, 1H), 2.70 - 2.54 (m, 1H), 2.23 (d, *J =* 8.0 Hz, 2H), 2.15 - 2.03 (m, 2H), 2.01 (s, 3H), 1.91 - 1.76 (m, 4H), 1.77 - 1.62 (m, 2H).

### Example 113

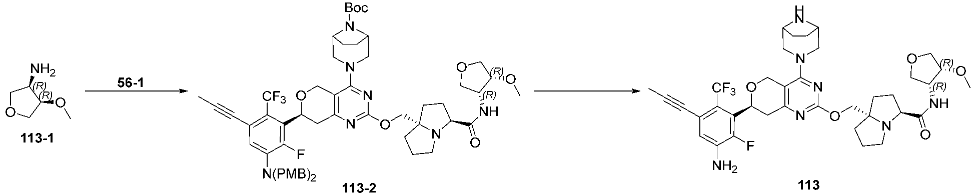

Referring to steps 2 to 3 of Example **56,** compound **113-1** was used as a starting material in place of (S)-3,3-difluorocyclopentylamine hydrochloride to obtain crude compound **113.** The crude product was separated by preparative HPLC (column: xbridge-C18, 30 x 250 mm, 5 µm; mobile phase: [water (0.1% ammonia)-acetonitrile]; gradient: (acetonitrile): 50%-85%) and dried to yield compound **113.** MS m/z = 744.6 [M+H]⁺. ¹H NMR (400 MHz, CD₃OD) δ ppm 6.90 (d, *J* = 8.0 Hz, 1H), 5.15 (dd, *J =* 8.0, 4.0 Hz, 1H), 4.80 (d, *J* = 12.0 Hz, 1H), 4.65 (d, *J =* 12.0 Hz, 1H), 4.45 (dd, *J =* 16.0, 8.0 Hz, 1H), 4.22 - 4.11 (m, 2H), 4.07 (d, *J =* 8.0 Hz, 1H), 4.03 - 3.80 (m, 5H), 3.61 - 3.47 (m, 4H), 3.43 (d, *J* = 12.0 Hz, 1H), 3.37 (s, 3H), 3.22 (dd, *J =* 16.0, 8.0 Hz, 1H), 3.09 - 2.96 (m, 2H), 2.90 - 2.69 (m, 2H), 2.17 - 2.07 (m, 2H), 2.06 - 1.77 (m, 11H), 1.77 - 1.64 (m, 2H).

### Example 114

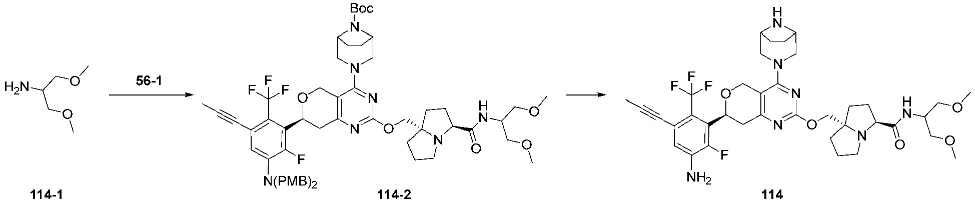

### Step 1

Compound **56-1** (35 mg, 35.53 µmol) was weighed and dichloromethane (2 mL) was added. N,N,N,N-tetramethyl-O-(7-azabenzotriazol-1-yl)uronium hexafluorophosphate (22.97 mg, 60.40 µmol) and diisopropylethylamine (22.96 mg, 177.65 µmol) were also added. Compound **114-1** (8.47 mg, 71.06 µmol) was then added and the mixture was stirred at room temperature for 2 hours. Column chromatography (petroleum ether:ethyl acetate = 0:1) afforded compound **114-2.** MS m/z = 1086.8 [M+H]⁺.

### Step 2

Compound **114-2** (17 mg, 15.65 µmol) was weighed and dichloromethane (0.5 mL) and trifluoroacetic acid (0.5 mL) were added. The mixture was stirred at room temperature for 0.5 hours. The reaction mixture was concentrated under reduced pressure to obtain a crude product, which was then separated by preparative HPLC (column: Waters Xbridge, 250 x 19 mm, 5 mm; mobile phase: [water (0.1% ammonia)-acetonitrile]; gradient: (acetonitrile): 30%-70%) to obtain compound **114.** MS m/z = 746.5 [M+H]⁺. ¹H NMR (400 MHz, CD₃OD) *δ* ppm 6.90 (d, *J =* 8.0 Hz, 1H), 5.15 (dd, *J =* 12.0, 4.0 Hz, 1H), 4.81 - 4.78 (m, 1H), 4.65 (d, *J* = 16.0 Hz, 1H), 4.21 - 4.11 (m, 3H), 4.06 (d, *J =* 12.0 Hz, 1H), 3.80 - 3.76 (m, 1H), 3.56 - 3.51 (m, 3H), 3.48 - 3.41 (m, 5H), 3.34 (d, *J =* 4.0 Hz, 6H), 3.25 - 3.18 (m, 1H), 3.05 (d, *J =* 12.0 Hz, 1H), 2.98 - 2.93 (m, 1H), 2.85 (dd, *J =* 12.0, 4.0 Hz, 1H), 2.77 - 2.70 (m, 1H), 2.14 - 2.07 (m, 2H), 2.04 - 1.98 (m, 5H), 1.95 - 1.89 (m, 4H), 1.86 - 1.81 (m, 2H), 1.77 - 1.68 (m, 2H).

### Example 115

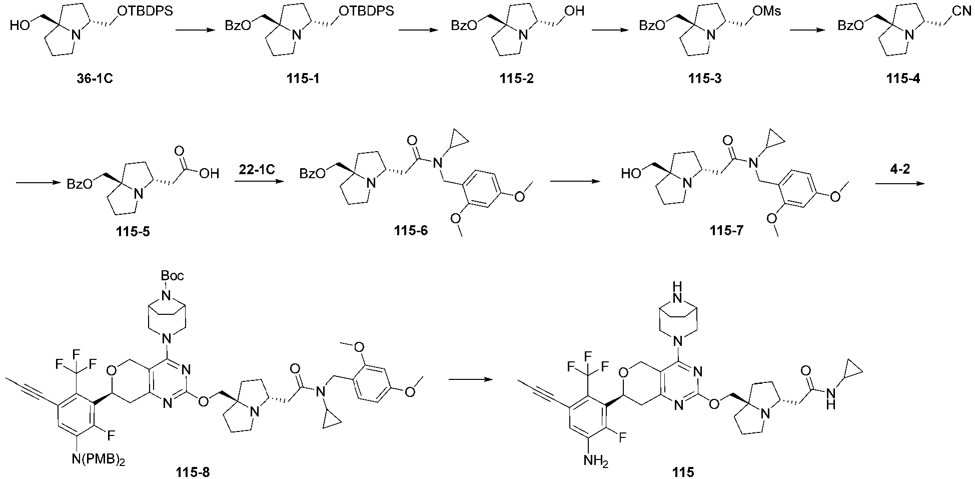

### Step 1

Compound **36-1C** (800 mg, 1.95 mmol) was dissolved in anhydrous dichloromethane (10 mL), and triethylamine (592.86 mg, 5.86 mmol) and benzoyl chloride (549.04 mg, 3.91 mmol) were added at 0°C. The mixture was heated to 25°C and stirred for 16 hours. Water (10 mL) was added to quench the reaction, and the mixture was extracted with dichloromethane (15 mL x 3). The mixture was washed with saturated brine (10 mL), dried over anhydrous sodium sulfate, and filtered. The filtrate was concentrated under reduced pressure, and separated by column chromatography (dichloromethane:methanol = 10:1) to afford compound **115-1.** MS m/z = 514.4 [M+H]⁺.

### Step 2

C ompound **115-1** (900 mg, 1.75 mmol) was dissolved in a solution of hydrochloric acid in dioxane (4 M, 15 mL). The mixture was heated to 50°C and stirred for 48 hours. The reaction solution was concentrated under reduced pressure, diluted with ethyl acetate (30 mL), and washed with saturated sodium bicarbonate solution (15 mL). The organic phase was dried over anhydrous sodium sulfate, and filtered. The filtrate was concentrated under reduced pressure, and separated by column chromatography (dichloromethane:methanol = 10:1) to provide compound **115-2.** MS m/z = 276.2 [M+H]⁺.

### Step 3

Compound **115-2** (420 mg, 1.53 mmol) was dissolved in anhydrous dichloromethane (10 mL). Triethylamine (617.41 mg, 6.10 mmol) and methanesulfonyl chloride (349.47 mg, 3.05 mmol) were added at 0°C. The mixture was warmed to 25°C and stirred for 16 hours. The reaction was quenched by the addition of water (10 mL). The product was extracted with dichloromethane (15 mL x 3), washed with saturated brine (10 mL), dried over anhydrous sodium sulfate, filtered, and concentrated under reduced pressure to obtain compound **115-3.** MS m/z = 354.2 [M+H]⁺.

### Step 4

Compound **115-3** (460 mg, 1.30 mmol) was dissolved in N,N-dimethylformamide (10 mL), and sodium cyanide (138.06 mg, 2.60 mmol) was added. The mixture was heated to 80°C and the reaction was stirred for 16 hours. After returning to room temperature, saturated sodium bicarbonate was added to adjust the *pH* to 9. The mixture was extracted with ethyl acetate (15 mL x 3), washed with saturated brine (10 mL), dried over anhydrous sodium sulfate, and filtered. The filtrate was concentrated under reduced pressure, and separated by column chromatography (dichloromethane:methanol = 10:1) to obtain compound **115-4.** MS m/z = 285.2 [M+H]⁺.

### Step 5

C ompound **115-4** (300 mg, 1.06 mmol) was dissolved in anhydrous dioxane (10 mL). Concentrated hydrochloric acid (3 mL, 1.06 mmol, 12 M) was added, and the mixture was heated to 50°C with stirring for 20 hours. The mixture was returned to room temperature, and the *pH* was adjusted to 6 by adding saturated sodium bicarbonate solution. The mixture was concentrated under reduced pressure and separated by C18 reverse -phase column chromatography (mobile phase: [water (0.1% ammonium bicarbonate)-acetonitrile]; gradient: (acetonitrile): 0%-5%) to obtain compound **115-5.** MS m/z = 304.2 [M+H]⁺.

### Step 6

C ompound **115-5** (280 mg, 923.02 µmol) was dissolved in N,N-dimethylformamide (5 mL). Compound **22-1C** (382.63 mg, 1.85 mmol), N,N-diisopropylethylamine (357.88 mg, 2.77 mmol), and N,N,N',N'-tetramethyl-O-(7-azabenzotriazol-1-yl)uronium hexafluorophosphate (526.44 mg, 1.38 mmol) were added sequentially. The mixture was stirred at 25°C for 3 hours. Water (10 mL) was added to quench the reaction, and the mixture was extracted with ethyl acetate (15 mL x 3). The extracted organic phases were combined and washed with saturated brine (15 mL), dried over anhydrous sodium sulfate, and filtered. The filtrate was concentrated under reduced pressure, and separated by column chromatography (100% ethyl acetate) to provide compound **115-6.** MS m/z = 493.4 [M+H]⁺.

### Step 7

C ompound **115-6** (70 mg, 142.10 µmol) was dissolved in methanol (2 mL) and water (2 mL). Lithium hydroxide monohydrate (17.89 mg, 426.30 µmol) was added and stirred at 25°C for 1 hour. The mixture was filtered. The filter cake was washed with methanol (5 mL), and the filtrate was concentrated under reduced pressure. The product was then separated by C18 reverse-phase column chromatography (mobile phase: [water (0.1% ammonium bicarbonate)-acetonitrile]; gradient: (acetonitrile): 0%-35%) to afford compound **115-7.** MS m/z = 389.4 [M+H]⁺.

### Step 8

Compound **115-7** (45 mg, 115.83 µmol) was dissolved in anhydrous tetrahydrofuran (2 mL). Sodium hydride (6.95 mg, 173.75 µmol, 60%) was added at 0°C and the reaction was stirred for 0.5 hours. Compound **4-2** (225 mg, 255.69 µmol) was added, and the mixture was heated to 25°C. The reaction was stirred for additional 1 hour. The reaction was quenched by the addition of water (5 mL) and extracted with ethyl acetate (10 mL x 3). The organic phases were combined, washed with saturated brine (10 mL), dried over anhydrous sodium sulfate, and filtered. The filtrate was concentrated under reduced pressure, and separated by column chromatography (dichloromethane:methanol = 10:1) to obtain compound **115-8.** MS m/z = 1188.9 [M+H]⁺.

### Step 9

Compound **115-8** (70 mg, 58.91 µmol) was dissolved in dichloromethane (2 mL), and trifluoroacetic acid (0.6 mL) was added. The mixture was heated to 35°C and the mixture was stirred for 1 hour. The reaction solution was concentrated under reduced pressure to obtain a crude product. The crude product was separated by preparative HPLC (Chromatographic column: sunfire-C18, 250 x 19 mm, 5 µm; mobile phase: [water (0.1% ammonia)-acetonitrile]; gradient: (acetonitrile): 32% - 52%) and dried to obtain compound **115.** MS m/z = 698.5 [M+H]⁺. ¹H NMR (400 MHz, CD₃OD) δ ppm 6.91 (d, *J =* 8.0 Hz, 1H), 5.16 (dd, *J =* 12.0, 4.0 Hz, 1H), 4.81 (s, 1H), 4.68 (d, *J =* 12.0 Hz, 1H), 4.40 (q, *J =* 12.0 Hz, 2H), 4.15 (d, *J =* 12.0 Hz, 1H), 3.91 (s, 1H), 3.71 (s, 2H), 3.62 (d, *J* = 12.0 Hz, 1H), 3.47 (d, *J =* 12.0 Hz, 1H), 3.28 - 3.23 (m, 1H), 3.14 (d, *J* = 12.0 Hz, 1H), 3.10 - 3.01 (m, 1H), 2.95 (dd, *J =* 20.0, 4.0 Hz, 1H), 2.69 - 2.55 (m, 3H), 2.36 - 2.31 (m, 1H), 2.15 - 2.02 (m, 8H), 1.98 - 1.86 (m, 6H), 1.82 - 1.75 (m, 1H), 0.73 - 0.64 (m, 2H), 0.50 - 0.42 (m, 2H).

### Example 116

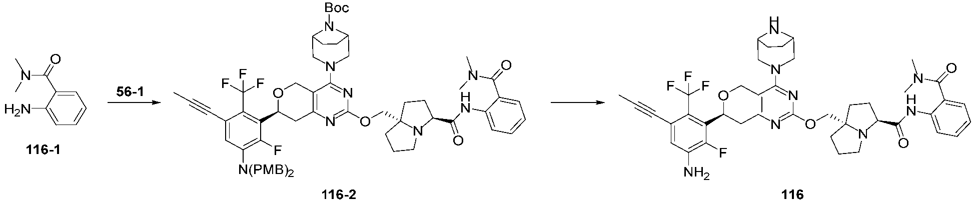

Referring to steps 2 to 3 of Example **56,** compound **116-1** was used as a starting material in place of (S)-3,3-difluorocyclopentylamine hydrochloride to obtain crude compound **116.** The crude product was separated by preparative HPLC (column: xbridge-C18, 250*19 mm, 5 µm; mobile phase: [water (0.1% ammonia)-acetonitrile]; gradient: (acetonitrile): 60%-90%) and dried to yield compound **116.** MS m/z = 791.6 [M+H]⁺. ¹H NMR (400 MHz, CD₃OD) δ ppm 7.58 (d, *J* = 8.0 Hz, 1H), 7.45 (td, *J* = 8.0, 4.0 Hz, 1H), 7.34 - 7.26 (m, 2H), 6.90 (d, *J =* 8.0 Hz, 1H), 5.16 (dd, *J =* 12.0, 4.0 Hz, 1H), 4.78 (s, 1H), 4.66 (d, *J =* 12.0 Hz, 1H), 4.21 (d, *J= 12.0* Hz, 1H), 4.15 (d, *J =* 12.0 Hz, 1H), 4.10 (d, *J =* 8.0 Hz, 1H), 3.97 - 3.94 (m, 1H), 3.62 - 3.55 (m, 3H), 3.45 (d, *J* = 12.0 Hz, 1H), 3.23 (dd, *J =* 16.0, 12.0 Hz, 1H), 3.09 - 3.03 (m, 5H), 2.98 (s, 3H), 2.89 - 2.77 (m, 2H), 2.19 - 2.10 (m, 3H), 2.05 - 2.02 (m, 4H), 1.99 - 194 (m, 3H), 1.90 - 1.84 (m, 2H), 1.81 - 1.71 (m, 3H).

### Example 117

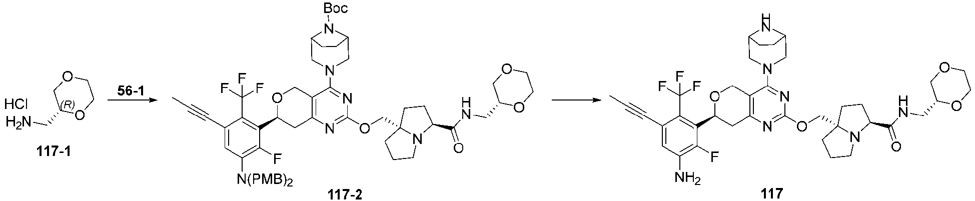

Referring to steps 2 to 3 of Example **56, 117-1** was used as a starting material in place of (S)-3,3-difluorocyclopentylamine hydrochloride to obtain crude compound **117D.** The crude product was separated by preparative HPLC (column: Xtimate C18, 250*19mm, 5µm; mobile phase: [water (0.1% ammonia)-acetonitrile]; gradient: (acetonitrile): 40%-80%), and dried to yield compound **117.** MS m/z = 744.5 [M+H]⁺. ¹H NMR (400 MHz, CD₃OD) δ ppm 6.90 (d, *J =* 8.0 Hz, 1H), 5.15 (dd, *J =* 12.0, 4.0 Hz, 1H), 4.78 (s, 1H), 4.65 (d, *J* = 12.0 Hz, 1H), 4.19 - 4.12 (m, 2H), 4.06 (d, *J =* 12.0 Hz, 1H), 3.79 - 3.73 (m, 3H), 3.70 - 3.61 (m, 3H), 3.59 - 3.52 (m, 4H), 3.42 (d, *J* = 12.0 Hz, 1H), 3.36 - 3.33 (m, 1H), 3.27 (d, *J =* 12.0 Hz, 1H), 3.23 - 3.14 (m, 2H), 3.05 (d, *J =* 16.0 Hz, 1H), 2.96 - 2.90 (m, 1H), 2.85 (dd, *J* = 20.0, 8.0 Hz, 1H), 2.78 - 2.69 (m, 1H), 2.14 - 2.06 (m, 2H), 2.05 - 1.97 (m, 5H), 1.95 - 1.81 (m, 6H), 1.76 - 1.67 (m, 2H).

### Example 118

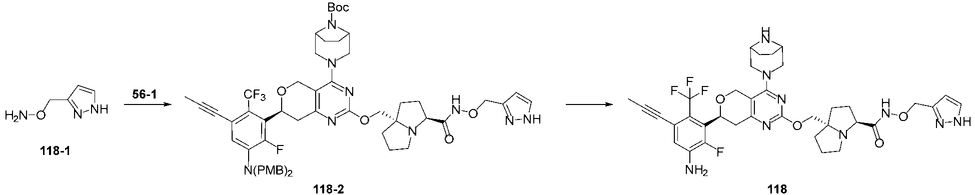

Referring to steps 2 to 3 of Example **56, 118-1** was used as a starting material in place of (S)-3,3-difluorocyclopentylamine hydrochloride to obtain crude compound **118.** The crude product was separated by preparative HPLC (column: xbridge-C18, 250*19 mm, 5 µm; mobile phase: [water (0.1% ammonia)-acetonitrile]; gradient: (acetonitrile): 40%-68%) and dried to yield compound **118.** MS m/z = 740.5 [M+H]⁺. ¹H NMR (400 MHz, CD₃OD) δ ppm 7.60 (s, 1H), 6.90 (d, *J* = 8.0 Hz, 1H), 6.44 (s, 1H), 5.14 (dd, *J =* 12.0, 4.0 Hz, 1H), 4.91 (s, 2H), 4.79 (d, *J =* 16.0 Hz, 1H), 4.65 (d, *J* = 12.0 Hz, 1H), 4.16 - 4.12 (m, 2H), 4.03 (d, *J =* 12.0 Hz, 1H), 3.61 (t, *J* = 4.0 Hz, 1H), 3.56 - 3.51 (m, 3H), 3.42 (d, *J* = 12.0 Hz, 1H), 3.21 (dd, *J =* 16.0, 12.0 Hz, 1H), 3.04 (d, *J* = 12.0 Hz, 1H), 2.92 - 2.76 (m, 3H), 2.12 - 2.05 (m, 3H), 2.04 - 1.96 (m, 7H), 1.89 - 1.79 (m, 3H), 1.77 - 1.67 (m, 2H).

### Example 120

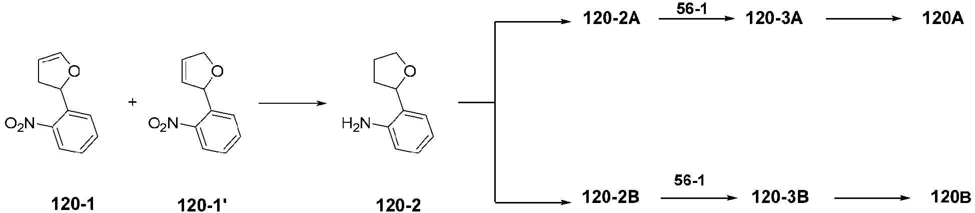

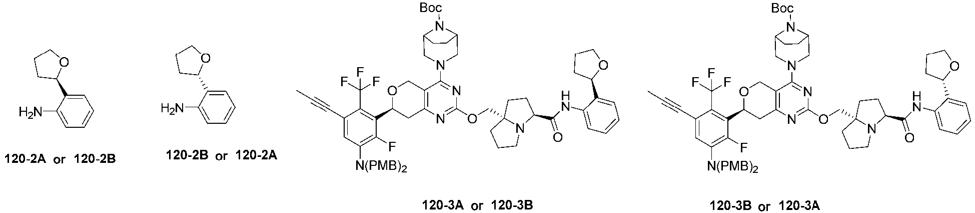

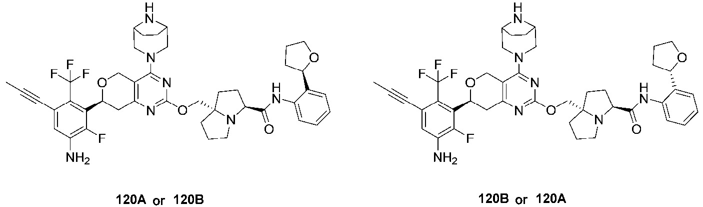

### Step 1

A mixture of compounds **120-1** and **120-1'** (4.4 g, 23.01 mmol) was weighed and methanol (100 mL) was added. Palladium on carbon (1.1 g, 10.36 mmol) and triethylamine (11.64 g, 115.07 mmol) were then added. The mixture was reacted at 25°C under a hydrogen atmosphere (15 psi) for 16 hours. The reaction mixture was filtered, and the filter cake was washed with 10 mL of methanol. The filtrate was concentrated and separated by column chromatography (petroleum ether:ethyl acetate = 5:1) to afford compound **120-2,** MS m/z = 164.1 [M+H]⁺. Preparative supercritical liquid chromatography (SFC) was then performed (chromatographic column: DAICEL CHIRALPAK^{®} IC-10, 25*250 mm 10 µm; mobile phase: A: supercritical carbon dioxide, B: methanol (0.05% diethylamine); B%: 10%) to obtain compound **120-2A** and compound **120-2B.** Analytical SFC method (Chromatographic column: DAICEL CHIRALPAK^{®}IC-3 (100*3 mm 3 µm); mobile phase: [A: supercritical carbon dioxide B: methanol (0.05% diethylamine)]; gradient B%: 5%-40%, 4 min), compound **120-2A,** Rt = 2.158 minutes, ee value 100%, MS m/z = 164.1 [M+H]⁺; compound **120-2B,** Rt= 2.398 minutes, ee value 100 %, MS m/z = 164.1 [M+H]⁺.

### Step 2

Compound **56-1** (20 mg, 20.30 µmol) was weighed and dichloromethane (1 mL) was added. N,N,N,N-tetramethyl-O-(7-azabenzotriazol-1-yl)uronium hexafluorophosphate (13.12 mg, 34.52 µmol) and diisopropylethylamine (7.87 mg, 60.91 µmol) were also added. Compound **120-2A** (4.97 mg, 30.45 µmol) was then added and stirred at room temperature for 16 hours. Column chromatography (petroleum ether:ethyl acetate = 1:1) afforded compound **120-3A.** MS m/z = 1130.8 [M+H]⁺.

Referring to step 2, compound **120-2B** was used as a starting material in place of compound **120-2A** to obtain compound **120-3B.** MS m/z = 1130.8 [M+H]⁺.

### Step 3

Compound **120-3A** (15 mg, 13.27 µmol) was weighed and dichloromethane (1 mL) was added. Trifluoroacetic acid (1 mL) was added and stirred at room temperature for 0.5 hours. The reaction solution was concentrated under reduced pressure to obtain a crude product, which was then separated by preparative HPLC (column: Waters Xbridge, 250*19 mm, 5 µm; mobile phase: [water (0.1% ammonia)-acetonitrile]; gradient: (acetonitrile): 30%-70%) to obtain compound **120A.** MS m/z = 790.5 [M+H]⁺. ¹H NMR (400 MHz, CD₃OD) δ ppm 7.69 (d, *J* = 8.0 Hz, 1H), 7.37 (d, *J* = 4.0, 1H), 7.30 - 7.26 (m, 1H), 7.22 - 7.14 (m, 1H), 6.90 (d, *J* = 8.0 Hz, 1H), 5.17 - 5.13 (m, 1H), 4.98 - 4.95 (m, 1H), 4.81 - 4.78 (m, 1H), 4.65 (d, *J =* 12.0 Hz, 1H), 4.27 - 4.08 (m, 4H), 4.02 - 3.98 (m, 1H), 3.89 - 3.84 (m, 1H), 3.56 - 3.47 (m, 3H), 3.43 (d, *J =* 12.0 Hz, 1H), 3.25 - 3.18 (m, 1H), 3.13 - 3.01 (m, 2H), 2.91 - 2.74 (m, 2H), 2.37 - 2.27 (m, 1H), 2.21 - 1.91 (m, 13H), 1.86 - 1.65 (m, 5H).

Compound **120-3B** (20 mg, 17.69 µmol) was weighed and dichloromethane (1 mL) was added. Trifluoroacetic acid (1 mL) was added and the mixture was stirred at room temperature for 0.5 hours. The reaction solution was concentrated under reduced pressure to obtain a crude product, which was then separated by preparative HPLC (column: Waters Xbridge, 250*19 mm, 5 µm; mobile phase: [water (0.1% ammonia)-acetonitrile]; gradient: (acetonitrile): 30%-70%) to obtain compound **120B.** MS m/z = 790.5 [M+H]⁺. ¹H NMR (400 MHz, CD₃OD) δ ppm 7.68 (dd, *J* = 8.0, 4.0 Hz, 1H), 7.39 - 7.37 (m, 1H), 7.30 - 7.26 (m, 1H), 7.21 - 7.17 (m, 1H), 6.90 (d, *J =* 8.0 Hz, 1H), 5.15 (dd, *J =* 12.0, 4.0 Hz, 1H), 5.01 - 4.95 (m, 1H), 4.81 - 4.78 (m, 1H), 4.65 (d, *J* = 12.0 Hz, 1H), 4.24 (d, *J* = 8.0 Hz, 1H), 4.19 - 4.07 (m, 3H), 3.98 (dd, *J =* 8.0, 4.0 Hz, 1H), 3.89 - 3.83 (m, 1H), 3.61 - 3.48 (m, 3H), 3.43 (d, *J =* 12.0 Hz, 1H), 3.26 - 3.19 (m, 1H), 3.09 - 2.95 (m, 2H), 3.88 - 2.78 (m, 2H), 2.39 - 2.29 (m, 1H), 2.24 - 1.89 (m, 13H), 1.86 - 1.64 (m, 5H).

### Example 121

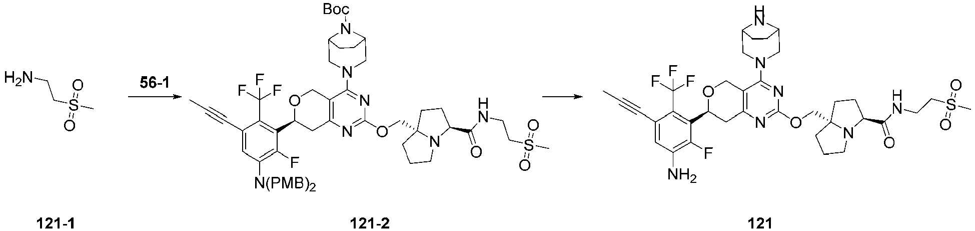

Referring to steps 2 to 3 of Example **56,** compound **121-1** was used as a starting material in place of (S)-3,3-difluorocyclopentaneamine hydrochloride to obtain crude compound **121.** The crude product was separated by preparative HPLC (column: Waters Xbridge, 250*19 mm, 5 µm; mobile phase: [water (0.1% ammonia)-acetonitrile]; gradient: (acetonitrile): 40%-70%) and dried to yield compound **121.** MS m/z = 750.5 [M+H]⁺. ¹H NMR (400 MHz, CD₃OD) δ ppm 6.90 (d, *J* = 8.0 Hz, 1H), 5.17 - 5.13 (m, 1H), 4.81 - 4.78 (m, 1H), 4.65 (d, *J* = 12.0 Hz, 1H), 4.19 - 4.12 (m, 2H), 4.06 (d, *J =* 12.0 Hz, 1H), 3.79 - 3.75 (m, 1H), 3.71 - 3.67 (m, 2H), 3.59 - 3.53 (m, 3H), 3.45-3.42 (m, 1H), 3.36 - 3.34 (m, 2H), 3.25-3.18 (m, 1H), 3.06 (d, *J* = 12.0 Hz, 1H), 3.01 (s, 3H), 2.98 - 2.95 (m, 1H), 2.87 - 2.82 (m, 1H), 2.76 - 2.70 (m, 1H), 2.14 - 2.09 (m, 2H), 2.04 - 1.99 (m, 5H), 1.96 - 1.85 (m, 6H), 1.77-1.69 (m, 2H).

### Example 122

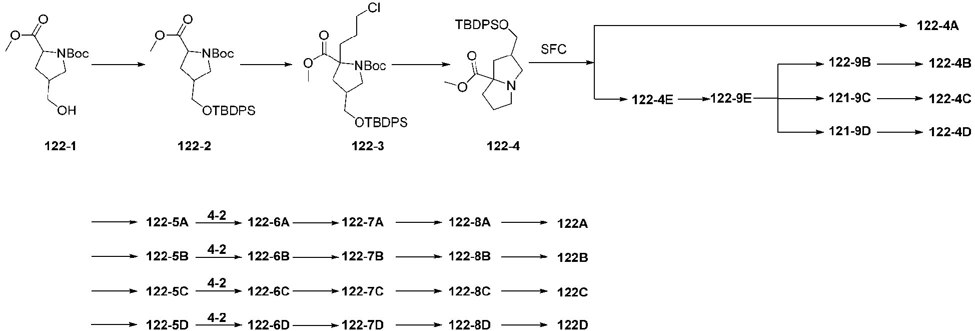

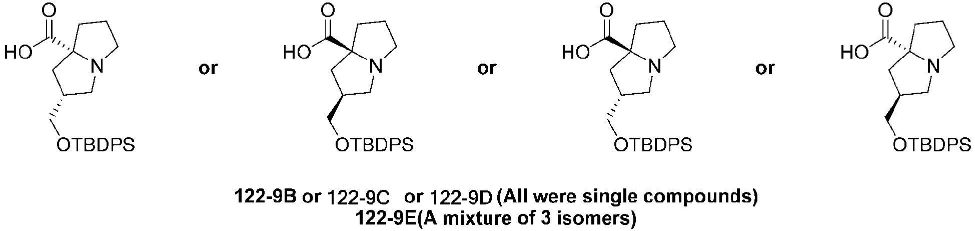

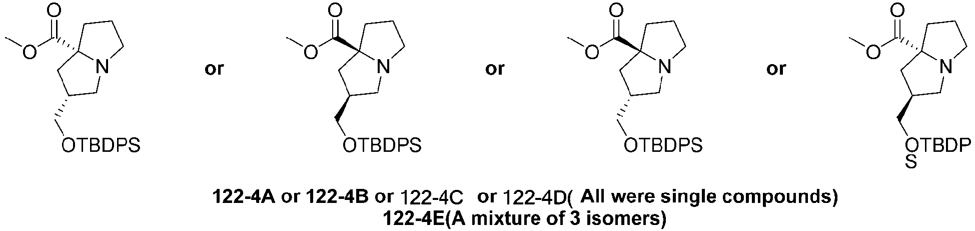

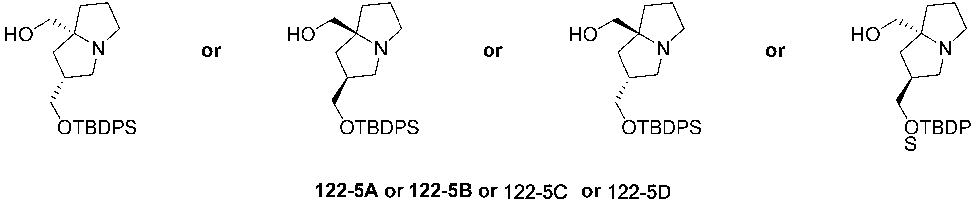

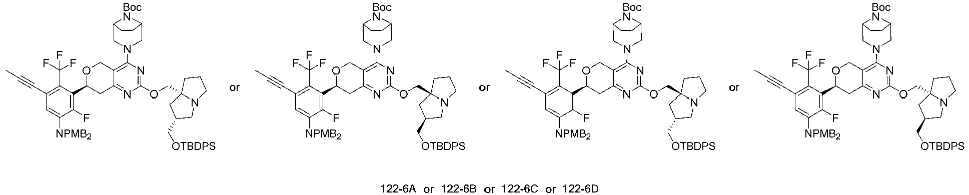

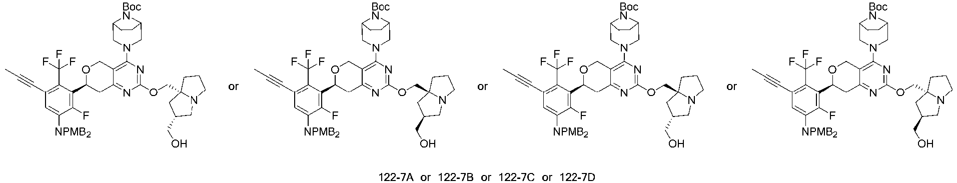

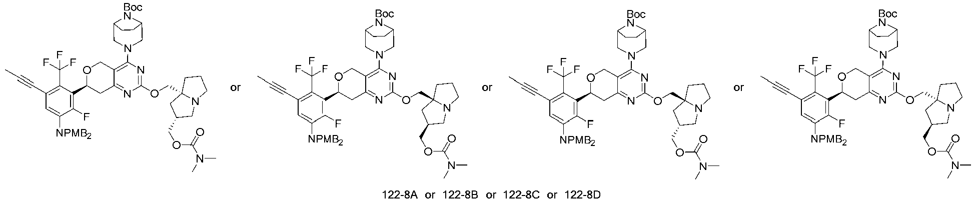

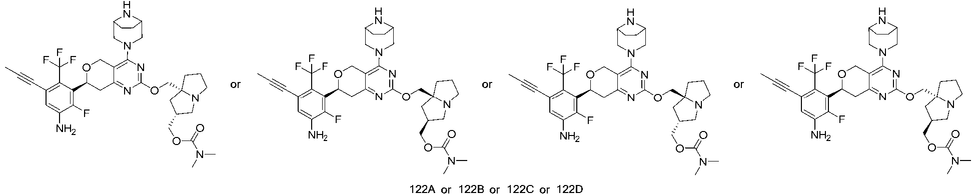

### Step 1

Compound **122-1** (4.4 g, 16.97 mol) was weighed. Dichloromethane (40 mL), imidazole (2.31 g, 33.94 mmol), and tert-butyldiphenylsilyl chloride (7 g, 25.45 mmol) were added. The mixture was reacted at room temperature for 16 hours. Water (50 mL) was added and the mixture was extracted with dichloromethane (50 mL x 3). The organic phase was concentrated under reduced pressure and separated by column chromatography (petroleum ether:ethyl acetate = 5:1) to obtain compound **122-2.** MS m/z = 498.2 [M+H]⁺.

### Step 2

Compound **122-2** (5.6 g, 11.25 mmol) was weighed and tetrahydrofuran (60 mL) was added under nitrogen. The mixture was cooled to -78°C, and lithium bistrimethylsilylamide (16.88 mmol, 16.88 mL, 1 M) was added. The mixture was reacted at -78°C for 30 minutes. 1-Chloro-3-iodopropane (3.45 g, 16.88 mmol) was added and the mixture was reacted at room temperature for 2 hours. Water (50 mL) was added, and the mixture was extracted with ethyl acetate (50 mL x 3). The organic phase was concentrated under reduced pressure and separated by column chromatography (petroleum ether:ethyl acetate = 10:1) to obtain compound **122-3.** MS m/z = 574.1 [M+H]⁺.

### Step 3

Compound **122-3** (5.7 g, 9.93 mmol) was weighed and dichloromethane (100 mL) was added. Zinc bromide (6.71 g, 29.78 mmol) was added and the reaction was performed at room temperature for 16 hours. Water (100 mL) was added, and the mixture was extracted with dichloromethane (100 mL x 3). The organic phase was concentrated under reduced pressure and separated by column chromatography (petroleum ether:ethyl acetate = 2:1) to afford compound **122-4.** MS m/z = 438.4 [M+H]⁺. Compound **122-4** was subjected to preparative separation by supercritical fluid chromatography (SFC) (column: DAICEL CHIRALPAK^{®} OJ-10, 25 x 250 mm 10 µm; mobile phase: A: supercritical carbon dioxide, B: [0.05% ethanol-diethylamine]; B%, 10%) to afford compounds **122-4A** and **122-4E** (a mixture). Analytical SFC method (Chromatographic column: ChiralPak OJ-3 (100mm*4.6mm, 3µm); mobile phase: [supercritical carbon dioxide-ethanol (0.05% diethylamine)]; (ethanol (0.05%-diethylamine)]: 5%-40%), Rt = 2.621 minutes for compound **122-4A,** MS m/z = 438.4 [M+H]⁺; Rt = 1.912- 2.026 minutes for compound **122-4E,** MS m/z = 438.4 [M+H]⁺.

### Step 4

To a solution of compound **122-4E** (2.0 g, 4.57 mmol) in tetrahydrofuran (20 mL) were added water (5 mL) and lithium hydroxide (547.21 mg, 20.85 mmol) at 25°C. The mixture was stirred at 25°C for 16 hours. 20 mL of saturated aqueous ammonium chloride was added to the system, and extraction was performed with ethyl acetate (20 mL x 3). The extracted organic phases were combined and washed with saturated brine, dried over anhydrous sodium sulfate, filtered, and concentrated under reduced pressure to obtain compound **122-9E** (a mixture). MS m/z = 424.2 [M+H]⁺. Compound **122-9E** was subjected to preparative separation by supercritical fluid chromatography (SFC) (column: DAICEL CHIRALPAK^{®} IC-10, 25*250mm 10 µm; mobile phase: A: supercritical carbon dioxide, B: [0.05% ammonia methanol-ethanol]; B%, 40%) to give compounds **122-9B, 122-9C,** and **122-9D.** Analytical SFC method (Chromatographic column: ChiralPak IC-3 (100mm*4.6mm, 3µm); mobile phase: [supercritical carbon dioxide-ethanol (0.05% ammonia methanol)]; (ethanol (0.05% ammonia methanol)]: 5%-40%), Rt = 6.561 minutes for compound **122-9B,** MS m/z = 424.2 [M+H]⁺; Rt = 7.912 minutes for compound **122-9C,** MS m/z = 424.2 [M+H]⁺; Rt = 10.380 minutes for compound **122-9D,** MS m/z = 424.2 [M+H]⁺.

### Step 5

To a solution of compound **122-9B** (450 mg, 1.06 mmol) in dichloromethane (6 mL) was slowly added oxalyl chloride at 0°C. The reaction mixture was stirred at 0°C for 0.5 h. The reaction mixture was then slowly added to a methanol solution (6 mL) at 0°C and stirred for additional 0.5 h. The reaction mixture was concentrated under reduced pressure. Column chromatography (dichloromethane:methanol = 20:1) afforded compound **122-4B.** MS m/z = 438.3 [M+H]⁺.

Referring to step 5, compound **122-9C** was used as a starting material in place of compound **122-9B** to obtain compound **122-4C.** m/z = 438.3 [M+1]⁺.

Referring to step 5, compound **122-9D** was used as a starting material in place of compound **122-9B** to obtain compound **122-4D.** m/z = 438.3 [M+1]⁺.

### Step 6

Referring to steps 11 to 15 of Example **36,** compound **122-4A** was used as a starting material in place of compound **16-5C** to obtain crude compound **122A.** The crude product was separated by preparative HPLC (Chromatographic column: Waters Xbridge, 250 x 19 mm, 5 mm; mobile phase: [water (0.1% formic acid)-acetonitrile]; (acetonitrile): 40%-70%) and dried to afford the formate salt of compound **122A.** MS m/z = 702.6 [M+H]⁺. ¹H NMR (400 MHz, DMSO-*d₆*) δ ppm 8.25 (s, 1.77H), 6.88 (d, *J =* 8.0 Hz, 1H), 6.02 (s, 2H), 5.05 - 5.01 (m, 1H), 4.76 (d, *J* = 12.0 Hz, 1H), 4.57 (d, *J =* 12.0 Hz, 1H), 4.02 - 3.86 (m, 5H), 3.69 (s, 2H), 3.45 (d, *J =* 12.0 Hz, 1H), 3.36 (d, *J =* 12.0 Hz, 1H), 3.16 - 2.99 (m, 3H), 2.81 - 2.72 (m, 8H), 2.67 - 2.60 (m, 3H), 2.32 - 2.27 (m, 1H), 2.09 - 1.65 (m, 12H), 1.33 - 1.30 (m, 1H).

Referring to steps 11 to 15 of Example **36,** compound **122-4B** was used as a starting material in place of compound **16-5C** to obtain crude compound **122B.** The crude product was separated by preparative HPLC (Chromatographic column: Waters Xbridge, 250 x 19 mm, 5 mm; mobile phase: [water (0.1% formic acid)-acetonitrile]; (acetonitrile): 40%-70%) and dried to afford the formate salt of compound **122B.** MS m/z = 702.6 [M+H]⁺. ¹H NMR (400 MHz, DMSO *-d₆*) δ ppm 8.24 (s, 1.63H), 6.88 (d, *J =* 8.0 Hz, 1H), 6.02 (s, 2H), 5.06 - 5.01 (m, 1H), 4.76 (d, *J* = 16.0 Hz, 1H), 4.58 (d, *J* = 12.0 Hz, 1H), 4.04 - 3.87 (m, 5H), 3.68 - 3.65 (m, 2H), 3.44 (d, *J* = 12.0 Hz, 1H), 3.35 (d, *J =* 12.0 Hz, 1H), 3.10 - 2.97 (m, 3H), 2.87 - 2.74 (m, 8H), 2.68 - 2.52 (m, 3H), 2.49 - 2.40 (m, 1H), 2.03 (s, 3H), 1.98 - 1.83 (m, 3H), 1.82 - 1.62 (m, 5H), 1.60 - 1.49 (m, 2H).

Referring to steps 11 to 15 of Example **36,** compound **122-4C** was used as a starting material in place of compound **16-5C** to obtain crude compound **122C.** The crude product was separated by preparative HPLC (Chromatographic column: Waters Xbridge, 250 x 19 mm, 5 mm; mobile phase: [water (0.1% formic acid)-acetonitrile]; (acetonitrile): 40%-70%) and dried to afford the formate salt of compound **122C.** MS m/z = 702.6 [M+H]⁺. ¹H NMR (400 MHz, DMSO *-d₆)* δ ppm 8.25 (s, 1.95H), 6.88 (d, *J =* 8.0 Hz, 1H), 6.02 (s, 2H), 5.06 - 5.00 (m, 1H), 4.76 (d, *J* = 12.0 Hz, 1H), 4.57 (d, *J* = 12.0 Hz, 1H), 4.02 - 3.89 (m, 5H), 3.64 - 3.58 (m, 2H), 3.42 (d, *J* = 12.0 Hz, 1H), 3.33 (d, *J =* 12.0 Hz, 1H), 3.10 - 2.94 (m, 3H), 2.85 - 2.72 (m, 8H), 2.68 - 2.51 (m, 3H), 2.47 - 2.38 (m, 1H), 2.03 (s, 3H), 1.97 - 1.62 (m, 8H), 1.57 - 1.49 (m, 2H).

Referring to steps 11 to 15 of Example **36,** compound **122-4D** was used as a starting material in place of compound **16-5C** to obtain crude compound **122D.** The crude product was separated by preparative HPLC (Chromatographic column: Waters Xbridge, 250 x 19 mm, 5 mm; mobile phase: [water (0.1% formic acid)-acetonitrile]; (acetonitrile): 40%-70%) and dried to afford the formate salt of compound **122D.** MS m/z = 702.6 [M+H]⁺. ¹H NMR (400 MHz, DMSO-*d₆*) δ ppm 8.24 (s, 1.30H), 6.88 (d, *J =* 8.0 Hz, 1H), 6.02 (s, 2H), 5.11 - 4.99 (m, 1H), 4.76 (d, *J* = 16.0 Hz, 1H), 4.58 (d, *J* = 12.0 Hz, 1H), 4.04 - 3.83 (m, 5H), 3.67 (s, 2H), 3.53 - 3.28 (m, 2H), 3.17 - 2.81 (m, 11H), 2.68 - 2.51 (m, 2H), 2.32 - 2.27 (m, 1H), 2.11 - 2.05 (m, 1H), 2.03 (s, 3H), 1.97 - 1.54 (m, 9H), 1.31 (t, *J =* 9.0 Hz, 1H).

### Example 123

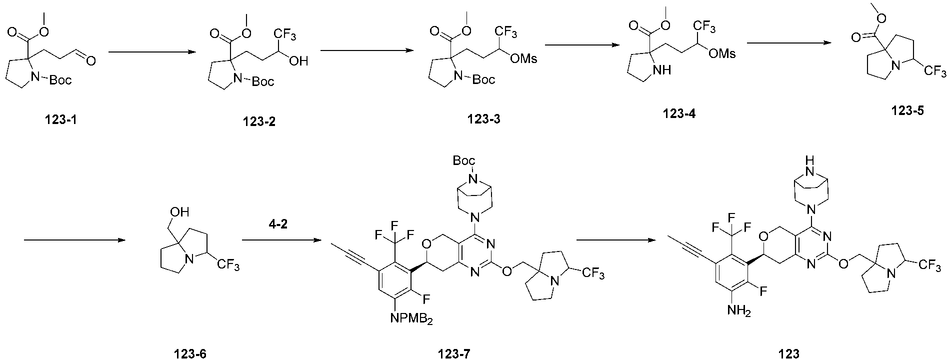

### Step 1

Compound **123-1** (800 mg, 2.81 mmol) was dissolved in anhydrous tetrahydrofuran (10 mL), and cooled to 0°C. (Trifluoromethyl)trimethylsilane (598 mg, 4.21 mmol) and tetrabutylammonium fluoride (2.8 mL, 1 M solution in tetrahydrofuran) were added. The reaction mixture was stirred at room temperature for 16 hours. Saturated aqueous sodium bicarbonate (5 mL) was added to quench the reaction, and the mixture was extracted with ethyl acetate (10 mL x 3). The extracted organic phases were combined and washed with saturated brine (20 mL), dried over anhydrous sodium sulfate, and filtered. The filtrate was concentrated under reduced pressure, and separated by column chromatography (petroleum ether:ethyl acetate = 5:1) to obtain compound **123-2.** MS m/z = 356.2 [M+H]⁺.

### Step 2

C ompound **123-2** (350 mg, 0.98 mmol) was dissolved in dichloromethane (5 mL). Methylsulfonyl chloride (226 mg, 1.97 mmol) and triethylamine (200 mg, 1.97 mmol) were added. The reaction mixture was stirred at room temperature for 2 hours. After completion of the reaction, the reaction mixture was concentrated under reduced pressure to obtain crude compound **123-3.** MS m/z = 434.1 [M+H]⁺.

### Step 3

Compound **123-3** (350 mg, crude) was dissolved in a solution of hydrochloride in dioxane (5 mL, 4M). The reaction mixture was stirred at room temperature for 2 hours. After completion of the reaction, the reaction mixture was concentrated under reduced pressure to obtain crude compound **123-4.** MS m/z = 334.1 [M+H]⁺.

### Step 4

Compound **123-4** (250 mg, crude) was dissolved in acetonitrile (8 mL), and potassium carbonate (311 mg, 2.25 mmol) was added. The reaction mixture was stirred at 65°C for 16 hours. After completion of the reaction, the reaction mixture was filtered and concentrated under reduced pressure to obtain crude compound **123-5.** MS m/z = 238.1 [M+H]⁺.

### Step 5

Compound **123-5** (100 mg, crude) was dissolved in anhydrous tetrahydrofuran (2 mL), and cooled to 0°C. Lithium aluminum tetrahydride (0.8 mL, 0.83 mmol, 1 M) was slowly added. The reaction mixture was stirred at room temperature for 1 hour. Sodium sulfate decahydrate was slowly added to quench the reaction. The reaction mixture was filtered and concentrated under reduced pressure to obtain crude compound **123-6.** MS m/z = 210.2 [M +H]⁺.

### Step 6

Compound **123-6** (70 mg, crude) was dissolved in anhydrous tetrahydrofuran (2 mL), and cooled to 0°C. Sodium hydride (31.33 mg, 0.78 mmol, 60% purity) was added. After stirring at 0°C for half an hour, compound **4-2** (294 mg, 0.33 mmol) was added and stirred at room temperature for 1 hour. The reaction was quenched with a saturated aqueous solution of ammonium chloride (2 mL). The mixture was extracted with ethyl acetate (10 mL x 3). The extracted organic phases were combined and washed with saturated brine (20 mL), dried over anhydrous sodium sulfate, and filtered. The filtrate was concentrated under reduced pressure, and separated by column chromatography (petroleum ether:ethyl acetate = 1:2) to obtain compound **123-7.** MS m/z = 1009.6 [M+H]⁺.

### Step 7

Compound **123-7** (40 mg, 39.64 µmol) was added to trifluoroacetic acid (1 mL) and stirred at 25°C for 1 hour. The reaction solution was concentrated under reduced pressure to obtain a crude product. The crude product was separated by preparative HPLC (Chromatographic column: Xbridge C18 250*19mm*5µm; mobile phase: [water (0.1% ammonia)-acetonitrile]; (acetonitrile): 55%-80%) and dried to obtain compound **123.** MS m/z = 669.4 [M+H]⁺. ¹H NMR (400 MHz, CD₃OD) δ ppm 6.90 (d, *J =* 8.0 Hz, 1H), 5.17 - 5.13 (m, 1H), 4.81 - 4.77 (m, 2H), 4.67 (d, *J =* 16.0 Hz, 1H), 4.19 - 4.15 (m, 2H), 4.10 - 4.17 (m, 1H), 3.83 - 3.82 (m, 2H), 3.63 - 3.59 (m, 1H), 3.53 - 3.48 (m, 1H), 3.24 - 3.13 (m, 3H), 2.96 - 2.84 (m, 2H), 2.10 - 2.09 (m, 3H), 2.05 - 2.01 (m, 7H), 1.9 - 1.89 (m, 4H), 1.62 - 1.59 (m, 1H).

### Example 124

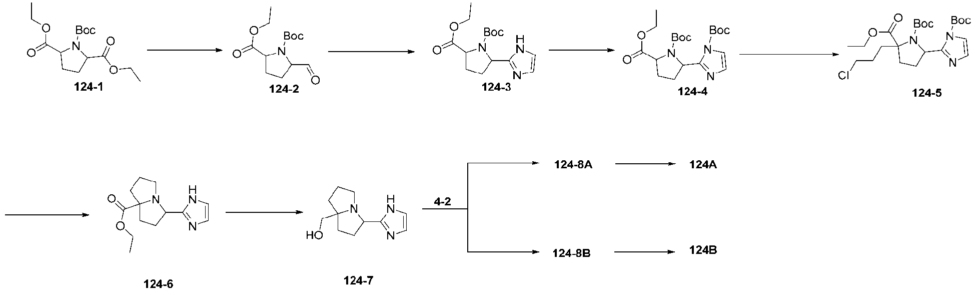

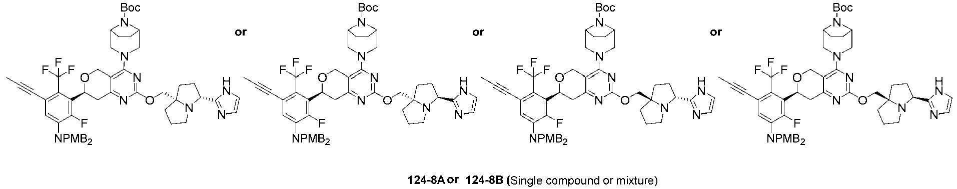

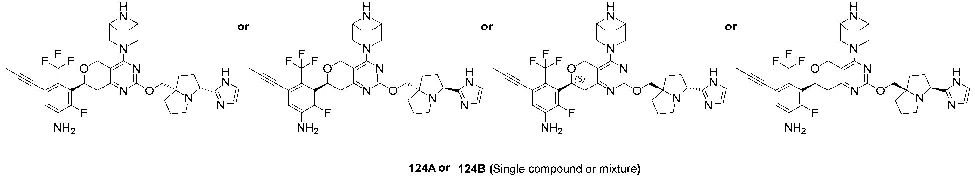

### Step 1

Compound **124-1** (1.55 g, 4.92 mol) was weighed and diethyl ether (20 mL) was added under nitrogen. The mixture was cooled to -78°C, and diisobutylaluminum hydride (7.38 mol, 7.38 mL, 1 M solution in tetrahydrofuran) was added. The mixture was reacted at -78°C for 1 hour. Water (1 mL) was added to the reaction solution at 0°C, and the reaction was quenched by saturated ammonium chloride solution (15 mL). The solution was extracted with ethyl acetate (15 mL x 3). The organic phase was concentrated under reduced pressure and separated by column chromatography (petroleum ether:ethyl acetate = 5:1) to obtain compound **124-2.** MS m/z = 272.1 [M+H]⁺.

### Step 2

Compound **124-2** (600 mg, 2.21 mmol) was weighed and methanol (15 mL), 40% aqueous glyoxal (1.28 mL, 8.85 mmol), and 25% aqueous ammonia (1.86 mL, 13.27 mmol) were added. The mixture was reacted at room temperature for 16 hours. The mixture was concentrated under reduced pressure. Water (10 mL) was added, and the mixture was extracted with ethyl acetate (15 mL x 3). The organic phase was concentrated under reduced pressure and separated by column chromatography (dichloromethane:methanol = 20:1) to obtain compound **124-3.** MS m/z = 310.2 [M+H]⁺.

### Step 3

Compound **124-3** (200 mg, 646.50 µmol) was weighed and dichloromethane (5 mL), di-tert-butyl dicarbonate (282 mg, 1.29 mmol), triethylamine (196 mg, 1.94 mmol), and 4-dimethylaminopyridine (15.8 mg, 129.3 µmol) were added. The mixture was reacted at room temperature for 2 hours. Water (15 mL) was added and the mixture was extracted with dichloromethane (15 mL x 3). The organic phase was concentrated under reduced pressure and separated by column chromatography (petroleum ether:ethyl acetate = 5:1) to obtain compound **124-4.** MS m/z = 410.3 [M+H]⁺.

### Step 4

Compound **124-4** (210 mg, 512.80 µmol) was weighed and tetrahydrofuran (10 mL) was added under nitrogen. The mixture was cooled to -78°C, and LiHMDS (1.03 mmol, 1.03 mL, 1 M in THF) was added. The mixture was reacted at -78°C for 30 minutes. 1-Chloro-3-iodopropane (209 mg, 1.03 mmol) was added and the reaction was continued at room temperature for 2 hours. Water (15 mL) was added, and the mixture was extracted with ethyl acetate (15 mL x 3). The organic phase was concentrated under reduced pressure and separated by column chromatography (petroleum ether:ethyl acetate = 5:1) to obtain compound **124-5.** MS m/z = 486.3 [M+H]⁺.

### Step 5

Compound **124-5** (140 mg, 288.07 µmol) was weighed and dissolved in dichloromethane (5 mL). A solution of hydrochloric acid in ethyl acetate (0.5 mL, 4 M) was added and allowed to react at room temperature for 30 minutes. The organic solvent was removed under reduced pressure, and methanol (5 mL) was added. Ammonia in methanol (0.2 mL, 1.4 mmol, 7 M) was also added, and the mixture was allowed to react at room temperature for 30 minutes. The reaction solution was concentrated under reduced pressure and separated by column chromatography (dichloromethane:methanol = 20:1) to obtain compound **124-6.** MS m/z = 250.0 [M+H]⁺.

### Step 6

Compound **124-6** (90 mg, 361 µmol) was weighed and anhydrous tetrahydrofuran (10 mL) was added under nitrogen. The mixture was cooled to 0°C, and lithium aluminum tetrahydride (541.5 µL, 541.5 µmol, 1 M tetrahydrofuran solution) was added. The reaction was allowed to react at 0°C for 2 hours. Dichloromethane (50 mL) was added for dilution. Water (0.2 mL) was added to the reaction mixture at 0°C, and the reaction was quenched by adding 15% sodium hydroxide solution (0.2 mL). The reaction mixture was stirred for 10 minutes, and filtered. The filter cake was washed with dichloromethane (10 mL), and dried over anhydrous sodium sulfate. The filtrate was concentrated to obtain compound **124-7.** MS m/z = 208.2 [M+H]⁺.

### Step 7

Compound **124-7** (40 mg, 192.98 µmol) was weighed and anhydrous tetrahydrofuran (5 mL) was added. 60% pure sodium hydride (23.16 mg, 964.92 µmol) was added under ice-cooling. The mixture was stirred at room temperature for 30 minutes, and compound **4-2** (169.82 mg, 192.98 µmol) was added. The reaction mixture was allowed to react at room temperature for 1 hour. The reaction mixture was quenched with water (10 mL) and extracted with ethyl acetate (20 mL x 3). The organic phase was dried over anhydrous sodium sulfate, concentrated under reduced pressure, and separated by column chromatography (petroleum ether:ethyl acetate = 1:1) to afford **124-8A** (dichloromethane:methanol = 10:1, Rf = 0.45), MS m/z = 1007.8 [M+H]⁺, and **124-8B** (dichloromethane:methanol = 10:1, Rf = 0.4), MS m/z = 1007.8 [M+H]⁺.

### Step 8

Compound **124-8A** (28 mg, 27.80 µmol) was weighed and dichloromethane (1 mL) was added. Trifluoroacetic acid (1 mL) was added and the mixture was stirred at room temperature for 0.5 hours. The reaction mixture was concentrated under reduced pressure to obtain a crude product, which was then separated by preparative HPLC (column: Waters Xbridge, 250 x 19 mm, 5 µm; mobile phase: [water (0.1% ammonia)-acetonitrile]; gradient: (acetonitrile): 30%-70%). After drying, compound **124A** was obtained. MS m/z = 667.5 [M+H]⁺. ¹H NMR (400 MHz, CD₃OD) δ ppm 6.99 - 6.72 (m, 3H), 5.15 (dd, *J =* 12.0, 4.0 Hz, 1H), 4.77 (d, *J =* 12.0 Hz, 1H), 4.66 (d, *J =* 12.0 Hz, 1H), 4.59 - 4.53 (m, 1H), 4.21 - 4.09 (m, 2H), 3.96 - 3.93 (m, 1H), 3.60 - 3.52 (m, 2H), 3.46 - 3.41 (m, 1H), 3.23 (dd, *J =* 16.0, 8.0 Hz, 1H), 3.09 - 2.83 (m, 3H), 2.23 - 2.15 (m, 3H), 2.11 - 1.94 (m, 6H), 1.90 - 1.61 (m, 8H).

Compound **124-8B** (56 mg, 55.60 µmol) was weighed and dichloromethane (1 mL) was added. Trifluoroacetic acid (1 mL) was added and stirred at room temperature for 0.5 hours. The reaction mixture was concentrated under reduced pressure to obtain a crude product, which was then separated by preparative HPLC (column: Waters Xbridge, 250*19 mm, 5 µm; mobile phase: [water (0.1% ammonia)-acetonitrile]; gradient: (acetonitrile): 30%-70%). After drying, compound **124B** was obtained. MS m/z = 667.5 [M+H]⁺. ¹H NMR (400 MHz, CD₃OD) δ ppm 7.02 (s, 2H), 6.90 (d, *J =* 8.0 Hz, 1H), 5.15 (dd, *J =* 12.0, 4.0 Hz, 1H), 4.82 - 4.78 (m, 1H), 4.65 (d, *J* = 12.0 Hz, 1H), 4.38 - 4.34 (m, 1H), 4.24 - 4.13 (m, 3H), 3.53 - 3.50 (m, 3H), 3.43 - 3.39 (m, 1H), 3.22 (dd, *J =* 20.0, 8.0 Hz, 1H), 3.04 (d, *J =* 12.0 Hz, 1H), 2.85 (dd, *J =* 16.0, 4.0 Hz, 1H), 2.61 - 2.54 (m, 1H), 2.45 - 2.40 (m, 2H), 2.19 - 2.14 (m, 1H), 2.09 - 1.96 (m, 7H), 1.83 - 1.67 (m, 6H).

### Example 125

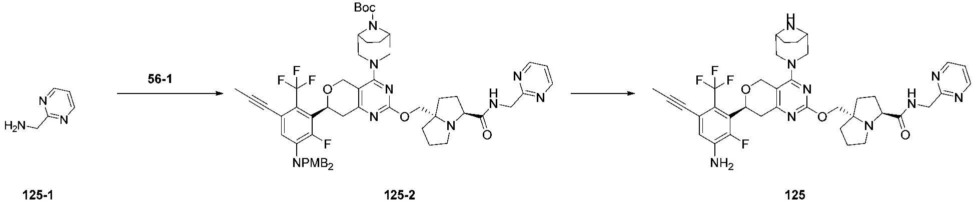

Referring to steps 2 to 3 of Example **56,** compound **125-1** was used as a starting material in place of (S)-3,3-difluorocyclopentaneamine hydrochloride to obtain crude compound **125.** This crude product was separated by preparative HPLC (column: Waters Xbridge, 250*19 mm, 5 µm; mobile phase: [water (0.1% formic acid)-acetonitrile]; gradient: (acetonitrile): 30%-70%) to afford the formate salt of compound 125. MS m/z = 736.6 [M+H]⁺. ¹H NMR (400 MHz, CD₃OD) δ ppm 8.74 (d, *J =* 4.0 Hz, 2H), 8.45 (brs, 0.91H), 7.37 (t, *J* = 4.0 Hz, 1H), 6.91 (d, *J =* 8.0 Hz, 1H), 5.22 - 5.14 (m, 1H), 4.88 - 4.86 (m, 1H), 4.74 - 4.61 (m, 3H), 4.48 - 4.24 (m, 4H), 4.10 - 4.04 (m, 2H), 3.77 (d, *J =* 16.0 Hz, 1H), 3.63 (d, *J =* 12.0 Hz, 1H), 3.54 - 3.45 (m, 1H), 3.31 - 3.24 (m, 2H), 3.23 - 3.10 (m, 1H), 2.92 (dd, *J* = 20.0, 4.0 Hz, 1H), 2.51 - 2.31 (m, 2H), 2.28 - 2.20 (m, 3H), 2.12 - 2.07 (m, 4H), 2.02 - 1.92 (m, 6H).

### Example 126

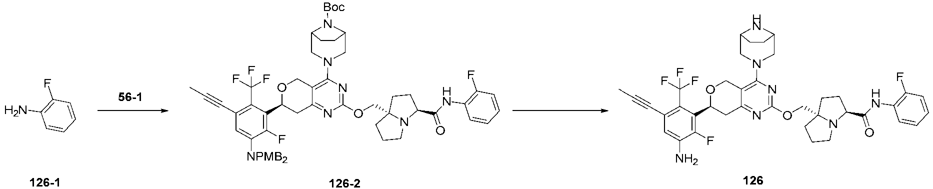

### Step 1

Compound **56-1** (50 mg, 50.76 µmol) was weighed and acetonitrile (1 mL) was added, followed by N,N,N',N'-tetramethylchloroformamidine hexafluorophosphate (15.67 mg, 55.83 µmol) and N-methylimidazole (8.75 mg, 106.59 µmol). Compound **126-1** (8.46 mg, 76.14 µmol) was then added and the mixture was stirred at room temperature for 16 hours. Column chromatography (petroleum ether:ethyl acetate = 1:1) afforded **126-2**. MS m/z = 1078.6 [M+H]⁺.

### Step 2

Compound **126-2** (17 mg, 15.77 µmol) was weighed and dichloromethane (0.5 mL) and trifluoroacetic acid (0.5 mL) were added. The mixture was stirred at room temperature for 0.5 hours. The reaction mixture was concentrated under reduced pressure to obtain a crude product, which was then separated by preparative HPLC (column: Waters Xbridge, 250*19 mm, 5 µm; mobile phase: [water (0.1% ammonia)-acetonitrile]; gradient: (acetonitrile): 30%-70%) to obtain compound **126.** MS m/z = 738.5 [M+H]⁺. ¹H NMR (400 MHz, CD₃OD) *δ* 7.94 - 7.84 (m, 1H), 7.22 - 7.11 (m, 3H), 6.91 (d, *J* = 8.0 Hz, 1H), 5.16 (dd, *J* = 12.0, 4.0 Hz, 1H), 4.81 - 4.79 (m, 1H), 4.67 (d, *J* = 12.0 Hz, 1H), 4.26 - 4.16 (m, 2H), 4.12 (d, *J* = 12.0 Hz, 1H), 4.06 - 4.02 (m, 1H), 3.73 - 3.68 (m, 2H), 3.63 - 3.58 (m, 1H), 3.48 (d, *J =* 12.0 Hz, 1H), 3.28 - 3.19 (m, 1H), 3.14 - 3.08 (m, 1H), 3.06 - 3.01 (m, 1H), 2.91 - 2.81 (m, 2H), 2.25 - 2.06 (m, 5H), 2.04 - 1.99 (m, 4H), 1.97 - 1.90 (m, 4H), 1.81 - 1.75 (m, 2H).

### Example 127

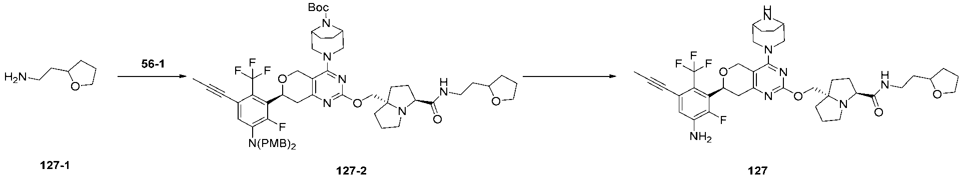

### Step 1

Compound **56-1** (60 mg, 60.91 µmol) was weighed and acetonitrile (1 mL) was added. N,N,N',N'-tetramethylchloroformamidine hexafluorophosphate (18.80 mg, 67.00 µmol), N-methylimidazole (10.50 mg, 127.91 µmol), and compound **127-1** (10.52 mg, 91.36 µmol) were also added. The mixture was stirred at room temperature for 16 hours. Column chromatography (petroleum ether:ethyl acetate = 0:1) afforded compound **127-2.** MS m/z = 1082.7 [M+H]⁺.

### Step 2

Compound **127-2** (15 mg, 13.86 µmol) was weighed and dichloromethane (0.5 mL) and trifluoroacetic acid (0.5 mL) were added. The mixture was stirred at room temperature for 0.5 hours. The reaction mixture was concentrated under reduced pressure to obtain a crude product, which was then separated by preparative HPLC (column: Waters Xbridge, 250*19 mm, 5 µm; mobile phase: [water (0.1% ammonia)-acetonitrile]; gradient: (acetonitrile): 30%-70%) to obtain compound **127.** MS m/z = 742.6 [M+H]⁺. ¹H NMR (400 MHz, CD₃OD) *δ* ppm 6.90 (d, *J* = 8.0 Hz, 1H), 5.16 - 5.12 (m, 1H), 4.81- 4.77 (m, 1H), 4.65 (d, *J* = 12.0 Hz, 1H), 4.18 - 4.12 (m, 2H), 4.05 (d, *J =* 12.0 Hz, 1H), 3.89 - 3.82 (m, 2H), 3.77 - 3.65 (m, 2H), 3.56 - 3.46 (m, 3H), 3.45 - 3.36 (m, 1H), 3.35 - 3.32 (m, 1H), 3.29 - 3.15 (m, 2H), 3.04 (d, *J =* 12.0 Hz, 1H), 2.95 - 2.79 (m, 2H), 2.77 - 2.66 (m, 1H), 2.20 - 2.03 (m, 3H), 2.03 - 1.99 (m, 4H), 1.99 - 1.77 (m, 9H), 1.77 - 1.63 (m, 4H), 1.56 - 1.47 (m, 1H).

### Example 128

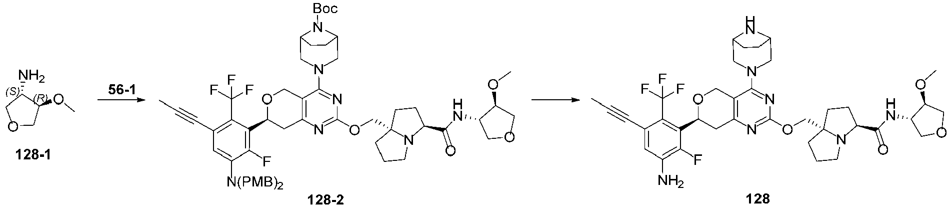

Referring to steps 2 to 3 of Example **56, 128-1** was used as a starting material in place of (S)-3,3-difluorocyclopentylamine hydrochloride to obtain crude compound **128.** The crude product was separated by preparative HPLC (Chromatographic column: Xtimate C18, 250 x 19 mm, 5 µm; mobile phase: [water (0.1% ammonia)-acetonitrile]; gradient: (acetonitrile): 20% - 42%) and dried to yield compound **128.** MS m/z = 744.6 [M+H]⁺. ¹H NMR (400 MHz, CD₃OD) δ ppm 6.90 (d, *J =* 8.0 Hz, 1H), 5.15 (dd, *J =* 12.0, 4.0 Hz, 1H), 4.79 (d, *J= 16.0* Hz, 1H), 4.65 (d, *J* = 16.0 Hz, 1H), 4.29 (s, 1H), 4.15 (t, *J* = 12.0 Hz, 2H), 4.05 (d, *J* = 12.0 Hz, 1H), 4.00 - 3.95 (m, 2H), 3.86 - 3.81 (m, 1H), 3.79 - 3.65 (m, 3H), 3.54 - 3.51 (m, 3H), 3.43 - 3.38 (m, 4H), 3.22 (dd, *J =* 16.0, 12.0 Hz, 1H), 3.04 (d, *J =* 12.0 Hz, 1H), 2.92 - 2.81 (m, 2H), 2.77 - 2.70 (m, 1H), 2.15 - 2.07 (m, 2H), 2.04 - 1.97 (m, 5H), 1.95 - 1.80 (m, 6H), 1.77 - 1.66 (m, 2H).

### Example 129

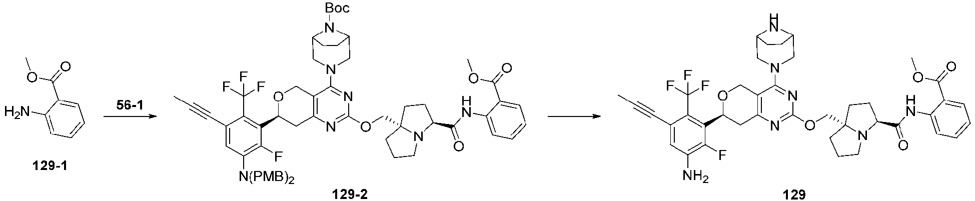

Referring to steps 2 to 3 of Example **56,** compound **129-1** was used as a starting material in place of (S)-3,3-difluorocyclopentylamine hydrochloride to obtain crude compound **129.** The crude product was separated by preparative HPLC (column: Waters Xbridge, 250*19 mm, 5 µm; mobile phase: [water (0.1% ammonia)-acetonitrile]; gradient: (acetonitrile): 60%-80%) and dried to yield compound **129.** MS m/z = 778.5 [M+H]⁺. ¹H NMR (400 MHz, CD₃OD) δ ppm 8.68 (d, *J =* 8.0 Hz, 1H), 8.04 (d, *J =* 8.0 Hz, 1H), 7.56 (t, *J* = *8.0 Hz,* 1H), 7.14 (t, *J =* 8.0 Hz, 1H), 6.90 (d, *J =* 8.0 Hz, 1H), 5.08 (dd, *J=* 12.0, 4.0 Hz, 1H), 4.74 (d, *J =* 12.0 Hz, 1H), 4.59 (d, *J* = 16.0 Hz, 1H), 4.30 (d, *J =* 12.0 Hz, 1H), 4.23 (d, *J =* 12.0 Hz, 1H), 4.03 (d, *J =* 12.0 Hz, 1H), 3.82 - 3.75 (m, 4H), 3.52 - 3.45 (m, 5H), 3.11 - 3.04 (m, 1H), 2.93 (d, *J* = 12.0 Hz, 1H), 2.79 - 2.70 (m, 2H), 2.43 - 2.35 (m, 1H), 2.21 - 2.16 (m, 2H), 2.01 - 1.99 (m, 5H), 1.94 - 1.88 (m, 2H), 1.82 - 1.71 (m, 4H), 1.60 (d, *J* = 12.0 Hz, 1H).

### Example 130

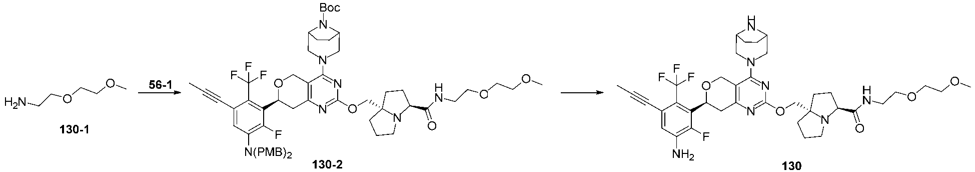

Referring to steps 2 to 3 of Example **56,** compound **130-1** was used as a starting material in place of (*S*)-3,3-difluorocyclopentylamine hydrochloride to obtain crude compound **130.** The crude product was separated by preparative HPLC (Chromatographic column: Xtimate C18, 250*19mm, 5µm; mobile phase: [water (0.1% ammonia)-acetonitrile]; gradient: (acetonitrile): 30%-70%) and dried to yield compound **130.** MS m/z = 746.6 [M+H]⁺. ¹H NMR (400 MHz, CD₃OD) δ ppm 6.90 (d, *J =* 8.0 Hz, 1H), 5.15 (dd, *J =* 12.0, 4.0 Hz, 1H), 4.80 (d, *J* = 16.0 Hz, 1H), 4.65 (d, *J =* 16.0 Hz, 1H), 4.15 (t, *J =* 12.0 Hz, 2H), 4.05 (d, *J =* 12.0 Hz, 1H), 3.79 - 3.75 (m, 1H), 3.62 - 3.58 (m, 2H), 3.58 - 3.49 (m, 7H), 3.48 - 3.40 (m, 2H), 3.39 - 3.34 (m, 4H), 3.21 (dd, *J =* 20.0, 12.0 Hz, 1H), 3.05 (d, *J =* 12.0 Hz, 1H), 2.98 - 2.92 (m, 1H), 2.84 (dd, *J =* 16.0, 4.0 Hz, 1H), 2.77 - 2.71 (m, 1H), 2.16 - 2.09 (m, 2H), 2.05 - 1.98 (m, 5H), 1.96 - 1.81 (m, 6H), 1.77 - 1.67 (m, 2H).

### Example 131

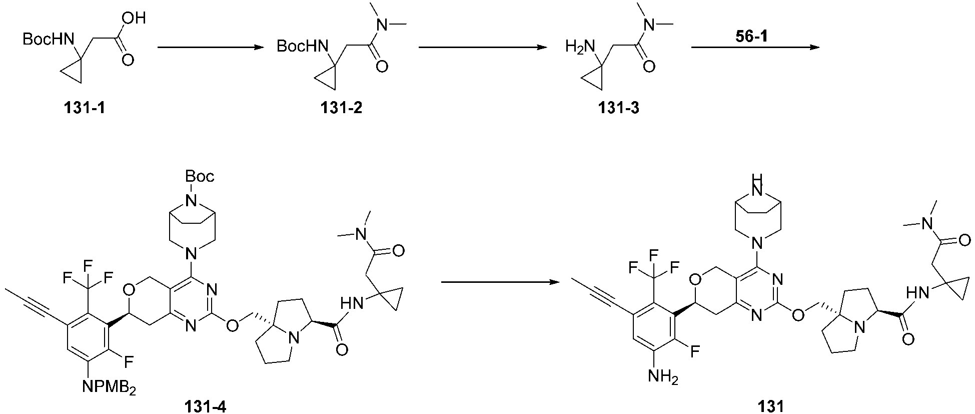

### Step 1

Compound **131-1** (85 mg, 394.90 µmol) was dissolved in N,N-dimethylformamide (2.0 mL), and N,N,N',N'-tetramethyl-O-(7-azabenzotriazol-1-yl)uronium hexafluorophosphate (330.30 mg, 789.80 µmol), N,N-diisopropylethylamine (153.12 mg, 1184.70 µmol), and dimethylamine hydrochloride (35.61 mg, 789.80 µmol) were added. The mixture was reacted at 25°C for 2 hours. The reaction solution was concentrated under reduced pressure and chromatographed (Flash Spherical C18; [water (0.1% formic acid)-acetonitrile]; gradient: (acetonitrile): 0%-40%) to obtain compound **131-2.** MS m/z = 243.2 [M+H]⁺.

### Step 2

Compound **131-2** (45 mg, 185.71 µmol) was weighed. Hydrochloric acid (1.0 mL, 2.5 M) was added, and the mixture was reacted at 25°C for 0.5 h. The reaction solution was concentrated under reduced pressure to obtain the hydrochloride salt of crude compound **131-3.** MS m/z = 143.2 [M+H]⁺.

### Step 3

Referring to steps 2 to 3 of Example **56,** compound **131-3** was used as a starting material in place of compound (S)-3,3-difluorocyclopentylamine hydrochloride to obtain crude compound **131.** The crude product was separated by preparative HPLC (Chromatographic column: Waters Xbridge, 250*19 mm, 5 µm; mobile phase: [water (0.1% ammonia)-acetonitrile]; gradient: (acetonitrile): 10%-40%) to afford compound **131.** MS m/z = 769.6 [M+H]⁺. ¹H NMR (400 MHz, CD₃OD) *δ* ppm 6.90 (d, *J =* 8.0 Hz, 1H), 5.14 (dd, *J* = 12.0, 4.0 Hz, 1H), 4.79 (d, *J =* 16.0 Hz, 1H), 4.65 (d, *J =* 12.0 Hz, 1H), 4.14 (d, *J =* 8.0 Hz, 2H), 4.03 (d, *J =* 12.0 Hz, 1H), 3.72 - 3.62 (m, 1H), 3.52 (d, *J* = 12.0 Hz, 3H), 3.41 (d, *J =* 12.0 Hz, 1H), 3.21 (dd, *J =* 16.0, 8.0 Hz, 1H), 3.06 - 3.01 (m, 4H), 2.91 (s, 3H), 2.88 - 2.78 (m, 3H), 2.73 - 2.63 (m, 2H), 2.13 - 1.98 (m, 6H), 1.97 - 1.80 (m, 7H), 1.75 - 1.65 (m, 2H), 0.80 (s, 4H).

### Example 132

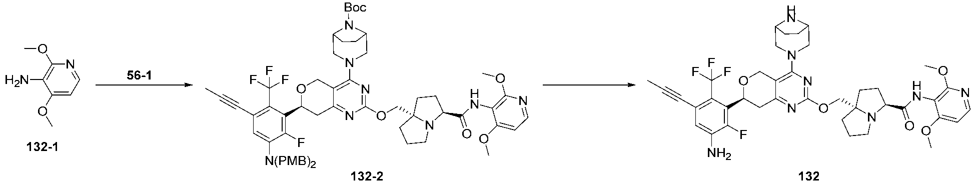

Referring to steps 2 to 3 of Example **56,** compound **132-1** was used as a starting material in place of (S)-3,3-difluorocyclopentylamine hydrochloride to obtain crude compound **132.** The crude product was separated by preparative HPLC (Chromatographic column: Waters Xbridge, 250*19 mm, 5 µm; mobile phase: [water (0.1% NH₃H₂O)-acetonitrile]; gradient: (acetonitrile): 10%-40%) and dried to yield compound **132.** MS m/z = 781.5 [M+H]⁺. ¹H NMR (400 MHz, CD₃OD) δ ppm 7.99 (d, *J =* 4.0 Hz, 1H), 6.90 (d, *J* = 8.0 Hz, 1H), 6.79 (d, *J =* 8.0 Hz, 1H), 5.16 (dd, *J =* 8.0, 4.0 Hz, 1H), 4.81 - 4.79 (m, 1H), 4.67 (d, *J =* 16.0 Hz, 1H), 4.22 (d, *J = 8.0* Hz, 1H), 4.11 (d, *J =* 8.0 Hz, 1H), 4.05 - 4.01 (m, 1H), 3.90 (d, *J =* 12.0 Hz, 6H), 3.69 - 3.64 (m, 2H), 3.58 (d, *J =* 12.0 Hz, 1H), 3.47 (d, *J =* 12.0 Hz, 1H), 3.28 - 3.16 (m, 2H), 3.15 - 3.06 (m, 2H), 2.98 - 2.92 (m, 1H), 2.87 (dd, *J =* 16.0, 4.0 Hz, 1H), 2.24 - 2.16 (m, 2H), 2.14 - 2.04 (m, 4H), 2.01 (s, 3H), 1.93 - 1.86 (m, 3H), 1.82 - 1.74 (m, 3H).

### Example 133

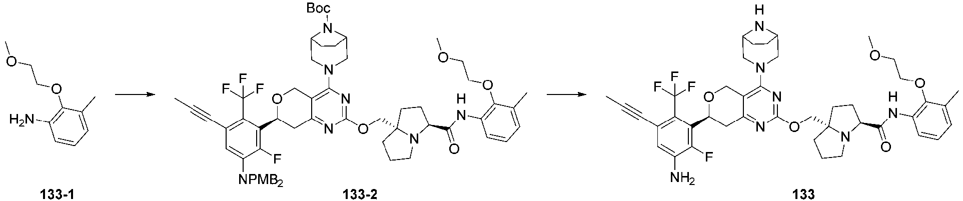

Referring to steps 2 to 3 of Example **56,** compound **133-1** was used as a starting material in place of (S)-3,3-difluorocyclopentylamine hydrochloride to obtain crude compound **133.** The crude product was separated by preparative HPLC (Chromatographic column: Waters Xbridge, 250 x 19 mm, 5 µm; mobile phase: [water (0.1% ammonia)-acetonitrile]; gradient: (acetonitrile): 45%-70%) and dried to yield compound **133.** MS m/z = 808.6 [M +H]⁺. ¹H NMR (400 MHz, CD₃OD) δ ppm 7.98 (dd, *J =* 8.0, 4.0 Hz, 1H), 7.02 - 6.89 (m, 3H), 5.15 (dd, *J =* 12.0, 4.0 Hz, 1H), 4.81 - 4.78 (m, 1H), 4.66 (d, *J =* 12.0 Hz, 1H), 4.20 - 4.10 (m, 3H), 4.09 - 4.04 (m, 1H), 4.00 - 3.92 (m, 2H), 3.71 - 3.68 (m, 2H), 3.60 - 3.54 (m, 3H), 3.48 (s, 3H), 3.44 (d, *J =* 12.0 Hz, 1H), 3.26 - 3.18 (m, 1H), 3.08 - 2.98 (m, 2H), 2.88 - 2.82 (m, 2H), 2.30 (s, 3H), 2.24 - 2.10 (m, 4H), 2.04 - 1.99 (m, 6H), 1.94 - 1.85 (m, 3H), 1.82 - 1.70 (m, 2H).

### Example 134

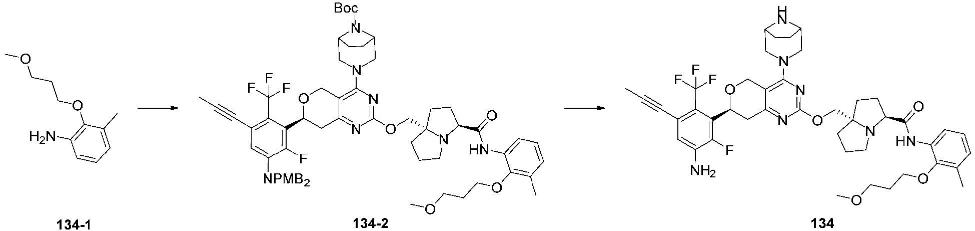

Referring steps 2 to 3 of Example **56,** 2-(3-methoxypropoxy)-3-methylaniline was used as a starting material in place of (S)-3,3-difluorocyclopentylamine hydrochloride to obtain crude compound **134.** The crude product was separated by preparative HPLC (Chromatographic column: Waters Xbridge, 250*19 mm, 5 µm; mobile phase: [water (0.1% ammonia)-acetonitrile]; gradient: (acetonitrile): 45%-70%) and dried to afford compound **134.** MS m/z = 822.6 [M+H]⁺. ¹H NMR (400 MHz, CD₃OD) δ ppm 7.81 (dd, *J =* 8.0, 4.0 Hz, 1H), 7.01 - 6.97 (m, 2H), 6.90 (d, *J =* 8.0 Hz, 1H), 5.16 (dd, *J =* 12.0, 4.0 Hz, 1H), 4.81 - 4.78 (m, 1H), 4.66 (d, *J =* 12.0 Hz, 1H), 4.24 (d, *J =* 8.0 Hz, 1H), 4.16 - 4.09 (m, 2H), 4.04 (dd, *J =* 8.0, 4.0 Hz, 1H), 3.90 - 3.87 (m, 2H), 3.67 - 3.52 (m, 5H), 3.44 (d, *J* = 12.0 Hz, 1H), 3.34 (s, 3H), 3.24 - 3.19 (m, 1H), 3.08 - 2.99 (m, 2H), 2.89 - 2.79 (m, 2H), 2.29 (s, 3H), 2.24 - 2.08 (m, 4H), 2.06 - 2.02 (m, 6H), 1.99 - 1.91 (m, 3H), 1.85 - 1.74 (m, 4H).

### Example 136

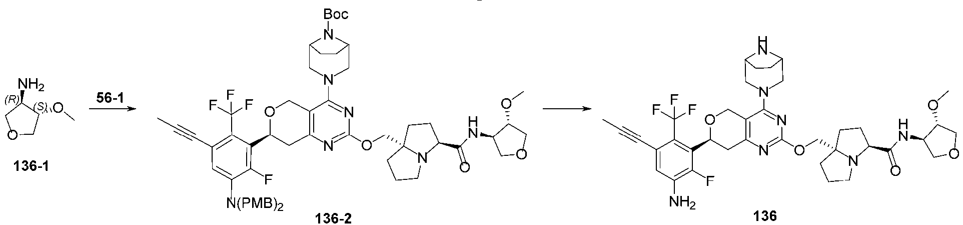

Referring steps 2 to 3 of Example **56, 136-1** was used as a starting material in place of (S)-3,3-difluorocyclopentylamine hydrochloride to obtain crude compound **136.** The crude product was separated by preparative HPLC (Chromatographic column: Waters Xbridge, 250*30mm, 5 µm; mobile phase: [water (0.1% ammonia)-acetonitrile]; gradient: (acetonitrile): 50%-80%) and dried to yield compound **136.** MS m/z = 744.5 [M+H]⁺. ¹H NMR (400 MHz, CD₃OD) δ ppm 6.90 (d, *J =* 8.0 Hz, 1H), 5.15 (dd, *J =* 12.0, 4.0 Hz, 1H), 4.80 (d, *J* = 16.0 Hz, 1H), 4.65 (d, *J =* 16.0 Hz, 1H), 4.32 - 4.30 (m, 1H), 4.15 (t, *J =* 12.0 Hz, 2H), 4.05 (d, *J =* 8.0 Hz, 1H), 4.00 - 3.95 (m, 2H), 3.84 - 3.82 (m, 1H), 3.78 - 3.70 (m, 2H), 3.66 (dd, *J* = 8.0, 4.0 Hz, 1H), 3.56 - 3.52 (m, 3H), 3.44 - 3.42 (m, 4H), 3.25 - 3.18 (m, 1H), 3.05 (d, *J =* 12.0 Hz, 1H), 2.91 - 2.82 (m, 2H), 2.79 - 2.73 (m, 1H), 2.15 - 2.07 (m, 2H), 2.05 - 1.96 (m, 5H), 1.95 - 1.80 (m, 6H), 1.77 - 1.66 (m, 2H).

### Example 137

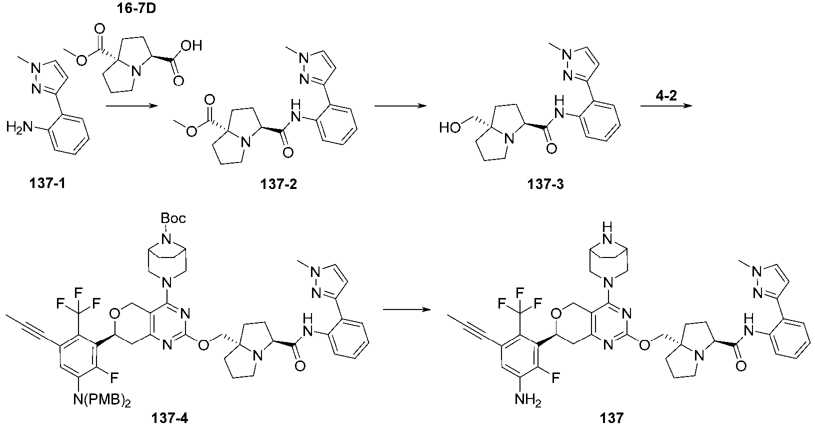

Referring to steps 7 to 10 of Example **16,** compound **137-1** was used as a starting material in place of N-ethyl-4-methoxybenzylamine to obtain crude compound **137.** The crude product was separated by preparative HPLC (Chromatographic column: Waters Xbridge, 250*30mm, 5 µm; mobile phase: [water (0.1% ammonia)-acetonitrile]; gradient: (acetonitrile): 50%-80%) and dried to yield compound **137.** MS m/z = 800.6 [M+H]⁺. ¹H NMR (400 MHz, CD₃OD) δ ppm 8.52 (d, *J* = 8.0 Hz, 1H), 7.62 (dd, *J* = 8.0, 4.0 Hz, 1H), 7.56 (d, *J* = 4.0 Hz, 1H), 7.29 (t, *J =* 8.0 Hz, 1H), 7.14 (t, *J =* 4.0 Hz, 1H), 6.90 (d, *J =* 8.0 Hz, 1H), 6.54 (d, *J =* 4.0 Hz, 1H), 5.08 (dd, *J =* 12.0, 4.0 Hz, 1H), 4.71 (d, *J* = 12.0 Hz, 1H), 4.58 (d, *J* = 12.0 Hz, 1H), 4.18 (d, *J* = 8.0 Hz, 1H), 4.09 (d, *J =* 12.0 Hz, 1H), 3.99 (s, 3H), 3.93 (d, *J =* 16.0 Hz, 1H), 3.51 (t, *J =* 4.0 Hz, 1H), 3.46 - 3.35 (m, 4H), 3.22 (d, *J* = 16.0 Hz, 1H), 3.11 (dd, *J =* 20.0, 12.0 Hz, 1H), 2.85 (d, *J =* 12.0 Hz, 1H), 2.81 - 2.71 (m, 2H), 2.37 - 2.30 (m, 1H), 2.25 - 2.18 (m, 1H), 2.06 - 1.97 (m, 7H), 1.88 - 1.70 (m, 5H), 1.61 - 1.55 (m, 1H).

### Example 138

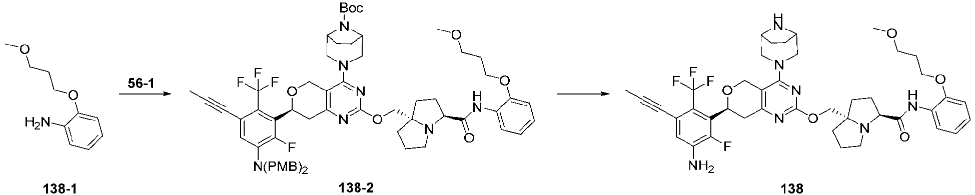

Referring to steps 2 to 3 of Example **56,** compound **138-1** was used as a starting material in place of (S)-3,3-difluorocyclopentylamine hydrochloride to obtain crude compound **138.** The crude product was separated by preparative HPLC (column: xbridge-C18, 250 x 19 mm, 5 µm; mobile phase: [water (0.1% ammonia)-acetonitrile]; gradient: (acetonitrile): 33% - 62%) and dried to yield compound **138.** MS m/z = 808.6 [M+H]⁺. ¹H NMR (400 MHz, CD₃OD) δ ppm 7.92 (dd, *J =* 8.0, 4.0 Hz, 1H), 7.13 - 7.08 (m, 1H), 7.02 (d, *J =* 8.0 Hz, 1H), 6.94 - 6.89 (m, 2H), 5.15 (dd, *J* = 12.0, 4.0 Hz, 1H), 4.79 (s, 1H), 4.66 (d, *J* = 12.0 Hz, 1H), 4.25 (d, *J =* 8.0 Hz, 1H), 4.17 - 4.10 (m, 4H), 4.05 (dd, *J =* 8.0, 4.0 Hz, 1H), 3.66 - 3.53 (m, 5H), 3.45 (d, *J* = 12.0 Hz, 1H), 3.33 (s, 3H), 3.23 (dd, *J =* 16.0, 12.0 Hz, 1H), 3.08 - 3.05 (m, 2H), 2.89 - 2.78 (m, 2H), 2.24 - 2.13 (m, 2H), 2.10 - 1.86 (m, 13H), 1.80 - 1.70 (m, 2H).

### Example 139

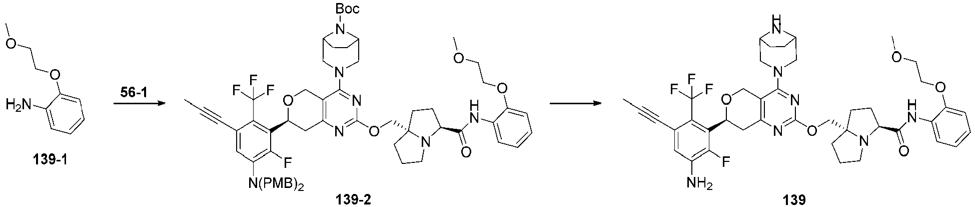

Referring to steps 2 to 3 of Example **56,** compound **139-1** was used as a starting material in place of (S)-3,3-difluorocyclopentylamine hydrochloride to obtain crude compound **139.** The crude product was separated by preparative HPLC (Chromatographic column: xbridge-C18, 30 x 250 mm, 5 µm; mobile phase: [water (0.1% ammonia)-acetonitrile]; gradient: (acetonitrile): 50%-85%) and dried to yield compound **139.** MS m/z = 794.6 [M+H]⁺. ¹H NMR (400 MHz, CD₃OD) δ ppm 7.97 (dd, *J =* 8.0, 1.6 Hz, 1H), 7.12 - 7.08 (m, 1H), 7.05 -7.02 (m, 1H), 6.97 - 6.93 (m, 1H), 6.91 - 6.88 (m, 1H), 5.15 (dd, *J* = 11.6, 4.8 Hz, 1H), 4.78 (s, 1H), 4.65 (d, *J* = 13.6 Hz, 1H), 4.23 - 4.16 (m, 3H), 4.14 - 4.09 (m, 2H), 4.04 - 4.00 (m, 1H), 3.77 - 3.74 (m, 2H), 3.55 - 3.52 (m, 3H), 3.45 - 3.43 (m, 1H), 3.42 (s, 3H), 3.25 - 3.18 (m, 1H), 3.10 - 3.02 (m, 2H), 2.88 - 2.77 (m, 2H), 2.23 - 2.08 (m, 4H), 2.01 (s, 3H), 1.99 - 1.93 (m, 4H), 1.85 - 1.80 (m, 2H), 1.78 - 1.69 (m, 2H).

### Example 140

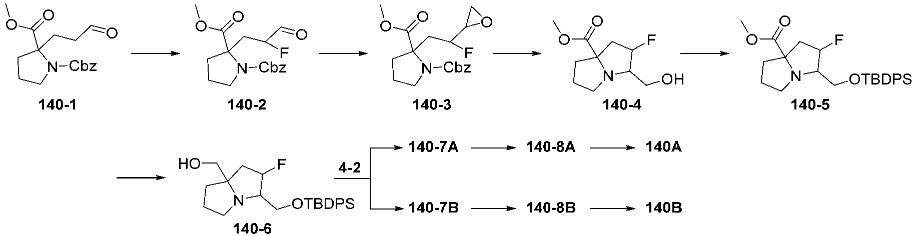

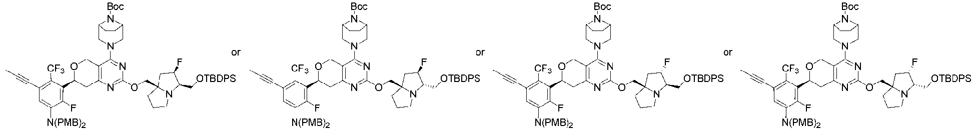

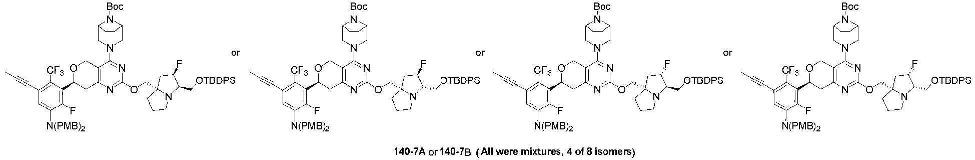

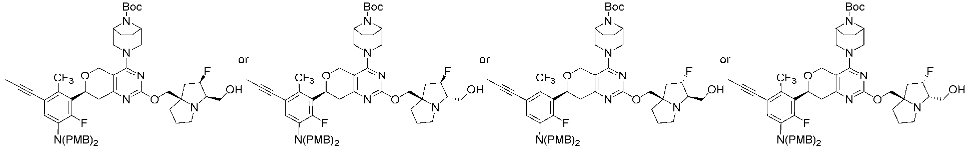

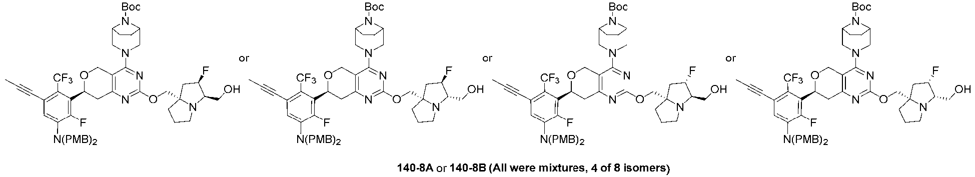

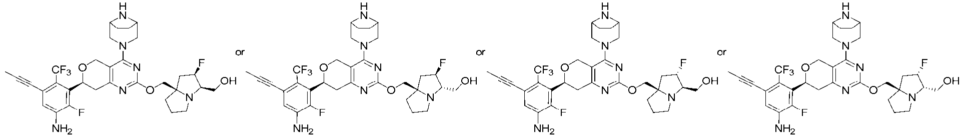

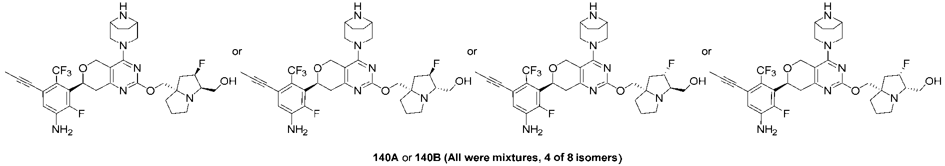

### Step 1

C ompound **140-1** (1.1 g, 3.44 mmol) was dissolved in dichloromethane (15 mL). Triethylamine (1.05 g, 10.33 mmol) and trimethylsilyl trifluoromethanesulfonate (1.53 g, 6.89 mmol) were added at 0°C, and the mixture was stirred at 25°C for 3 hours. Water (10 mL) was added to quench the reaction, and the mixture was extracted with petroleum ether (20 mL x 3). The extracted organic phases were combined and washed with saturated brine (15 mL), dried over anhydrous sodium sulfate, filtered, and concentrated under reduced pressure. The residue was dissolved in acetonitrile (20 mL), and 1-chloromethyl-4-fluoro-1,4-diazabicyclo[2.2.2]octane ditetrafluoroborate (1.83 g, 5.17 mmol) was added. The mixture was stirred at 25°C for 12 hours. Water (10 mL) was added to quench the reaction, and the product was extracted with ethyl acetate (20 mL * 3). The extracted organic phases were combined and washed with saturated brine (15 mL), dried over anhydrous sodium sulfate, and filtered. The filtrate was concentrated under reduced pressure, and separated by column chromatography (petroleum ether: ethyl acetate = 1:1) to obtain compound **140-2.** MS m/z = 338.3 [M+H]⁺.

### Step 2

Sodium hydride (70.43 mg, 2.93 mmol, 60%) was dissolved in anhydrous dimethyl sulfoxide (8 mL) at 0°C, and the mixture was heated to 55°C, and stirred for 2 hours. The temperature was then lowered to 0°C, and trimethylsulfoxide iodide (587.13 mg, 2.67 mmol) was added. The mixture was then warmed to 25°C and stirred for 15 minutes. Compound **140-2** (450 mg, 1.33 mmol) was added, and the mixture was reacted at 25°C with stirring for 2 hours. The reaction was quenched by the addition of water (10 mL), and extracted with ethyl acetate (20 mL x 3). The extracted organic phases were combined and washed with saturated brine (15 mL), dried over anhydrous sodium sulfate, filtered, and concentrated under reduced pressure to yield compound **140-3.** MS m/z = 352.2 [M+H]⁺.

### Step 3

Compound **140-3** (400 mg, 1.14 mmol) was dissolved in methanol (5 mL), and palladium on carbon (121.15 mg, 1.14 mmol, 10%) was added. The mixture was stirred at room temperature under a hydrogen atmosphere (15 psi) for 12 hours. The mixture was filtered, and the filtrate was concentrated under reduced pressure to obtain compound **140-4.** MS m/z = 218.2 [M+H]⁺.

### Step 4

Compound **140-4** (210 mg, 966.69 µmol) was dissolved in dichloromethane (4 mL). Imidazole (65.81 mg, 966.69 µmol) and tert-butyldiphenylsilyl chloride (265.70 mg, 966.69 µmol) were added at 0°C. The mixture was stirred at 25°C for 3 hours. The reaction solution was concentrated and purified using a silica gel preparative plate (petroleum ether:ethyl acetate = 10:1) to obtain compound **140-5.** MS m/z = 456.3 [M+H]⁺.

### Step 5

Compound **140-5** (200 mg, 438.95 µmol) was dissolved in anhydrous tetrahydrofuran (4 mL). Lithium aluminum hydride solution (658.4 µL, 658.42 µmol, 1 M solution in tetrahydrofuran) was added dropwise at 0°C. The mixture was heated to 25°C and stirred for 0.5 hours. The reaction was quenched by the addition of water (2 mL). The product was extracted with dichloromethane (10 mL x 3). The extracted organic phases were combined and washed with saturated brine (10 mL), dried over anhydrous sodium sulfate, and filtered. The filtrate was concentrated under reduced pressure, and purified on a silica gel preparative plate (petroleum ether:ethyl acetate = 2:1) to obtain compound **140-6.** MS m/z = 428.3 [M+H]⁺.

### Step 6

Compound **140-6** (61.73 mg, 70.15 µmol) was dissolved in anhydrous N,N-dimethylformamide (2 mL). Sodium hydride (2.53 mg, 105.23 µmol, 60%) was added at 0°C, and the mixture was heated to 25°C with stirring for 45 minutes. Compound **4-2** (30 mg, 70.15 µmol) was added, and the reaction was continued with stirring at 25°C for 1 hour. The reaction was quenched by the addition of water (2 mL), and the mixture was extracted with dichloromethane (10 mL x 3). The organic phases were combined, dried over anhydrous sodium sulfate, filtered, and concentrated under reduced pressure. The mixture was then separated and purified on a silica gel preparative plate (petroleum ether:ethyl acetate = 3:1) to afford compounds **140-7A** (mixture) and **140-7B** (mixture). Compound **140-7A** (mixture) (developing solvent: petroleum ether:ethyl acetate = 3:1, Rf = 0.6), MS m/z = 1227.9 [M+H]⁺; compound **140-7B** (mixture) (developing solvent: petroleum ether:ethyl acetate = 3:1, Rf = 0.4), MS m/z = 1227.9 [M+H]⁺.

### Step 7

Compound **140-7A** (23 mg, 18.74 µmol) was dissolved in tetrahydrofuran (1 mL). Tetrabutylammonium fluoride solution (0.1 mL, 100 µmol, 1 M in tetrahydrofuran) was added and stirred at 25°C for 1 hour. The reaction was quenched with water (2 mL) and extracted with dichloromethane (10 mL x 3). The combined organic phases were dried over anhydrous sodium sulfate, filtered, and concentrated under reduced pressure to afford compound **140-8A** (a mixture). MS m/z = 989.7 [M+H]⁺.

Referring to step 7, compound **140-7B** was used as a starting material in place of compound **140-7A** to obtain compound **140-8B** (a mixture). MS m/z = 989.7 [M+H]⁺.

### Step 8

Compound **140-8A** (17 mg, 17.19 µmol) was dissolved in dichloromethane (1.2 mL), and trifluoroacetic acid (0.3 mL) was added. The mixture was stirred at 25°C for 1 hour. The reaction solution was concentrated under reduced pressure to obtain a crude product. The crude product was separated by preparative HPLC (column: Sunfire-C18, 19 x 250 mm, 5 µm; mobile phase: [water (0.1% formic acid)-acetonitrile]; gradient: (acetonitrile): 12%-30%), and dried to obtain the formate salt of compound **140A** (mixture). MS m/z = 649.4 [M+H]⁺. ¹H NMR (400 MHz, CD₃OD) δ ppm 8.47 (s, 1.69H), 6.91 (d, *J =* 8.0 Hz, 1H), 5.31 (dt, *J =* 52.0, 4.0 Hz, 1H), 5.17 (dd, *J =* 12.0, 4.0 Hz, 1H), 4.81 - 4.80 (m, 1H), 4.70 (d, *J* = 12.0 Hz, 1H), 4.30 - 4.17 (m, 3H), 4.06 (d, *J* = 4.0 Hz, 2H), 3.85 - 3.80 (m, 1H), 3.75 (d, *J =* 12.0 Hz, 1H), 3.69 - 3.60 (m, 2H), 3.30 - 3.23 (m, 2H), 3.15 - 3.04 (m, 2H), 2.97 - 2.86 (m, 2H), 2.56 - 2.46 (m, 1H), 2.29 - 2.22 (m, 1H), 2.10 - 2.04 (m, 3H), 2.03 - 1,99 (m, 4H), 1.97 - 1.90 (m, 3H), 1.86 - 1.81 (m, 1H).

Compound **140-8B** (18 mg, 18.20 µmol) was dissolved in dichloromethane (1.2 mL), and trifluoroacetic acid (0.3 mL) was added. The mixture was stirred at 25°C for 1 hour. The reaction solution was concentrated under reduced pressure to obtain a crude product. The crude product was separated by preparative HPLC (column: sunfire-C18, 19 x 250 mm, 5 µm; mobile phase: [water (0.1% formic acid)-acetonitrile]; gradient: (acetonitrile): 12%-30%), and dried to obtain the formate salt of compound **140B** (mixture). MS m/z = 649.4 [M+H]⁺. ¹H NMR (400 MHz, CD₃OD) δ ppm 8.48 (s, 1.10H), 6.91 (d, *J =* 8.0 Hz, 1H), 5.18 (dd, *J* = 12.0, 4.0 Hz, 1H), 4.81 - 4.80 (m, 1H), 4.73 - 4.69 (m, 1H), 4.63 - 4.48 (m, 1H), 4.46 - 4.31 (m, 1H), 4.30 - 4.18 (m, 2H), 4.08 - 4.02 (m, 2H), 3.95 - 3.82 (m, 1H), 3.74 (d, *J* = 12.0 Hz, 1H), 3.63 - 3.57 (m, 1H), 3.28 - 3.22 (m, 2H), 3.15 - 3.09 (m, 3H), 2.94 - 2.81 (m, 2H), 2.30 - 2.21 (m, 2H), 2.12 - 1.84 (m, 10H), 1.72 - 1.64 (m, 1H).

### Example 141

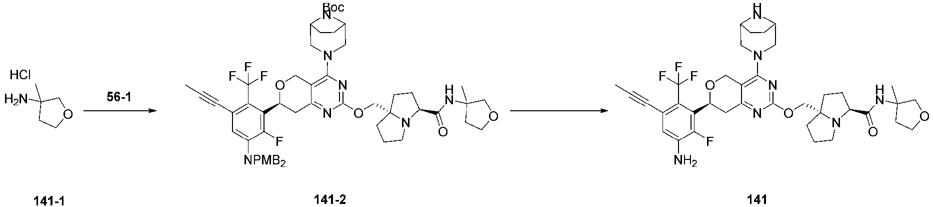

### Step 1

Compound **56-1** (90 mg, 101.15 µmol) was weighed and N,N-dimethylformamide (2.5 mL) was added. N,N,N,N-tetramethyl-O-(7-azabenzotriazol-1-yl)uronium hexafluorophosphate (69.44 mg, 182.74 µmol), diisopropylethylamine (70.72 mg, 548.22 µmol), and compound **141-1** (30.80 mg, 304.55 µmol) were also added. The mixture was stirred at room temperature for 1 hour. Column chromatography (dichloromethane:methanol = 20:1) afforded compound **141-2.** MS m/z = 1068.7 [M+H]⁺.

### Step 2

Compound **141-2** (80 mg, 74.89 µmol) was weighed. Trifluoroacetic acid (2 mL) was added, and the mixture was stirred at room temperature for 0.5 h. The reaction solution was concentrated under reduced pressure to obtain a crude product, which was then separated by preparative high performance liquid chromatography (Chromatographic column: Waters Xbridge, 250*19 mm, 5 µm; mobile phase: [water (0.1% ammonia water)-acetonitrile]; gradient: (acetonitrile): 5%-50%) to obtain compound 141. MS m/z = 728.5 [M+H]⁺. ¹H NMR (400 MHz, CD₃OD) δ ppm 6.90 (d, *J =* 8.0 Hz, 1H), 5.17 - 5.13 (m, 1H), 4.83 - 4.78 (m, 1H), 4.65 (d, *J* = 16.0 Hz, 1H), 4.18 - 4.12 (m, 2H), 4.06 - 3.96 (m, 2H), 3.93 - 3.88 (m, 2H), 3.75 - 3.72 (m, 1H), 3.66 - 3.62 (m, 1H), 3.54 - 3.51 (m, 3H), 3.43 - 3.40 (m, 1H), 3.25 - 3.18 (m, 1H), 3.04 (d, *J =* 16.0 Hz, 1H), 2.94 - 2.91 (m, 1H), 2.87 - 2.82 (m, 1H), 2.77 - 2.72 (m, 1H), 2.32 - 2.26 (m, 1H), 2.13 - 2.07 (m, 2H), 2.04 - 2.00 (m, 4H), 1.95 - 1.88 (m, 6H), 1.85 - 1.83 (m, 2H), 1.75 - 1.69 (m, 2H), 1.48 (s, 3H).

### Example 142

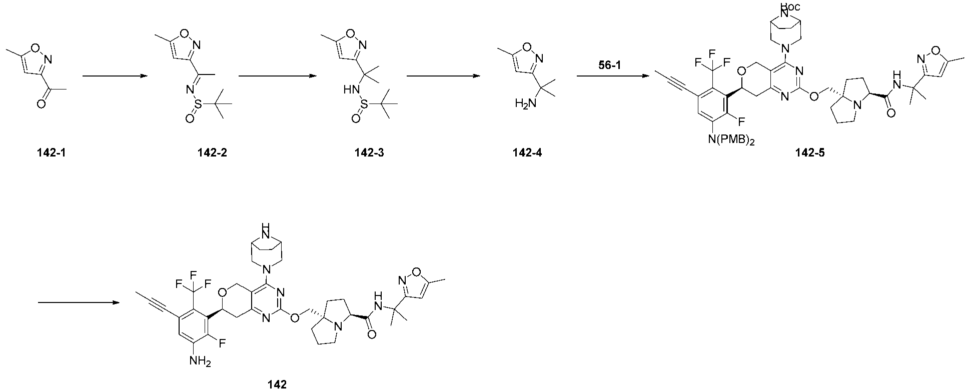

### Step 1

Compound **142-1** (500 mg, 4.0 mmol) was weighed and tetrahydrofuran (5 mL), tetraisopropyl titanate (2.27 g, 8.0 mmol), and tert-butylsulfenamide (581 mg, 4.8 mmol) were added. The mixture was stirred at 70°C for 4 hours. Water (1 mL) was added to quench the reaction, and the mixture was filtered. The filtrate was concentrated under reduced pressure and separated by column chromatography (petroleum ether:ethyl acetate = 2:1) to obtain compound **142-2.** MS m/z = 229.1 [M+H]⁺.

### Step 2

Compound **142-2** (400 mg, 1.75 mmol) was weighed and anhydrous tetrahydrofuran (5 mL) was added under nitrogen. The mixture was cooled to -78°C, and methylmagnesium bromide (1.75 mL, 5.26 mmol, 3.0 M in THF) was added. The reaction mixture was slowly returned to room temperature and allowed to react for 2 hours. Water (0.2 mL) was added to quench the reaction, and the mixture was extracted with ethyl acetate (50 mL*3). The mixture was dried over anhydrous sodium sulfate, and the organic phase was concentrated under reduced pressure. The mixture was separated by column chromatography (petroleum ether:ethyl acetate = 1:1) to afford compound **142-3.** MS m/z = 245.2 [M+H]⁺.

### Step 3

Compound **142-3** (100 mg, 0.49 mmol) was weighed and dichloromethane (2 mL) was added. Ethyl acetate hydrochloride (1 mL, 4 mmol, 4.0 M) was added and the mixture was stirred at room temperature for 1 hour. The reaction mixture was concentrated under reduced pressure to obtain the hydrochloride salt of compound **142-4.** MS m/z = 141.2 [M+H]⁺.

### Step 4

Compound **56-1** (50 mg, 50.76 µmol) was weighed and acetonitrile (2 mL), N-methylimidazole (12.5 mg, 152.28 µmol), N,N,N',N'-tetramethylchloroformamidine hexafluorophosphate (21.4 mg, 76.14 µmol), and **142-4** (14.2 mg, hydrochloride) were added. The mixture was stirred at room temperature for 2 hours. Column chromatography (petroleum ether:ethyl acetate = 1:5) afforded compound **142-5.** MS m/z = 1107.8 [M+H]⁺.

### Step 5

Compound **142-5** (30 mg, 27.09 µmol) was weighed and dichloromethane (2 mL) and trifluoroacetic acid (1 mL) were added. The mixture was stirred at room temperature for 1 hour. The reaction solution was concentrated under reduced pressure to obtain a crude product, which was then separated by preparative HPLC (column: Waters Xbridge, 250*19 mm, 5 µm; mobile phase: [water (0.1% ammonia)-acetonitrile]; gradient: (acetonitrile): 30%-70%) to obtain compound **142.** MS m/z = 767.6 [M+H]⁺. ¹H NMR (400 MHz, CD₃OD) δ ppm 6.90 (d, *J =* 8.0 Hz, 1H), 6.09 (s, 1H), 5.25 - 5.12 (m, 1H), 4.83 - 4.76 (m, 1H), 4.65 (d, *J* = 12.0 Hz, 1H), 4.19 - 4.13 (m, 2H), 4.05 (d, *J =* 12.0 Hz, 1H), 3.84 - 3.76 (m, 1H), 3.70 - 3.65 (m, 1H), 3.61 - 3.50 (m, 3H), 3.48 - 3.40 (m, 1H), 3.28 - 3.17 (m, 1H), 3.05 (d, *J =* 12.0 Hz, 1H), 2.99 - 2.66 (m, 3H), 2.37 (s, 3H), 2.15 - 1.79 (m, 12H), 1.78 - 1.68 (m, 2H), 1.66 (s, 3H), 1.62 (s, 3H).

### Example 143

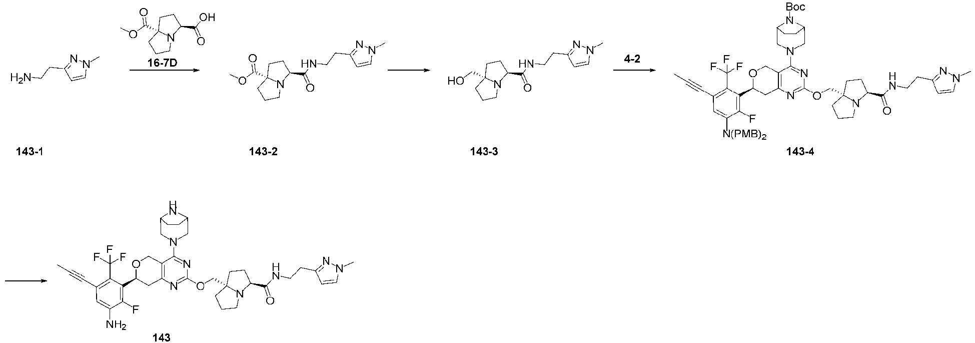

Referring to steps 7 to 10 of Example **16,** compound **143-1** was used as a starting material in place of N-ethyl-4-methoxybenzylamine to obtain crude compound **143.** The crude product was separated by preparative HPLC (Chromatographic column: Waters Xbridge, 250 x 19 mm, 5 µm; mobile phase: [water (0.1% ammonia)-acetonitrile]; gradient: (acetonitrile): 45%-70%) and dried to yield compound **143.** MS m/z = 752.6 [M+H]⁺. ¹H NMR (400 MHz, CD₃OD) δ ppm 7.46 (d, *J =* 2.0 Hz, 1H), 6.91 (d, *J =* 8.0 Hz, 1H), 6.14 (d, *J =* 2.0 Hz, 1H), 5.18 - 5.14 (m, 1H), 4.80 - 4.77 (m, 1H), 4.68 (d, *J* = 12.0 Hz, 1H), 4.23 - 4.07 (m, 4H), 3.81 (s, 3H), 3.63 (d, *J* = 12.0 Hz, 1H), 3.56 - 3.44 (m, 4H), 3.28 - 3.11 (m, 2H), 3.01 - 2.72 (m, 6H), 2.22 - 2.05 (m, 5H), 2.02 (s, 3H), 1.99 - 1.90 (m, 4H), 1.88 - 1.70 (m, 3H).

### Example 144

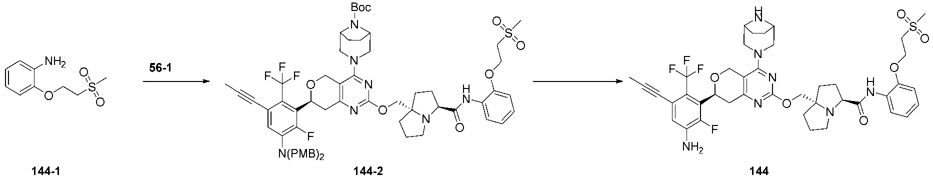

Referring to steps 2 to 3 of Example **56,** compound **144-1** was used as a starting material in place of (S)-3,3-difluorocyclopentylamine hydrochloride to obtain crude compound **144.** The crude product was separated by preparative HPLC (column: Waters Xbridge, 250*19 mm, 5 µm; mobile phase: [water (0.1% ammonia)-acetonitrile]; gradient: (acetonitrile): 45%-70%) and dried to yield compound **144.** MS m/z = 842.5 [M+H]⁺. ¹H NMR (400 MHz, CD₃OD) δ ppm 8.06 (d, *J=* 8.0 Hz, 1H), 7.16 - 7.09 (m, 2H), 7.02 - 6.98 (m, 1H), 6.90 (d, *J =* 8.0 Hz, 1H), 5.18 - 5.14 (m, 1H), 4.81 - 4.78 (m, 1H), 4.68 - 4.64 (m, 1H), 4.54 - 4.50 (m, 2H), 4.22 - 4.08 (m, 3H), 4.02 - 3.98 (m, 1H), 3.66 - 3.63 (m, 2H), 3.62 - 3.60 (m, 1H), 3.56 - 3.53 (m, 2H), 3.45 - 3.42 (m, 2H), 3.27 - 3.19 (m, 1H), 3.08 (s, 3H), 3.00 - 2.96 (m, 1H), 2.89 - 2.77 (m, 2H), 2.25 - 2.05 (m, 4H), 2.04 - 1.99 (m, 4H), 1.98 - 1.84 (m, 5H), 1.80 - 1.68 (m, 2H).

### Example 145

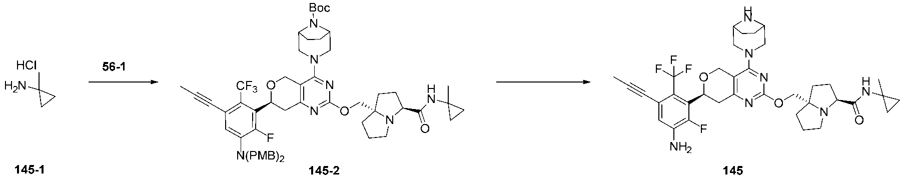

Referring to steps 2 to 3 of Example **56,** compound **145-1** was used as a starting material in place of (S)-3,3-difluorocyclopentylamine hydrochloride to obtain crude compound **145.** The crude product was separated by preparative HPLC (column: Waters Xbridge, 250*19 mm, 5 µm; mobile phase: [water (0.1% ammonia)-acetonitrile]; gradient: (acetonitrile): 45%-70%) and dried to yield compound **145.** MS m/z = 698.6 [M+H]⁺. ¹H NMR (400 MHz, CD₃OD) δ ppm 6.90 *(d, J=* 8.0 Hz, 1H), 5.16 - 5.13 (m, 1H), 4.81 - 4.78 (m, 1H), 4.65 (d, *J* = 16.0 Hz, 1H), 4.17 - 4.12 (m, 2H), 4.04 (d, *J* = 8.0 Hz, 1H), 3.65 - 3.63 (m, 1H), 3.55 - 3.51 (m, 3H), 3.44 - 3.41 (m, 1H), 3.22 - 3.18 (m, 1H), 3.05 (d, *J =* 12.0 Hz, 1H), 2.87 - 2.86 (m, 2H), 2.77 - 2.71 (m, 1H), 2.12 - 2.05 (m, 2H), 2.01 (s, 3H), 1.97 - 1.89 (m, 5H), 1.86 - 1.82 (m, 3H), 1.74 - 1.67 (m, 2H), 1.35 (s, 3H), 0.73 - 0.69 (m, 2H), 0.66 - 0.60 (m, 2H).

### Example 146

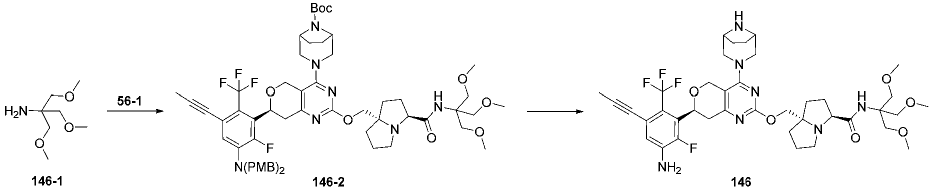

Referring to steps 2 to 3 of Example **56,** compound **146-1** was used as a starting material in place of (*S*)-3,3-difluorocyclopentylamine hydrochloride to obtain crude compound **146.** The crude product was separated by preparative HPLC (Chromatographic column: xbridge-C18, 30 x 250 mm, 5 µm; mobile phase: [water (0.1% ammonium bicarbonate)-acetonitrile]; gradient: (acetonitrile): 35%-75%) and dried to yield compound **146.** MS m/z = 790.6 [M+H]⁺. ¹H NMR (400 MHz, DMSO-*d₆*) δ ppm 7.17 (s, 1H), 6.88 (d, *J* = 8.0 Hz, 1H), 6.01 (s, 2H), 5.02 (dd, *J =* 12.0 Hz, 4.0 Hz, 1H), 4.75 (d, *J =* 12.0 Hz, 1H), 4.55 (d, *J =* 16.0 Hz, 1H), 3.94 - 3.89 (m, 2H), 3.83 (d, *J =* 8.0 Hz, 1H), 3.61 (t, *J =* 4.0 Hz, 1H), 3.54 (s, 6H), 3.44 (s, 2H), 3.36 - 3.32 (m, 2H), 3.27 (s, 1H), 3.23 (s, 9H), 3.07 - 3.00 (m, 1H), 2.88 (d, *J= 12.0* Hz, 1H), 2.82 - 2.71 (m, 3H), 2.03 (s, 3H), 1.88 - 1.81 (m, 6H), 1.77-1.72 (m, 3H), 1.62 - 1.55 (m, 3H).

### Example 147

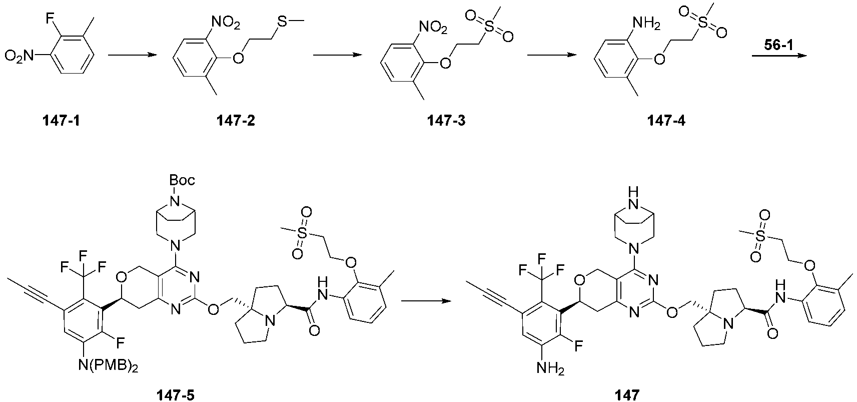

### Step 1

Compound 2-methylmercaptoethanol (500 mg, 5.43 mmol) was dissolved in N,N-dimethylformamide (10 mL). Potassium carbonate (2.25 g, 16.28 mmol) and compound **147-1** (841.62 mg, 5.43 mmol) were added sequentially at room temperature. The mixture was heated to 50°C and stirred for 12 hours. The reaction was quenched by the addition of water (10 mL). The mixture was extracted with ethyl acetate (20 mL x 3). The combined organic phases were dried over anhydrous sodium sulfate, and filtered. The filtrate was concentrated under reduced pressure, and separated by column chromatography (petroleum ether:ethyl acetate = 4:1) to afford compound **147-2.** ¹H NMR (400 MHz, DMSO-*d₆*) δ ppm 7.70 (dd, *J* = 8.0, 4.0 Hz, 1H), 7.58 (d, *J =* 4.0 Hz, 1H), 7.24 (t, *J* = 8.0 Hz, 1H), 4.09 (t, *J =* 4.0 Hz, 2H), 2.84 (t, *J =* 4.0 Hz, 2H), 2.36 (s, 3H), 2.13 (s, 3H).

### Step 2

Compound **147-2** (600 mg, 2.64 mmol) was dissolved in dichloromethane (10 mL), and m-chloroperbenzoic acid (1.37 g, 7.92 mmol) was added. The mixture was stirred at room temperature for 2 hours. The reaction was quenched by the addition of saturated aqueous sodium sulfite (20 mL). The mixture was extracted with ethyl acetate (30 mL x 3). The combined organic phases were dried over anhydrous sodium sulfate, and filtered. The filtrate was concentrated under reduced pressure, and separated by column chromatography (petroleum ether:ethyl acetate = 2:1) to afford compound **147-3**. ¹H NMR (400 MHz, DMSO-*d₆*) δ ppm 7.76 (d, *J* = 8.0 Hz, 1H), 7.61 (d, *J* = 8.0 Hz, 1H), 7.29 (t, *J* = 8.0 Hz, 1H), 4.31 (t, *J* = 4.0 Hz, 2H), 3.64 (t, *J* = 4.0 Hz, 2H), 3.08 (s, 3H), 2.37 (s, 3H).

### Step 3

Compound **147-3** (300 mg, 1.16 mmol) was dissolved in methanol (10 mL), and 10% palladium on carbon (12.31 mg, 115.71 µmol) was added. The mixture was stirred at 25°C under a hydrogen atmosphere for 2 hours. The mixture was filtered, concentrated under reduced pressure, and separated by column chromatography (petroleum ether:ethyl acetate = 1:1) to obtain compound **147-4.** MS m/z = 230.1 [M+H]⁺.

### Step 4

Referring to steps 2 to 3 of Example **56,** compound **147-4** was used as a starting material in place of (S)-3,3-difluorocyclopentylamine hydrochloride to obtain crude compound **147.** The crude product was separated by preparative HPLC (column: xbridge-C18, 30 x 250 mm, 5 µm; mobile phase: [water (0.1% ammonia)-acetonitrile]; gradient: (acetonitrile): 50%-70%) and dried to yield compound **147.** MS m/z = 856.6 [M+H]⁺. ¹H NMR (400 MHz, CD₃OD) δ ppm 7.94 (dd, *J =* 8.0, 4.0 Hz, 1H), 7.07 - 6.99 (m, 2H), 6.90 (d, *J* = 8.0 Hz, 1H), 5.16 (dd, *J* = 8.0, 4.0 Hz, 1H), 4.79 (s, 1H), 4.67 (d, *J* = 12.0 Hz, 1H), 4.24 - 4.08 (m, 6H), 3.74 - 3.67 (m, 1H), 3.66 - 3.55 (m, 4H), 3.45 (d, *J* = 12.0 Hz, 1H), 3.27 - 3.20 (m, 1H), 3.16 (s, 3H), 3.07 (d, *J* = 12.0 Hz, 1H), 2.97 - 2.94 (m, 1H), 2.88 - 2.82 (m, 2H), 2.34 (s, 3H), 2.25 - 2.09 (m, 4H), 2.04 - 1.94 (m, 7H), 1.90 - 1.84 (m, 2H), 1.81 - 1.73 (m, 2H).

### Example 148

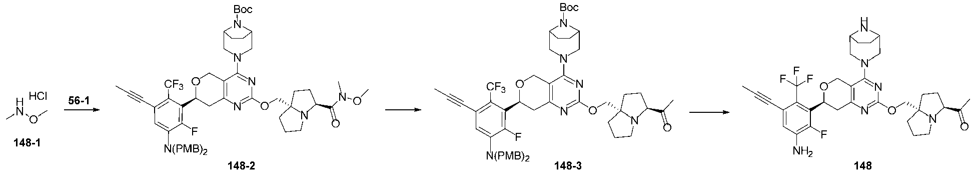

### Step 1

C ompound **56-1** (50 mg, 0.05 mmol) was dissolved in dichloromethane (4 mL). N,N-diisopropylethylamine (16 mg, 0.13 mmol), N,N,N',N'-tetramethyl-O-(7-azabenzotriazol-1-yl)uronium hexafluorophosphate (25 mg, 0.07 mmol), and compound **148-1** (6 mg, 0.06 mmol) were added sequentially. The mixture was stirred at 25°C for 6 hours. The reaction solution was concentrated under reduced pressure and separated by column chromatography (dichloromethane:methanol = 20:1) to obtain compound **148-2.** MS m/z = 1028.7 [M+H]⁺.

### Step 2

Compound **148-2** (30 mg, 28.19 µmol) was dissolved in anhydrous tetrahydrofuran (2 mL). Methylmagnesium bromide (58 mmL, 2.5 M in THF) was added at 0°C, and the mixture was stirred at 25°C for 1 hour. The reaction was quenched with saturated ammonium chloride solution (1 mL), and extracted with ethyl acetate (20 mL × 3). The extracted organic phases were combined and washed with saturated brine (10 mL × 3), dried over anhydrous sodium sulfate, and filtered. The filtrate was concentrated under reduced pressure, and separated by column chromatography (dichloromethane:methanol = 20:1) to obtain compound **148-3.** MS m/z = 983.7 [M+H]⁺.

### Step 3

Compound **148-3** (20 mg, 20.34 µmol) was dissolved in dichloromethane (1 mL). Trifluoroacetic acid (1 mL) was added, and the mixture was stirred at 25°C for 1 hour. The reaction solution was concentrated under reduced pressure to obtain a crude product. The crude product was separated by preparative HPLC (Chromatographic column: Xbridge-C18, 250*19 mm, 5 µm; mobile phase: [water (0.1% ammonia)-acetonitrile]; gradient: (acetonitrile): 30%-60%) and dried to obtain compound **148.** MS m/z = 643.5 [M+H]⁺. ¹H NMR (400 MHz, CD₃OD) δ ppm 6.90 (d, *J* = 8.0 Hz, 1H), 5.14 (dd, *J* = 12.0, 4.0 Hz, 1H), 4.77 (s, 1H), 4.65 (d, *J* = 12.0 Hz, 1H), 4.17 - 4.10 (m, 3H), 3.58 - 3.52 (m, 3H), 3.43 (d, *J* = 12.0 Hz, 2H), 3.20 (dd, *J* = 16.0, 12.0 Hz, 1H), 3.10 - 3.04 (m, 2H), 2.84 (dd, *J =* 16.0, 4.0 Hz, 1H), 2.68 - 2.62 (m, 1H), 2.17 - 2.13 (m, 5H), 2.05 - 2.01 (m, 5H), 1.96 - 1.85 (m, 5H), 1.73 - 1.69 (m, 3H).

### Example 149

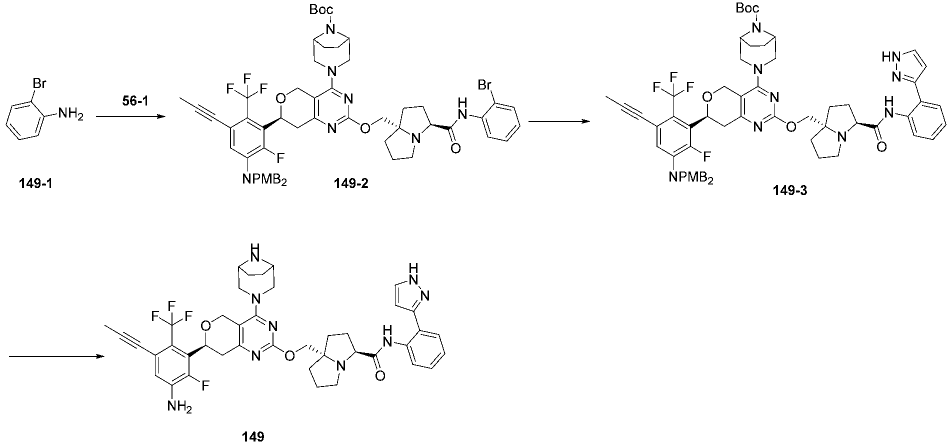

### Step 1

Compound **56-1** (60 mg, 60.91 µmol), N,N,N',N'-tetramethylchloroformamidine hexafluorophosphate (20.51 mg, 73.09 µmol), compound **149-1** (20.96 mg, 121.82 µmol), and N-methylimidazole (17.50 mg, 213.18 mmol) were weighed and anhydrous acetonitrile (2 mL) was added. Under nitrogen protection, the mixture was stirred at room temperature for 12 hours. The filtrate was concentrated under reduced pressure and separated by column chromatography (petroleum ether:ethyl acetate = 1:1) to obtain compound **149-2.** MS m/z =1138.7 [M+H]⁺.

### Step 2

Compound **149-2** (30 mg, 26.34 µmol), cesium carbonate (25.74 mg, 79.01 µmol), pyrazole-3-boronic acid (8.84 mg, 79.01 µmol), and methanesulfonic acid (2-dicyclohexylphosphino-2',4',6'-tri-isopropyl-1,1'-biphenyl)(2'-amino-1,1'-biphenyl-2-yl)palladium(II) (4.46 mg, 5.27 µmol) were weighed. 1,4-Dioxane (0.25 mL) and water (1 mL) were added, and the mixture was stirred at 60°C under nitrogen for 2 hours. The mixture was concentrated under reduced pressure and separated by column chromatography (petroleum ether:ethyl acetate = 1:1) to obtain compound **149-3.** MS m/z = 1126.6 [M+H]⁺.

### Step 3

Compound **149-3** (15 mg, 13.32 µmol) was weighed. Dichloromethane (0.5 mL) and trifluoroacetic acid (0.5 mL) were added, and the mixture was stirred at room temperature for 0.5 hours. The reaction solution was concentrated under reduced pressure to obtain a crude product, which was then separated by preparative HPLC (column: Waters Xbridge, 250*19 mm, 5 µm; mobile phase: [water (0.1% ammonia)-acetonitrile]; gradient: (acetonitrile): 30%-70%) to obtain compound **149.** MS m/z = 786.6 [M+H]⁺. ¹H NMR (400 MHz, CD₃OD) δ ppm 8.54 (d, *J* = 8.0 Hz, 1H), 7.73 - 7.59 (m, 2H), 7.30 (t, *J* = 8.0 Hz, 1H), 7.15 (t, *J =* 8.0 Hz, 1H), 6.90 (d, *J =* 8.0 Hz, 1H), 6.66 - 6.57 (m, 1H), 5.13 - 5.04 (m, 1H), 4.72 (d, *J =* 16.0 Hz, 1H), 4.60 (d, *J =* 16.0 Hz, 1H), 4.30 (d, *J* = 12.0 Hz, 1H), 4.09 (d, *J =* 8.0 Hz, 1H), 3.95 (d, *J =* 12.0 Hz, 1H), 3.48 - 3.38 (m, 3H), 3.26 - 3.17 (m, 2H), 3.15 - 3.10 (m, 1H), 2.90 - 2.84 (m, 1H), 2.81 - 2.74 (m, 1H), 2.71 - 2.65 (m, 1H), 2.42 - 2.36 (m, 1H), 2.19 - 2.09 (m, 3H), 2.01 (s, 3H), 1.97 - 1.89 (m, 2H), 1.87 - 1.80 (m, 2H), 1.76 - 1.69 (m, 3H), 1.62 - 1.54 (m, 2H).

### Example 150

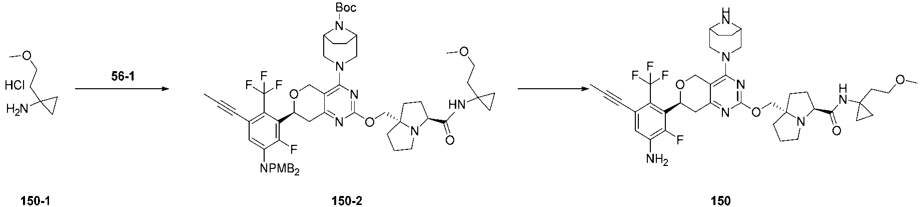

### Step 1

Compound **56-1** (50 mg, 50.76 µmol) was weighed and dichloromethane (2.0 mL) was added. N,N,N,N-tetramethyl-O-(7-azabenzotriazol-1-yl)uronium hexafluorophosphate (38.60 mg, 101.52 µmol) and diisopropylethylamine (19.68 mg, 152.27 µmol) were also added. Compound **150-1** (11.69 mg, 101.52 µmol) was then added and stirred at room temperature for 2 hours. Column chromatography (petroleum ether:ethyl acetate = 0:1) afforded compound **150-2.** MS m/z = 1082.8 [M+H]⁺.

### Step 2

Compound **150-2** (50 mg, 46.20 µmol) was weighed and dichloromethane (1 mL) was added. Trifluoroacetic acid (1 mL) was added and the mixture was stirred at room temperature for 0.5 hours. The reaction solution was concentrated under reduced pressure to obtain a crude product, which was then separated by preparative HPLC (column: Waters Xbridge, 250*19 mm, 5 µm; mobile phase: [water (0.1% ammonia)-acetonitrile]; gradient: (acetonitrile): 30%-70%) to obtain compound **150.** MS m/z = 742.6 [M+H]⁺. ¹H NMR (400 MHz, CD₃OD) *δ* ppm 6.90 (d, *J* = 8.0 Hz, 1H), 5.19 - 5.11 (m, 1H), 4.79 (d, *J =* 16.0 Hz, 1H), 4.65 (d, *J =* 12.0 Hz, 1H), 4.18 - 4.10 (m, 2H), 4.03 (d, *J =* 8.0 Hz, 1H), 3.69 - 3.63 (m, 1H), 3.58 - 3.48 (m, 5H), 3.45 - 3.39 (m, 1H), 3.30 (s, 3H), 3.26 - 3.17 (m, 1H), 3.08 - 3.00 (m, 1H), 2.91 - 2.80 (m, 2H), 2.79 - 2.70 (m, 1H), 2.13 - 2.05 (m, 2H), 2.04 - 1.95 (m, 5H), 1.94 - 1.78 (m, 8H), 1.77 - 1.68 (m, 2H), 0.76 - 0.64 (m, 4H).

### Example 151

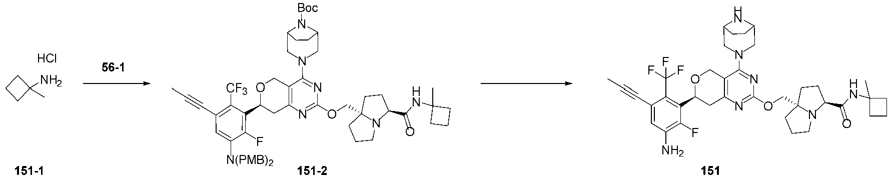

Referring to steps 2 to 3 of Example **56,** compound **151-1** was used as a starting material in place of (S)-3,3-difluorocyclopentaneamine hydrochloride to obtain crude compound **151.** The crude product was separated by preparative HPLC (column: Waters Xbridge, 250*19 mm, 5 µm; mobile phase: [water (0.1% ammonia)-acetonitrile]; gradient: (acetonitrile): 45%-70%) and dried to yield compound **151.** MS m/z = 712.5 [M+H]⁺. ¹H NMR (400 MHz, CD₃OD) δ ppm 6.90 (d, *J =* 8.0 Hz, 1H), 5.17 - 5.13 (m, 1H), 4.81 - 4.78 (m, 1H), 4.66 (d, *J = 12.0* Hz, 1H), 4.19 - 4.13 (m, 2H), 4.06 (d, *J* = 12.0 Hz, 1H), 3.73 - 3.71 (m, 1H), 3.59 - 3.53 (m, 3H), 3.45 - 3.42 (m, 1H), 3.26 - 3.19 (m, 1H), 3.06 (d, *J =* 12.0 Hz, 1H), 2.95 - 2.93 (m, 1H), 2.88 - 2.79 (m, 2H), 2.34 - 2.26 (m, 2H), 2.13 - 2.07 (m, 2H), 2.05 - 2.01 (m, 6H), 1.96 - 1.82 (m, 9H), 1.76 - 1.69 (m, 2H), 1.46 (s, 3H).

### Example 152

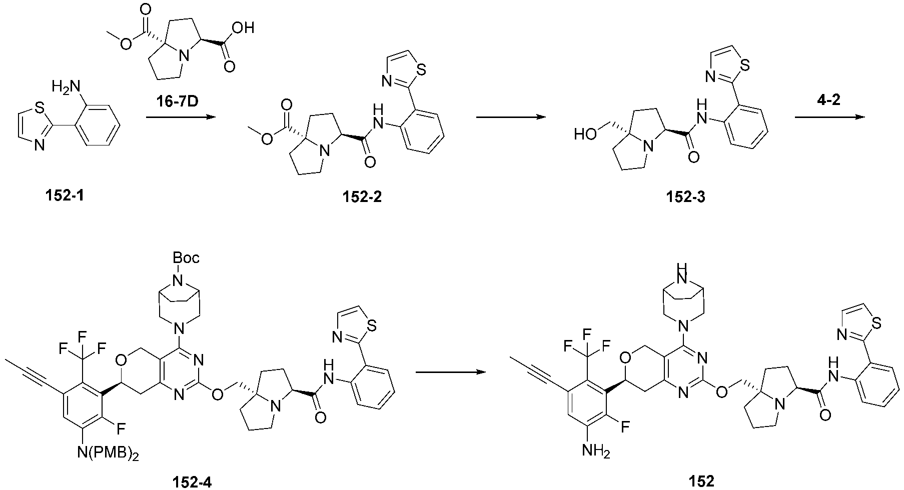

Referring to steps 7 to 10 of Example **16,** compound **152-1** was used as a starting material in place of *N*-ethyl-4-methoxybenzylamine to obtain crude compound **152.** The crude product was separated by preparative HPLC (Chromatographic column: Waters Xbridge, 250 x 19 mm, 5 µm; mobile phase: [water (0.1% ammonia)-acetonitrile]; gradient: (acetonitrile): 25%-60%) and dried to yield compound **152.** MS m/z = 803.5 [M+H]⁺. ¹H NMR (400 MHz, CD₃OD) δ ppm 8.60 (d, *J =* 8.0 Hz, 1H), 7.91 - 7.86 (m, 2H), 7.62 (d, *J =* 4.0 Hz, 1H), 7.44 (t, *J* = 8.0 Hz, 1H), 7.21 (t, *J =* 8.0 Hz, 1H), 6.90 (d, *J =* 8.0 Hz, 1H), 5.14 (dd, *J =* 12.0, 4.0 Hz, 1H), 4.78 (d, *J =* 16.0 Hz, 1H), 4.65 (d, *J* = 16.0 Hz, 1H), 4.31 (d, *J* = 8.0 Hz, 1H), 4.17 - 4.09 (m, 3H), 3.60 - 3.54 (m, 3H), 3.45 (d, *J = 12.0* Hz, 1H), 3.27 - 3.20 (m, 1H), 3.07 - 3.04 (m, 2H), 2.87 (dd, *J =* 20.0, 4.0 Hz, 1H), 2.78 - 2.71 (m, 1H), 2.26 - 2.16 (m, 3H), 2.07 - 2.02 (m, 5H), 1.91 - 1.67 (m, 7H).

### Example 153

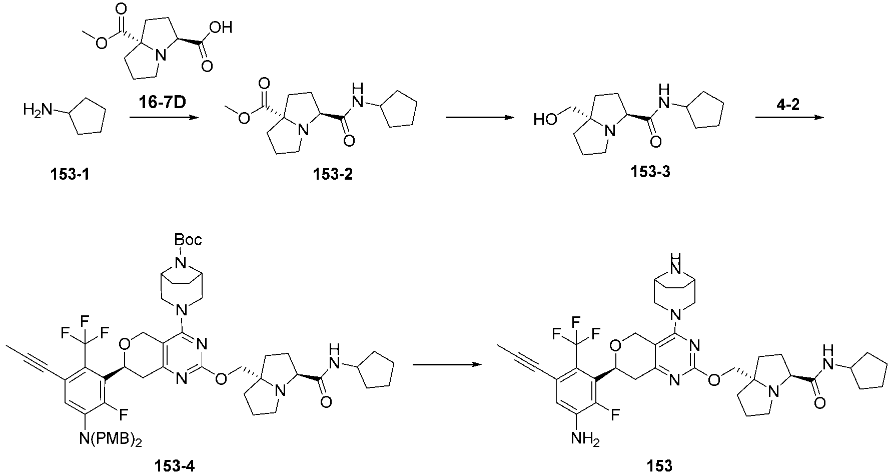

Referring to steps 7 to 10 of Example **16,** compound **153-1** was used as a starting material in place of *N*-ethyl-4-methoxybenzylamine to obtain crude compound **153.** The crude product was separated by preparative HPLC (Chromatographic column: xbridge-C18 250*19 mm, 5µm; mobile phase: [water (0.1% ammonia)-acetonitrile]; gradient: (acetonitrile): 45%-85%) and dried to yield compound **153.** MS m/z = 712.3 [M+H]⁺. ¹H NMR (400 MHz, CD₃OD) δ ppm 6.90 (d, *J* = 8.0 Hz, 1H), 5.15 (dd, *J* = 12.0, 4.0 Hz, 1H), 4.80 (d, *J =* 12.0 Hz, 1H), 4.65 (d*, J =* 16.0 Hz, 1H), 4.18 - 4.10 (m, 3H), 4.05 (d, *J =* 8.0 Hz, 1H), 3.75 - 3.71 (m, 1H), 3.55 - 3.52 (m, 3H), 3.43 (d, *J =* 12.0 Hz, 1H), 3.25 - 3.18 (m, 1H), 3.05 (d, *J* = 16.0 Hz, 1H), 2.92 - 2.82 (m, 2H), 2.78 - 2.72 (m, 1H), 2.13 - 2.08 (m, 2H), 2.04 - 1.89 (m, 11H), 1.86 - 1.84 (m, 2H), 1.77 - 1.59 (m, 6H), 1.53 - 1.45 (m, 2H).

### Example 154

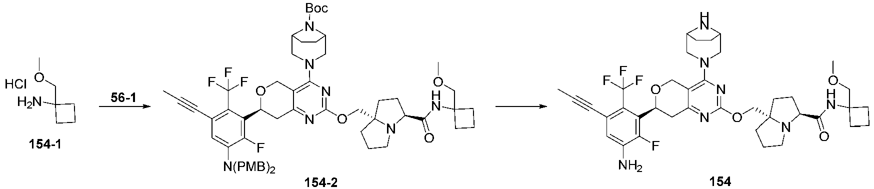

Referring to steps 2 to 3 of Example **56,** compound **154-1** was used as a starting material in place of (S)-3,3-difluorocyclopentylamine hydrochloride to obtain crude compound **154.** The crude product was separated by preparative HPLC (column: xbridge-C18, 30 x 250 mm, 5 µm; mobile phase: [water (0.1% ammonia)-acetonitrile]; gradient: (acetonitrile): 50%-70%) and dried to yield compound **154.** MS m/z = 742.5 [M+H]⁺. ¹H NMR (400 MHz, CD₃OD) δ ppm 6.90 (d, *J =* 8.0 Hz, 1H), 5.15 (dd, *J =* 12.0, 4.0 Hz, 1H), 4.80 (d, *J* = 12.0 Hz, 1H), 4.65 (d, *J =* 12.0 Hz, 1H), 4.18 - 4.13 (m, 2H), 4.06 (d, *J =* 12.0 Hz, 1H), 3.76 - 3.73 (m, 1H), 3.63 - 3.52 (m, 5H), 3.43 (d, *J =* 12.0 Hz, 1H), 3.36 (s, 3H), 3.22 (dd, *J =* 20.0, 12.0 Hz, 1H), 3.05 (d, *J* = 12.0 Hz, 1H), 2.99 - 2.97 (m, 1H), 2.88 - 2.74 (m, 2H), 2.30 - 2.01 (m, 10H), 1.97 - 1.80 (m, 9H), 1.76 - 1.68 (m, 2H).

### Example 155

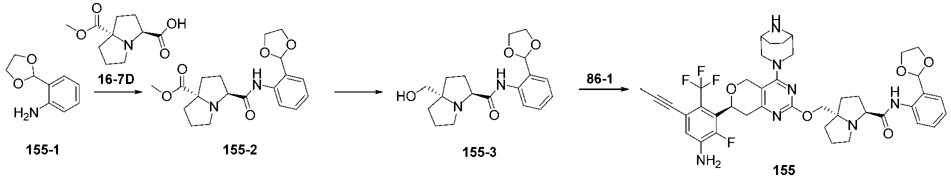

### Step 1

Referring to steps 7 to 8 of Example **16,** compound **155-1** was used as a starting material in place of *N*-ethyl-4-methoxybenzylamine to obtain compound **155-3.** MS m/z = 333.2 [M+H]⁺.

### Step 2

Compound **155-3** (45 mg, 135.38 µmol) was dissolved in anhydrous tetrahydrofuran (2 mL). Sodium hydride (8.12 mg, 203.07 µmol, 60% purity) was added at 0°C and stirred for 0.5 hours. Compound **86-1** (73.04 mg, 135.38 µmol) was added, and the mixture was warmed to 25°C and stirred for 1.5 hours. The reaction was quenched by the addition of water (5 mL) and extracted with ethyl acetate (15 mL x 3). The organic phases were combined, dried over anhydrous sodium sulfate, filtered, and concentrated under reduced pressure to obtain a crude product. The crude product was separated by preparative HPLC (column: Xbridge Prep C18, 250 x 19 mm, 5 µm; mobile phase: [water (0.1% ammonia)-acetonitrile]; gradient: (acetonitrile): 60%-80%) and dried to obtain compound 155. MS m/z = 792.6 [M+H]⁺. ¹H NMR (400 MHz, CD₃OD) δ ppm 7.96 (dd, *J =* 8.0, 4.0 Hz, 1H), 7.45 (d, *J =* 8.0 Hz, 1H), 7.39 (t, *J* = 8.0 Hz, 1H), 7.18 (t, *J* = 8.0 Hz, 1H), 6.90 (d, *J =* 8.0 Hz, 1H), 5.79 (s, 1H), 5.15 (dd, *J =* 12.0, 4.0 Hz, 1H), 4.80 (d, *J =* 16.0 Hz, 1H), 4.66 (d, *J* = 12.0 Hz, 1H), 4.25 (d, *J =* 8.0 Hz, 1H), 4.22 - 4.18 (m, 1H), 4.17 - 4.09 (m, 3H), 4.03 - 3.95 (m, 3H), 3.57 - 3.52 (m, 3H), 3.43 (d, *J =* 12.0 Hz, 1H), 3.26 - 3.19 (m, 1H), 3.12 - 3.03 (m, 2H), 2.86 (dd, *J =* 16.0, 4.0 Hz, 1H), 2.76 - 2.69 (m, 1H), 2.19 - 2.14 (m, 2H), 2.05 - 2.00 (m, 5H), 1.97 - 1.88 (m, 4H), 1.87 - 1.81 (m, 2H), 1.79 - 1.71 (m, 2H).

### Example 156

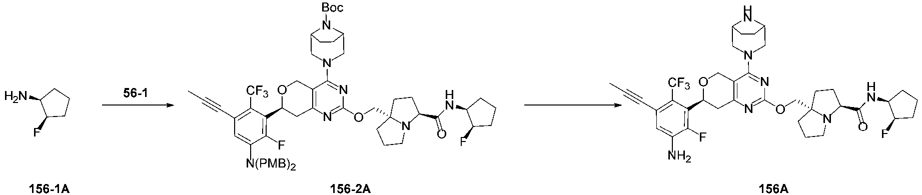

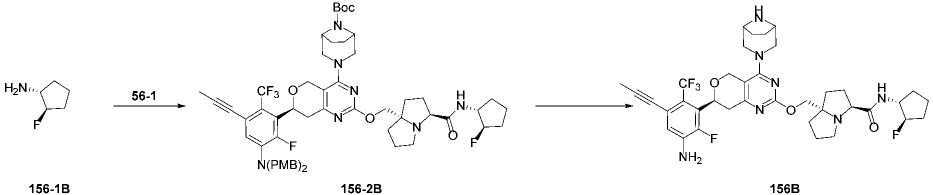

### Step 1

Compound **56-1** (90 mg, 101.15 µmol) was weighed and N,N-dimethylformamide (2.5 mL) was added. N,N,N,N-tetramethyl-O-(7-azabenzotriazol-1-yl)uronium hexafluorophosphate (69.44 mg, 182.74 µmol), diisopropylethylamine (70.72 mg, 548.22 µmol), and compound **156-1A** (28.23 mg, 274.41 µmol) were also added. The mixture was stirred at room temperature for 1 hour. Column chromatography (dichloromethane:methanol = 20:1) afforded compound **156-2A**. MS m/z = 1070.7 [M+H]⁺.

Referring to step 1, compound **156-1B** was used as a starting material in place of compound **156-1A** to obtain compound **156-2B.** MS m/z = 1070.7 [M+H]⁺.

### Step 2

Compound **156-2A** (60 mg, 56.07 µmol) was weighed and trifluoroacetic acid (2 mL) was added. The mixture was stirred at room temperature for 0.5 hours. The reaction mixture was concentrated under reduced pressure to obtain a crude product, which was then separated by preparative HPLC (Chromatographic column: Waters Xbridge, 250 x 19 mm, 5 µm; mobile phase: [water (0.1% ammonia)-acetonitrile]; gradient: (acetonitrile): 5% to 50%) to afford compound **156A.** MS m/z = 730.5 [M+H]⁺. ¹H NMR (400 MHz, CD₃OD) δ ppm 6.90 (d, *J* = 8.0 Hz, 1H), 5.19 - 5.09 (m, 1H), 4.93 - 4.77 (m, 2H), 4.65 (d, *J =* 12.0 Hz, 1H), 4.27 - 4.11 (m, 3H), 4.04 (d, *J =* 12.0 Hz, 1H), 3.77 - 3.71 (m, 1H), 3.57 - 3.47 (m, 3H), 3.42 (d, *J =* 12.0 Hz, 1H), 3.26 - 3.17 (m, 1H), 3.04 (d, *J* = 16.0 Hz, 1H), 2.91 - 2.80 (m, 2H), 2.77 - 2.69 (m, 1H), 2.17 - 2.06 (m, 3H), 2.03 - 1.96 (s, 5H), 1.96 - 1.77 (m, 10H), 1.76 - 1.64 (m, 2H), 1.59 - 1.50 (m, 1H).

Compound **156-2B** (50 mg, 46.69 µmol) was weighed and trifluoroacetic acid (2 mL) was added. The mixture was stirred at room temperature for 0.5 hours. The reaction mixture was concentrated under reduced pressure to obtain a crude product, which was then separated by preparative HPLC (Chromatographic column: Waters Xbridge, 250 x 19 mm, 5 µm; mobile phase: [water (0.1% ammonia)-acetonitrile]; gradient: (acetonitrile): 5%-50%) to afford compound **156B.** MS m/z = 730.5 [M+H]⁺. ¹H NMR (400 MHz, CD₃OD) δ ppm 6.90 (d, *J* = 8.0 Hz, 1H), 5.17 - 5.11 (m, 1H), 4.94 - 4.78 (m, 2H), 4.64 (d, *J =* 16.0 Hz, 1H), 4.26 - 4.11 (m, 3H), 4.04 (d, *J* = 12.0 Hz, 1H), 3.76 - 3.71 (m, 1H), 3.55 - 3.47 (m, 3H), 3.45 - 3.37 (m, 1H), 3.25 - 3.17 (m, 1H), 3.03 (d, *J =* 12.0 Hz, 1H), 2.90 - 2.80 (m, 2H), 2.77 - 2.69 (m, 1H), 2.16 - 2.06 (m, 3H), 2.01 (s, 3H), 2.00 - 1.66 (m, 14H), 1.58 - 1.50 (m, 1H).

### Example 157

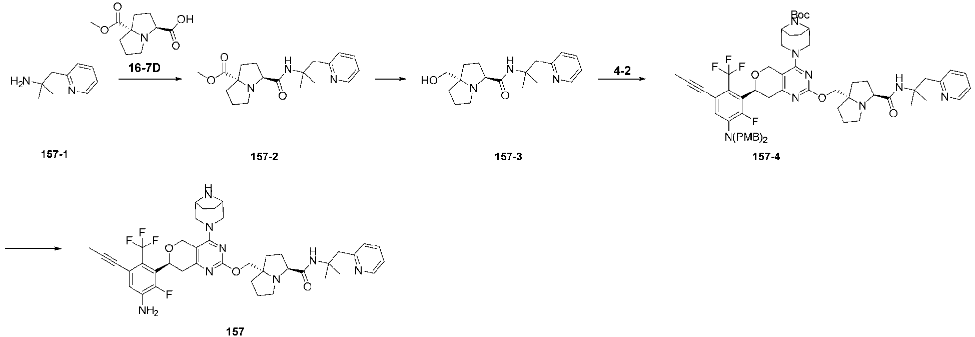

Referring to steps 7 to 10 of Example **16,** compound **157-1** was used as a starting material in place of *N*-ethyl-4-methoxybenzylamine to obtain crude compound **157.** The crude product was separated by preparative HPLC (Chromatographic column: Waters Xbridge, 250 x 19 mm, 5 µm; mobile phase: [water (0.1% ammonia)-acetonitrile]; gradient: (acetonitrile): 30%-60%) and dried to afford compound **157.** MS m/z = 777.5 [M+H]⁺. ¹H NMR (400 MHz, CD₃OD) δ 8.56 - 8.40 (m, 1H), 7.75 (t, *J =* 8.0 Hz, 1H), 7.35 - 7.18 (m, 2H), 6.90 (d, *J* = 8.0 Hz, 1H), 5.15 (d, *J =* 8.0 Hz, 1H), 4.81 - 4.79 (m, 1H), 4.67 (d, *J =* 12.0 Hz, 1H), 4.29 - 4.16 (m, 3H), 4.10 (d, *J* = 12.0 Hz, 2H), 3.86 - 3.81 (m, 1H), 3.65 - 3.62 (m, 1H), 3.50 (d, *J =* 12.0 Hz, 1H), 3.37 (d, *J =* 12.0 Hz, 1H), 3.26 - 3.18 (m, 1H), 3.16 - 3.09 (m, 1H), 3.03 (d, *J =* 12.0 Hz, 2H), 2.95 - 2.82 (m, 2H), 2.16 - 2.11 (m, 2H), 2.02 (s, 3H), 1.97 - 1.92 (m, 4H), 1.84 - 1.75 (m, 4H), 1.60 - 1.50 (m, 2H), 1.44 (s, 3H), 1.30 (s, 3H).

### Example 158

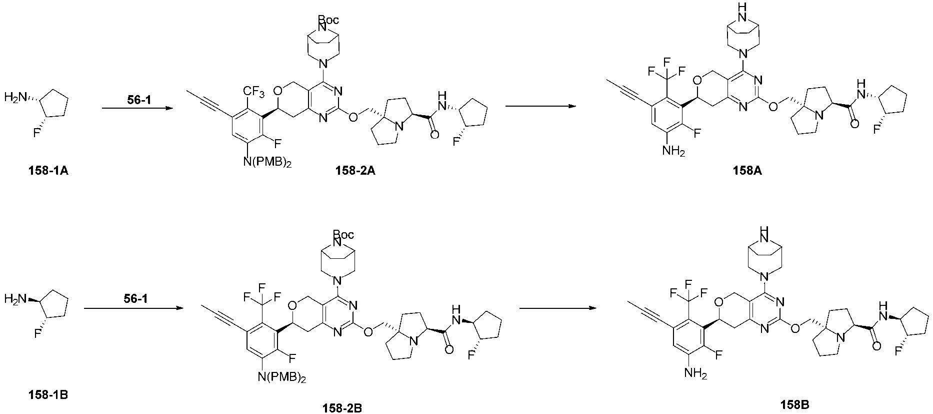

Referring to steps 2 to 3 of Example **56,** compound **158-1A** was used as a starting material in place of (S)-3,3-difluorocyclopentaneamine hydrochloride to obtain crude compound **158A.** The crude product was separated by preparative HPLC (column: Waters Xbridge, 250 x 19 mm, 5 µm; mobile phase: [water (0.1% ammonia)-acetonitrile]; gradient: (acetonitrile): 45%-70%) and dried to yield compound **158A.** MS m/z = 730.5 [M+H]⁺. ¹H NMR (400 MHz, CD₃OD) δ ppm 6.90 (d, *J =* 8.0 Hz, 1H), 5.24 - 5.05 (m, 1H), 4.95 - 4.78 (m, 2H), 4.65 (d, *J* = 12.0 Hz, 1H), 4.27 - 4.11 (m, 3H), 4.05 (d, *J* = 8.0 Hz, 1H), 3.79 - 3.68 (m, 1H), 3.63 - 3.51 (m, 3H), 3.43 (d, *J =* 12.0 Hz, 1H), 3.25 - 3.17 (m, 1H), 3.06 (d, *J =* 12.0 Hz, 1H), 2.92 - 2.79 (m, 2H), 2.77 - 2.71 (m, 1H), 2.14 - 2.09 (m, 2H), 2.04 - 2.00 (m, 4H), 1.97 - 1.89 (m, 5H), 1.90 - 1.78 (m,6H), 1.80 - 1.70 (m, 2H), 1.63 - 1.45 (m, 2H).

Referring to steps 2 to 3 of Example **56,** compound **158-1B** was used as a starting material in place of (S)-3,3-difluorocyclopentaneamine hydrochloride to obtain crude compound **158B.** The crude product was separated by preparative HPLC (column: Waters Xbridge, 250*19 mm, 5 µm; mobile phase: [water (0.1% ammonia)-acetonitrile]; gradient: (acetonitrile): 45%-70%) and dried to yield compound **158B.** MS m/z = 730.5 [M+H]⁺. ¹H NMR (400 MHz, CD₃OD) δ ppm 6.92 (d, *J =* 8.0 Hz, 1H), 5.17 (d, *J =* 8.0 Hz, 1H), 4.95 - 4.78 (m, 2H), 4.67 (d, *J =* 16.0 Hz, 1H), 4.36 - 4.11 (m, 3H), 4.07 (d, *J =* 12.0 Hz, 1H), 3.81 - 3.72 (m, 1H), 3.60 - 3.53 (m, 3H), 3.45 (d, *J =* 12.0 Hz, 1H), 3.28 - 3.17 (m, 1H), 3.07 (d, *J =* 12.0 Hz, 1H), 2.96 - 2.81 (m, 2H), 2.79 - 2.71 (m, 1H), 2.23 - 2.08 (m, 3H), 2.06 - 1.99 (m, 5H), 1.98 - 1.92 (m, 5H), 1.89 - 1.79 (m,5H), 1.78 - 1.66 (m, 2H), 1.62 - 1.52 (m, 1H).

### Example 159

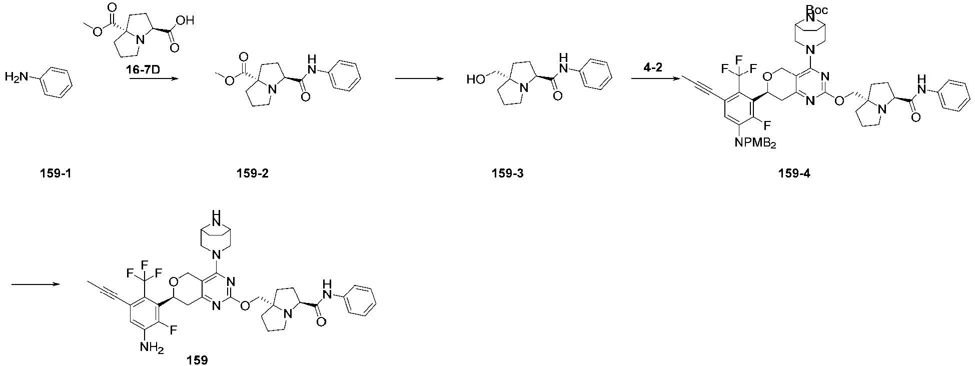

Referring to steps 7 to 10 of Example **16,** compound **159-1** was used as a starting material in place of *N*-ethyl-4-methoxybenzylamine to obtain crude compound **159.** The crude product was separated by preparative HPLC (Chromatographic column: Waters Xbridge, 250 x 19 mm, 5 µm; mobile phase: [water (0.1% ammonia)-acetonitrile]; gradient: (acetonitrile): 45%-70%) and dried to yield compound **159.** MS m/z = 720.5 [M+H]⁺. ¹H NMR (400 MHz, CD₃OD) δ ppm 7.59 - 7.56 (m, 2H), 7.33 - 7.29 (m, 2H), 7.12 - 7.08 (m, 1H), 6.90 (d, *J =* 8.0 Hz, 1H), 5.21 - 5.11 (m, 1H), 4.80 (d, *J =* 12.0 Hz, 1H), 4.66 (d, *J =* 12.0 Hz, 1H), 4.23 - 4.08 (m, 3H), 3.96 - 3.93 (m, 1H), 3.57 - 3.52 (m, 3H), 3.45 - 3.42 (m, 1H), 3.27 - 3.20 (m, 1H), 3.06 (d, *J =* 12.0 Hz, 1H), 2.98 - 2.93 (m, 1H), 2.89 - 2.80 (m, 2H), 2.26 - 2.13 (m, 1H), 2.16 - 2.02 (m, 2H), 2.04 - 1.91 (m, 7H), 1.94 - 1.78 (m, 3H), 1.81 - 1.67 (m, 2H).

### Example 160

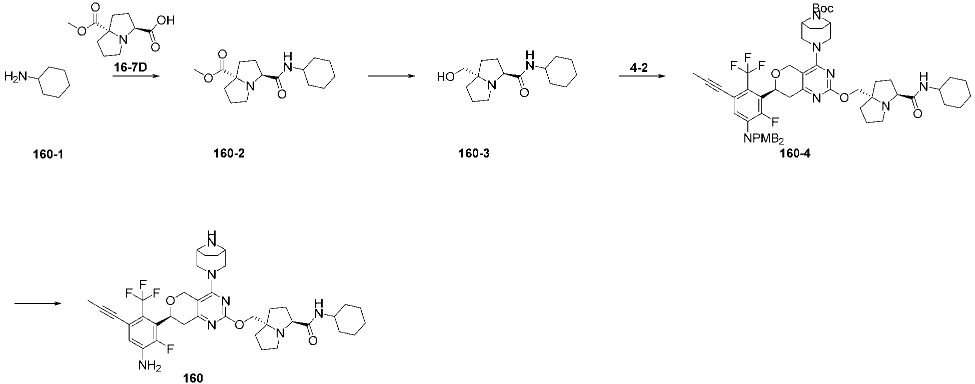

Referring to steps 7 to 10 of Example **16,** compound **160-1** was used as a starting material in place of *N*-ethyl-4-methoxybenzylamine to obtain crude compound **160.** The crude product was separated by preparative HPLC (Chromatographic column: Waters Xbridge, 250 x 19 mm, 5 µm; mobile phase: [water (0.1% ammonia)-acetonitrile]; gradient: (acetonitrile): 45%-70%) and dried to afford compound **160.** MS m/z = 726.6 [M+H]⁺. ¹H NMR (400 MHz, CD₃OD) δ ppm 6.90 (d, *J =* 8.0 Hz, 1H), 5.19 - 5.11 (m, 1H), 4.79 (d, *J =* 12.0 Hz, 1H), 4.65 (d, *J* = 12.0 Hz, 1H), 4.18 - 4.11 (m, 2H), 4.04 (d, *J =* 12.0 Hz, 1H), 3.75 - 3.64 (m, 2H), 3.57 - 3.49 (m, 3H), 3.44 - 3.38 (m, 1H), 3.26 - 3.17 (m, 1H), 3.07 - 3.01 (m, 1H), 2.93 - 2.82 (m, 2H), 2.78 - 2.71 (m, 1H), 2.14 - 2.07 (m, 2H), 2.05 - 1.95 (m, 5H), 1.94 - 1.80 (m, 8H), 1.79 - 1.68 (m, 4H), 1.66 - 1.61 (m, 1H), 1.41 - 1.31 (m, 2H), 1.30 - 1.20 (m, 3H).

### Example 161

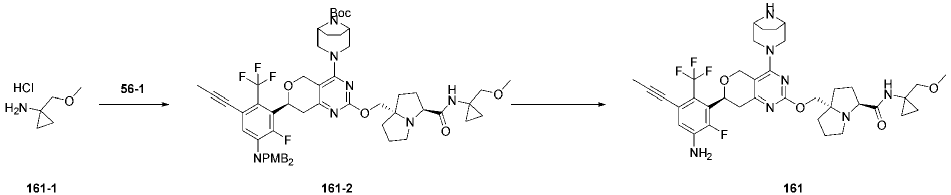

### Step 1

Compound **56-1** (50 mg, 50.76 µmol) was weighed and N,N-dimethylformamide (2 mL) was added. N,N,N,N-tetramethyl-O-(7-azabenzotriazol-1-yl)uronium hexafluorophosphate (28.95 mg, 76.14 µmol), diisopropylethylamine (32.80 mg, 253.78 µmol), and compound **161-1** (13.97 mg, 101.51 µmol) were also added. The mixture was stirred at room temperature for 1 hour. Column chromatography (dichloromethane:methanol = 20:1) afforded compound **161-2.** MS m/z = 1068.7 [M+H]⁺.

### Step 2

Compound **161-2** (45 mg, 42.12 µmol) was weighed and trifluoroacetic acid (1 mL) was added. The mixture was stirred at room temperature for 0.5 hours. The reaction mixture was concentrated under reduced pressure to obtain a crude product, which was then separated by preparative HPLC (Chromatographic column: Waters Xbridge, 250*19 mm, 5 µm; mobile phase: [water (0.1% ammonia)-acetonitrile]; gradient: (acetonitrile): 5%-50%) to afford compound **161.** MS m/z = 728.6 [M+H]⁺. ¹H NMR (400 MHz, CD₃OD) δ ppm 6.90 (d, *J* = 8.0 Hz, 1H), 5.20 - 5.10 (m, 1H), 4.79 (d, *J =* 12.0 Hz, 1H), 4.65 (d, *J =* 16.0 Hz, 1H), 4.18 - 4.11 (m, 2H), 4.04 (d, *J =* 12.0 Hz, 1H), 3.72 - 3.67 (m, 1H), 3.57 - 3.53 (m, 2H), 3.52 - 3.49 (m, 1H), 3.48 - 3.36 (m, 3H), 3.34 (s, 3H), 3.27 - 3.17 (m, 1H), 3.04 (d, *J =* 12.0 Hz, 1H), 2.95 - 2.89 (m, 1H), 2.88 - 2.81 (m, 1H), 2.78 - 2.70 (m, 1H), 2.15 - 2.05 (m, 2H), 2.04 - 1.99 (m, 4H), 1.99 - 1.87 (m, 5H), 1.87 - 1.81 (m, 2H), 1.77 - 1.66 (m, 2H), 0.84 - 0.72 (m, 4H).

### Example 162

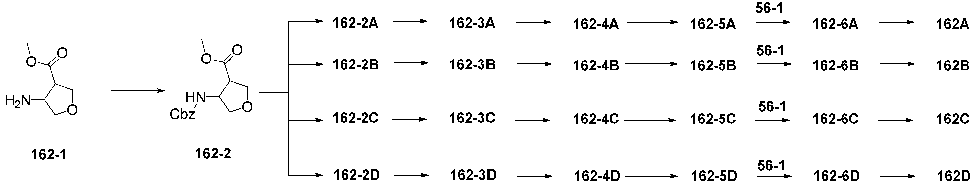

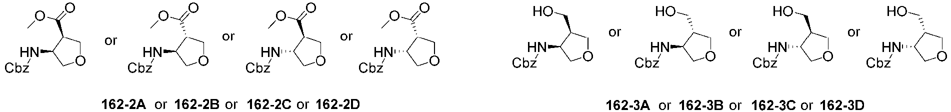

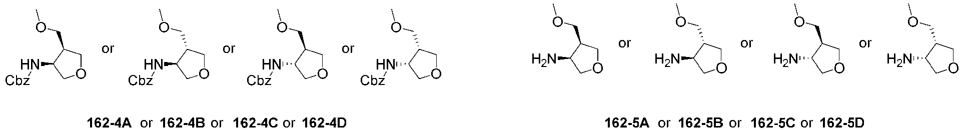

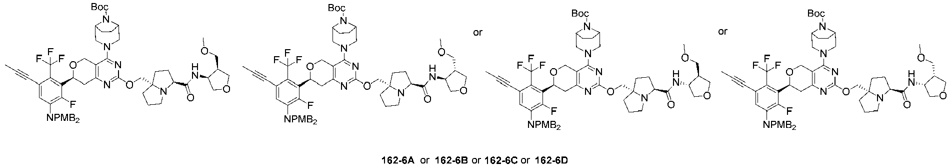

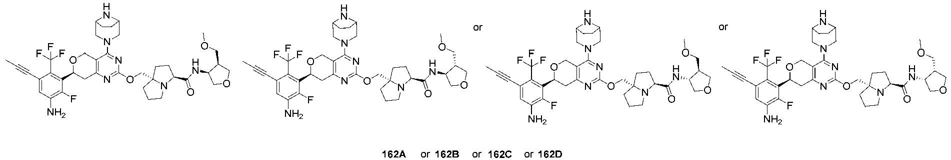

### Step 1

Compound **162-1** (1.2 g, 8.27 mmol) was weighed and tetrahydrofuran (30 mL), water (30 mL), sodium carbonate (2.63 g, 24.80 mmol), and benzyl chloroformate (2.12 g, 12.40 mmol) were added. The mixture was stirred at room temperature for 1 hour. Water (30 mL) was then added and the mixture was extracted with ethyl acetate (20 mL x 3). The mixture was dried over anhydrous sodium sulfate, and the organic phase was concentrated under reduced pressure. The mixture was separated by column chromatography (petroleum ether:ethyl acetate = 8:1) to afford compound **162-2,** MS m/z = 280.0 [M+H]⁺. Preparative supercritical liquid chromatography (SFC) was then performed (column: DAICEL CHIRALPAK^{®} AD-10, 25*250 mm 10 µm; mobile phase: A: supercritical carbon dioxide, B: methanol + (0.05%) ammonia methanol; B%: 20%) to obtain compounds **162-2A, 162-2B, 162-2C** and compound **162-2D.** Analytical SFC method (Chromatographic column: DAICEL CHIRALPAK^{®} AD-3 (100*3 mm3 µm); mobile phase: [A: supercritical carbon dioxide B: methanol (0.05% diethylamine)]; gradient B%: 5%-40%, 4 min), compound **162-2A,** Rt = 2.317 minutes, ee value 97.96%, MS m/z = 280.0 [M+H]⁺; compound **162-2B,** Rt = 2.817 minutes, ee value 100%, MS m/z = 280.0 [M+H]⁺; compound **162-2C,** Rt = 2.951 minutes, ee value 99.12%, MS m/z = 280.0 [M+H]⁺; compound **162-2D,** Rt = 3.332 minutes, ee value 100%, MS m/z = 280.0 [M+H]⁺.

### Step 2

Compound **162-2A** (190 mg, 680.30 µmol) was weighed and anhydrous tetrahydrofuran (2 mL) was added. Under nitrogen, lithium aluminum tetrahydride (77.46 mg, 2.04 mmol, 2.5 M solution in tetrahydrofuran) was added at -60°C. The mixture was stirred at -60°C for 2 hours and diluted with dichloromethane (10 mL). Water (0.1 mL) was added to the reaction mixture at 0°C, and the reaction was quenched with 15% sodium hydroxide solution (0.1 mL). The mixture was stirred for 10 minutes, and dried over anhydrous sodium sulfate. The organic phase was concentrated under reduced pressure, and separated by column chromatography (dichloromethane:methanol = 40:1) to obtain compound **162-3A.** MS m/z = 252.2 [M+H]⁺.

Referring to step 2, compound **162-2B** was used as a starting material in place of compound **162-2A** to obtain compound **162-3B.** MS m/z = 252.2 [M+H]⁺.

Referring to step 2, compound **162-2C** was used as a starting material in place of compound **162-2A** to obtain compound **162-3C.** MS m/z = 252.2 [M+H]⁺.

Referring to step 2, compound **162-2D** was used as a starting material in place of compound **162-2A** to obtain compound **162-3D.** MS m/z = 252.2 [M+H]⁺.

### Step 3

Compound **162-3A** (115 mg, 457.66 µmol) was weighed and anhydrous dichloromethane (3 mL) was added. 4Å molecular sieves (780.15 mg, 457.66 µmol), trimethyloxonium tetrafluoroborate (135.38 mg, 915.32 µmol), and tetramethyl(1,8-naphthalenediamine) (245.20 mg, 1.14 mmol) were also added. The mixture was stirred at room temperature for 2 hours under nitrogen. Water (30 mL) was added, and the mixture was extracted with ethyl acetate (20 mL x 3). The mixture was dried over anhydrous sodium sulfate, and the organic phase was concentrated under reduced pressure. The mixture was separated by column chromatography (petroleum ether:ethyl acetate = 1:1) to afford compound **162-4A.** MS m/z = 266.2 [M+H]⁺.

Referring to step 3, compound **162-3B** was used as a starting material in place of compound **162-3A** to obtain compound **162-4B.** MS m/z = 266.2 [M+H]⁺.

Referring to step 3, compound **162-3C** was used as a starting material in place of compound **162-3A** to obtain compound **162-4C.** MS m/z = 266.2 [M+H]⁺.

Referring to step 3, compound **162-3D** was used as a starting material in place of compound **162-3A** to obtain compound **162-4D.** MS m/z = 266.2 [M+H]⁺.

### Step 4

Compound **162-4A** (65 mg, 245.00 µmol) was weighed and methanol (10 mL) was added. Palladium on carbon (26.07 mg, 245.00 µmol) was added and the mixture was reacted at 20°C under a hydrogen atmosphere (15 psi) for 16 hours. The mixture was filtered, and the filtrate was concentrated under reduced pressure to afford compound **162-5A.** MS m/z = 132.1 [M+H]⁺.

Referring to step 4, compound **162-4B** was used as a starting material in place of compound **162-4A** to obtain compound **162-5B.** MS m/z = 132.1 [M+H]⁺.

Referring to step 4, compound **162-4C** was used as a starting material in place of compound **162-4A** to obtain compound **162-5C.** MS m/z = 132.1 [M+H]⁺.

Referring to step 4, compound **162-4D** was used as a starting material in place of compound **162-4A** to obtain compound **162-5D.** MS m/z = 132.1 [M+H]⁺.

### Step 5

Compound **162-5A** (11.98 mg, 91.36 µmol) was weighed and dichloromethane (1.0 mL) was added. N,N,N,N-tetramethyl-O-(7-azabenzotriazol-1-yl)uronium hexafluorophosphate (39.37 mg, 103.55 µmol) and diisopropylethylamine (23.62 mg, 182.73 µmol) were also added. Compound **56-1** (60 mg, 60.91 µmol) was then added and stirred at room temperature for 2 hours. Column chromatography (petroleum ether:ethyl acetate = 0:1) afforded compound **162-6A.** MS m/z = 1098.7 [M+H]⁺.

Referring to step 5, compound **162-5B** was used as a starting material in place of compound **162-5A** to obtain compound **162-6B.** MS m/z = 1098.7 [M+H]⁺.

Referring to step 5, compound **162-5C** was used as a starting material in place of compound **162-5A** to obtain compound **162-6C.** MS m/z = 1098.7 [M+H]⁺.

Referring to step 5, compound **162-5D** was used as a starting material in place of compound **162-5A** to obtain compound **162-6D.** MS m/z = 1098.7 [M+H]⁺.

### Step 6

Compound **162-6A** (65 mg, 29.59 µmol) was weighed. Dichloromethane (1 mL) and trifluoroacetic acid (1 mL) were added, and the mixture was stirred at room temperature for 0.5 hours. The reaction solution was concentrated under reduced pressure to obtain a crude product, which was then separated by preparative HPLC (Chromatographic column: Waters Xbridge, 250*19 mm, 5 µm; mobile phase: [water (0.1% ammonia)-acetonitrile]; gradient: (acetonitrile): 30%-70%) to afford compound **162A.** MS m/z = 758.5 [M+H]⁺. ¹H NMR (400 MHz, CD₃OD) *δ* ppm 6.90 (d, *J =* 8.0 Hz, 1H), 5.17 - 5.13 (m, 1H), 4.80 (d, *J* = 12.0 Hz, 1H), 4.65 (d, *J* = 16.0 Hz, 1H), 4.59 - 4.53 (m, 1H), 4.18 - 4.13 (m, 2H), 4.06 (d, *J =* 12.0 Hz, 1H), 3.99 - 3.92 (m, 2H), 3.81 - 3.77 (m, 1H), 3.72 - 3.63 (m, 2H), 3.57 - 3.47 (m, 4H), 3.46 - 3.32 (m, 3H), 3.29 - 3.11 (m, 3H), 3.04 (d, *J=* 12.0 Hz, 1H), 2.95 - 2.81 (m, 2H), 2.75 - 2.64 (m, 2H), 2.15 - 2.03 (m, 3H), 2.02 (s, 3H), 1.97 - 1.88 (m, 4H), 1.86 - 1.80 (m, 2H), 1.77 - 1.64 (m, 3H).

Compound **162-6B** (65 mg, 29.59 µmol) was weighed. Dichloromethane (1 mL) and trifluoroacetic acid (1 mL) were added, and the mixture was stirred at room temperature for 0.5 hours. The reaction solution was concentrated under reduced pressure to obtain a crude product, which was then separated by preparative HPLC (Chromatographic column: Waters Xbridge, 250*19 mm, 5 µm; mobile phase: [water (0.1% ammonia)-acetonitrile]; gradient: (acetonitrile): 30%-70%) to afford compound **162B.** MS m/z = 758.5 [M+H]⁺. ¹HNMR (400 MHz, CD₃OD) *δ* ppm 6.90 (d, *J =* 8.0 Hz, 1H), 5.14 (dd, *J =* 8.0, 4.0 Hz, 1H), 4.80 (d, *J =* 12.0 Hz, 1H), 4.65 (d, *J =* 12.0 Hz, 1H), 4.21 - 4.10 (m, 3H), 4.08 - 3.90 (m, 3H), 3.81 - 3.72 (m, 1H), 3.62 - 3.50 (m, 5H), 3.49 - 3.37 (m, 3H), 3.34 (s, 3H), 3.22 (dd, *J =* 16.0, 12.0 Hz, 1H), 3.04 (d, *J =* 12.0 Hz, 1H), 2.94 - 2.79 (m, 2H), 2.78 - 2.66 (m, 1H), 2.46 - 2.34 (m, 1H), 2.15 - 2.06 (m, 2H), 2.05 - 2.00 (m, 4H), 2.00 - 1.86 (m, 5H), 1.85 - 1.79 (m, 2H), 1.77 - 1.66 (m, 2H).

Compound **162-6C** (65 mg, 29.59 µmol) was weighed. Dichloromethane (1 mL) and trifluoroacetic acid (1 mL) were added, and the mixture was stirred at room temperature for 0.5 hours. The reaction solution was concentrated under reduced pressure to obtain a crude product, which was then separated by preparative HPLC (Chromatographic column: Waters Xbridge, 250*19 mm, 5 µm; mobile phase: [water (0.1% ammonia)-acetonitrile]; gradient: (acetonitrile): 30%-70%) to afford compound **162C.** MS m/z = 758.5 [M+H]⁺. ¹H NMR (400 MHz, CD₃OD) *δ* ppm 6.90 (d, *J =* 8.0 Hz, 1H), 5.16 - 5.12 (m, 1H), 4.81 - 4.78 (m, 1H), 4.65 (d, J = 12.0 Hz, 1H), 4.23 - 4.10 (m, 3H), 4.07 - 3.93 (m, 3H), 3.79 - 3.72 (m, 1H), 3.61 - 3.53 (m, 4H), 3.51 - 3.37 (m, 4H), 3.34 - 3.32 (m, 3H), 3.21 (dd, *J =* 16.0, 12.0 Hz, 1H), 3.05 (d, *J =* 12.0 Hz, 1H), 2.95 - 2.88 (m, 1H), 2.88 - 2.72 (m, 2H), 2.47 - 2.35 (m, 1H), 2.14 - 2.06 (m, 2H), 2.04 - 2.00 (m, 4H), 1.99 - 1.87 (m, 5H), 1.86 - 1.80 (m, 2H),1.77 - 1.66 (m, 2H).

Compound **162-6D** (65 mg, 29.59 µmol) was weighed. Dichloromethane (1 mL) and trifluoroacetic acid (1 mL) were added, and the mixture was stirred at room temperature for 0.5 hours. The reaction solution was concentrated under reduced pressure to obtain a crude product, which was then separated by preparative HPLC (Chromatographic column: Waters Xbridge, 250*19 mm, 5 µm; mobile phase: [water (0.1% ammonia)-acetonitrile]; gradient: (acetonitrile): 30%-70%) to obtain compound **162D.** MS m/z = 758.5 [M+H]⁺. ¹H NMR (400 MHz, CD₃OD) *δ* ppm 6.90 (d, *J =* 8.0 Hz, 1H), 5.20 - 5.07 (m, 1H), 4.79 (d, *J =* 12.0 Hz, 1H), 4.65 (d, *J =* 12.0 Hz, 1H), 4.57 - 4.52 (m, 1H), 4.19 - 4.13 (m, 2H), 4.06 (d, *J =* 8.0 Hz, 1H), 4.01 - 3.91 (m, 2H), 3.80 - 3.77 (m, 1H), 3.70 (t, *J =* 8.0 Hz, 1H), 3.64 (dd, *J =* 8.0, 4.0 Hz, 1H), 3.58 - 3.48 (m, 4H), 3.47 - 3.38 (m, 2H), 3.31 (s, 3H), 3.21 (dd, *J =* 20.0, 12.0 Hz, 1H), 3.04 (d, *J =* 16.0 Hz, 1H), 2.98 - 2.93 (m, 1H), 2.86 - 2.76 (m, 2H), 2.71 - 2.63 (m, 1H), 2.15 - 2.07 (m, 2H), 2.05 - 1.98 (m, 5H), 1.97 - 1.87 (m, 4H), 1.86 - 1.80 (s, 2H), 1.79 - 1.68 (m, 2H).

### Example 163

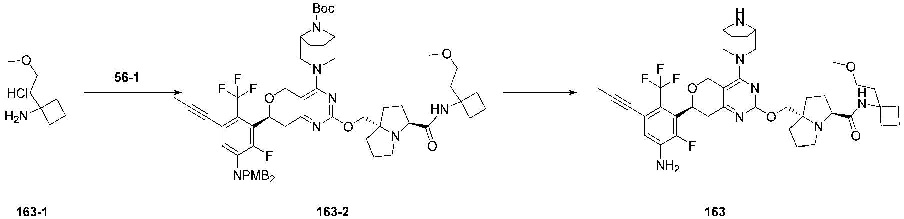

### Step 1

Compound **56-1** (50 mg, 50.76 µmol) was weighed and dichloromethane (2.0 mL) was added. N,N,N,N-tetramethyl-O-(7-azabenzotriazol-1-yl)uronium hexafluorophosphate (38.60 mg, 101.52 µmol) and diisopropylethylamine (19.68 mg, 152.27 µmol) were also added. Compound **163-1** (19.68 mg, 152.28 µmol) was then added and stirred at room temperature for 2 hours. Column chromatography (petroleum ether:ethyl acetate = 0:1) afforded compound **163-2.** MS m/z = 1096.8 [M+H]⁺.

### Step 2

Compound **163-2** (30 mg, 27.37 µmol) was weighed and dichloromethane (1 mL) was added. Trifluoroacetic acid (1 mL) was added and stirred at room temperature for 0.5 hours. The reaction solution was concentrated under reduced pressure to obtain a crude product, which was then separated by preparative HPLC (column: Waters Xbridge, 250*19 mm, 5 µm ; mobile phase: [water (0.1% ammonia)-acetonitrile]; gradient: (acetonitrile): 30%-70%) to obtain compound **163.** MS m/z = 756.6 [M+H]⁺. ¹H NMR (400 MHz, CD₃OD) *δ* ppm 6.92 (d, *J =* 8.0 Hz, 1H), 5.21 - 5.13 (m, 1H), 4.79 (d, *J =* 16.0 Hz, 1H), 4.67 (d, *J =* 12.0 Hz, 1H), 4.23 - 4.13 (m, 2H), 4.07 (d, *J* = 12.0 Hz, 1H), 3.78 - 3.71 (m, 1H), 3.60 - 3.51 (m, 3H), 3.50 - 3.41 (m, 3H), 3.30 (s, 3H), 3.28 - 3.19 (m, 1H), 3.06 (d, J = 12.0 Hz, 1H), 3.03 - 2.93 (m, 1H), 2.92 - 2.77 (m, 2H), 2.30 - 2.24 (m, 2H), 2.20 - 2.06 (m, 6H), 2.01 (s, 3H), 2.01 - 1.79 (m, 10H), 1.76 - 1.67 (m, 2H).

### Example 164

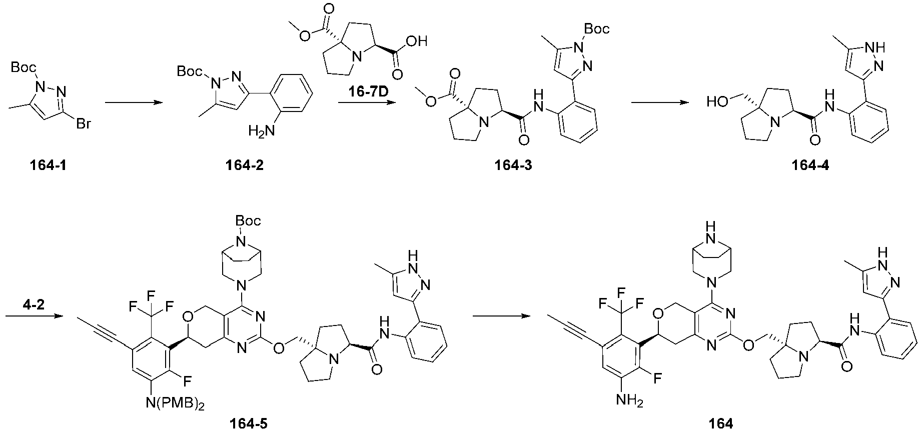

### Step 1

Compound **164-1** (300 mg, 1.15 mmol) and 2-aminophenylboronic acid (188.80 mg, 1.38 mmol) were dissolved in 1,4-dioxane (8 mL) and water (2 mL). 1,1-bis(diphenylphosphino)ferrocenepalladium dichloride (168.13 mg, 229.78 µmol) and potassium carbonate (476.36 mg, 3.45 mmol) were added. Under nitrogen protection, the mixture was heated to 90°C and stirred for 4 hours. After returning to room temperature, water (2 mL) was added to quench the reaction. The mixture was extracted with ethyl acetate (15 mL * 3). The organic phases were combined, dried over anhydrous sodium sulfate, filtered, and concentrated under reduced pressure. Column chromatography (petroleum ether:ethyl acetate = 10:1) afforded compound **164-2.** MS m/z = 274.2 [M+H]⁺.

### Step 2

Referring to steps 7 to 10 of Example **16,** compound **164-2** was used as a starting material in place of *N*-ethyl-4-methoxybenzylamine to obtain crude compound **164.** The crude product was separated by preparative HPLC (Chromatographic column: Xbridge-C18 250*19 mm, 5µm; mobile phase: [water (0.1% ammonia)-acetonitrile]; gradient: (acetonitrile): 45%-85%) and dried to yield compound **164.** MS m/z = 800.6 [M+H]⁺. ¹H NMR (400 MHz, DMSO-*d₆*) δ ppm 12.98 (s, 1H), 11.80 (s, 1H), 8.52 (d, *J* = 8.0 Hz, 1H), 7.71 (d, *J* = 8.0 Hz, 1H), 7.26 (t, *J =* 8.0 Hz, 1H), 7.13 - 7.09 (m, 1H), 6.88 (d, *J =* 8.0 Hz, 1H), 6.57 (s, 1H), 6.02 (s, 2H), 5.04 (dd, *J =* 8.0, 4.0 Hz, 1H), 4.76 (d, *J =* 16.0 Hz, 1H), 4.56 (d, *J* = 12.0 Hz, 1H), 4.04 (d, *J =* 12.0 Hz, 1H), 3.91 (d, *J =* 12.0 Hz, 2H), 3.79 (t, *J =* 12.0 Hz, 1H), 3.43 - 3.38 (m, 4H), 3.25 (s, 1H), 3.09 - 3.02 (m, 1H), 2.90 - 2.78 (m, 3H), 2.64 - 2.60 (m, 1H), 2.30 (s, 3H), 2.16 - 2.07 (m, 2H), 2.03 (s, 3H), 1.97 - 1.93 (m, 1H), 1.89 - 1.77 (m, 5H), 1.66 - 1.54 (m, 4H).

### Example 165

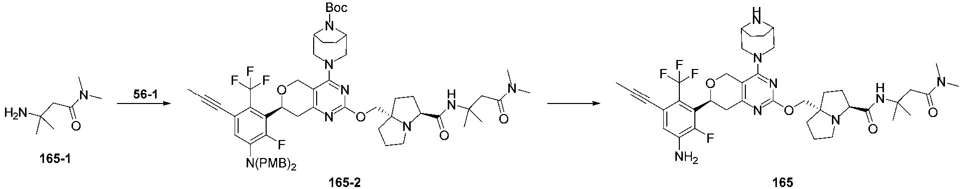

Referring to steps 2 to 3 of Example **56,** compound **165-1** was used as a starting material in place of (S)-3,3-difluorocyclopentylamine hydrochloride to obtain crude compound **165.** The crude product was separated by preparative HPLC (Chromatographic column: Xbridge-C18, 30 x 250 mm, 5 µm; mobile phase: [water (0.1% ammonia)-acetonitrile]; gradient: (acetonitrile): 40%-70%) and dried to yield compound **165.** MS m/z = 771.6 [M+H]⁺. ¹H NMR (400 MHz, CD₃OD) δ ppm 6.90 (d, *J =* 8.0 Hz, 1H), 5.15 (dd, *J* = 8.0 Hz, 4.0 Hz, 1H), 4.80 (d, *J* = 12.0 Hz, 1H), 4.65 (d, *J =* 16.0 Hz, 1H), 4.18 - 4.13 (m, 2H), 4.06 (d, *J =* 12.0 Hz, 1H), 3.76 - 3.70 (m, 1H), 3.57 - 3.52 (m, 3H), 3.43 (d, *J =* 12.0 Hz, 1H), 3.22 (dd, *J* = 16.0 Hz, 8.0 Hz, 1H), 3.10 (s, 3H), 3.05 (d, *J* = 12.0 Hz, 1H), 3.01 - 2.95 (m, 1H), 2.93 - 2.90 (m, 4H), 2.87 - 2.79 (m, 2H), 2.77 - 2.70 (m, 1H), 2.15 - 2.04 (m, 3H), 2.02 (s, 3H), 1.95 - 1.83 (m, 7H), 1.75 - 1.70 (m, 2H), 1.47 (s, 3H), 1.44 (s, 3H).

### Example 166

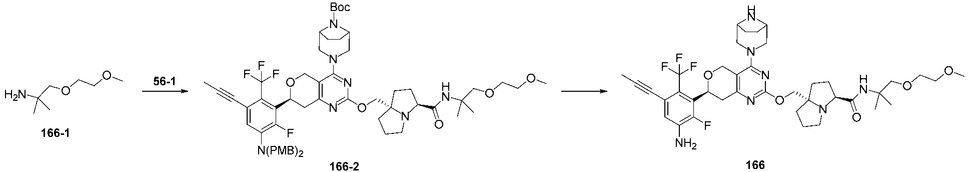

Referring to steps 2 to 3 of Example **56,** compound **166-1** was used as a starting material in place of (*S*)-3,3-difluorocyclopentylamine hydrochloride to obtain crude compound **166.** The crude product was separated by preparative HPLC (Chromatographic column: Xbridge-C18, 30 x 250 mm, 5 µm; mobile phase: [water (0.1% formic acid)-acetonitrile]; gradient: (acetonitrile): 20%-65%) and dried to afford the formate salt of compound **166.** MS m/z = 774.6 [M+H]⁺. ¹H NMR (400 MHz, CD₃OD) δ ppm 8.51 (s, 1.32H), 6.91 (d, *J* = 8.0 Hz, 1H), 5.17 (dd, *J =* 12.0 Hz, 4.0 Hz, 1H), 4.81 (s, 1H), 4.69 (d, *J =* 12.0 Hz, 1H), 4.30 - 4.15 (m, 3H), 3.99 - 3.90 (m, 3H), 3.71 - 3.67 (m, 1H), 3.62 - 3.52 (m, 6H), 3.48 - 3.46 (m, 1H), 3.34 (s, 3H), 3.27 - 3.17 (m, 3H), 2.98 - 2.87 (m, 2H), 2.22 - 2.13 (m, 3H), 2.05 - 1.81 (m, 12H), 1.34 (s, 3H), 1.33 (s, 3H).

### Example 167

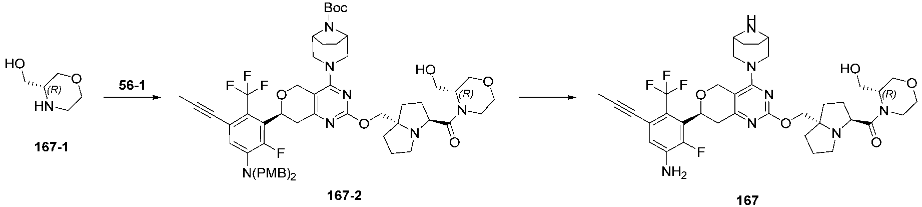

Referring to steps 2 to 3 of Example **56,** compound **167-1** was used as a starting material in place of (*S*)-3,3-difluorocyclopentylamine hydrochloride to obtain crude compound **167.** The crude product was separated by preparative HPLC (Chromatographic column: Xbridge-C18, 30 x 250 mm, 5 µm; mobile phase: [water (0.1% ammonia)-acetonitrile]; gradient: (acetonitrile): 35%-75%) and dried to yield compound **167.** MS m/z = 744.5 [M+H]⁺. ¹H NMR (400 MHz, CD₃OD) δ ppm 6.91 (d, *J* = 8.0 Hz, 1H), 5.15 (dd, *J* = 12.0 Hz, 4.0 Hz, 1H), 4.80 (d, *J =* 16.0 Hz, 1H), 4.67 - 4.62 (m, 1H), 4.48 - 4.30 (m, 2H), 4.11 - 4.01 (m, 2H), 3.97 (s, 1H), 3.94 - 3.86 (m, 1H), 3.85 - 3.77 (m, 1H), 3.73 - 3.52 (m, 5H), 3.49 - 3.35 (m, 4H), 3.24 - 3.14 (m, 2H), 3.08 (d, *J* = 12.0 Hz, 1H), 2.88 - 2.83 (m, 2H), 2.61 - 2.49 (m, 1H), 2.20 - 2.13 (m, 1H), 2.08 - 2.04 (m, 1H), 2.02 (s, 3H), 1.96 - 1.71 (m, 8H), 1.63 - 1.52 (m, 2H).

### Example 168

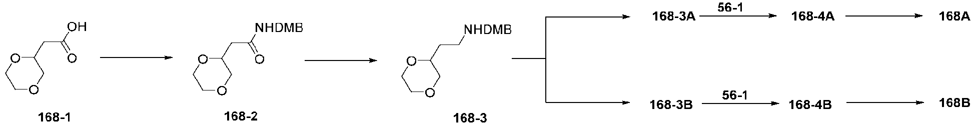

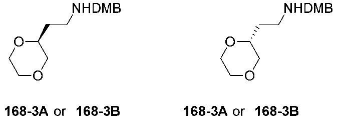

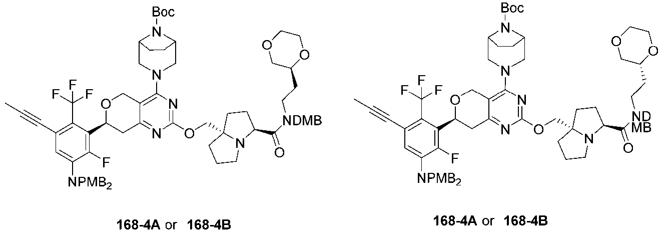

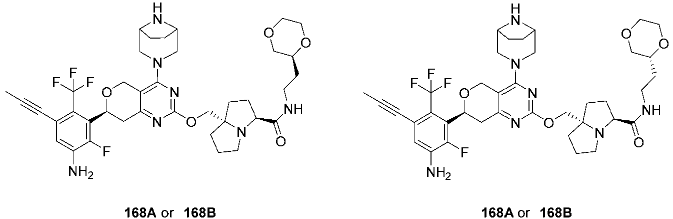

### Step 1

Compound **168-1** (200 mg, 1.37 mmol) was weighed and N,N-dimethylformamide (4 mL) was added. N,N,N,N-tetramethyl-O-(7-azabenzotriazol-1-yl)uronium hexafluorophosphate (780.54 mg, 2.05 mmol), diisopropylethylamine (530.62 mg, 4.11 mmol), and 2,4-dimethoxybenzylamine (457.65 mg, 2.74 mmol) were added. The mixture was reacted at 25°C for 12 hours. The reaction was quenched by the addition of 10 mL of water. The product was extracted with ethyl acetate (10 mL x 3), dried over anhydrous sodium sulfate, and filtered. The filtrate was concentrated under reduced pressure, and separated by column chromatography (petroleum ether:ethyl acetate = 1:1) to obtain compound **168-2.** MS m/z = 296.1 [M+H]⁺

### Step 2

Compound **168-2** (450 mg, 1.52 mmol) was weighed and tetrahydrofuran (10 mL) was added. Lithium aluminum tetrahydride (2.5 M, 4.57 mmol, 1.83 mL) was then added at 0°C. The mixture was reacted at 60°C for 12 hours. The reaction was quenched by the addition of 3 mL of water, dried over anhydrous sodium sulfate, and filtered. The filtrate was concentrated under reduced pressure, and subjected to reverse-phase separation to afford compound **168-3.** MS m/z = 281.95 [M+H]⁺. Chiral preparative HPLC separation (Chromatographic column: Regis (S,S)Whelk-O 1, 25 x 250 mm x 10 mm; mobile phase: A: n-hexane, B: ethanol; B%: 20%) afforded compounds **168-3A** and **168-3B.** Analytical SFC method (Chromatographic column: Regis (s,s) WHELK-01 (4.6 mmI.D*150 mmL, 5 µm); mobile phase: [A: supercritical carbon dioxide B: methanol (0.05% diethylamine)]; gradient B%: 5%-40%, 15 min), compound **168-3A,** Rt = 7.591 minutes, ee value 99.38%, MS m/z = 281.99 [M+H]⁺; compound **168-3B,** Rt = 9.609 minutes, ee value 92.14%, MS m/z = 281.96 [M+H]⁺.

### Step 3

Compound **168-3A** (70 mg, 248.80 µmol) was weighed and N,N-dimethylformamide (2 mL) was added. Compound **56-1** (269.60 mg, 273.68 µmol), N,N,N,N-tetramethyl-O-(7-azabenzotriazol-1-yl)uronium hexafluorophosphate (141.90 mg, 373.21 µmol), and diisopropylethylamine (96.47 mg, 746.41 µmol) were then added. The mixture was reacted at 25°C for 12 hours. The reaction was quenched by the addition of 10 mL of water. The mixture was extracted with ethyl acetate (10 mL x 3), dried over anhydrous sodium sulfate, and filtered. The filtrate was concentrated under reduced pressure, and separated by column chromatography (petroleum ether:ethyl acetate = 1:1) to obtain compound **168-4A.** MS m/z = 1248.9 [M+H]⁺.

Referring to step 3, compound **168-3B** was used as a starting material in place of compound **168-3A** to obtain compound **168-4B.** MS m/z = 1248.9 [M+H]⁺.

### Step 4

Compound **168-4A** (50 mg, 40.06 µmol) was weighed and trifluoroacetic acid (2 mL) was added. The mixture was stirred at room temperature for 0.5 hours. The reaction mixture was concentrated under reduced pressure to obtain a crude product, which was then separated by preparative HPLC (Chromatographic column: Waters Xbridge, 250 x 19 mm, 5 µm; mobile phase: [water (0.1% ammonia)-acetonitrile]; gradient: (acetonitrile): 5%-50%) to afford compound **168A.** MS m/z = 758.6 [M+H]⁺. ¹H NMR (400 MHz, CD₃OD) δ ppm 6.90 (d, *J* = 8.0 Hz, 1H), 5.18 - 5.11 (m, 1H), 4.80 (d, *J* = 12.0 Hz, 1H), 4.65 (d, *J =* 12.0 Hz, 1H), 4.19 - 4.12 (m, 2H), 4.05 (d, *J =* 8.0 Hz, 1H), 3.77 - 3.64 (m, 5H), 3.61 - 3.49 (m, 5H), 3.45 - 3.39 (m, 1H), 3.37 - 3.33 (m, 1H), 3.30 - 3.16 (m, 3H), 3.04 (d, *J =* 12.0 Hz, 1H), 2.95 - 2.89 (m, 1H), 2.88 - 2.81 (m, 1H), 2.77 - 2.70 (m, 1H), 2.14 - 2.07 (m, 2H), 2.02 (s, 5H), 1.95 - 1.81 (m, 6H), 1.77 - 1.68 (m, 2H), 1.60 - 1.53 (m, 2H).

Compound **168-4B** (110 mg, 88.11 µmol) was weighed. Trifluoroacetic acid (1 mL) and dichloromethane (3 mL) were added, and the mixture was reacted at 25°C for 1 hour. The reaction solution was concentrated under reduced pressure to obtain a crude product, which was then separated by preparative HPLC (Chromatographic column: Waters Xbridge, 250*19 mm, 5 µm; mobile phase: [water (0.1% ammonia)-acetonitrile]; gradient: (acetonitrile): 30%-70%) to afford compound **168B.** MS m/z = 758.5 [M+H]⁺. ¹HNMR (400 MHz, CD₃OD) δ ppm 6.90 (d, *J =* 8.0 Hz, 1H), 5.14 (dd, *J =* 12.0, 4.0 Hz, 1H), 4.81-4.78 (m, 1H), 4.66 - 4.63(m, 1H), 4.18 - 4.13(m, 2H), 4.05 (d, *J = 12.0* Hz, 1H), 3.82 - 3.63 (m, 5H), 3.63 - 3.46 (m, 5H), 3.45 - 3.33 (m, 2H), 3.29 - 3.13 (m, 3H), 3.06 - 3.02 (m, 1H), 2.96-2.67 (m, 3H), 2.15 - 2.06 (m, 2H), 2.02 (s, 3H), 2.00 - 1.81 (m, 8H), 1.77 - 1.64 (m, 2H), 1.60-1.50 (m, 2H).

### Biological test data

### Experimental Example 1. Testing for inhibition of p-ERK in AsPC-1 cells

### 1. Purpose

The inhibitory activity of the compounds on p-ERK levels in pancreatic cancer AsPC-1 cells harboring KRAS^{G12D} mutation was evaluated by HTRF method.

### 2. Experimental Materials:

ASPC-1 cells were purchased from ATCC; RPMI-1640 medium was purchased from GIbco; fetal bovine serum was purchased from Hyclone; Advanced Phospho-ERK1/2 (THR202/TYR204) KIT was purchased from Bioauxilium-

**Table 1. Advanced Phospho-ERK1/2 (THR202/TYR204) KIT Ingredients**

| Ingredient Name | Storage temperature |
|---|---|
| Advanced PhosphoERK1/2 Eu Cryptate antibody | ≤-16 °C |
| Advanced PhosphoERK1/2 d2 antibody | ≤-16 °C |
| Blocking reagent (stock solution 100X) | ≤-16 °C |
| Lysis buffer # 1 (stock solution 4X) | ≤-16 °C |
| Detection buffer (ready-to-use) | ≤-16 °C |

### 3. Experimental Methods

1) ASPC-1 cells were inoculated in a white-bottomed 384-well cell culture plate, with 8 µL of cell suspension per well (7,500 cells per well). The cell plate was placed in a CO₂ incubator and incubated overnight at 37°C;
2) The test compound was diluted with 100% DMSO to 3 mM as the first concentration, and then diluted with a pipette to ten concentrations of 3000, 1000, 300, 100, 30, 10, 3, 1, 0.3, and 0.1 µM. 2 µL of compound was added to 198 µL of cell starvation medium and mixed well. 15 µL of compound solution was added to 35 µL of cell starvation medium and mixed well. 4 µL of the compound solution from the last step was then added to each corresponding well of the cell plate. The cell plate was returned to the carbon dioxide incubator and incubated for 3 hours. At this time, the compound concentrations were 3000, 1000, 300, 100, 30, 10, 3, 1, 0.3, and 0.1 nM;
3) After the incubation, 3 µL of 5X cell lysis buffer was added to each well and the plate was incubated with shaking at room temperature for 30 minutes;
4) Phospho-ERK1/2 Eu Cryptate antibody and Phospho-ERK1/2 d2 antibody were diluted 20-fold with detection buffer and mixed at a 1:1 ratio. 5 µL was added to each well of the cell culture plate and incubated at room temperature for 2 h;
5) After incubation, a multi-label analyzer was used to read HTRF excitation: 320 nm, emission: 615 nm, 665 nm.

### 4. Data Analysis:

Raw data were converted to inhibition ratio using the equation **(Sample-Min)/(Max-Min)*100%.** IC₅₀ values were then derived using a four-parameter curve fit (using the log(inhibitor) vs. response -- Variable slope mode in GraphPad Prism). Table 1 provides the inhibitory effects of the compounds of the present disclosure on p-ERK.
**Max well:** The positive control wells were read as 1X lysate
**Min well:** The negative control wells were read as cell lysate in the 0.5% DMSO cell well

### 5. Experimental Results

The results are shown in Table 2.

**Table 2 IC₅₀ values of compounds for inhibition of p-ERK in AsPC-1 cells**

| **Compound number** | **AsPC-1 p-ERK IC₅₀ (nM)** |
|---|---|
| Hydrochloride of compound **1** | 164 |
| Trifluoroacetate salt of compound **3A** | 28.9 |

Experimental conclusion: The compounds of the present disclosure have a significant inhibitory effect on p-ERK in KRAS^{G12D} mutant AsPC-1 cells.

### Experimental Example 2. Testing for inhibition of p-ERK in AsPC-1 cells

### Experimental Materials:

1640 culture medium and penicillin/streptomycin antibiotics were purchased from Gibco, and fetal bovine serum was purchased from Biosera. CellTiter-Glo (a chemiluminescent cell viability assay reagent) reagent was purchased from Promega. The AsPC-1 cell line was purchased from ATCC, and the Envision Multilabel Analyzer was purchased from PerkinElmer.

### Experimental methods:

AsPC-1 (KRAS^{G12D} mutation, pancreatic cancer) cells were inoculated in an ultra-low attachment 96-well black-wall plate, with 80 µL of cell suspension per well containing 10,000 AsPC-1 cells purchased from ATCC. The plate was incubated overnight in a CO₂ incubator.

The compounds to be tested were diluted five-fold using a pipette to eight concentrations, ranging from 2 mM to 25.6 nM, in duplicate. 78 µL of culture medium was added to the middle plate. 2 µL of the serially diluted compound was then transferred to each well of the corresponding position of the middle plate. After mixing well, 20 µL of the compound was transferred to each well of the cell plate. The concentration of the compound transferred to the cell plate ranged from 10 µM to 0.128 nM. The cell plate was incubated in a CO₂ incubator for 10 days. A separate cell plate was prepared, and the signal value was read on the day of drug addition, which was used as the maximum value (Max value in the equation below) for data analysis.

100 µL of chemiluminescent detection reagent for cell viability was added to each well of the cell plate and incubated at room temperature for 30 minutes to allow the luminescent signal to stabilize. The cells were read using a multilabel analyzer.

### Data Analysis:

Raw data were converted to inhibition ratio using the equation (Sample-Min)/(Max-Min)* 100%. IC₅₀ values were then derived using a four-parameter curve fit (using the "log(inhibitor) vs. responseVariable slope" mode in GraphPad Prism). Table 3 provides the inhibitory activity of the compounds of this disclosure against pERK levels in AsPC-1 cells.

**Table 3 IC₅₀ values of compounds for inhibition of p-ERK in AsPC-1 cells**

| **Compound number** | **AsPC-1 p-ERK IC₅₀ (nM)** |
|---|---|
| Trifluoroacetate salt of compound **5A** | 7.4 |
| Trifluoroacetate salt of compound **7** | 24.9 |
| Trifluoroacetate salt of compound **12** | 35.0 |

Experimental conclusion: The compounds of the present disclosure have a significant inhibitory effect on p-ERK in KRAS^{G12D} mutant AsPC-1 cells.

### Experimental Example 3. p-ERK inhibition test in AsPC-1 and Panc0403 cells

### Experimental methods:

On the first day, ASPC-1 (ATCC, CRL-1682, KRAS^{G12D} mutation, pancreatic cancer) or PANC04.03 (ATCC, CRL-2555, KRAS^{G12D} mutation, pancreatic cancer) cells were plated at 6,000 cells/well in 384-well plate (Corning, 354663) and incubated overnight at 37°C in a 5% CO₂ incubator. On the second day, serial dilutions of compounds were added to the plates (0.1% DMSO) using an echo 655 and incubated at 37°C in 5% CO₂ for 1.5 hours. The plate was fixed with 40 µL of 8% cell tissue fixative for 20 minutes at room temperature. The supernatant was discarded, and the plate was washed twice with PBS for two minutes each. The plate was permeabilized with 40 µL of methanol for 10 minutes at room temperature. The supernatant was discarded, and the plate was washed twice with PBS for two minutes each. The plate was then blocked with 20 µL of fixative for 1 hour at room temperature. The supernatant was discarded. 20 µL of a prepared primary antibody dilution (1:1000 dilution of Phospho-p44/42 MAPK (Erk1/2) (Thr202/Tyr204) (D13.14.4E) XP^{®} Rabbit mAb and 1:2000 dilution of GAPDH (D4C6R) Mouse mAb) was added. The plate was incubated in a refrigerator at 4°C overnight. On the third day, the primary antibody was discarded and the plate was washed three times with 1xPBST for 3 minutes each. A prepared secondary antibody dilution (1:2000 dilution of IRDye 800CW Goat anti-Rabbit IgG (H+L) (0.5 mg) and 1:2000 dilution of IRDye 680RD Goat anti-Mouse IgG (H+L) (0.5 mg)) was added and the plate was incubated at room temperature in the dark for 1 hour. The secondary antibody was discarded and the plate was washed three times with 1xPBST for 3 minutes each. The cells were centrifuged upside down at 1000 rpm for 1 minute and air-dried. pERK and GAPDH signals were quantified using a Li-Cor Odyssey machine at 800 nM and 700 nM, respectively.

### Experimental results

### The results are shown in Table 4.

**Table 4. IC₅₀ values of compounds for inhibition of p-ERK in AsPC-1 and Panc0403 cells**

| Compound number | AsPC-1 p-ERK IC₅₀ (nM) | Panc0403 p-ERK IC₅₀ (nM) |
|---|---|---|
| Trifluoroacetate salt of compound **4B** | 5.6 | 9.0 |
| Trifluoroacetate salt of compound **9B** | 12 | 21.1 |
| Trifluoroacetate salt of compound **10** | 9.3 | 19.7 |
| Trifluoroacetate salt of compound **13A** | 6.1 | 7.8 |
| Trifluoroacetate salt of compound **14B** | 19.4 | 23.9 |
| Trifluoroacetate salt of compound **15B** | 5.3 | 5.0 |
| Compound **16D** | 1.9 | 4.5 |
| Compound **17D** | 22.4 | 23.8 |
| Compound **18D** | 3.2 | 6.9 |
| Formate salt of compound **19D** | 1.1 | 3.6 |
| Compound **20D** | 0.4 | 1 |
| Formate salt of compound **21D** | 10.9 | 13.7 |
| Compound **22D** | 1.3 | 4.2 |
| Compound **23D** | 0.6 | 2.1 |
| Formate salt of compound **24D** | 4.7 | 8.2 |
| Compound **25B** | 110 | 121 |
| Compound **27D** | 6.2 | 12.6 |
| Hydrochloride of compound **28B** | 35.1 | 33.3 |
| Formate salt of compound **29** | 43.8 | 27.0 |
| Compound **30** | >1000 | 592.0 |
| Compound **31** | 190.0 | 335.0 |
| Hydrochloride of compound 32 | 628.0 | 371.2 |
| Compound 33A | 305.0 | 157.7 |
| Compound 33B | 273.0 | 84.2 |
| Compound 34 | 560.0 | 257.3 |
| Hydrochloride of compound 35A | 113.0 | 50.9 |
| Hydrochloride of compound 35B | 42.0 | 11.9 |
| Formate salt of compound 36B | 44.7 | 98.7 |
| Formate salt of compound 36C | 30.3 | 72.2 |
| Formate salt of compound 36D | 6.2 | 12.4 |
| Hydrochloride of compound 37A | 251.4 | 576.6 |
| Hydrochloride of compound 37B | 31.0 | 61.4 |
| Hydrochloride of compound 38 | 3.7 | 4.1 |
| Formate salt of compound 39A | 77.7 | 162.0 |
| Formate salt of compound 39B | 1066.9 | 1932.8 |
| Compound 39C | 12.0 | 22.7 |
| Compound 40A | 2166.1 | 3898.3 |
| Formate salt of compound 40B | 564.3 | 1007.1 |
| Compound 41C | 1356.6 | 1847.7 |
| Formate salt of compound 41D | 274.0 | 392.9 |
| Formate salt of compound 42D | 25.9 | 44.7 |
| Compound 43 | 5741.0 | 8645.0 |
| Compound 44A | 137.5 | 114.6 |
| Formate salt of compound 44B | 2802.0 | 523.9 |
| Compound 45 | >1000 | >10000 |
| Hydrochloride of compound 46 | 1162.0 | 331.8 |
| Compound 47 | 142.0 | 57.4 |
| Formate salt of compound 48 | 600.6 | 2179.4 |
| Compound 49 | 100.3 | 135.8 |
| Formate salt of compound 50 | 52.0 | 132.6 |
| Compound 51 | 2959.7 | >10000 |
| Hydrochloride of compound 52 | 179.6 | 437.3 |
| Formate salt of compound 53A | 33.9 | 34.6 |
| Formate salt of compound 53B | 1143.2 | 1396.3 |
| Compound 54A | 557.7 | 457.7 |
| Formate salt of compound 54B | 9284.9 | >10000 |
| Formate salt of compound 55 | 5449.9 | 9852.7 |
| Compound 58 | 1.2 | 2.6 |
| Compound 59 | 1.4 | 2.9 |
| Compound 60 | 2789.2 | 4865.6 |
| Compound 61 | 1115.5 | 1732.5 |
| Compound 42C | 249.6 | 399.7 |
| Compound 62 | 0.9 | 1.3 |
| Hydrochloride of compound 63 | 19.6 | 33.1 |
| Compound 64 | 95.2 | 285.1 |
| Compound 65 | 8.5 | 19.0 |
| Formate salt of compound 66 | 3140.8 | 6966.0 |
| Compound 67 | 5767.6 | >10000 |
| Compound 68 | 0.7 | 1.8 |
| Compound 69 | 16.5 | 21.4 |
| Formate salt of compound 70A1 | 5600.4 | >10000 |
| Formate salt of compound 70A2 | >10000 | >10000 |
| Formate salt of compound 70B1 | 559.4 | 1220.6 |
| Formate salt of compound 70B2 | 866.5 | 1977.1 |
| Compound 70C | 10.1 | 15.8 |
| Compound 71 | 3179.4 | >10000 |
| Compound 72 | 54.5 | 124.6 |
| Compound 73 | 2.4 | 4.0 |
| Compound 74 | 2.1 | 3.5 |
| Compound 75 | 33.8 | 63.5 |
| Compound 76 | 0.9 | 1.8 |
| Compound 77 | 0.7 | 1.4 |
| Compound 78 | 0.9 | 1.8 |
| Compound 79 | 6.9 | 16.1 |
| Compound 80 | 1.6 | 3.6 |
| Compound 81 | 3.8 | 5.3 |
| Compound 82B | 1.0 | 1.8 |
| Compound 82A | 37.8 | 94.6 |
| Compound 83 | 0.7 | 1.4 |
| Compound 84 | 6.3 | 13.6 |
| Formate salt of compound 85 | 1855.5 | 3770.3 |
| Compound 86 | 12.1 | 17.5 |
| Compound 87 | 212.4 | 338.9 |
| Compound 88 | 1.7 | 2.9 |
| Compound 89 | 0.9 | 1.4 |
| Compound 90 | 1.6 | 2.3 |
| Compound 91 | 5366.1 | 5439.3 |
| Compound 92 | 933.3 | 1489.4 |
| Compound 93 | 51.0 | 60.9 |
| Compound 94 | 294.4 | 448.6 |
| Compound 95 | 2.6 | 4.8 |
| Compound 96A | 1.5 | 2.8 |
| Compound 96B | 3.0 | 3.3 |
| Compound 97 | 3.3 | 4.5 |
| Compound 98 | >10000 | >10000 |
| Compound 99 | >10000 | >10000 |
| Compound 100A | 1078.2 | 1642.3 |
| Compound 100B | 7.6 | 6.3 |
| Compound 101 | 2563.1 | 3108.3 |
| Compound 102 | 2.4 | 2.7 |
| Compound 57 | 1.2 | 1.6 |
| Compound 103 | 28.5 | 28.3 |
| Compound 104 | 3.4 | 3.3 |
| Compound 105 | 4.2 | 4.1 |
| Compound 106 | 3.1 | 3.7 |
| Formate salt of compound 107 | 3.4 | 7.1 |
| Compound 108 | 5.0 | 7.5 |
| Compound 109B | 1.9 | 2.8 |
| Compound 109A | 235.3 | 658.2 |
| Compound 110 | 695.9 | 1405.3 |
| Compound 111 | 2.5 | 4.3 |
| Compound 112 | 74.6 | 131.7 |
| Compound 113 | 1.2 | 2.2 |
| Compound 56 | 0.5 | 1.2 |
| Compound 114 | 0.6 | 1.1 |
| Compound 115 | 564.7 | 1091.8 |
| Compound 116 | 5.2 | 14.2 |
| Compound 117 | 3.1 | 4.6 |
| Compound 118 | 114.5 | 169.5 |
| Compound 119A | 2236.8 | 9032.0 |
| Compound 119B | 2996.5 | >10000 |
| Compound 120A | 12.0 | 37.7 |
| Compound 120B | 2.0 | 5.0 |
| Compound 121 | 55.2 | 75.4 |
| Formate salt of compound 122A | 73.9 | 94.9 |
| Formate salt of compound 122B | 59.7 | 54.4 |
| Formate salt of compound 122C | 90.4 | 123.5 |
| Formate salt of compound 122D | 9.0 | 11.1 |
| Compound 123 | 934.3 | 1703.1 |
| Compound 124A | 172.5 | 344.7 |
| Compound 124B | 42.8 | 39.5 |
| Formate salt of compound 125 | 7.6 | 7.3 |
| Compound 126 | 2.0 | 2.9 |
| Compound 127 | 1.3 | 1.4 |
| Compound 128 | 2.2 | 2.4 |
| Compound 129 | 77.4 | 124.7 |
| Compound 130 | 4.3 | 5.0 |
| Compound 131 | 14.8 | 28.6 |
| Compound 132 | 2.0 | 5.1 |
| Compound 133 | 9.9 | 21.0 |
| Compound 134 | 15.6 | 41.6 |
| Compound 135 | 24.8 | 45.6 |
| Compound 136 | 2.7 | 5.1 |
| Compound 137 | 649.2 | 1300.6 |
| Compound 138 | 4.5 | 12.5 |
| Compound 139 | 4.3 | 11.4 |
| Formate salt of compound 140A | 30.3 | 57.7 |
| Formate salt of compound 140B | 60.2 | 113.7 |
| Compound 141 | 0.6 | 1.2 |
| Compound 142 | 0.4 | 0.9 |
| Compound 143 | 2.1 | 4.2 |
| Compound 144 | 2.0 | 5.3 |
| Compound 145 | 0.3 | 0.7 |
| Compound 146 | 0.3 | 0.7 |
| Compound 147 | 3.4 | 10.0 |
| Compound 148 | 162.2 | 266.1 |
| Compound 149 | >10000 | >10000 |
| Compound 150 | 1.2 | 2.8 |
| Compound 151 | 0.5 | 1.3 |
| Compound 152 | 39.8 | 61.6 |
| Compound 153 | 0.6 | 1.4 |
| Compound 154 | 0.7 | 1.5 |
| Compound 155 | 4.8 | 11.0 |
| Compound 156A | 0.4 | 0.8 |
| Compound 157 | 1.9 | 3.6 |
| Compound 158A | 0.5 | 1.1 |
| Compound 159 | 2.2 | 3.6 |
| Compound 160 | 0.7 | 1.5 |
| Compound 161 | 0.8 | 2.4 |
| Compound 162A | 2.1 | 4.5 |
| Compound 162D | 2.1 | 4.7 |
| Compound 163 | 0.3 | 0.7 |
| Compound 164 | 12.2 | 30.9 |
| Compound 165 | 6.4 | 15.4 |
| Formate salt of compound 166 | 1.4 | 2.9 |
| Compound 167 | 62.8 | 122.6 |
| Compound 168A | 4.4 | 8.2 |
| Compound 168B | 4.0 | 8.5 |
| Compound 156B | 0.4 | 0.7 |
| Compound 158B | 0.7 | 1.1 |
| Compound 162B | 1.8 | 2.0 |
| Compound 162C | 3.1 | 4.1 |

Experimental conclusion: The compounds of the present disclosure have a significant inhibitory effect on p-ERK in AsPC-1 and Panc0403 cells with KRAS^{G12D} mutation.

### Experimental Example 4. Test of Inhibition on the Proliferation of AsPC-1 and Panc0403 cells

### Experimental methods:

ASPC-1 (ATCC, CRL-1682, KRAS^{G12D} mutation, pancreatic cancer) and PANC04.03 (ATCC, CRL-2555, KRAS^{G12D} mutation, pancreatic cancer) cells were inoculated at a density of 2000 cells/well/195 µL in a CellCarrier-96 ULA/CS plate (PE, 6055330). The diluted compounds were added to the cell plate and cultured in a 37°C cell culture incubator for 7 days. The CellTiter-Glo^{®} 3D Assay Kit (3D-CTG, Promega, G9683) was placed together with cells at room temperature for 30 min. An equal volume of CTG was then added to the plate and shaken for 20 minutes for cell lysis. The cells were placed at room temperature for 1 h, and the luminescence signal was read using BMG (PHERAstar FSX). The percentage of viability of cells in wells treated with compounds was calculated between the control and blank groups. Wells containing cell culture medium served as blank groups, and wells containing cells and the same percentage of DMSO served as control groups.

Inhibition rate (%) = (luminescence reading of control group - luminescence reading of compound-treated wells) / (luminescence reading of control group - luminescence reading of blank group) * 100%; then, data were analyzed by fitting a 4-parameter logistic model or Excel to calculate IC₅₀ values, which are the concentration on the curve that achieves 50% inhibition.

### Experimental results

The results are shown in Table 5.

**Table 5 IC₅₀ values of compounds for inhibition of AsPC-1 and Panc0403 cell proliferation**

| **Compound number** | **AsPC-1 IC₅₀ (nM)** | **Panc0403 IC₅₀ (nM)** |
|---|---|---|
| Trifluoroacetate salt of compound **4B** | 34.3 | 295 |
| Trifluoroacetate salt of compound **9B** | 48.5 | 409 |
| Trifluoroacetate salt of compound **10** | 55.7 | 415 |
| Trifluoroacetate salt of compound **13A** | 20.7 | 142 |
| Trifluoroacetate salt of compound **14B** | 96.5 | 414 |
| Trifluoroacetate salt of compound **15B** | 6.0 | 97 |
| Compound **16D** | 2.3 | 15.5 |
| Compound **17D** | 67.3 | 644 |
| Compound **18D** | 2.8 | 12.6 |
| Formate salt of compound **19D** | 2.9 | 7.9 |
| Compound **20D** | 0.7 | 3.7 |
| Formate salt of compound **21D** | 2.1 | 6.8 |
| Compound **22D** | 3.4 | 14.1 |
| Compound **23D** | 1.7 | 5.3 |
| Formate salt of compound **24D** | 6.3 | 54 |
| Compound **25B** | 124 | |
| Compound **27D** | 8.7 | 71.4 |
| Hydrochloride of compound **28B** | 69.8 | 124 |

Experimental conclusion: The compounds of the present disclosure have significant anti-proliferation inhibitory effects on AsPC-1 and Panc0403 cells with KRAS^{G12D} mutation.

## Claims

1. A compound represented by formula (I") or a stereoisomer thereof or a pharmaceutically acceptable salt thereof: 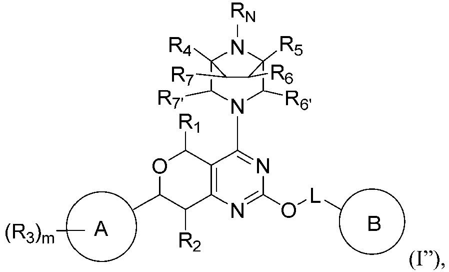 wherein,
R_{N} is selected from H and C₁₋₃ alkyl, wherein the C₁₋₃ alkyl is optionally substituted with 1, 2 or 3 F or Cl; Ring A is selected from C₆ aryl and 5- to 6-membered heteroaryl;
Ring B is selected from 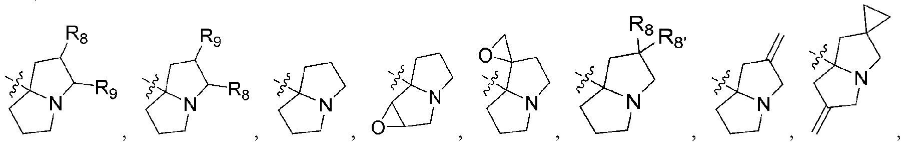 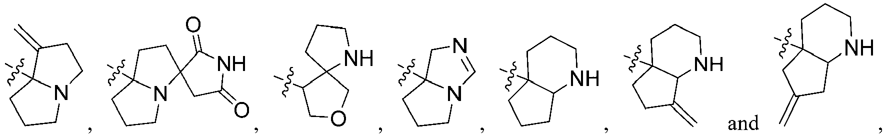
wherein ring B is optionally substituted with 1, 2, 3 or 4 R₁₀;
L is selected from -C(R_{L1}R_{L2})-, wherein R_{L1} and R_{L2} are each independently selected from H, D and C₁₋₃ alkyl; R₁ and R₂ are each independently selected from oxo, H, F, Cl, Br, I and CN;
each R₃ is independently selected from F, Cl, Br, I, OH, NH₂, CN, C₁₋₃ alkyl, C₁₋₃ alkoxy, C₁₋₃ alkylamino, di-C₁₋₃ alkylamino, C₂₋₄ alkenyl, C₂₋₄ alkynyl and C₃₋₅ cycloalkyl, wherein the C₁₋₃ alkyl, C₁₋₃ alkoxy, C₁₋₃ alkylamino, di-C₁₋₃ alkylamino, C₂₋₄ alkenyl, C₂₋₄ alkynyl and C₃₋₅ cycloalkyl are each independently optionally substituted with 1, 2, 3, 4 or 5 Rₐ;
each Rₐ is independently selected from D, F, Cl, Br and I;
R₄, R₅, R₆, R₇, R_{6'} and R_{7'} are each independently selected from oxo, H, F, Cl, Br, I, OH, NH₂, CN, C₁₋₃ alkyl, C₂₋₄ alkenyl, C₁₋₃ alkoxy, -C(=O)-R_{d}, -C(=O)-NR_{b1}R_{b2} and =NO(C₁₋₃ alkyl), wherein the C₁₋₃ alkyl, C₂₋₄ alkenyl and C₁₋₃ alkoxy are each independently optionally substituted with 1, 2, 3, 4 or 5 R_{b};
alternatively, R₆ and R₇ together with the carbon atoms to which they are attached form a 3- to 5-membered heterocycloalkyl group;
each R_{b} is independently selected from D, F, Cl, Br, I, OH, NH₂, CN, C₁₋₃ alkoxy, and -C(=O)-NR_{b1}R_{b2};
R₈ is selected from H, F, Cl, Br, I, OH, NH₂, CN, C₁₋₃ alkyl and C₁₋₃ alkoxy, wherein the C₁₋₃ alkyl and C₁₋₃ alkoxy are each independently optionally substituted with 1, 2, 3, 4 or 5 Rₐ;
alternatively, R₈ and R_{8'} together with the carbon atom to which they are attached form a C₃₋₅ cycloalkyl or a 3-to 5-membered heterocycloalkyl, wherein the C₃₋₅ cycloalkyl and the 3- to 5-membered heterocycloalkyl are each independently optionally substituted with 1, 2 or 3 R₁₀;
R₉ is selected from -C(=O)-NR_{b3}R_{b4} and -CH₂R_{c};
each R₁₀ is independently selected from oxo, D, F, Cl, Br, I, OH, NH₂, CN, C₁₋₃ alkyl, C₁₋₃ alkoxy, C₁₋₃ alkylamino, di-C₁₋₃ alkylamino, -C(=O)-Rₐ, -S-R_{d}, -S(=O)-Rₐ, -S(=O)₂-Rₐ, -NH-C(=O)-R_{d}, C₆₋₁₀ aryl, and 5- to 10-membered heteroaryl, wherein the C₁₋₃ alkyl is optionally substituted with 1, 2, or 3 OH or F, and the C₆₋₁₀ aryl and 5- to 10-membered heteroaryl are each independently optionally substituted with 1, 2, 3, 4, or 5 Rₛ₁; R_{b1} and R_{b2} are each independently selected from H, C₁₋₆ alkyl, C₁₋₆ alkoxy, C₃₋₁₀ cycloalkyl, 3- to 10-membered heterocycloalkyl, C₆₋₁₀ aryl and 5- to 10-membered heteroaryl, wherein the C₁₋₆ alkyl, C₁₋₆ alkoxy, C₃₋₁₀ cycloalkyl, 3- to 10-membered heterocycloalkyl, C₆₋₁₀ aryl and 5- to 10-membered heteroaryl are each independently optionally substituted with 1, 2, 3 or 4 Rₑ₁;
alternatively, R_{b1} and R_{b2} together with the nitrogen atom to which they are attached form a 3- to 6-membered heterocycloalkyl group, wherein the 3- to 6-membered heterocycloalkyl group is optionally substituted with 1, 2, 3 or 4 Rₑ₁ groups;
R_{b3} and R_{b4} are each independently selected from H, C₁₋₆ alkyl, C₁₋₆ alkoxy, C₃₋₁₀ cycloalkyl, 3- to 10-membered heterocycloalkyl, C₆₋₁₀ aryl and 5- to 10-membered heteroaryl, wherein the C₁₋₆ alkyl, C₁₋₆ alkoxy, C₃₋₁₀ cycloalkyl, 3- to 10-membered heterocycloalkyl, C₆₋₁₀ aryl and 5- to 10-membered heteroaryl are each independently optionally substituted with 1, 2, 3 or 4 Rₑ₂;
alternatively, R_{b3} and R_{b4} together with the nitrogen atom to which they are attached form a 3- to 6-membered heterocycloalkyl group, wherein the 3- to 6-membered heterocycloalkyl group is optionally substituted with 1, 2, 3 or 4 Rₑ₂ groups;
R_{c} is selected from F, Cl, Br, I, OH, NH₂, -(C=O)NR_{C1}R_{C2}, -O(C=O)NR_{C1}R_{C2}, -NR_{C0}(C=O)R_{C1} and - NR_{C0}(C=O)NR_{C1}R_{C2};
R_{C0}, R_{C1} and R_{C2} are each independently selected from H, C₁₋₆ alkyl, C₃₋₆ cycloalkyl and 3- to 6-membered heterocycloalkyl;
R_{d} is selected from C₁₋₃ alkyl;
Rₑ₁ is selected from F, Cl, Br, I, OH, NH₂, NO₂, C₁₋₃ alkyl, C₁₋₃ alkylamino, di-C₁₋₃ alkylamino, CN, C₁₋₃ alkoxy, -S(=O)₂-(C₁₋₃ alkyl), -(C=O)(C₁₋₃ alkyl), -(C=O)O(C₁₋₃ alkyl), -(C=O)NH(C₁₋₃ alkyl), -(C=O)N(C₁₋₃ alkyl)₂, C₃₋₁₀ cycloalkyl, 3- to 10-membered heterocycloalkyl, C₆₋₁₀ aryl and 5- to 10-membered heteroaryl;
Rₑ₂ is selected from F, Cl, Br, I, OH, NH₂, NO₂, C₁₋₃ alkyl, C₁₋₃ alkylamino, di-C₁₋₃ alkylamino, CN, C₁₋₃ alkoxy, -S(=O)₂-(C₁₋₃ alkyl), -(C=O)(C₁₋₃ alkyl), -(C=O)O(C₁₋₃ alkyl), -(C=O)NH(C₁₋₃ alkyl), -(C=O)N(C₁₋₃ alkyl)₂, C₃₋₁₀ cycloalkyl, 3- to 10-membered heterocycloalkyl, C₆₋₁₀ aryl and 5- to 10-membered heteroaryl, wherein the C₃₋₁₀ cycloalkyl, 3- to 10-membered heterocycloalkyl, C₆₋₁₀ aryl and 5- to 10-membered heteroaryl are each independently optionally substituted with 1, 2, 3, 4 or 5 Rₛ₁, and wherein the C₁₋₃ alkyl and C₁₋₃ alkoxy are each independently optionally substituted with 1, 2, 3, 4 or 5 Rₛ₂;
alternatively, two or more Rₑ₂ together with the carbon atom (s) to which they are attached form a C₆ aryl group or a 5- or 6 -membered heteroaryl group;
Rₛ₁ is selected from oxo, F, Cl, Br, I, OH, NH₂, NO₂, C₁₋₆ alkyl, C₁₋₆ alkylamino, di-C₁₋₆ alkylamino, CN, C₁₋₆ alkoxy, -S(=O)₂-(C₁₋₃ alkyl), -(C=O)(C₁₋₃ alkyl), -(C=O)O(C₁₋₃ alkyl), -(C=O)NH(C₁₋₃ alkyl) and -(C=O)N(C₁₋₃ alkyl)₂;
Rₛ₂ is selected from F, Cl, Br, I, OH, NH₂, C₁₋₆ alkylamino, di-C₁₋₆ alkylamino, CN, C₁₋₆ alkoxy, -S(=O)₂-(C₁₋₃ alkyl), -(C=O)(C₁₋₃ alkyl), -(C=O)O(C₁₋₃ alkyl), -(C=O)NH(C₁₋₃ alkyl) and -(C=O)N(C₁₋₃ alkyl)₂; and
m is selected from 0, 1, 2, 3, 4 and 5;
with the proviso that,
1) when ring B is selected from 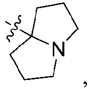 wherein the 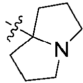 is substituted with one R₁₀, and R₁₀ is F, at least one of R₁, R₂, R₄, R₅, R₆, R₇, R_{6'} and R_{7'} is not H;
2) when ring B is selected from 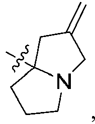 wherein the 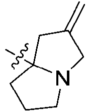 is optionally substituted with 1, 2, 3 or 4 R₁₀, at least one of R₁, R₂, R₄, R₅, R₆, R₇, R_{6'} and R_{7'} is not H; and
3) the compound is not

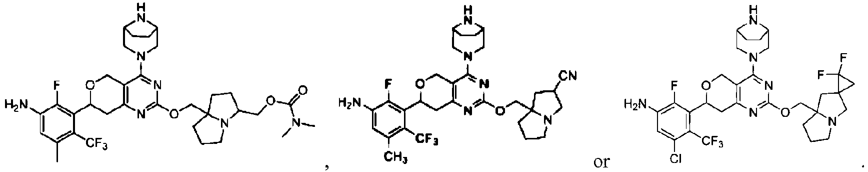

2. The compound according to claim 1, or a stereoisomer thereof, or a pharmaceutically acceptable salt thereof, wherein R_{N} is H.

3. The compound according to claim 1, or a stereoisomer thereof, or a pharmaceutically acceptable salt thereof, wherein R_{N} is a C₁₋₃ alkyl group optionally substituted with 1, 2 or 3 F or Cl groups.

4. A compound represented by formula (I') or a stereoisomer thereof or a pharmaceutically acceptable salt thereof: 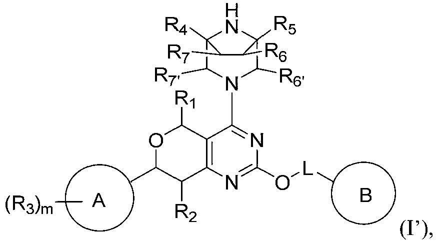 wherein,
Ring A is selected from C₆ aryl and 5- to 6-membered heteroaryl;
Ring B is selected from 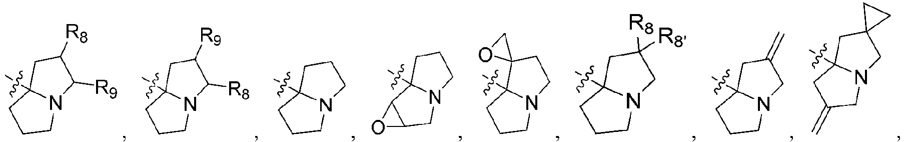 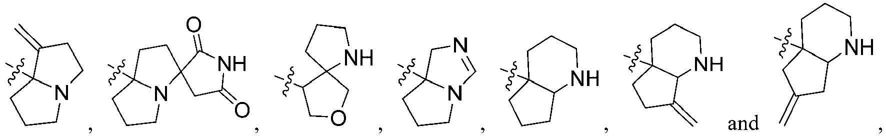
wherein ring B is optionally substituted with 1, 2, 3 or 4 R₁₀;
L is selected from -CH₂-, wherein the -CH₂- is optionally substituted with 1 or 2 D;
R₁ and R₂ are each independently selected from oxo, H, F, Cl, Br, I and CN;
each R₃ is independently selected from F, Cl, Br, I, OH, NH₂, CN, C₁₋₃ alkyl, C₁₋₃ alkoxy, C₁₋₃ alkylamino, di-C₁₋₃ alkylamino, C₂₋₄ alkenyl, C₂₋₄ alkynyl and C₃₋₅ cycloalkyl, wherein the C₁₋₃ alkyl, C₁₋₃ alkoxy, C₁₋₃ alkylamino, di-C₁₋₃ alkylamino, C₂₋₄ alkenyl, C₂₋₄ alkynyl and C₃₋₅ cycloalkyl are each independently optionally substituted with 1, 2, 3, 4 or 5 Rₐ;
each Rₐ is independently selected from D, F, Cl, Br and I;
R₄, R₅, R₆, R₇, R_{6'} and R_{7'} are each independently selected from oxo, H, F, Cl, Br, I, OH, NH₂, CN, C₁₋₃ alkyl, C₂₋₄ alkenyl, C₁₋₃ alkoxy, -C(=O)-R_{d}, -C(=O)-NR_{b1}R_{b2} and =NO(C₁₋₃ alkyl), wherein the C₁₋₃ alkyl, C₂₋₄ alkenyl and C₁₋₃ alkoxy are each independently optionally substituted with 1, 2, 3, 4 or 5 R_{b};
alternatively, R₆ and R₇ together with the carbon atoms to which they are attached form a 3- to 5-membered heterocycloalkyl group;
each R_{b} is independently selected from D, F, Cl, Br, I, OH, NH₂, CN, C₁₋₃ alkoxy, and -C(=O)-NR_{b1}R_{b2};
R₈ is selected from H, F, Cl, Br, I, OH, NH₂, CN, C₁₋₃ alkyl and C₁₋₃ alkoxy, wherein the C₁₋₃ alkyl and C₁₋₃ alkoxy are each independently optionally substituted with 1, 2, 3, 4 or 5 Rₐ;
alternatively, R₈ and R_{8'} together with the carbon atom to which they are attached form a C₃₋₅ cycloalkyl or a 3-to 5-membered heterocycloalkyl, wherein the C₃₋₅ cycloalkyl and the 3- to 5-membered heterocycloalkyl are each independently optionally substituted with 1, 2 or 3 R₁₀;
R₉ is selected from -C(=O)-NR_{b1}R_{b2} and -CH₂R_{c};
each R₁₀ is independently selected from oxo, D, F, Cl, Br, I, OH, NH₂, CN, C₁₋₃ alkyl, C₁₋₃ alkoxy, C₁₋₃ alkylamino, di-C₁₋₃ alkylamino, -S-R_{d}, -S(=O)-R_{d}, -S(=O)₂-R_{d} and -NH-C(=O)-R_{d}, wherein the C₁₋₃ alkyl is optionally substituted with 1, 2 or 3 OH groups;
R_{b1} and R_{b2} are each independently selected from H, C₁₋₆ alkyl, C₃₋₁₀ cycloalkyl, 3- to 10-membered heterocycloalkyl, C₆₋₁₀ aryl and 5- to 10-membered heteroaryl, wherein the C₁₋₆ alkyl, C₃₋₁₀ cycloalkyl, 3- to 10-membered heterocycloalkyl, C₆₋₁₀ aryl and 5- to 10-membered heteroaryl are each independently optionally substituted with 1, 2 or 3 Rₑ₁;
alternatively, R_{b1} and R_{b2} together with the nitrogen atom to which they are attached form a 3- to 6-membered heterocycloalkyl group;
R_{c} is selected from F, Cl, Br, I, OH, NH₂, -O(C=O)NR_{C1}R_{C2}, -NR_{C0}(C=O)R_{C1} and -NR_{C0}(C=O)NR_{C1}R_{C2};
R_{C0}, R_{C1} and R_{C2} are each independently selected from H, C₁₋₆ alkyl, C₃₋₆ cycloalkyl and 3- to 6-membered heterocycloalkyl;
R_{d} is selected from C₁₋₃ alkyl;
Rₑ₁ is selected from F, Cl, Br, I, OH, NH₂, C₁₋₃ alkylamino, di-C₁₋₃ alkylamino, CN, C₁₋₃ alkoxy, C₃₋₁₀ cycloalkyl, 3- to 10-membered heterocycloalkyl, C₆₋₁₀ aryl, and 5- to 10-membered heteroary; and
m is selected from 0, 1, 2, 3, 4 and 5;
with the proviso that,
1) when ring B is selected from 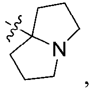 wherein the 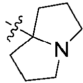 is substituted with one R₁₀, and R₁₀ is F, at least one of R₁, R₂, R₄, R₅, R₆, R₇, R_{6'} and R_{7'} is not H;
2) when ring B is selected from 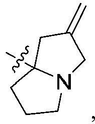 wherein the 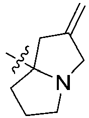 is optionally substituted with 1, 2, 3 or 4 R₁₀, at least one of R₁, R₂, R₄, R₅, R₆, R₇, R_{6'} and R_{7'} is not H; and
3) the compound is not

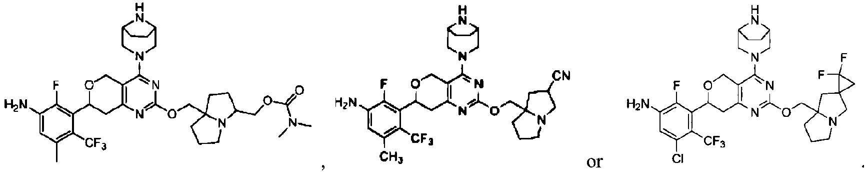

5. The compound according to any one of claims 1 to 4, or a stereoisomer thereof, or a pharmaceutically acceptable salt thereof, wherein ring A is selected from C₆ aryl.

6. The compound according to any one of claims 1 to 4, or a stereoisomer thereof, or a pharmaceutically acceptable salt thereof, wherein ring A is selected from a 5-membered heteroaryl group.

7. The compound according to any one of claims 1 to 6, or a stereoisomer thereof, or a pharmaceutically acceptable salt thereof, wherein ring B is selected from 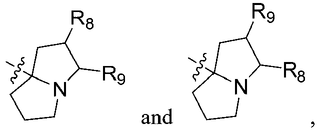 which are each optionally substituted with 1, 2, 3 or 4 R₁₀.

8. The compound according to any one of claims 1 to 7, or a stereoisomer thereof, or a pharmaceutically acceptable salt thereof, wherein ring B is selected from 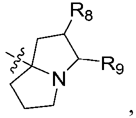 which is optionally substituted with 1, 2, 3 or 4 R₁₀.

9. The compound according to claim 8, or a stereoisomer thereof, or a pharmaceutically acceptable salt thereof, which is selected from a compound of formula (I'-1-i), or a stereoisomer thereof, or a pharmaceutically acceptable salt thereof,

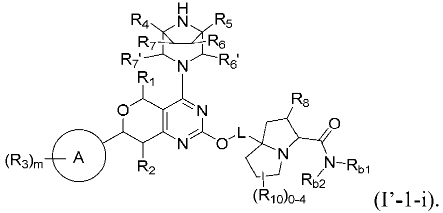

10. The compound according to claim 8, or a stereoisomer thereof, or a pharmaceutically acceptable salt thereof, which is selected from a compound of formula (I'-2-i), a stereoisomer thereof, or a pharmaceutically acceptable salt thereof,

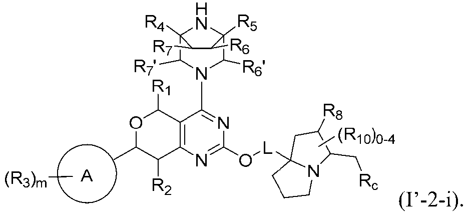

11. The compound according to any one of claims 1 to 7, or a stereoisomer thereof, or a pharmaceutically acceptable salt thereof, wherein ring B is 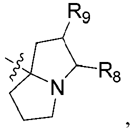 which is optionally substituted with 1, 2, 3 or 4 R₁₀.

12. The compound according to claim 11, or a stereoisomer thereof, or a pharmaceutically acceptable salt thereof, which is selected from a compound of formula (I'-1-ii), or a stereoisomer thereof, or a pharmaceutically acceptable salt thereof,

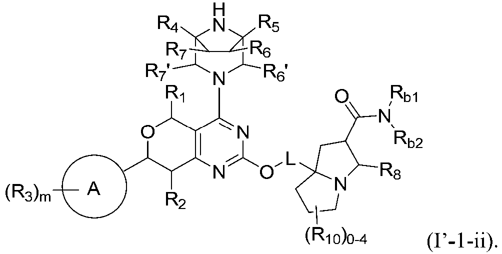

13. The compound according to claim 11, or a stereoisomer thereof, or a pharmaceutically acceptable salt thereof, which is selected from a compound of formula (I'-2-ii), or a stereoisomer thereof, or a pharmaceutically acceptable salt thereof,

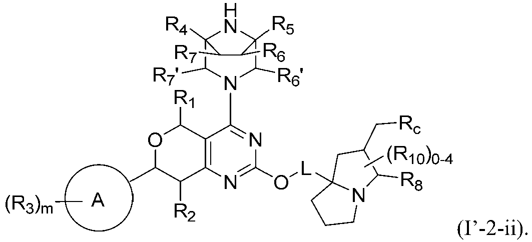

14. The compound according to any one of claims 1 to 6, or a stereoisomer thereof, or a pharmaceutically acceptable salt thereof, wherein ring B is selected from 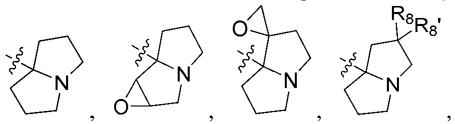 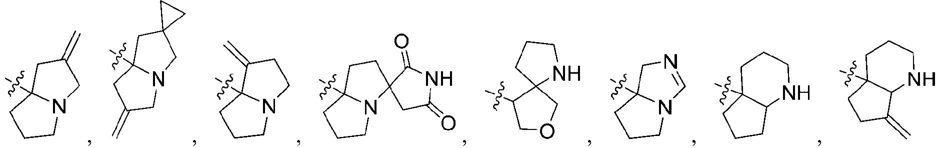 and 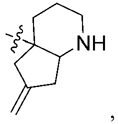 which are optionally substituted with 1, 2, 3 or 4 R₁₀.

15. The compound according to claim 14, or a stereoisomer thereof, or a pharmaceutically acceptable salt thereof, which is selected from a compound of formulas (I'-3), (I'-4), (I'-5), (I'-6), (I'-7), (I'-8), (I'-9), (I'-10), (I'-11), (I'-12) and (I'-13), or a stereoisomer thereof, or a pharmaceutically acceptable salt thereof, 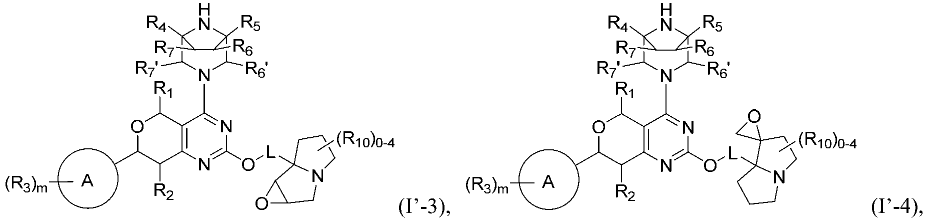 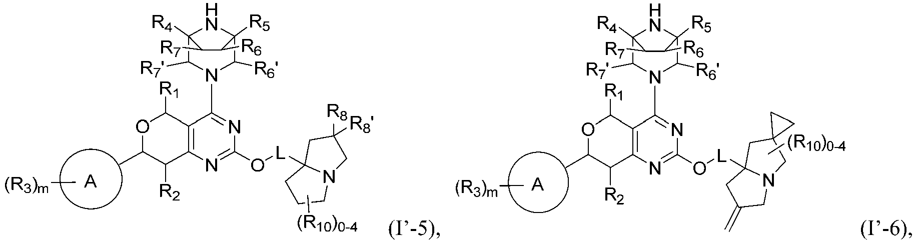 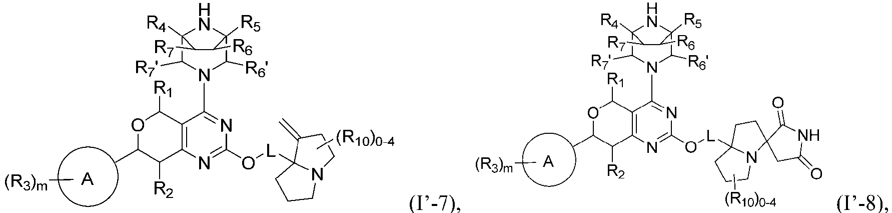 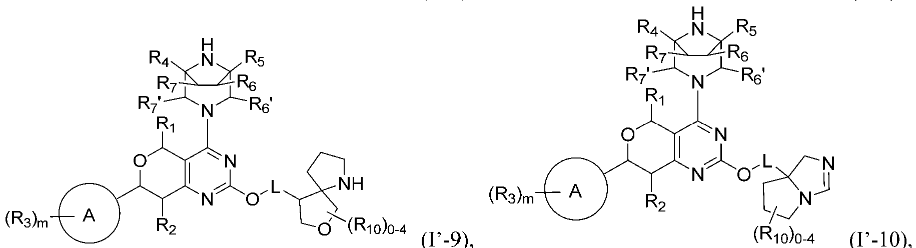 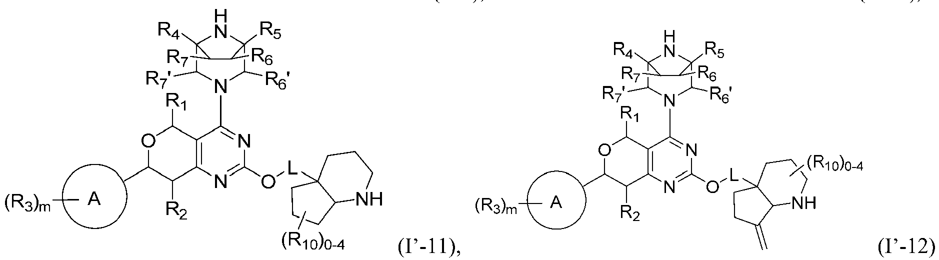 and

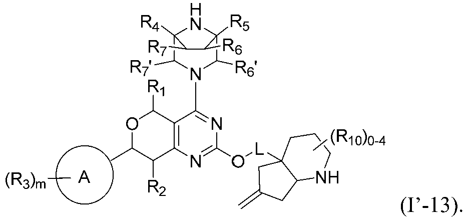

16. The compound according to any one of claims 7 to 13 and 15, or a stereoisomer thereof, or a pharmaceutically acceptable salt thereof, wherein R₄, R₅, R₆, R₇, R_{6'} and R_{7'} are selected from H.

17. The compound according to claim 14, or a stereoisomer thereof, or a pharmaceutically acceptable salt thereof, which is selected from a compound of formulas (I'-14) and (I'-15), or a stereoisomer thereof, or a pharmaceutically acceptable salt thereof,

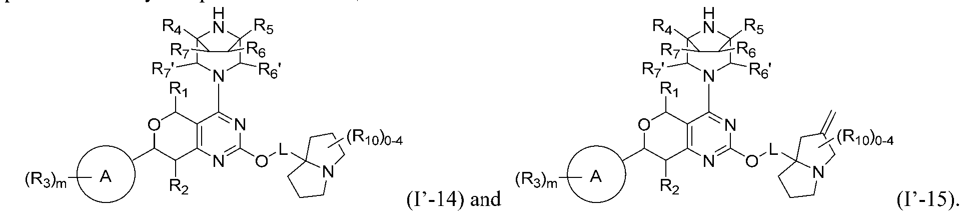

18. The compound according to any one of claims 1 to 5 and 7 to 17, or a stereoisomer thereof, or a pharmaceutically acceptable salt thereof, wherein ring A is phenyl and is substituted with at least one R₃, each R₃ being independently selected from F, OH, NH₂, CF₃, OCH₃, -C≡CCH₃, -C≡CCH₂F, -C≡CCHF₂, -C≡CCF₃, -C≡CCD₃, -C≡CH, -C≡CCl, -C≡CF, and cyclopropyl.

19. The compound according to any one of claims 1 to 5 and 7 to 17, or a stereoisomer thereof, or a pharmaceutically acceptable salt thereof, wherein 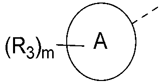 is selected from

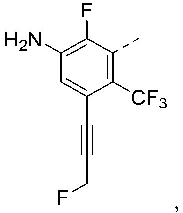

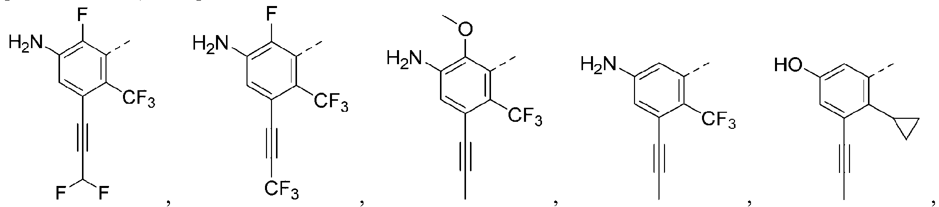

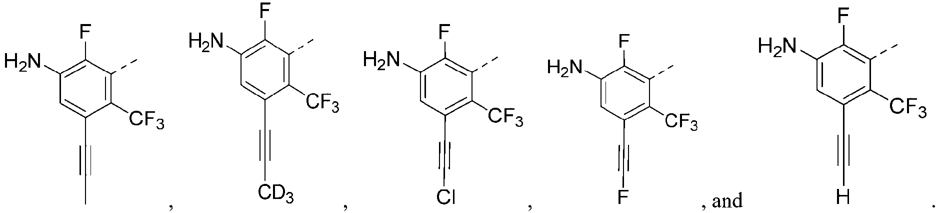

20. The compound according to any one of claims 1 to 5 and 7 to 17, or a stereoisomer thereof, or a pharmaceutically acceptable salt thereof, wherein 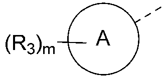 is selected from

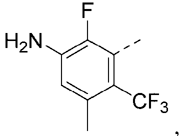

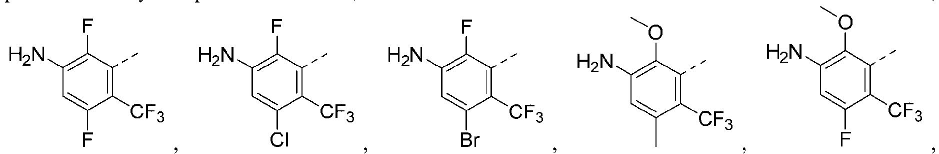

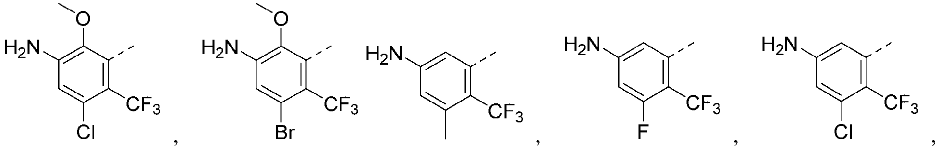

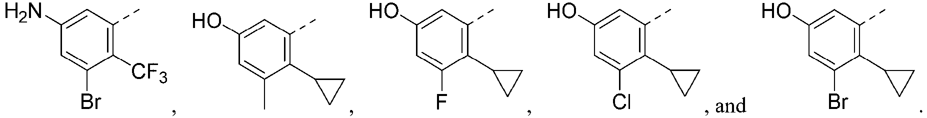

21. The compound according to claim 19, or a stereoisomer thereof, or a pharmaceutically acceptable salt thereof, which is selected from a compound of formula (I'-1'-i), or a stereoisomer thereof, or a pharmaceutically acceptable salt thereof,

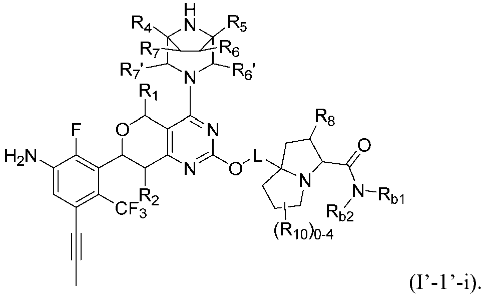

22. The compound according to claim 19, or a stereoisomer thereof, or a pharmaceutically acceptable salt thereof, which is selected from a compound of formula (I'-1'-ii), or a stereoisomer thereof, or a pharmaceutically acceptable salt thereof,

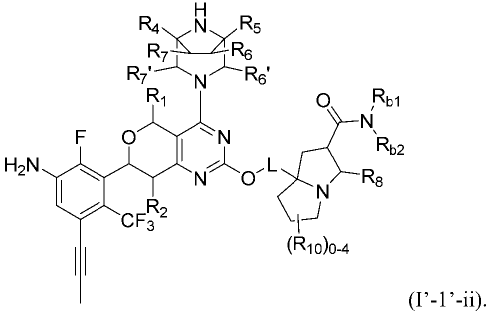

23. The compound according to claim 19, or a stereoisomer thereof, or a pharmaceutically acceptable salt thereof, which is selected from a compound of formula (I'-2'-i), or a stereoisomer thereof, or a pharmaceutically acceptable salt thereof,

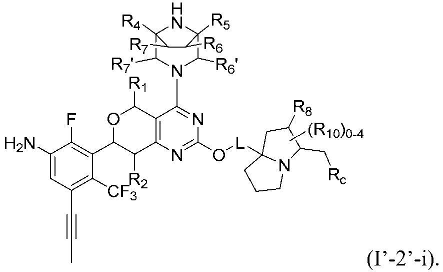

24. The compound according to claim 19, or a stereoisomer thereof, or a pharmaceutically acceptable salt thereof, which is selected from a compound of formula (I'-2'-ii), or a stereoisomer thereof, or a pharmaceutically acceptable salt thereof,

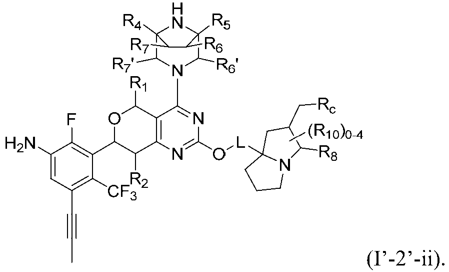

25. The compound according to claim 19, or a stereoisomer thereof, or a pharmaceutically acceptable salt thereof, which is selected from a compound of formulas (I'-3'), (I'-4'), (I'-5'), (I'-6'), (I'-7'), (I'-8'), (I'-9'), (I'-10'), (I'-11'), (I'-12') and (I'-13'), or a stereoisomer thereof, or a pharmaceutically acceptable salt thereof, 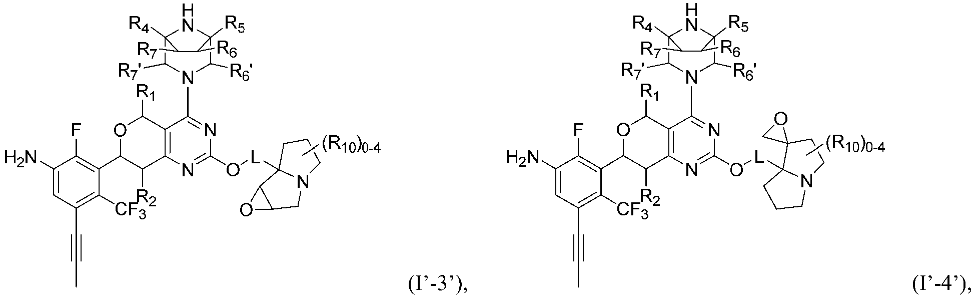 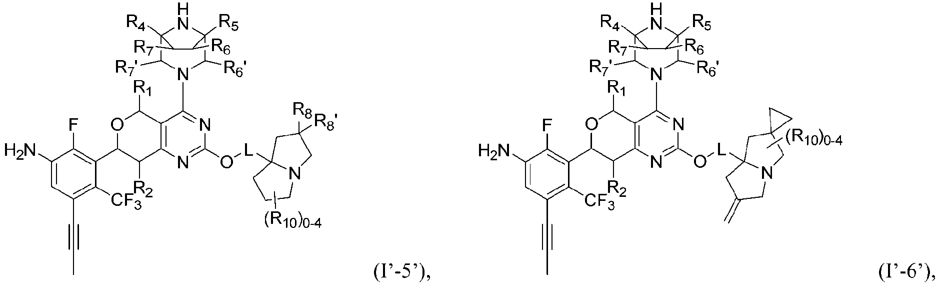 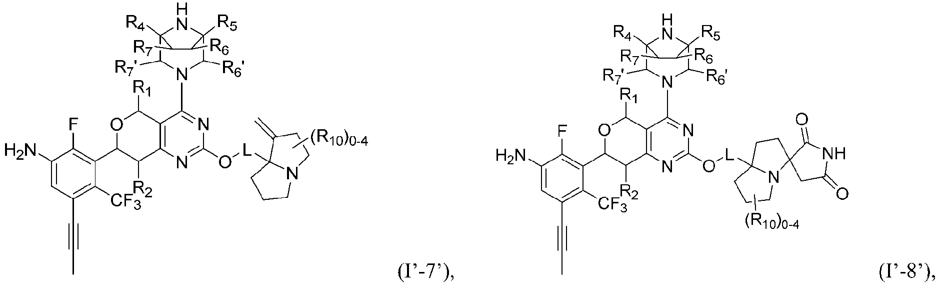 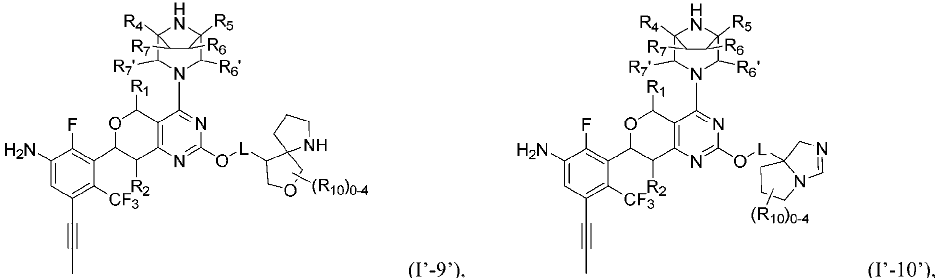 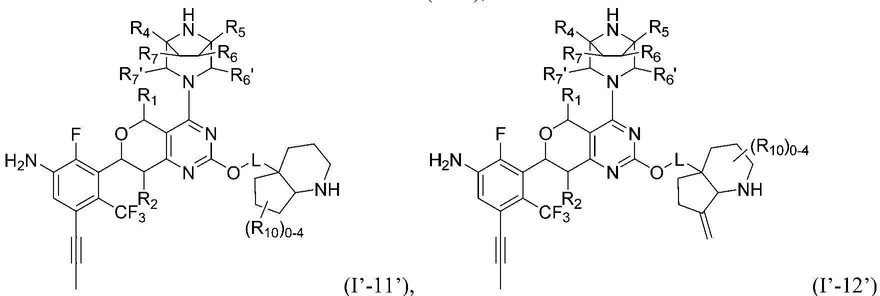 or

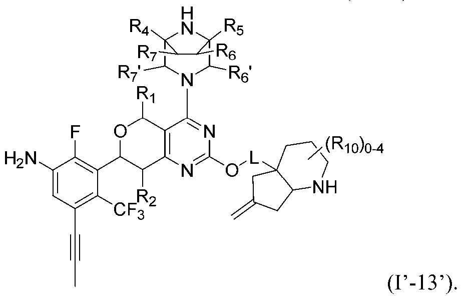

26. The compound according to claim 25, or a stereoisomer thereof, or a pharmaceutically acceptable salt thereof, wherein R₄, R₅, R₆, R₇, R_{6'} and R_{7'} are each H.

27. The compound according to claim 19, or a stereoisomer thereof, or a pharmaceutically acceptable salt thereof, which is selected from a compound of formulas (I'-14') and (I'-15'), or a stereoisomer thereof, or a pharmaceutically acceptable salt thereof,

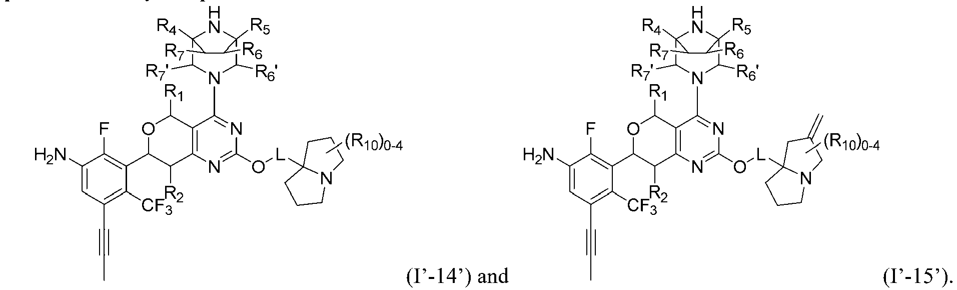

28. The compound according to any one of claims 1 to 27, or a stereoisomer thereof, or a pharmaceutically acceptable salt thereof, wherein L is -C(R_{L1}R_{L2})-, wherein R_{L1} and R_{L2} are each independently selected from H and D.

29. The compound according to any one of claims 1 to 27, or a stereoisomer thereof, or a pharmaceutically acceptable salt thereof, wherein L is -C(R_{L1}R_{L2})-, wherein R_{L1} and R_{L2} are each independently selected from H.

30. The compound according to any one of claims 1 to 27, or a stereoisomer thereof, or a pharmaceutically acceptable salt thereof, wherein L is -C(R_{L1}R_{L2})-, wherein at least one of R_{L1} and R_{L2} is selected from C₁₋₃ alkyl.

31. The compound according to any one of claims 1 to 30, or a stereoisomer thereof, or a pharmaceutically acceptable salt thereof, wherein R₁ and R₂ are selected from H.

32. The compound according to claim 1, or a stereoisomer thereof, or a pharmaceutically acceptable salt thereof, which is selected from those in Table 1.

33. The compound according to claim 32, or a stereoisomer thereof, or a pharmaceutically acceptable salt thereof, which is selected from those in Table 2 and Table 2a.

34. Use of a compound according to any one of claims 1 to 33, or a stereoisomer thereof, or a pharmaceutically acceptable salt thereof, in the manufacture of a medicament for treating a disease or condition associated with a KRAS mutation.

35. A compound according to any one of claims 1 to 33, or a stereoisomer thereof, or a pharmaceutically acceptable salt thereof, for use in treating a disease or condition associated with a KRAS mutation.

36. A method for treating a disease or condition associated with a KRAS mutation, comprising administering to a subject in need thereof a compound according to any one of claims 1 to 33, or a stereoisomer thereof, or a pharmaceutically acceptable salt thereof.

37. The use of claim 34, the compound for use of claim 35, or the method of claim 36, wherein the KRAS mutation is a KRAS^{G12D} mutation.
